(19) Europäisches Patentamt
European Patent Office
Office européen des brevets

(11) EP 2 674 423 A1

# (12) EUROPEAN PATENT APPLICATION

published in accordance with Art. 153(4) EPC

(43) Date of publication:
**18.12.2013 Bulletin 2013/51**

(21) Application number: **12745275.3**

(22) Date of filing: **09.02.2012**

(51) Int Cl.:
***C07D 401/04*** *(2006.01)* ***A01N 43/56*** *(2006.01)*
***A01N 43/78*** *(2006.01)* ***A01N 43/80*** *(2006.01)*
***A01N 43/832*** *(2006.01)* ***A01N 47/18*** *(2006.01)*
***A01N 47/36*** *(2006.01)* ***A01N 55/00*** *(2006.01)*
***A01P 7/04*** *(2006.01)* ***A61K 31/4439*** *(2006.01)*
***A61K 31/5355*** *(2006.01)* ***A61P 33/14*** *(2006.01)*
***C07D 401/14*** *(2006.01)* ***C07D 413/14*** *(2006.01)*

(86) International application number:
**PCT/JP2012/052999**

(87) International publication number:
**WO 2012/108511 (16.08.2012 Gazette 2012/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2011 JP 2011025875**
**12.05.2011 JP 2011106993**
**29.06.2011 JP 2011143871**
**11.08.2011 JP 2011176256**
**11.10.2011 JP 2011223837**
**26.10.2011 JP 2011234671**
**18.11.2011 JP 2011252596**

(71) Applicant: **Nissan Chemical Industries, Ltd.**
**Chiyoda-ku**
**Tokyo 101-0054 (JP)**

(72) Inventors:
- **NUMATA, Akira**
**Funabashi-shi, Chiba 274-8507 (JP)**
- **TANIMA, Daisuke**
**Funabashi-shi, Chiba 274-8507 (JP)**
- **ANDO, Masanori**
**Minamisaitama-gun, Saitama 349-0294 (JP)**
- **SAITO, Fumiyo**
**Funabashi-shi, Chiba 274-8507 (JP)**
- **IWAWAKI, Yuji**
**Funabashi-shi, Chiba 274-8507 (JP)**

(74) Representative: **Blodig, Wolfgang**
**Wächtershäuser & Hartz**
**Patentanwaltspartnerschaft**
**Ottostrasse 4**
**80333 München (DE)**

## (54) PYRAZOLE DERIVATIVE AND PEST CONTROL AGENT

(57) Novel pesticide, especially insecticides or miticides are provided. Pyrazole derivative and pesticides represented by the formula (1):

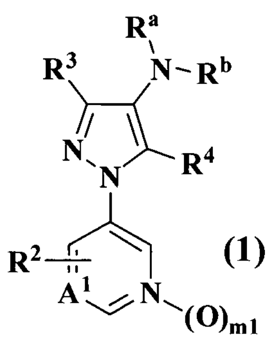

(1)

wherein $A^1$ is $-N(O)_{m2}$ or $-CR^1$, each of $R^1$, $R^3$, $R^4$ is a hydrogen atom, a halogen atom, $C_1$-$C_6$ alkyl or the like, $R^a$ is a hydrogen atom, $C_1$-$C_6$ alkyl or the like, $R^b$ is $-C(O)R^7$, $-C(O)N(R^{8a})R^8$ or the like, each of $R^7$, $R^8$, $R^{8a}$ is a hydrogen atom, $C_1$-$C_6$ alkyl or the like, and m1 is an integer of 0 or 1.



Printed by Jouve, 75001 PARIS (FR)

## Description

TECHNICAL FIELD

The present invention relates to a pesticide containing pyrazole derivatives and salts thereof and pesticides containing them as an active ingredient. In the present invention, a pesticide means an agent for controlling agriculturally, zootechnically or hygienically noxious arthropods (such as internal and external parasites in and on farm animals or pet mammals and birds and hygienic or annoying home and workplace insects) as target pests. In the present invention, an agrochemical means an insecticide, miticide, nematocide, herbicide or fungicide or the like used in the field of agriculture.

BACKGROUND ART

Although pyrazole derivatives are disclosed, for example, in Patent Documents 1 to 9, they do not disclose the pyrazole derivatives of the present invention at all. Their usefulness as pesticides, especially, as insecticides, miticides or parasticides for internal or external, mammal or bird parasites is not known at all, either.

PRIOR ART DOCUMENT

Patent Document 1: WO2011/128304
Patent Document 2: WO2009/076454
Patent Document 3: WO2008/044767
Patent Document 4: US-A-2004-214838
Patent Document 5: JP-A-2003-313103
Patent Document 6: WO99/010350
Patent Document 7: WO97/034893
Patent Document 8: JP-A-63-174905
Patent Document 9: JP-A-62-153273

DISCLOSURE OF THE INVENTION

TECHNICAL PROBLEM

With the advance of development of pesticides targeted at various pest insects such as agricultural pest insects, forest pest insects or hygienic pest insects, various pesticides have been put into practical use.

However, recently, control of pest insects with conventional insecticides or fungicides has become difficult in more and more cases, as pest insects acquire resistance to them over many years of their use. Problems of the high toxicity of some conventional pesticides and of the disturbance of the ecosystem by some conventional pesticides which remain in the environment for a long period are becoming apparent. Under these circumstances, development of novel pesticides with high pesticidal activity, low toxicity and low persistence is always expected.

SOLUTION TO PROBLEMS

As a result of their extensive studies to accomplish the above object and, the present inventors found that the novel pyrazole derivative of the present invention represented by the following formula (I) are very useful compounds which are excellent in pesticidal activities, especially in insecticidal and miticidal activities, and have little harmful effect on non-target organisms such as mammals, fish and beneficial insects and accomplished the present invention.

Namely, the present invention relates to the following [1] to [25].

[1] A pyrazole derivative represented by the formula (1):

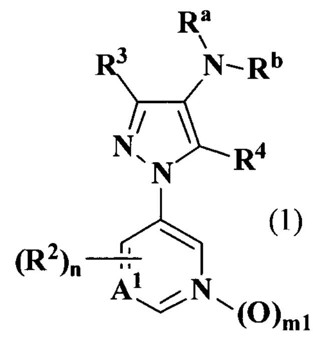

(1)

wherein $A^1$ is $-N(-O)_{m2}$ or $-CR^1$,

each of $R^1$ and $R^3$ is independently a hydrogen atom, a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkoxycarbonyl, $C_1$-$C_6$ haloalkoxycarbonyl, $C_1$-$C_6$ alkylaminosulfonyl, di($C_1$-$C_6$ alkyl)aminosulfonyl, $C_1$-$C_6$ alkylamino or di($C_1$-$C_6$ alkyl)amino,

$R^2$ is a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkoxycarbonyl, $C_1$-$C_6$ haloalkoxycarbonyl, $C_1$-$C_6$ alkylaminosulfonyl, di($C_1$-$C_6$ alkyl)aminosulfonyl, $C_1$-$C_6$ alkylamino or di($C_1$-$C_6$ alkyl)amino, provided that when n is an integer of at least 2, each $R^2$ may be identical with or different from one another,

$R^4$ is a hydrogen atom, a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkylamino or di($C_1$-$C_6$ alkyl)amino,

$R^a$ is a hydrogen atom, cyano, $C_1$-$C_{15}$ alkyl, ($C_1$-$C_{15}$) alkyl optionally substituted with $R^5$, $C_3$-$C_{15}$ cycloalkyl, ($C_3$-$C_{15}$) cycloalkyl optionally substituted with $R^5$, $C_2$-$C_{15}$ alkenyl, ($C_2$-$C_{15}$) alkenyl optionally substituted with $R^5$, $C_3$-$C_{12}$ cycloalkenyl, ($C_3$-$C_{15}$) cycloalkenyl optionally substituted with $R^5$, $C_2$-$C_{12}$ alkynyl, ($C_2$-$C_{15}$) alkynyl optionally substituted with $R^5$, $-OR^6$, $-S(O)_rR^6$, $-C(O)R^{7a}$, $-C(O)OR^{6a}$, $-NR^{8c}R^{8d}$, $-C(=NR^{8b})R^{7a}$, $-S(O)_rN(R^{8a})R^8$, phenyl, phenyl substituted with (Z)q, naphthyl, naphthyl substituted with (Z)q or D1-1 to D1-99,

$R^b$ is $-S(O)_rR^6$, $-C(O)R^7$, $-C(S)R^7$, $-C(O)OR^{6a}$, $-C(O)SR^{6a}$, $-C(S)OR^{6a}$, $-C(S)SR^{6a}$, $-C(O)N(R^{8a})R^8$, $-C(S)N(R^{8a})R^8$, $-C(O)N(R^{8b})N(R^{8a})R^8$, $-C(O)N(R^{8a})OR^{6a}$, $-C(=NR^{8b})OR^{6a}$, $-C(=NR^{8b})SR^{6a}$, $-C(=NR^{8b})N(R^{8a})R^8$, $-C(=NR^{8b})R^7$, D1-49, D1-51, D1-53, D1-59, D1-61 or D1-63 or $R^b$ may form $=C(R^{b2})R^{b3}$ together with $R^a$,

$R^{b2}$ is a hydrogen atom, $C_1$-$C_{15}$ alkyl or $-S(O)_rR^6$,

$R^{b3}$ is ($C_1$-$C_{15}$) alkyl optionally substituted with $R^{14}$, $-OR^6$, $-S(O)_rR^6$ or $-N(R^{8b})R^8$, or $R^{b3}$ may form, together with $R^{b2}$, a $C_4$-$C_6$ alkylene chain or a $C_4$-$C_6$ alkenylene chain to form a 5 to 7-membered ring together with the carbon atom attached to $R^{b3}$ and $R^{b2}$, wherein the alkylene chain or the alkenylene chain may contain from 1 to 3 oxygen atoms, sulfur atoms or nitrogen atoms and may optionally be substituted with a halogen atom, a cyano group, a nitro group, $C_1$-$C_6$ alkyl, ($C_1$-$C_{12}$) alkyl optionally substituted with $R^{14}$, $-S(O)_rR^6$, $-C(O)R^7$, $-C(S)R^7$, $-C(O)OR^{6a}$, $-C(O)SR^{6a}$, $-C(S)OR^{6a}$, $-C(S)SR^{6a}$, $-C(O)N(R^{8a})R^8$, $-C(S)N(R^{8a})R^8$, $-C(O)N(R^{8b})N(R^{8a})R^8$, $-C(O)N(R^{8a})OR^6$, $-C(=NR^{8b})OR^{6a}$, $-C(=NR^{8b})SR^{6a}$, $-C(=NR^{8b})N(R^{8a})R^8$, $-C(=NR^{8b})R^7$, phenyl, phenyl substituted with (Z)q, an oxo group, a thioxo group, $=NR^{8b}$ or a $C_1$-$C_6$ alkylidene group,

$R^5$ is a halogen atom, cyano, nitro, $C_3$-$C_{15}$ cycloalkyl, ($C_3$-$C_{15}$) cycloalkyl optionally substituted with $R^{14}$, -OH, $-OR^{11}$, -SH, $-S(O)_rR^{11}$, $-C(O)R^{12}$, $-C(O)OR^{11a}$, $-C(O)SR^{6a}$, $-C(S)OR^{6a}$, $-C(S)SR^{6a}$, $-C(O)N(R^{13a})R^{13}$, $-C(S)N(R^{13a})R^{13}$, $-C(=NOH)R^{12}$, $-C(=NOR^{11})R^{12}$, $-N(R^{13a})R^{13}$, $-Si(R^{9a})(R^{9b})R^9$, $-P(O)(OR^{10})_2$, $-P(S)(OR^{10})_2$, phenyl, phenyl substituted with $(Z^3)_q$, naphthyl, naphthyl substituted with (Z)q or D1-1 to D1-99,

each of $R^6$ and $R^{6a}$ is independently $C_1$-$C_{15}$ alkyl, ($C_1$-$C_{15}$) alkyl optionally substituted with $R^{14}$, $C_3$-$C_{15}$ cycloalkyl,

$(C_3-C_{15})$ cycloalkyl optionally substituted with $R^{14}$, $C_2-C_{15}$ alkenyl, $(C_2-C_{15})$ alkenyl optionally substituted with $R^{14}$, $C_3-C_{15}$ cycloalkenyl, $(C_3-C_{15})$ cycloalkenyl optionally substituted with $R^{14}$, $C_2-C_{15}$ alkynyl, $(C_2-C_{15})$ alkynyl optionally substituted with $R^{14}$, phenyl, phenyl substituted with (Z)q, naphthyl, naphthyl substituted with (Z)q or D1-1 to D1-99,

$R^7$ is a hydrogen atom, $C_1-C_{15}$ alkyl, $(C_1-C_{15})$ alkyl optionally substituted with $R^{14}$, $C_3-C_{15}$ cycloalkyl, $(C_3-C_{15})$ cycloalkyl optionally substituted with $R^{14}$, $C_2-C_{15}$ alkenyl, $(C_2-C_{15})$ alkenyl optionally substituted with $R^{14}$, $C_3-C_{15}$ cycloalkenyl, $(C_3-C_{15})$ cycloalkenyl optionally substituted with $R^{14}$, $C_2-C_{15}$ alkynyl, $(C_2-C_{15})$ alkynyl optionally substituted with $R^{14}$, $-C(O)R^{12}$, $-C(O)OR^{11}$, $-C(O)N(R^{13a})R^{13}$, $-C(S)N(R^{13a})R^{13}$, $-C(=NOH)R^{12}$, $-C(=NOR^{11})R^{12}$, $-C\{=NN(R^{13a})R^{13}\}R^{12}$, phenyl, phenyl substituted with (Z)q, naphthyl, naphthyl substituted with (Z)q or D1-1 to D1-99, or $R^7$ may form, together with $R^a$, a $C_2-C_6$ alkylene chain or a $C_2-C_6$ alkenylene chain containing a double bond to form a 4 to 8-membered ring together with the carbon atom attached to $R^7$ and the nitrogen atom attached to $R^a$, wherein the alkylene chain or the alkenylene chain may contain one or two oxygen atoms, sulfur atoms or nitrogen atoms and may optionally be substituted with a halogen atom, cyano, nitro, $C_1-C_6$ alkyl, $(C_1-C_6)$ alkyl optionally substituted with $R^{14}$, -OH,$-OR^{11}$, -SH, $-S(O)_rR^{11}$, an oxo group, a thioxo group, $=NR^{8b}$ or a $C_1-C_6$ alkylidene group,

$R^{7a}$ is a hydrogen atom or $R^{6a}$,

$R^8$ is a hydrogen atom, cyano, $R^{6a}$, $-S(O)_rR^{11}$, $-C(O)R^{12}$, $-C(O)OR^{11a}$, $-C(O)N(R^{13a})R^{13}$, $-C(S)N(R^{13a})R^{13}$, $-C(=NOH)R^{12}$, $-C(=NOR^{11})R^{12}$, $-S(O)_2N(R^{13a})R^{13}$, phenyl, phenyl substituted with (Z)q, naphthyl, naphthyl substituted with (Z)q or D1-1 to D1-99, or $R^8$

may form, together with $R^{8a}$, a $C_2-C_7$ alkylene chain to form a 3 to 8-membered ring together with the nitrogen atom attached to $R^8$ and $R^{8a}$, wherein the alkylene chain may contain an oxygen atom, a sulfur atom or a nitrogen atom and may optionally be substituted with a halogen atom, cyano, $C_1-C_6$ alkyl, $(C_1-C_6)$ alkyl optionally substituted with $R^{14}$, phenyl, phenyl substituted with (Z)q, an oxo group or a thioxo group, or $R^8$ may form $=C(R^{8f})R^{8e}$ together with $R^{8a}$,

$R^{8a}$ is a hydrogen atom, cyano, $C_1-C_{15}$ alkyl, $(C_1-C_{15})$ alkyl optionally substituted with $R^{14a}$, $C_3-C_{15}$ cycloalkyl, $(C_3-C_{15})$ cycloalkyl optionally substituted with $R^{14a}$, $C_2-C_{15}$ alkenyl, $(C_2-C_{15})$ alke or nyl optionally substituted with $R^{14a}$, $C_3-C_{15}$ cycloalkenyl, $(C_3-C_{15})$ cycloalkenyl optionally substituted with $R^{14a}$, $C_2-C_{15}$ alkynyl or $(C_2-C_{15})$ alkynyl optionally substituted with $R^{14a}$, or $R^{8a}$ may form, together with $R^a$, a $C_2-C_5$ alkylene chain to form a 5 to 8-membered ring together with the nitrogen atom attached to $R^{8a}$ and the nitrogen atom attached to $R^a$, wherein the alkylene chain may contain an oxygen atom, a sulfur atom or a nitrogen atom and may optionally be substituted with a halogen atom, cyano, nitro, $C_1-C_6$ alkyl, $(C_1-C_6)$ alkyl optionally substituted with $R^{14}$, -OH,$-OR^{11}$, -SH, $-S(O)_rR^{11}$, an oxo group, a thioxo group or $=NR^{8b}$,

$R^{8b}$ is a hydrogen atom, cyano, nitro, $C_1-C_{15}$ alkyl, $(C_1-C_{15})$ alkyl optionally substituted with $R^{14}$, $C_3-C_{15}$ cycloalkyl, $(C_3-C_{15})$ cycloalkyl optionally substituted with $R^{14}$, $C_2-C_{15}$ alkenyl, $(C_2-C_{15})$ alkenyl optionally substituted with $R^{14}$, $C_3-C_{15}$ cycloalkenyl, $(C_3-C_{15})$ cycloalkenyl optionally substituted with $R^{14}$, $C_2-C_{15}$ alkynyl, $(C_2-C_{15})$ alkynyl optionally substituted with $R^{14}$, $-OR^{11}$, $-S(O)_rR^{11}$, $-C(O)OR^{11a}$, $-C(O)R^{12}$, $-C(O)N(R^{13a})R^{13}$, $-C(S)N(R^{13a})R^{13}$ or $-S(O)_2N(R^{13a})R^{13}$,

each of $R^{8c}$ and $R^{8d}$ is independently a hydrogen atom, $C_1-C_6$ alkyl, $-S(O)_rR^{11}$ $-C(O)OR^{11a}$, $-C(O)R^{12}$, $-C(O)N(R^{13a})R^{13}$ or $-C(S)N(R^{13a})R^{13}$,

$R^{8e}$ is a hydrogen atom, $C_1-C_{15}$ alkyl or $-S(O)_rR^{11}$,

$R^{8f}$ is $C_1-C_{15}$ alkyl, $(C_1-C_{15})$ alkyl optionally substituted with $R^{14}$, $-OR^{11}$, $-S(O)_rR^{11}$, $-N(R^{13a})R^{13}$, phenyl, phenyl substituted with (Z)q, naphthyl, naphthyl substituted with (Z)q or or D1-1 to D1-99,

each of $R^9$, $R^{9a}$ and $R^{9b}$ is independently $C_1-C_6$ alkyl,

$R^{10}$ is a hydrogen atom or $C_1-C_6$ alkyl,

each of $R^{11}$ and $R^{11a}$ is independently $C_1-C_6$ alkyl, $(C_1-C_6)$ alkyl optionally substituted with $R^{14a}$, $C_2-C_6$ alkenyl, $(C_2-C_6)$ alkenyl optionally substituted with $R^{14a}$, $C_3-C_8$ cycloalkenyl, $(C_3-C_8)$ cycloalkenyl optionally substituted with $R^{14a}$, $C_2-C_6$ alkynyl, $(C_2-C_6)$ alkynyl optionally substituted with $R^{14a}$, $C_3-C_8$ cycloalkyl, $(C_3-C_8)$ cycloalkyl optionally substituted with $R^{14a}$, $-Si(R^{9a})(R^{9b})R^9$, phenyl, phenyl substituted with (Z)q, naphthyl, naphthyl substituted with (Z)q or or D1-1 to D1-99,

$R^{12}$ is a hydrogen atom, $R^{11a}$, $-C(O)R^{16}$, $-C(=NOH)R^{16}$, $-C(=NOR^{15})R^{16}$, phenyl, phenyl substituted with (Z)q, naphthyl, naphthyl substituted with (Z)q or or D1-1 to D1-99,

each of $R^{13}$ and $R^{13a}$ is independently a hydrogen atom, $R^{11a}$, $-S(O)_rR^{15}$, $-C(O)OR^{15a}$, $-C(O)R^{16}$, $-C(O)N(R^{17a})R^{17}$, $-C(S)N(R^{17a})R^{17}$, $-S(O)_2N(R^{17a})R^{17}$, phenyl, phenyl substituted with (Z)q, naphthyl, naphthyl substituted with (Z)q or or D1-1 to D1-99, or $R^{13}$ and $R^{13a}$ may form, together with each other, a $C_2-C_7$ alkylene chain to form a 3 to 8-membered ring together with the nitrogen atom attached to $R^{13}$ and $R^{13a}$, wherein the alkylene chain may contain an oxygen atom, a sulfur atom or a nitrogen atom and may optionally be substituted with a halogen atom, cyano, $C_1-C_6$ alkyl, $(C_1-C_6)$ alkyl optionally substituted with $R^{14}$, phenyl, phenyl substituted with (Z)q, an oxo group or a thioxo group,

each of $R^{14}$ and $R^{14a}$ is independently a halogen atom, cyano, nitro, $C_3-C_8$ cycloalkyl, $(C_3-C_8)$ cycloalkyl optionally substituted with $R^{19}$, -OH, $-OR^{15}$, -SH, $-S(O)_rR^{15}$, $-S(=NR^{17b})R^{15a}$, $-S(O)(=NR^{17b})R^{15a}$, -C(O)OH, $-C(O)OR^{15a}$,

$-C(O)SR^{15a}$, $-C(S)OR^{15a}$, $-C(S)SR^{15a}$, $-C(O)R^{16}$, $-C(O)N(R^{17a})R^{17}$, $-C(S)N(R^{17a})R^{17}$, $-N(R^{17a})R^{17}$, $-C(=NOH)R^{16}$, $-C(=NOR^{15})R^{16}$, $-ON=C(R^{16a})R^{16}$, $-S(O)_2N(R^{17a})R^{17}$, $-Si(R^{9a})(R^{9b})R^9$, $-P(O)(OR^{10})_2$, $-P(S)(OR^{10})_2$, phenyl, phenyl substituted with (Z)q, naphthyl, naphthyl substituted with (Z)q or D1-1 to D1-99,

each of $R^{15}$ and $R^{15a}$ is independently cyano, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, $-S(O)_rR^{20}$, $-C(O)OR^{20a}$, $-C(O)R^{21}$, $-C(O)N(R^{22a})R^{22}$, $-C(S)N(R^{22a})R^{22}$, phenyl, phenyl substituted with $(Z)_{q2}$, naphthyl, naphthyl substituted with (Z)q or D1-1 to D1-99,

each of $R^{16}$ and $R^{16a}$ is independently a hydrogen atom, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, $-C(O)R^{21}$, phenyl, phenyl substituted with (Z)q, naphthyl, naphthyl substituted with (Z)q or D1-1 to D1-99,

each of $R^{17}$ and $R^{17a}$ is independently a hydrogen atom, cyano, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, -CHO, $-S(O)_rR^{20}$, $-C(O)R^{21}$, $-C(O)OR^{20a}$, $-C(O)N(R^{22a})R^{22}$, $-C(S)N(R^{22a})R^{22}$ or $-S(O)_2N(R^{22a})R^{22}$, or $R^{17}$ and $R^{17a}$ may form, together with each other, a $C_2$-$C_7$ alkylene chain to form a 3 to 8-membered ring together with the nitrogen atom attached to $R^{17}$ and $R^{17a}$, wherein the alkylene chain may contain an oxygen atom, a sulfur atom or a nitrogen atom and may optionally be substituted with a halogen atom, cyano, $C_1$-$C_6$ alkyl, ($C_1$-$C_6$) alkyl optionally substituted with $R^{14}$, phenyl, phenyl substituted with (Z)q, an oxo group or a thioxo group,

$R^{17b}$ is a hydrogen atom, cyano, nitro, $-OR^{20}$, $-S(O)_rR^{20}$, $-C(O)OR^{20a}$, $-C(O)R^{21}$, $-C(O)N(R^{22a})R^{22}$, $-C(S)N(R^{22a})R^{22}$ or $-S(O)_2N(R^{22a})R^{22}$,

$R^{19}$ is a halogen atom, cyano, $C_3$-$C_8$ cycloalkyl, $-OR^{20}$, $-S(O)_rR^{20}$, $-C(O)OR^{20a}$, $-C(O)N(R^{22a})R^{22}$, $-C(S)N(R^{22a})R^{22}$, $-C(=NOR^{20})R^{21}$, $-Si(R^{9a})(R^{9b})R^9$, phenyl, phenyl substituted with (Z)q, naphthyl, naphthyl substituted with (Z)q or D1-1 to D1-99,

each of $R^{20}$ and $R^{21}$ is independently a hydrogen atom, $C_1$-$C_6$ alkyl, ($C_1$-$C_6$) alkyl optionally substituted with $R^{32}$, $C_2$-$C_6$ alkenyl, ($C_2$-$C_6$) alkenyl optionally substituted with $R^{32}$, $C_2$-$C_6$ alkynyl, ($C_2$-$C_6$) alkynyl optionally substituted with $R^{32}$, $C_3$-$C_8$ cycloalkyl, ($C_3$-$C_8$) cycloalkyl optionally substituted with $R^{32}$, $C_3$-$C_8$ cycloalkenyl, ($C_3$-$C_8$) cycloalkenyl optionally substituted with $R^{32}$, phenyl, phenyl substituted with (Z)q, naphthyl, naphthyl substituted with (Z)q or D1-1 to D1-99,

each of $R^{20a}$, $R^{22}$ and $R^{22a}$ is independently a hydrogen atom, $C_1$-$C_6$ alkyl, ($C_1$-$C_6$) alkyl optionally substituted with $R^{32}$, $C_2$-$C_6$ alkenyl, ($C_2$-$C_6$) alkenyl optionally substituted with $R^{32}$, $C_2$-$C_6$ alkynyl, ($C_2$-$C_6$) alkynyl optionally substituted with $R^{32}$, $C_3$-$C_8$ cycloalkyl, ($C_3$-$C_8$) cycloalkyl optionally substituted with $R^{32}$, $C_3$-$C_8$ cycloalkenyl, ($C_3$-$C_8$) cycloalkenyl optionally substituted with $R^{32}$, phenyl, phenyl substituted with (Z)q, naphthyl, naphthyl substituted with (Z)q or D1-1 to D1-99,

D1-1 to D1-99 are rings represented by the following structures, respectively,

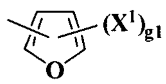
D1-1

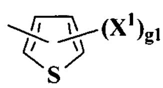
D1-2

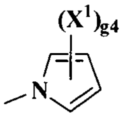
D1-3

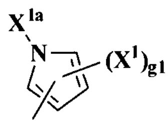
D1-4

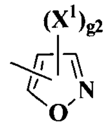
D1-5

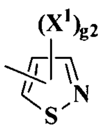
D1-6

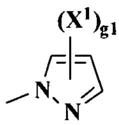
D1-7

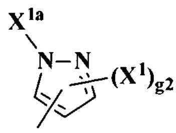
D1-8

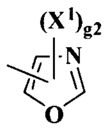
D1-9

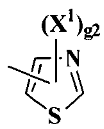
D1-10

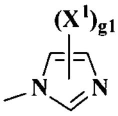
D1-11

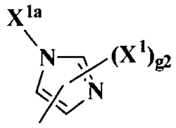
D1-12

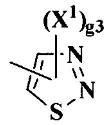
D1-13

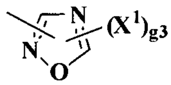
D1-14

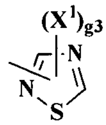
D1-15

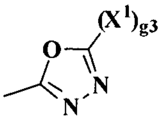
D1-16

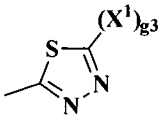
D1-17

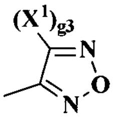
D1-18

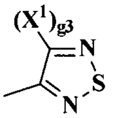
D1-19

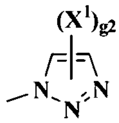
D1-20

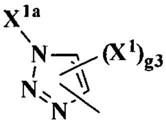
D1-21

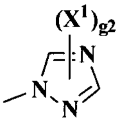
D1-22

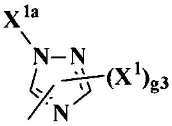
D1-23

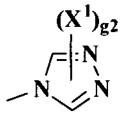
D1-24

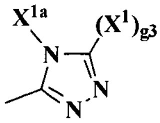
D1-25

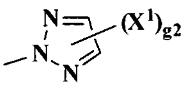
D1-26

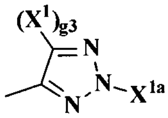
D1-27

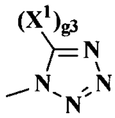
D1-28

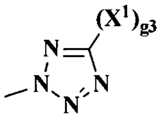
D1-29

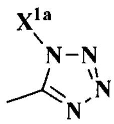
D1-30

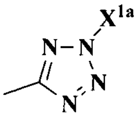
D1-31

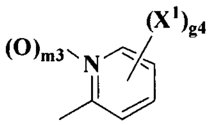
D1-32

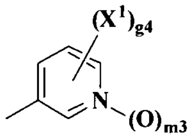
D1-33

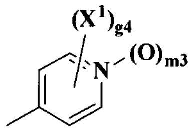
D1-34

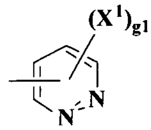
D1-35

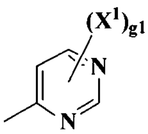
D1-36

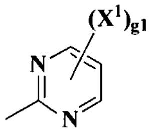
D1-37

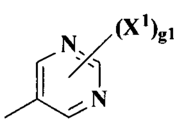
D1-38

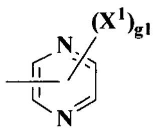
D1-39

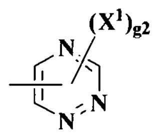
D1-40

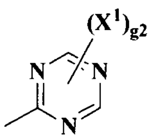
D1-41

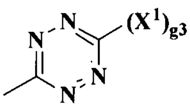
D1-42

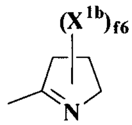
D1-43

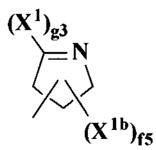
D1-44

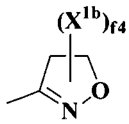
D1-45

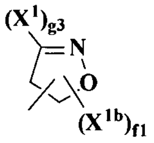
D1-46

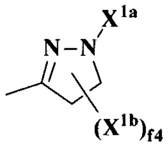
D1-47

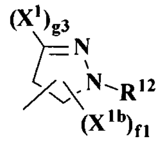
D1-48

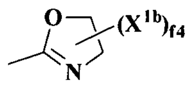
D1-49

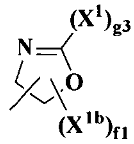
D1-50

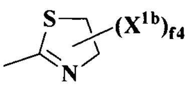
D1-51

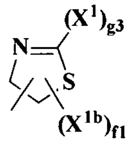
D1-52

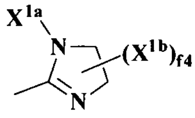
D1-53

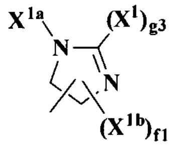
D1-54

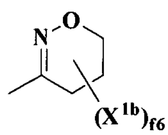
D1-55

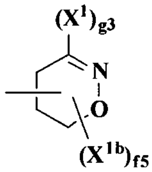
D1-56

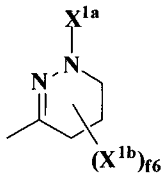
D1-57

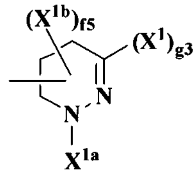
D1-58

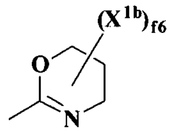
D1-59

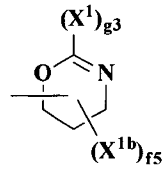
D1-60

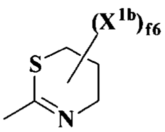
D1-61

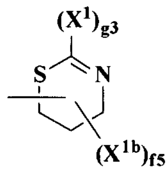
D1-62

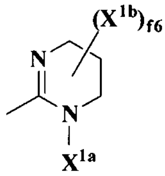
D1-63

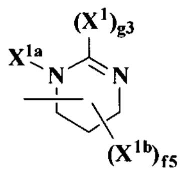
D1-64

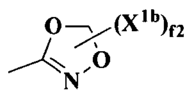
D1-65

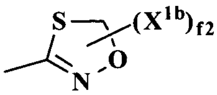
D 1-66

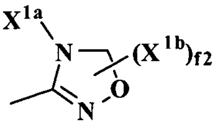
D1-67

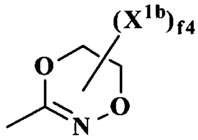
D 1-68

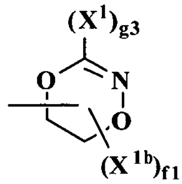
D 1-69

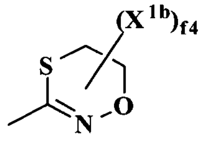
D 1-70

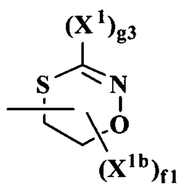
D1-71

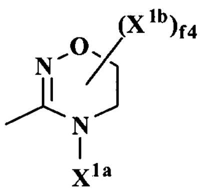
D1-72

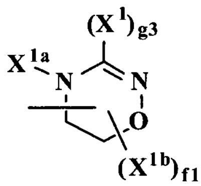
D1-73

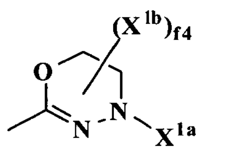
D1-74

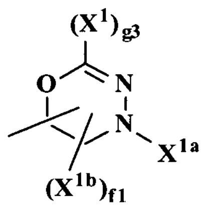
D1-75

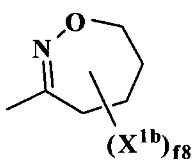 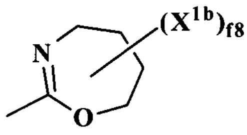 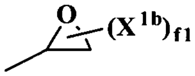 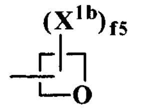 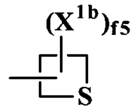

D1-76 D1-77 D1-78 D1-79 D1-80

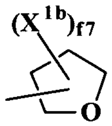 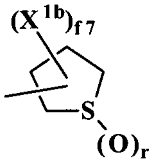 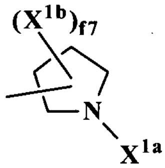 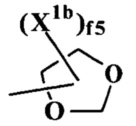 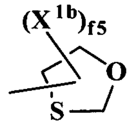

D1-81 D1-82 D1-83 D1-84 D1-85

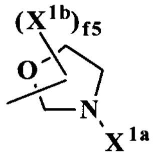 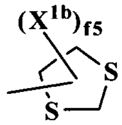 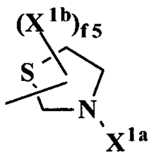 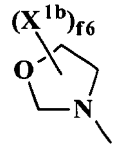 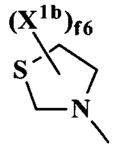

D1-86 D1-87 D1-88 D1-89 D1-90

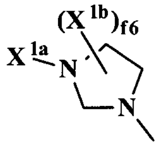 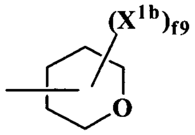 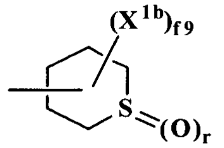 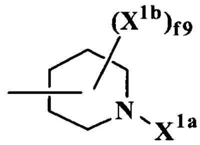 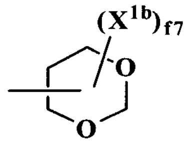

D1-91 D1-92 D1-93 D1-94 D1-95

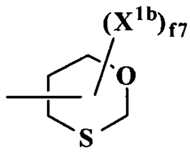 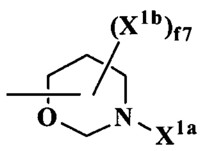 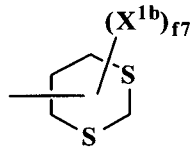 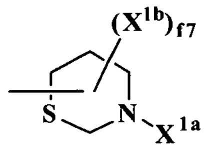

D1-96 D1-97 D1-98 D1-99

$X^1$ is a halogen atom, cyano, nitro, -OH, -SH or $R^{24}$, provided that when g1, g2 or g4 is an integer of at least 2, each $X^1$ may be identical with or different from one another, and when there are two neighboring $X^1$'s, the two neighboring $X^1$'s may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon atoms attached to the $X^1$'s, a 5-membered ring or 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$X^{1a}$ is a hydrogen atom, cyano, -OH or $R^{24}$,

$X^{1b}$ is a halogen atom, cyano, nitro, -OH, -SH or $R^{24}$, provided that when f1, f2, f4, f5, f6, f7, f8 or f9 is an integer of at least 2, each $X^{1b}$ may be identical with or different from one another, and when there are two $X^{1b}$'s on the same carbon, the two $X^{1b}$'s may form oxo, thioxo, imino, $C_1$-$C_6$ alkylimino, $C_1$-$C_6$ alkoxyimino or $C_1$-$C_6$ alkylidene together with each other,

Z is a halogen atom, cyano, nitro, -OH, -SH or $R^{24}$, provided that q is an integer of at least 2, each Z may be identical with or different from one another, and when there are two neighboring Z's, the two neighboring Z's may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon

atoms attached to the Z's, a 5-membered ring or a 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$Z^3$ is a halogen atom, nitro, -OH, -SH or $R^{24}$, provided that q is an integer of at least 2, each $Z^3$ may be identical with or different from one another, and when there are two neighboring $Z^3$'s, the neighboring two Z's may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon atoms attached to the $Z^3$'s, a 5-membered ring or a 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$R^{24}$ is a $C_1$-$C_6$ alkyl, ($C_1$-$C_6$) alkyl optionally substituted with $R^{28}$, $C_2$-$C_6$ alkenyl, ($C_2$-$C_6$) alkenyl optionally substituted with $R^{28}$, $C_3$-$C_8$ cycloalkenyl, ($C_3$-$C_8$) cycloalkenyl optionally substituted with $R^{28}$, $C_2$-$C_6$ alkynyl, ($C_2$-$C_6$) alkynyl optionally substituted with $R^{28}$, $C_3$-$C_8$ cycloalkyl, ($C_3$-$C_8$) cycloalkyl optionally substituted with $R^{28}$, $-OR^{25}$, $-S(O)_rR^{25}$, -C(O)OH, $-C(O)OR^{25a}$, $-C(O)R^{26}$, $-C(O)N(R^{27a})R^{27}$, $-C(S)N(R^{27a})R^{27}$, $-N(R^{27a})R^{27}$, - $C(=NOR^{25})R^{26}$ or $-S(O)_2N(R^{27a})R^{27}$,

each of $R^{25}$, $R^{25a}$ and $R^{26}$ is independently $C_1$-$C_6$ alkyl or ($C_1$-$C_6$) alkyl optionally substituted with $R^{32}$a, each of $R^{27}$ and $R^{27a}$ is independently a hydrogen atom, $C_1$-$C_6$ alkyl, $-S(O)_rR^{34}$ or $-C(O)R^{34}$,

each of $R^{28}$ and $R^{28a}$ is independently a halogen atom, -OH, $-OR^{29}$, -SH or $-S(O)_rR^{29}$,

each of $R^{29}$ and $R^{33}$ is independently $C_1$-$C_6$ alkyl,

each of $R^{32}$ and $R^{32a}$ is independently a halogen atom, $-OR^{33}$ or $-S(O)_rR^{33}$,

$R^{34}$ is $C_1$-$C_6$ alkyl, $C_3$-$C_8$ cycloalkyl or $C_1$-$C_6$ haloalkyl,

each of n, g1 and f1 is independently an integer of from 0 to 3,

each of g2 and f2 is independently an integer of from 0 to 2,

g3 is an integer of from 0 to 1,

each of g4 and f4 is independently an integer of from 0 to 4,

f5 is an integer of from 0 to 5,

f6 is an integer of from 0 to 6,

f7 is an integer of from 0 to 7,

f8 is an integer of from 0 to 8,

f9 is an integer of from 0 to 9,

q is independently an integer of from1 to 5,

q2 is an integer of from 0, 1 or 3 to 5,

each of m1, m2 and m3 is independently an integer of from 0 or 1, and

r is an integer of from 0 to 2.

[2] The pyrazole derivative or a salt thereof according to [1], wherein $R^b$ is $-C(O)R^7$, $-C(S)R^7$, $-C(O)OR^{6a}$, $-C(O)SR^{6a}$, $-C(S)OR^{6a}$, $-C(S)SR^{6a}$, $-C(O)N(R^{8a})R^8$, $-C(S)N(R^{8a})R^8$, $-C(O)N(R^{8b})N(R^{8a})R^8$, $-C(O)N(R^{8a})OR^{6a}$, $-C(=NR^{8b})OR^{6a}$, $-C(=NR^{8b})SR^{6a}$, $-C(=NR^{8b})N(R^{8a})R^8$, $-C(=NR^{8b})R^7$, D1-49, D1-51, D1-53, D1-59, D1-61 or D1-63, or $R^b$ may form $=C(R^{b2})R^{b3}$ together with $R^a$.

[3] The pyrazole derivative or a salt thereof according to [2], wherein $A^1$ is $-CR^1$, each of $R^1$ and $R^3$ is independently a hydrogen atom, a halogen atom, cyano, $-NH_2$, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfinyl, $C_1$-$C_6$ alkylsulfonyl or $C_1$-$C_6$ alkoxycarbonyl,

$R^2$ is a halogen atom, $C_1$-$C_6$ alkyl or $C_1$-$C_6$ alkoxy, and

$R^4$ is a hydrogen atom, a halogen atom, cyano, $-NH_2$, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfinyl or $C_1$-$C_6$ alkylsulfonyl.

[4] The pyrazole derivative or a salt thereof according to [2], wherein $A^1$ is $-N(-O)_{m2}$, $R^2$ is a halogen atom, $C_1$-$C_6$ alkyl or $C_1$-$C_6$ alkoxy, and

each of $R^3$ and $R^4$ is independently a hydrogen atom, a halogen atom, $C_1$-$C_6$ alkyl or $C_1$-$C_6$ alkoxy.

[5] The pyrazole derivative or a salt thereof according to [3], wherein $R^1$ is a hydrogen atom, a halogen atom, cyano, $-NH_2$, $C_1$-$C_6$ alkyl, $C_1$-$C_6$ haloalkyl or $C_1$-$C_6$ alkoxy,

$R^2$ is $C_1$-$C_6$ alkyl,

$R^3$ is a hydrogen atom, a halogen atom, cyano, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfonyl or $C_1$-$C_6$ alkoxycarbonyl,

$R^4$ is a hydrogen atom, a halogen atom, $C_1$-$C_6$ alkyl or $C_1$-$C_6$ alkylthio,

$R^a$ is a hydrogen atom, $C_1$-$C_6$ alkyl, ($C_1$-$C_6$) alkyl optionally substituted with $R^5$, $C_2$-$C_6$ alkenyl, $C_2$-$C_6$ alkynyl, $-S(O)_rR^6$, $-C(O)R^{7a}$, $-C(O)OR^{6a}$, $-NR^{8c}R^{8d}$ or $-C(=NR^{8b})R^{7a}$, $R^b$ is $-C(O)R^7$, $-C(S)R^7$, $-C(O)OR^{6a}$, $-C(O)SR^{6a}$, $-C(O)N(R^{8a})R^8$, $-C(S)N(R^{8a})R^8$, $-C(O)N(R^{8b})N(R^{8a})R^8$, $-C(=NR^{8b})SR^{6a}$, $-C(=NR^{8b})N(R^{8a})R^8$, $-C(=NR^{8b})R^7$ or

D1-51, or $R^b$ may form $=C(R^{b2})R^{b3}$ together with $R^a$,

$R^{b2}$ is a hydrogen atom, $C_1$-$C_6$ alkyl or $-S(O)_rR^6$,

$R^{b3}$ is ($C_1$-$C_6$) alkyl optionally substituted with $R^{14}$, $-OR^6$ or $-N(R^{8b})R^8$, or $R^{b3}$ may form, together with $R^{b2}$, a $C_4$ alkylene chain to form a 5-membered ring together with the carbon atom attached to $R^{b3}$ and $R^{b2}$, wherein the alkylene chain may contain a sulfur atom and a nitrogen atom and may optionally be substituted with $-C(O)R^7$,

$R^5$ is a halogen atom, cyano, $C_3$-$C_8$ cycloalkyl, $-OR^{11}$, $-S(O)_rR^{11}$, $-C(O)OR^{11a}$, $-C(=NOR^{11})R^{12}$, $-Si(R^{9a})(R^{9b})R^9$, phenyl or D1-37,

$R^6$ is $C_1$-$C_6$ alkyl or ($C_1$-$C_6$) alkyl optionally substituted with $R^{14}$,

$R^{6a}$ is $C_1$-$C_6$ alkyl or phenyl substituted with (Z)q,

$R^7$ is $C_1$-$C_{15}$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$) alkenyl optionally substituted with $R^{14}$, $C_3$-$C_8$ cycloalkenyl, $C_2$-$C_6$ alkynyl, ($C_2$-$C_6$) alkynyl optionally substituted with $R^{14}$, $-C(O)OR^{11a}$, $-C(O)R^{12}$, $-C(O)N(R^{13a})R^{13}$, $-C(=NOR^{11})R^{12}$, phenyl substituted with (Z)q, D1-1, D1-2, D1-4, D1-5, D1-6, D1-8, D1-9, D1-10, D1-12, D1-13, D1-19, D1-32, D1-33, D1-35, D1-38, D1-45, D1-81, D1-82, D1-87, D1-88, D1-92 or D1-94, or $R^7$ may form, together with $R^a$, a $C_2$-$C_4$ alkylene chain or a $C_2$-$C_4$ alkenylene chain containing a double bond to form a 4 to 6-membered ring together with the carbon atom attached to $R^7$ and the nitrogen atom attached to $R^a$, wherein the alkylene chain may contain one or two oxygen atoms or nitrogen atoms and may optionally be substituted with a halogen atom, $C_1$-$C_6$ alkyl, ($C_1$-$C_6$) alkyl optionally substituted with $R^{14}$, $-OR^{11}$, $-S(O)_rR^{11}$, an oxo group or a methylidene group,

$R^{7a}$ is a hydrogen atom or $C_1$-$C_6$ alkyl,

$R^8$ is a hydrogen atom, $C_1$-$C_6$ alkyl, ($C_1$-$C_6$) alkyl optionally substituted with $R^{14}$, $C_3$-$C_8$ cycloalkyl, $C_2$-$C_6$ alkenyl, $C_2$-$C_6$ alkynyl, $-C(O)R^{12}$, $-C(O)OR^{11a}$, $-C(=NOR^{11})R^{12}$, $-S(O)_2R^{11a}$, phenyl, phenyl substituted with (Z)q, D1-32, D1-33, D1-34 or D1-80,

$R^{8a}$ is a hydrogen atom, $C_1$-$C_6$ alkyl or ($C_1$-$C_6$) alkyl optionally substituted with $R^{14a}$, or $R^{8a}$ may form, together with $R^a$, a $C_2$-$C_3$ alkylene chain to form a 5 to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group, or $R^8$ and $R^{8a}$ may form, together with each other, a $C_5$ alkylene chain to form a 6-membered ring together with the nitrogen atom attached to $R^8$ and $R^{8a}$, wherein the alkylene chain may optionally be substituted with $C_1$-$C_6$ alkyl, or $R^8$ may form $=C(R^{8f})R^{8e}$ together with $R^{8a}$, $R^{8b}$ is a hydrogen atom, cyano, $C_1$-$C_6$ alkyl or $-OR^{11}$,

$R^{8c}$ is a hydrogen atom, $C_1$-$C_6$ alkyl or $-C(O)OR^{11a}$,

$R^{8d}$ is a hydrogen atom, $-C(O)OR^{11a}$ or $-C(O)R^{12}$,

$R^{8e}$ is a hydrogen atom,

$R^{8f}$ is $-N(R^{13a})R^{13}$,

each of $R^9$, $R^{9a}$, $R^{9b}$ and $R^{10}$ is independently $C_1$-$C_6$ alkyl,

$R^{11}$ is $C_1$-$C_6$ alkyl, ($C_1$-$C_6$) alkyl optionally substituted with $R^{14a}$ or $C_1$-$C_6$ alkenyl,

$R^{11a}$ is $C_1$-$C_6$ alkyl, ($C_1$-$C_6$) alkyl optionally substituted with $R^{14a}$ or phenyl,

$R^{12}$ is a hydrogen atom, $C_1$-$C_6$ alkyl, ($C_1$-$C_6$) alkyl optionally substituted with $R^{14a}$, $C_3$-$C_8$ cycloalkyl, $-C(=NOR^{15})R^{16}$ or D1-2,

$R^{13}$ is $C_1$-$C_6$ alkyl or phenyl,

$R^{13a}$ is a hydrogen atom or $C_1$-$C_6$ alkyl,

$R^{14}$ is a halogen atom, cyano, $C_3$-$C_8$ cycloalkyl, -OH, $-OR^{15}$, -SH, $-S(O)_rR^{15}$, $-S(=NR^{17b})R^{15a}$, $-S(O)(=NR^{17b})R^{15a}$, -C(O)OH, $-C(O)OR^{15a}$, $-C(O)N(R^{17a})R^{17}$, $-C(=NOR^{15})R^{16}$, $-N(R^{17a})R^{17}$, $-ON=C(R^{16a})R^{16}$, $-S(O)_2N(R^{17a})R^{17}$, $-Si(R^{9a})(R^{9b})R^9$, $-P(O)(OR^{10})_2$, phenyl, phenyl substituted with (Z)q, D1-1, D1-2, D1-5, D1-7, D1-8, D1-28, D1-32, D1-33, D1-34, D1-84, D1-85, D1-87, D1-93 or D1-98,

$R^{14a}$ is a halogen atom, $-OR^{15}$, $-S(O)_rR^{15}$, $-Si(R^{9a})(R^{9b})R^9$ or phenyl,

$R^{15}$ is cyano, $C_1$-$C_6$ alkyl, $C_2$-$C_6$ alkenyl, $C_2$-$C_6$ alkynyl, ($C_1$-$C_6$) alkyl optionally substituted with $R^{19}$, $-C(O)R^{21}$, phenyl substituted with $(Z)_{q2}$, D1-12, D1-32, D1-37 or D1-51,

$R^{15a}$ is $C_1$-$C_6$ alkyl,

$R^{16}$ is $C_1$-$C_6$ alkyl or ($C_1$-$C_6$) alkyl optionally substituted with $R^{19}$,

$R^{16a}$ is a hydrogen atom,

$R^{17}$ is a hydrogen atom, $C_1$-$C_6$ alkyl, ($C_1$-$C_6$) alkyl optionally substituted with $R^{19}$, $-S(O)_rR^{20}$, $-C(O)OR^{20a}$, $-C(O)R^{21}$, $-C(O)N(R^{22a})R^{22}$, $-C(S)N(R^{22a})R^{22}$, $-C(=NOR^{20})R^{21}$, $-S(O)_2N(R^{22a})R^{22}$ or phenyl,

$R^{17a}$ is a hydrogen atom or $C_1$-$C_6$ alkyl, or $R^{17a}$ may form $=C(R^{17c})R^{17d}$ together with $R^{17}$, $R^{17b}$ is cyano,

$R^{17c}$ is $-N(R^{22a})R^{22}$,

$R^{17d}$ is a hydrogen atom,

$R^{19}$ is a halogen atom, cyano, $C_3$-$C_8$ cycloalkyl, $-OR^{20}$, $-S(O)_rR^{20}$, $-C(O)OR^{20a}$, $-C(O)N(R^{22a})R^{22}$, $-C(=NOR^{20})R^{21}$, $-Si(R^{9a})(R^{9b})R^9$, phenyl or D1-34, $-C(O)N(R^{22a})R^{22}$, $-C(=NOR^{20})R^{21}$, $-Si(R^{9a})(R^{9b})R^9$, phenyl or D1-34,

$R^{20}$ is $C_1$-$C_6$ alkyl or ($C_1$-$C_6$) alkyl optionally substituted with $R^{32}$,

$R^{20a}$ is $C_1$-$C_6$ alkyl,
$R^{21}$ is a hydrogen atom, $C_1$-$C_6$ alkyl or ($C_1$-$C_6$) alkyl optionally substituted with $R^{32}$,
$R^{22}$ is $C_1$-$C_6$ alkyl,
$R^{22a}$ is a hydrogen atom or $C_1$-$C_6$ alkyl,
$X^1$ is a halogen atom, cyano, nitro, $C_1$-$C_6$ alkyl, ($C_1$-$C_6$) alkyl optionally substituted with $R^{28}$, $C_3$-$C_8$ cycloalkyl, -$OR^{25}$, -$S(O)_rR^{25}$, -$C(O)R^{26}$, -C(=$NOR^{25}$)$R^{26}$ or -$S(O)_2N(R^{27a})R^{27}$, provided that when g2 is an integer of 2, each $X^1$ may be identical with or different from each other,
$X^{1a}$ is $C_1$-$C_6$ alkyl, ($C_1$-$C_6$) alkyl optionally substituted with $R^{28}$, -$C(O)OR^{25a}$ or -$C(O)R^{26}$, $X^{1b}$ is ($C_1$-$C_6$) alkyl optionally substituted with $R^{28}$, provided that when f5 is an integer of 3, each $X^{1b}$ may be identical with or different from one another, and when there are two $X^{1b}$'s on the same carbon, the two $X^{1b}$'s may form oxo together with each other,
Z is a halogen atom, nitro, $C_1$-$C_6$ alkyl, ($C_1$-$C_6$) alkyl optionally substituted with $R^{28}$, -$S(O)_rR^{25}$, -C(O)OH or -$C(O)N(R^{27a})R^{27}$, provided that when q is an integer of at least 2, and there are two neighboring Z's, the two neighboring Z's may form -$OCH_2O$- to form, together with the carbon atoms attached to the Z's, a 5-membered ring which may have one or more hydrogen atoms on the ring-constituting carbon atoms optionally replaced by one or more halogen atoms,
each of $R^{25}$ and $R^{26}$ is independently $C_1$-$C_6$ alkyl or ($C_1$-$C_6$) alkyl optionally substituted with $R^{32a}$,
each of $R^{25a}$, $R^{29}$, $R^{33}$ and $R^{34}$ is independently $C_1$-$C_6$ alkyl,
$R^{27}$ is a hydrogen atom or -$S(O)_rR^{34}$,
$R^{27a}$ is a hydrogen atom,
each of $R^{28}$ and $R^{28a}$ is independently a halogen atom, -OH, -$OR^{29}$ or -$S(O)_rR^{29}$,
$R^{32}$ is a halogen atom, -$OR^{33}$ or -$S(O)_rR^{33}$,
$R^{32a}$ is a halogen atom or -$S(O)_rR^{33}$,
n is an integer of from 0 to 1,
each of g1 and g2 is independently an integer of from 0 to 2,
each of g3, f7, f9 and m3 is independently an integer of 0,
g4 is an integer of from 0 to 1,
f5 is an integer of from 0 to 3,
q is an integer of from 1 to 2,
q2 is an integer of 1, and
r is an integer of from 0 to 2.

[6] The pyrazole derivative or a salt thereof according to [3], wherein $R^1$, $R^2$ and $R^4$ are hydrogen atoms,
$R^a$ is a hydrogen atom or $C_1$-$C_6$ alkyl,
$R^b$ is -$C(O)R^7$, -$C(O)OR^{6a}$, -$C(O)N(R^{8a})R^8$ or -C(=$NR^{8b}$)$N(R^{8a})R^8$,
each of $R^{6a}$, $R^{11}$ and $R^{15}$ is independently $C_1$-$C_6$ alkyl,
$R^7$ is $C_1$-$C_6$ alkyl, $C_1$-$C_6$ alkyl optionally substituted with $R^{14}$, $C_1$-$C_6$ alkenyl optionally substituted with $R^{14}$ or D1-8, or $R^7$ may form, together with $R^a$, a $C_3$-$C_4$ alkylene chain or a $C_3$-$C_4$ alkenylene chain containing a double bond to form a 5 to 6-membered ring together with the carbon atom attached to $R^7$ and the nitrogen atom attached to $R^a$, wherein the alkylene chain may contain an oxygen atom and may optionally be substituted with a halogen atom, ($C_1$-$C_6$) alkyl optionally substituted with $R^{14}$ or -$S(O)_rR^{11}$, $R^8$ is $C_1$-$C_6$ alkyl or $C_1$-$C_6$ alkyl optionally substituted with $R^{14}$,
$R^{8a}$ is a hydrogen atom, or $R^{8a}$ may form, together with $R^a$, a $C_2$ alkylene chain to form a 6-membered ring together with the nitrogen atom attached to $R^{8a}$ and the nitrogen atom attached to $R^a$, wherein the alkylene chain may contain an oxygen atom,
$R^{8b}$ is cyano,
$R^{14}$ is a halogen atom, -OH, -$S(O)_rR^{15}$ or phenyl substituted with (Z)q,
each of $X^{1a}$ and Z is independently $C_1$-$C_6$ haloalkyl,
g2 is an integer of 0,
q is an integer of 1, and
r is an integer of from 0 to 2.

[7] The pyrazole derivative or a salt thereof according to [1], wherein $R^1$, $R^2$ and $R^4$ are hydrogen atoms,
$R^a$ is a hydrogen atom or $C_1$-$C_6$ alkyl,
$R^b$ is -$S(O)_rR^6$,
$R^6$ is $C_1$-$C_6$ alkyl, $C_2$-$C_6$ alkenyl or ($C_1$-$C_6$) alkyl optionally substituted with $R^{14}$,
$R^{14}$ is -$S(O)_rR^{15}$,
$R^{15}$ is $C_1$-$C_6$ alkyl, and
r is an integer of from 0 to 2.

[8] The pyrazole derivative or a salt thereof according to [5], wherein $R^7$ is $C_1$-$C_{15}$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$)

alkenyl optionally substituted with $R^{14}$, $C_3$-$C_8$ cycloalkenyl, $C_2$-$C_6$ alkynyl, ($C_2$-$C_6$) alkynyl optionally substituted with $R^{14}$, -C(O)$OR^{11a}$, -C(O)$R^{12}$, -C(O)N($R^{13a}$)$R^{13}$, -C(=$NOR^{11}$)$R^{12}$, phenyl substituted with (Z)q, D1-1, D1-2, D1-4, D1-5, D1-6, D1-8, D1-9, D1-10, D1-12, D1-13, D1-19, D1-32, D1-33, D1-35, D1-38, D1-45, D1-81, D1-82, D1-87, D1-88, D1-92 or D1-94, $R^{8a}$ is a hydrogen atom, $C_1$-$C_6$ alkyl or ($C_1$-$C_6$) alkyl optionally substituted with $R^{14a}$, or $R^8$ may form, together with $R^{8a}$, a $C_5$ alkylene chain to form a 6-membered ring together with the nitrogen atom attached to $R^8$ and $R^{8a}$, wherein the alkylene chain may optionally be substituted with $C_1$-$C_6$ alkyl, or $R^8$ and $R^{8a}$ may form =C($R^{8f}$)$R^{8e}$ together with each other.

[9] The pyrazole derivative or a salt thereof according to [5], wherein $R^7$ is $C_1$-$C_6$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$) alkenyl optionally substituted with $R^{14}$, $C_3$-$C_8$ cycloalkenyl, $C_2$-$C_6$ alkynyl, ($C_2$-$C_6$) alkynyl optionally substituted with $R^{14}$, -C(O)$R^{12}$, -C(=$NOR^{11}$)$R^{12}$, D1-1, D1-2, D1-5, D1-6, D1-8, D1-10, D1-12, D1-13, D1-19, D1-32, D1-33, D1-35, D1-38, D1-45, D1-81, D1-82, D1-87 or D1-88, and

$R^{8a}$ is a hydrogen atom or $C_1$-$C_6$ alkyl.

[10] The pyrazole derivative or a salt thereof according to [5], wherein $R^7$ is $C_1$-$C_6$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$) alkenyl optionally substituted with

$R^{14}$, $C_3$-$C_8$ cycloalkenyl, -C(O)$R^{12}$, -C(=$NOR^{11}$)$R^{12}$, D1-8 or D1-82, and

$R^{8a}$ is a hydrogen atom or $C_1$-$C_6$ alkyl.

[11] The pyrazole derivative or a salt thereof according to [5], wherein $R^7$ is $C_1$-$C_6$ alkyl, ($C_1$-$C_6$) alkyl optionally substituted with $R^{14}$, $C_2$-$C_6$ alkenyl, ($C_2$-$C_6$) alkenyl optionally substituted with $R^1$, -C(O)$R^{12}$, -C(=$NOR^{11}$)$R^{12}$, D1-8 or D1-82, and $R^{8a}$ is a hydrogen atom or $C_1$-$C_6$ alkyl.

[12] The pyrazole derivative or a salt thereof according to [5], wherein $R^7$ is $C_1$-$C_6$ alkyl, $C_1$-$C_6$ alkyl optionally substituted with $R^{14}$, $C_1$-$C_6$ alkenyl optionally substituted with $R^{14}$, -C(O)$R^{12}$, D1-8 or D1-82, and

$R^{8a}$ is a hydrogen atom or $C_1$-$C_6$ alkyl.

[13] The pyrazole derivative or a salt thereof according to [5], wherein $R^7$ may form, together with $R^a$, a $C_2$-$C_4$ alkylene chain or a $C_2$-$C_4$ alkenylene chain containing a double bond to form a 4 to 6-membered ring together with the carbon atom attached to $R^7$ and the nitrogen atom attached to $R^a$, wherein the alkylene chain may contain one or two oxygen atoms or nitrogen atoms and may optionally be substituted with a halogen atom, $C_1$-$C_6$ alkyl, ($C_1$-$C_6$) alkyl optionally substituted with $R^{14}$, -$OR^{11}$, -S(O)$_r R^{11}$, an oxo group or a methylidene group,

$R^8$ is a hydrogen atom, $C_1$-$C_6$ alkyl, ($C_1$-$C_6$) alkyl optionally substituted with $R^{14}$, $C_3$-$C_8$ cycloalkyl, $C_2$-$C_6$ alkenyl, $C_2$-$C_6$ alkynyl, -C(O)$R^{12}$, -C(O)$OR^{11a}$, -C(=$NOR^{11}$)$R^{12}$, -S(O)$_2R^{11a}$, phenyl, phenyl substituted with (Z)q, D1-32, D1-33, D1-34 or D1-80,

$R^{8a}$ may form, together with $R^a$, a $C_2$-$C_3$ alkylene chain to form a 5 to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group,

$R^{14}$ is a halogen atom or -S(O)$_r R^{15}$, and

$R^{15}$ is $C_1$-$C_6$ alkyl.

[14] The pyrazole derivative or a salt thereof according to [5], wherein $R^7$ may form, together with $R^a$, a $C_2$-$C_4$ alkylene chain or a $C_2$-$C_4$ alkenylene chain containing a double bond to form a 4 to 6-membered ring together with the carbon atom attached to $R^7$ and the nitrogen atom attached to $R^a$, wherein the alkylene chain may contain one or two oxygen atoms or nitrogen atoms and may optionally be substituted with a halogen atom, $C_1$-$C_6$ alkyl, ($C_1$-$C_6$) alkyl optionally substituted with $R^{14}$, -$OR^{11}$, -S(O)$_r R^{11}$, an oxo group or a methylidene group,

$R^8$ is a hydrogen atom or $C_1$-$C_6$ alkyl optionally substituted with $R^{14}$,

$R^{8a}$ may form, together with $R^a$, a $C_2$ alkylene chain to form a 5 to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group, $R^{14}$ is a halogen atom or -S(O)$_r R^{15}$, and

$R^{15}$ is $C_1$-$C_6$ alkyl.

[15] The pyrazole derivative or a salt thereof according to [5], wherein $R^7$ may form, together with $R^a$, a $C_3$-$C_4$ alkylene chain or a $C_3$-$C_4$ alkenylene chain containing a double bond to form a 5 to 6-membered ring together with the carbon atom attached to $R^7$ and the nitrogen atom attached to $R^a$, wherein the alkylene chain may contain an oxygen atom and may optionally be substituted with a halogen atom, $C_1$-$C_6$ alkyl, ($C_1$-$C_6$) alkyl optionally substituted with $R^{14}$, -S(O)$_r R^{11}$, oxo group or a methylidene group, $R^8$ is a hydrogen atom or $C_1$-$C_6$ alkyl optionally substituted with $R^{14}$,

$R^{8a}$ may form, together with $R^a$, a $C_2$ alkylene chain to form a 5- to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group, $R^{14}$ is a halogen atom or -S(O)$_r R^{15}$, and

$R^{15}$ is $C_1$-$C_6$ alkyl.

[16] The pyrazole derivative or a salt thereof according to [5], wherein $R^7$ may form, together with $R^a$, a $C_3$-$C_4$ alkylene chain or a $C_3$-$C_4$ alkenylene containing a double bond to form a 5- to 6-membered ring together with the carbon atom attached to $R^7$ and the nitrogen atom attached to $R^a$, wherein the alkylene chain may contain an oxygen atom and may optionally be substituted with a halogen atom, $C_1$-$C_6$ alkyl, ($C_1$-$C_6$) alkyl optionally substituted with $R^{14}$, -S(O)$_r$$R^{11}$, oxo group or a methyliedene group, $R^8$ is $C_1$-$C_6$ alkyl optionally substituted with $R^{14}$,

$R^{8a}$ may form, together with $R^a$, a $C_2$ alkylene chain to form a 6-membered ring together with the nitrogen atom attached to $R^{8a}$ and the nitrogen atom attached to $R^a$, wherein the alkylene chain may contain an oxygen atom, and $R^{14}$ is a halogen atom.

[17] The pyrazole derivative or a salt thereof according to [4] wherein $R^4$ is a hydrogen atom,

$R^3$ is a halogen atom,

$R^a$ is $C_1$-$C_6$ alkyl,

$R^b$ is -C(O)$R^7$,

$R^7$ is ($C_1$-$C_6$) alkyl optionally substituted with $R^{14}$,

$R^{14}$ is -S(O)$_r$$R^{15}$,

$R^{15}$ is $C_1$-$C_6$ alkyl, and

m2, n and r are integers of 0.

[18] A pesticide containing one or more pyrazole derivatives or salts thereof selected from the pyrazole derivatives and salts thereof as defined in [1] to [17], as active ingredient(s).

[19] An agrochemical containing one or more pyrazole derivatives or salts thereof selected from the pyrazole derivatives and salts thereof as defined in [1] to [17], as active ingredient(s).

[20] A parasticide for an internal or external parasite in or on a mammal or bird, which contains one or more pyrazole derivatives or salts thereof selected from the pyrazole derivatives and salts thereof as defined in [1] to [17], as active ingredient(s).

[21] An insecticide or miticide containing one or more pyrazole derivatives or salts thereof selected from the pyrazole derivatives and salts thereof as defined in [1] to [17], as active ingredient(s).

[22] A seed treatment agent containing one or more pyrazole derivatives or salts thereof selected from the pyrazole derivatives and salts thereof as defined in [1] to [17], as active ingredient(s).

[23] The seed treatment agent according to [22], which is used to treat seeds by dipping.

[24] A soil treatment agent containing one or more pyrazole derivatives or salts thereof selected from the pyrazole derivatives and salts thereof as defined in [1] to [17], as active ingredient(s).

[25] The soil treatment agent according to [24], which is used to treat soil by irrigation.

## ADVANTAGEOUS EFFECT(S) OF INVENTION

The compounds of the present invention have excellent insecticidal and miticidal activities on many agricultural pest insects, spider mites, internal or external mammal or bird parasites and have sufficient controlling effect on pest insects which have acquired resistance to conventional insecticides. The compounds of the present invention have little harmful effect on mammals, fish and beneficial insects, show low persistence and are environmentally friendly.

Thus, the present invention can provide useful novel pesticides.

## DESCRIPTION OF EMBODIMENT(S)

The compounds of the present invention can have geometrical isomers such as E-isomers and Z-isomers, depending on the types of substituents in them, and the present invention covers both E-isomers and Z-isomers and mixtures containing them in any ratios. The compounds of the present invention can have optically active isomers due to the presence of one or more asymmetric carbon atoms, and the present invention covers any optically active isomers and any racemates.

Some of the compounds of the present invention can be converted, by ordinary methods, to acid addition salts with hydrogen halides such as hydrofluoric acid, hydrochloric acid, hydrobromic acid and hydroiodic acid, with inorganic acids such as nitric acid, sulfuric acid, phosphoric acid, chloric acid and perchloric acid, with sulfonic acids such as methanesulfonic acid, ethanesulfonic acid, trifluoromethanesuflonic acid, benzenesulfonic acid and p-toluenesulfonic acid, with carboxylic acids such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid and citric acid, with amino acids such as glutamic acid aspartic acid.

Some of the compounds of the present invention can be converted, by ordinary methods, to metal salts with alkali metals such as lithium sodium and potassium, with alkaline earth metals such as calcium, barium and magnesium, with metals such as aluminum.

Next, specific examples of each substituent used herein will be given below. n-denotes normal, i- iso, s- secondary, tert- tertiary, and Ph phenyl.

As a halogen atom in the compounds of the present invention, a fluorine atom, a chlorine atom, a bromine atom or an iodine atom may be mentioned. Herein, the expression "halo" also means such a halogen atom.

The expression $C_a$-$C_b$ alkyl herein means a linear or branched hydrocarbon group containing from a to b carbon atoms such as a methyl group, an ethyl group, a n-propyl group, an i-propyl group, a n-butyl group, an i-butyl group, a s-butyl group, a t-butyl group, a n-pentyl group, a 1,1-dimethylpropyl group or a n-hexyl group, and those within the designated carbon number range are selected.

The expression $C_a$-$C_b$ haloalkyl herein means a linear or branched hydrocarbon group containing from a to b carbon atoms in which hydrogen atom(s) on carbon atom(s) are optionally substituted with halogen atom(s) which may be identical with or different from one another if two or more halogen atoms are present, such as a fluoromethyl group, a chloromethyl group, a bromomethyl group, an iodomethyl group, a difluoromethyl group, a dichloromethyl group, a trifluoromethyl group, a chlorodifluoromethyl group, a trichloromethyl group, a bromodifluoromethyl group, a 2-fluoroethyl group, a 2-chloroethyl group, a 2-bromoethyl group, a 2,2-difluoroethyl group, a 2,2,2-trifluoroethyl group, a 2-chloro-2,2-difluoroethyl group, a 2,2,2-trichloroethyl group, a 1,1,2,2-tetrafluoroethyl group, a 2-chloro-1,1,2-trifluoroethyl group, a pentafluoroethyl group, a 3,3,3-trifluoropropyl group, a 2,2,3,3,3-pentafluoropropyl group, a 1,1,2,3,3,3-hexafluoropropyl group, a heptafluoropropyl group, a 2,2,2-trifluoro-1-(trifluoromethyl)ethyl group, a 1,2,2,2-tetrafluoro-1-(trifluoromethyl)ethyl group,a 2,2,3,3,4,4,4-heptafluorobutyl group or a nonafluorobutyl group, and those within the designated carbon number range are selected.

The expression $C_a$-$C_b$ cycloalkyl herein means a cyclic hydrocarbon group containing from a to b carbon atoms in the form of a 3- to 6-membered monocyclic or polycyclic ring which may optionally be substituted with an alkyl group as long as the number of carbon atoms does not exceed the designated carbon number range, such as a cyclopropyl group, a 1-methylcyclopropyl group, a 2-methylcyclopropyl group, a 2,2-dimethylcyclopropyl group, a cyclobutyl group, a cyclopentyl group or a cyclohexyl group, and those within the designated carbon number range are selected.

The expression $C_a$-$C_b$ halocycloalkyl means a cyclic hydrocarbon group containing from a to b carbon atoms in the form of a 3- to 6-membered monocyclic or polycyclic ring which may optionally be substituted with an alkyl group as long as the number of carbon atoms does not exceed the designated carbon number range, in which hydrogen atom(s) on carbon atom(s) in a ring moiety and/or in a side chain are optionally substituted with halogen atom(s) which may be identical with or different from one another if two or more halogen atoms are present, such as a 2,2-difluorocyclopropyl group, a 2,2-dichlorocyclopropyl group, a 2,2-dibromocyclopropyl group, a 2,2-difluoro1-methylcyclopropyl group, a 2,2-dichloro-1-methylcyclopropyl group, a 2,2-dibromo-1-methylcyclopropyl group or a 2,2,3,3-tetrafluorocyclobutyl group, and those within the designated carbon atom range are selected.

The expression $C_a$-$C_b$ alkenyl herein means a linear or branched unsaturated hydrocarbon group containing from a to b carbon atoms and having one or more double bonds in the molecule such as a vinyl group, a 1-propenyl group, a 2-propenyl group, a 1-methylethenyl group, a 2-butenyl group, a 2-methyl-2-propenyl group, a 3-methyl-2-butenyl group or a 1,1-dimethyl-2-propenyl group, and those within the designated carbon atom range are selected.

The expression $C_a$-$C_b$ haloalkenyl herein means a linear or branched unsaturated hydrocarbon group containing from a to b carbon atoms and having one or more double bonds in the molecule, in which hydrogen atom(s) on carbon atom(s) are optionally substituted with halogen atom(s) which may be identical with or different from one another if two or more halogen atoms are present, such as a 2,2-dichlorovinyl group, a 2-fluoro2-propenyl group, a 2-chloro-2-propenyl group, a 3-chloro-2-propenyl group, a 2-bromo-2-propenyl group, a 3,3-difluoro-2-propenyl group, a 2,3-dichloro-2-propenyl group, a 3,3-dichloro-2-propenyl group, a 2,3,3-trifluoro-2-propenyl group, a 2,3,3-trichloro-2-propenyl group, a 1-(trifluoromethyl)ethenyl group, a 4,4-difluoro-3-butenyl group, a 3,4,4-trifluoro-3-butenyl group or a 3-chloro-4,4,4-trifluoro-2-butenyl group, and those within the designated carbon atom range are selected.

The expression $C_a$-$C_b$ cycloalkenyl herein means a cyclic unsaturated hydrocarbon group containing from a to b carbon atoms and containing one or more end or exo double bonds in the form of a 3- to 6-membered monocyclic or polycyclic ring which may optionally be substituted with an alkyl group as long as the number of carbon atoms does not exceed the designated carbon number range, such as a 1-cyclopenten-1-yl group, a 2-cyclopenten-1-yl group, a 1-cyclohexen-1-yl group or a 2-cyclohexen-1-yl group, and those within the designated carbon atom range are selected.

The expression $C_a$-$C_b$ alkylidene herein means a linear or branched hydrocarbon group containing from a to b carbon atoms which attaches by a double bond , such as a methylidene group, an ethylidene group, a propylidene group or a 1-methylethylidene group, and those within the designated carbon number range are selected.

The expression $C_a$-$C_b$ alkynyl herein means a linear or branched unsaturated hydrocarbon group containing from a to b carbon atoms and having one or more triple bonds in the molecule such as an ethynyl group, a 1-propynyl group, a 2-propynyl group, a 1-butynyl group, a 2-butylnyl group, a 3-butynyl group or a 1,1-dimethyl-2-propynyl group, and those within the designated carbon atom range are selected.

The expression $C_a$-$C_b$ haloalkynyl herein means a linear or branched unsaturated hydrocarbon group containing from a to b carbon atoms and having one or more triple bonds in the molecule, in which hydrogen atom(s) on carbon

atom(s) are optionally substituted with halogen atom(s) which may be identical with or different from one another if two or more halogen atoms are present, such as a 2-chloroethynyl group, a 2-bromoethynyl group, a 2-iodoethynyl group, a 3-chloro-2-propynyl group or a 3-bromo-2-propynyl group, a 3-iodo-2-propynyl group, and those within the designated carbon number range are selected.

The expression $C_a$-$C_b$ alkoxy herein means an alkyl-O- group in which the alkyl is a previously mentioned alkyl group containing from a to b carbon atoms, such as a methoxy group, an ethoxy group, a n-propyloxy group, an i-propyloxy group, a n-butyloxy group, an i-butyloxy group, a s-butyloxy group or a tert-butyloxy group, and those within the designated carbon atom range are selected.

The expression $C_a$-$C_b$ haloalkoxy herein means a haloalkyl-O- group in which the haloalkyl is a previously mentioned haloalkyl group containing from a to b carbon atoms, such as a difluoromethoxy group, a trifluoromethoxy group, a chlorodifluoromethoxy group, a bromodifluoromethoxy group, a 2-fluoroethoxy group, a 2-chloroethoxy group, a 2,2,2-trifluoroethoxy group, a 1,1,2,2,-tetrafluoroethoxy group, a 2-chloro-1, 1,2-trifluoroethoxy group or a 1,1,2,3,3, 3-hexafluoropropyloxy group, and those within the designated carbon atom range are selected.

The expression $C_a$-$C_b$ alkylthio herein means an alkyl-S- group in which the alkyl is a previously mentioned alkyl group containing from a to b carbon atoms, such as a methylthio group, an ethylthio group, a n-propylthio group, an i-propylthio group, a n-butylthio group, an i-butylthio group, a s-butylthio group or a tert-butylthio group, and those within the designated carbon atom range are selected.

The expression $C_a$-$C_b$ haloalkylthio herein means a haloalkyl-S- group in which the haloalkyl is a previously mentioned haloalkyl group containing from a to b carbon atoms, such as a difluoromethylthio group, a trifluoromethylthio group, a chlorodifluoromethylthio group, a bromodifluoromethylthio group, a 2,2,2-trifluoroethylthio group, a 1,1,2, 2-tetrafluoroethylthio group, a 2-chloro-1,1,2-trifluoroethylthio group, a pentafluoroethylthio group, a 1,1,2,3,3,3-hexafluoropropylthio group, a heptafluoropropylthio group, a 1,2,2,2-tetrafluoro-1-(trifluoromethyl)ethylthio group or a nonafluorobutylthio group, and those within the designated carbon atom range are selected.

The expression $C_a$-$C_b$ alkylsulfinyl herein means an alkyl-S(O)- group in which the alkyl is a previously mentioned alkyl group containing from a to b carbon atoms, such as a methylsulfinyl group, an ethylsulfinyl group, a n-propylsulfinyl group, an i-propylsulfinyl group, a n-butylsulfinyl group, an i-butylsulfinyl group, a s-butylsulfinyl group or a tert-butylsulfinyl group , and those within the designated carbon atom range are selected.

The expression $C_a$-$C_b$ haloalkylsulfinyl herein means a haloalkyl-S(O)- group in which the haloalkyl is a previously mentioned haloalkyl group containing from a to b carbon atoms, such as a difluoromethylsulfinyl group, a trifluoromethylsulfinyl group, a chlorodifluoromethylsulfinyl group, a bromodifluoromethylsulfinyl group, a 2,2,2-trifluoroethylsulfinyl group, a 1,2,2,2-tetrafluoro-1-(trifluoromethyl)ethylsulfinyl group or a nonafluorobutylsulfinyl group , and those within the designated carbon atom range are selected.

The expression $C_a$-$C_b$ alkylsulfonyl herein means an alkyl-$SO_2$- group in which the alkyl is a previously mentioned alkyl group containing from a to b carbon atoms, such as a methylsulfonyl group, an ethylsulfonyl group, a n-propylsulfonyl group, an i-propylsulfonyl group, a n-butylsulfonyl group, an i-butylsulfonyl group, a s-butylsulfonyl group or a tert-butylsulfonyl group, and those within the designated carbon atom range are selected.

The expression $C_a$-$C_b$ haloalkylsulfonyl herein means a haloalkyl-$SO_2$- group in which the haloalkyl is a previously mentioned haloalkyl group containing from a to b carbon atoms, such as a difluoromethylsulfonyl group, a trifluoromethylsulfonyl group, a chlorodifluoromethylsulfonyl group, a bromodifluoromethylsulfonyl group, a 2,2,2-trifluoroethylsulfonyl group, a 1,1,2,2-tetrafluoroethylsulfonyl group or a 2-chloro-1,1,2-trifluoroethylsulfonyl group, and those within the designated carbon atom range are selected.

The expression $C_a$-$C_b$ alkylamino herein means an amino group in which either hydrogen atom is replaced by a previously mentioned alkyl group containing from a to b carbon atoms, such as a methylamino group, an ethylamino group, a n-propylamino group, an i-propylamino group, a n-butylamino group, an i-butylamino group or a tert-butylamino group, and those within the designated carbon atom range are selected.

The expression di($C_a$-$C_b$ alkyl)amino herein means an amino group in which both hydrogen atoms are replaced by previously mentioned alkyl groups containing from a to b carbon atoms which may be identical with or different from each other, such as a dimethylamino group, an ethyl(methyl)amino group, a diethylamino group, a n-propyl(methyl)amino group, an i-propyl(methyl)amino group, a di(n-propyl)amino group or a di(n-butyl)amino group, and those within the designated carbon atom range are selected.

The expression $C_a$-$C_b$ alkylimino herein means an alkyl-N= group in which the alkyl means a previously mentioned alkyl group containing from a to b carbon atoms, such as a methylimino group, an ethylimino group, a n-propylimino group, an i-propylimino group, a n-butylimino group, an i-butylimino group or a s-butylimino group, and those within the designated carbon atom range are selected.

The expression $C_a$-$C_b$ alkoxyimino herein means an alkoxy-N= group in which the alkoxy means a previously mentioned alkoxy group containing from a to b carbon atoms, such as a methoxyimino group, an ethoxyimino group, a n-propyloxyimino group, an i-propyloxyimino group or a n-butyloxyimino group, and those within the designated carbon atom range are selected.

The expression $C_a$-$C_b$ alkylcarbonyl herein means an alkyl-C(O)- group in which the alkyl means a previously mentioned alkyl group containing from a to b carbon atoms, such as an acetyl group, a propionyl group, a butyryl group, an isobutyryl group, a valeryl group, an isovaleryl group, a 2-methylbutanoyl group, a pivaloyl group, a hexanoyl group or a heptanoyl group, and those within the designated carbon atom range are selected.

The expression $C_a$-$C_b$ haloalkylcarbonyl herein means a haloalkyl-C(O)- group in which the haloalkyl means a previously mentioned haloalkyl group containing from a to b carbon atoms, such as a fluoroacetyl group, a chloroacetyl group, a difluoroacetyl group, a dichloroacetyl group, a trifluoroacetyl group, a chlorodifluoroacetyl group, a bromodifluoroacetyl group, a trichloroacetyl group, a pentafluoropropionyl group, a heptafluorobutanoyl group or a 3-chloro-2, 2-dimethylpropanoyl group, and those within the designated carbon atom range are selected.

The expression $C_a$-$C_b$ cycloalkylcarbonyl herein means a cycloalkyl-C(O)- group in which the cycloalkyl means a previously mentioned cycloalkyl group containing from a to b carbon atoms, such as a cyclopropylcarbonyl group, a 2-methylcyclopropylcarbonyl group or a cyclobutylcarbonyl group, and those within the designated carbon atom range are selected.

The expression $C_a$-$C_b$ alkoxycarbonyl herein means an alkyl-O-C(O)- group in which the alkyl means a previously mentioned alkyl group containing from a to b carbon atoms, such as a methoxycarbonyl group, an ethoxycarbonyl group, a n-propyloxycarbonyl group, an i-propyloxycarbonyl group, a n-butoxycarbonyl group, an i-butoxycarbonyl group or a tert-butoxycarbonyl group, and those within the designated carbon atom range are selected.

The expression $C_a$-$C_b$ haloalkoxycarbonyl herein means a haloalkyl-O-C(O)-group in which the haloalkyl means a previously mentioned haloalkyl group containing from a to b carbon atoms, such as a chloromethoxycarbonyl group, a 2-chloroethoxycarbonyl group, a 2,2-difluoroethoxycarbonyl group, a 2,2,2-trifluoroethoxycarbonyl group or a 2,2, 2-trichloroethoxycarbonyl group, and those within the designated carbon atom range are selected.

The expression $C_a$-$C_b$ alkylaminocarbonyl herein means a carbamoyl group in which either hydrogen atom is replaced by a previously mentioned alkyl group containing from a to b carbon atoms, such as a methylcarbamoyl group, an ethylcarbamoyl group, a n-propylcarbamoyl group, an i-propylcarbamoyl group, a n-butylcarbamoyl group, an i-butylcarbamoyl group, a s-butylcarbamoyl group or a tert-butylcarbamoyl group, and those within the designated carbon atom range are selected.

The expression $C_a$-$C_b$ haloalkylaminocarbonyl herein means a carbamoyl group in which either hydrogen atom is replaced by a previously mentioned haloalkyl group containing from a to b carbon atoms, such as a 2-fluoroethylcarbamoyl group, a 2-chloroethylcarbamoyl group, a 2,2-difluoroethylcarbamoyl group or a 2,2,2-trifluoroethylcarbamoyl group, and those within the designated carbon atom range are selected.

The expression di($C_a$-$C_b$ alkyl)aminocarbonyl herein means a carbamoyl group in which both hydrogen atoms are replaced by previously mentioned alkyl groups containing from a to b carbon atoms which may be identical with or different from each other, such as an N,N-dimethylcarbamoyl group, an N-ethyl-N-methylcarbamoyl group, an N,N-diethylcarbamoyl group, an N,N-di(n-propyl)carbamoyl group or an N,N-di(n-butyl)carbamoyl group, and those within the designated carbon atom range are selected.

The expression $C_a$-$C_b$ alkylaminosulfonyl herein means a sulfamoyl group in which either hydrogen atom is replaced by a previously mentioned alkyl group containing from a to b carbon atoms, such as a methylsulfamoyl group, an ethylsulfamoyl group, a n-propylsulfamoyl group, an i-propylsulfamoyl group, a n-butylsulfamoyl group, an i-butylsulfamoyl group, a s-butylsulfamoyl group or a tert-butylsulfamoyl group, and those within the designated carbon atom range are selected.

The expression di($C_a$-$C_b$ alkyl)aminosulfonyl herein means a sulfamoyl group in which both hydrogen atoms are replaced by previously mentioned alkyl groups containing from a to b carbon atoms which may be identical with or different from each other, such as an N,N-dimethylsulfamoyl group, an N-ethyl-N-methylsulfamoyl group, an N,N-diethylsulfamoyl group, an N,N-di(n-propyl)sulfamoyl group or an N,N-di(n-butyl)sulfamoyl group, and those within the designated carbon atom range are selected.

The expression such as ($C_a$-$C_b$) alkyl optionally substituted with $R^5$, ($C_a$-$C_b$) alkyl optionally substituted with $R^{14}$, ($C_a$-$C_b$) alkyl optionally substituted with $R^{14a}$, ($C_a$-$C_b$) alkyl optionally substituted with $R^{19}$, ($C_a$-$C_b$) alkyl optionally substituted with $R^{28}$, ($C_a$-$C_b$) alkyl optionally substituted with $R^{28a}$, ($C_a$-$C_b$) alkyl optionally substituted with $R^{32}$ or ($C_a$-$C_b$) alkyl optionally substituted with $R^{32a}$ herein means a previously mentioned alkyl group having from a to b carbon atoms in which hydrogen atom(s) on carbon atom(s) are optionally substituted with optional $R^5$, $R^{14}$, $R^{14a}$, $R^{19}$, $R^{28}$, $R^{28a}$, $R^{32}$ or $R^{32a}$, and those within the designated carbon atom range are selected. When there are two or more $R^5$'s, $R^{14}$'s, $R^{14a}$'s, $R^{19}$'s, $R^{28}$'s, $R^{28a}$'s, $R^{32}$'s or $R^{32a}$'s on a ($C_a$-$C_b$) alkyl group, each $R^5$, $R^{14}$, $R^{14a}$, $R^{19}$, $R^{28}$, $R^{28a}$, $R^{32}$ or $R^{32a}$ may be identical with or different from one another.

The expression such as ($C_a$-$C_b$) cycloalkyl optionally substituted with $R^5$, ($C_a$-$C_b$) cycloalkyl optionally substituted with $R^{14}$, ($C_a$-$C_b$) cycloalkyl optionally substituted with $R^{14a}$, ($C_a$-$C_b$) cycloalkyl optionally substituted with $R^{19}$, ($C_a$-$C_b$) cycloalkyl optionally substituted with $R^{28}$, ($C_a$-$C_b$) cycloalkyl optionally substituted with $R^{28a}$ or ($C_a$-$C_b$) cycloalkyl optionally substituted with $R^{32}$ herein means a previously mentioned cycloalkyl group having from a to b carbon atoms in which hydrogen atom(s) on carbon atom(s) in the ring moiety and/or in the side chain are optionally substituted with ($C_a$-$C_b$)

cycloalkyl optionally substituted with optional $R^5$, $R^{14}$, $R^{14a}$, $R^{19}$, $R^{28}$, $R^{28a}$ or $R^{32}$, and those within the designated carbon atom range are selected. When there are two or more $R^5$'s, $R^{14}$'s, $R^{14a}$'s, $R^{19}$'s, $R^{28}$'s, $R^{28a}$'s or $R^{32}$'s on a ($C_a$-$C_b$) cycloalkyl group, each $R^5$, $R^{14}$, $R^{14a}$, $R^{19}$, $R^{28}$, $R^{28a}$ or $R^{32}$ may be identical with or different from one another.

The expression such as ($C_a$-$C_b$) alkenyl optionally substituted with $R^5$, ($C_a$-$C_b$) alkenyl optionally substituted with $R^{14}$, ($C_a$-$C_b$) alkenyl optionally substituted with $R^{14a}$, ($C_a$-$C_b$) alkenyl optionally substituted with $R^{19}$, ($C_a$-$C_b$) alkenyl optionally substituted with $R^{28}$, ($C_a$-$C_b$) alkenyl optionally substituted with $R^{28a}$ or ($C_a$-$C_b$) alkenyl optionally substituted with $R^{32}$ herein means a previously mentioned alkenyl group having from a to b carbon atoms in which hydrogen atom(s) on carbon atom(s) are optionally substituted with optional $R^5$, $R^{14}$, $R^{14a}$, $R^{19}$, $R^{28}$, $R^{28a}$ or $R^{32}$, and those within the designated carbon atom range are selected. When there are two or more $R^5$'s, $R^{14}$'s, $R^{14a}$'s, $R^{19}$'s, $R^{28}$'s, $R^{28a}$'s or $R^{32}$'s on a ($C_a$-$C_b$) alkenyl group, each $R^5$, $R^{14}$, $R^{14a}$, $R^{19}$, $R^{28}$, $R^{28a}$ or $R^{32}$ may be identical with or different from one another.

The expression such as ($C_a$-$C_b$) cycloalkenyl optionally substituted with $R^5$, ($C_a$-$C_b$) cycloalkenyl optionally substituted with $R^{14}$, ($C_a$-$C_b$) cycloalkenyl optionally substituted with $R^{14a}$, ($C_a$-$C_b$) cycloalkenyl optionally substituted with $R^{19}$, ($C_a$-$C_b$) cycloalkenyl optionally substituted with $R^{28}$, ($C_a$-$C_b$) cycloalkenyl optionally substituted with $R^{28a}$ or ($C_a$-$C_b$) cycloalkenyl optionally substituted with $R^{32}$ herein mean a previously mentioned cycloalkenyl group having from a to b carbon atoms in which hydrogen atom(s) on carbon atom(s) in the ring moiety and/or in the side chain are optionally substituted with optional $R^5$, $R^{14}$, $R^{14a}$, $R^{19}$, $R^{28}$, $R^{28a}$ or $R^{32}$, and those within the designated carbon atom range are selected. When there are two or more $R^5$'s, $R^{14}$'s, $R^{14a}$'s, $R^{19}$'s, $R^{28}$'s, $R^{28a}$'s or $R^{32}$'s on a ($C_a$-$C_b$) cycloalkenyl group, each $R^5$, $R^{14}$, $R^{14a}$, $R^{19}$, $R^{28}$, $R^{28a}$ or $R^{32}$ may be identical with or different from one another.

The expression such as ($C_a$-$C_b$) alkynyl optionally substituted with $R^5$, ($C_a$-$C_b$) alkynyl optionally substituted with $R^{14}$, ($C_a$-$C_b$) alkynyl optionally substituted with $R^{14a}$, ($C_a$-$C_b$) alkynyl optionally substituted with $R^{19}$, ($C_a$-$C_b$) alkynyl optionally substituted with $R^{28}$, ($C_a$-$C_b$) alkynyl optionally substituted with $R^{28a}$ or ($C_a$-$C_b$) alkynyl optionally substituted with $R^{32}$ herein means a previously mentioned alkynyl group having from a to b carbon atoms in which hydrogen atom(s) on carbon atom(s) are optionally substituted with optional $R^5$, $R^{14}$, $R^{14a}$, $R^{19}$, $R^{28}$, $R^{28a}$ or $R^{32}$, and those within the designated carbon atom range are selected. When there are two or more $R^5$'s, $R^{14}$'s, $R^{14a}$'s, $R^{19}$'s, $R^{28}$'s, $R^{28a}$'s or $R^{32}$'s on a ($C_a$-$C_b$) alkynyl group, each $R^5$, $R^{14}$, $R^{14a}$, $R^{19}$, $R^{28}$, $R^{28a}$ or $R^{32}$ may be identical with or different from one another.

The expression such as "$R^8$ may form, together with $R^{8a}$, a $C_2$-$C_7$ alkylene chain to form a 3 to 8-membered ring together with the nitrogen atom attached to $R^8$ and $R^{8a}$, wherein the alkylene chain may contain an oxygen atom, sulfur atom or nitrogen atom", "$R^{13}$ and $R^{13a}$ may form, together with each other, a $C_2$-$C_7$ alkylene chain to form a 3 to 8-membered ring together with the nitrogen atom attached to $R^{13}$ and $R^{13a}$, wherein the alkylene chain may contain an oxygen atom, sulfur atom or nitrogen atom" or "$R^{17}$ and $R^{17a}$ may form, together with each other, a $C_2$-$C_7$ alkylene chain to form a 3 to 8-membered ring together with the nitrogen atom attached to $R^{17}$ and $R^{17a}$, wherein the alkylene chain may contain an oxygen atom, sulfur atom or nitrogen atom" is specifically exemplified by aziridine, azetidine, azetidin-2-one, pyrrolidine, pyrrolidin-2-one, oxazolidine, oxazolidin-2-one, oxazolidine-2-thione, thiazolidine, thiazolidin-2-one, thiazolidine-2-thione, imidazolidine, imidazolidin-2-one, imidazolidine-2-thione, piperidine, piperidin-2-one, piperidine-2-thione, 2H-3,4,5,6-tetrahydro-1,3-oxazin-2-one, 2H-3,4,5,6-tetrahydro-1,3-oxazine-2-thione, morpholine, 2H-3,4,5,6-tetrahydro-1,3-thiazin-2-one, 2H-3,4,5,6-tetrahydro-1,3-thiazine-2-thione, thiomorpholine, perhydropyrimidin-2-one, piperazine, homopiperidine, homopiperidin-2-one, heptamethylenimine or the like, and those within the designated carbon atom range are selected.

The expression "$R^{b3}$ may form, together with $R^{b2}$, a $C_4$-$C_6$ alkylene chain or a $C_4$-$C_6$ alkenylene chain to form a 5 to 7-membered ring together with the carbon atom attached to $R^{b3}$ and $R^{b2}$, wherein the alkylene chain or the alkenylene chain may contain from 1 to 3 oxygen atoms, sulfur atoms or nitrogen atoms" is specifically exemplified by thiazolidin-2-ylidene, 2,3-dihydrothiazol-2-ylidene, imidazolidin-2-ylidene, 2,3-dihydroimidazol-2-ylidene, 2,3-dihydro-1,3,4-thiadiazol-2-ylidene, 1,2-dihydropyridin-2-ylidene, 2,3-dihydropyridazin-3-ylidene, 1,2-dihydropyrazin-2-ylidene, 1,2-dihydropyrimidin-2-ylidene, 6H-2,3-dihydro-1,3,4-thiadiazin-2-ylidene or the like, and those within the designated carbon atom range are selected.

The expression "$R^7$ may form, together with $R^a$, a $C_2$-$C_6$ alkylene chain or a $C_2$-$C_6$ alkenylene chain containing a double bond to form a 4 to 8-membered ring together with the carbon atom attached to $R^7$ and the nitrogen atom attached to $R^a$, wherein the alkylene chain or the alkenylene chain may contain one or two oxygen atoms, sulfur atoms or nitrogen atoms" is specifically exemplified by pyrrolidin-2-one, pyrrolidine-2-thione, 1 H-pyrrol-2(5H)-one, pyrrolidine-2,5-dione, pyrrolidin-2-imine, piperidin-2-one, piperidine-2-thione, piperidin-2-imine, 5,6-dihydropyridin-2(1H)-one, 1,3-oxazinan-2-one, 1,3-oxazinane-2-thione, 1,3-oxazinan-4-one, 1,3-thiazinan-4-one, tetrahydropyrimidin-4(1 H)-one, piperidine-2,3-dione, piperidine-2,6-dione or the like, and those within the designated carbon atom range are selected.

The expression "$R^{8a}$ may form, together with $R^a$, a $C_2$-$C_5$ alkylene chain to form a 5 to 8-membered ring together with the nitrogen atom attached to $R^{8a}$ and the nitrogen atom attached to $R^a$, wherein the alkylene chain may contain an oxygen atom, a sulfur atom or a nitrogen atom" is specifically exemplified by imidazolidin-2-one, imidazolidine-2-thione, imidazolidin-2-imine, imidazolidine-2,4-dione, tetrahydropyrimidin-2(1H)-one, tetrahydropyrimidine-2(1H)-thione, tetrahydropyrimidin-2(1H)-imine, 1,2,4-oxazinan-3-one, 1,3,5-oxazinan-4-one, 1,3,5-thiazinan-4-one, 1,3,5-triazinan-2-one, dihydropyrimidine-2,4(1H,3H)-dione or the like, and those within the designated carbon atom range are selected.

As the scope of the substituent represented by $R^3$ in the compounds which fall within the present invention, the following sets may, for example, be mentioned.

$R^3$I: a hydrogen atom, a halogen atom, $C_1$-$C_6$ alkyl.

$R^3$II: a halogen atom, $C_1$-$C_6$ alkyl.

$R^3$III: a halogen atom.

As the scope of the substituent represented by $R^a$ in the compounds which fall within the present invention, the following sets may, for example, be mentioned.

$R^a$I: $C_1$-$C_6$ alkyl, ($C_1$-$C_6$) alkyl optionally substituted with $R^5$ [wherein $R^5$ is -$OR^{11}$, and $R^{11}$ is $C_1$-$C_6$ alkyl], -C(=$NR^{8b}$)$R^{7a}$ [wherein $R^{7a}$ is a hydrogen atom or -$C_1$-$C_6$ alkyl, $R^{8b}$ is -$OR^{11}$, and $R^{11}$ is $C_1$-$C_6$ alkyl], ($C_1$-$C_6$) alkyl optionally substituted with $R^5$ [wherein $R^5$ is -C(=$NOR^{11}$)$R^{12}$, and each of $R^{11}$ and $R^{12}$ is independently $C_1$-$C_6$ alkyl], ($C_1$-$C_6$) alkyl optionally substituted with $R^5$ [wherein $R^5$ is -Si($R^{9a}$)($R^{9b}$)$R^9$, and each of $R^9$, $R^{9a}$ and $R^{9b}$ is independently $C_1$-$C_6$ alkyl].

$R^a$II: ($C_1$-$C_6$) alkyl optionally substituted with $R^5$ [wherein $R^5$ is -$OR^{11}$, and $R^{11}$ is $C_1$-$C_6$ alkyl].

$R^a$III: $C_1$-$C_6$ alkyl.

$R^a$IV: -C(=$NR^{8b}$)$R^{7a}$ [wherein $R^{7a}$ is a hydrogen atom or -$C_1$-$C_6$ alkyl, $R^{8b}$ is -$OR^{11}$, and $R^{11}$ is $C_1$-$C_6$ alkyl].

$R^a$V: ($C_1$-$C_6$) alkyl optionally substituted with $R^5$ [wherein $R^5$ is -C(=$NOR^{11}$)$R^{12}$, and each of $R^{11}$ and $R^{12}$ is independently $C_1$-$C_6$ alkyl].

$R^a$VI: ($C_1$-$C_6$) alkyl optionally substituted with $R^5$ [wherein $R^5$ is -Si($R^{9a}$)($R^{9b}$)$R^9$, and each of $R^9$, $R^{9a}$ and $R^{9b}$ is indepedendently $C_1$-$C_6$ alkyl].

$R^a$VII: ($C_1$-$C_6$) alkyl optionally substituted with $R^5$ [wherein $R^5$ is -$S(O)_rR^{11}$, $R^{11}$ is $C_1$-$C_6$ alkyl, and r is an integer of from 0 to 2].

$R^a$VIII: ($C_1$-$C_6$) alkyl optionally substituted with $R^5$ [wherein $R^5$ is cyano].

$R^a$IX: ($C_1$-$C_6$) alkyl optionally substituted with $R^5$ [wherein $R^5$ is $C_3$-$C_8$ cycloalkyl].

$R^a$X: $C_2$-$C_{12}$ alkynyl.

As the scope of the substituent represented by $R^b$ in the compounds which fall within the present invention, the following sets may, for example, be mentioned.

$R^b$I: -C(O)$R^7$, -C(S)$R^7$, -C(O)$OR^{6a}$ [wherein $R^{6a}$ is $C_1$-$C_6$ alkyl], -C(O)N($R^{8a}$)$R^8$ [wherein $R^8$ is $C_1$-$C_6$ alkyl, ($C_1$-$C_6$) alkyl optionally substituted with $R^{14}$ or $C_3$-$C_8$ cycloalkyl, $R^{8a}$ is a hydrogen atom or $C_1$-$C_6$ alkyl, and $R^{14}$ is a halogen atom], -C(S)N($R^{8a}$)$R^8$ [wherein $R^8$ is $C_1$-$C_6$ alkyl, ($C_1$-$C_6$) alkyl optionally substituted with $R^{14}$ or $C_3$-$C_8$ cycloalkyl, $R^{8a}$ is a hydrogen atom or $C_1$-$C_6$ alkyl, and $R^{14}$ is a halogen atom].

$R^b$II: -C(S)$R^7$.

$R^b$III: -C(O)$R^7$.

$R^b$IV: -C(O)$OR^{6a}$ [wherein $R^{6a}$ is $C_1$-$C_6$ alkyl].

$R^b$V: -C(O)N($R^{8a}$)$R^8$ [wherein $R^8$ is $C_1$-$C_6$ alkyl, ($C_1$-$C_6$) alkyl optionally substituted with $R^{14}$ or $C_3$-$C_8$ cycloalkyl, $R^{8a}$ is a hydrogen atom or $C_1$-$C_6$ alkyl, and $R^{14}$ is a halogen atom].

$R^b$VI: -C(S)N($R^{8a}$)$R^8$ [wherein $R^8$ is $C_1$-$C_6$ alkyl, ($C_1$-$C_6$) alkyl optionally substituted with $R^{14}$ or $C_3$-$C_8$ cycloalkyl, $R^{8a}$ is a hydrogen atom or $C_1$-$C_6$ alkyl, and $R^{14}$ is a halogen atom].

$R^b$VII: $R^b$ forms =C($R^{b2}$)$R^{b3}$ together with $R^a$ [wherein $R^{b2}$ is a hydrogen atom or $C_1$-$C_{15}$ alkyl, $R^{b3}$ is -N($R^{8b}$)$R^8$, $R^8$ is -C(O)$R^{12}$, $R^{8b}$ is $C_1$-$C_{15}$ alkyl or -$OR^{11}$, $R^{11}$ is $C_1$-$C_6$ alkyl, and $R^{12}$ is ($C_1$-$C_6$) alkyl optionally substituted with $R^{14a}$].

As the scope of the substituent represented by $R^7$ in the compounds which fall within the present invention, the following sets may, for example, be mentioned.

$R^7$I: ($C_1$-$C_6$) alkyl optionally substituted with $R^{14}$ [wherein $R^{14}$ is -$S(O)_rR^{15}$, $R^{15}$ is $C_1$-$C_6$ alkyl, r is an integer of from 0 to 2], -C(=$NOR^{11}$)$R^{12}$ [wherein $R^{11}$ is $C_1$-$C_6$ alkyl, $R^{12}$ is ($C_1$-$C_6$) alkyl optionally substituted with $R^{14a}$, $R^{14a}$ is -$S(O)_rR^{15}$, $R^{15}$ is $C_1$-$C_6$ alkyl, and r is an integer of from 0 to 2].

$R^7$II: -C(=$NOR^{11}$)$R^{12}$ [wherein $R^{11}$ is $C_1$-$C_6$ alkyl, $R^{12}$ is ($C_1$-$C_6$) alkyl optionally substituted with $R^{14a}$, $R^{14a}$ is -$S(O)_rR^{15}$, $R^{15}$ is $C_1$-$C_6$ alkyl, and r is an integer of from 0 to 2].

$R^7$III: ($C_1$-$C_6$) alkyl optionally substituted with $R^{14}$ [wherein $R^{14}$ is -$S(O)_rR^{15}$, $R^{15}$ is $C_1$-$C_6$ alkyl, and r is an integer of from 0 to 2].

$R^7$IV: ($C_1$-$C_6$) alkyl optionally substituted with $R^{14}$ [wherein $R^{14}$ is D1-82, D1-87, D1-93 or D1-98, and r is an integer of from 0 to 2].

$R^7$V: ($C_1$-$C_6$) alkyl optionally substituted with $R^{14}$ [wherein $R^{14}$ is D1-82 or D1-93, and r is an integer of from 0 to 2].

$R^7$VI: ($C_1$-$C_6$) alkyl optionally substituted with $R^{14}$ [wherein $R^{14}$ is D1-87 or D1-98].

$R^7$VII: ($C_1$-$C_6$) alkyl optionally substituted with $R^{14}$ [wherein $R^{14}$ is -$S(O)_rR^{15}$, $R^{15}$ is D1-32, and r is an integer of from 0 to 2].

$R^7$VIII: ($C_1$-$C_6$) alkyl optionally substituted with $R^{14}$ [wherein $R^{14}$ is -S(=$NR^{17b}$)$R^{15a}$ or -S(O)(=$NR^{17b}$)$R^{15a}$, $R^{15a}$

is $C_1$-$C_6$ alkyl, and $R^{17b}$ is cyano].

$R^7$IX: ($C_1$-$C_6$) alkyl optionally substituted with $R^{14}$ [wherein $R^{14}$ is -C(O)N($R^{17a}$)$R^{17}$, $R^{17}$ is ($C_1$-$C_6$) alkyl optionally substituted with $R^{19}$ or -S(O)$_r$$R^{20}$, $R^{17a}$ is a hydrogen atom or -$C_1$-$C_6$ alkyl, $R^{19}$ is a halogen atom, $R^{20}$ is $C_1$-$C_6$ alkyl, ($C_1$-$C_6$) alkyl optionally substituted with $R^{32}$, and $R^{32}$ is a halogen atom].

The sets indicating the scope of each substituent in the compounds which fall within the present invention may be combined arbitrarily to indicate the scope of the compounds of the present invention. The scope of $R^3$, $R^a$, $R^b$ or $R^7$ may be combined, for example, as shown in Table 1. The combinations shown in Table 1 merely exemplify the present invention, and the present invention is by no means restricted thereto.

TABLE 1

| $R^3$ | $R^a$ | $R^b$ | $R^7$ |
|---|---|---|---|
| $R^3$I | $R^a$I | $R^b$I | $R^7$I |
| $R^3$I | $R^a$I | $R^b$I | $R^7$II |
| $R^3$I | $R^a$I | $R^b$I | $R^7$III |
| $R^3$I | $R^a$I | $R^b$I | $R^7$IV |
| $R^3$I | $R^a$I | $R^b$I | $R^7$V |
| $R^3$I | $R^a$I | $R^b$I | $R^7$VI |
| $R^3$I | $R^a$I | $R^b$I | $R^7$VII |
| $R^3$I | $R^a$I | $R^b$I | $R^7$VIII |
| $R^3$I | $R^a$I | $R^b$I | $R^7$IX |
| $R^3$I | $R^a$I | $R^b$II | $R^7$I |
| $R^3$I | $R^a$I | $R^b$II | $R^7$II |
| $R^3$I | $R^a$I | $R^b$II | $R^7$III |
| $R^3$I | $R^a$I | $R^b$II | $R^7$IV |
| $R^3$I | $R^a$I | $R^b$II | $R^7$V |
| $R^3$I | $R^a$I | $R^b$II | $R^7$VI |
| $R^3$I | $R^a$I | $R^b$II | $R^7$VII |
| $R^3$I | $R^a$I | $R^b$II | $R^7$VIII |
| $R^3$I | $R^a$I | $R^b$II | $R^7$IX |
| $R^3$I | $R^a$I | $R^b$III | $R^7$I |
| $R^3$I | $R^a$I | $R^b$III | $R^7$II |
| $R^3$I | $R^a$I | $R^b$III | $R^7$III |
| $R^3$I | $R^a$I | $R^b$III | $R^7$IV |
| $R^3$I | $R^a$I | $R^b$III | $R^7$V |
| $R^3$I | $R^a$I | $R^b$III | $R^7$VI |
| $R^3$I | $R^a$I | $R^b$III | $R^7$VII |
| $R^3$I | $R^a$I | $R^b$III | $R^7$VIII |
| $R^3$I | $R^a$I | $R^b$III | $R^7$IX |
| $R^3$I | $R^a$I | $R^b$IV | -- |
| $R^3$I | $R^a$I | $R^b$V | -- |
| $R^3$I | $R^a$I | $R^b$VI | -- |
| $R^3$I | $R^a$II | $R^b$I | $R^7$I |
| $R^3$I | $R^a$II | $R^b$I | $R^7$II |
| $R^3$I | $R^a$II | $R^b$I | $R^7$III |
| $R^3$I | $R^a$II | $R^b$I | $R^7$IV |
| $R^3$I | $R^a$II | $R^b$I | $R^7$V |
| $R^3$I | $R^a$II | $R^b$I | $R^7$VI |
| $R^3$I | $R^a$II | $R^b$I | $R^7$VII |
| $R^3$I | $R^a$II | $R^b$I | $R^7$VIII |
| $R^3$I | $R^a$II | $R^b$I | $R^7$IX |
| $R^3$I | $R^a$II | $R^b$II | $R^7$I |
| $R^3$I | $R^a$II | $R^b$II | $R^7$II |

(continued)

| $R^3$ | $R^a$ | $R^b$ | $R^7$ |
|---|---|---|---|
| $R^3$I | $R^a$II | $R^b$II | $R^7$III |
| $R^3$I | $R^a$II | $R^b$II | $R^7$IV |
| $R^3$I | $R^a$II | $R^b$II | $R^7$V |
| $R^3$I | $R^a$II | $R^b$II | $R^7$VI |
| $R^3$I | $R^a$II | $R^b$II | $R^7$VII |
| $R^3$I | $R^a$II | $R^b$II | $R^7$VIII |
| $R^3$I | $R^a$II | $R^b$II | $R^7$IX |
| $R^3$I | $R^a$II | $R^b$III | $R^7$I |
| $R^3$I | $R^a$II | $R^b$III | $R^7$II |
| $R^3$I | $R^a$II | $R^b$III | $R^7$III |
| $R^3$I | $R^a$II | $R^b$III | $R^7$IV |
| $R^3$I | $R^a$II | $R^b$III | $R^7$V |
| $R^3$I | $R^a$II | $R^b$III | $R^7$VI |
| $R^3$I | $R^a$II | $R^b$III | $R^7$VII |
| $R^3$I | $R^a$II | $R^b$III | $R^7$VIII |
| $R^3$I | $R^a$II | $R^b$III | $R^7$IX |
| $R^3$I | $R^a$II | $R^b$IV | -- |
| $R^3$I | $R^a$II | $R^b$V | -- |
| $R^3$I | $R^a$II | $R^b$VI | -- |
| $R^3$I | $R^a$III | $R^b$I | $R^7$I |
| $R^3$I | $R^a$III | $R^b$I | $R^7$II |
| $R^3$I | $R^a$III | $R^b$I | $R^7$III |
| $R^3$I | $R^a$III | $R^b$I | $R^7$IX |
| $R^3$I | $R^a$III | $R^b$I | $R^7$IV |
| $R^3$I | $R^a$III | $R^b$I | $R^7$V |
| $R^3$I | $R^a$III | $R^b$I | $R^7$VI |
| $R^3$I | $R^a$III | $R^b$I | $R^7$VII |
| $R^3$I | $R^a$III | $R^b$I | $R^7$VIII |
| $R^3$I | $R^a$III | $R^b$II | $R^7$I |
| $R^3$I | $R^a$III | $R^b$II | $R^7$II |
| $R^3$I | $R^a$III | $R^b$II | $R^7$III |
| $R^3$I | $R^a$III | $R^b$II | $R^7$IV |
| $R^3$I | $R^a$III | $R^b$II | $R^7$V |
| $R^3$I | $R^a$III | $R^b$II | $R^7$VI |
| $R^3$I | $R^a$III | $R^b$II | $R^7$VII |
| $R^3$I | $R^a$III | $R^b$II | $R^7$VIII |
| $R^3$I | $R^a$III | $R^b$II | $R^7$IX |
| $R^3$I | $R^a$III | $R^b$III | $R^7$I |
| $R^3$I | $R^a$III | $R^b$III | $R^7$II |
| $R^3$I | $R^a$III | $R^b$III | $R^7$III |
| $R^3$I | $R^a$III | $R^b$III | $R^7$IV |
| $R^3$I | $R^a$III | $R^b$III | $R^7$V |
| $R^3$I | $R^a$III | $R^b$III | $R^7$VI |
| $R^3$I | $R^a$III | $R^b$III | $R^7$VII |
| $R^3$I | $R^a$III | $R^b$III | $R^7$VII |
| $R^3$I | $R^a$III | $R^b$III | $R^7$IX |
| $R^3$I | $R^a$III | $R^b$IV | -- |
| $R^3$I | $R^a$III | $R^b$V | -- |
| $R^3$I | $R^a$III | $R^b$VI | -- |

(continued)

| $R^3$ | $R^a$ | $R^b$ | $R^7$ |
|---|---|---|---|
| $R^3$I | $R^a$IV | $R^b$I | $R^7$I |
| $R^3$I | $R^a$IV | $R^b$I | $R^7$II |
| $R^3$I | $R^a$IV | $R^b$I | $R^7$III |
| $R^3$I | $R^a$IV | $R^b$I | $R^7$IV |
| $R^3$I | $R^a$IV | $R^b$I | $R^7$V |
| $R^3$I | $R^a$IV | $R^b$I | $R^7$VI |
| $R^3$I | $R^a$IV | $R^b$I | $R^7$VII |
| $R^3$I | $R^a$IV | $R^b$I | $R^7$VIII |
| $R^3$I | $R^a$IV | $R^b$I | $R^7$IX |
| $R^3$I | $R^a$IV | $R^b$II | $R^7$I |
| $R^3$I | $R^a$IV | $R^b$II | $R^7$II |
| $R^3$I | $R^a$IV | $R^b$II | $R^7$III |
| $R^3$I | $R^a$IV | $R^b$II | $R^7$IV |
| $R^3$I | $R^a$IV | $R^b$II | $R^7$V |
| $R^3$I | $R^a$IV | $R^b$II | $R^7$VI |
| $R^3$I | $R^a$IV | $R^b$II | $R^7$VII |
| $R^3$I | $R^a$IV | $R^b$II | $R^7$VIII |
| $R^3$I | $R^a$IV | $R^b$II | $R^7$IX |
| $R^3$I | $R^a$IV | $R^b$III | $R^7$I |
| $R^3$I | $R^a$IV | $R^b$III | $R^7$II |
| $R^3$I | $R^a$IV | $R^b$III | $R^7$III |
| $R^3$I | $R^a$IV | $R^b$III | $R^7$IV |
| $R^3$I | $R^a$IV | $R^b$III | $R^7$V |
| $R^3$I | $R^a$IV | $R^b$III | $R^7$VI |
| $R^3$I | $R^a$IV | $R^b$III | $R^7$VII |
| $R^3$I | $R^a$IV | $R^b$III | $R^7$VIII |
| $R^3$I | $R^a$IV | $R^b$III | $R^7$IX |
| $R^3$I | $R^a$IV | $R^b$IV | -- |
| $R^3$I | $R^a$IV | $R^b$V | -- |
| $R^3$I | $R^a$IV | $R^b$VI | -- |
| $R^3$I | $R^a$V | $R^b$I | $R^7$I |
| $R^3$I | $R^a$V | $R^b$I | $R^7$II |
| $R^3$I | $R^a$V | $R^b$I | $R^7$III |
| $R^3$I | $R^a$V | $R^b$I | $R^7$IV |
| $R^3$I | $R^a$V | $R^b$I | $R^7$V |
| $R^3$I | $R^a$V | $R^b$I | $R^7$VI |
| $R^3$I | $R^a$V | $R^b$I | $R^7$VII |
| $R^3$I | $R^a$V | $R^b$I | $R^7$VIII |
| $R^3$I | $R^a$V | $R^b$I | $R^7$IX |
| $R^3$I | $R^a$V | $R^b$II | $R^7$I |
| $R^3$I | $R^a$V | $R^b$II | $R^7$II |
| $R^3$I | $R^a$V | $R^b$II | $R^7$III |
| $R^3$I | $R^a$V | $R^b$II | $R^7$IV |
| $R^3$I | $R^a$V | $R^b$II | $R^7$V |
| $R^3$I | $R^a$V | $R^b$II | $R^7$VI |
| $R^3$I | $R^a$V | $R^b$II | $R^7$VII |
| $R^3$I | $R^a$V | $R^b$II | $R^7$VIII |
| $R^3$I | $R^a$V | $R^b$II | $R^7$IX |
| $R^3$I | $R^a$V | $R^b$III | $R^7$I |

(continued)

| $R^3$ | $R^a$ | $R^b$ | $R^7$ |
|---|---|---|---|
| $R^3I$ | $R^aV$ | $R^bIII$ | $R^7II$ |
| $R^3I$ | $R^aV$ | $R^bIII$ | $R^7III$ |
| $R^3I$ | $R^aV$ | $R^bIII$ | $R^7IV$ |
| $R^3I$ | $R^aV$ | $R^bIII$ | $R^7V$ |
| $R^3I$ | $R^aV$ | $R^bIII$ | $R^7VI$ |
| $R^3I$ | $R^aV$ | $R^bIII$ | $R^7VII$ |
| $R^3I$ | $R^aV$ | $R^bIII$ | $R^7VIII$ |
| $R^3I$ | $R^aV$ | $R^bIII$ | $R^7IX$ |
| $R^3I$ | $R^aV$ | $R^bIV$ | -- |
| $R^3I$ | $R^aV$ | $R^bV$ | -- |
| $R^3I$ | $R^aV$ | $R^bVI$ | -- |
| $R^3I$ | $R^aVI$ | $R^bI$ | $R^7I$ |
| $R^3I$ | $R^aVI$ | $R^bI$ | $R^7II$ |
| $R^3I$ | $R^aVI$ | $R^bI$ | $R^7III$ |
| $R^3I$ | $R^aVI$ | $R^bI$ | $R^7IV$ |
| $R^3I$ | $R^aVI$ | $R^bI$ | $R^7V$ |
| $R^3I$ | $R^aVI$ | $R^bI$ | $R^7VI$ |
| $R^3I$ | $R^aVI$ | $R^bI$ | $R^7VII$ |
| $R^3I$ | $R^aVI$ | $R^bI$ | $R^7VIII$ |
| $R^3I$ | $R^aVI$ | $R^bI$ | $R^7IX$ |
| $R^3I$ | $R^aVI$ | $R^bII$ | $R^7I$ |
| $R^3I$ | $R^aVI$ | $R^bII$ | $R^7II$ |
| $R^3I$ | $R^aVI$ | $R^bII$ | $R^7III$ |
| $R^3I$ | $R^aVI$ | $R^bII$ | $R^7IV$ |
| $R^3I$ | $R^aVI$ | $R^bII$ | $R^7V$ |
| $R^3I$ | $R^aVI$ | $R^bII$ | $R^7VI$ |
| $R^3I$ | $R^aVI$ | $R^bII$ | $R^7VII$ |
| $R^3I$ | $R^aVI$ | $R^bII$ | $R^7VIII$ |
| $R^3I$ | $R^aVI$ | $R^bII$ | $R^7IX$ |
| $R^3I$ | $R^aVI$ | $R^bIII$ | $R^7I$ |
| $R^3I$ | $R^aVI$ | $R^bIII$ | $R^7II$ |
| $R^3I$ | $R^aVI$ | $R^bIII$ | $R^7III$ |
| $R^3I$ | $R^aVI$ | $R^bIII$ | $R^7IV$ |
| $R^3I$ | $R^aVI$ | $R^bIII$ | $R^7V$ |
| $R^3I$ | $R^aVI$ | $R^bIII$ | $R^7VI$ |
| $R^3I$ | $R^aVI$ | $R^bIII$ | $R^7VII$ |
| $R^3I$ | $R^aVI$ | $R^bIII$ | $R^7VIII$ |
| $R^3I$ | $R^aVI$ | $R^bIII$ | $R^7IX$ |
| $R^3I$ | $R^aVI$ | $R^bIV$ | -- |
| $R^3I$ | $R^aVI$ | $R^bV$ | -- |
| $R^3I$ | $R^aVI$ | $R^bVI$ | -- |
| $R^3I$ | $R^aVII$ | $R^bI$ | $R^7I$ |
| $R^3I$ | $R^aVII$ | $R^bI$ | $R^7II$ |
| $R^3I$ | $R^aVII$ | $R^bI$ | $R^7III$ |
| $R^3I$ | $R^aVII$ | $R^bI$ | $R^7$ IV |
| $R^3I$ | $R^aVII$ | $R^bI$ | $R^7V$ |
| $R^3I$ | $R^aVII$ | $R^bI$ | $R^7VI$ |
| $R^3I$ | $R^aVII$ | $R^bI$ | $R^7VII$ |
| $R^3I$ | $R^aVII$ | $R^bI$ | $R^7VIII$ |

(continued)

| $R^3$ | $R^a$ | $R^b$ | $R^7$ |
|---|---|---|---|
| $R^3$I | $R^a$VII | $R^b$I | $R^7$IX |
| $R^3$I | $R^a$VII | $R^b$II | $R^7$I |
| $R^3$I | $R^a$VII | $R^b$II | $R^7$II |
| $R^3$I | $R^a$VII | $R^b$II | $R^7$III |
| $R^3$I | $R^a$VII | $R^b$II | $R^7$IV |
| $R^3$I | $R^a$VII | $R^b$II | $R^7$V |
| $R^3$I | $R^a$VII | $R^b$II | $R^7$VI |
| $R^3$I | $R^a$VII | $R^b$II | $R^7$VII |
| $R^3$I | $R^a$VII | $R^b$II | $R^7$VIII |
| $R^3$I | $R^a$VII | $R^b$II | $R^7$IX |
| $R^3$I | $R^a$VII | $R^b$III | $R^7$I |
| $R^3$I | $R^a$VII | $R^b$III | $R^7$II |
| $R^3$I | $R^a$VII | $R^b$III | $R^7$III |
| $R^3$I | $R^a$VII | $R^b$III | $R^7$IV |
| $R^3$I | $R^a$VII | $R^b$III | $R^7$V |
| $R^3$I | $R^a$VII | $R^b$III | $R^7$VI |
| $R^3$I | $R^a$VII | $R^b$III | $R^7$VII |
| $R^3$I | $R^a$VII | $R^b$III | $R^7$VIII |
| $R^3$I | $R^a$VII | $R^b$III | $R^7$IX |
| $R^3$I | $R^a$VII | $R^b$IV | -- |
| $R^3$I | $R^a$VII | $R^b$V | -- |
| $R^3$I | $R^a$VII | $R^b$VI | -- |
| $R^3$I | $R^a$VIII | $R^b$I | $R^7$I |
| $R^3$I | $R^a$VIII | $R^b$I | $R^7$II |
| $R^3$I | $R^a$VIII | $R^b$I | $R^7$III |
| $R^3$I | $R^a$VIII | $R^b$I | $R^7$IV |
| $R^3$I | $R^a$VIII | $R^b$I | $R^7$V |
| $R^3$I | $R^a$VIII | $R^b$I | $R^7$VI |
| $R^3$I | $R^a$VIII | $R^b$I | $R^7$VII |
| $R^3$I | $R^a$VIII | $R^b$I | $R^7$VIII |
| $R^3$I | $R^a$VIII | $R^b$I | $R^7$IX |
| $R^3$I | $R^a$VIII | $R^b$II | $R^7$I |
| $R^3$I | $R^a$VIII | $R^b$II | $R^7$II |
| $R^3$I | $R^a$VIII | $R^b$II | $R^7$III |
| $R^3$I | $R^a$VIII | $R^b$II | $R^7$IV |
| $R^3$I | $R^a$VIII | $R^b$II | $R^7$V |
| $R^3$I | $R^a$VIII | $R^b$II | $R^7$VI |
| $R^3$I | $R^a$VIII | $R^b$II | $R^7$VII |
| $R^3$I | $R^a$VIII | $R^b$II | $R^7$VIII |
| $R^3$I | $R^a$VIII | $R^b$II | $R^7$IX |
| $R^3$I | $R^a$VIII | $R^b$III | $R^7$I |
| $R^3$I | $R^a$VIII | $R^b$III | $R^7$II |
| $R^3$I | $R^a$VIII | $R^b$III | $R^7$III |
| $R^3$I | $R^a$VIII | $R^b$III | $R^7$IV |
| $R^3$I | $R^a$VIII | $R^b$III | $R^7$V |
| $R^3$I | $R^a$VIII | $R^b$III | $R^7$VI |
| $R^3$I | $R^a$VIII | $R^b$III | $R^7$VII |
| $R^3$I | $R^a$VIII | $R^b$III | $R^7$VIII |
| $R^3$I | $R^a$VIII | $R^b$III | $R^7$IX |

(continued)

| $R^3$ | $R^a$ | $R^b$ | $R^7$ |
|---|---|---|---|
| $R^3$I | $R^a$ VIII | $R^b$IV | -- |
| $R^3$I | $R^a$VIII | $R^b$V | -- |
| $R^3$I | $R^a$VIII | $R^b$VI | -- |
| $R^3$I | $R^a$IX | $R^b$I | $R^7$I |
| $R^3$I | $R^a$IX | $R^b$I | $R^7$II |
| $R^3$I | $R^a$IX | $R^b$I | $R^7$III |
| $R^3$I | $R^a$IX | $R^b$I | $R^7$IV |
| $R^3$I | $R^a$IX | $R^b$I | $R^7$V |
| $R^3$I | $R^a$IX | $R^b$I | $R^7$VI |
| $R^3$I | $R^a$IX | $R^b$I | $R^7$VII |
| $R^3$I | $R^a$IX | $R^b$I | $R^7$VIII |
| $R^3$I | $R^a$IX | $R^b$I | $R^7$IX |
| $R^3$I | $R^a$IX | $R^b$II | $R^7$I |
| $R^3$I | $R^a$IX | $R^b$II | $R^7$II |
| $R^3$I | $R^a$IX | $R^b$II | $R^7$III |
| $R^3$I | $R^a$IX | $R^b$II | $R^7$IV |
| $R^3$I | $R^a$IX | $R^b$II | $R^7$V |
| $R^3$I | $R^a$IX | $R^b$II | $R^7$VI |
| $R^3$I | $R^a$IX | $R^b$II | $R^7$VII |
| $R^3$I | $R^a$IX | $R^b$II | $R^7$VIII |
| $R^3$I | $R^a$IX | $R^b$II | $R^7$IX |
| $R^3$I | $R^a$IX | $R^b$III | $R^7$I |
| $R^3$I | $R^a$IX | $R^b$III | $R^7$II |
| $R^3$I | $R^a$IX | $R^b$III | $R^7$III |
| $R^3$I | $R^a$IX | $R^b$III | $R^7$IV |
| $R^3$I | $R^a$IX | $R^b$III | $R^7$V |
| $R^3$I | $R^a$IX | $R^b$III | $R^7$VI |
| $R^3$I | $R^a$IX | $R^b$III | $R^7$VII |
| $R^3$I | $R^a$IX | $R^b$III | $R^7$VIII |
| $R^3$I | $R^a$IX | $R^b$III | $R^7$IX |
| $R^3$I | $R^a$IX | $R^b$IV | -- |
| $R^3$I | $R^a$IX | $R^b$V | -- |
| $R^3$I | $R^a$IX | $R^b$VI | -- |
| $R^3$I | $R^a$X | $R^b$I | $R^7$I |
| $R^3$I | $R^a$X | $R^b$I | $R^7$II |
| $R^3$I | $R^a$X | $R^b$I | $R^7$III |
| $R^3$I | $R^a$X | $R^b$I | $R^7$IV |
| $R^3$I | $R^a$X | $R^b$I | $R^7$V |
| $R^3$I | $R^a$X | $R^b$I | $R^7$VI |
| $R^3$I | $R^a$X | $R^b$I | $R^7$VII |
| $R^3$I | $R^a$X | $R^b$I | $R^7$VIII |
| $R^3$I | $R^a$X | $R^b$I | $R^7$IX |
| $R^3$I | $R^a$X | $R^b$II | $R^7$I |
| $R^3$I | $R^a$X | $R^b$II | $R^7$II |
| $R^3$I | $R^a$X | $R^b$II | $R^7$III |
| $R^3$I | $R^a$X | $R^b$II | $R^7$IV |
| $R^3$I | $R^a$X | $R^b$II | $R^7$V |
| $R^3$I | $R^a$X | $R^b$II | $R^7$VI |
| $R^3$I | $R^a$X | $R^b$II | $R^7$VII |

(continued)

| $R^3$ | $R^a$ | $R^b$ | $R^7$ |
|---|---|---|---|
| $R^3$I | $R^a$X | $R^b$II | $R^7$VIII |
| $R^3$I | $R^a$X | $R^b$II | $R^7$IX |
| $R^3$I | $R^a$X | $R^b$III | $R^7$I |
| $R^3$I | $R^a$X | $R^b$III | $R^7$II |
| $R^3$I | $R^a$X | $R^b$III | $R^7$III |
| $R^3$I | $R^a$X | $R^b$III | $R^7$IV |
| $R^3$I | $R^a$X | $R^b$III | $R^7$V |
| $R^3$I | $R^a$X | $R^b$III | $R^7$VI |
| $R^3$I | $R^a$X | $R^b$III | $R^7$VII |
| $R^3$I | $R^a$X | $R^b$III | $R^7$VIII |
| $R^3$I | $R^a$X | $R^b$III | $R^7$IX |
| $R^3$I | $R^a$X | $R^b$IV | -- |
| $R^3$I | $R^a$X | $R^b$V | -- |
| $R^3$I | $R^a$X | $R^b$VI | -- |
| $R^3$II | $R^a$I | $R^b$I | $R^7$I |
| $R^3$II | $R^a$I | $R^b$I | $R^7$II |
| $R^3$II | $R^a$I | $R^b$I | $R^7$III |
| $R^3$II | $R^a$I | $R^b$I | $R^7$IV |
| $R^3$II | $R^a$I | $R^b$I | $R^7$V |
| $R^3$II | $R^a$I | $R^b$I | $R^7$VI |
| $R^3$II | $R^a$I | $R^b$I | $R^7$VII |
| $R^3$II | $R^a$I | $R^b$I | $R^7$VIII |
| $R^3$II | $R^a$I | $R^b$I | $R^7$IX |
| $R^3$II | $R^a$I | $R^b$II | $R^7$I |
| $R^3$II | $R^a$I | $R^b$II | $R^7$II |
| $R^3$II | $R^a$I | $R^b$II | $R^7$III |
| $R^3$II | $R^a$I | $R^b$II | $R^7$IV |
| $R^3$II | $R^a$I | $R^b$II | $R^7$V |
| $R^3$II | $R^a$I | $R^b$II | $R^7$VI |
| $R^3$II | $R^a$I | $R^b$II | $R^7$VII |
| $R^3$II | $R^a$I | $R^b$II | $R^7$VIII |
| $R^3$II | $R^a$I | $R^b$II | $R^7$IX |
| $R^3$II | $R^a$I | $R^b$III | $R^7$I |
| $R^3$II | $R^a$I | $R^b$III | $R^7$II |
| $R^3$II | $R^a$I | $R^b$III | $R^7$III |
| $R^3$II | $R^a$I | $R^b$III | $R^7$IV |
| $R^3$II | $R^a$I | $R^b$III | $R^7$V |
| $R^3$II | $R^a$I | $R^b$III | $R^7$VI |
| $R^3$II | $R^a$I | $R^b$III | $R^7$VII |
| $R^3$II | $R^a$I | $R^b$III | $R^7$VIII |
| $R^3$II | $R^a$I | $R^b$III | $R^7$IX |
| $R^3$II | $R^a$I | $R^b$IV | -- |
| $R^3$II | $R^a$I | $R^b$V | -- |
| $R^3$II | $R^a$I | $R^b$VI | -- |
| $R^3$II | $R^a$II | $R^b$I | $R^7$I |
| $R^3$II | $R^a$II | $R^b$I | $R^7$II |
| $R^3$II | $R^a$II | $R^b$I | $R^7$III |
| $R^3$II | $R^a$II | $R^b$I | $R^7$IV |
| $R^3$II | $R^a$II | $R^b$I | $R^7$V |

(continued)

| $R^3$ | $R^a$ | $R^b$ | $R^7$ |
|---|---|---|---|
| $R^3$II | $R^a$II | $R^b$I | $R^7$VI |
| $R^3$II | $R^a$II | $R^b$I | $R^7$VII |
| $R^3$II | $R^a$II | $R^b$I | $R^7$VIII |
| $R^3$II | $R^a$II | $R^b$I | $R^7$IX |
| $R^3$II | $R^a$II | $R^b$II | $R^7$I |
| $R^3$II | $R^a$II | $R^b$II | $R^7$II |
| $R^3$II | $R^a$II | $R^b$II | $R^7$III |
| $R^3$II | $R^a$II | $R^b$II | $R^7$IV |
| $R^3$II | $R^a$II | $R^b$II | $R^7$V |
| $R^3$II | $R^a$II | $R^b$II | $R^7$VI |
| $R^3$II | $R^a$II | $R^b$II | $R^7$VII |
| $R^3$II | $R^a$II | $R^b$II | $R^7$VIII |
| $R^3$II | $R^a$II | $R^b$II | $R^7$IX |
| $R^3$II | $R^a$II | $R^b$III | $R^7$I |
| $R^3$II | $R^a$II | $R^b$III | $R^7$II |
| $R^3$II | $R^a$II | $R^b$III | $R^7$III |
| $R^3$II | $R^a$II | $R^b$III | $R^7$IV |
| $R^3$II | $R^a$II | $R^b$III | $R^7$V |
| $R^3$II | $R^a$II | $R^b$III | $R^7$VI |
| $R^3$II | $R^a$II | $R^b$III | $R^7$VII |
| $R^3$II | $R^a$II | $R^b$III | $R^7$VIII |
| $R^3$II | $R^a$II | $R^b$III | $R^7$IX |
| $R^3$II | $R^a$II | $R^b$IV | -- |
| $R^3$II | $R^a$II | $R^b$V | -- |
| $R^3$II | $R^a$II | $R^b$VI | -- |
| $R^3$II | $R^a$III | $R^b$I | $R^7$I |
| $R^3$II | $R^a$III | $R^b$I | $R^7$II |
| $R^3$II | $R^a$III | $R^b$I | $R^7$III |
| $R^3$II | $R^a$III | $R^b$I | $R^7$IV |
| $R^3$II | $R^a$III | $R^b$I | $R^7$V |
| $R^3$II | $R^a$III | $R^b$I | $R^7$VI |
| $R^3$II | $R^a$III | $R^b$I | $R^7$VII |
| $R^3$II | $R^a$ III | $R^b$I | $R^7$VIII |
| $R^3$II | $R^a$III | $R^b$I | $R^7$IX |
| $R^3$II | $R^a$III | $R^b$II | $R^7$I |
| $R^3$II | $R^a$III | $R^b$II | $R^7$II |
| $R^3$II | $R^a$III | $R^b$II | $R^7$III |
| $R^3$II | $R^a$III | $R^b$II | $R^7$IV |
| $R^3$II | $R^a$III | $R^b$II | $R^7$V |
| $R^3$II | $R^a$III | $R^b$II | $R^7$VI |
| $R^3$II | $R^a$III | $R^b$II | $R^7$VII |
| $R^3$II | $R^a$III | $R^b$II | $R^7$VIII |
| $R^3$II | $R^a$III | $R^b$II | $R^7$IX |
| $R^3$II | $R^a$III | $R^b$III | $R^7$I |
| $R^3$II | $R^a$III | $R^b$III | $R^7$II |
| $R^3$II | $R^a$III | $R^b$III | $R^7$III |
| $R^3$II | $R^a$III | $R^b$III | $R^7$IV |
| $R^3$II | $R^a$III | $R^b$III | $R^7$V |
| $R^3$II | $R^a$III | $R^b$III | $R^7$VI |

(continued)

| $R^3$ | $R^a$ | $R^b$ | $R^7$ |
|---|---|---|---|
| $R^3$II | $R^a$III | $R^b$III | $R^7$VII |
| $R^3$II | $R^a$III | $R^b$III | $R^7$VIII |
| $R^3$II | $R^a$III | $R^b$III | $R^7$IX |
| $R^3$I | $R^a$III | $R^b$IV | -- |
| $R^3$II | $R^a$III | $R^b$V | -- |
| $R^3$II | $R^a$III | $R^b$VI | -- |
| $R^3$II | $R^a$IV | $R^b$I | $R^7$I |
| $R^3$II | $R^a$IV | $R^b$I | $R^7$II |
| $R^3$II | $R^a$IV | $R^b$I | $R^7$III |
| $R^3$II | $R^a$IV | $R^b$I | $R^7$IV |
| $R^3$II | $R^a$IV | $R^b$I | $R^7$V |
| $R^3$II | $R^a$IV | $R^b$I | $R^7$VI |
| $R^3$II | $R^a$IV | $R^b$I | $R^7$VII |
| $R^3$II | $R^a$IV | $R^b$I | $R^7$VIII |
| $R^3$II | $R^a$IV | $R^b$I | $R^7$IX |
| $R^3$II | $R^a$IV | $R^b$II | $R^7$I |
| $R^3$II | $R^a$IV | $R^b$II | $R^7$II |
| $R^3$II | $R^a$IV | $R^b$II | $R^7$III |
| $R^3$II | $R^a$IV | $R^b$II | $R^7$IV |
| $R^3$II | $R^a$IV | $R^b$II | $R^7$V |
| $R^3$II | $R^a$IV | $R^b$II | $R^7$VI |
| $R^3$II | $R^a$IV | $R^b$II | $R^7$VII |
| $R^3$II | $R^a$IV | $R^b$II | $R^7$VIII |
| $R^3$II | $R^a$IV | $R^b$II | $R^7$IX |
| $R^3$II | $R^a$IV | $R^b$III | $R^7$I |
| $R^3$II | $R^a$IV | $R^b$III | $R^7$II |
| $R^3$II | $R^a$IV | $R^b$III | $R^7$III |
| $R^3$II | $R^a$IV | $R^b$III | $R^7$IV |
| $R^3$II | $R^a$IV | $R^b$III | $R^7$V |
| $R^3$II | $R^a$IV | $R^b$III | $R^7$VI |
| $R^3$II | $R^a$IV | $R^b$III | $R^7$VII |
| $R^3$II | $R^a$IV | $R^b$III | $R^7$VIII |
| $R^3$II | $R^a$IV | $R^b$III | $R^7$IX |
| $R^3$II | $R^a$IV | $R^b$IV | -- |
| $R^3$II | $R^a$IV | $R^b$V | -- |
| $R^3$II | $R^a$IV | $R^b$VI | -- |
| $R^3$II | $R^a$V | $R^b$I | $R^7$I |
| $R^3$II | $R^a$V | $R^b$I | $R^7$II |
| $R^3$II | $R^a$V | $R^b$I | $R^7$III |
| $R^3$II | $R^a$V | $R^b$I | $R^7$IV |
| $R^3$II | $R^a$V | $R^b$I | $R^7$V |
| $R^3$II | $R^a$V | $R^b$I | $R^7$VI |
| $R^3$II | $R^a$V | $R^b$I | $R^7$VII |
| $R^3$II | $R^a$V | $R^b$I | $R^7$VIII |
| $R^3$II | $R^a$V | $R^b$I | $R^7$IX |
| $R^3$II | $R^a$V | $R^b$II | $R^7$I |
| $R^3$II | $R^a$V | $R^b$II | $R^7$II |
| $R^3$II | $R^a$V | $R^b$II | $R^7$III |
| $R^3$II | $R^a$V | $R^b$II | $R^7$IV |

(continued)

| $R^3$ | $R^a$ | $R^b$ | $R^7$ |
|---|---|---|---|
| $R^3$II | $R^a$V | $R^b$II | $R^7$V |
| $R^3$II | $R^a$V | $R^b$II | $R^7$VI |
| $R^3$II | $R^a$V | $R^b$II | $R^7$VII |
| $R^3$II | $R^a$V | $R^b$II | $R^7$VII |
| $R^3$II | $R^a$V | $R^b$II | $R^7$IX |
| $R^3$II | $R^a$V | $R^b$III | $R^7$I |
| $R^3$II | $R^a$V | $R^b$III | $R^7$II |
| $R^3$II | $R^a$V | $R^b$III | $R^7$III |
| $R^3$II | $R^a$V | $R^b$III | $R^7$IV |
| $R^3$II | $R^a$V | $R^b$III | $R^7$V |
| $R^3$II | $R^a$V | $R^b$II | $R^7$VI |
| $R^3$II | $R^a$V | $R^b$III | $R^7$VI |
| $R^3$II | $R^a$V | $R^b$III | $R^7$VIII |
| $R^3$II | $R^a$V | $R^b$III | $R^7$IX |
| $R^3$II | $R^a$V | $R^b$IV | -- |
| $R^3$II | $R^a$V | $R^b$V | -- |
| $R^3$II | $R^a$V | $R^b$VI | -- |
| $R^3$II | $R^a$VI | $R^b$I | $R^7$I |
| $R^3$II | $R^a$VI | $R^b$I | $R^7$II |
| $R^3$II | $R^a$VI | $R^b$I | $R^7$III |
| $R^3$II | $R^a$VI | $R^b$I | $R^7$IV |
| $R^3$II | $R^a$VI | $R^b$I | $R^7$V |
| $R^3$II | $R^a$VI | $R^b$I | $R^7$VI |
| $R^3$II | $R^a$VI | $R^b$I | $R^7$VII |
| $R^3$II | $R^a$VI | $R^b$I | $R^7$VIII |
| $R^3$II | $R^a$VI | $R^b$I | $R^7$IX |
| $R^3$II | $R^a$VI | $R^b$II | $R^7$I |
| $R^3$II | $R^a$VI | $R^b$II | $R^7$II |
| $R^3$II | $R^a$VI | $R^b$II | $R^7$III |
| $R^3$II | $R^a$VI | $R^b$II | $R^7$IV |
| $R^3$II | $R^a$VI | $R^b$II | $R^7$V |
| $R^3$II | $R^a$VI | $R^b$II | $R^7$VI |
| $R^3$II | $R^a$VI | $R^b$II | $R^7$VII |
| $R^3$II | $R^a$VI | $R^b$II | $R^7$VIII |
| $R^3$II | $R^a$VI | $R^b$II | $R^7$IX |
| $R^3$II | $R^a$VI | $R^b$III | $R^7$I |
| $R^3$II | $R^a$VI | $R^b$III | $R^7$II |
| $R^3$II | $R^a$VI | $R^b$III | $R^7$III |
| $R^3$II | $R^a$VI | $R^b$III | $R^7$IV |
| $R^3$II | $R^a$VI | $R^b$III | $R^7$V |
| $R^3$II | $R^a$VI | $R^b$III | $R^7$VI |
| $R^3$II | $R^a$VI | $R^b$III | $R^7$VII |
| $R^3$II | $R^a$VI | $R^b$III | $R^7$VIII |
| $R^3$II | $R^a$VI | $R^b$III | $R^7$IX |
| $R^3$II | $R^a$VI | $R^b$IV | -- |
| $R^3$II | $R^a$VI | $R^b$V | -- |
| $R^3$II | $R^a$VI | $R^b$VI | -- |
| $R^3$II | $R^a$VII | $R^b$I | $R^7$I |
| $R^3$II | $R^a$VII | $R^b$I | $R^7$II |

(continued)

| $R^3$ | $R^a$ | $R^b$ | $R^7$ |
|---|---|---|---|
| $R^3$II | $R^a$VII | $R^b$I | $R^7$III |
| $R^3$II | $R^a$VII | $R^b$I | $R^7$IV |
| $R^3$II | $R^a$VII | $R^b$I | $R^7$V |
| $R^3$II | $R^a$VII | $R^b$I | $R^7$VI |
| $R^3$II | $R^a$VI | $R^b$I | $R^7$VII |
| $R^3$II | $R^a$VII | $R^b$I | $R^7$VIII |
| $R^3$II | $R^a$VII | $R^b$I | $R^7$IX |
| $R^3$II | $R^a$VII | $R^b$II | $R^7$I |
| $R^3$II | $R^a$VII | $R^b$II | $R^7$II |
| $R^3$II | $R^a$VII | $R^b$II | $R^7$III |
| $R^3$II | $R^a$VII | $R^b$II | $R^7$IV |
| $R^3$II | $R^a$VII | $R^b$II | $R^7$V |
| $R^3$II | $R^a$VII | $R^b$II | $R^7$VI |
| $R^3$II | $R^a$VII | $R^b$II | $R^7$VII |
| $R^3$II | $R^a$VII | $R^b$II | $R^7$VIII |
| $R^3$II | $R^a$VII | $R^b$II | $R^7$IX |
| $R^3$II | $R^a$VII | $R^b$III | $R^7$I |
| $R^3$II | $R^a$VII | $R^b$III | $R^7$II |
| $R^3$II | $R^a$VII | $R^b$III | $R^7$III |
| $R^3$II | $R^a$VII | $R^b$III | $R^7$IV |
| $R^3$II | $R^a$VII | $R^b$III | $R^7$V |
| $R^3$II | $R^a$VII | $R^b$III | $R^7$VI |
| $R^3$II | $R^a$VII | $R^b$III | $R^7$VII |
| $R^3$II | $R^a$VI | $R^b$III | $R^7$VIII |
| $R^3$II | $R^a$VII | $R^b$II | $R^7$IX |
| $R^3$II | $R^a$VII | $R^b$IV | -- |
| $R^3$II | $R^a$VI | $R^b$V | -- |
| $R^3$II | $R^a$VII | $R^b$VI | -- |
| $R^3$II | $R^a$VIII | $R^b$I | $R^7$I |
| $R^3$II | $R^a$VIII | $R^b$I | $R^7$II |
| $R^3$II | $R^a$VIII | $R^b$I | $R^7$III |
| $R^3$II | $R^a$VIII | $R^b$I | $R^7$IV |
| $R^3$II | $R^a$VIII | $R^b$I | $R^7$V |
| $R^3$II | $R^a$VIII | $R^b$I | $R^7$VI |
| $R^3$II | $R^a$VIII | $R^b$I | $R^7$VII |
| $R^3$II | $R^a$VIII | $R^b$I | $R^7$VIII |
| $R^3$II | $R^a$VIII | $R^b$I | $R^7$IX |
| $R^3$II | $R^a$VIII | $R^b$II | $R^7$I |
| $R^3$II | $R^a$VIII | $R^b$II | $R^7$II |
| $R^3$II | $R^a$VIII | $R^b$II | $R^7$III |
| $R^3$II | $R^a$VIII | $R^b$II | $R^7$IV |
| $R^3$II | $R^a$VIII | $R^b$II | $R^7$V |
| $R^3$II | $R^a$VIII | $R^b$II | $R^7$VI |
| $R^3$II | $R^a$VIII | $R^b$II | $R^7$VII |
| $R^3$II | $R^a$VIII | $R^b$II | $R^7$VIII |
| $R^3$II | $R^a$VIII | $R^b$II | $R^7$IX |
| $R^3$II | $R^a$VII | $R^b$III | $R^7$I |
| $R^3$II | $R^a$VIII | $R^b$III | $R^7$II |
| $R^3$II | $R^a$VIII | $R^b$III | $R^7$III |

(continued)

| $R^3$ | $R^a$ | $R^b$ | $R^7$ |
|---|---|---|---|
| $R^3$II | $R^a$VIII | $R^b$III | $R^7$IV |
| $R^3$II | $R^a$VIII | $R^b$III | $R^7$V |
| $R^3$II | $R^a$VIII | $R^b$III | $R^7$VI |
| $R^3$II | $R^a$VIII | $R^b$III | $R^7$VII |
| $R^3$II | $R^a$VIII | $R^b$III | $R^7$VIII |
| $R^3$II | $R^a$VIII | $R^b$III | $R^7$IX |
| $R^3$II | $R^a$VIII | $R^b$IV | -- |
| $R^3$II | $R^a$VIII | $R^b$V | -- |
| $R^3$II | $R^a$VIII | $R^b$VI | -- |
| $R^3$II | $R^a$IX | $R^b$I | $R^7$I |
| $R^3$II | $R^a$IX | $R^b$I | $R^7$II |
| $R^3$II | $R^a$IX | $R^b$I | $R^7$III |
| $R^3$II | $R^a$IX | $R^b$I | $R^7$IV |
| $R^3$II | $R^a$IX | $R^b$I | $R^7$V |
| $R^3$II | $R^a$IX | $R^b$I | $R^7$VI |
| $R^3$II | $R^a$IX | $R^b$I | $R^7$VII |
| $R^3$II | $R^a$IX | $R^b$I | $R^7$VIII |
| $R^3$II | $R^a$IX | $R^b$I | $R^7$IX |
| $R^3$II | $R^a$IX | $R^b$II | $R^7$I |
| $R^3$II | $R^a$IX | $R^b$II | $R^7$II |
| $R^3$II | $R^a$IX | $R^b$II | $R^7$III |
| $R^3$II | $R^a$IX | $R^b$II | $R^7$IV |
| $R^3$II | $R^a$IX | $R^b$II | $R^7$V |
| $R^3$II | $R^a$IX | $R^b$II | $R^7$VI |
| $R^3$II | $R^a$IX | $R^b$II | $R^7$VII |
| $R^3$II | $R^a$IX | $R^b$II | $R^7$VIII |
| $R^3$II | $R^a$IX | $R^b$II | $R^7$IX |
| $R^3$II | $R^a$IX | $R^b$III | $R^7$I |
| $R^3$II | $R^a$IX | $R^b$III | $R^7$II |
| $R^3$II | $R^a$IX | $R^b$III | $R^7$III |
| $R^3$II | $R^a$IX | $R^b$III | $R^7$IV |
| $R^3$II | $R^a$IX | $R^b$III | $R^7$V |
| $R^3$II | $R^a$IX | $R^b$III | $R^7$VI |
| $R^3$II | $R^a$IX | $R^b$III | $R^7$VII |
| $R^3$II | $R^a$IX | $R^b$III | $R^7$VIII |
| $R^3$II | $R^a$IX | $R^b$III | $R^7$IX |
| $R^3$II | $R^a$IX | $R^b$IV | -- |
| $R^3$II | $R^a$IX | $R^b$V | -- |
| $R^3$II | $R^a$IX | $R^b$VI | -- |
| $R^3$II | $R^a$X | $R^b$I | $R^7$I |
| $R^3$II | $R^a$X | $R^b$I | $R^7$II |
| $R^3$II | $R^a$X | $R^b$I | $R^7$III |
| $R^3$II | $R^a$X | $R^b$I | $R^7$IV |
| $R^3$II | $R^a$X | $R^b$I | $R^7$V |
| $R^3$II | $R^a$X | $R^b$I | $R^7$VI |
| $R^3$II | $R^a$X | $R^b$I | $R^7$VII |
| $R^3$II | $R^a$X | $R^b$I | $R^7$VIII |
| $R^3$II | $R^a$X | $R^b$I | $R^7$IX |
| $R^3$II | $R^a$X | $R^b$II | $R^7$I |

(continued)

| $R^3$ | $R^a$ | $R^b$ | $R^7$ |
|---|---|---|---|
| $R^3$II | $R^a$X | $R^b$II | $R^7$II |
| $R^3$II | $R^a$X | $R^b$II | $R^7$III |
| $R^3$II | $R^a$X | $R^b$II | $R^7$IV |
| $R^3$II | $R^a$X | $R^b$II | $R^7$V |
| $R^3$II | $R^a$X | $R^b$II | $R^7$VI |
| $R^3$II | $R^a$X | $R^b$II | $R^7$VII |
| $R^3$II | $R^a$X | $R^b$II | $R^7$VIII |
| $R^3$II | $R^a$X | $R^b$II | $R^7$IX |
| $R^3$II | $R^a$X | $R^b$III | $R^7$I |
| $R^3$II | $R^a$X | $R^b$III | $R^7$II |
| $R^3$II | $R^a$X | $R^b$III | $R^7$III |
| $R^3$II | $R^a$X | $R^b$III | $R^7$IV |
| $R^3$II | $R^a$X | $R^b$III | $R^7$V |
| $R^3$II | $R^a$X | $R^b$III | $R^7$VI |
| $R^3$II | $R^a$X | $R^b$III | $R^7$VII |
| $R^3$II | $R^a$X | $R^b$III | $R^7$VIII |
| $R^3$II | $R^a$X | $R^b$III | $R^7$IX |
| $R^3$II | $R^a$X | $R^b$IV | -- |
| $R^3$II | $R^a$X | $R^b$V | -- |
| $R^3$II | $R^a$X | $R^b$VI | -- |
| $R^3$III | $R^a$I | $R^b$I | $R^7$I |
| $R^3$III | $R^a$I | $R^b$I | $R^7$II |
| $R^3$III | $R^a$I | $R^b$I | $R^7$III |
| $R^3$III | $R^a$I | $R^b$I | $R^7$IV |
| $R^3$III | $R^a$I | $R^b$I | $R^7$V |
| $R^3$III | $R^a$I | $R^b$I | $R^7$VI |
| $R^3$III | $R^a$I | $R^b$I | $R^7$VII |
| $R^3$III | $R^a$I | $R^b$I | $R^7$VIII |
| $R^3$III | $R^a$I | $R^b$I | $R^7$IX |
| $R^3$III | $R^a$I | $R^b$II | $R^7$I |
| $R^3$III | $R^a$I | $R^b$II | $R^7$I |
| $R^3$III | $R^a$I | $R^b$II | $R^7$III |
| $R^3$III | $R^a$I | $R^b$II | $R^7$IV |
| $R^3$III | $R^a$I | $R^b$II | $R^7$V |
| $R^3$III | $R^a$I | $R^b$II | $R^7$VI |
| $R^3$III | $R^a$I | $R^b$II | $R^7$VII |
| $R^3$III | $R^a$I | $R^b$II | $R^7$VIII |
| $R^3$III | $R^a$I | $R^b$II | $R^7$IX |
| $R^3$III | $R^a$I | $R^b$III | $R^7$I |
| $R^3$III | $R^a$I | $R^b$III | $R^7$II |
| $R^3$III | $R^a$I | $R^b$III | $R^7$III |
| $R^3$III | $R^a$I | $R^b$ III | $R^7$IV |
| $R^3$III | $R^a$I | $R^b$III | $R^7$V |
| $R^3$III | $R^a$I | $R^b$III | $R^7$VI |
| $R^3$III | $R^a$I | $R^b$III | $R^7$VII |
| $R^3$III | $R^a$I | $R^b$III | $R^7$VIII |
| $R^3$III | $R^a$I | $R^b$III | $R^7$IX |
| $R^3$III | $R^a$I | $R^b$IV | -- |
| $R^3$III | $R^a$I | $R^b$V | -- |

(continued)

| $R^3$ | $R^a$ | $R^b$ | $R^7$ |
|---|---|---|---|
| $R^3$III | $R^a$I | $R^b$VI | -- |
| $R^3$III | $R^a$II | $R^b$I | $R^7$I |
| $R^3$III | $R^a$II | $R^b$I | $R^7$II |
| $R^3$III | $R^a$II | $R^b$I | $R^7$III |
| $R^3$III | $R^a$II | $R^b$I | $R^7$IV |
| $R^3$III | $R^a$II | $R^b$I | $R^7$V |
| $R^3$III | $R^a$II | $R^b$I | $R^7$VI |
| $R^3$III | $R^a$II | $R^b$I | $R^7$VII |
| $R^3$III | $R^a$II | $R^b$I | $R^7$VIII |
| $R^3$III | $R^a$II | $R^b$I | $R^7$IX |
| $R^3$III | $R^a$II | $R^b$II | $R^7$I |
| $R^3$III | $R^a$II | $R^b$II | $R^7$II |
| $R^3$III | $R^a$II | $R^b$II | $R^7$III |
| $R^3$III | $R^a$II | $R^b$II | $R^7$IV |
| $R^3$III | $R^a$II | $R^b$II | $R^7$V |
| $R^3$III | $R^a$II | $R^b$II | $R^7$VI |
| $R^3$III | $R^a$II | $R^b$II | $R^7$VII |
| $R^3$III | $R^a$II | $R^b$II | $R^7$VIII |
| $R^3$III | $R^a$II | $R^b$II | $R^7$IX |
| $R^3$III | $R^a$II | $R^b$III | $R^7$I |
| $R^3$III | $R^a$II | $R^b$III | $R^7$II |
| $R^3$III | $R^a$II | $R^b$III | $R^7$III |
| $R^3$III | $R^a$II | $R^b$III | $R^7$IV |
| $R^3$III | $R^a$II | $R^b$III | $R^7$V |
| $R^3$III | $R^a$II | $R^b$III | $R^7$VI |
| $R^3$III | $R^a$II | $R^b$III | $R^7$VII |
| $R^3$III | $R^a$II | $R^b$III | $R^7$VIII |
| $R^3$III | $R^a$II | $R^b$III | $R^7$IX |
| $R^3$III | $R^a$II | $R^b$IV | -- |
| $R^3$III | $R^a$II | $R^b$V | -- |
| $R^3$III | $R^a$II | $R^b$VI | -- |
| $R^3$III | $R^a$III | $R^b$I | $R^7$I |
| $R^3$III | $R^a$III | $R^b$I | $R^7$II |
| $R^3$III | $R^a$III | $R^b$I | $R^7$III |
| $R^3$III | $R^a$III | $R^b$I | $R^7$IV |
| $R^3$III | $R^a$III | $R^b$I | $R^7$V |
| $R^3$III | $R^a$III | $R^b$I | $R^7$VI |
| $R^3$III | $R^a$III | $R^b$I | $R^7$VII |
| $R^3$III | $R^a$III | $R^b$I | $R^7$VIII |
| $R^3$III | $R^a$III | $R^b$I | $R^7$IX |
| $R^3$III | $R^a$III | $R^b$II | $R^7$I |
| $R^3$III | $R^a$III | $R^b$II | $R^7$II |
| $R^3$III | $R^a$III | $R^b$II | $R^7$III |
| $R^3$III | $R^a$III | $R^b$II | $R^7$IV |
| $R^3$III | $R^a$III | $R^b$II | $R^7$V |
| $R^3$III | $R^a$III | $R^b$II | $R^7$VI |
| $R^3$III | $R^a$III | $R^b$II | $R^7$VII |
| $R^3$III | $R^a$III | $R^b$II | $R^7$VIII |
| $R^3$III | $R^a$III | $R^b$II | $R^7$IX |

(continued)

| $R^3$ | $R^a$ | $R^b$ | $R^7$ |
|---|---|---|---|
| $R^3III$ | $R^aIII$ | $R^bIII$ | $R^7I$ |
| $R^3III$ | $R^aIII$ | $R^bIII$ | $R^7II$ |
| $R^3III$ | $R^aIII$ | $R^bIII$ | $R^7III$ |
| $R^3III$ | $R^aIII$ | $R^bIII$ | $R^7IV$ |
| $R^3III$ | $R^aIII$ | $R^bIII$ | $R^7V$ |
| $R^{3I}II$ | $R^aIII$ | $R^bIII$ | $R^7VI$ |
| $R^3III$ | $R^aIII$ | $R^bIII$ | $R^7VII$ |
| $R^3III$ | $R^aIII$ | $R^bIII$ | $R^7VIII$ |
| $R^3III$ | $R^aIII$ | $R^bIII$ | $R^7IX$ |
| $R^3III$ | $R^aIII$ | $R^bIV$ | -- |
| $R^3III$ | $R^aIII$ | $R^bV$ | -- |
| $R^3III$ | $R^aIII$ | $R^bVI$ | -- |
| $R^3III$ | $R^aIV$ | $R^bI$ | $R^7I$ |
| $R^3III$ | $R^aIV$ | $R^bI$ | R7II |
| $R^3III$ | $R^aIV$ | $R^bI$ | $R^7III$ |
| $R^3III$ | $R^aIV$ | $R^bI$ | $R^7IV$ |
| $R^3III$ | $R^aIV$ | $R^bI$ | $R^7V$ |
| $R^3III$ | $R^aIV$ | $R^bI$ | $R^7VI$ |
| $R^3III$ | $R^aIV$ | $R^bI$ | $R^7VII$ |
| $R^3III$ | $R^aIV$ | $R^bI$ | $R^7VIII$ |
| $R^3III$ | $R^aIV$ | $R^bI$ | $R^7IX$ |
| $R^3III$ | $R^aIV$ | $R^bII$ | $R^7I$ |
| $R^3III$ | $R^aIV$ | $R^bII$ | $R^7II$ |
| $R^3III$ | $R^aIV$ | $R^bII$ | $R^7IV$ |
| $R^3III$ | $R^aIV$ | $R^bII$ | $R^7V$ |
| $R^3III$ | $R^aIV$ | $R^bII$ | $R^7VI$ |
| $R^3III$ | $R^aIV$ | $R^bII$ | $R^7III$ |
| $R^3III$ | $R^aIV$ | $R^bII$ | $R^7VII$ |
| $R^3III$ | $R^aIV$ | $R^bII$ | $R^7VIII$ |
| $R^3III$ | $R^aIV$ | $R^bII$ | $R^7IX$ |
| $R^3III$ | $R^aIV$ | $R^bIII$ | $R^7I$ |
| $R^3III$ | $R^aIV$ | $R^bIII$ | $R^7II$ |
| $R^3III$ | $R^aIV$ | $R^bIII$ | $R^7III$ |
| $R^3III$ | $R^aIV$ | $R^b$ III | $R^7$ IV |
| $R^3III$ | $R^aIV$ | $R^bIII$ | $R^7V$ |
| $R^3III$ | $R^aIV$ | $R^bIII$ | $R^7VI$ |
| $R^3III$ | $R^aIV$ | $R^bIII$ | $R^7VII$ |
| $R^3III$ | $R^aIV$ | $R^bIII$ | $R^7VIII$ |
| $R^3III$ | $R^aIV$ | $R^bIII$ | $R^7IX$ |
| $R^3III$ | $R^aIV$ | $R^bIV$ | -- |
| $R^3III$ | $R^aIV$ | $R^bV$ | -- |
| $R^3III$ | $R^aIV$ | $R^bVI$ | -- |
| $R^3III$ | $R^aV$ | $R^bI$ | $R^7I$ |
| $R^3III$ | $R^aV$ | $R^bI$ | R7II |
| $R^3III$ | $R^aV$ | $R^bI$ | $R^7III$ |
| $R^3III$ | $R^aV$ | $R^bI$ | $R^7IV$ |
| $R^3III$ | $R^aV$ | $R^bI$ | $R^7V$ |
| $R^3III$ | $R^aV$ | $R^bI$ | $R^7VI$ |
| $R^3III$ | $R^aV$ | $R^bI$ | $R^7VII$ |

(continued)

| $R^3$ | $R^a$ | $R^b$ | $R^7$ |
|---|---|---|---|
| $R^3$III | $R^a$V | $R^b$I | $R^7$VIII |
| $R^3$III | $R^a$V | $R^b$I | $R^7$IX |
| $R^3$III | $R^a$V | $R^b$II | $R^7$I |
| $R^3$III | $R^a$V | $R^b$II | $R^7$II |
| $R^3$III | $R^a$V | $R^b$II | $R^7$III |
| $R^3$III | $R^a$V | $R^b$II | $R^7$IV |
| $R^3$III | $R^a$V | $R^b$II | $R^7$V |
| $R^3$III | $R^a$V | $R^b$II | $R^7$VI |
| $R^3$III | $R^a$V | $R^b$II | $R^7$VII |
| $R^3$III | $R^a$V | $R^b$ II | $R^7$VIII |
| $R^3$III | $R^a$V | $R^b$ II | $R^7$IX |
| $R^3$III | $R^a$V | $R^b$III | $R^7$I |
| $R^3$III | $R^a$V | $R^b$III | $R^7$II |
| $R^3$III | $R^a$V | $R^b$III | $R^7$III |
| $R^3$III | $R^a$V | $R^b$III | $R^7$IV |
| $R^3$III | $R^a$V | $R^b$III | $R^7$V |
| $R^3$III | $R^a$V | $R^b$III | $R^7$VI |
| $R^3$III | $R^a$V | $R^b$III | $R^7$VII |
| $R^3$III | $R^a$V | $R^b$III | $R^7$VIII |
| $R^3$III | $R^a$V | $R^b$III | $R^7$IX |
| $R^3$III | $R^a$V | $R^b$IV | -- |
| $R^3$III | $R^a$V | $R^b$V | -- |
| $R^3$III | $R^a$V | $R^b$VI | -- |
| $R^3$III | $R^a$VI | $R^b$I | $R^7$I |
| $R^3$III | $R^a$VI | $R^b$I | $R^7$II |
| $R^3$III | $R^a$VI | $R^b$I | $R^7$III |
| $R^3$III | $R^a$VI | $R^b$I | $R^7$IV |
| $R^3$III | $R^a$VI | $R^b$I | $R^7$V |
| $R^3$III | $R^a$VI | $R^b$I | $R^7$VI |
| $R^3$III | $R^a$VI | $R^b$I | $R^7$VII |
| $R^3$III | $R^a$VI | $R^b$I | $R^7$VIII |
| $R^3$III | $R^a$VI | $R^b$I | $R^7$IX |
| $R^3$III | $R^a$VI | $R^b$II | $R^7$I |
| $R^3$III | $R^a$VI | $R^b$II | $R^7$II |
| $R^3$III | $R^a$VI | $R^b$II | $R^7$III |
| $R^3$III | $R^a$VI | $R^b$II | $R^7$IV |
| $R^3$III | $R^a$VI | $R^b$II | $R^7$V |
| $R^3$III | $R^a$VI | $R^b$II | $R^7$VI |
| $R^3$III | $R^a$VI | $R^b$II | $R^7$VII |
| $R^3$III | $R^a$VI | $R^b$II | $R^7$VIII |
| $R^3$III | $R^a$VI | $R^b$II | $R^7$IX |
| $R^3$III | $R^a$VI | $R^b$III | $R^7$I |
| $R^3$III | $R^a$VI | $R^b$III | $R^7$II |
| $R^3$III | $R^a$VI | $R^b$III | $R^7$III |
| $R^3$III | $R^a$VI | $R^b$III | $R^7$IV |
| $R^3$III | $R^a$VI | $R^b$III | $R^7$V |
| $R^3$III | $R^a$VI | $R^b$III | $R^7$VI |
| $R^3$III | $R^a$VI | $R^b$III | $R^7$VII |
| $R^3$III | $R^a$VI | $R^b$III | $R^7$VIII |

(continued)

| $R^3$ | $R^a$ | $R^b$ | $R^7$ |
|---|---|---|---|
| $R^3$III | $R^a$VI | $R^b$III | $R^7$IX |
| $R^3$III | $R^a$VI | $R^b$IV | -- |
| $R^3$III | $R^a$VI | $R^b$V | -- |
| $R^3$III | $R^a$VI | $R^b$VI | -- |
| $R^3$III | $R^a$VII | $R^b$I | $R^7$I |
| $R^3$III | $R^a$VII | $R^b$I | $R^7$II |
| $R^3$III | $R^a$VII | $R^b$I | $R^7$III |
| $R^3$III | $R^a$VII | $R^b$I | $R^7$IV |
| $R^3$III | $R^a$VII | $R^b$I | $R^7$V |
| $R^3$III | $R^a$VII | $R^b$I | $R^7$VI |
| $R^3$III | $R^a$VII | $R^b$I | $R^7$VII |
| $R^3$III | $R^a$VII | $R^b$I | $R^7$VIII |
| $R^3$III | $R^a$VII | $R^b$I | $R^7$IX |
| $R^3$III | $R^a$VII | $R^b$II | $R^7$I |
| $R^3$III | $R^a$VII | $R^b$II | $R^7$II |
| $R^3$III | $R^a$VII | $R^b$II | $R^7$III |
| $R^3$III | $R^a$VII | $R^b$II | $R^7$IV |
| $R^3$III | $R^a$VII | $R^b$II | $R^7$V |
| $R^3$III | $R^a$VII | $R^b$II | $R^7$VI |
| $R^3$III | $R^a$VII | $R^b$II | $R^7$VII |
| $R^3$III | $R^a$VII | $R^b$II | $R^7$VIII |
| $R^3$III | $R^a$VII | $R^b$II | $R^7$IX |
| $R^3$III | $R^a$VII | $R^b$III | $R^7$I |
| $R^3$III | $R^a$VII | $R^b$III | $R^7$II |
| $R^3$III | $R^a$VII | $R^b$III | $R^7$III |
| $R^3$III | $R^a$VII | $R^b$III | $R^7$IV |
| $R^3$III | $R^a$VII | $R^b$III | $R^7$V |
| $R^3$III | $R^a$VII | $R^b$III | $R^7$VI |
| $R^3$III | $R^a$VII | $R^b$III | $R^7$VII |
| $R^3$III | $R^a$VII | $R^b$III | $R^7$VIII |
| $R^3$III | $R^a$VII | $R^b$III | $R^7$IX |
| $R^3$III | $R^a$VII | $R^b$IV | -- |
| $R^3$III | $R^a$VII | $R^b$V | -- |
| $R^3$III | $R^a$VII | $R^b$VI | -- |
| $R^3$III | $R^a$VIII | $R^b$I | $R^7$I |
| $R^3$III | $R^a$VIII | $R^b$I | $R^7$II |
| $R^3$III | $R^a$VIII | $R^b$I | $R^7$III |
| $R^3$III | $R^a$VIII | $R^b$I | $R^7$IV |
| $R^3$III | $R^a$VIII | $R^b$I | $R^7$V |
| $R^3$III | $R^a$VIII | $R^b$I | $R^7$VI |
| $R^3$III | $R^a$VIII | $R^b$I | $R^7$VII |
| $R^3$III | $R^a$VIII | $R^b$I | $R^7$VIII |
| $R^3$III | $R^a$VIII | $R^b$I | $R^7$IX |
| $R^3$III | $R^a$VIII | $R^b$II | $R^7$I |
| $R^3$III | $R^a$VIII | $R^b$II | $R^7$II |
| $R^3$III | $R^a$VIII | $R^b$II | $R^7$III |
| $R^3$III | $R^a$VIII | $R^b$II | $R^7$IV |
| $R^3$III | $R^a$VIII | $R^b$II | $R^7$V |
| $R^3$III | $R^a$VIII | $R^b$II | $R^7$VI |

(continued)

| $R^3$ | $R^a$ | $R^b$ | $R^7$ |
|---|---|---|---|
| $R^3$III | $R^a$VIII | $R^b$II | $R^7$VII |
| $R^3$III | $R^a$VIII | $R^b$II | $R^7$VIII |
| $R^3$III | $R^a$III | $R^b$II | $R^7$IX |
| $R^3$III | $R^a$VIII | $R^b$III | $R^7$I |
| $R^3$III | $R^a$VIII | $R^b$III | $R^7$II |
| $R^3$III | $R^a$VIII | $R^b$III | $R^7$III |
| $R^3$III | $R^a$VIII | $R^b$III | $R^7$IV |
| $R^3$III | $R^a$VIII | $R^b$III | $R^7$V |
| $R^3$III | $R^a$VIII | $R^b$III | $R^7$VI |
| $R^3$III | $R^a$VIII | $R^b$III | $R^7$VII |
| $R^3$III | $R^a$VIII | $R^b$III | $R^7$VIII |
| $R^3$III | $R^a$VIII | $R^b$III | $R^7$IX |
| $R^3$III | $R^a$VIII | $R^b$IV | -- |
| $R^3$III | $R^a$VIII | $R^b$V | -- |
| $R^3$III | $R^a$VIII | $R^b$VI | -- |
| $R^3$III | $R^a$IX | $R^b$I | $R^7$I |
| $R^3$III | $R^a$IX | $R^b$I | $R^7$II |
| $R^3$III | $R^a$IX | $R^b$I | $R^7$III |
| $R^3$III | $R^a$IX | $R^b$I | $R^7$IV |
| $R^3$III | $R^a$IX | $R^b$I | $R^7$V |
| $R^3$III | $R^a$IX | $R^b$I | $R^7$VI |
| $R^3$III | $R^a$IX | $R^b$I | $R^7$VII |
| $R^3$III | $R^a$IX | $R^b$I | $R^7$VIII |
| $R^3$III | $R^a$IX | $R^b$I | $R^7$IX |
| $R^3$III | $R^a$IX | $R^b$II | $R^7$I |
| $R^3$III | $R^a$IX | $R^b$II | $R^7$II |
| $R^3$III | $R^a$IX | $R^b$II | $R^7$III |
| $R^3$III | $R^a$IX | $R^b$II | $R^7$IV |
| $R^3$III | $R^a$IX | $R^b$II | $R^7$V |
| $R^3$III | $R^a$IX | $R^b$II | $R^7$VI |
| $R^3$III | $R^a$IX | $R^b$II | $R^7$VII |
| $R^3$III | $R^a$IX | $R^b$II | $R^7$VIII |
| $R^3$III | $R^a$IX | $R^b$II | $R^7$IX |
| $R^3$III | $R^a$IX | $R^b$III | $R^7$I |
| $R^3$III | $R^a$IX | $R^b$III | $R^7$II |
| $R^3$III | $R^a$IX | $R^b$III | $R^7$III |
| $R^3$III | $R^a$IX | $R^b$III | $R^7$IV |
| $R^3$III | $R^a$IX | $R^b$III | $R^{7V}$ |
| $R^3$III | $R^a$IX | $R^b$III | $R^7$VI |
| $R^3$III | $R^a$IX | $R^b$III | $R^7$VII |
| $R^3$III | $R^a$IX | $R^b$III | $R^7$VIII |
| $R^3$III | $R^a$IX | $R^b$III | $R^7$IX |
| $R^3$III | $R^a$IX | $R^b$IV | -- |
| $R^3$III | $R^a$IX | $R^b$V | -- |
| $R^3$III | $R^a$IX | $R^b$VI | -- |
| $R^3$III | $R^a$X | $R^b$I | $R^7$I |
| $R^3$III | $R^a$X | $R^b$I | $R^7$II |
| $R^3$III | $R^a$X | $R^b$I | $R^7$III |
| $R^3$III | $R^a$X | $R^b$I | $R^7$IV |

(continued)

| $R^3$ | $R^a$ | $R^b$ | $R^7$ |
|---|---|---|---|
| $R^3$III | $R^a$X | $R^b$I | $R^7$V |
| $R^3$III | $R^a$X | $R^b$I | $R^7$VI |
| $R^3$III | $R^a$X | $R^b$I | $R^7$VII |
| $R^3$III | $R^a$X | $R^b$I | $R^7$VIII |
| $R^3$III | $R^a$X | $R^b$I | $R^7$IX |
| $R^3$III | $R^a$X | $R^b$II | $R^7$I |
| $R^3$III | $R^a$X | $R^b$II | $R^7$II |
| $R^3$III | $R^a$X | $R^b$II | $R^7$III |
| $R^3$III | $R^a$X | $R^b$II | $R^7$IV |
| $R^3$III | $R^a$X | $R^b$II | $R^7$V |
| $R^3$III | $R^a$X | $R^b$II | $R^7$VI |
| $R^3$III | $R^a$X | $R^b$II | $R^7$VI |
| $R^3$III | $R^a$X | $R^b$II | $R^7$VIII |
| $R^3$III | $R^a$X | $R^b$II | $R^7$IX |
| $R^3$III | $R^a$X | $R^b$III | $R^7$I |
| $R^3$III | $R^a$X | $R^b$III | $R^7$II |
| $R^3$III | $R^a$X | $R^b$III | $R^7$III |
| $R^3$III | $R^a$X | $R^b$III | $R^7$IV |
| $R^3$III | $R^a$X | $R^b$III | $R^7$V |
| $R^3$III | $R^a$X | $R^b$III | $R^7$VI |
| $R^3$III | $R^a$X | $R^b$III | $R^7$VII |
| $R^3$III | $R^a$X | $R^b$III | $R^7$VIII |
| $R^3$III | $R^a$X | $R^b$III | $R^7$IX |
| $R^3$III | $R^a$X | $R^b$IV | -- |
| $R^3$III | $R^a$X | $R^b$V | -- |
| $R^3$III | $R^a$X | $R^b$VI | -- |
| $R^3$I | -- | $R^b$VII | -- |
| $R^3$II | -- | $R^b$VII | -- |
| $R^3$III | -- | $R^b$VII | -- |

The compounds of the present invention can be produced, for example, by the following processes.

## Process A

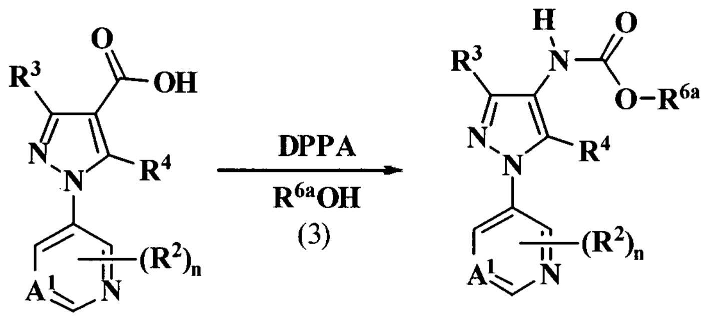


(2) (4)

A compound represented by the formula (2) [wherein $A^1$, $R^2$, $R^3$, $R^4$ and n are the same as defined above] is reacted with diphenylphosphoryl azide (DPPA) and a compound represented by the formula (3) [wherein $R^{6a}$ is the same

as defined above] to obtain a compound of the present invention represented by the formula (4) [wherein $A^1$, $R^2$, $R^3$, $R^4$, $R^{6a}$ and n are the same as defined above].

In the reaction, DPPA can be used in an amount of from 0.5 to 50 equivalents per 1 equivalent of the compound represented by the formula (2), and the compound represented by the formula (3) can be used in an amount of 1 equivalent to an amount sufficient as a solvent per 1 equivalent of the compound represented by the formula (2). If necessary, a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon such as pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (3) are known compounds, and some of them are commercially available. The rest of them can be readily synthesized from known compounds by known methods disclosed in the literature.

Process B

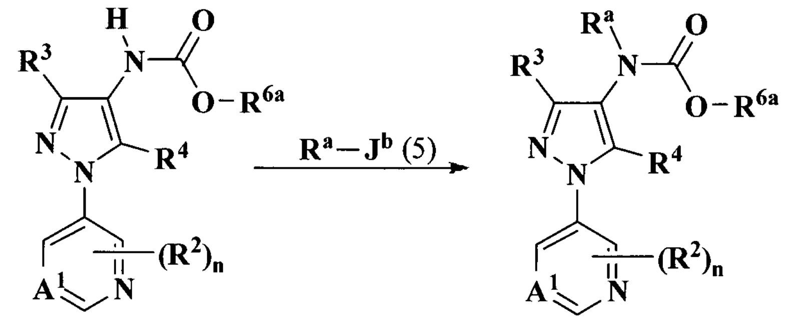


(4) (6)

A compound represented by the formula (4) obtainable by Process A is reacted with a compound represented by the formula (5) [wherein $R^a$ is the same as defined above, $J^b$ is a leaving group such as a halogen atom, -OH, $-OSO_2Me$ or $-OSO_2CF_3$] to obtain a compound of the present invention represented by the formula (6) [wherein $A^1$, $R^2$, $R^3$, $R^4$, $R^{6a}$, $R^a$ and n are the same as defined above].

In the reaction, the compound represented by the formula (5) is used in an amount of from 0.5 to 50 equivalents per 1 equivalent of the compound represented by the formula (4). If necessary, an acid such as hydrochloric acid, sulfuric acid or p-toluenesulfonic acid or a base such as potassium carbonate, triethylamine, pyridine, 4-(dimethylamino)pyridine, sodium hydride, sodium hydroxide or potassium hydroxide or a Mitsunobu reaction using diethylazodicarboxylate, triphenylphosphine and the like may be used.

The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (5) are known compounds, and some of them are commercially available. The rest of them can be readily synthesized from known compounds by known methods disclosed in the literature.

Process C

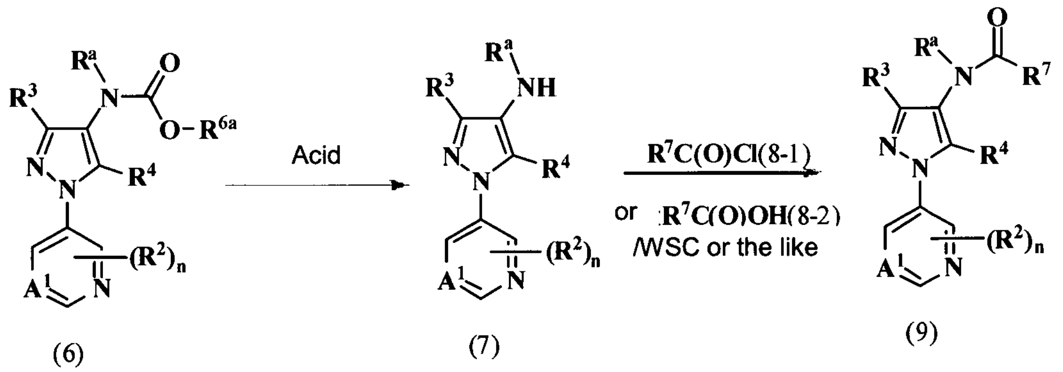


(6) (7) (9)

A compound represented by the formula (6) obtainable by Process B is reacted with an acid to obtain a compound represented by the formula (7) [wherein $A^1$, $R^2$, $R^3$, $R^4$, $R^a$ and n are the same as defined above].

As the acid, hydrochloric acid, sulfuric acid, p-toluenesulfonic acid, trifluoroacetic acid or the like may be used.

The reaction may be carried out without a solvent or may be carried out in a solvent such as a polar solvent like acetonitrile or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

The compound represented by the formula (7) thus obtained is reacted with a compound represented by the formula (8-1) [wherein $R^7$ is the same as defined above] to obtain a compound of the present invention represented by the formula (9) [wherein $A^1$, $R^2$, $R^3$, $R^4$, $R^7$, $R^a$ and n are the same as defined above]. In the reaction, the compound represented by the formula(8-1) is used in an amount of from 0.5 to 50 equivalents per 1 equivalent of the compound represented by the formula (7). If necessary, a base such as potassium carbonate, triethylamine, pyridine or 4- (dimethylamino)pyridine may be used. The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (8-1) are known compounds, and some of them are commercially available. The rest of them can be readily synthesized from known compounds by known methods disclosed in the literature.

A compound represented by the formula (7) may also be reacted with a compound represented by the formula (8-2) [wherein $R^7$ is the same as defined above] in the presence of a condensation agent to obtain a compound of the present invention represented by the formula (9). In the reaction, a condensation agent such as WSC {1-ethyl-3-(3-dimethylaminopropyl)carbodiimide hydrochloride} or CDI (carbonyldiimidazole) is used in an amount of from 1 to 4 equivalents per 1 equivalent of the compound represented by the formula (8-2). The compound represented by the formula (8-2) is used in an amount of from 0.5 to 50 equivalents per 1 equivalent of the compound (7). If necessary, a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of

the reaction mixture, and the reaction time may be set arbitrarily within the range of from 5 minutes to 100 hours, though it depends on the concentrations of the reactants and the reaction temperature.

Some of the compounds represented by the formula (8-2) are known compounds, and some of them are commercially available. The rest of them can be readily synthesized from known compounds by known methods disclosed in the literature.

Process D

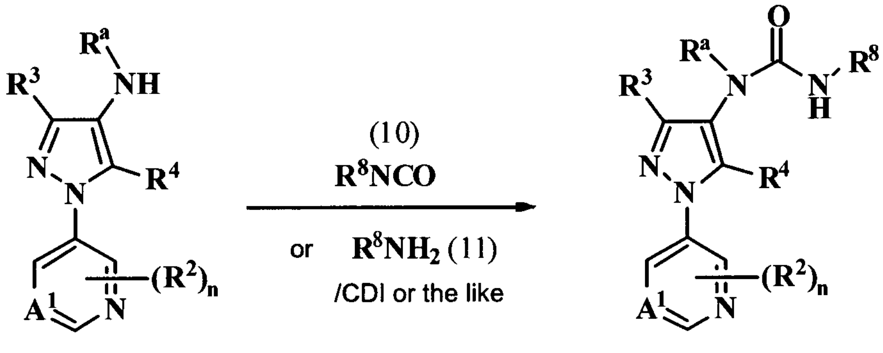


(7) (12)

A compound represented by the formula (7) obtainable by Process C is reacted with a compound represented by the formula (10) [wherein $R^8$ is the same as defined above] to obtain a compound of the present invention represented by the formula (12) [wherein $A^1$, $R^2$, $R^3$, $R^4$, $R^8$, $R^a$ and n are the same as defined above]. In the reaction, the compound represented by the formula(1 0) is used in an amount of from 0.5 to 50 equivalents per 1 equivalent of the compound represented by the formula (7). If necessary, a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (5) are known compounds, and some of them are commercially available. The rest of them can be readily synthesized from known compounds by known methods disclosed in the literature.

A compound represented by the formula (7) may also be reacted with a compound represented by the formula (11) [wherein $R^8$ is the same as defined above] to obtain a compound represented by the formula (12).

In the reaction, CD I(carbonyldiimidazole), bis(trichloromethyl) carbonate or the like is used in an amount of from 1 to 4 equivalents per 1 equivalent of the compound represented by the formula (11). The compound represented by the formula (11) is used in an amount of from 0.5 to 50 equivalents per 1 equivalent of the compound represented by the formula (7). If necessary, a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (11) are known compounds, and some of them are commercially available. The rest of them can be readily synthesized from known compounds by known methods disclosed in the literature

in the literature.

Process E

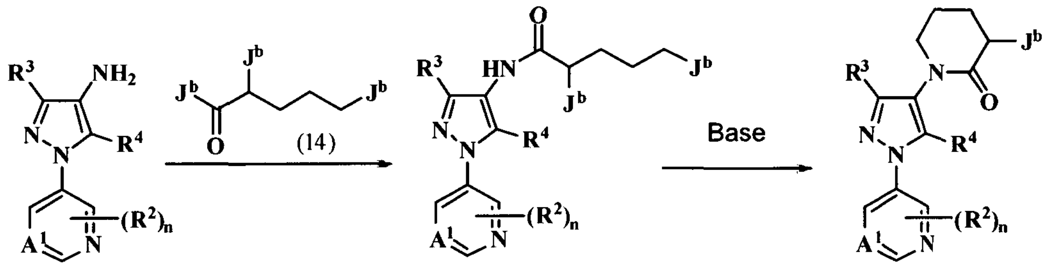


(13) (15) (16)

A compound represented by the formula (13) [wherein $A^1$, $R^2$, $R^3$, $R^4$ and n are the same as defined above] is reacted with a compound represented by the formula (14) [wherein $J^b$ is the same as defined above] in the same manner as in Process C to obtain a compound of the present invention represented by the formula (15) [wherein $A^1$, $R^2$, $R^3$, $R^4$, $J^b$ and n are the same as defined above].

The compounds represented by the formula (13) can be produced by a known method disclosed, for example, in Farmaco, Edizione Scientifica,(1970),25(8),592. Some of the compounds represented by the formula (14) [wherein $J^b$ is the same as defined above]are commercially available. The rest of them can be readily synthesized from known compounds by known methods disclosed in the literature such as Tetrahetron: Asymmetry, 2003,vol. 14, pp. 2587.

The compound represented by the formula (15) is reacted with a base such as potassium carbonate, triethylamine, pyridine or 4-(dimethylamino)pyridine to obtain a compound represented by the formula (16) [wherein $A^1$, $R^2$, $R^3$, $R^4$, $J^b$ and n are the same as defined above]. The base is used in an amount of from 0.5 to 50 equivalents per 1 equivalent of the compound represented by the formula (15).

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Process F

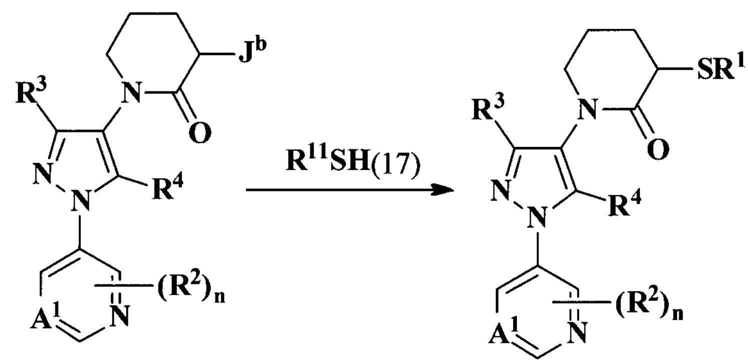


(16) (18)

A compound represented by the formula (16) obtainable by Process E is reacted with a compound represented by the formula (17) [wherein $R^{11}$ is the same as defined above] to obtain a compound of the present invention represented by the formula (18) [wherein $A^1$, $R^2$, $R^3$, $R^4$, $R^{11}$ and n are the same as defined above]. In the reaction, the compound represented by the formula (17) is used in an amount of from 0.5 to 50 equivalent s per 1 equivalent of the compound represented by the formula (16). If necessary, an acid such as hydrochloric acid, sulfuric acid or p-toluenesulfonic acid

or a base such as potassium carbonate, triethylamine, pyridine, 4-(dimethylamino)pyridine, sodium hydride, sodium hydroxide or potassium hydroxide may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (17) are known compounds, and some of them are commercially available. The rest of them can be readily synthesized from known compounds by known methods disclosed in the literature.

Process G

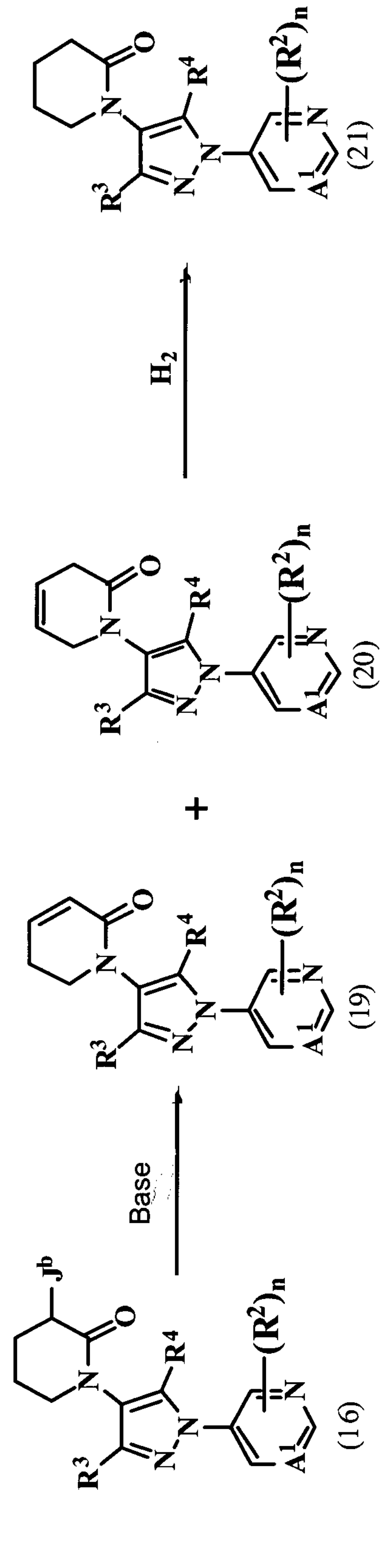


(16) (19) (20) (21)

A compound represented by the formula (16) obtainable by Process E is reacted with a base to obtain a compound of the present invention represented by the formula (19) [wherein $A^1$, $R^2$, $R^3$, $R^4$ and n are the same as defined above] and a compound of the present invention represented by the formula (20) [wherein $A^1$, $R^2$, $R^3$, $R^4$ and n are the same

as defined above].

As the base, potassium carbonate, triethylamine, pyridine, 4-(dimethylamino)pyridine, 1,8-diazabicyclo[5.4.0]-7-undecene, sodium hydroxide, potassium hydroxide or the like may be used.

The reaction may be carried out without a solvent or may be carried out in a solvent such as a polar solvent like acetonitrile or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Then, the compound represented by the formula (19) or the compound represented by the formula (20) is hydrogenated in the presence of a catalyst to obtain a compound of the present invention represented by the formula (21) [wherein $A^1$, $R^2$, $R^3$, $R^4$ and n are the same as defined above]. As the catalyst, a palladium-activated carbon catalyst, a platinum-activated carbon catalyst, Lindlar's catalyst or the like may be used.

Process H

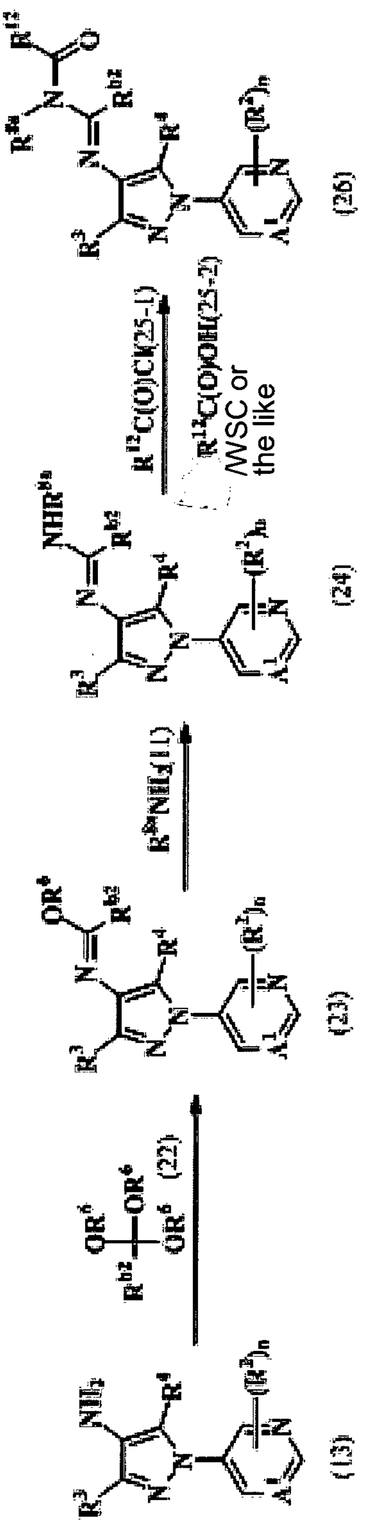


A compound represented by the formula (13) is reacted with a compound represented by the formula (22)

[wherein $R^{b2}$ and $R^6$ are the same as defined above] to obtain a compound of the present invention represented by the formula (23) [wherein $A^1$, $R^2$, $R^3$, $R^4$, $R^{b2}$, $R^6$ and n are the same as defined above]. The compound represented by the formula (22) is used in an amount of from 0.5 equivalent to an amount sufficient as a solvent, per 1 equivalent of the compound represented by the formula (13). If necessary, an acid such as hydrochloric acid, sulfuric acid or p-toluenesulfonic acid may be used.

The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (22) are known compounds, and some of them are commercially available. The rest of them can be readily synthesized from known compounds by known methods disclosed in the literature.

The compound represented by the formula (23) is then reacted with a compound represented by the formula (11) to obtain a compound of the present invention represented by the formula (24) [wherein $A^1$, $R^2$, $R^3$, $R^4$, $R^{8a}$, $R^{b2}$ and n are the same as defined above]. The compound represented by the formula (11) is used in an amount of from 0.5 equivalent to 50 equivalents per 1 equivalent of the compound represented by the formula (23). If necessary, an acid such as hydrochloric acid, sulfuric acid or p-toluenesulfonic acid or a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

The compound of the present invention represented by the formula (24) thus obtained is reacted with a compound represented by the formula (25-1) [wherein $R^{12}$ is the same as defined above] or a compound represented by the formula (25-2) [wherein $R^{12}$ is the same as defined above] in the same manner as in Process C to obtain a compound of the present invention represented by the formula (26) [wherein $A^1$, $R^2$, $R^3$, $R^4$, $R^{8a}$, $R^{12}$, $R^{b2}$ and n are the same as defined above].

Some of the compounds represented by the formulae (25-1) and (25-2) are known compounds, and some of them are commercially available. The rest of them can be readily synthesized from known compounds by known methods disclosed in the literature.

Process I

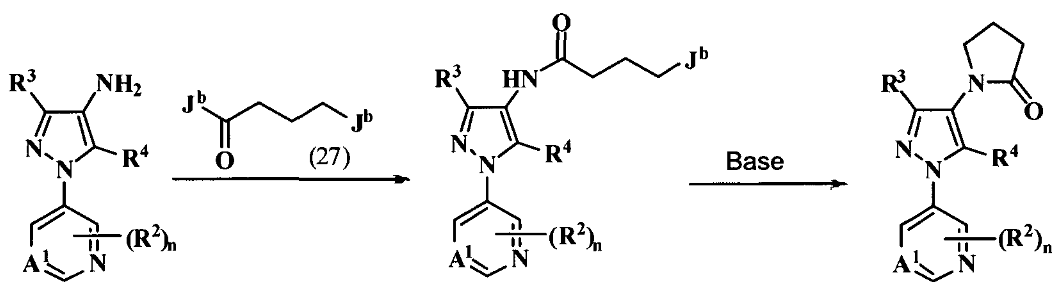


(13) (28) (29)

A compound represented by the formula (13) is reacted with a compound represented by the formula (27) [wherein $J^b$ is the same as defined above] in the same manner as in Process to obtain a compound of the present

invention represented by the formula (28) [wherein $A^1$, $R^2$, $R^3$, $R^4$, $J^b$ and n are the same as defined above].

Some of the compounds represented by the formula (27) are known compounds, and some of them are commercially available. The rest of them can be readily synthesized from known compounds by known methods disclosed in the literature.

The compound represented by the formula (28) is reacted with a base such as sodium hydride, potassium carbonate, triethylamine, pyridine or 4-(dimethylamino)pyridine in the same manner as in Process E to obtain a compound of the present invention represented by the formula (29) [wherein $A^1$, $R^2$, $R^3$, $R^4$ and n are the same as defined above].

## Process J

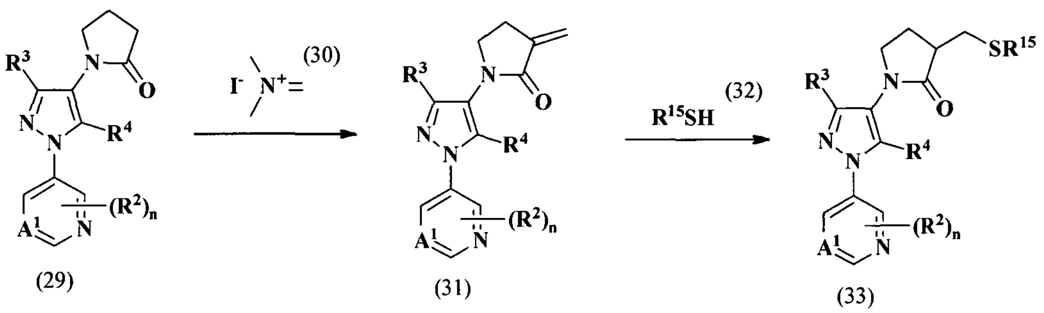


(29) (31) (33)

A compound represented by the formula (29) is reacted with dimethylmethyleneammonium iodide (Eschenmoser's reagent) to obtain a compound of the present invention represented by the formula (31) [wherein $A^1$, $R^2$, $R^3$, $R^4$ and n are the same as defined above].

Dimethylmethyleneammonium iodide is used in an amount of from 0.5 equivalent to 50 equivalents per 1 equivalent of the compound represented by the formula (29). If necessary, an acid such as trimethylsilyl trifluoromethanesulfonate or a base such as lithium bis(trimethylsilyl)amide may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

The compound of the present invention represented by the formula (31) thus obtained is reacted with a compound represented by the formula (32) [wherein $R^{15}$ is the same as defined above] in the same manner as in Process F to obtain a compound of the present invention represented by the formula (33) [wherein $A^1$, $R^2$, $R^3$, $R^4$, $R^{15}$ and n are the same as defined above].

Some of the compounds represented by the formula (17) are known compounds, and some of them are commercially available. The rest of them can be readily synthesized from known compounds by known methods disclosed in the literature.

Process K

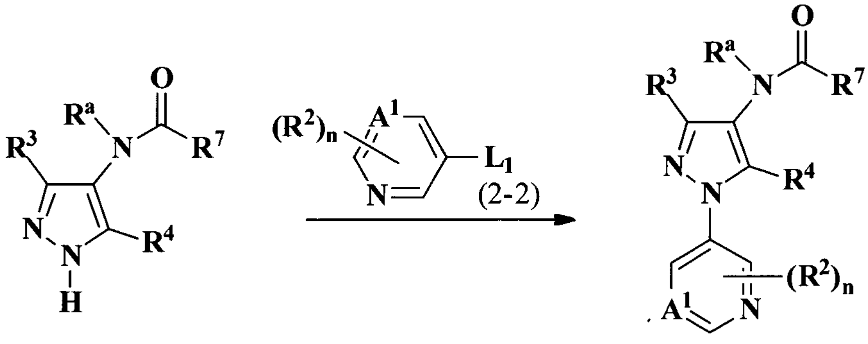

(2-2)

(2-12) (9)

A compound represented by the formula (2-12) [wherein $R^3$, $R^4$, $R^7$ and $R^a$ are the same as defined above] is reacted with a compound represented by the formula (2-2) [wherein $A^1$, $R^2$ and n are the same as defined above, and $L_1$ is a fluorine atom, a chlorine atom, a bromine atom, an iodine atom, a $-B(OH)_2$ group or a $-B(OR^{51})_2$ group (wherein $R^{51}$ is a hydrogen atom or identical or different $C_1$-$C_6$ alkyl, or two $R^{51}$'s may form $-CH_2CH_2-$ or $-C(CH_3)_2C(CH_3)_2-$ together with each other)] in the presence of a catalyst and a base to obtain a compound of the present invention represented by the formula (9).

The compound represented by the formula (2-2) is used in an amount of from 0.8 to 5 equivalent s per 1 equivalent of the compound represented by the formula (2-12).

Some of the compounds represented by the formula (2-2) used herein are known compounds, and some of them are commercially available. The rest of them can be readily synthesized from known compounds by known methods disclosed in the literature.

In the reaction, as the catalyst, for example, a palladium catalyst such as palladium-carbon, palladium chloride, palladium acetate, bis(triphenylphosphine)palladium chloride or tetrakis(triphenylphosphine)palladium or a copper catalyst such as metal copper, copper (I) acetate, copper (II) acetate, copper (I) oxide, copper(II) oxide or copper iodide may be used. The catalyst may be used in an amount of from 0.001 to 1.0 equivalent, preferably from 0.01 to 0.5 equivalent, more preferably from 0.05 to 0.2 equivalent per 1 equivalent of the compound represented by the formula (2-12).

As the base, a tertiary amine compound such as pyridine, diisopropylethylamine or triethylamine, an inorganic base such as sodium hydroxide, potassium hydroxide, sodium carbonate, potassium carbonate, cesium carbonate or sodium hydrogen carbonate or the like may be used. The base is used in an amount of from 0.1 to 10.0 equivalents per 1 equivalent of the compound represented by the formula (2-12).

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Process L

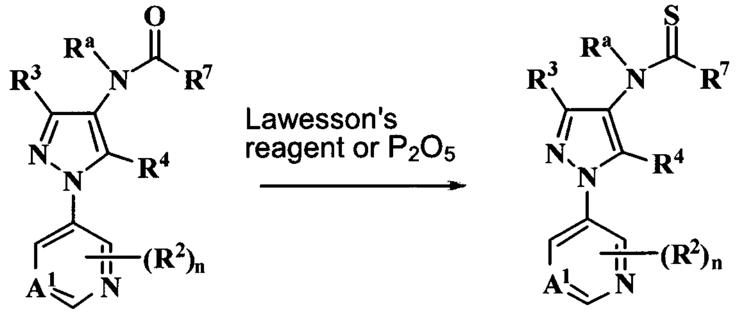


(9) (35)

A compound represented by the formula (9) and a sulfidizing agent such as phosphorus pentasulfide, phosphorus pentasulfide-HMDO (hexamethyldisiloxane) or Lawesson's Reagent (2,4-bis(4-methoxyphenyl)-1,3,2,4-dithiadiphosphetane-2,4-disulfide) are reacted to obtain a compound of the present invention represented by the formula (35) [wherein $A^1$, $R^2$, $R^3$, $R^4$, $R^7$, $R^a$ and n are the same as defined above].

In the reaction, the sulfidizing agent is used in an amount of from 0.5 to 50 equivalents per 1 equivalent of the compound represented by the formula (9).

If necessary, a base such as potassium carbonate, triethylamine, pyridine or 4-(dimethylamino)pyridine may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Process M

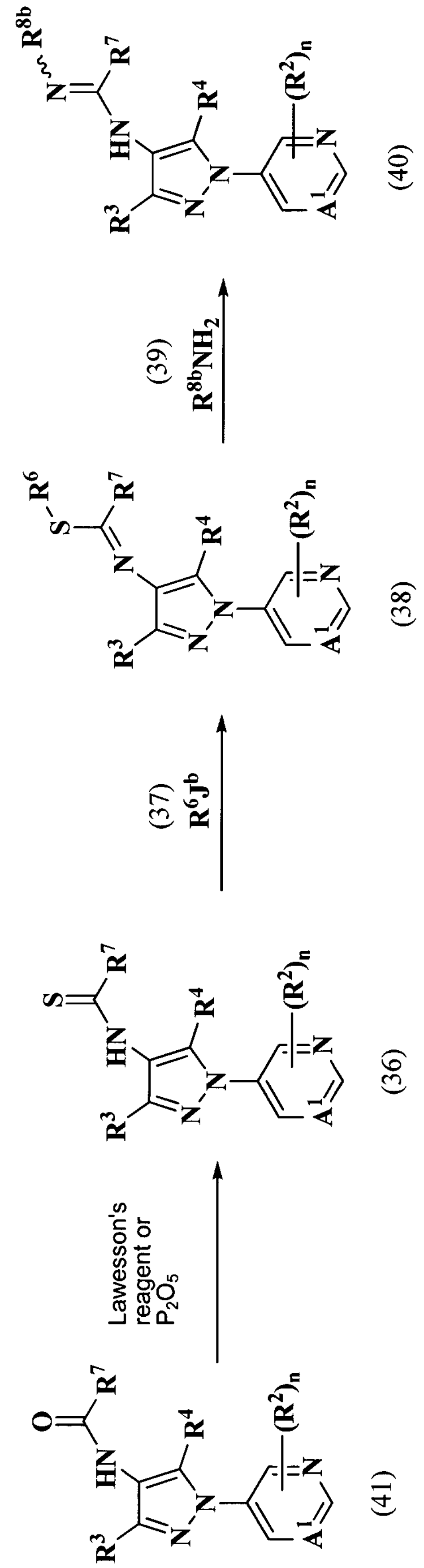


From a compound represented by the formula (41) [wherein $A^1$, $R^2$, $R^3$, $R^4$, $R^7$ and n are the same as defined

above], a compound of the present invention represented by the formula (36) [wherein $A^1$, $R^2$, $R^3$, $R^4$, $R^7$ and n are the same as defined above] can be obtained in the same manner as in Process L.

Then, the compound represented by the formula (36) is reacted with a compound represented by the formula (37) [wherein $R^6$ and $J^b$ are the same as defined above] to obtain a compound of the present invention represented by the formula (38) [wherein $A^1$, $R^2$, $R^3$, $R^4$, $R^6$, $R^7$ and n are the same as defined above].

In the reaction, the compound represented by the formula (37) is used in an amount of from 0.5 to 50 equivalents per 1 equivalent of the compound represented by the formula (36). If necessary, an acid such as hydrochloric acid, sulfuric acid or p-toluenesulfonic acid or a base such as potassium carbonate, triethylamine, pyridine, 4-(dimethylamino)pyridine, sodium hydride, sodium hydroxide or potassium hydroxide or a Mitsunobu reaction using diethylazodicarboxylate, triphenylphosphine and the like may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (37) are known compounds, and some of them are commercially available. The rest of them can be readily synthesized from known compounds by known methods disclosed in the literature.

Then, the compound represented by the formula (38) is reacted with a compound represented by the formula (39) [wherein $R^{8b}$ is the same as defined above] to obtain a compound of the present invention represented by the formula (40) [wherein $A^1$, $R^2$, $R^3$, $R^4$, $R^7$, $R^{8b}$ and n are the same as defined above].

In the reaction, the compounds represented by the formula (39) is used in an amount of from 0.5 to 50 equivalents per 1 equivalent of the compounds represented by the formula (38). If necessary, an acid such as hydrochloric acid, sulfuric acid or p-toluenesulfonic acid or a base such as potassium carbonate, triethylamine, pyridine, 4-(dimethylamino)pyridine, sodium hydride, sodium hydroxide or potassium hydroxide may be used.

The reaction may be carried out without a solvent or may be carried out in a solvent such as a polar solvent like N,N-dimethylformamide, N,N-dimethylacetamide, acetonitrile, dimethyl sulfoxide, 1 ,3-dimethyl-2-imidazolinone or water, an alcohol like methanol, ethanol, propanol, 2-propanol or ethylene glycol, an ether like diethyl ether, tetrahydrofuran or diphenyl ether, an aromatic hydrocarbon like benzene, toluene or xylene, a halohydrocarbon like methylene chloride, chloroform or carbon tetrachloride, or an aliphatic hydrocarbon like pentane or n-hexane. These solvents may be used alone or in combinations of two or more.

The reaction temperature may be set arbitrarily within the range of from -60°C to the refluxing temperature of the reaction mixture, and the reaction time may be set arbitrarily within the range of from 5 minutes to 100 hours, though it depends on the concentrations of the reactants and the reaction temperature.

Some of the compounds represented by the formula (39) are known compounds, and some of them are commercially available. The rest of them can be readily synthesized from known compounds by known methods disclosed in the literature.

## Process N

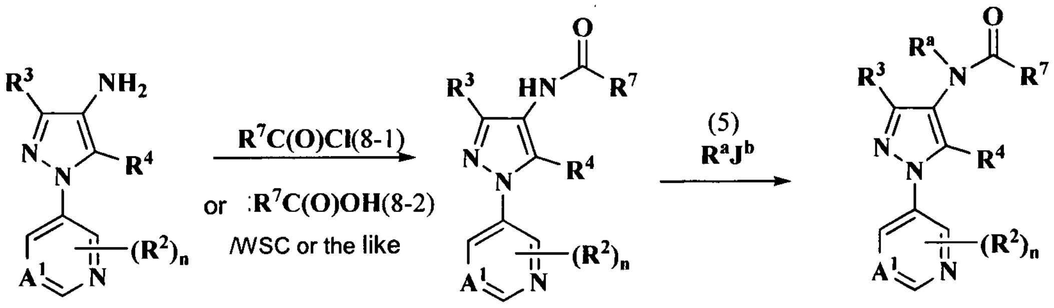


(13) (41) (9)

A compound represented by the formula (13) [wherein $A^1$, $R^2$, $R^3$, $R^4$ and n are the same as defined above] is reacted with a compound represented by the formula (8-1) or a compound represented by the formula (8-2) in the same manner as in Process C to obtain a compound of the present invention represented by the formula (41) [wherein $A^1$, $R^2$, $R^3$, $R^4$, $R^7$ and n are the same as defined above].

Then, the compound represented by the formula (41) is reacted with a compound represented by the formula (5) in the same manner as in Process B to obtain a compound of the present invention represented by the formula (9).

If necessary, the reactions in Processes A to N may be carried out in an atmosphere of an inert gas such as nitrogen or argon.

In Processes A to N, the reaction mixture after a reaction can be worked up by an ordinary procedure such as direct concentration, concentration of a solution in an organic solvent after washing with water, pouring into ice-water or extraction with an organic solvent followed by concentration to obtain the desired product. If purification is needed, the desired product may be isolated or purified by recrystallization or fractionation by column chromatography, thin layer chromatography or liquid chromatography.

The compound represented by the formula (2) used in Process A may be synthesized, for example, as follows.

## Reaction Scheme 1

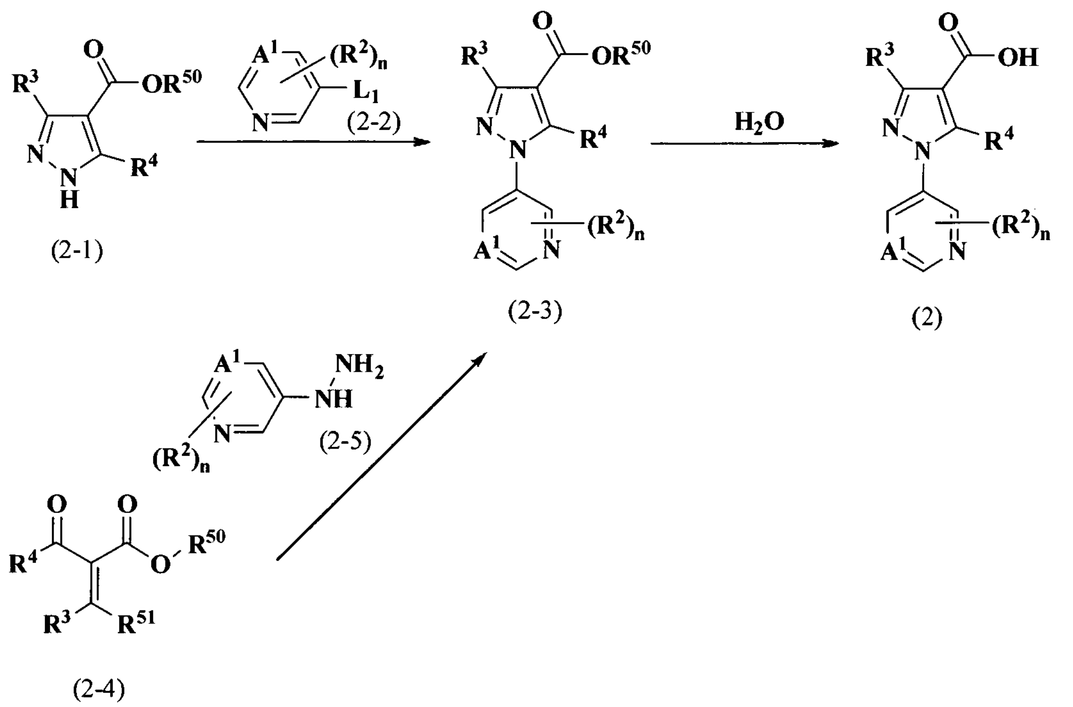


(2-1) (2-2) (2-3) (2) (2-5) (2-4)

A compound represented by the formula (2-1) [wherein $R^3$ and $R^4$ are the same as defined above, $R^{50}$ is a $C_1$-$C_6$ alkyl group] can be synthesized by a known method disclosed in Synlett, 2004, vol. 4, p. 703 or the like.

Namely, the compound represented by the formula (2-1) is reacted with a known compound represented by the formula (2-2) [wherein $A^1$, $R^2$ and n are the same as defined above, $L_1$ is a fluorine atom, a chlorine atom, a bromine atom, an iodine atom, a $-B(OH)_2$ group or a $-B(OR^{51})_2$ group (wherein $R^{51}$ is a hydrogen atom or identical or different $C_1$-$C_6$ alkyl, or two $R^{51}$'s may form $-CH_2CH_2-$ or $-C(CH_3)_2C(CH_3)_2-$ together with each other] in the presence of a metal catalyst such as copper and a base or in the presence of a transition metal catalyst such palladium and a base by a method disclosed in the literature such as Journal of Organic Chemistry, 2004, vol. 69, p. 5578 to obtain a compound represented by the formula (2-3) [wherein $A^1$, $R^2$, $R^3$, $R^4$ and n are the same as defined above, $R^{50}$ is a $C_1$-$C_6$ alkyl group].

A compound represented by the formula (2-3) is also obtained by reacting a compound represented by the formula (2-4) [wherein $R^3$, $R^4$ and $R^{50}$ are the same as defined above, $R^{51}$ is a leaving group such as a methoxy group, an ethoxy group or a dimethylamino group] and a compound represented by the formula (2-5) [wherein $A^1$, $R^2$ and n are the same as defined above] a method disclosed in the literature such as Journal of Heterocyclic Chemistry, 1987, vol. 24, p.1669. The compound represented by the formula (2-4) can be synthesized by a known method disclosed in Journal of Heterocyclic Chemistry, 1987, vol. 24, p. 693, and the compound represented by the formula (2-5) by a known method disclosed in Journal of Medicinal Chemistry, 2002, vol. 45, p.5397.

The compound represented by the formula (2-3) may be hydrolyzed by a known method disclosed in the literature to obtain a compound represented by the formula (2) [wherein $A^1$, $R^2$, $R^3$, $R^4$ and n are the same as defined above].

The compound represented by the formula (2-12) used in Process K may be synthesized, for example, as follows.

Reaction Scheme 2

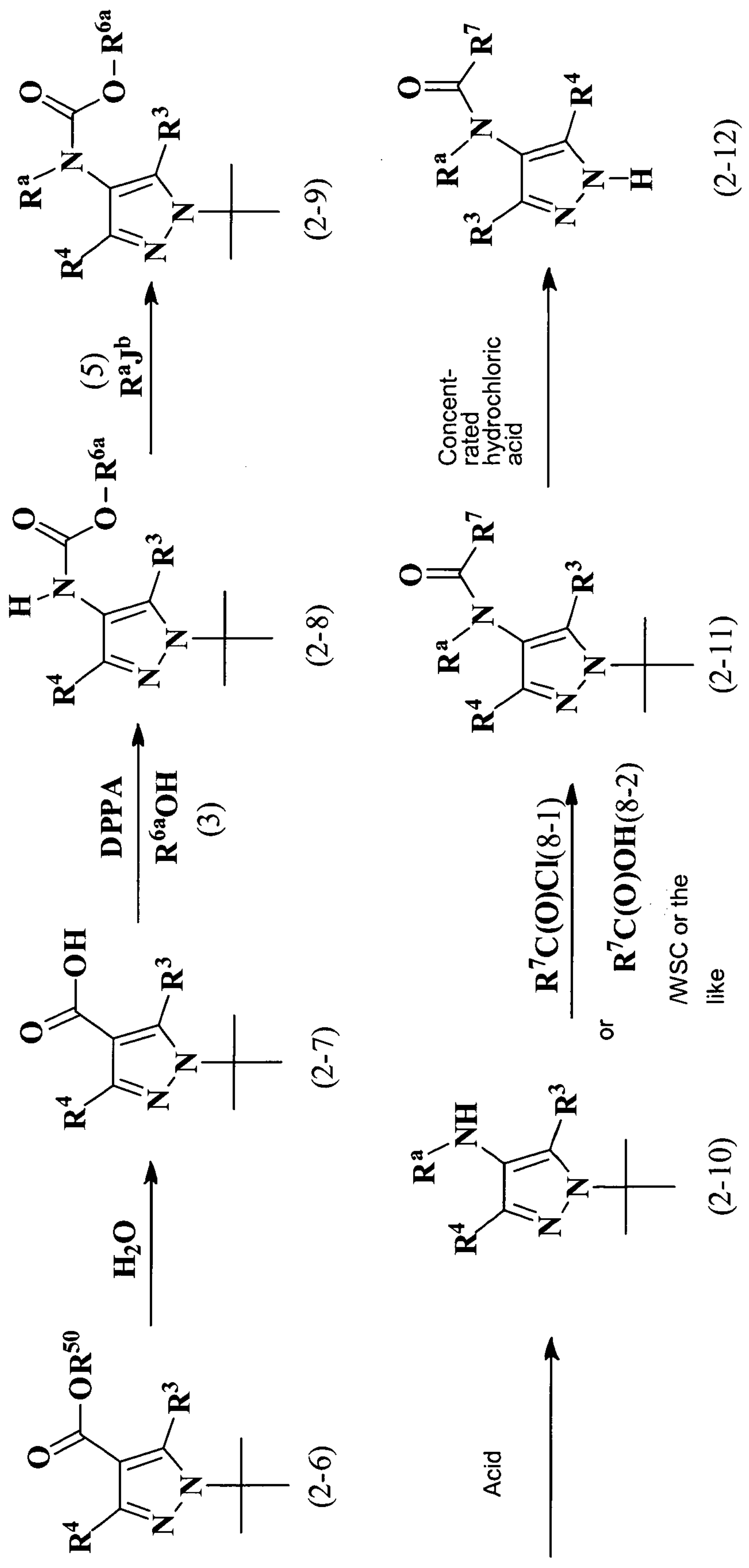


A compound represented by the formula (2-6) [wherein $R^3$, $R^4$ and $R^{50}$ are the same as defined above] can

be synthesized by a known method disclosed in Journal of Heterocyclic Chemistry, 1987, vo. 24, p.693.

The compound represented by the formula (2-6) may be hydrolyzed by a known method in the literature to obtain a compound represented by the formula (2-7) [wherein $R^3$ and $R^4$ are the same as defined above].

Then, from the compound represented by the formula (2-7), a compound represented by the formula (2-8) [wherein $R^3$, $R^4$ and $R^{6a}$ are the same as defined above] in the same manner as in Process A.

Then, the compound represented by the formula (2-8) is reacted with a compound represented by the formula (5) in the same manner as in Process B to obtain a compound represented by the formula (2-9) [wherein $R^3$, $R^4$, $R^a$ and $R^{6a}$ are the same as defined above].

Further, from the compound represented by the formula (2-9), a compound represented by the formula (2-11) [wherein $R^3$, $R^4$, $R^7$ and $R^a$ are the same as defined above]can be synthesized by Process C through a compound represented by the formula (2-10) [wherein $R^3$, $R^4$ and $R^a$ are the same as defined above].

The compound represented by the formula (2-11) thus obtained is deprotected in concentrated hydrochloric acid to obtain a compound represented by the formula (2-12) [wherein $R^3$, $R^4$, $R^7$ and $R^a$ are the same as defined above].

The compound represented by the formula (7) used in Process D may be synthesized, for example, as follows.

Reaction Scheme 3

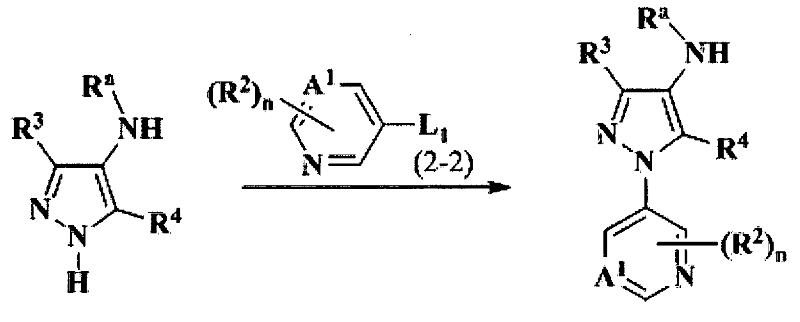


(2-13)

(7)

A compound represented by the formula (2-13) [wherein $R^3$, $R^4$ and $R^a$ are the same as defined above] can be synthesized by a known method disclosed in WO2011/048082 or the like.

A compound represented by the formula (2-13) and a compound represented by the formula (2-2) are reacted in the same manner as in Process K to obtain a compound represented by the formula (7).

As specific active compounds covered by the present invention, for example, those shown in Tables 2 and 3 may be mentioned. However, the compounds merely exemplify the present invention, and the present invention is by no means restricted thereto. In the Tables, Me denotes methyl group, and similarly, Et denotes ethyl group, n-Pr and Pr-n denote normal propyl group, i-Pr and Pr-i denote isopropyl group, c-Pr and Pr-c denote cyclopropyl group, n-Bu and Bu-n denote normal butyl group, s-Bu and Bu-s denote secondary butyl group, i-Bu and Bu-i denote isobutyl group, t-Bu and Bu-t denote tertiary-butyl group, c-Bu and Bu-c denote cyclobutyl group, n-Pen and Pen-n denote normal pentyl group, c-Pen and Pen-c denote cyclopentyl group, n-Hex and Hex-n denote normal hexyl group, c-Hex and Hex-c denote cyclohexyl group, Hept denotes heptyl group, Oct denotes octyl group, and Ph denotes phenyl group.

In the Tables, D1-1a, D1-1b, D1-2a, D1-2b, D1-2c, D1-4a, D1-4b, D1-5a, D1-5b, D1-5c, D1-5d, D1-5e, D1-5f, D1-6a, D1-6b, D1-6c, D1-6d, D1-7a, D1-7b, D1-8a, D1-8b, D1-8c, D1-8f, D1-9a, D1-9b, D1-9c, D1-9e, D1-9f, D1-10a, D1-10b, D1-10c, D1-10e, D1-10f, D1-11a, D1-12a, D1-12b, D1-12c, D1-12f, D1-13a, D1-13b, D1-13d, D1-14a, D1-14b, D1-15a, D1-15b, D1-16a, D1-16b, D1-17a, D1-18a, D1-19a, D1-20a, D1-21a, D1-21b, D1-22a, D1-23a, D1-23b, D1-24a, D1-25a, D1-26a, D1-27a, D1-27b, D1-28a, D1-29a, D1-30a, D1-31a, D1-32a, D1-32b, D1-33a, D1-33b, D1-34a, D1-35a, D1-35b, D1-36a, D1-37a, D1-38a, D1-38b, D1-39a, D1-39b, D1-40a, D1-40b, D1-40c, D1-41a, D1-42a, D1-43a, D1-45a, D1-45d, D1-49a, D1-51a, D1-59a, D1-61a, D1-79a, D1-80a, D1-81a, D1-81b, D1-82a, D1-82b, D1-82c, D1-84d, D1-85d, D1-87a, D1-88a, D1-88b, D1-92c, D1-93a, D1-94b, D1-94c, D1-98a, D1-103b, D1-103c, D1-103d, D1-103e, D1-103f, D1-103g, D1-103h, D1-103i, D1-108a and , D1-108b represent the following structures, and the numbers in the structural formulae D1-1b, D1-2c, D1-5d, D1-5e, D1-5f, D1-6d, D1-7b, D1-8f, D1-9e, D1-9f, D1-10e, D1-10f, D1-12f, D1-32b, D1-33b, D1-38b, D1-39b indicate the positions of the substituent $X^1$, the numbers in the structural forma D1-45d indicate the positions of the substituent $X^{1b}$, and the numbers in the structural formula D1-108b indicate the positions of the substituent Z.

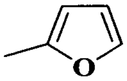
D1-1a

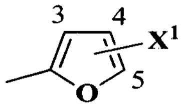
D1-1b

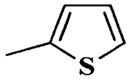
D1-2a

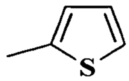
D1-2b

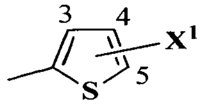
D1-2c

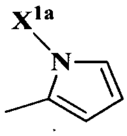
D1-4a

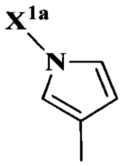
D1-4b

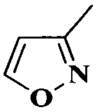
D1-5a

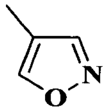
D1-5b

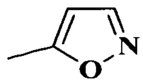
D1-5c

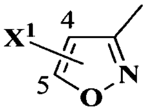
D1-5d

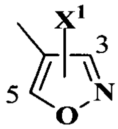
D1-5e

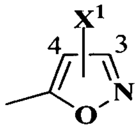
D1-5f

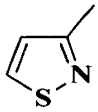
D1-6a

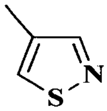
D1-6b

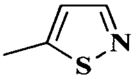
D1-6c

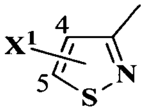
D1-6d

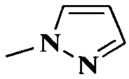
D1-7a

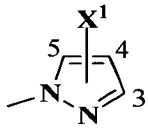
D1-7b

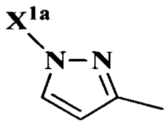
D1-8a

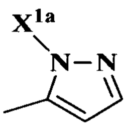
D1-8b

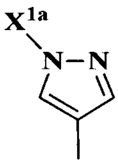
D1-8c

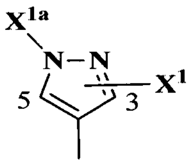
D1-8f

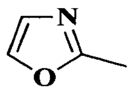
D1-9a

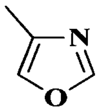
D1-9b

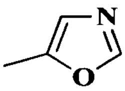
D1-9c

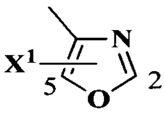
D1-9e

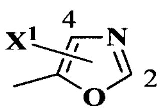
D1-9f

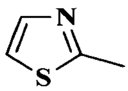
D1-10a

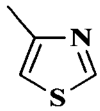
D1-10b

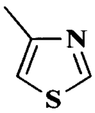
D1-10c

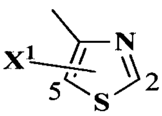
D1-10e

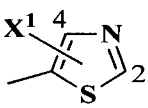
D1-10f

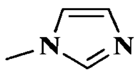
D1-11a

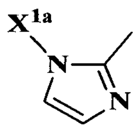
D1-12a

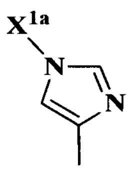
D1-12b

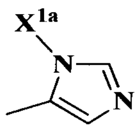
D1-12c

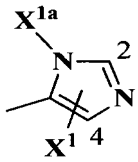
D1-12f

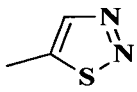
D1-13a

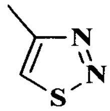
D1-13b

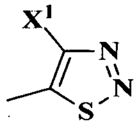
D1-13d

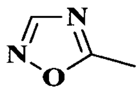
D1-14a

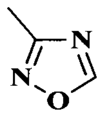
D1-14b

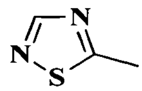
D1-15a

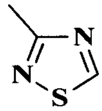
D1-15b

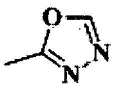
D1-16a

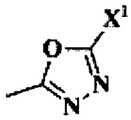
D1-16b

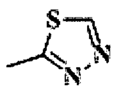
D1-17a

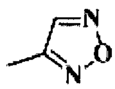
D1-18a

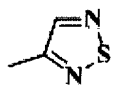
D1-19a

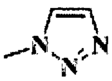
D1-20a

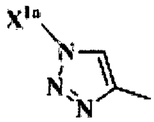
D1-21a

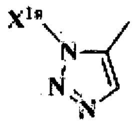
D1-21b

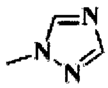
D1-22a

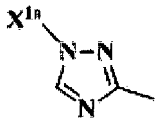
D1-23a

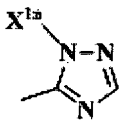
D1-23b

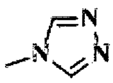
D1-24a

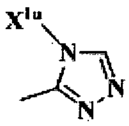
D1-25a

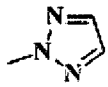
D1-26a

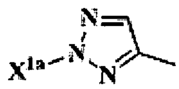
D1-27a

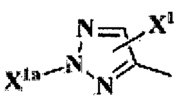
D1-27b

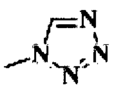
D1-28a

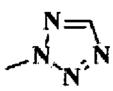
D1-29a

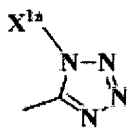
D1-30a

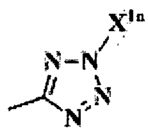
D1-31a

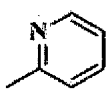
D1-32a

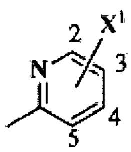
D1-32b

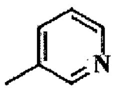
D1-33a

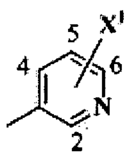
D1-33b

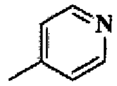
D1-34a

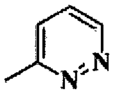
D1-35a

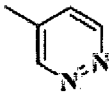
D1-35b

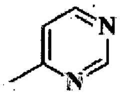
D1-36a

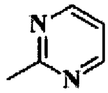
D1-37a

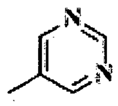
D1-38a

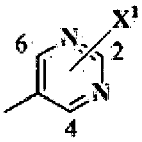
D1-38b

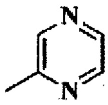
D1-39a

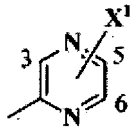
D1-39b

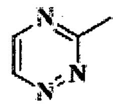
D1-40a

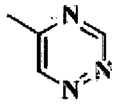
D1-40b

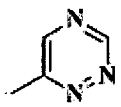
D1-40c

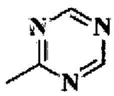
D1-41a

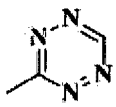
D1-42a

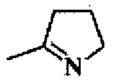
D1-43a

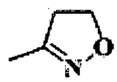
D1-45a

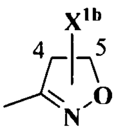
D1-45d

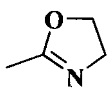
D1-49a

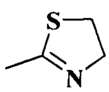
D1-51a

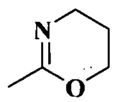
D1-59a

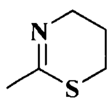
D1-61a

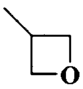
D1-79a

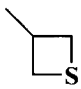
D1-80a

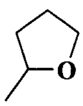
D1-81a

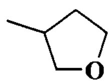
D1-81b

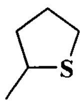
D1-82a

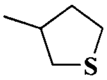
D1-82b

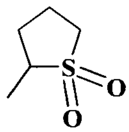
D1-82c

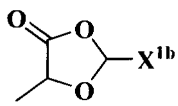
D1-84d

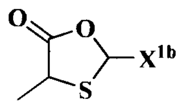
D1-85d

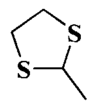
D1-87a

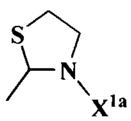
D1-88a

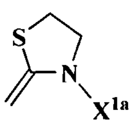
D1-88b

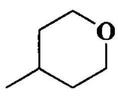
D1-92c

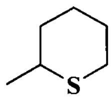
D1-93a

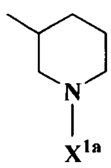
D1-94b

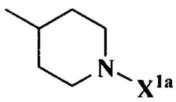
D1-94c

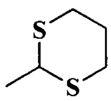
D1-98a

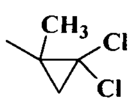
D1-103b

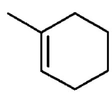
D1-103c

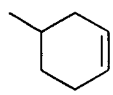
D1-103d

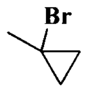
D1-103e

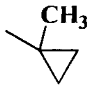
D1-103f

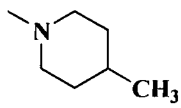
D1-103g

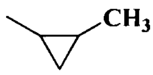
D1-103h

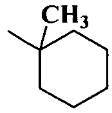
D1-103i

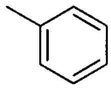
D1-108a

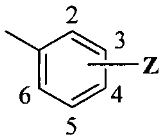
D1-108b

[Table 2]

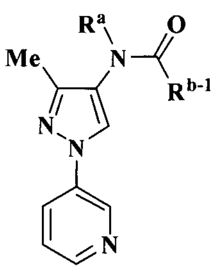

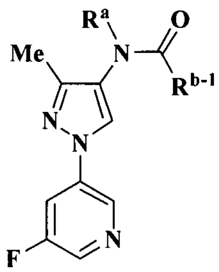

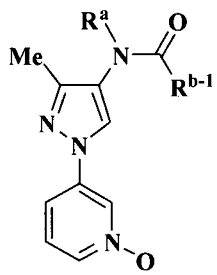

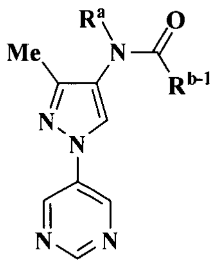

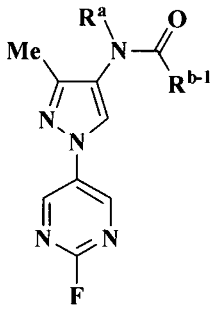

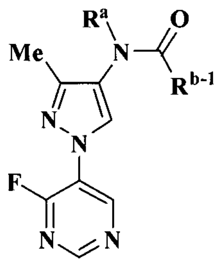

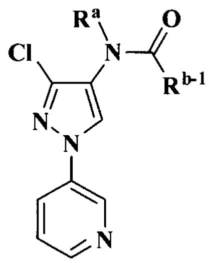

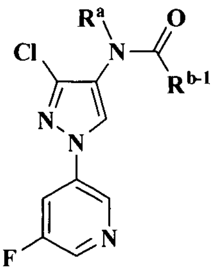

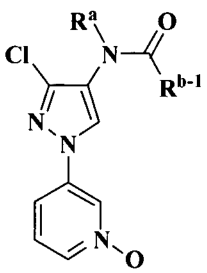

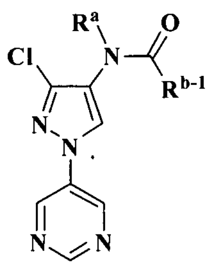

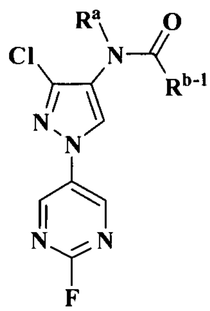

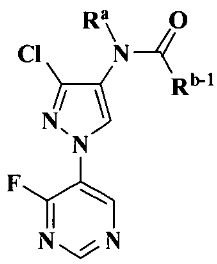

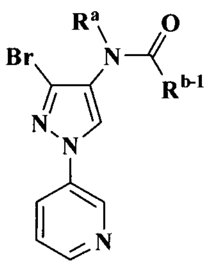

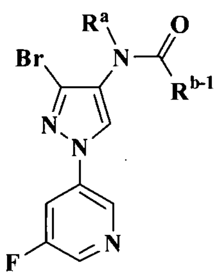

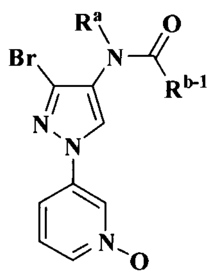

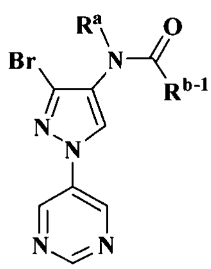

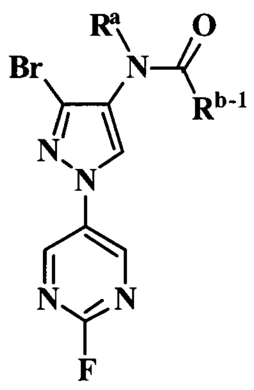
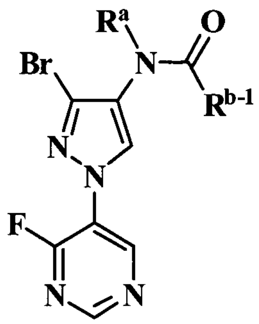
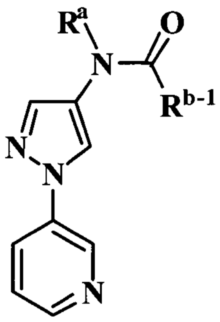
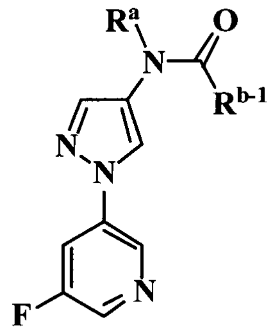

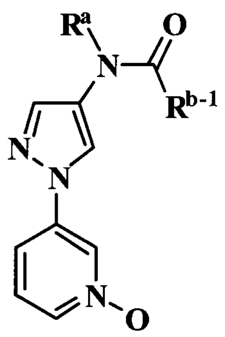
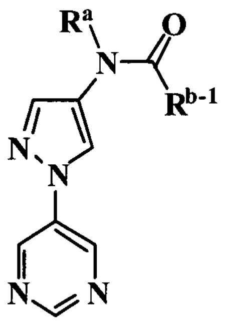
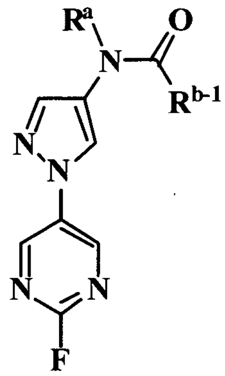
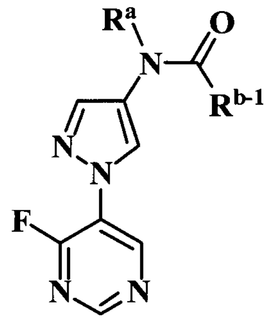

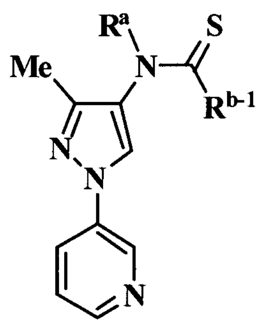
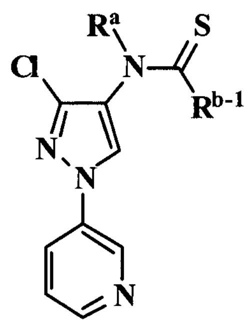
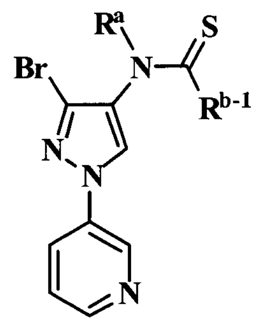
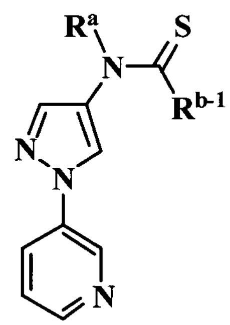

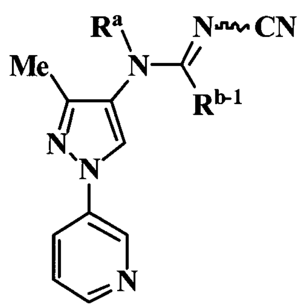
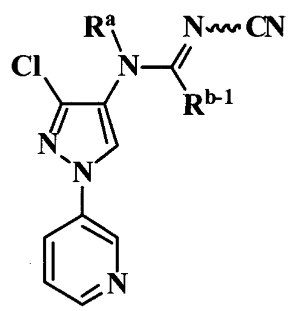
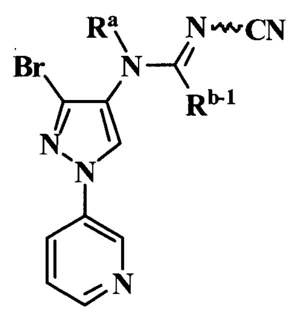
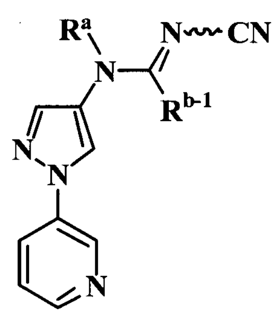

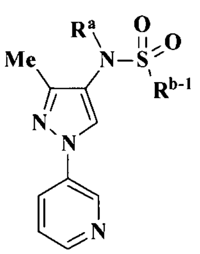 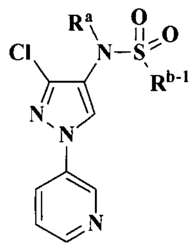 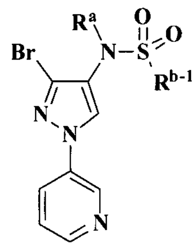 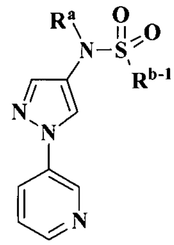

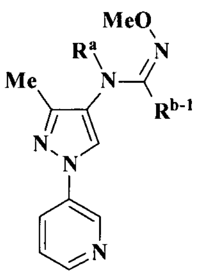 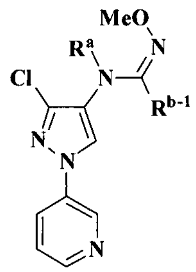 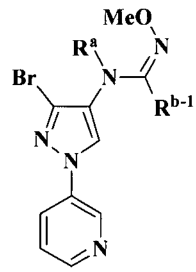 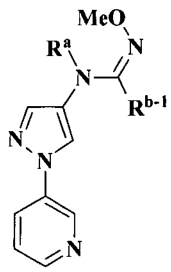

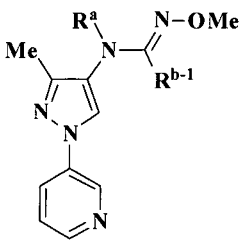 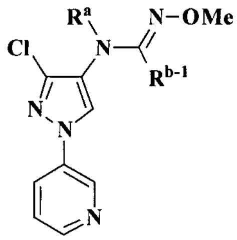 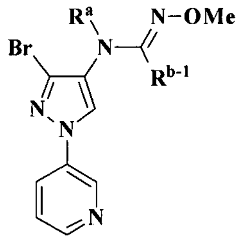 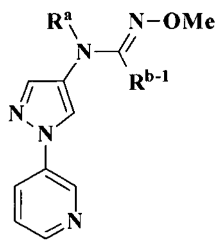

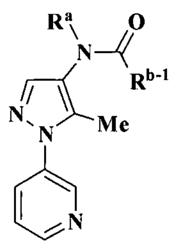 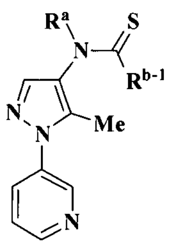 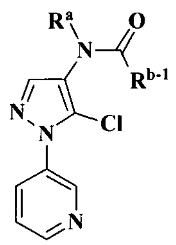 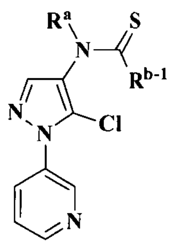

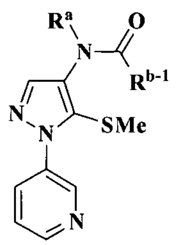 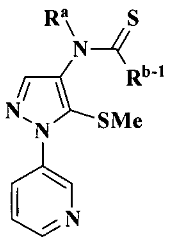 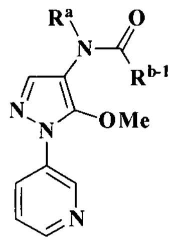 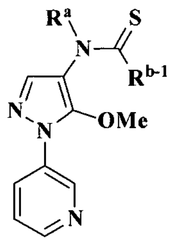

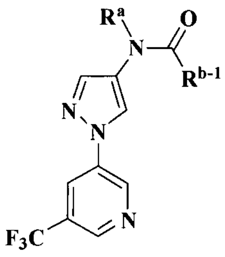
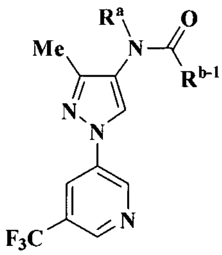
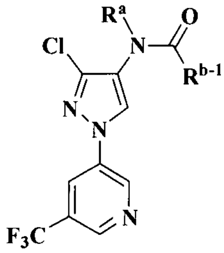
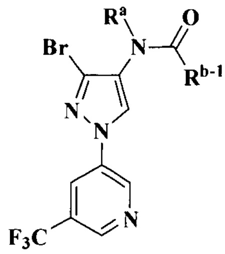

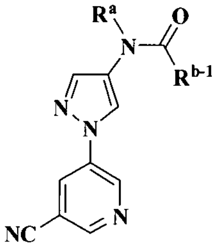
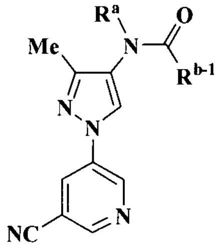
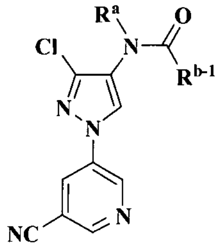
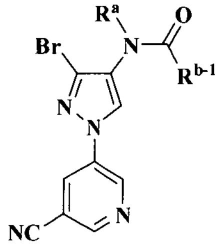

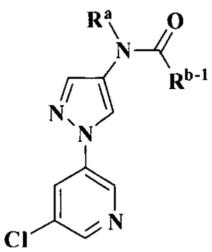
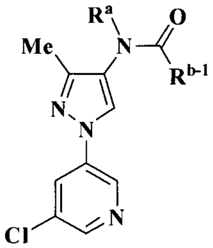
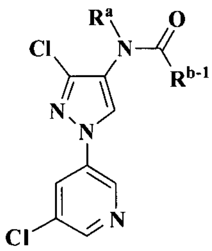
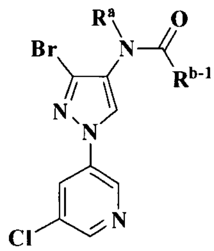

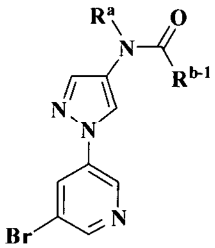
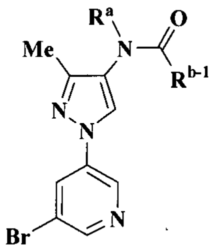
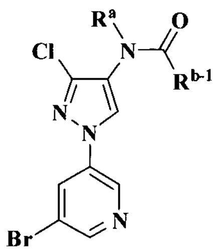
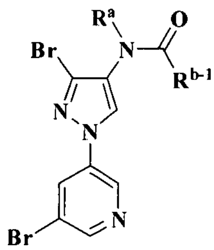

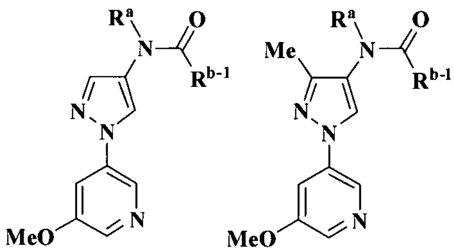
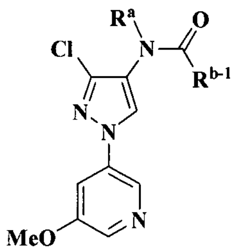
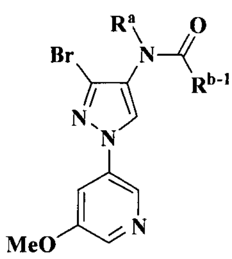

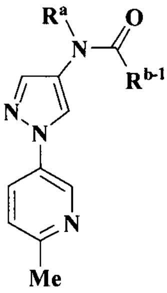
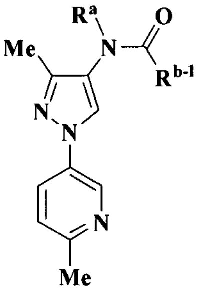
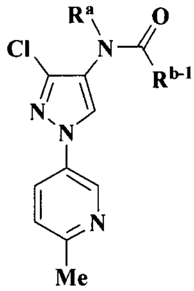
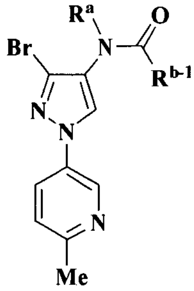

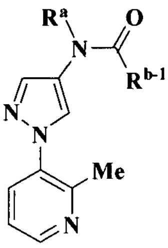
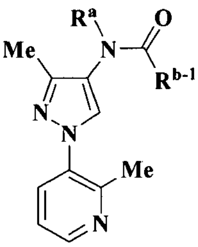
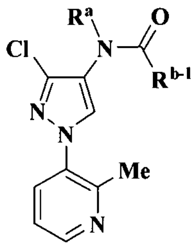
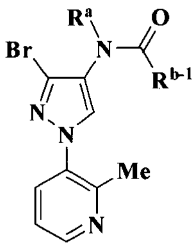

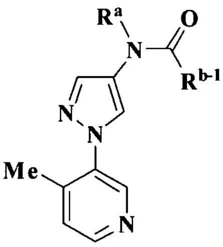
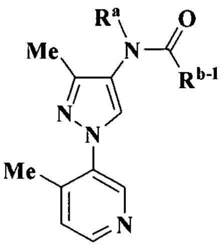
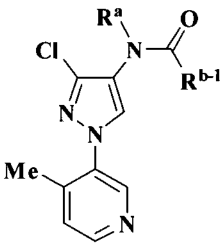
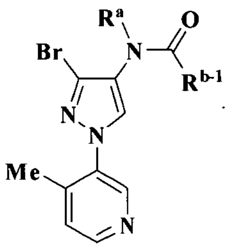

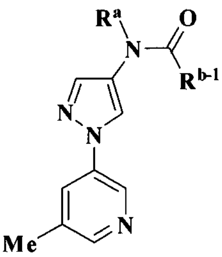
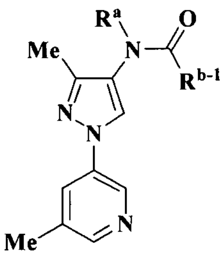
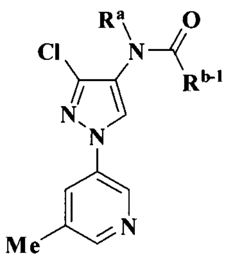
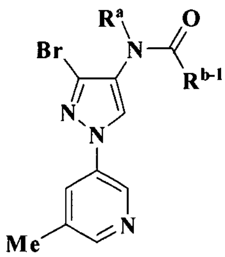

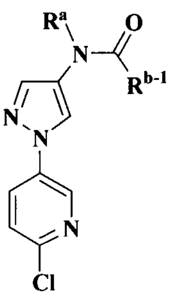
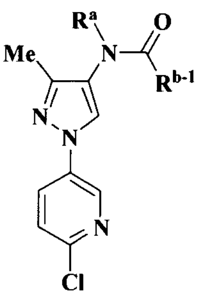
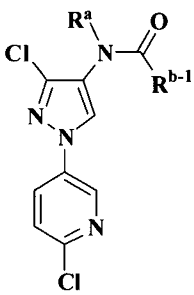
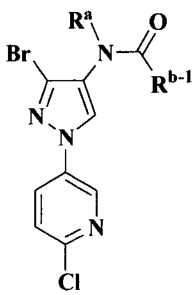

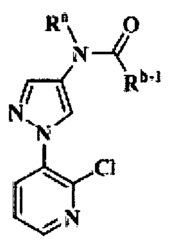 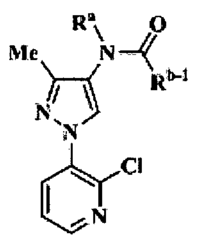 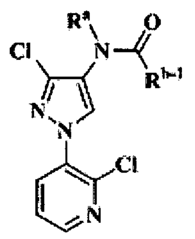 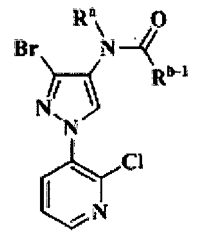

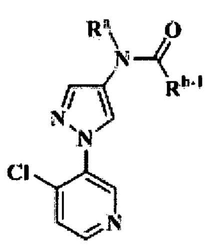 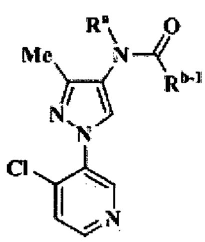 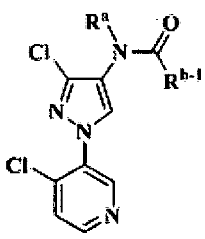 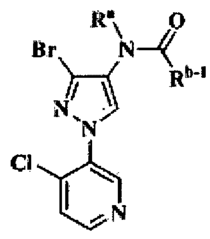

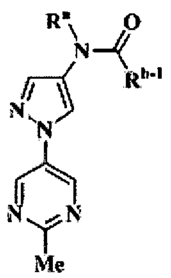 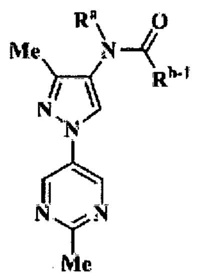 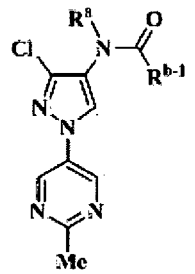 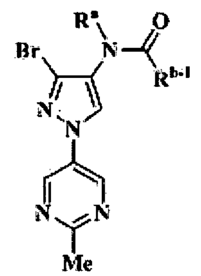

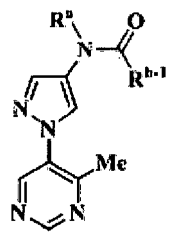 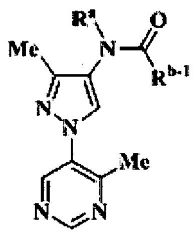 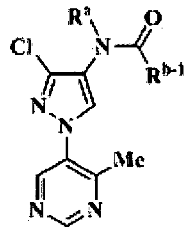 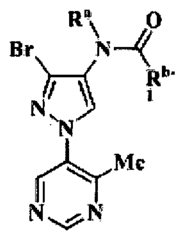

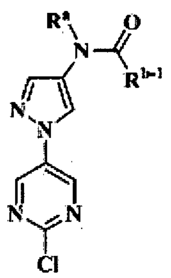 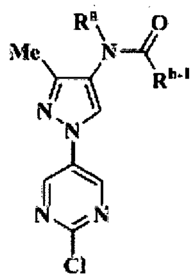 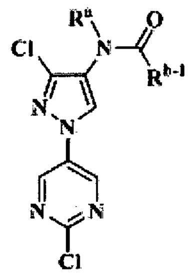 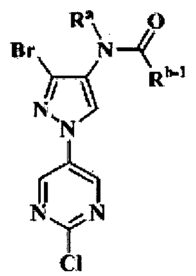

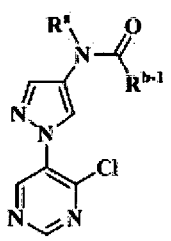 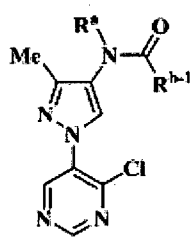 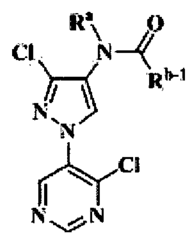 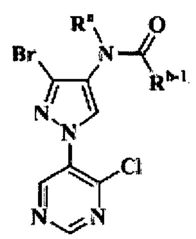

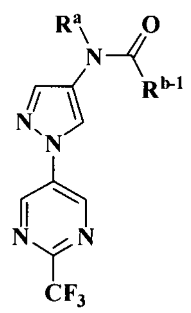
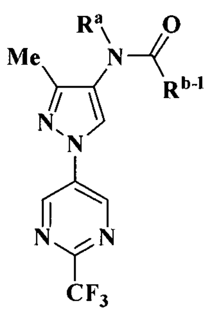
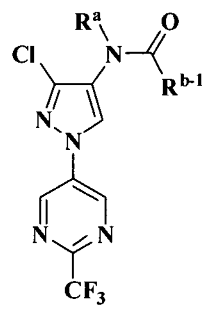
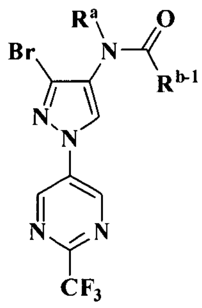
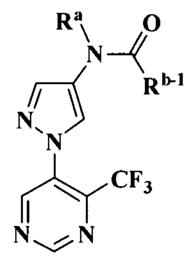
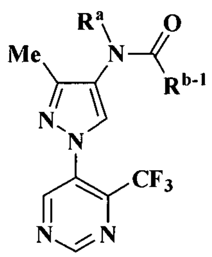
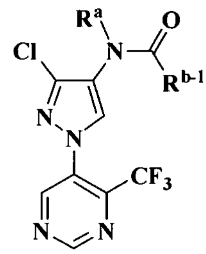
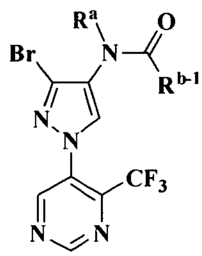
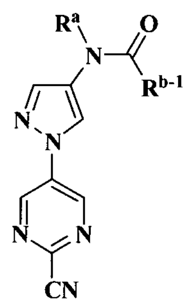
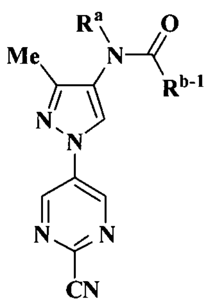
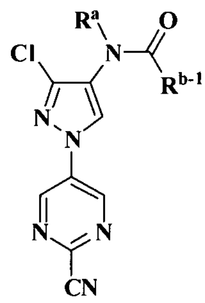
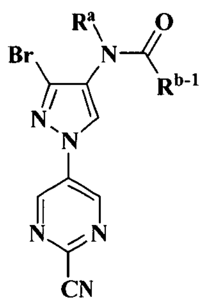
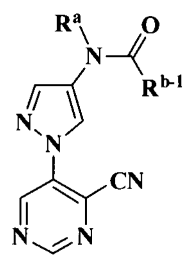
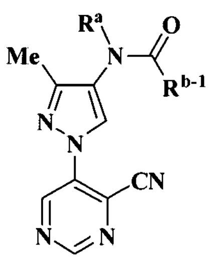
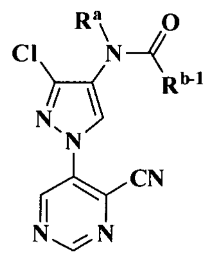
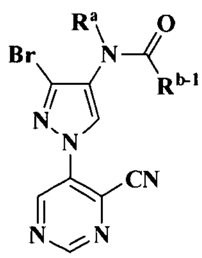
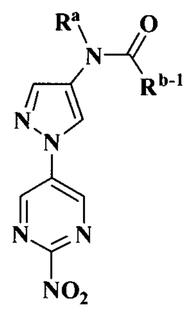
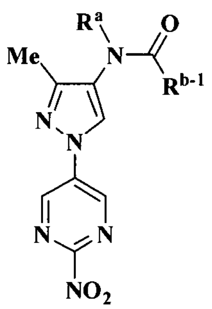
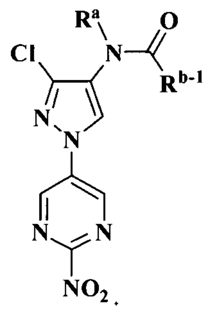
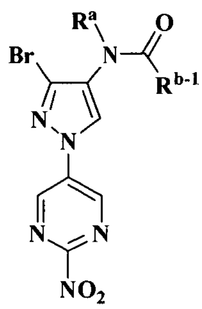

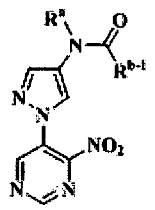
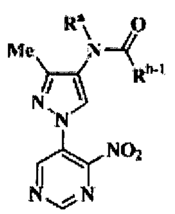
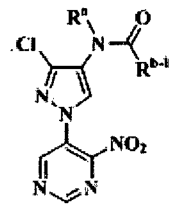
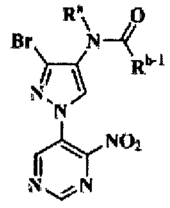

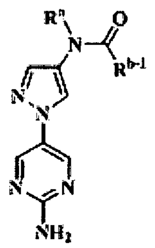
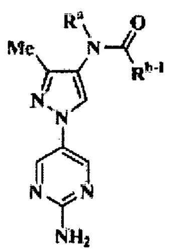
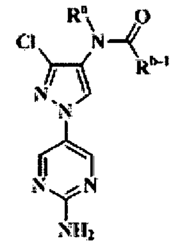
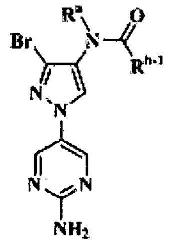

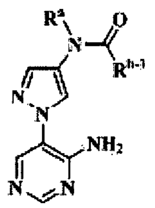
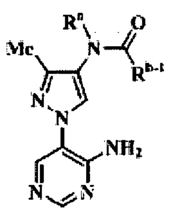
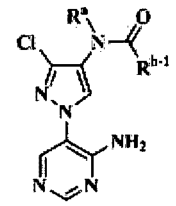
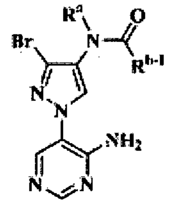

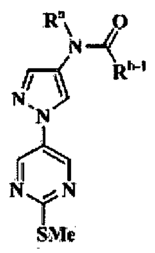
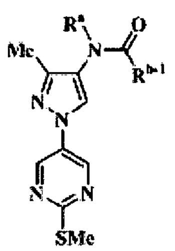
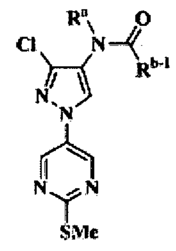
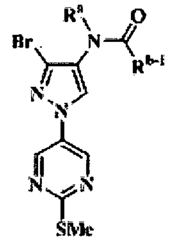

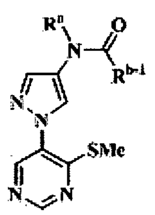
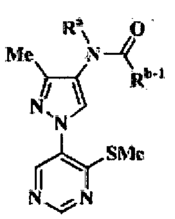
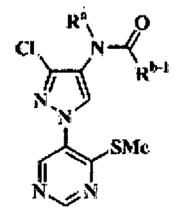
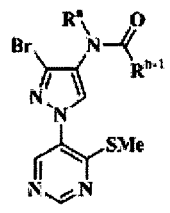

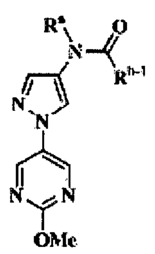
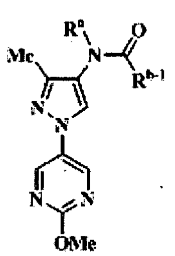
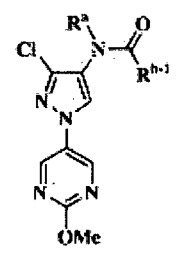
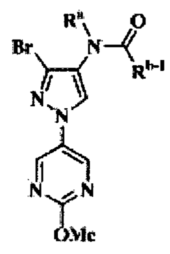

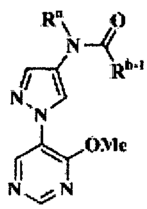 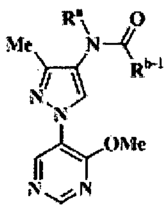 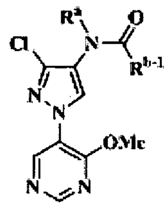 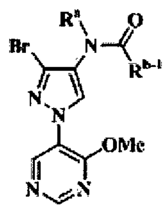

TABLE 2

| $R^a$ | $R^{b-1}$ |
|---|---|
| H | C(O)Me |
| H | Me |
| H | Et |
| H | Pr-n |
| H | Pr-i |
| H | Pr-c |
| H | Bu-n |
| H | Bu-c |
| H | Bu-i |
| H | Bu-s |
| H | Bu-t |
| H | Pen-n |
| H | Pen-c |
| H | Hex-n |
| H | Hex-c |
| H | $CH_2Br$ |
| H | CH(Br)Me |
| H | $CH_2CH_2CH_2Cl$ |
| H | $CH(Br)CH_2CH_2CH_2Br$ |
| H | $CH_2CF_3$ |
| H | $CH_2CH(OH)CF_3$ |
| H | $CH_2CN$ |
| H | $CH_2C(O)OMe$ |
| H | $CH_2C(O)NHMe$ |
| H | $CH=CH_2$ |
| H | CH=CHSMe |
| H | $CH=CHCF_3$ |
| H | $CH=C(Me)CF_3$ |
| H | $CH\{D1\text{-}108b(4\text{-}CF_3)\}$ |
| H | C≡CH |
| H | $C≡CSiMe_3$ |
| H | $CH_2OMe$ |
| H | $CH_2OEt$ |
| H | $CH(Me)OCH_2CF_3$ |
| H | CH(Me)ON=CHMe |
| H | $CH_2CH_2SC(O)Me$ |
| H | $CH_2OC(O)Me$ |
| H | $CH_2SMe$ |
| H | $CH_2S(O)Me$ |
| H | $CH_2S(O)_2Me$ |
| H | $CH_2SEt$ |

(continued)

| $R^a$ | $R^{b-1}$ |
|---|---|
| H | $CH_2S(O)Et$ |
| H | $CH_2S(O)_2Et$ |
| H | $CH_2SCH_2CH=CH_2$ |
| H | $CH_2S(O)CH_2CH=CH_2$ |
| H | $CH_2S(O)_2CH_2CH=CH_2$ |
| H | $CH_2SCH_2CF_3$ |
| H | $CH_2S(O)CH_2CF_3$ |
| H | $CH_2S(O)_2CH_2CF_3$ |
| H | CH(Me)SMe |
| H | CH(Me)S(O)Me |
| H | $CH(Me)S(O)_2Me$ |
| H | CH(Me)SEt |
| H | CH(Me)S(O)Et |
| H | $CH(Me)S(O)_2Et$ |
| H | CH(Me)S(=NCN)Et |
| H | CH(Me)S(O)(=NCN)Et |
| H | CH(Me)SPr-n |
| H | CH(Me)S(O)Pr-n |
| H | $CH(Me)S(O)_2Pr$-n |
| H | CH(Me)SPr-i |
| H | CH(Me)S(O)Pr-i |
| H | $CH(Me)S(O)_2Pr$-i |
| H | CH(Me)SBu-t |
| H | CH(Me)S(O)Bu-t |
| H | $CH(Me)S(O)_2Bu$-t |
| H | $CH(Me)SCH_2Pr$-c |
| H | $CH(Me)S(O)CH_2Pr$-c |
| H | $CH(Me)S(O)_2CH_2Pr$-c |
| H | $CH(Me)SCH_2OMe$ |
| H | $CH(Me)SCH_2SMe$ |
| H | $CH(Me)S(O)CH_2SMe$ |
| H | $CH(Me)S(O)_2CH_2SMe$ |
| H | $CH(Me)SCH_2C\equiv CH$ |
| H | $CH(Me)S(O)CH_2C\equiv CH$ |
| H | $CH(Me)S(O)_2CH_2C\equiv CH$ |
| H | $CH(Me)SCH_2C(O)NHMe$ |
| H | $CH(Me)S(O)CH_2C(O)NHMe$ |
| H | $CH(Me)S(O)_2CH_2C(O)NHMe$ |
| H | $CH(Me)SCH_2C(O)OMe$ |
| H | $CH(Me)S(O)CH_2C(O)OMe$ |
| H | $CH(Me)S(O)_2CH_2C(O)OMe$ |
| H | $CH(Me)SCH_2CH_2CH_2Cl$ |
| H | $CH(Me)S(O)CH_2CH_2CH_2Cl$ |
| H | $CH(Me)S(O)_2CH_2CH_2CH_2Cl$ |
| H | $CH(Me)SCH_2CF_3$ |
| H | $CH(Me)S(O)CH_2CF_3$ |
| H | $CH(Me)S(O)_2CH_2CF_3$ |
| H | $CH(Me)SCH_2$(D1-34a) |
| H | $CH(Me)S(O)CH_2$(D1-34a) |

(continued)

| $R^a$ | $R^{b-1}$ |
|---|---|
| H | $CH(Me)S(O)_2CH_2$(D1-34a) |
| H | $CH(CH_3)SCH_2Si(CH_3)_3$ |
| H | CH(Me)SCN |
| H | $CH(Me)SCH_2CN$ |
| H | CH(Me)SC(O)Me |
| H | CH(Me)S(D1-37a) |
| H | CH(Et)SMe |
| H | CH(Et)S(O)Me |
| H | $CH(Et)S(O)_2Me$ |
| H | CH(Et)SEt |
| H | CH(Et)S(O)Et |
| H | $CH(Et)S(O)_2Et$ |
| H | CH(F)SMe |
| H | CH(F)S(O)Me |
| H | $CH(F)S(O)_2Me$ |
| H | CH(F)SEt |
| H | CH(F)S(O)Et |
| H | $CH(F)S(O)_2Et$ |
| H | CH{OC(O)Me}SMe |
| H | CH{OC(O)Me}S(O)Me |
| H | $CH\{OC(O)Me\}S(O)_2Me$ |
| H | $CH(SMe)_2$ |
| H | $C(Me)_2SMe$ |
| H | $C(Me)_2S(O)Me$ |
| H | $C(Me)_2S(O)_2Me$ |
| H | $CH_2CH_2SH$ |
| H | $CH_2CH_2SMe$ |
| H | $CH_2CH_2S(O)Me$ |
| H | $CH_2CH_2S(O)_2Me$ |
| H | $CH_2CH_2SEt$ |
| H | $CH_2CH_2S(O)Et$ |
| H | $CH_2CH_2S(O)_2Et$ |
| H | $CH_2CH_2SPr$-n |
| H | $CH_2CH_2S(O)Pr$-n |
| H | $CH_2CH_2S(O)_2Pr$-n |
| H | $CH_2CH_2SPr$-i |
| H | $CH_2CH_2S(O)Pr$-i |
| H | $CH_2CH_2S(O)_2Pr$-i |
| H | $CH_2CH_2SCH_2CF3$ |
| H | $CH_2CH_2S(O)CH_2CF_3$ |
| H | $CH_2CH_2S(O)_2CH_2CF_3$ |
| H | $CH_2CH_2SC(O)Me$ |
| H | $CH(Me)CH_2SMe$ |
| H | $CH(Me)CH_2S(O)Me$ |
| H | $CH(Me)CH_2S(O)_2Me$ |
| H | $CH(Me)CH_2SEt$ |
| H | $CH(Me)CH_2S(O)Et$ |
| H | $CH(Me)CH_2S(O)_2Et$ |
| H | $CH(Me)CH_2SPr$-n |

(continued)

| $R^a$ | $R^{b-1}$ |
|---|---|
| H | $CH(Me)CH_2S(O)Pr$-n |
| H | $CH(Me)CH_2S(O)_2Pr$-n |
| H | $CH(Me)CH_2SPr$-i |
| H | $CH(Me)CH_2S(O)Pr$-i |
| H | $CH(Me)CH_2S(O)_2Pr$-i |
| H | $CH(Me)CH_2SCH_2CF_3$ |
| H | $CH(Me)CH_2S(O)CH_2CF_3$ |
| H | $CH(Me)CH_2S(O)_2CH_2CF_3$ |
| H | $CH(Me)CH_2SC(O)Me$ |
| H | $CH(Et)CH_2SMe$ |
| H | $CH(Et)CH_2S(O)Me$ |
| H | $CH(Et)CH_2S(O)_2Me$ |
| H | $CH(OMe)CH_2SMe$ |
| H | $CH(OMe)CH_2S(O)Me$ |
| H | $CH(OMe)CH_2S(O)_2Me$ |
| H | $CH(SMe)CH_2SMe$ |
| H | $CH(SMe)CH_2S(O)Me$ |
| H | $CH(SMe)CH_2S(O)_2Me$ |
| H | $CH(CH_2SMe)CH_2SMe$ |
| H | $CH(CH_2SMe)CH_2S(O)Me$ |
| H | $CH(CH_2SMe)CH_2S(O)_2Me$ |
| H | $CH(Cl)CH_2SMe$ |
| H | $CH(Cl)CH_2S(O)Me$ |
| H | $CH(Cl)CH_2S(O)_2Me$ |
| H | $CH(CN)CH_2SMe$ |
| H | $CH(CN)CH_2S(O)Me$ |
| H | $CH(CN)CH_2S(O)_2Me$ |
| H | $CH(CN)CH_2SEt$ |
| H | $CH(CN)CH_2S(O)Et$ |
| H | $CH(CN)CH_2S(O)_2$ Et |
| H | $CH\{C(O)OMe\}CH_2SMe$ |
| H | $CH\{C(O)OMe\}CH_2S(O)Me$ |
| H | $CH\{C(O)OMe\}CH_2S(O)_2$ Me |
| H | $CH\{C(O)OMe\}CH_2SEt$ |
| H | $CH\{C(O)OMe\}CH_2S(O)Et$ |
| H | $CH\{C(O)OMe\}CH_2S(O)_2Et$ |
| H | $CH(Ph)CH_2SMe$ |
| H | $CH(Ph)CH_2S(O)Me$ |
| H | $CH(Ph)CH_2S(O)_2Me$ |
| H | $C(Me)_2CH_2SMe$ |
| H | $C(Me)_2CH_2S(O)Me$ |
| H | $C(Me)_2CH_2S(O)_2Me$ |
| H | $C(=CH_2)CH_2SMe$ |
| H | $C(=CH_2)CH_2S(O)Me$ |
| H | $C(=CH_2)CH_2S(O)_2Me$ |
| H | $C(=CH_2)CH_2SEt$ |
| H | $C(=CH_2)CH_2S(O)Et$ |
| H | $C(=CH_2)CH_2S(O)_2$ Et |
| H | $CH_2CH_2CH_2SEt$ |

(continued)

| $R^a$ | $R^{b-1}$ |
|---|---|
| H | $CH_2CH_2CH_2S(O)Et$ |
| H | $CH_2CH_2CH_2S(O)_2Et$ |
| H | $CH(Me)CH_2CH_2SEt$ |
| H | $CH(Me)CH_2CH_2S(O)Et$ |
| H | $CH(Me)CH_2CH_2S(O)_2Et$ |
| H | $CH_2CH(Me)CH_2SEt$ |
| H | $CH_2CH(Me)CH_2S(O)Et$ |
| H | $CH_2CH(Me)CH_2S(O)_2Et$ |
| H | $CH_2CH_2CH(Me)SEt$ |
| H | $CH_2CH_2CH(Me)S(O)Et$ |
| H | $CH_2CH_2CH(Me)S(O)_2Et$ |
| H | $CH_2CH_2CH_2SPr$-n |
| H | $CH_2CH_2CH_2S(O)Pr$-n |
| H | $CH_2CH_2CH_2S(O)_2Pr$-n |
| H | $CH(Me)CH_2CH_2SPr$-n |
| H | $CH(Me)CH_2CH_2S(O)Pr$-n |
| H | $CH(Me)CH_2CH_2S(O)_2Pr$-n |
| H | $CH_2CH(Me)CH_2SPr$-n |
| H | $CH_2CH(Me)CH_2S(O)Pr$-n |
| H | $CH_2CH(Me)CH_2S(O)_2Pr$-n |
| H | $CH_2CH_2CH_2CH_2SMe$ |
| H | $CH_2CH_2CH_2CH_2S(O)Me$ |
| H | $CH_2CH_2CH_2CH_2S(O)_2Me$ |
| H | $CH_2CH_2CH_2CH_2SEt$ |
| H | $CH_2CH_2CH_2CH_2S(O)Et$ |
| H | $CH_2CH_2CH_2CH_2S(O)_2$ Et |
| H | $CH_2CH_2CH_2CH_2SPr$-n |
| H | $CH_2CH_2CH_2CH_2S(O)Pr$-n |
| H | $CH_2CH_2CH_2CH_2S(O)_2$ Pr-n |
| H | $CH_2CH(Me)SMe$ |
| H | $CH_2OCH_2SMe$ |
| H | $CH_2CH(SMe)_2$ |
| H | $C(=CH_2)CH_2C(O)OH$ |
| H | $C(=NOMe)CH_3$ |
| H | C(=NOMe)Et |
| H | C(=NOMe)Pr-n |
| H | $C(=NOEt)CH_3$ |
| H | C(=NOPr-n)$CH_3$ |
| H | C(=NOPr-i)$CH_3$ |
| H | C(=NOBu-n)$CH_3$ |
| H | $C(=NOCH_2Ph)CH_3$ |
| H | $C(=NOMe)CH_2OCH_2CF_3$ |
| H | $C(=NOMe)CH_2SMe$ |
| H | $C(=NOMe)CH_2S(O)Me$ |
| H | $C(=NOMe)CH_2S(O)_2Me$ |
| H | $C(=NOMe)CH_2SEt$ |
| H | $C(=NOMe)CH_2S(O)Et$ |
| H | $C(=NOMe)CH_2S(O)_2Et$ |
| H | $C(=NOMe)CH_2SCF_3$ |

(continued)

| $R^a$ | $R^{b-1}$ |
|---|---|
| H | $C(=NOMe)CH_2S(O)CF_3$ |
| H | $C(=NOMe)CH_2S(O)_2CF_3$ |
| H | $C(=NOMe)CH_2SCH_2CF_3$ |
| H | $C(=NOMe)CH_2S(O)CH_2CF_3$ |
| H | $C(=NOMe)CH_2S(O)_2CH_2CF_3$ |
| H | $C(=NOEt)CH_2SMe$ |
| H | $C(=NOEt)CH_2S(O)Me$ |
| H | $C(=NOEt)CH_2S(O)_2Me$ |
| H | $C(=NOPr\text{-}n)CH_2SMe$ |
| H | $C(=NOPr\text{-}n)CH_2S(O)Me$ |
| H | $C(=NOPr\text{-}n)CH_2S(O)_2Me$ |
| H | $C(=NOPr\text{-}i)CH_2SMe$ |
| H | $C(=NOPr\text{-}i)CH_2S(O)Me$ |
| H | $C(=NOPr\text{-}i)CH_2S(O)_2Me$ |
| H | $C(=NOBu\text{-}n)CH_2SMe$ |
| H | $C(=NOBu\text{-}n)CH_2S(O)Me$ |
| H | $C(=NOBu\text{-}n)CH_2S(O)_2Me$ |
| H | $C(=NOCH_2Ph)CH_2SMe$ |
| H | $C(=NOCH_2Ph)CH_2S(O)Me$ |
| H | $C(=NOCH_2Ph)CH_2S(O)_2Me$ |
| H | $C(=NOCH_2CH=CH_2)CH_2SMe$ |
| H | $C(=NOCH_2OMe)CH_2SMe$ |
| H | $C(=NOCH_2CH_2OMe)CH_2SMe$ |
| H | $C\{=NOCH_2CH_2Si(Me)_3\}CH_2SMe$ |
| H | $CH_2CH(=NOMe)$ |
| H | $CH_2C(=NOMe)Me$ |
| H | $CH_2C(=NOMe)Et$ |
| H | $CH_2C(=NOMe)Pr\text{-}n$ |
| H | $CH_2C(=NOMe)Pr\text{-}i$ |
| H | $CH_2C(=NOMe)Bu\text{-}n$ |
| H | $CH_2C(=NOMe)Bu\text{-}i$ |
| H | $CH_2C(=NOMe)Bu\text{-}s$ |
| H | $CH_2C(=NOMe)Bu\text{-}t$ |
| H | $CH_2C(=NOMe)CF_3$ |
| H | $CH_2C(=NOCH_3)CH_2SCH_3$ |
| H | $CH(SMe)C(=NOMe)Me$ |
| H | $CH\{S(O)Me\}C(=NOMe)Me$ |
| H | $CH\{S(O)_2Me\}C(=NOMe)Me$ |
| H | $CH(SEt)C(=NOMe)Me$ |
| H | $CH\{S(O)Et\}C(=NOMe)Me$ |
| H | $CH\{S(O)_2Et\}C(=NOMe)Me$ |
| H | $CH_2NH_2$ |
| H | $CH_2NHC(O)Me$ |
| H | $CH_2NHC(O)OMe$ |
| H | $CH_2NHC(O)OBu\text{-}t$ |
| H | $CH_2NHSO_2Me$ |
| H | $CH_2N(Me)C(O)Me$ |
| H | $CH_2N(Me)C(O)OMe$ |
| H | $CH_2N(Me)C(O)OBu\text{-}t$ |

(continued)

| $R^a$ | $R^{b-1}$ |
|---|---|
| H | $CH_2N(Me)SO_2Me$ |
| H | $CH(CH_2SMe)NH_2$ |
| H | $CH(CH_2SMe)NHC(O)Me$ |
| H | $CH(CH_2SMe)NHC(O)OMe$ |
| H | $CH(CH_2SMe)NHC(O)OBu$-t |
| H | $CH(CH_2SMe)NHSO_2Me$ |
| H | $CH_2P(O)(OMe)_2$ |
| H | $CH_2P(O)(OEt)_2$ |
| H | OBu-t |
| H | O{D1-108b(4-$NO_2$)} |
| H | SMe |
| H | $NH_2$ |
| H | $NHCH_2CH_2SMe$ |
| H | $N(Me)CH_2CH_2SMe$ |
| H | $NHCH_2CH_2CH_2SMe$ |
| H | NHMe |
| H | NHEt |
| H | NPr-n |
| H | $NHCH(Me)_2$ |
| H | $NHCH_2C=CH_2$ |
| H | $NHCH_2C(O)OMe$ |
| H | $NHCH_2CF_3$ |
| H | $N(Me)CH_2CF_3$ |
| H | NHC(O)OEt |
| H | $NHC(O)CCl_3$ |
| H | $NHC(O)CH_2SMe$ |
| H | $N(CH_2CH_3)C(O)CH_2SCH_3$ |
| H | $NHS(O)_2$(D1-108a) |
| H | NHCH=NOMe |
| H | $N=CHNMe_2$ |
| H | NHPh |
| H | NH(D1-1a) |
| H | NH(D1-2a) |
| H | NH(D1-2b) |
| H | NH{D1-4a($X^{1a}$=Me)} |
| H | NH{D1-4b($X^{1a}$=Me)} |
| H | NH(D1-5a) |
| H | NH(D1-5b) |
| H | NH(D1-5c) |
| H | NH(D1-6a) |
| H | NH(D1-6b) |
| H | NH(D1-6c) |
| H | NH(D1-7a) |
| H | NH{D1-8a($X^{1a}$=Me)} |
| H | NH{D1-8b($X^{1a}$=Me)} |
| H | NH{D1-8c($X^{1a}$=Me)} |
| H | NH(D1-9a) |
| H | NH(D1-9b) |
| H | NH(D1-9c) |

(continued)

| $R^a$ | $R^{b-1}$ |
|---|---|
| H | NH(D1-10a) |
| H | NH(D1-10b) |
| H | NH(D1-10c) |
| H | NH(D1-11a) |
| H | NH{D1-12a($X^{1a}$=Me)} |
| H | NH{D1-12b($X^{1a}$=Me)} |
| H | NH{D1-12c($X^{1a}$=Me)} |
| H | NH(D1-13a) |
| H | NH(D1-13b) |
| H | NH(D1-14a) |
| H | NH(D1-14b) |
| H | NH(D1-15a) |
| H | NH(D1-15b) |
| H | NH(D1-16a) |
| H | NH(D1-17a) |
| H | NH(D1-18a) |
| H | NH(D1-19a) |
| H | NH(D1-20a) |
| H | NH{D1-21a($X^{1a}$=Me)} |
| H | NH{D1-21b($X^{1a}$=Me)} |
| H | NH(D1-22a) |
| H | NH{D1-23a($X^{1a}$=Me)} |
| H | NH{D1-23b($X^{1a}$=Me)} |
| H | NH(D1-24a) |
| H | NH{D1-25a($X^{1a}$=Me)} |
| H | NH(D1-26a) |
| H | NH{D1-27a($X^{1a}$=Me)} |
| H | NH(D1-28a) |
| H | NH(D1-29a) |
| H | NH{D1-30a($X^{1a}$=Me)} |
| H | NH{D1-31a($X^{1a}$=Me)} |
| H | NH(D1-32a) |
| H | NH(D1-33a) |
| H | NH(D1-34a) |
| H | NH(D1-35a) |
| H | NH(D1-35b) |
| H | NH(D1-36a) |
| H | NH(D1-37a) |
| H | NH(D1-38a) |
| H | NH(D1-39a) |
| H | NH(D1-40a) |
| H | NH(D1-40b) |
| H | NH(D1-40c) |
| H | NH(D1-41a) |
| H | NH(D1-42a) |
| H | NH(D1-43a) |
| H | NH(D1-45a) |
| H | NH(D1-50a) |
| H | NH(D1-51a) |

(continued)

| $R^a$ | $R^{b-1}$ |
|---|---|
| H | NH(D1-59a) |
| H | NH(D1-61a) |
| H | NH(D1-79a) |
| H | NH(D1-80a) |
| H | NH{D1-108b(2-Cl)} |
| H | NH{D1-108b(3-Cl)} |
| H | NH{D1-108b(4-Cl)} |
| H | NH{D1-108b(2-Me)} |
| H | NH{D1-108b(3-Me)} |
| H | NH{D1-108b(4-Me)} |
| H | N(Me)Ph |
| H | N(Me)(D1-1a) |
| H | N(Me)(D1-2a) |
| H | N(Me)(D1-2b) |
| H | N(Me){D1-4a($X^{1a}$=Me)} |
| H | N(Me){D1-4b($X^{1a}$=Me)} |
| H | N(Me)(D1-5a) |
| H | N(Me)(D1-5b) |
| H | N(Me)(D1-5c) |
| H | N(Me)(D1-6a) |
| H | N(Me)(D1-6b) |
| H | N(Me)(D1-6c) |
| H | N(Me)(D1-7a) |
| H | N(Me){D1-8a($X^{1a}$=Me)} |
| H | N(Me){D1-Bb($X^{1a}$=Me)} |
| H | N(Me){D1-8c($X^{1a}$=Me)} |
| H | N(Me)(D1-9a) |
| H | N(Me)(D1-9b) |
| H | N(Me)(D1-9c) |
| H | N(Me)(D1-10a) |
| H | N(Me)(D1-10b) |
| H | N(Me)(D1-10c) |
| H | N(Me)(D1-11a) |
| H | N(Me){D1-12a($X^{1a}$=Me)} |
| H | N(Me){D1-12b($X^{1a}$=Me)} |
| H | N(Me){D1-12c($X^{1a}$=Me)} |
| H | N(Me)(D1-13a) |
| H | N(Me)(D1-13b) |
| H | N(Me)(D1-14a) |
| H | N(Me)(D1-14b) |
| H | N(Me)(D1-15a) |
| H | N(Me)(D1-15b) |
| H | N(Me)(D1-16a) |
| H | N(Me)(D1-17a) |
| H | N(Me)(D1-18a) |
| H | N(Me)(D1-19a) |
| H | N(Me)(D1-20a) |
| H | N(Me){D1-21a($X^{1a}$=Me)} |
| H | N(Me){D1-21b($X^{1a}$=Me)} |

(continued)

| $R^a$ | $R^{b-1}$ |
|---|---|
| H | N(Me)(D1-22a) |
| H | N(Me){D1-23a($X^{1a}$=Me)} |
| H | N(Me){D1-23b($X^{1a}$=Me)} |
| H | N(Me)(D1-24a) |
| H | N(Me){D1-25a($X^{1a}$=Me)} |
| H | N(Me)(D1-26a) |
| H | N(Me){D1-27a($X^{1a}$=Me)} |
| H | N(Me)(D1-28a) |
| H | N(Me)(D1-29a) |
| H | N(Me){D1-30a($X^{1a}$=Me)} |
| H | N(Me){D1-31a($X^{1a}$=Me)} |
| H | N(Me)(D1-32a) |
| H | N(Me)(D1-33a) |
| H | N(Me)(D1-34a) |
| H | N(Me)(D1-35a) |
| H | N(Me)(D1-35b) |
| H | N(Me)(D1-36a) |
| H | N(Me)(D1-37a) |
| H | N(Me)(D1-38a) |
| H | N(Me)(D1-39a) |
| H | N(Me)(D1-40a) |
| H | N(Me)(D1-40b) |
| H | N(Me)(D1-40c) |
| H | N(Me)(D1-41a) |
| H | N(Me)(D1-42a) |
| H | N(Me)(D1-43a) |
| H | N(Me)(D1-45a) |
| H | N(Me)(D1-50a) |
| H | N(Me)(D1-51a) |
| H | N(Me)(D1-59a) |
| H | N(Me)(D1-61a) |
| H | N(Me)(D1-79a) |
| H | N(Me)(D1-80a) |
| H | N(Me){D1-108b(2-Cl)} |
| H | N(Me){D1-108b(3-Cl)} |
| H | N(Me){D1-108b(4-Cl)} |
| H | N(Me){D1-108b(2-Me)} |
| H | N(Me){D1-108b(3-Me)} |
| H | N(Me){D1-108b(4-Me)} |
| H | D1-8c($CH_2CF_3$) |
| H | D1-1b(5-Br) |
| H | D1-2c(5-SMe) |
| H | D1-2c{5-C(O)Me} |
| H | D1-4a(Me) |
| H | D1-5d{5-(Pr-c)} |
| H | D1-5e(3,5-$Me_2$) |
| H | D1-6d(4,5-$Cl_2$) |
| H | D1-9b |
| H | D1-10e(2-$CF_3$) |

(continued)

| $R^a$ | $R^{b-1}$ |
|---|---|
| H | D1-10f{2-($CH_2SCH_3$)-4-$CH_3$} |
| H | D1-12f($X^{1a}$=Me,$X^1$=4-$NO_2$) |
| H | D1-13d(Me) |
| H | D1-19a |
| H | D1-32b(2-Br) |
| H | D1-33b(2-OMe) |
| H | D1-34a |
| H | D1-35b |
| H | D1-38b(4-SMe) |
| H | D1-39b(5-Me) |
| H | D1-45d(5,5-$Me_2$) |
| H | D1-82a |
| H | D1-82b |
| H | D1-82c |
| H | D1-87a |
| H | D1-88a{$X^{1a}$=C(O)Me} |
| H | D1-88a{$X^{1a}$=C(O)OBu-t} |
| H | D1-103b |
| H | D1-103c |
| H | D1-103d |
| H | D1-103e |
| H | $CH_2$(D1-2a) |
| H | $CH_2${D1-5f(3-Me)} |
| H | $CH_2${D1-8f($X^{1a}$=Me,$X^1$ =3,5-$Me_2$)} |
| H | $CH_2${D1-8f($X^{1a}$=Me,$X^1$ =3,5-$Cl_2$)} |
| H | $CH_2${D1-9e(2-Ph,5-Me)} |
| H | $CH_2$(D1-28a) |
| H | $CH_2$(D1-32a) |
| H | $CH_2$(D1-33a) |
| H | $CH_2$(D1-34a) |
| H | $CH_2${D1-33b(6-Cl)} |
| H | $CH_2$(D1-34a) |
| H | $CH_2CH_2${D1-7b(3-$CF_3$,5-Me) |
| H | $CH_2CH_2${D1-7b(3-$CF_3$,5-Pr-c) |
| Me | C(O)Me |
| Me | Me |
| Me | Et |
| Me | Pr-n |
| Me | Pr-i |
| Me | Pr-c |
| Me | Bu-n |
| Me | Bu-i |
| Me | Bu-c |
| Me | Bu-s |
| Me | Bu-t |
| Me | Pen-n |
| Me | Pen-c |
| Me | Hex-n |
| Me | Hex-c |

(continued)

| $R^a$ | $R^{b-1}$ |
|---|---|
| Me | $CH=CH_2$ |
| Me | $C\equiv CH$ |
| Me | $C\equiv CSiMe_3$ |
| Me | $CH_2Br$ |
| Me | $CH_2Br$ |
| Me | CH(Br)Me |
| Me | $CH_2CF_3$ |
| Me | $CH_2CH(OH)CF_3$ |
| Me | $CH_2CN$ |
| Me | CH(Me)CN |
| Me | $CH_2CH_2CN$ |
| Me | $CH(Me)CH_2CN$ |
| Me | $CH_2CH_2CH_2CN$ |
| Me | $CH(Me)CH_2CH_2CN$ |
| Me | $CH_2CH_2CH_2CH_2CN$ |
| Me | $CH(Me)CH_2CH_2CH_2CN$ |
| Me | $CH_2C(O)OMe$ |
| Me | CH(Me)C(O)OMe |
| Me | $CH_2CH_2C(O)OMe$ |
| Me | $CH(Me)CH_2C(O)OMe$ |
| Me | $CH_2CH_2CH_2C(O)OMe$ |
| Me | $CH(Me)CH_2CH_2C(O)OMe$ |
| Me | $CH_2CH_2CH_2CH_2C(O)OMe$ |
| Me | $CH_2C(O)OEt$ |
| Me | CH(Me)C(O)OEt |
| Me | $CH_2CH_2C(O)OEt$ |
| Me | $CH(Me)CH_2C(O)OEt$ |
| Me | $CH_2CH_2CH_2C(O)OEt$ |
| Me | $CH(Me)CH_2CH_2C(O)OEt$ |
| Me | $CH_2CH_2CH_2CH_2C(O)OEt$ |
| Me | $CH_2C(O)NHMe$ |
| Me | $CH_2OMe$ |
| Me | $CH_2OEt$ |
| Me | $CH_2OPr$-n |
| Me | $CH_2OPr$-i |
| Me | $CH_2OPr$-c |
| Me | $CH_2OBu$-n |
| Me | $CH_2OBu$-i |
| Me | $CH_2OBu$-s |
| Me | $CH_2OBu$-t |
| Me | $CH_2OBu$-c |
| Me | $CH_2OPen$-n |
| Me | $CH_2OPen$-c |
| Me | $CH_2OHex$-n |
| Me | $CH_2OHex$-c |
| Me | $CH_2OCH_2Pr$-c |
| Me | $CH_2OCH_2CH=CH_2$ |
| Me | $CH_2OCH_2C\equiv CH$ |
| Me | $CH_2OCH_2OMe$ |

(continued)

| $R^a$ | $R^{b-1}$ |
|---|---|
| Me | $CH_2OCH_2SMe$ |
| Me | $CH_2OCH_2Si(CH_3)_3$ |
| Me | $CH_2OCH_2$(D1-34a) |
| Me | $CH_2OCF_3$ |
| Me | $CH_2OCF_2H$ |
| Me | $CH_2OCF_2Cl$ |
| Me | $CH_2OCF_2Br$ |
| Me | $CH_2OCH_2CF_3$ |
| Me | $CH_2OCH_2CF_2CF_3$ |
| Me | $CH_2OCH_2CN$ |
| Me | $CH_2OC(O)Me$ |
| Me | $CH_2OCH_2C(O)OMe$ |
| Me | $CH_2OCH_2C(O)OEt$ |
| Me | $CH_2ON{=}CHMe$ |
| Me | $CH_2ON{=}C(Me)_2$ |
| Me | CH(Me)OMe |
| Me | CH(Me)OEt |
| Me | CH(Me)OPr-n |
| Me | CH(Me)OPr-i |
| Me | CH(Me)OPr-c |
| Me | CH(Me)OBu-n |
| Me | CH(Me)OBu-i |
| Me | CH(Me)OBu-s |
| Me | CH(Me)OBu-t |
| Me | CH(Me)OBu-c |
| Me | CH(Me)OPen-n |
| Me | CH(Me)OPen-c |
| Me | CH(Me)OHex-n |
| Me | CH(Me)OHex-c |
| Me | $CH(Me)OCH_2$Pr-c |
| Me | $CH(Me)OCH_2CH{=}CH_2$ |
| Me | $CH(Me)OCH_2C{\equiv}CH$ |
| Me | $CH(Me)OCH_2OMe$ |
| Me | $CH(Me)OCH_2SMe$ |
| Me | $CH(Me)OCH_2Si(CH_3)_3$ |
| Me | $CH(Me)OCH_2$(D1-34a) |
| Me | $CH(Me)OCF_3$ |
| Me | $CH(Me)OCF_2H$ |
| Me | $CH(Me)OCF_2Cl$ |
| Me | $CH(Me)OCF_2Br$ |
| Me | $CH(Me)OCH_2CF_3$ |
| Me | $CH(Me)OCH_2CF_2CF_3$ |
| Me | $CH(Me)OCH_2CN$ |
| Me | CH(Me)OC(O)Me |
| Me | $CH(Me)OCH_2C(O)OMe$ |
| Me | $CH(Me)OCH_2C(O)OEt$ |
| Me | CH(Me)ON=CHMe |
| Me | $CH(Me)ON{=}C(Me)_2$ |
| Me | $CH_2SMe$ |

(continued)

| $R^a$ | $R^{b-1}$ |
|---|---|
| Me | $CH_2S(O)Me$ |
| Me | $CH_2S(O)_2Me$ |
| Me | $CH_2SEt$ |
| Me | $CH_2S(O)Et$ |
| Me | $CH_2S(O)_2Et$ |
| Me | $CH_2SCH_2CH=CH_2$ |
| Me | $CH_2S(O)CH_2CH=CH_2$ |
| Me | $CH_2S(O)_2CH_2CH=CH_2$ |
| Me | $CH_2SCH_2CF_3$ |
| Me | $CH_2S(O)CH_2CF_3$ |
| Me | $CH_2S(O)_2CH_2CF_3$ |
| Me | CH(Me)SMe |
| Me | CH(Me)S(O)Me |
| Me | $CH(Me)S(O)_2Me$ |
| Me | CH(Me)SEt |
| Me | CH(Me)S(O)Et |
| Me | $CH(Me)S(O)_2$ Et |
| Me | CH(Me)S(=NCN)Et |
| Me | CH(Me)S(O)(=NCN)Et |
| Me | CH(Me)SPr-n |
| Me | CH(Me)S(O)Pr-n |
| Me | $CH(Me)S(O)_2Pr$-n |
| Me | CH(Me)SPr-i |
| Me | CH(Me)S(O)Pr-i |
| Me | $CH(Me)S(O)_2Pr$-i |
| Me | CH(Me)SBu-t |
| Me | CH(Me)S(O)Bu-t |
| Me | $CH(Me)S(O)_2Bu$-t |
| Me | $CH(Me)SCH_2Pr$-c |
| Me | $CH(Me)S(O)CH_2Pr$-c |
| Me | $CH(Me)S(O)_2CH_2Pr$-c |
| Me | $CH(Me)SCH_2OMe$ |
| Me | $CH(Me)SCH_2SMe$ |
| Me | $CH(Me)S(O)CH_2SMe$ |
| Me | $CH(Me)S(O)_2CH_2SMe$ |
| Me | $CH(Me)SCH_2C\equiv CH$ |
| Me | $CH(Me)S(O)CH_2C\equiv CH$ |
| Me | $CH(Me)S(O)_2CH_2C\equiv CH$ |
| Me | $CH(Me)SCH_2C(O)NHMe$ |
| Me | $CH(Me)S(O)CH_2C(O)NHMe$ |
| Me | $CH(Me)S(O)_2CH_2C(O)NHMe$ |
| Me | $CH(Me)SCH_2C(O)OMe$ |
| Me | $CH(Me)S(O)CH_2C(O)OMe$ |
| Me | $CH(Me)S(O)_2CH_2C(O)OMe$ |
| Me | $CH(Me)SCH_2CF_3$ |
| Me | $CH(Me)S(O)CH_2CF_3$ |
| Me | $CH(Me)S(O)_2CH_2CF_3$ |
| Me | $CH(Me)SCH_2$ (D1-34a) |
| Me | $CH(Me)S(O)CH_2$ (D1-34a) |

(continued)

| $R^a$ | $R^{b-1}$ |
|---|---|
| Me | $CH(Me)S(O)_2CH_2$ (D1-34a) |
| Me | $CH(CH_3)SCH_2Si(CH_3)_3$ |
| Me | CH(Me)SCN |
| Me | $CH(Me)SCH_2CN$ |
| Me | CH(Me)SC(O)Me |
| Me | CH(Me)S(D1-37a) |
| Me | CH(Et)SMe |
| Me | CH(Et)S(O)Me |
| Me | $CH(Et)S(O)_2Me$ |
| Me | CH(Et)SEt |
| Me | CH(Et)S(O)Et |
| Me | $CH(Et)S(O)_2$ Et |
| Me | $CH(Et)SCH_2$ $CF_3$ |
| Me | $CH(Et)S(O)CH_2$ $CF_3$ |
| Me | $CH(Et)S(O)_2$ $CH_2$ $CF_3$ |
| Me | CH(F)SMe |
| Me | CH(F)S(O)Me |
| Me | $CH(F)S(O)_2Me$ |
| Me | CH(F)SEt |
| Me | CH(F)S(O)Et |
| Me | $CH(F)S(O)_2Et$ |
| Me | CH{OC(O)Me}SMe |
| Me | CH{OC(O)Me}S(O)Me |
| Me | $CH\{OC(O)Me\}S(O)_2Me$ |
| Me | $CH(SMe)_2$ |
| Me | $C(Me)_2SMe$ |
| Me | $C(Me)_2S(O)Me$ |
| Me | $C(Me)_2S(O)_2Me$ |
| Me | $CH_2CH_2SH$ |
| Me | $CH_2CH_2SMe$ |
| Me | $CH_2CH_2S(O)Me$ |
| Me | $CH_2CH_2S(O)_2Me$ |
| Me | $CH_2CH_2SEt$ |
| Me | $CH_2CH_2S(O)Et$ |
| Me | $CH_2CH_2S(O)_2Et$ |
| Me | $CH_2CH_2SPr$-n |
| Me | $CH_2CH_2S(O)Pr$-n |
| Me | $CH_2CH_2S(O)_2Pr$-n |
| Me | $CH_2CH_2SPr$-i |
| Me | $CH_2CH_2S(O)Pr$-i |
| Me | $CH_2CH_2S(O)_2Pr$-i |
| Me | $CH(Me)SCH_2CH_2CH_2Cl$ |
| Me | $CH(Me)S(O)CH_2CH_2CH_2Cl$ |
| Me | $CH(Me)S(O)_2CH_2CH_2CH_2Cl$ |
| Me | $CH_2CH_2SCH_2CF_3$ |
| Me | $CH_2CH_2S(O)CH_2CF_3$ |
| Me | $CH_2CH_2S(O)_2CH_2CF_3$ |
| Me | $CH_2CH_2SC(O)Me$ |
| Me | $CH(Me)CH_2SMe$ |

(continued)

| $R^a$ | $R^{b-1}$ |
|---|---|
| Me | $CH(Me)CH_2S(O)Me$ |
| Me | $CH(Me)CH_2S(O)_2Me$ |
| Me | $CH(Me)CH_2SEt$ |
| Me | $CH(Me)CH_2S(O)Et$ |
| Me | $CH(Me)CH_2S(O)_2Et$ |
| Me | $CH(Me)CH_2SPr$-n |
| Me | $CH(Me)CH_2S(O)Pr$-n |
| Me | $CH(Me)CH_2S(O)_2Pr$-n |
| Me | $CH(Me)CH_2SPr$-i |
| Me | $CH(Me)CH_2S(O)Pr$-i |
| Me | $CH(Me)CH_2S(O)_2Pr$-i |
| Me | $CH(Me)CH_2SCH_2CF_3$ |
| Me | $CH(Me)CH_2S(O)CH_2CF_3$ |
| Me | $CH(Me)CH_2S(O)_2CH_2CF_3$ |
| Me | $CH(Me)CH_2SC(O)Me$ |
| Me | $CH(Et)CH_2SMe$ |
| Me | $CH(Et)CH_2S(O)Me$ |
| Me | $CH(Et)CH_2S(O)_2Me$ |
| Me | $CH(OMe)CH_2SMe$ |
| Me | $CH(OMe)CH_2S(O)Me$ |
| Me | $CH(OMe)CH_2S(O)_2Me$ |
| Me | $CH(SMe)CH_2SMe$ |
| Me | $CH(SMe)CH_2S(O)Me$ |
| Me | $CH(SMe)CH_2S(O)_2Me$ |
| Me | $CH(CH_2SMe)CH_2SMe$ |
| Me | $CH(CH_2SMe)CH_2S(O)Me$ |
| Me | $CH(CH_2SMe)CH_2S(O)_2Me$ |
| Me | $CH(Cl)CH_2SMe$ |
| Me | $CH(Cl)CH_2S(O)Me$ |
| Me | $CH(Cl)CH_2S(O)_2Me$ |
| Me | $CH(CN)CH_2SMe$ |
| Me | $CH(CN)CH_2S(O)Me$ |
| Me | $CH(CN)CH_2S(O)_2Me$ |
| Me | $CH(CN)CH_2SEt$ |
| Me | $CH(CN)CH_2S(O)Et$ |
| Me | $CH(CN)CH_2S(O)_2Et$ |
| Me | $CH\{C(O)OMe\}CH_2SMe$ |
| Me | $CH\{C(O)OMe\}CH_2S(O)Me$ |
| Me | $CH\{C(O)OMe\}CH_2S(O)_2Me$ |
| Me | $CH\{C(O)OMe\}CH_2SEt$ |
| Me | $CH\{C(O)OMe\}CH_2S(O)Et$ |
| Me | $CH\{C(O)OMe\}CH_2S(O)_2Et$ |
| Me | $CH(Ph)CH_2SMe$ |
| Me | $CH(Ph)CH_2S(O)Me$ |
| Me | $CH(Ph)CH_2S(O)_2Me$ |
| Me | $C(Me)_2CH_2SMe$ |
| Me | $C(Me)_2CH_2S(O)Me$ |
| Me | $C(Me)_2CH_2S(O)_2Me$ |
| Me | $C(=CH_2)CH_2SMe$ |

(continued)

| $R^a$ | $R^{b-1}$ |
|---|---|
| Me | $C(=CH_2)CH_2S(O)Me$ |
| Me | $C(=CH_2)CH_2S(O)_2Me$ |
| Me | $C(=CH_2)CH_2SEt$ |
| Me | $C(=CH_2)CH_2S(O)Et$ |
| Me | $C(=CH_2)CH_2S(O)_2Et$ |
| Me | $CH_2CH_2CH_2SEt$ |
| Me | $CH_2CH_2CH_2S(O)Et$ |
| Me | $CH_2CH_2CH_2S(O)_2Et$ |
| Me | $CH(Me)CH_2CH_2SEt$ |
| Me | $CH(Me)CH_2CH_2S(O)Et$ |
| Me | $CH(Me)CH_2CH_2S(O)_2Et$ |
| Me | $CH_2CH(Me)CH_2SEt$ |
| Me | $CH_2CH(Me)CH_2S(O)Et$ |
| Me | $CH_2CH(Me)CH_2S(O)_2Et$ |
| Me | $CH_2CH_2CH(Me)SEt$ |
| Me | $CH_2CH_2CH(Me)S(O)Et$ |
| Me | $CH_2CH_2CH(Me)S(O)_2Et$ |
| Me | $CH_2CH_2CH_2SPr$-n |
| Me | $CH_2CH_2CH_2S(O)Pr$-n |
| Me | $CH_2CH_2CH_2S(O)_2Pr$-n |
| Me | $CH(Me)CH_2CH_2SPr$-n |
| Me | $CH(Me)CH_2CH_2S(O)Pr$-n |
| Me | $CH(Me)CH_2CH_2S(O)_2Pr$-n |
| Me | $CH_2CH(Me)CH_2SPr$-n |
| Me | $CH_2CH(Me)CH_2S(O)Pr$-n |
| Me | $CH_2CH(Me)CH_2S(O)_2Pr$-n |
| Me | $CH_2CH_2CH_2CH_2SMe$ |
| Me | $CH_2CH_2CH_2CH_2S(O)Me$ |
| Me | $CH_2CH_2CH_2CH_2S(O)_2Me$ |
| Me | $CH_2CH_2CH_2CH_2SEt$ |
| Me | $CH_2CH_2CH_2CH_2S(O)Et$ |
| Me | $CH_2CH_2CH_2CH_2S(O)_2Et$ |
| Me | $CH_2CH_2CH_2CH_2SPr$-n |
| Me | $CH_2CH_2CH_2CH_2S(O)Pr$-n |
| Me | $CH_2CH_2CH_2CH_2S(O)_2Pr$-n |
| Me | $CH_2CH(Me)SMe$ |
| Me | $CH_2OCH_2SMe$ |
| Me | CH=CHSMe |
| Me | $CH_2CH(SMe)_2$ |
| Me | $CH_2CH_2SC(O)Me$ |
| Me | $C(=CH_2)CH_2C(O)OH$ |
| Me | $C(=NOMe)CH_3$ |
| Me | C(=NOMe)Et |
| Me | C(=NOMe)Pr-n |
| Me | $C(=NOEt)CH_3$ |
| Me | $C(=NOPr\text{-}n)CH_3$ |
| Me | $C(=NOPr\text{-}i)CH_3$ |
| Me | $C(=NOBu\text{-}n)CH_3$ |
| Me | $C(=NOCH_2Ph)CH_3$ |

(continued)

| $R^a$ | $R^{b-1}$ |
|---|---|
| Me | $C(=NOMe)CH_2OCH_2CF_3$ |
| Me | $C(=NOMe)CH_2SMe$ |
| Me | $C(=NOMe)CH_2S(O)Me$ |
| Me | $C(=NOMe)CH_2S(O)_2Me$ |
| Me | $C(=NOMe)CH_2SEt$ |
| Me | $C(=NOMe)CH_2S(O)Et$ |
| Me | $C(=NOMe)CH_2S(O)_2Et$ |
| Me | $C(=NOMe)CH_2SCF_3$ |
| Me | $C(=NOMe)CH_2S(O)CF_3$ |
| Me | $C(=NOMe)CH_2S(O)_2CF_3$ |
| Me | $C(=NOMe)CH_2SCH_2CF_3$ |
| Me | $C(=NOMe)CH_2S(O)CH_2CF_3$ |
| Me | $C(=NOMe)CH_2S(O)_2CH_2CF_3$ |
| Me | $C(=NOEt)CH_2SMe$ |
| Me | $C(=NOEt)CH_2S(O)Me$ |
| Me | $C(=NOEt)CH_2S(O)_2Me$ |
| Me | $C(=NOPr\text{-}n)CH_2SMe$ |
| Me | $C(=NOPr\text{-}n)CH_2S(O)Me$ |
| Me | $C(=NOPr\text{-}n)CH_2S(O)_2Me$ |
| Me | $C(=NOPr\text{-}i)CH_2SMe$ |
| Me | $C(=NOPr\text{-}i)CH_2S(O)Me$ |
| Me | $C(=NOPr\text{-}i)CH_2S(O)_2Me$ |
| Me | $C(=NOBu\text{-}n)CH_2SMe$ |
| Me | $C(=NOBu\text{-}n)CH_2S(O)Me$ |
| Me | $C(=NOBu\text{-}n)CH_2S(O)_2Me$ |
| Me | $C(=NOCH_2Ph)CH_2SMe$ |
| Me | $C(=NOCH_2Ph)CH_2S(O)Me$ |
| Me | $C(=NOCH_2Ph)CH_2S(O)_2Me$ |
| Me | $C(=NOCH_2CH=CH_2)CH_2SMe$ |
| Me | $C(=NOCH_2OMe)CH_2SMe$ |
| Me | $C(=NOCH_2CH_2OMe)CH_2SMe$ |
| Me | $C\{=NOCH_2CH_2Si(Me)_3\}CH_2SMe$ |
| Me | $CH_2CH(=NOMe)$ |
| Me | $CH_2C(=NOMe)Me$ |
| Me | $CH_2C(=NOMe)Et$ |
| Me | $CH_2C(=NOMe)Pr\text{-}n$ |
| Me | $CH_2C(=NOMe)Pr\text{-}i$ |
| Me | $CH_2C(=NOMe)Bu\text{-}n$ |
| Me | $CH_2C(=NOMe)Bu\text{-}i$ |
| Me | $CH_2C(=NOMe)Bu\text{-}s$ |
| Me | $CH_2C(=NOMe)Bu\text{-}t$ |
| Me | $CH_2C(=NOMe)CF_3$ |
| Me | $CH_2C(=NOCH_3)CH_2SCH_3$ |
| Me | $CH(SMe)C(=NOMe)Me$ |
| Me | $CH\{S(O)Me\}C(=NOMe)Me$ |
| Me | $CH\{S(O)_2Me\}C(=NOMe)Me$ |
| Me | $CH(SEt)C(=NOMe)Me$ |
| Me | $CH\{S(O)Et\}C(=NOMe)Me$ |
| Me | $CH\{S(O)_2Et\}C(=NOMe)Me$ |

(continued)

| $R^a$ | $R^{b-1}$ |
|---|---|
| Me | $CH_2NH_2$ |
| Me | $CH_2NHC(O)Me$ |
| Me | $CH_2NHC(O)OMe$ |
| Me | $CH_2NHC(O)OBu$-t |
| Me | $CH_2NHSO_2Me$ |
| Me | $CH_2N(Me)C(O)Me$ |
| Me | $CH_2N(Me)C(O)OMe$ |
| Me | $CH_2N(Me)C(O)OBu$-t |
| Me | $CH_2N(Me)SO_2Me$ |
| Me | $CH(CH_2SMe)NH_2$ |
| Me | $CH(CH_2SMe)NHC(O)Me$ |
| Me | $CH(CH_2SMe)NHC(O)OMe$ |
| Me | $CH(CH_2SMe)NHC(O)OBu$-t |
| Me | $CH(CH_2SMe)NHSO_2Me$ |
| Me | $CH_2P(O)(OMe)_2$ |
| Me | $CH_2P(O)(OEt)_2$ |
| Me | OBu-t |
| Me | SMe |
| Me | $NH_2$ |
| Me | $NHCH_2CH_2SMe$ |
| Me | $N(Me)CH_2CH_2SMe$ |
| Me | $NHCH_2CH_2CH_2SMe$ |
| Me | NHMe |
| Me | NHEt |
| Me | NPr-n |
| Me | $NHCH(Me)_2$ |
| Me | $NHCH_2C(O)OMe$ |
| Me | $NHCH_2CF_3$ |
| Me | $N(Me)CH_2CF_3$ |
| Me | NHC(O)OEt |
| Me | $NHC(O)CCl_3$ |
| Me | $NHC(O)CH_2SMe$ |
| Me | $N(CH_2CH_3)C(O)CH_2SCH_3$ |
| Me | $NHS(O)_2$(D1-108a) |
| Me | NHPh |
| Me | NH(D1-1a) |
| Me | NH(D1-2a) |
| Me | NH(D1-2b) |
| Me | NH{D1-4a($X^{1a}$=Me)} |
| Me | NH{D1-4b($X^{1a}$=Me)} |
| Me | NH(D1-5a) |
| Me | NH(D1-5b) |
| Me | NH(D1-5c) |
| Me | NH(D1-6a) |
| Me | NH(D1-6b) |
| Me | NH(D1-6c) |
| Me | NH(D1-7a) |
| Me | NH{D1-Ba($X^{1a}$=Me)} |
| Me | NH{D1-8b($X^{1a}$=Me)} |

(continued)

| $R^a$ | $R^{b-1}$ |
|---|---|
| Me | NH{D1-8c($X^{1a}$=Me)} |
| Me | NH(D1-9a) |
| Me | NH(D1-9b) |
| Me | NH(D1-9c) |
| Me | NH(D1-10a) |
| Me | NH(D1-10b) |
| Me | NH(D1-10c) |
| Me | NH(D1-11a) |
| Me | NH{D1-12a($X^{1a}$=Me)} |
| Me | NH{D1-12b($X^{1a}$=Me)} |
| Me | NH{D1-12c($X^{1a}$=Me)} |
| Me | NH(D1-13a) |
| Me | NH(D1-13b) |
| Me | NH(D1-14a) |
| Me | NH(D1-14b) |
| Me | NH(D1-15a) |
| Me | NH(D1-15b) |
| Me | NH(D1-16a) |
| Me | NH(D1-17a) |
| Me | NH(D1-18a) |
| Me | NH(D1-19a) |
| Me | NH(D1-20a) |
| Me | NH{D1-21a($X^{1a}$=Me)} |
| Me | NH{D1-21b($X^{1a}$=Me)} |
| Me | NH(D1-22a) |
| Me | NH{D1-23a($X^{1a}$=Me)} |
| Me | NH{D1-23b($X^{1a}$=Me)} |
| Me | NH(D1-24a) |
| Me | NH{D1-25a($X^{1a}$=Me)} |
| Me | NH(D1-26a) |
| Me | NH{D1-27a($X^{1a}$=Me)} |
| Me | NH(D1-28a) |
| Me | NH(D1-29a) |
| Me | NH{D1-30a($X^{1a}$=Me)} |
| Me | NH{D1-31a($X^{1a}$=Me)} |
| Me | NH(D1-32a) |
| Me | NH(D1-33a) |
| Me | NH(D1-34a) |
| Me | NH(D1-35a) |
| Me | NH(D1-35b) |
| Me | NH(D1-36a) |
| Me | NH(D1-37a) |
| Me | NH(D1-38a) |
| Me | NH(D1-39a) |
| Me | NH(D1-40a) |
| Me | NH(D1-40b) |
| Me | NH(D1-40c) |
| Me | NH(D1-41a) |
| Me | NH(D1-42a) |

(continued)

| $R^a$ | $R^{b-1}$ |
|---|---|
| Me | NH(D1-43a) |
| Me | NH(D1-45a) |
| Me | NH(D1-50a) |
| Me | NH(D1-51a) |
| Me | NH(D1-59a) |
| Me | NH(D1-61a) |
| Me | NH(D1-79a) |
| Me | NH(D1-80a) |
| Me | NH{D1-108b(2-Cl)} |
| Me | NH{D1-108b(3-Cl)} |
| Me | NH{D1-108b(4-Cl)} |
| Me | NH{D1-108b(2-Me)} |
| Me | NH{D1-108b(3-Me)} |
| Me | NH{D1-108b(4-Me)} |
| Me | N(Me)Ph |
| Me | N(Me)(D1-1a) |
| Me | N(Me)(D1-2a) |
| Me | N(Me)(D1-2b) |
| Me | N(Me){D1-4a($X^{1a}$=Me)} |
| Me | N(Me){D1-4b($X^{1a}$=Me)} |
| Me | N(Me)(D1-5a) |
| Me | N(Me)(D1-5b) |
| Me | N(Me)(D1-5c) |
| Me | N(Me)(D1-6a) |
| Me | N(Me)(D1-6b) |
| Me | N(Me)(D1-6c) |
| Me | N(Me)(D1-7a) |
| Me | N(Me){D1-8a($X^{1a}$=Me)} |
| Me | N(Me){D1-8b($X^{1a}$=Me)} |
| Me | N(Me){D1-8c($X^{1a}$=Me)} |
| Me | N(Me)(D1-9a) |
| Me | N(Me)(D1-9b) |
| Me | N(Me)(D1-9c) |
| Me | N(Me)(D1-10a) |
| Me | N(Me)(D1-10b) |
| Me | N(Me)(D1-10c) |
| Me | N(Me)(D1-11a) |
| Me | N(Me){D1-12a($X^{1a}$=Me)} |
| Me | N(Me){D1-12b($X^{1a}$=Me)} |
| Me | N(Me){D1-12c($X^{1a}$=Me)} |
| Me | N(Me)(D1-13a) |
| Me | N(Me)(D1-13b) |
| Me | N(Me)(D1-14a) |
| Me | N(Me)(D1-14b) |
| Me | N(Me)(D1-15a) |
| Me | N(Me)(D1-15b) |
| Me | N(Me)(D1-16a) |
| Me | N(Me)(D1-17a) |
| Me | N(Me)(D1-18a) |

(continued)

| $R^a$ | $R^{b-1}$ |
|---|---|
| Me | N(Me)(D1-19a) |
| Me | N(Me)(D1-20a) |
| Me | N(Me){D1-21a($X^{1a}$=Me)} |
| Me | N(Me){D1-21b($X^{1a}$=Me)} |
| Me | N(Me)(D1-22a) |
| Me | N(Me){D1-23a($X^{1a}$=Me)} |
| Me | N(Me){D1-23b($X^{1a}$=Me)} |
| Me | N(Me)(D1-24a) |
| Me | N(Me){D1-25a($X^{1a}$=Me)} |
| Me | N(Me)(D1-26a) |
| Me | N(Me){D1-27a($X^{1a}$=Me)} |
| Me | N(Me)(D1-28a) |
| Me | N(Me)(D1-29a) |
| Me | N(Me){D1-30a($X^{1a}$=Me)} |
| Me | N(Me){D1-31a($X^{1a}$=Me)} |
| Me | N(Me)(D1-32a) |
| Me | N(Me)(D1-33a) |
| Me | N(Me)(D1-34a) |
| Me | N(Me)(D1-35a) |
| Me | N(Me)(D1-35b) |
| Me | N(Me)(D1-36a) |
| Me | N(Me)(D1-37a) |
| Me | N(Me)(D1-38a) |
| Me | N(Me)(D1-39a) |
| Me | N(Me)(D1-40a) |
| Me | N(Me)(D1-40b) |
| Me | N(Me)(D1-40c) |
| Me | N(Me)(D1-41a) |
| Me | N(Me)(D1-42a) |
| Me | N(Me)(D1-43a) |
| Me | N(Me)(D1-45a) |
| Me | N(Me)(D1-50a) |
| Me | N(Me)(D1-51a) |
| Me | N(Me)(D1-59a) |
| Me | N(Me)(D1-61a) |
| Me | N(Me)(D1-79a) |
| Me | N(Me)(D1-80a) |
| Me | N(Me){D1-108b(2-Cl)} |
| Me | N(Me){D1-108b(3-Cl)} |
| Me | N(Me){D1-108b(4-Cl)} |
| Me | N(Me){D1-108b(2-Me)} |
| Me | N(Me){D1-108b(3-Me)} |
| Me | N(Me){D1-108b(4-Me)} |
| Me | D1-8c($CH_2CF_3$) |
| Me | D1-1b(5-Br) |
| Me | D1-2c(5-SMe) |
| Me | D1-2c{5-C(O)Me} |
| Me | D1-4a(Me) |
| Me | D1-5d{5-(Pr-c)} |

(continued)

| $R^a$ | $R^{b-1}$ |
|---|---|
| Me | D1-5e(3,5-$Me_2$) |
| Me | D1-6d(4,5-$Cl_2$) |
| Me | D1-9b |
| Me | D1-10e(2-$CF_3$) |
| Me | D1-10f{2-($CH_2SCH_3$)-4-$CH_3$} |
| Me | D1-12f($X^{1a}$=Me,$X^1$=4-$NO_2$) |
| Me | D1-13d(Me) |
| Me | D1-19a |
| Me | D1-32b(2-Br) |
| Me | D1-33b(2-OMe) |
| Me | D1-35b |
| Me | D1-38b(4-SMe) |
| Me | D1-39b(5-Me) |
| Me | D1-45d(5,5-$Me_2$) |
| Me | D1-82a |
| Me | D1-82b |
| Me | D1-82c |
| Me | D1-87a |
| Me | D1-88a{$X^{1a}$=C(O)Me} |
| Me | D1-88a{$X^{1a}$=C(O)OBu-t) |
| Me | D1-103b |
| Me | D1-103c |
| Me | D1-103d |
| Me | D1-103e |
| Me | $CH_2$(D1-2a) |
| Me | $CH_2${D1-5f(3-Me)} |
| Me | $CH_2${D1-8f($X^{1a}$=Me,$X^1$ =3,5-$Me_2$)} |
| Me | $CH_2${D1-8f($X^{1a}$=Me,$X^1$ =3,5-$Cl_2$)} |
| Me | $CH_2${D1-9e(2-Ph,5-Me)} |
| Me | $CH_2$(D1-28a) |
| Me | $CH_2$(D1-32a) |
| Me | $CH_2$(D1-33a) |
| Me | $CH_2${D1-33b(6-Cl)} |
| Me | $CH_2$(D1-34a) |
| Me | $CH_2CH_2${D1-7b(3-$CF_3$,5-Me) |
| Me | $CH_2CH_2${D1-7b(3-$CF_3$,5-Pr-c) |
| Et | C(O)(D1-2a) |
| Et | C(O)Me |
| Et | C(O)Et |
| Et | C(O)Pr-n |
| Et | C(O)Pr-i |
| Et | C(O)Pr-c |
| Et | C(O)Bu-n |
| Et | C(O)Bu-i |
| Et | C(O)Bu-s |
| Et | C(O)Bu-t |
| Et | C(O)Pen-n |
| Et | C(O)Pen-c |
| Et | C(O)Hex-n |

(continued)

| $R^a$ | $R^{b-1}$ |
|---|---|
| Et | C(O)Hex-c |
| Et | $C(O)CF_3$ |
| Et | C(O)OMe |
| Et | C(O)OEt |
| Et | C(O)NHPh |
| Et | Me |
| Et | Et |
| Et | Pr-n |
| Et | Pr-i |
| Et | Pr-c |
| Et | Bu-n |
| Et | Bu-i |
| Et | Bu-c |
| Et | Bu-s |
| Et | Bu-t |
| Et | Pen-n |
| Et | Pen-c |
| Et | Hex-n |
| Et | Hex-c |
| Et | $(CH_2)_6CH_3$ |
| Et | $(CH_2)_7CH_3$ |
| Et | $(CH_2)_8CH_3$ |
| Et | $(CH_2)_9CH_3$ |
| Et | $(CH_2)_{10}CH_3$ |
| Et | $(CH_2)_{11}CH_3$ |
| Et | $CH(Me)(CH_2)_2CH_3$ |
| Et | $CH(Me)(CH_2)_3CH_3$ |
| Et | $CH(Me)(CH_2)_4CH_3$ |
| Et | $CH(Me)(CH_2)_5CH_3$ |
| Et | $CH(Me)(CH_2)_6CH_3$ |
| Et | $CH(Me)(CH_2)_7CH_3$ |
| Et | $CH(Me)(CH_2)_8CH_3$ |
| Et | $CH_2$Pr-c |
| Et | $CH_2$Bu-t |
| Et | $CH=CH_2$ |
| Et | $C(Me)=CH_2$ |
| Et | CH=CH(D1-1a) |
| Et | CH=CH(D1-2a) |
| Et | CH=CH(D1-33a) |
| Et | CH=CH{D1-108b(4-$CF_3$)} |
| Et | C≡CH |
| Et | $C≡CSiMe_3$ |
| Et | $CHCl_2$ |
| Et | $CH_2Br$ |
| Et | CH(Br)Me |
| Et | $CH_2CH_2CH_2CH_2Br$ |
| Et | CH(Br)Bu-t |
| Et | $CH_2CF_3$ |
| Et | $CF_2CF_2Cl$ |

(continued)

| $R^a$ | $R^{b-1}$ |
|---|---|
| Et | $CH_2CH(CF_3)_2$ |
| Et | $CH_2CH(OH)CF_3$ |
| Et | $CH_2CH_2C(F)=CF_2$ |
| Et | $CH_2CN$ |
| Et | CH(Me)CN |
| Et | $CH_2CH_2CN$ |
| Et | $CH(Me)CH_2CN$ |
| Et | $CH_2CH_2CH_2CN$ |
| Et | $CH(Me)CH_2CH_2CN$ |
| Et | $CH_2CH_2CH_2CH_2CN$ |
| Et | $CH(Me)CH_2CH_2CH_2CN$ |
| Et | $CH_2C(O)OMe$ |
| Et | CH(Me)C(O)OMe |
| Et | $CH_2CH_2C(O)OMe$ |
| Et | $CH(Me)CH_2C(O)OMe$ |
| Et | $CH_2CH_2CH_2C(O)OMe$ |
| Et | $CH(Me)CH_2CH_2C(O)OMe$ |
| Et | $CH_2CH_2CH_2CH_2C(O)OMe$ |
| Et | $CH_2C(O)OEt$ |
| Et | CH(Me)C(O)OEt |
| Et | $CH_2CH_2C(O)OEt$ |
| Et | $CH(Me)CH_2C(O)OEt$ |
| Et | $CH_2CH_2CH_2C(O)OEt$ |
| Et | $CH(Me)CH_2CH_2C(O)OEt$ |
| Et | $CH_2CH_2CH_2CH_2C(O)OEt$ |
| Et | $CH_2C(O)NHMe$ |
| Et | $CH_2C(O)NHCH_2CF_3$ |
| Et | $CH_2OMe$ |
| Et | $CH_2OEt$ |
| Et | $CH_2OPr$-n |
| Et | $CH_2OPr$-i |
| Et | $CH_2OPr$-c |
| Et | $CH_2OBu$-n |
| Et | $CH_2OBu$-i |
| Et | $CH_2OBu$-s |
| Et | $CH_2OBu$-t |
| Et | $CH_2OBu$-c |
| Et | $CH_2OPen$-n |
| Et | $CH_2OPen$-c |
| Et | $CH_2OHex$-n |
| Et | $CH_2OHex$-c |
| Et | $CH_2OCH_2Pr$-c |
| Et | $CH_2OCH_2CH=CH_2$ |
| Et | $CH_2OCH_2C\equiv CH$ |
| Et | $CH_2OCH_2OMe$ |
| Et | $CH_2OCH_2SMe$ |
| Et | $CH_2OCH_2Si(CH_3)_3$ |
| Et | $CH_2OCH_2$(D1-34a) |
| Et | $CH_2OCF_3$ |

(continued)

| $R^a$ | $R^{b-1}$ |
|---|---|
| Et | $CH_2OCF_2H$ |
| Et | $CH_2OCF_2Cl$ |
| Et | $CH_2OCF_2Br$ |
| Et | $CH_2OCH_2CF_3$ |
| Et | $CH_2OCH_2CF_2CF_3$ |
| Et | $CH_2OCH_2CN$ |
| Et | $CH_2OC(O)Me$ |
| Et | $CH_2OCH_2C(O)OMe$ |
| Et | $CH_2OCH_2C(O)OEt$ |
| Et | $CH_2ON=CHMe$ |
| Et | $CH_2ON=C(Me)_2$ |
| Et | CH(Me)OMe |
| Et | CH(Me)OEt |
| Et | CH(Me)OPr-n |
| Et | CH(Me)OPr-i |
| Et | CH(Me)OPr-c |
| Et | CH(Me)OBu-n |
| Et | CH(Me)OBu-i |
| Et | CH(Me)OBu-s |
| Et | CH(Me)OBu-t |
| Et | CH(Me)OBu-c |
| Et | CH(Me)OPen-n |
| Et | CH(Me)OPen-c |
| Et | CH(Me)OHex-n |
| Et | CH(Me)OHex-c |
| Et | $CH(Me)OCH_2Pr\text{-}c$ |
| Et | $CH(Me)OCH_2CH=CH_2$ |
| Et | $CH(Me)OCH_2C\equiv CH$ |
| Et | $CH(Me)OCH_2OMe$ |
| Et | $CH(Me)OCH_2SMe$ |
| Et | $CH(Me)OCH_2Si(CH_3)_3$ |
| Et | $CH(Me)OCH_2$(D1-34a) |
| Et | $CH(Me)OCF_3$ |
| Et | $CH(Me)OCF_2H$ |
| Et | $CH(Me)OCF_2Cl$ |
| Et | $CH(Me)OCF_2Br$ |
| Et | $CH(Me)OCH_2CF_3$ |
| Et | $CH(Me)OCH_2CF_2CF_3$ |
| Et | $CH(Me)OCH_2CN$ |
| Et | CH(Me)OC(O)Me |
| Et | $CH(Me)OCH_2C(O)OMe$ |
| Et | $CH(Me)OCH_2C(O)OEt$ |
| Et | CH(Me)ON=CHMe |
| Et | $CH(Me)ON=C(Me)_2$ |
| Et | $C(Me)_2OC(O)Me$ |
| Et | $CH_2SMe$ |
| Et | $CH_2S(O)Me$ |
| Et | $CH_2S(O)_2Me$ |
| Et | $CH_2SEt$ |

(continued)

| $R^a$ | $R^{b-1}$ |
|---|---|
| Et | $CH_2S(O)Et$ |
| Et | $CH_2S(O)_2Et$ |
| Et | $CH_2SPr$-n |
| Et | $CH_2S(O)Pr$-n |
| Et | $CH_2S(O)_2Pr$-n |
| Et | $CH_2SPr$-i |
| Et | $CH_2S(O)Pr$-i |
| Et | $CH_2S(O)_2Pr$-i |
| Et | $CH_2SPr$-c |
| Et | $CH_2S(O)Pr$-c |
| Et | $CH_2S(O)_2Pr$-c |
| Et | $CH_2SCH_2CH=CH_2$ |
| Et | $CH_2S(O)CH_2CH=CH_2$ |
| Et | $CH_2S(O)_2CH_2CH=CH_2$ |
| Et | $CH_2SCH_2C\equiv CH$ |
| Et | $CH_2S(O)CH_2C\equiv CH$ |
| Et | $CH_2S(O)_2CH_2C\equiv CH$ |
| Et | $CH_2SCH_2CF_3$ |
| Et | $CH_2S(O)CH_2CF_3$ |
| Et | $CH_2S(O)_2CH_2CF_3$ |
| Et | $CH_2SCH_2CH(OMe)_2$ |
| Et | $CH_2SCH_2CH(=NOMe)$ |
| Et | CH(Me)SMe |
| Et | CH(Me)S(O)Me |
| Et | $CH(Me)S(O)_2Me$ |
| Et | CH(Me)SEt |
| Et | CH(Me)S(O)Et |
| Et | $CH(Me)S(O)_2Et$ |
| Et | CH(Me)S(=NCN)Et |
| Et | CH(Me)S(O)(=NCN)Et |
| Et | CH(Me)SPr-n |
| Et | CH(Me)S(O)Pr-n |
| Et | $CH(Me)S(O)_2Pr$-n |
| Et | CH(Me)SPr-i |
| Et | CH(Me)S(O)Pr-i |
| Et | $CH(Me)S(O)_2Pr$-i |
| Et | CH(Me)SBu-t |
| Et | CH(Me)S(O)Bu-t |
| Et | $CH(Me)S(O)_2Bu$-t |
| Et | $CH(Me)SCH_2Pr$-c |
| Et | $CH(Me)S(O)CH_2Pr$-c |
| Et | $CH(Me)S(O)_2CH_2Pr$-c |
| Et | $CH(Me)SCH_2OMe$ |
| Et | $CH(Me)SCH_2SMe$ |
| Et | $CH(Me)S(O)CH_2SMe$ |
| Et | $CH(Me)S(O)_2CH_2SMe$ |
| Et | $CH(Me)SCH_2C=CH_2$ |
| Et | $CH(Me)S(O)CH_2C=CH_2$ |
| Et | $CH(Me)S(O)_2CH_2C=CH_2$ |

(continued)

| $R^a$ | $R^{b-1}$ |
|---|---|
| Et | CH(Me)SCH2C≡CH |
| Et | $CH(Me)S(O)CH_2C{\equiv}CH$ |
| Et | $CH(Me)S(O)_2CH_2C{=}CH$ |
| Et | $CH(Me)SCH_2C(O)NHMe$ |
| Et | $CH(Me)S(O)CH_2C(O)NHMe$ |
| Et | $CH(Me)S(O)_2CH_2C(O)NHMe$ |
| Et | $CH(Me)SCH_2C(O)OMe$ |
| Et | $CH(Me)S(O)CH_2\ C(O)OMe$ |
| Et | $CH(Me)S(O)_2CH_2C(O)OMe$ |
| Et | $CH(Me)SCH_2CH_2CH_2Cl$ |
| Et | $CH(Me)S(O)CH_2CH_2CH_2Cl$ |
| Et | $CH(Me)S(O)_2CH_2CH_2CH_2Cl$ |
| Et | $CH(Me)SCH_2CF_3$ |
| Et | $CH(Me)S(O)CH_2CF_3$ |
| Et | $CH(Me)S(O)_2CH_2CF_3$ |
| Et | $CH(Me)SCH_2Ph$ |
| Et | $CH(Me)S(O)CH_2Ph$ |
| Et | $CH(Me)S(O)_2CH_2Ph$ |
| Et | $CH(Me)SCH_2$(D1-34a) |
| Et | $CH(Me)S(O)CH_2$(D1-34a) |
| Et | $CH(Me)S(O)_2CH_2$(D1-34a) |
| Et | $CH(CH_3)SCH_2Si(CH_3)_3$ |
| Et | CH(Me)SCN |
| Et | $CH(Me)SCH_2CN$ |
| Et | CH(Me)SC(O)Me |
| Et | CH(Me)S{D1-12a($X^{1a}$=Me)} |
| Et | CH(Me)S(D1-32a) |
| Et | CH(Me)S{D1-32b(3-$NO_2$)} |
| Et | CH(Me)S{D1-32b(3-$CF_3$)} |
| Et | CH(Me)S(D1-37a) |
| Et | CH(Me)S(D1-51a) |
| Et | CH(Et)SMe |
| Et | CH(Et)S(O)Me |
| Et | $CH(Et)S(O)_2Me$ |
| Et | CH(Et)SEt |
| Et | CH(Et)S(O)Et |
| Et | $CH(Et)S(O)_2Et$ |
| Et | CH(Et)S(=NCN)Et |
| Et | CH(Et)S(O)(=NCN)Et |
| Et | CH(Et)SPr-n |
| Et | $CH(Et)S(O)_2$Pr-n |
| Et | $CH(Et)S(O)_2$Pr-n |
| Et | CH(Et)SPr-i |
| Et | CH(Et)S(O)Pr-i |
| Et | $CH(Et)S(O)_2$Pr-i |
| Et | CH(Et)SPr-c |
| Et | $CH(Et)S(O)_2$Pr-c |
| Et | $CH(Et)S(O)_2$Pr-c |
| Et | $CH(Et)SCH_2CF_3$ |

(continued)

| $R^a$ | $R^{b-1}$ |
|---|---|
| Et | $CH(Et)S(O)CH_2CF_3$ |
| Et | $CH(Et)S(O)_2CH_2CF_3$ |
| Et | CH(Pr-n)SMe |
| Et | $CH(Pr-n)S(O)_2Me$ |
| Et | $CH(Pr-n)S(O)_2Me$ |
| Et | CH(Pr-n)SEt |
| Et | $CH(Pr-n)S(O)_2Et$ |
| Et | $CH(Pr-n)S(O)_2Et$ |
| Et | CH(Pr-n)S(=NCN)Et |
| Et | CH(Pr-n)S(O)(=NCN)Et |
| Et | CH(Pr-n)SPr-n |
| Et | $CH(Pr-n)S(O)_2Pr-n$ |
| Et | $CH(Pr-n)S(O)_2Pr-n$ |
| Et | CH(Pr-n)SPr-i |
| Et | $CH(Pr-n)S(O)_2Pr-i$ |
| Et | $CH(Pr-n)S(O)_2Pr-i$ |
| Et | CH(Pr-n)SPr-c |
| Et | $CH(Pr-n)S(O)_2Pr-c$ |
| Et | $CH(Pr-n)S(O)_2Pr-c$ |
| Et | $CH(Pr-n)SCH_2CF_3$ |
| Et | $CH(Pr-n)S(CH_2CF_3$ |
| Et | $CH(Pr-n)S(O)_2CH_2CF_3$ |
| Et | CH(Pr-i)SMe |
| Et | CH(Pr-i)S(O)Me |
| Et | $CH(Pr-i)S(O)_2Me$ |
| Et | CH(Pr-i)SEt |
| Et | CH(Pr-i)S(O)Et |
| Et | $CH(Pr-i)S(O)_2Et$ |
| Et | CH(Pr-i)S(=NCN)Et |
| Et | CH(Pr-i)S(O)(=NCN)Et |
| Et | CH(Pr-i)SPr-n |
| Et | CH(Pr-i)S(O)Pr-n |
| Et | $CH(Pr-i)S(O)_2Pr-n$ |
| Et | CH(Pr-i)SPr-i |
| Et | CH(Pr-i)S(O)Pr-i |
| Et | $CH(Pr-i)S(O)_2Pr-i$ |
| Et | CH(Pr-i)SPr-c |
| Et | CH(Pr-i)S(O)Pr-c |
| Et | $CH(Pr-i)S(O)_2Pr-c$ |
| Et | $CH(Pr-i)SCH_2CF_3$ |
| Et | $CH(Pr-i)S(O)CH_2CF_3$ |
| Et | $CH(Pr-i)S(O)_2CH_2CF_3$ |
| Et | CH(Pr-c)SMe |
| Et | CH(Pr-c)S(O)Me |
| Et | $CH(Pr-c)S(O)_2Me$ |
| Et | CH(Pr-c)SEt |
| Et | CH(Pr-c)S(O)Et |
| Et | $CH(Pr-c)S(O)_2Et$ |
| Et | CH(Pr-c)S(=NCN)Et |

(continued)

| $R^a$ | $R^{b-1}$ |
|---|---|
| Et | CH(Pr-c)S(O)(=NCN)Et |
| Et | CH(Pr-c)SPr-n |
| Et | CH(Pr-c)S(O)Pr-n |
| Et | $CH(Pr-c)S(O)_2Pr-n$ |
| Et | CH(Pr-c)SPr-i |
| Et | CH(Pr-c)S(O)Pr-i |
| Et | $CH(Pr-c)S(O)_2Pr-i$ |
| Et | CH(Pr-c)SPr-c |
| Et | CH(Pr-c)S(O)Pr-c |
| Et | $CH(Pr-c)S(O)_2Pr-c$ |
| Et | $CH(Pr-c)SCH_2CF_3$ |
| Et | $CH(Pr-c)S(O)CH_2CF_3$ |
| Et | $CH(Pr-c)S(O)_2CH_2CF_3$ |
| Et | CH(Bu-n)SMe |
| Et | CH(Bu-n)S(O)Me |
| Et | $CH(Bu-n)S(O)_2Me$ |
| Et | CH(Bu-n)SEt |
| Et | CH(Bu-n)S(O)Et |
| Et | $CH(Bu-n)S(O)_2Et$ |
| Et | CH(Bu-n)S(=NCN)Et |
| Et | CH(Bu-n)S(O)(=NCN)Et |
| Et | CH(Bu-n)SPr-n |
| Et | CH(Bu-n)S(O)Pr-n |
| Et | $CH(Bu-n)S(O)_2Pr-n$ |
| Et | CH(Bu-n)SPr-i |
| Et | CH(Bu-n)S(O)Pr-i |
| Et | $CH(Bu-n)S(O)_2Pr-i$ |
| Et | CH(Bu-n)SPr-c |
| Et | CH(Bu-n)S(O)Pr-c |
| Et | $CH(Bu-n)S(O)_2Pr-c$ |
| Et | $CH(Bu-n)SCH_2CF_3$ |
| Et | $CH(Bu-n)S(O)CH_2CF_3$ |
| Et | $CH(Bu-n)S(O)_2CH_2CF_3$ |
| Et | CH(F)SMe |
| Et | CH(F)S(O)Me |
| Et | $CH(F)S(O)_2Me$ |
| Et | CH(F)SEt |
| Et | CH(F)S(O)Et |
| Et | $CH(F)S(O)_2Et$ |
| Et | CH(F)S(=NCN)Et |
| Et | CH(F)S(O)(=NCN)Et |
| Et | $CH(F)SCH_2CF_3$ |
| Et | $CH(F)S(O)CH_2CF_3$ |
| Et | $CH(F)S(O)_2CH_2CF_3$ |
| Et | $C(F)_2SMe$ |
| Et | $C(F)_2S(O)Me$ |
| Et | $C(F)_2S(O)_2Me$ |
| Et | $C(F)_2SEt$ |
| Et | $C(F)_2S(O)Et$ |

(continued)

| $R^a$ | $R^{b-1}$ |
|---|---|
| Et | $C(F)_2S(O)_2Et$ |
| Et | $C(F)_2S(=NCN)Et$ |
| Et | $C(F)_2S(O)(=NCN)Et$ |
| Et | $C(F)_2SCH_2CF_3$ |
| Et | $C(F)_2S(O)CH_2CF_3$ |
| Et | $C(F)_2S(O)_2CH_2CF_3$ |
| Et | CH(Cl)SMe |
| Et | CH(Cl)S(O)Me |
| Et | $CH(Cl)S(O)_2Me$ |
| Et | CH(Cl)SEt |
| Et | CH(Cl)S(O)Et |
| Et | $CH(Cl)S(O)_2Et$ |
| Et | CH(Cl)S(=NCN)Et |
| Et | CH(Cl)S(O)(=NCN)Et |
| Et | $CH(Cl)SCH_2CF_3$ |
| Et | $CH(Cl)S(O)CH_2CF_3$ |
| Et | $CH(Cl)S(O)_2CH_2CF_3$ |
| Et | $C(Cl)_2SMe$ |
| Et | $C(Cl)_2S(O)Me$ |
| Et | $C(Cl)_2S(O)_2Me$ |
| Et | $C(Cl)_2SEt$ |
| Et | $C(Cl)_2S(O)Et$ |
| Et | $C(Cl)_2S(O)_2Et$ |
| Et | $C(Cl)_2S(=NCN)Et$ |
| Et | $C(Cl)_2S(O)(=NCN)Et$ |
| Et | $C(Cl)_2SCH_2CF_3$ |
| Et | $C(Cl)_2S(O)CH_2CF_3$ |
| Et | $C(Cl)_2S(O)_2CH_2CF_3$ |
| Et | CH(Br)SMe |
| Et | CH(Br)S(O)Me |
| Et | $CH(Br)S(O)_2Me$ |
| Et | CH(Br)SEt |
| Et | CH(Br)S(O)Et |
| Et | $CH(Br)S(O)_2Et$ |
| Et | CH(Br)S(=NCN)Et |
| Et | CH(Br)S(O)(=NCN)Et |
| Et | $CH(Br)SCH_2CF_3$ |
| Et | $CH(Br)S(O)CH_2CF_3$ |
| Et | $CH(Br)S(O)_2CH_2CF_3$ |
| Et | CH(I)SMe |
| Et | CH(I)S(O)Me |
| Et | $CH(I)S(O)_2Me$ |
| Et | CH(I)SEt |
| Et | CH(I)S(O)Et |
| Et | $CH(I)S(O)_2Et$ |
| Et | CH(I)S(=NCN)Et |
| Et | CH(I)S(O)(=NCN)Et |
| Et | $CH(I)SCH_2CF_3$ |
| Et | $CH(I)S(O)CH_2CF_3$ |

(continued)

| $R^a$ | $R^{b-1}$ |
|---|---|
| Et | $CH(I)S(O)_2CH_2CF_3$ |
| Et | CH{OC(O)Me}SMe |
| Et | CH{OC(O)Me}S(O)Me |
| Et | CH{OC(O)Me}$S(O)_2$Me |
| Et | CH{OC(O)OMe}SEt |
| Et | CH{OC(O)OMe}S(O)Et |
| Et | CH{OC(O)OMe}$S(O)_2$Et |
| Et | CH{OC(O)OMe}S(=NCN)Et |
| Et | CH{OC(O)OMe}S(O)(=NCN)Et |
| Et | CH{OC(O)OEt}SMe |
| Et | CH{OC(O)OEt}S(O)Me |
| Et | CH{OC(O)OEt}$S(O)_2$Me |
| Et | CH{OC(O)OEt}SEt |
| Et | CH{OC(O)OEt}S(O)Et |
| Et | CH{OC(O)OEt}$S(O)_2$Et |
| Et | CH{OC(O)OEt}S(=NCN)Et |
| Et | CH{OC(O)OEt}S(O)(=NCN)Et |
| Et | CH(OMe)SMe |
| Et | CH(OMe)S(O)Me |
| Et | $CH(OMe)S(O)_2Me$ |
| Et | CH(OMe)SEt |
| Et | CH(OMe)S(O)Et |
| Et | $CH(OMe)S(O)_2Et$ |
| Et | CH(OMe)S(=NCN)Et |
| Et | CH(OMe)S(O)(=NCN)Et |
| Et | CH(OEt)SMe |
| Et | CH(OEt)S(O)Me |
| Et | $CH(OEt)S(O)_2Me$ |
| Et | CH(OEt)SEt |
| Et | CH(OEt)S(O)Et |
| Et | $CH(OEt)S(O)_2Et$ |
| Et | CH(OEt)S(=NCN)Et |
| Et | CH(OEt)S(O)(=NCN)Et |
| Et | $CH(SMe)_2$ |
| Et | CH(SMe)S(O)Me |
| Et | $CH(SMe)S(O)_2Me$ |
| Et | CH(SMe)SEt |
| Et | CH(SMe)S(O)Et |
| Et | $CH(SMe)S(O)_2Et$ |
| Et | CH(SMe)S(=NCN)Et |
| Et | CH(SMe)S(O)(=NCN)Et |
| Et | CH(SEt)SMe |
| Et | CH(SEt)S(O)Me |
| Et | $CH(SEt)S(O)_2Me$ |
| Et | CH(SEt)SEt |
| Et | CH(SEt)S(O)Et |
| Et | $CH(SEt)S(O)_2Et$ |
| Et | CH(SEt)S(=NCN)Et |
| Et | CH(SEt)S(O)(=NCN)Et |

(continued)

| $R^a$ | $R^{b-1}$ |
|---|---|
| Et | CH(CN)SMe |
| Et | CH(CN)S(O)Me |
| Et | $CH(CN)S(O)_2Me$ |
| Et | CH(CN)SEt |
| Et | CH(CN)S(O)Et |
| Et | $CH(CN)S(O)_2Et$ |
| Et | CH(CN)S(=NCN)Et |
| Et | CH(CN)S(O)(=NCN)Et |
| Et | CH{C(O)OMe}SMe |
| Et | CH{C(O)OMe}S(O)Me |
| Et | $CH\{C(O)OMe\}S(O)_2Me$ |
| Et | CH{C(O)OMe}SEt |
| Et | CH{C(O)OMe}S(O)Et |
| Et | $CH\{C(O)OMe\}S(O)_2Et$ |
| Et | CH{C(O)OMe}S(=NCN)Et |
| Et | CH{C(O)OMe}S(O)(=NCN)Et |
| Et | CH{C(O)OEt}SMe |
| Et | CH{C(O)OEt}S(O)Me |
| Et | $CH\{C(O)OEt\}S(O)_2Me$ |
| Et | CH{C(O)OEt}SEt |
| Et | CH{C(O)OEt}S(O)Et |
| Et | CH{C(O)OEt}S(O)Et |
| Et | CH{C(O)OEt}S(=NCN)Et |
| Et | CH{C(O)OEt}S(O)(=NCN)Et |
| Et | $C(Me)_2SMe$ |
| Et | $C(Me)_2S(O)Me$ |
| Et | $C(Me)_2S(O)_2Me$ |
| Et | $CH_2CH_2SH$ |
| Et | $CH_2CH_2SMe$ |
| Et | $CH_2CH_2S(O)Me$ |
| Et | $CH_2CH_2S(O)_2Me$ |
| Et | $CH_2CH_2SEt$ |
| Et | $CH_2CH_2S(O)Et$ |
| Et | $CH_2CH_2S(O)_2Et$ |
| Et | $CH_2CH_2SPr$-n |
| Et | $CH_2CH_2S(O)Pr$-n |
| Et | $CH_2CH_2S(O)_2Pr$-n |
| Et | $CH_2CH_2SPr$-i |
| Et | $CH_2CH_2S(O)Pr$-i |
| Et | $CH_2CH_2S(O)_2Pr$-i |
| Et | $CH_2CH_2SCH_2CF_3$ |
| Et | $CH_2CH_2S(O)CH_2CF_3$ |
| Et | $CH_2CH_2S(O)_2CH_2CF_3$ |
| Et | $CH_2CH_2SC(O)Me$ |
| Et | $CH(Me)CH_2SMe$ |
| Et | $CH(Me)CH_2S(O)Me$ |
| Et | $CH(Me)CH_2S(O)_2Me$ |
| Et | $CH(Me)CH_2SEt$ |
| Et | $CH(Me)CH_2S(O)Et$ |

(continued)

| $R^a$ | $R^{b-1}$ |
|---|---|
| Et | $CH(Me)CH_2S(O)_2Et$ |
| Et | $CH(Me)CH_2SPr$-n |
| Et | $CH(Me)CH_2S(O)Pr$-n |
| Et | $CH(Me)CH_2S(O)_2Pr$-n |
| Et | $CH(Me)CH_2SPr$-i |
| Et | $CH(Me)CH_2S(O)Pr$-i |
| Et | $CH(Me)CH_2S(O)_2Pr$-i |
| Et | $CH(Me)CH_2SCH_2CF_3$ |
| Et | $CH(Me)CH_2S(O)CH_2CF_3$ |
| Et | $CH(Me)CH_2S(O)_2CH_2CF_3$ |
| Et | $CH(Me)CH_2SC(O)Me$ |
| Et | $CH(Et)CH_2SMe$ |
| Et | $CH(Et)CH_2S(O)Me$ |
| Et | $CH(Et)CH_2S(O)_2Me$ |
| Et | $CH(Et)CH_2SEt$ |
| Et | $CH(Et)CH_2S(O)Et$ |
| Et | $CH(Et)CH_2S(O)_2Et$ |
| Et | CH(Pr-n)$CH_2SMe$ |
| Et | CH(Pr-n)$CH_2S(O)Me$ |
| Et | CH(Pr-n)$CH_2S(O)_2Me$ |
| Et | CH(Pr-n)$CH_2SEt$ |
| Et | CH(Pr-n)$CH_2S(O)Et$ |
| Et | CH(Pr-n)$CH_2S(O)_2Et$ |
| Et | CH(Pr-i)$CH_2SMe$ |
| Et | CH(Pr-i)$CH_2S(O)Me$ |
| Et | CH(Pr-i)$CH_2S(O)_2Me$ |
| Et | CH(Pr-i)$CH_2SEt$ |
| Et | CH(Pr-i)$CH_2S(O)Et$ |
| Et | CH(Pr-i)$CH_2S(O)_2Et$ |
| Et | CH(Pr-c)$CH_2SMe$ |
| Et | CH(Pr-c)$CH_2S(O)Me$ |
| Et | CH(Pr-c)$CH_2S(O)_2Me$ |
| Et | CH(Pr-c)$CH_2SEt$ |
| Et | CH(Pr-c)$CH_2S(O)Et$ |
| Et | CH(Pr-c)$CH_2S(O)_2Et$ |
| Et | CH(Bu-n)$CH_2SMe$ |
| Et | CH(Bu-n)$CH_2S(O)Me$ |
| Et | CH(Bu-n)$CH_2S(O)_2Me$ |
| Et | CH(Bu-n)$CH_2SEt$ |
| Et | CH(Bu-n)$CH_2S(O)Et$ |
| Et | CH(Bu-n)$CH_2S(O)_2Et$ |
| Et | CH(Bu-i)$CH_2SMe$ |
| Et | CH(Bu-i)$CH_2S(O)Me$ |
| Et | CH(Bu-i)$CH_2S(O)_2Me$ |
| Et | CH(Bu-i)$CH_2SEt$ |
| Et | CH(Bu-i)$CH_2S(O)Et$ |
| Et | CH(Bu-i)$CH_2S(O)_2Et$ |
| Et | CH(Bu-s)$CH_2SMe$ |
| Et | CH(Bu-s)$CH_2S(O)Me$ |

(continued)

| $R^a$ | $R^{b-1}$ |
|---|---|
| Et | $CH(Bu-s)CH_2S(O)_2Me$ |
| Et | $CH(Bu-s)CH_2SEt$ |
| Et | $CH(Bu-s)CH_2S(O)Et$ |
| Et | $CH(Bu-s)CH_2S(O)_2Et$ |
| Et | $CH(Bu-t)CH_2SMe$ |
| Et | $CH(Bu-t)CH_2S(O)Me$ |
| Et | $CH(Bu-t)CH_2S(O)_2Me$ |
| Et | $CH(Bu-t)CH_2SEt$ |
| Et | $CH(Bu-t)CH_2S(O)Et$ |
| Et | $CH(Bu-t)CH_2S(O)_2Et$ |
| Et | $CH(OMe)CH_2SMe$ |
| Et | $CH(OMe)CH_2S(O)Me$ |
| Et | $CH(OMe)CH_2S(O)_2Me$ |
| Et | $CH(OMe)CH_2SEt$ |
| Et | $CH(OMe)CH_2S(O)Et$ |
| Et | $CH(OMe)CH_2S(O)_2Et$ |
| Et | $CH(SMe)CH_2SMe$ |
| Et | $CH(SMe)CH_2S(O)Me$ |
| Et | $CH(SMe)CH_2S(O)_2Me$ |
| Et | $CH(SMe)CH_2SEt$ |
| Et | $CH(SMe)CH_2S(O)Et$ |
| Et | $CH(SMe)CH_2S(O)_2Et$ |
| Et | $CH(CH_2OMe)CH_2SMe$ |
| Et | $CH(CH_2OMe)CH_2S(O)Me$ |
| Et | $CH(CH_2OMe)CH_2S(O)_2Me$ |
| Et | $CH(CH_2OMe)CH_2SEt$ |
| Et | $CH(CH_2OMe)CH_2S(O)Et$ |
| Et | $CH(CH_2OMe)CH_2S(O)_2Et$ |
| Et | $CH(CH_2SMe)CH_2SMe$ |
| Et | $CH(CH_2SMe)CH_2S(O)Me$ |
| Et | $CH(CH_2SMe)CH_2S(O)_2Me$ |
| Et | $CH(CH_2SMe)CH_2SEt$ |
| Et | $CH(CH_2SMe)CH_2S(O)Et$ |
| Et | $CH(CH_2SMe)CH_2S(O)_2Et$ |
| Et | $CH(F)CH_2SMe$ |
| Et | $CH(F)CH_2S(O)Me$ |
| Et | $CH(F)CH_2S(O)_2Me$ |
| Et | $CH(F)CH_2SEt$ |
| Et | $CH(F)CH_2S(O)Et$ |
| Et | $CH(F)CH_2S(O)_2Et$ |
| Et | $C(F)_2CH_2SMe$ |
| Et | $C(F)_2CH_2S(O)Me$ |
| Et | $C(F)_2CH_2S(O)_2Me$ |
| Et | $C(F)_2CH_2SEt$ |
| Et | $C(F)_2CH_2S(O)Et$ |
| Et | $C(F)_2CH_2S(O)_2Et$ |
| Et | $CH(Cl)CH_2SMe$ |
| Et | $CH(Cl)CH_2S(O)Me$ |
| Et | $CH(Cl)CH_2S(O)_2Me$ |

(continued)

| $R^a$ | $R^{b-1}$ |
|---|---|
| Et | $CH(Cl)CH_2SEt$ |
| Et | $CH(Cl)CH_2S(O)Et$ |
| Et | $CH(Cl)CH_2S(O)_2Et$ |
| Et | $C(Cl)_2CH_2SMe$ |
| Et | $C(Cl)_2CH_2S(O)Me$ |
| Et | $C(Cl)_2CH_2S(O)_2Me$ |
| Et | $C(Cl)_2CH_2SEt$ |
| Et | $C(Cl)_2CH_2S(O)Et$ |
| Et | $C(Cl)_2CH_2S(O)_2Et$ |
| Et | $CH(Br)CH_2SMe$ |
| Et | $CH(Br)CH_2S(O)Me$ |
| Et | $CH(Br)CH_2S(O)_2Me$ |
| Et | $CH(Br)CH_2SEt$ |
| Et | $CH(Br)CH_2S(O)Et$ |
| Et | $CH(Br)CH_2S(O)_2Et$ |
| Et | $CH(I)CH_2SMe$ |
| Et | $CH(I)CH_2S(O)Me$ |
| Et | $CH(I)CH_2S(O)_2Me$ |
| Et | $CH(I)CH_2SEt$ |
| Et | $CH(I)CH_2S(O)Et$ |
| Et | $CH(I)CH_2S(O)_2Et$ |
| Et | $CH(CN)CH_2SMe$ |
| Et | $CH(CN)CH_2S(O)Me$ |
| Et | $CH(CN)CH_2S(O)_2Me$ |
| Et | $CH(CN)CH_2SEt$ |
| Et | $CH(CN)CH_2S(O)Et$ |
| Et | $CH(CN)CH_2S(O)_2Et$ |
| Et | $CH\{C(O)OMe\}CH_2SMe$ |
| Et | $CH\{C(O)OMe\}CH_2S(O)Me$ |
| Et | $CH\{C(O)OMe\}CH_2S(O)_2Me$ |
| Et | $CH\{C(O)OMe\}CH_2SEt$ |
| Et | $CH\{C(O)OMe\}CH_2S(O)Et$ |
| Et | $CH\{C(O)OMe\}CH_2S(O)_2Et$ |
| Et | $CH\{C(O)OEt\}CH_2SMe$ |
| Et | $CH\{C(O)OEt\}CH_2S(O)Me$ |
| Et | $CH\{C(O)OEt\}CH_2S(O)_2Me$ |
| Et | $CH\{C(O)OEt\}CH_2SEt$ |
| Et | $CH\{C(O)OEt\}CH_2S(O)Et$ |
| Et | $CH\{C(O)OEt\}CH_2S(O)_2Et$ |
| Et | $CH(Ph)CH_2SMe$ |
| Et | $CH(Ph)CH_2S(O)Me$ |
| Et | $CH(Ph)CH_2S(O)_2Me$ |
| Et | $CH(Ph)CH_2SEt$ |
| Et | $CH(Ph)CH_2S(O)Et$ |
| Et | $CH(Ph)CH_2S(O)_2Et$ |
| Et | $C(Me)_2CH_2SMe$ |
| Et | $C(Me)_2CH_2S(O)Me$ |
| Et | $C(Me)_2CH_2S(O)_2Me$ |
| Et | $C(Me)_2CH_2SEt$ |

(continued)

| $R^a$ | $R^{b-1}$ |
|---|---|
| Et | $C(Me)_2CH_2S(O)Et$ |
| Et | $C(Me)_2CH_2S(O)_2Et$ |
| Et | $C(Et)_2CH_2SMe$ |
| Et | $C(Et)_2CH_2S(O)Me$ |
| Et | $C(Et)_2CH_2S(O)_2Me$ |
| Et | $C(Et)_2CH_2SEt$ |
| Et | $C(Et)_2CH_2S(O)Et$ |
| Et | $C(Et)_2CH_2SO)_2Et$ |
| Et | $C(=CH_2)CH_2SMe$ |
| Et | $C(=CH_2)CH_2S(O)Me$ |
| Et | $C(=CH_2)CH_2S(O)_2Me$ |
| Et | $C(=CH_2)CH_2SEt$ |
| Et | $C(=CH_2)CH_2S(O)Et$ |
| Et | $C(=CH_2)CH_2S(O)_2Et$ |
| Et | $CH_2CH_2CH_2SMe$ |
| Et | $CH_2CH_2CH_2S(O)Me$ |
| Et | $CH_2CH_2CH_2S(O)_2Me$ |
| Et | $CH(Me)CH_2CH_2SMe$ |
| Et | $CH(Me)CH_2CH_2S(O)Me$ |
| Et | $CH(MeCH_2CH_2S(O)_2Me$ |
| Et | $CH_2CH(Me)CH_2SMe$ |
| Et | $CH_2CH(Me)CH_2S(O)Me$ |
| Et | $CH_2CHMe)CH_2SO)_2Me$ |
| Et | $CH_2CH_2CH(Me)SMe$ |
| Et | $CH_2CH_2CH(Me)S(O)Me$ |
| Et | $CH_2CH_2CH(Me)S(O)_2Me$ |
| Et | $CH_2CH_2CH_2SEt$ |
| Et | $CH_2CH_2CH_2S(O)Et$ |
| Et | $CH_2CH_2CH_2S(O)_2Et$ |
| Et | $CH(Me)CH_2CH_2SEt$ |
| Et | $CH(Me)CH_2CH_2S(O)Et$ |
| Et | $CHMeCH_2CH_2S(O)_2Et$ |
| Et | $CH_2CH(Me)CH_2SEt$ |
| Et | $CH_2CH(Me)CH_2S(O)Et$ |
| Et | $CH_2CH(MeCH_2S(O)_2Et$ |
| Et | $CH_2CH_2CH(Me)SEt$ |
| Et | $CH_2CH_2CH(Me)S(O)Et$ |
| Et | $CH_2CH_2CH(Me)S(O)_2Et$ |
| Et | $CH_2CH_2CH_2SPr$-n |
| Et | $CH_2CH_2CH_2S(O)Pr$-n |
| Et | $CH_2CH_2CH_2S(O)_2Pr$-n |
| Et | $CH(Me)CH_2CH_2SPr$-n |
| Et | $CH(Me)CH_2CH_2S(O)Pr$-n |
| Et | $CH(Me)CH_2CH_2S(O)_2Pr$-n |
| Et | $CH_2CH(Me)CH_2SPr$-n |
| Et | $CH_2CH(Me)CH_2S(O)Pr$-n |
| Et | $CH_2CH(Me)CH_2S(O)_2Pr$-n |
| Et | $CH_2CH_2CH_2CH_2SMe$ |
| Et | $CH_2CH_2CH_2CH_2S(O)Me$ |

(continued)

| $R^a$ | $R^{b-1}$ |
|---|---|
| Et | $CH_2CH_2CH_2CH_2S(O)_2Me$ |
| Et | $CH_2CH_2CH_2CH_2SEt$ |
| Et | $CH_2CH_2CH_2CH_2S(O)Et$ |
| Et | $CH_2CH_2CH_2CH_2S(O)_2Et$ |
| Et | $CH_2CH_2CH_2CH_2SPr$-n |
| Et | $CH_2CH_2CH_2CH_2S(O)Pr$-n |
| Et | $CH_2CH_2CH_2CH_2S(O)_2Pr$-n |
| Et | $CH_2CH(Me)SMe$ |
| Et | $CH_2CH(Me)S(O)Me$ |
| Et | $CH_2CH(Me)S(O)_2Me$ |
| Et | $CH_2CH(Me)SEt$ |
| Et | $CH_2CH(Me)S(O)Et$ |
| Et | $CH_2CH(Me)S(O)_2Et$ |
| Et | $CH_2CH(Et)SMe$ |
| Et | $CH_2CH(Et)S(O)Me$ |
| Et | $CH_2CH(Et)S(O)_2Me$ |
| Et | $CH_2CH(Et)SEt$ |
| Et | $CH_2CH(Et)S(O)Et$ |
| Et | $CH_2CH(Et)S(O)_2Et$ |
| Et | $CH_2CH$(Pr-n)SMe |
| Et | $CH_2CH$(Pr-n)S(O)Me |
| Et | $CH_2CH$(Pr-n)$S(O)_2Me$ |
| Et | $CH_2CH$(Pr-n)SEt |
| Et | $CH_2CH$(Pr-n)S(O)Et |
| Et | $CH_2CH$(Pr-n)$S(O)_2Et$ |
| Et | $CH_2CH$(Pr-i)SMe |
| Et | $CH_2CH$(Pr-i)S(O)Me |
| Et | $CH_2CH$(Pr-i)$S(O)_2Me$ |
| Et | $CH_2CH$(Pr-i)SEt |
| Et | $CH_2CH$(Pr-i)S(O)Et |
| Et | $CH_2CH$(Pr-i)$S(O)_2Et$ |
| Et | $CH_2CH$(Pr-c)SMe |
| Et | $CH_2CH$(Pr-c)S(O)Me |
| Et | $CH_2CH$(Pr-c)$S(O)_2Me$ |
| Et | $CH_2CH$(Pr-c)SEt |
| Et | $CH_2CH$(Pr-c)S(O)Et |
| Et | $CH_2CH$(Pr-c)$S(O)_2Et$ |
| Et | $CH_2CH$(Bu-n)SMe |
| Et | $CH_2CH$(Bu-n)S(O)Me |
| Et | $CH_2CH$(Bu-n)$S(O)_2Me$ |
| Et | $CH_2CH$(Bu-n)SEt |
| Et | $CH_2CH$(Bu-n)S(O)Et |
| Et | $CH_2CH$(Bu-n)$S(O)_2Et$ |
| Et | $CH_2CH$(Bu-i)SMe |
| Et | $CH_2CH$(Bu-i)S(O)Me |
| Et | $CH_2CH$(Bu-i)$S(O)_2Me$ |
| Et | $CH_2CH$(Bu-i)SEt |
| Et | $CH_2CH$(Bu-i)S(O)Et |
| Et | $CH_2CH$(Bu-i)$S(O)_2Et$ |

(continued)

| $R^a$ | $R^{b-1}$ |
|---|---|
| Et | $CH_2CH(Bu-s)SMe$ |
| Et | $CH_2CH(Bu-s)S(O)Me$ |
| Et | $CH_2CH(Bu-s)S(O)_2Me$ |
| Et | $CH_2CH(Bu-s)SEt$ |
| Et | $CH_2CH(Bu-s)S(O)Et$ |
| Et | $CH_2CH(Bu-s)S(O)_2Et$ |
| Et | $CH_2CH(Bu-t)SMe$ |
| Et | $CH_2CH(Bu-t)S(O)Me$ |
| Et | $CH_2CH(Bu-t)S(O)_2Me$ |
| Et | $CH_2CH(Bu-t)SEt$ |
| Et | $CH_2CH(Bu-t)S(O)Et$ |
| Et | $CH_2CH(Bu-t)S(O)_2Et$ |
| Et | $CH_2C(Et)_2SMe$ |
| Et | $CH_2C(Et)_2S(O)Me$ |
| Et | $CH_2C(Et)_2S(O)_2Me$ |
| Et | $CH_2C(Et)_2SEt$ |
| Et | $CH_2C(Et)_2S(O)Et$ |
| Et | $CH_2C(Et)_2S(O)_2Et$ |
| Et | CH(Et)CH(Me)SMe |
| Et | CH(Et)CH(Me)S(O)Me |
| Et | $CH(Et)CH(Me)S(O)_2Me$ |
| Et | CH(Et)CH(Me)SEt |
| Et | CH(Et)CH(Me)S(O)Et |
| Et | $CH(Et)CH(Me)S(O)_2Et$ |
| Et | CH(Et)CH(Et)SMe |
| Et | CH(Et)CH(Et)S(O)Me |
| Et | $CH(Et)CH(Et)S(O)_2Me$ |
| Et | CH(Et)CH(Et)SEt |
| Et | CH(Et)CH(Et)S(O)Et |
| Et | $CH(Et)CH(Et)S(O)_2Et$ |
| Et | CH=CHSMe |
| Et | $CH_2CH(SMe)_2$ |
| Et | $CH_2CH_2SC(O)Me$ |
| Et | $C(CH_2)CH_2C(O)OH$ |
| Et | C(=NOMe)Me |
| Et | C(=NOMe)Et |
| Et | C(=NOMe)Pr-n |
| Et | C(=NOEt)Me |
| Et | C(=NOPr-n)Me |
| Et | C(=NOPr-i)Me |
| Et | C(=NOBu-n)Me |
| Et | $C(=NOCH_2Ph)Me$ |
| Et | C(=NOMe)C(=NOMe)Me |
| Et | $C(=NOMe)CH_2OCH_2CF_3$ |
| Et | $C(=NOMe)CH_2SMe$ |
| Et | $C(=NOMe)CH_2S(O)Me$ |
| Et | $C(=NOMe)CH_2S(O)_2Me$ |
| Et | $C(=NOMe)CH_2SEt$ |
| Et | $C(=NOMe)CH_2S(O)Et$ |

(continued)

| $R^a$ | $R^{b-1}$ |
|---|---|
| Et | $C(=NOMe)CH_2S(O)_2Et$ |
| Et | $C(=NOMe)CH_2SCF_3$ |
| Et | $C(=NOMe)CH_2S(O)CF_3$ |
| Et | $C(=NOMe)CH_2S(O)_2CF_3$ |
| Et | $C(=NOMe)CH_2SCH_2CF_3$ |
| Et | $C(=NOMe)CH_2S(O)CH_2CF_3$ |
| Et | $C(=NOMe)CH_2S(O)_2CH_2CF_3$ |
| Et | $C(=NOEt)CH_2SMe$ |
| Et | $C(=NOEt)CH_2S(O)Me$ |
| Et | $C(=NOEt)CH_2S(O)_2Me$ |
| Et | $C(=NOPr\text{-}n)CH_2SMe$ |
| Et | $C(=NOPr\text{-}n)CH_2S(O)Me$ |
| Et | $C(=NOPr\text{-}n)CH_2S(O)_2Me$ |
| Et | $C(=NOPr\text{-}i)CH_2SMe$ |
| Et | $C(=NOPr\text{-}i)CH_2S(O)Me$ |
| Et | $C(=NOPr\text{-}i)CH_2S(O)_2Me$ |
| Et | $C(=NOBu\text{-}n)CH_2SMe$ |
| Et | $C(=NOBu\text{-}n)CH_2S(O)Me$ |
| Et | $C(=NOBu\text{-}n)CH_2S(O)_2Me$ |
| Et | $C(=NOCH_2Ph)CH_2SMe$ |
| Et | $C(=NOCH_2Ph)CH_2S(O)Me$ |
| Et | $C(=NOCH_2Ph)CH_2S(O)_2Me$ |
| Et | $C(=NOCH_2CH=CH_2)CH_2SMe$ |
| Et | $C(=NOCH_2OMe)CH_2SMe$ |
| Et | $C(=NOCH_2CH_2OMe)CH_2SMe$ |
| Et | $C\{=NOCH_2CH_2Si(Me)_3\}CH_2SMe$ |
| Et | $CH_2CH(=NOMe)$ |
| Et | $CH_2C(=NOMe)Me$ |
| Et | $CH_2C(=NOMe)Et$ |
| Et | $CH_2C(=NOMe)Pr\text{-}n$ |
| Et | $CH_2C(=NOMe)Pr\text{-}i$ |
| Et | $CH_2C(=NOMe)Bu\text{-}n$ |
| Et | $CH_2C(=NOMe)Bu\text{-}i$ |
| Et | $CH_2C(=NOMe)Bu\text{-}s$ |
| Et | $CH_2C(=NOMe)Bu\text{-}t$ |
| Et | $CH_2C(=NOMe)CF_3$ |
| Et | $CH_2C(=NOCH_3)CH_2SCH_3$ |
| Et | $CH(SMe)C(=NOMe)Me$ |
| Et | $CH\{S(O)Me\}C(=NOMe)Me$ |
| Et | $CH\{S(O)_2Me\}C(=NOMe)Me$ |
| Et | $CH(SEt)C(=NOMe)Me$ |
| Et | $CH\{S(O)Et\}C(=NOMe)Me$ |
| Et | $CH\{S(O)_2Et\}C(=NOMe)Me$ |
| Et | $CH_2NH_2$ |
| Et | $CH_2NMe_2$ |
| Et | $CH_2NHC(O)Me$ |
| Et | $CH_2NHC(O)CF_3$ |
| Et | $CH_2NHC(O)CH_2OMe$ |
| Et | $CH_2NHC(O)CH_2SMe$ |

(continued)

| $R^a$ | $R^{b-1}$ |
|---|---|
| Et | $CH_2NHC(O)OMe$ |
| Et | $CH_2NHC(O)OBu$-t |
| Et | $CH_2NHSO_2Me$ |
| Et | $CH_2NHSO_2NMe_2$ |
| Et | $CH_2NHSO_2CF_3$ |
| Et | $CH_2N(Me)C(O)Me$ |
| Et | $CH_2N(Me)C(O)OMe$ |
| Et | $CH_2N(Me)C(O)OBu$-t |
| Et | $CH_2N(Me)SO_2Me$ |
| Et | CH(Me)NHC(O)OBu-t |
| Et | $CH(CH_2SMe)NH_2$ |
| Et | $CH(CH_2SMe)NHC(O)Me$ |
| Et | $CH(CH_2SMe)NHC(O)OMe$ |
| Et | $CH(CH_2SMe)NHC(O)OBu$-t |
| Et | $CH(CH_2SMe)NHSO_2Me$ |
| Et | $CH_2NHC(O)NHMe$ |
| Et | $CH_2NHC(O)NHEt$ |
| Et | $CH_2NHC(O)NHPr$-n |
| Et | $CH_2NHC(O)NHPr$-i |
| Et | $CH_2NHC(O)NHPr$-c |
| Et | $CH_2NHC(S)NHMe$ |
| Et | $CH_2NHC(S)NHEt$ |
| Et | $CH_2NHC(S)NHPr$-n |
| Et | $CH_2NHC(S)NHPr$-i |
| Et | $CH_2NHC(S)NHPr$-c |
| Et | $CH_2CH_2NHC(O)OBu$-t |
| Et | $CH_2P(O)(OMe)_2$ |
| Et | $CH_2P(O)(OEt)_2$ |
| Et | SMe |
| Et | OBu-t |
| Et | O{D1-108(4-$NO_2$)} |
| Et | $NH_2$ |
| Et | $NHCH_2SMe$ |
| Et | $NHCH_2CH_2SMe$ |
| Et | $N(Me)CH_2SMe$ |
| Et | $N(Me)CH_2CH_2SMe$ |
| Et | $NHCH_2CH_2CH_2SMe$ |
| Et | NHMe |
| Et | NHEt |
| Et | NPr-n |
| Et | NHPr-i |
| Et | $NHCH_2C(O)OMe$ |
| Et | $NHCH_2CF_3$ |
| Et | $N(Me)CH_2CF_3$ |
| Et | NHC(O)OEt |
| Et | $NHC(O)CCl_3$ |
| Et | $NHC(O)CH_2SMe$ |
| Et | $N(CH_2CH_3)C(O)CH_2SCH_3$ |
| Et | $NHS(O)_2$(D1-108a) |

(continued)

| $R^a$ | $R^{b-1}$ |
|---|---|
| Et | NHPh |
| Et | NH(D1-1a) |
| Et | NH(D1-2a) |
| Et | NH(D1-2b) |
| Et | NH{D1-4a($X^{1a}$=Me)} |
| Et | NH{D1-4b($X^{1a}$=Me)} |
| Et | NH(D1-5a) |
| Et | NH(D1-5b) |
| Et | NH(D1-5c) |
| Et | NH(D1-6a) |
| Et | NH(D1-6b) |
| Et | NH(D1-6c) |
| Et | NH(D1-7a) |
| Et | NH{D1-8a($X^{1a}$=Me)} |
| Et | NH{D1-8b($X^{1a}$=Me)} |
| Et | NH{D1-8c($x^{1a}$=Me)} |
| Et | NH(D1-9a) |
| Et | NH(D1-9b) |
| Et | NH(D1-9c) |
| Et | NH(D1-10a) |
| Et | NH(D1-10b) |
| Et | NH(D1-10c) |
| Et | NH(D1-11a) |
| Et | NH{D1-12a($X^{1a}$=Me)} |
| Et | NH{D1-12b($x^{1a}$=Me)} |
| Et | NH{D1-12c($x^{1a}$=Me)} |
| Et | NH(D1-13a) |
| Et | NH(D1-13b) |
| Et | NH(D1-14a) |
| Et | NH(D1-14b) |
| Et | NH(D1-15a) |
| Et | NH(D1-15b) |
| Et | NH(D1-16a) |
| Et | NH(D1-17a) |
| Et | NH(D1-18a) |
| Et | NH(D1-19a) |
| Et | NH(D1-20a) |
| Et | NH{D1-21a($X^{1a}$=Me)} |
| Et | NH{D1-21b($X^{1a}$=Me)} |
| Et | NH(D1-22a) |
| Et | NH{D1-23a($X^{1a}$=Me)} |
| Et | NH{D1-23b($X^{1a}$=Me)} |
| Et | NH(D1-24a) |
| Et | NH{D1-25a($X^{1a}$=Me)} |
| Et | NH(D1-26a) |
| Et | NH{D1-27a($X^{1a}$=Me)} |
| Et | NH(D1-28a) |
| Et | NH(D1-29a) |
| Et | NH{D1-30a($X^{1a}$=Me)} |

(continued)

| $R^a$ | $R^{b-1}$ |
|---|---|
| Et | NH{D1-31a($X^{1a}$=Me)} |
| Et | NH(D1-32a) |
| Et | NH(D1-33a) |
| Et | NH(D1-34a) |
| Et | NH(D1-35a) |
| Et | NH(D1-35b) |
| Et | NH(D1-36a) |
| Et | NH(D1-37a) |
| Et | NH(D1-38a) |
| Et | NH(D1-39a) |
| Et | NH(D1-40a) |
| Et | NH(D1-40b) |
| Et | NH(D1-40c) |
| Et | NH(D1-41a) |
| Et | NH(D1-42a) |
| Et | NH(D1-43a) |
| Et | NH(D1-45a) |
| Et | NH(D1-49a) |
| Et | NH(D1-51a) |
| Et | NH(D1-59a) |
| Et | NH(D1-61a) |
| Et | NH(D1-79a) |
| Et | NH(D1-80a) |
| Et | NH{D1-108b(2-Cl)} |
| Et | NH{D1-108b(3-Cl)} |
| Et | NH{D1-108b(4-Cl)} |
| Et | NH{D1-108b(2-Me)} |
| Et | NH{D1-108b(3-Me)} |
| Et | NH{D1-108b(4-Me)} |
| Et | N(Me)Ph |
| Et | N(Me)(D1-1a) |
| Et | N(Me)(D1-2a) |
| Et | N(Me)(D1-2b) |
| Et | N(Me){D1-4a($X^{1a}$=Me)} |
| Et | N(Me){D1-4b($X^{1a}$=Me)} |
| Et | N(Me)(D1-5a) |
| Et | N(Me)(D1-5b) |
| Et | N(Me)(D 1-5c) |
| Et | N(Me)(D1-6a) |
| Et | N(Me)(D1-6b) |
| Et | N(Me)(D 1-6c) |
| Et | N(Me)(D1-7a) |
| Et | N(Me){D1-8a($X^{1a}$=Me)} |
| Et | N(Me){D1-8b($X^{1a}$=Me)} |
| Et | N(Me){D1-8c($X^{1a}$=Me)} |
| Et | N(Me)(D1-9a) |
| Et | N(Me)(D1-9b) |
| Et | N(Me)(D1-9c) |
| Et | N(Me)(D1-10a) |

(continued)

| $R^a$ | $R^{b-1}$ |
|---|---|
| Et | N(Me)(D1-10b) |
| Et | N(Me)(D1-10c) |
| Et | N(Me)(D1-11a) |
| Et | N(Me){D1-12a($X^{1a}$=Me)} |
| Et | N(Me){D1-12b($X^{1a}$=Me)} |
| Et | N(Me){D1-12c($X^{1a}$=Me)} |
| Et | N(Me)(D1-13a) |
| Et | N(Me)(D1-13b) |
| Et | N(Me)(D1-14a) |
| Et | N(Me)(D1-14b) |
| Et | N(Me)(D1-15a) |
| Et | N(Me)(D1-15b) |
| Et | N(Me)(D1-16a) |
| Et | N(Me)(D1-17a) |
| Et | N(Me)(D1-18a) |
| Et | N(Me)(D1-19a) |
| Et | N(Me)(D1-20a) |
| Et | N(Me){D1-21a($X^{1a}$=Me)} |
| Et | N(Me){D1-21b($X^{1a}$=Me)} |
| Et | N(Me)(D1-22a) |
| Et | N(Me){D1-23a($X^{1a}$=Me)} |
| Et | N(Me){D1-23b($X^{1a}$=Me)} |
| Et | N(Me)(D1-24a) |
| Et | N(Me){D1-25a($X^{1a}$=Me)} |
| Et | N(Me)(D1-26a) |
| Et | N(Me){D1-27a($X^{1a}$=Me)} |
| Et | N(Me)(D1-28a) |
| Et | N(Me)(D1-29a) |
| Et | N(Me){D1-30a($X^{1a}$=Me)} |
| Et | N(Me){D1-31a($X^{1a}$=Me)} |
| Et | N(Me)(D1-32a) |
| Et | N(Me)(D1-33a) |
| Et | N(Me)(D1-34a) |
| Et | N(Me)(D1-35a) |
| Et | N(Me)(D1-35b) |
| Et | N(Me)(D1-36a) |
| Et | N(Me)(D1-37a) |
| Et | N(Me)(D1-38a) |
| Et | N(Me)(D1-39a) |
| Et | N(Me)(D1-40a) |
| Et | N(Me)(D1-40b) |
| Et | N(Me)(D1-40c) |
| Et | N(Me)(D1-41a) |
| Et | N(Me)(D1-42a) |
| Et | N(Me)(D1-43a) |
| Et | N(Me)(D1-45a) |
| Et | N(Me)(D1-49a) |
| Et | N(Me)(D1-51a) |
| Et | N(Me)(D1-59a) |

(continued)

| $R^a$ | $R^{b-1}$ |
|---|---|
| Et | N(Me)(D1-61a) |
| Et | N(Me)(D1-79a) |
| Et | N(Me)(D1-80a) |
| Et | N(Me){D1-108b(2-Cl)} |
| Et | N(Me){D1-108b(3-Cl)} |
| Et | N(Me){D1-108b(4-Cl)} |
| Et | N(Me){D1-108b(2-Me)} |
| Et | N(Me){D1-108b(3-Me)} |
| Et | N(Me){D1-108b(4-Me)} |
| Et | D1-8c($CH_2CF_3$) |
| Et | D1-1b(5-Br) |
| Et | D1-2c(5-SMe) |
| Et | D1-2c{5-C(O)Me} |
| Et | D1-5d{5-(Pr-c)} |
| Et | D1-5e(3,5-$Me_2$) |
| Et | D1-6d(4,5-$Cl_2$) |
| Et | D1-8f{$X^{1a}$=Me,X=3-Cl,5-$S(O)_2NH_2$} |
| Et | D1-4a($X^{1a}$=Me) |
| Et | D1-9b |
| Et | D1-9f(4-$CH_3$) |
| Et | D1-10e(2-$CF_3$) |
| Et | D1-10f{2-($CH_2SCH_3$)-4-$CH_3$} |
| Et | D1-10f{2-($CH_2OCH_3$)-4-$CF_3$} |
| Et | D1-12f($X^{1a}$=Me,$X^1$=4-$NO_2$) |
| Et | D1-13d($X^{1a}$=Me) |
| Et | D1-19a |
| Et | D1-32b(2-Br) |
| Et | D1-33a |
| Et | D1-33b(2-OMe) |
| Et | D1-33b(6-CN) |
| Et | D1-34a |
| Et | D1-35b |
| Et | D1-38b(4-SMe) |
| Et | D1-39b(5-Me) |
| Et | D1-45d(5,5-$Me_2$) |
| Et | D1-81a |
| Et | D1-81b |
| Et | D1-82a |
| Et | D1-82b |
| Et | D1-82c |
| Et | D1-84d{$X^{1b}$=$C(Cl)_3$} |
| Et | D1-85d{$X^{1b}$=$C(Cl)_3$} |
| Et | D1-87a |
| Et | D1-88a{$X^{1a}$=C(O)Me} |
| Et | D1-88a{$X^1$=C(O)$CH_2$SMe} |
| Et | D1-88a{$X^{1a}$=C(O)OBu-t} |
| Et | D1-88a{$X^{1a}$=C(O)$CF_3$} |
| Et | D1-92c |
| Et | D1-94b{$X^{1a}$=C(O)OBu-t} |

(continued)

| $R^a$ | $R^{b-1}$ |
|---|---|
| Et | D1-94c{$X^{1a}$=C(O)OBu-t} |
| Et | D1-103b |
| Et | D1-103c |
| Et | D1-103d |
| Et | D1-103e |
| Et | D1-103f |
| Et | D1-103g |
| Et | D1-103h |
| Et | D1-103i |
| Et | D1-108b(2-Cl) |
| Et | D1-108b(3-Cl) |
| Et | D1-108b(4-Cl) |
| Et | D1-108b(2-SMe) |
| Et | D1-108b(3-SMe) |
| Et | D1-108b(4-SMe) |
| Et | D1-108b(3-$SCF_3$) |
| Et | D1-108b(3,4-$OCF_2O$-) |
| Et | $CH_2$(D1-2a) |
| Et | $CH_2${D1-5f(3-Me)} |
| Et | $CH_2${D1-8f($X^{1a}$=Me,$X^1$=3,5-$Me_2$)} |
| Et | $CH_2${D1-8f($X^{1a}$=Me,$X^1$=3,5-$Cl_2$)} |
| Et | $CH_2${D1-9e(2-Ph,5-Me)} |
| Et | $CH_2$(D1-28a) |
| Et | $CH_2$(D1-32a) |
| Et | $CH_2$(D1-33a) |
| Et | $CH_2${D1-33b(6-Cl)} |
| Et | $CH_2$(D1-34a) |
| Et | $CH_2${D1-84d[$X^{1b}$=$C(Cl)_3$]} |
| Et | $CH_2${D1-85d[$X^{1b}$=$C(Cl)_3$]} |
| Et | $CH_2$(D1-87a) |
| Et | $CH_2$(D1-93a) |
| Et | $CH_2$(D1-98a) |
| Et | $CH_2${D1-108b(2,3-$OCH_2O$-)} |
| Et | $CH_2CH_2${D1-7b(3-$CF_3$,5-Me)} |
| Et | $CH_2CH_2${D1-7b(3-$CF_3$,5-Pr-c)} |
| n-Pr | CH(Me)SMe |
| n-Pr | CH(Me)S(O)Me |
| n-Pr | CH(Me)$S(O)_2$Me |
| n-Pr | CH(Me)SEt |
| n-Pr | CH(Me)S(O)Et |
| n-Pr | CH(Me)$S(O)_2$Et |
| n-Pr | $CH_2CH_2$SMe |
| n-Pr | $CH_2CH_2$S(O)Me |
| n-Pr | $CH_2CH_2S(O)_2$Me |
| n-Pr | CH(Me)$CH_2$SMe |
| n-Pr | CH(Me)$CH_2$S(O)Me |
| n-Pr | CH(Me)$CH_2S(O)_2$Me |
| n-Pr | OBu-t |
| i-Pr | CH(Me)SMe |

(continued)

| $R^a$ | $R^{b-1}$ |
|---|---|
| i-Pr | CH(Me)S(O)Me |
| i-Pr | $CH(Me)S(O)_2Me$ |
| i-Pr | CH(Me)SEt |
| i-Pr | CH(Me)S(O)Et |
| i-Pr | $CH(Me)S(O)_2Et$ |
| i-Pr | $CH_2CH_2SMe$ |
| i-Pr | $CH_2CH_2S(O)Me$ |
| i-Pr | $CH_2CH_2S(O)_2Me$ |
| i-Pr | $CH(Me)CH_2SMe$ |
| i-Pr | $CH(Me)CH_2S(O)Me$ |
| i-Pr | $CH(Me)CH_2S(O)_2Me$ |
| i-Pr | OBu-t |
| $CH_2$Pr-c | CH(Me)SMe |
| $CH_2$Pr-c | CH(Me)S(O)Me |
| $CH_2$Pr-c | $CH(Me)S(O)_2Me$ |
| $CH_2$Pr-c | CH(Me)SEt |
| $CH_2$Pr-c | CH(Me)S(O)Et |
| $CH_2$Pr-c | $CH(Me)S(O)_2Et$ |
| $CH_2$Pr-c | $CH_2CH_2SMe$ |
| $CH_2$Pr-c | $CH_2CH_2S(O)Me$ |
| $CH_2$Pr-c | $CH_2CH_2S(O)_2Me$ |
| $CH_2$Pr-c | $CH(Me)CH_2SMe$ |
| $CH_2$Pr-c | $CH(Me)CH_2S(O)Me$ |
| $CH_2$Pr-c | $CH(Me)CH_2S(O)_2Me$ |
| $CH_2$Pr-c | OBu-t |
| $CH_2CH=CH_2$ | CH(Me)SMe |
| $CH_2CH=CH_2$ | CH(Me)S(O)Me |
| $CH_2CH=CH_2$ | $CH(Me)S(O)_2Me$ |
| $CH_2CH=CH_2$ | CH(Me)SEt |
| $CH_2CH=CH_2$ | CH(Me)S(O)Et |
| $CH_2CH=CH_2$ | $CH(Me)S(O)_2Et$ |
| $CH_2CH=CH_2$ | $CH_2CH_2SMe$ |
| $CH_2CH=CH_2$ | $CH_2CH_2S(O)Me$ |
| $CH_2CH=CH_2$ | $CH_2CH_2S(O)_2Me$ |
| $CH_2CH=CH_2$ | $CH(Me)CH_2SMe$ |
| $CH_2CH=CH_2$ | $CH(Me)CH_2S(O)Me$ |
| $CH_2CH=CH_2$ | $CH(Me)CH_2S(O)_2Me$ |
| $CH_2C\equiv CH$ | CH(Me)SMe |
| $CH_2C\equiv CH$ | CH(Me)S(O)Me |
| $CH_2C\equiv CH$ | $CH(Me)S(O)_2Me$ |
| $CH_2C\equiv CH$ | CH(Me)SEt |
| $CH_2C\equiv CH$ | CH(Me)S(O)Et |
| $CH_2C\equiv CH$ | $CH(Me)S(O)_2Et$ |
| $CH_2C\equiv CH$ | $CH_2CH_2SMe$ |
| $CH_2C\equiv CH$ | $CH_2CH_2S(O)Me$ |
| $CH_2C\equiv CH$ | $CH_2CH_2S(O)_2Me$ |
| $CH_2C\equiv CH$ | $CH(Me)CH_2SMe$ |
| $CH_2C\equiv CH$ | $CH(Me)CH_2S(O)Me$ |
| $CH_2C\equiv CH$ | $CH(Me)CH_2S(O)_2Me$ |

(continued)

| $R^a$ | $R^{b-1}$ |
|---|---|
| $CH_2OMe$ | CH(Me)SMe |
| $CH_2OMe$ | CH(Me)S(O)Me |
| $CH_2OMe$ | $CH(Me)S(O)_2Me$ |
| $CH_2OMe$ | CH(Me)SEt |
| $CH_2OMe$ | CH(Me)S(O)Et |
| $CH_2OMe$ | $CH(Me)S(O)_2Et$ |
| $CH_2OMe$ | $CH_2CH_2SMe$ |
| $CH_2OMe$ | $CH_2CH_2S(O)Me$ |
| $CH_2OMe$ | $CH_2CH_2S(O)_2Me$ |
| $CH_2OMe$ | $CH(Me)CH_2SMe$ |
| $CH_2OMe$ | $CH(Me)CH_2S(O)Me$ |
| $CH_2OMe$ | $CH(Me)CH_2S(O)_2Me$ |
| $CH_2OEt$ | CH(Me)SEt |
| $CH_2OEt$ | CH(Me)S(O)Et |
| $CH_2OEt$ | $CH(Me)S(O)_2Et$ |
| $CH_2CH_2OMe$ | CH(Me)SEt |
| $CH_2CH_2OMe$ | CH(Me)S(O)Et |
| $CH_2CH_2OMe$ | $CH(Me)S(O)_2Et$ |
| $CH_2CN$ | CH(Me)SMe |
| $CH_2CN$ | CH(Me)S(O)Me |
| $CH_2CN$ | $CH(Me)S(O)_2Me$ |
| $CH_2CN$ | CH(Me)SEt |
| $CH_2CN$ | CH(Me)S(O)Et |
| $CH_2CN$ | $CH(Me)S(O)_2Et$ |
| $CH_2CN$ | $CH_2CH_2SMe$ |
| $CH_2CN$ | $CH_2CH_2S(O)Me$ |
| $CH_2CN$ | $CH_2CH_2S(O)_2Me$ |
| $CH_2CN$ | $CH(Me)CH_2SMe$ |
| $CH_2CN$ | $CH(Me)CH_2S(O)Me$ |
| $CH_2CN$ | $CH(Me)CH_2S(O)_2Me$ |
| $CH_2CN$ | OBu-t |
| $CH_2C(O)OMe$ | $CH_2SMe$ |
| $CH_2C(O)OMe$ | $CH_2S(O)Me$ |
| $CH_2C(O)OMe$ | $CH_2S(O)_2Me$ |
| $CH_2C(O)OMe$ | $CH_2SEt$ |
| $CH_2C(O)OMe$ | $CH_2S(O)Et$ |
| $CH_2C(O)OMe$ | $CH_2S(O)_2Et$ |
| $CH_2C(O)OMe$ | $CH_2SPr$-n |
| $CH_2C(O)OMe$ | $CH_2S(O)Pr$-n |
| $CH_2C(O)OMe$ | $CH_2S(O)_2Pr$-n |
| $CH_2C(O)OMe$ | $CH_2SPr$-i |
| $CH_2C(O)OMe$ | $CH_2S(O)Pr$-i |
| $CH_2C(O)OMe$ | $CH_2S(O)_2Pr$-i |
| $CH_2C(O)OMe$ | $CH_2SPr$-c |
| $CH_2C(O)OMe$ | $CH_2S(O)Pr$-c |
| $CH_2C(O)OMe$ | $CH_2S(O)_2Pr$-c |
| $CH_2C(O)OMe$ | $CH_2SCH_2CH=CH_2$ |
| $CH_2C(O)OMe$ | $CH_2S(O)CH_2CH=CH_2$ |
| $CH_2C(O)OMe$ | $CH_2S(O)_2CH_2CH=CH_2$ |

(continued)

| $R^a$ | $R^{b-1}$ |
|---|---|
| $CH_2C(O)OMe$ | $CH_2SCH_2C\equiv CH$ |
| $CH_2C(O)OMe$ | $CH_2S(O)CH_2C\equiv CH$ |
| $CH_2C(O)OMe$ | $CH_2S(O)_2CH_2C\equiv CH$ |
| $CH_2C(O)OMe$ | $CH_2SCH_2CF_3$ |
| $CH_2C(O)OMe$ | $CH_2S(O)CH_2CF_3$ |
| $CH_2C(O)OMe$ | $CH_2S(O)_2CH_2CF_3$ |
| $CH_2C(O)OMe$ | $CH_2SCH_2CH(OMe)_2$ |
| $CH_2C(O)OMe$ | $CH_2SCH_2CH(=NOMe)$ |
| $CH_2C(O)OMe$ | CH(Me)SMe |
| $CH_2C(O)OMe$ | CH(Me)S(O)Me |
| $CH_2C(O)OMe$ | $CH(Me)S(O)_2Me$ |
| $CH_2C(O)OMe$ | CH(Me)SEt |
| $CH_2C(O)OMe$ | CH(Me)S(O)Et |
| $CH_2C(O)OMe$ | $CH(Me)S(O)_2Et$ |
| $CH_2C(O)OMe$ | CH(Me)S(=NCN)Et |
| $CH_2C(O)OMe$ | CH(Me)S(O)(=NCN)Et |
| $CH_2C(O)OMe$ | CH(Me)SPr-n |
| $CH_2C(O)OMe$ | CH(Me)S(O)Pr-n |
| $CH_2C(O)OMe$ | $CH(Me)S(O)_2Pr$-n |
| $CH_2C(O)OMe$ | CH(Me)SPr-i |
| $CH_2C(O)OMe$ | CH(Me)S(O)Pr-i |
| $CH_2C(O)OMe$ | $CH(Me)S(O)_2Pr$-i |
| $CH_2C(O)OMe$ | CH(Me)SBu-t |
| $CH_2C(O)OMe$ | CH(Me)S(O)Bu-t |
| $CH_2C(O)OMe$ | $CH(Me)S(O)_2Bu$-t |
| $CH_2C(O)OMe$ | $CH(Me)SCH_2Pr$-c |
| $CH_2C(O)OMe$ | $CH(Me)S(O)CH_2Pr$-c |
| $CH_2C(O)OMe$ | $CH(Me)S(O)_2CH_2Pr$-c |
| $CH_2C(O)OMe$ | $CH(Me)SCH_2OMe$ |
| $CH_2C(O)OMe$ | $CH(Me)SCH_2SMe$ |
| $CH_2C(O)OMe$ | $CH(Me)S(O)CH_2SMe$ |
| $CH_2C(O)OMe$ | $CH(Me)S(O)_2CH_2SMe$ |
| $CH_2C(O)OMe$ | $CH(Me)SCH_2C=CH_2$ |
| $CH_2C(O)OMe$ | $CH(Me)S(O)CH_2C=CH_2$ |
| $CH_2C(O)OMe$ | $CH(Me)S(O)_2CH_2C=CH_2$ |
| $CH_2C(O)OMe$ | $CH(Me)SCH_2C\equiv CH$ |
| $CH_2C(O)OMe$ | $CH(Me)S(O)CH_2C\equiv CH$ |
| $CH_2C(O)OMe$ | $CH(Me)S(O)_2CH_2C\equiv CH$ |
| $CH_2C(O)OMe$ | $CH(Me)SCH_2C(O)NHMe$ |
| $CH_2C(O)OMe$ | $CH(Me)S(O)CH_2C(O)NHMe$ |
| $CH_2C(O)OMe$ | $CH(Me)S(O)_2CH_2C(O)NHMe$ |
| $CH_2C(O)OMe$ | $CH(Me)SCH_2C(O)OMe$ |
| $CH_2C(O)OMe$ | $CH(Me)S(O)CH_2C(O)OMe$ |
| $CH_2C(O)OMe$ | $CH(Me)S(O)_2CH_2C(O)OMe$ |
| $CH_2C(O)OMe$ | $CH(Me)SCH_2CH_2CH_2Cl$ |
| $CH_2C(O)OMe$ | $CH(Me)S(O)CH_2CH_2CH_2Cl$ |
| $CH_2C(O)OMe$ | $CH(Me)S(O)_2CH_2CH_2CH_2Cl$ |
| $CH_2C(O)OMe$ | $CH(Me)SCH_2CF_3$ |
| $CH_2C(O)OMe$ | $CH(Me)S(O)CH_2CF_3$ |

(continued)

| $R^a$ | $R^{b-1}$ |
|---|---|
| $CH_2C(O)OMe$ | $CH(Me)S(O)_2CH_2CF_3$ |
| $CH_2C(O)OMe$ | $CH(Me)SCH_2Ph$ |
| $CH_2C(O)OMe$ | $CH(Me)S(O)CH_2Ph$ |
| $CH_2C(O)OMe$ | $CH(Me)S(O)_2CH_2Ph$ |
| $CH_2C(O)OMe$ | $CH(Me)SCH_2$(D1-34a) |
| $CH_2C(O)OMe$ | $H(Me)S(O)CH_2$(D1-34a) |
| $CH_2C(O)OMe$ | $H(Me)S(O)_2CH_2$(D1-34a) |
| $CH_2C(O)OMe$ | $CH(CH_3)SCH_2Si(CH_3)_3$ |
| $CH_2C(O)OMe$ | CH(Me)SCN |
| $CH_2C(O)OMe$ | $CH(Me)SCH_2CN$ |
| $CH_2C(O)OMe$ | CH(Me)SC(O)Me |
| $CH_2C(O)OMe$ | CH(Me)S{D1-12a($X^{1a}$=Me)} |
| $CH_2C(O)OMe$ | CH(Me)S(D1-32a) |
| $CH_2C(O)OMe$ | CH(Me)S{D1-32b(3-$NO_2$)} |
| $CH_2C(O)OMe$ | CH(Me)S{D1-32b(3-$CF_3$)} |
| $CH_2C(O)OMe$ | CH(Me)S(D1-37a) |
| $CH_2C(O)OMe$ | CH(Me)S(D1-51a) |
| $CH_2C(O)OMe$ | CH(Et)SMe |
| $CH_2C(O)OMe$ | CH(Et)S(O)Me |
| $CH_2C(O)OMe$ | $CH(Et)S(O)_2Me$ |
| $CH_2C(O)OMe$ | CH(Et)SEt |
| $CH_2C(O)OMe$ | CH(Et)S(O)Et |
| $CH_2C(O)OMe$ | $CH(Et)S(O)_2Et$ |
| $CH_2C(O)OMe$ | $CH(Et)SCH_2CF_3$ |
| $CH_2C(O)OMe$ | $CH(Et)S(O)CH_2CF_3$ |
| $CH_2C(O)OMe$ | $CH(Et)S(O)_2CH_2CF_3$ |
| $CH_2C(O)OMe$ | CH(Pr-n)SMe |
| $CH_2C(O)OMe$ | CH(Pr-n)S(O)Me |
| $CH_2C(O)OMe$ | $CH(Pr-n)S(O)_2Me$ |
| $CH_2C(O)OMe$ | CH(Pr-n)SEt |
| $CH_2C(O)OMe$ | CH(Pr-n)S(O)Et |
| $CH_2C(O)OMe$ | $CH(Pr-n)S(O)_2Et$ |
| $CH_2C(O)OMe$ | CH(Pr-n)S(=NCN)Et |
| $CH_2C(O)OMe$ | CH(Pr-n)S(O)(=NCN)Et |
| $CH_2C(O)OMe$ | CH(Pr-n)SPr-n |
| $CH_2C(O)OMe$ | CH(Pr-n)S(O)Pr-n |
| $CH_2C(O)OMe$ | $CH(Pr-n)S(O)_2Pr-n$ |
| $CH_2C(O)OMe$ | CH(Pr-n)SPr-i |
| $CH_2C(O)OMe$ | CH(Pr-n)S(O)Pr-i |
| $CH_2C(O)OMe$ | $CH(Pr-n)S(O)_2Pr-i$ |
| $CH_2C(O)OMe$ | CH(Pr-n)SPr-c |
| $CH_2C(O)OMe$ | CH(Pr-n)S(O)Pr-c |
| $CH_2C(O)OMe$ | $CH(Pr-n)S(O)_2Pr-c$ |
| $CH_2C(O)OMe$ | $CH(Pr-n)SCH_2CF_3$ |
| $CH_2C(O)OMe$ | $CH(Pr-n)S(O)CH_2CF_3$ |
| $CH_2C(O)OMe$ | $CH(Pr-n)S(O)_2CH_2CF_3$ |
| $CH_2C(O)OMe$ | CH(Pr-i)SMe |
| $CH_2C(O)OMe$ | CH(Pr-i)S(O)Me |
| $CH_2C(O)OMe$ | $CH(Pr-i)S(O)_2Me$ |

(continued)

| $R^a$ | $R^{b-1}$ |
|---|---|
| $CH_2C(O)OMe$ | CH(Pr-i)SEt |
| $CH_2C(O)OMe$ | CH(Pr-i)S(O)Et |
| $CH_2C(O)OMe$ | CH(Pr-i)$S(O)_2$Et |
| $CH_2C(O)OMe$ | CH(Pr-i)S(=NCN)Et |
| $CH_2C(O)OMe$ | CH(Pr-i)S(O)(=NCN)Et |
| $CH_2C(O)OMe$ | CH(Pr-i)SPr-n |
| $CH_2C(O)OMe$ | CH(Pr-i)S(O)Pr-n |
| $CH_2C(O)OMe$ | CH(Pr-i)$S(O)_2$Pr-n |
| $CH_2C(O)OMe$ | CH(Pr-i)SPr-i |
| $CH_2C(O)OMe$ | CH(Pr-i)S(O)Pr-i |
| $CH_2C(O)OMe$ | CH(Pr-i)$S(O)_2$Pr-i |
| $CH_2C(O)OMe$ | CH(Pr-i)SPr-c |
| $CH_2C(O)OMe$ | CH(Pr-i)S(O)Pr-c |
| $CH_2C(O)OMe$ | CH(Pr-i)$S(O)_2$Pr-c |
| $CH_2C(O)OMe$ | CH(Pr-i)$SCH_2CF_3$ |
| $CH_2C(O)OMe$ | CH(Pr-i)$S(O)CH_2CF_3$ |
| $CH_2C(O)OMe$ | CH(Pr-i)$S(O)_2CH_2CF_3$ |
| $CH_2C(O)OMe$ | CH(Pr-c)SMe |
| $CH_2C(O)OMe$ | CH(Pr-c)S(O)Me |
| $CH_2C(O)OMe$ | CH(Pr-c)$S(O)_2$Me |
| $CH_2C(O)OMe$ | CH(Pr-c)SEt |
| $CH_2C(O)OMe$ | CH(Pr-c)S(O)Et |
| $CH_2C(O)OMe$ | CH(Pr-c)$S(O)_2$Et |
| $CH_2C(O)OMe$ | CH(Pr-c)S(=NCN)Et |
| $CH_2C(O)OMe$ | CH(Pr-c)S(O)(=NCN)Et |
| $CH_2C(O)OMe$ | CH(Pr-c)SPr-n |
| $CH_2C(O)OMe$ | CH(Pr-c)S(O)Pr-n |
| $CH_2C(O)OMe$ | CH(Pr-c)$S(O)_2$Pr-n |
| $CH_2C(O)OMe$ | CH(Pr-c)SPr-i |
| $CH_2C(O)OMe$ | CH(Pr-c)S(O)Pr-i |
| $CH_2C(O)OMe$ | CH(Pr-c)$S(O)_2$Pr-i |
| $CH_2C(O)OMe$ | CH(Pr-c)SPr-c |
| $CH_2C(O)OMe$ | CH(Pr-c)S(O)Pr-c |
| $CH_2C(O)OMe$ | CH(Pr-c)$S(O)_2$Pr-c |
| $CH_2C(O)OMe$ | CH(Pr-c)$SCH_2CF_3$ |
| $CH_2C(O)OMe$ | CH(Pr-c)$S(O)CH_2CF_3$ |
| $CH_2C(O)OMe$ | CH(Pr-c)$S(O)_2CH_2CF_3$ |
| $CH_2C(O)OMe$ | CH(Bu-n)SMe |
| $CH_2C(O)OMe$ | CH(Bu-n)S(O)Me |
| $CH_2C(O)OMe$ | CH(Bu-n)$S(O)_2$Me |
| $CH_2C(O)OMe$ | CH(Bu-n)SEt |
| $CH_2C(O)OMe$ | CH(Bu-n)S(O)Et |
| $CH_2C(O)OMe$ | CH(Bu-n)$S(O)_2$Et |
| $CH_2C(O)OMe$ | CH(Bu-n)S(=NCN)Et |
| $CH_2C(O)OMe$ | CH(Bu-n)S(O)(=NCN)Et |
| $CH_2C(O)OMe$ | CH(Bu-n)SPr-n |
| $CH_2C(O)OMe$ | CH(Bu-n)S(O)Pr-n |
| $CH_2C(O)OMe$ | CH(Bu-n)$S(O)_2$Pr-n |
| $CH_2C(O)OMe$ | CH(Bu-n)SPr-i |

(continued)

| $R^a$ | $R^{b-1}$ |
|---|---|
| $CH_2C(O)OMe$ | CH(Bu-n)S(O)Pr-i |
| $CH_2C(O)OMe$ | CH(Bu-n)$S(O)_2$Pr-i |
| $CH_2C(O)OMe$ | CH(Bu-n)SPr-c |
| $CH_2C(O)OMe$ | CH(Bu-n)S(O)Pr-c |
| $CH_2C(O)OMe$ | CH(Bu-n)$S(O)_2$Pr-c |
| $CH_2C(O)OMe$ | CH(Bu-n)$SCH_2CF_3$ |
| $CH_2C(O)OMe$ | CH(Bu-n)$S(O)CH_2CF_3$ |
| $CH_2C(O)OMe$ | CH(Bu-n)$S(O)_2CH_2CF_3$ |
| $CH_2C(O)OMe$ | CH(F)SMe |
| $CH_2C(O)OMe$ | CH(F)S(O)Me |
| $CH_2C(O)OMe$ | CH(F)$S(O)_2$Me |
| $CH_2C(O)OMe$ | CH(F)SEt |
| $CH_2C(O)OMe$ | CH(F)S(O)Et |
| $CH_2C(O)OMe$ | CH(F)$S(O)_2$Et |
| $CH_2C(O)OMe$ | CH(F)S(=NCN)Et |
| $CH_2C(O)OMe$ | CH(F)S(O)(=NCN)Et |
| $CH_2C(O)OMe$ | CH(F)$SCH_2CF_3$ |
| $CH_2C(O)OMe$ | CH(F)$S(O)CH_2CF_3$ |
| $CH_2C(O)OMe$ | CH(F)$S(O)_2CH_2CF_3$ |
| $CH_2C(O)OMe$ | $C(F)_2SMe$ |
| $CH_2C(O)OMe$ | $C(F)_2S(O)Me$ |
| $CH_2C(O)OMe$ | $C(F)_2S(O)_2Me$ |
| $CH_2C(O)OMe$ | $C(F)_2SEt$ |
| $CH_2C(O)OMe$ | $C(F)_2S(O)Et$ |
| $CH_2C(O)OMe$ | $C(F)_2S(O)_2Et$ |
| $CH_2C(O)OMe$ | $C(F)_2S(=NCN)Et$ |
| $CH_2C(O)OMe$ | $C(F)_2S(O)(=NCN)Et$ |
| $CH_2C(O)OMe$ | $C(F)_2SCH_2CF_3$ |
| $CH_2C(O)OMe$ | $C(F)_2S(O)CH_2CF_3$ |
| $CH_2C(O)OMe$ | $C(F)_2S(O)_2CH_2CF_3$ |
| $CH_2C(O)OMe$ | CH(Cl)SMe |
| $CH_2C(O)OMe$ | CH(Cl)S(O)Me |
| $CH_2C(O)OMe$ | CH(Cl)$S(O)_2$Me |
| $CH_2C(O)OMe$ | CH(Cl)SEt |
| $CH_2C(O)OMe$ | CH(Cl)S(O)Et |
| $CH_2C(O)OMe$ | CH(Cl)$S(O)_2$Et |
| $CH_2C(O)OMe$ | CH(Cl)S(=NCN)Et |
| $CH_2C(O)OMe$ | CH(Cl)S(O)(=NCN)Et |
| $CH_2C(O)OMe$ | CH(Cl)$SCH_2CF_3$ |
| $CH_2C(O)OMe$ | CH(Cl)$S(O)CH_2CF_3$ |
| $CH_2C(O)OMe$ | CH(Cl)$S(O)_2CH_2CF_3$ |
| $CH_2C(O)OMe$ | $C(Cl)_2SMe$ |
| $CH_2C(O)OMe$ | $C(Cl)_2S(O)Me$ |
| $CH_2C(O)OMe$ | $C(Cl)_2S(O)_2Me$ |
| $CH_2C(O)OMe$ | $C(Cl)_2SEt$ |
| $CH_2C(O)OMe$ | $C(Cl)_2S(O)Et$ |
| $CH_2C(O)OMe$ | $C(Cl)_2S(O)_2Et$ |
| $CH_2C(O)OMe$ | $C(Cl)_2S(=NCN)Et$ |
| $CH_2C(O)OMe$ | $C(Cl)_2S(O)(=NCN)Et$ |

(continued)

| $R^a$ | $R^{b-1}$ |
|---|---|
| $CH_2C(O)OMe$ | $C(Cl)_2SCH_2CF_3$ |
| $CH_2C(O)OMe$ | $C(Cl)_2S(O)CH_2CF_3$ |
| $CH_2C(O)OMe$ | $C(Cl)_2S(O)_2CH_2CF_3$ |
| $CH_2C(O)OMe$ | CH(Br)SMe |
| $CH_2C(O)OMe$ | CH(Br)S(O)Me |
| $CH_2C(O)OMe$ | $CH(Br)S(O)_2Me$ |
| $CH_2C(O)OMe$ | CH(Br)SEt |
| $CH_2C(O)OMe$ | CH(Br)S(O)Et |
| $CH_2C(O)OMe$ | $CH(Br)S(O)_2Et$ |
| $CH_2C(O)OMe$ | CH(Br)S(=NCN)Et |
| $CH_2C(O)OMe$ | CH(Br)S(O)(=NCN)Et |
| $CH_2C(O)OMe$ | $CH(Br)SCH_2CF_3$ |
| $CH_2C(O)OMe$ | $CH(Br)S(O)CH_2CF_3$ |
| $CH_2C(O)OMe$ | $CH(Br)S(O)_2CH_2CF_3$ |
| $CH_2C(O)OMe$ | CH(I)SMe |
| $CH_2C(O)OMe$ | CH(I)S(O)Me |
| $CH_2C(O)OMe$ | $CH(I)S(O)_2Me$ |
| $CH_2C(O)OMe$ | CH(I)SEt |
| $CH_2C(O)OMe$ | CH(I)S(O)Et |
| $CH_2C(O)OMe$ | $CH(I)S(O)_2Et$ |
| $CH_2C(O)OMe$ | CH(I)S(=NCN)Et |
| $CH_2C(O)OMe$ | CH(I)S(O)(=NCN)Et |
| $CH_2C(O)OMe$ | $CH(I)SCH_2CF_3$ |
| $CH_2C(O)OMe$ | $CH(I)S(O)CH_2CF_3$ |
| $CH_2C(O)OMe$ | $CH(I)S(O)_2CH_2CF_3$ |
| $CH_2C(O)OMe$ | CH{OC(O)Me}SMe |
| $CH_2C(O)OMe$ | CH{OC(O)Me}S(O)Me |
| $CH_2C(O)OMe$ | $CH\{OC(O)Me\}S(O)_2Me$ |
| $CH_2C(O)OMe$ | CH{OC(O)OMe}SEt |
| $CH_2C(O)OMe$ | CH{OC(O)OMe}S(O)Et |
| $CH_2C(O)OMe$ | $CH\{OC(O)OMe\}S(O)_2Et$ |
| $CH_2C(O)OMe$ | CH{OC(O)OMe}S(=NCN)Et |
| $CH_2C(O)OMe$ | CH{OC(O)OMe}S(O)(=NCN)Et |
| $CH_2C(O)OMe$ | CH{OC(O)OEt}SMe |
| $CH_2C(O)OMe$ | CH{OC(O)OEt}S(O)Me |
| $CH_2C(O)OMe$ | $CH\{OC(O)OEt\}S(O)_2Me$ |
| $CH_2C(O)OMe$ | CH{OC(O)OEt}SEt |
| $CH_2C(O)OMe$ | CH{OC(O)OEt}S(O)Et |
| $CH_2C(O)OMe$ | $CH\{OC(O)OEt\}S(O)_2Et$ |
| $CH_2C(O)OMe$ | CH{OC(O)OEt}S(=NCN)Et |
| $CH_2C(O)OMe$ | CH{OC(O)OEt}S(O)(=NCN)Et |
| $CH_2C(O)OMe$ | CH(OMe)SMe |
| $CH_2C(O)OMe$ | CH(OMe)S(O)Me |
| $CH_2C(O)OMe$ | $CH(OMe)S(O)_2Me$ |
| $CH_2C(O)OMe$ | CH(OMe)SEt |
| $CH_2C(O)OMe$ | CH(OMe)S(O)Et |
| $CH_2C(O)OMe$ | $CH(OMe)S(O)_2Et$ |
| $CH_2C(O)OMe$ | CH(OMe)S(=NCN)Et |
| $CH_2C(O)OMe$ | CH(OMe)S(O)(=NCN)Et |

(continued)

| $R^a$ | $R^{b-1}$ |
|---|---|
| $CH_2C(O)OMe$ | CH(OEt)SMe |
| $CH_2C(O)OMe$ | CH(OEt)S(O)Me |
| $CH_2C(O)OMe$ | $CH(OEt)S(O)_2Me$ |
| $CH_2C(O)OMe$ | CH(OEt)SEt |
| $CH_2C(O)OMe$ | CH(OEt)S(O)Et |
| $CH_2C(O)OMe$ | $CH(OEt)S(O)_2Et$ |
| $CH_2C(O)OMe$ | CH(OEt)S(=NCN)Et |
| $CH_2C(O)OMe$ | CH(OEt)S(O)(=CN)Et |
| $CH_2C(O)OMe$ | $CH(SMe)_2$ |
| $CH_2C(O)OMe$ | CH(SMe)S(O)Me |
| $CH_2C(O)OMe$ | $CH(SMe)S(O)_2Me$ |
| $CH_2C(O)OMe$ | CH(SMe)SEt |
| $CH_2C(O)OMe$ | CH(SMe)S(O)Et |
| $CH_2C(O)OMe$ | $CH(SMe)S(O)_2Et$ |
| $CH_2C(O)OMe$ | CH(SMe)S(=NCN)Et |
| $CH_2C(O)OMe$ | CH(SMe)S(O)(=NCN)Et |
| $CH_2C(O)OMe$ | CH(SEt)SMe |
| $CH_2C(O)OMe$ | CH(SEt)S(O)Me |
| $CH_2C(O)OMe$ | $CH(SEt)S(O)_2Me$ |
| $CH_2C(O)OMe$ | CH(SEt)SEt |
| $CH_2C(O)OMe$ | CH(SEt)S(O)Et |
| $CH_2C(O)OMe$ | $CH(SEt)S(O)_2Et$ |
| $CH_2C(O)OMe$ | CH(SEt)S(=NCN)Et |
| $CH_2C(O)OMe$ | CH(SEt)S(O)(=NCN)Et |
| $CH_2C(O)OMe$ | CH(CN)SMe |
| $CH_2C(O)OMe$ | CH(CN)S(O)Me |
| $CH_2C(O)OMe$ | $CH(CN)S(O)_2Me$ |
| $CH_2C(O)OMe$ | CH(CN)SEt |
| $CH_2C(O)OMe$ | CH(CN)S(O)Et |
| $CH_2C(O)OMe$ | $CH(CN)S(O)_2Et$ |
| $CH_2C(O)OMe$ | CH(CN)S(=NCN)Et |
| $CH_2C(O)OMe$ | CH(CN)S(O)(=NCN)Et |
| $CH_2C(O)OMe$ | CH{C(O)OMe}SMe |
| $CH_2C(O)OMe$ | CH{C(O)OMe}S(O)Me |
| $CH_2C(O)OMe$ | $CH\{C(O)OMe\}S(O)_2Me$ |
| $CH_2C(O)OMe$ | CH{C(O)OMe}SMe |
| $CH_2C(O)OMe$ | CH{C(O)OMe}S(O)Me |
| $CH_2C(O)OMe$ | $CH\{C(O)OMe\}S(O)_2Me$ |
| $CH_2C(O)OMe$ | CH{C(O)OMe}SEt |
| $CH_2C(O)OMe$ | CH{C(O)OMe}S(O)Et |
| $CH_2C(O)OMe$ | $CH\{C(O)OMe\}S(O)_2Et$ |
| $CH_2C(O)OMe$ | CH{C(O)OMe}S(=NCN)Et |
| $CH_2C(O)OMe$ | CH{C(O)OMe}S(O)(=NCN)Et |
| $CH_2C(O)OMe$ | CH{C(O)OEt}SMe |
| $CH_2C(O)OMe$ | CH{C(O)OEt}S(O)Me |
| $CH_2C(O)OMe$ | $CH\{C(O)OEt\}S(O)_2Me$ |
| $CH_2C(O)OMe$ | CH{C(O)OEt}SEt |
| $CH_2C(O)OMe$ | CH{C(O)OEt}S(O)Et |
| $CH_2C(O)OMe$ | $CH\{C(O)OEt\}S(O)_2Et$ |

(continued)

| $R^a$ | $R^{b-1}$ |
|---|---|
| $CH_2C(O)OMe$ | CH{C(O)OEt}S(=NCN)Et |
| $CH_2C(O)OMe$ | CH{C(O)OEt}S(O)(=NCN)Et |
| $CH_2C(O)OMe$ | $C(Me)_2SMe$ |
| $CH_2C(O)OMe$ | $C(Me)_2S(O)Me$ |
| $CH_2C(O)OMe$ | $C(Me)_2S(O)_2Me$ |
| $CH_2C(O)OMe$ | $CH_2CH_2SMe$ |
| $CH_2C(O)OMe$ | $CH_2CH_2S(O)Me$ |
| $CH_2C(O)OMe$ | $CH_2CH_2S(O)_2Me$ |
| $CH_2C(O)OMe$ | $CH(Me)CH_2SMe$ |
| $CH_2C(O)OMe$ | $CH(Me)CH_2S(O)Me$ |
| $CH_2C(O)OMe$ | $CH(Me)CH_2S(O)_2Me$ |
| $CH_2C(O)OMe$ | NHPh |
| $CH_2C(O)OMe$ | NH(D1-1a) |
| $CH_2C(O)OMe$ | NH(D1-2a) |
| $CH_2C(O)OMe$ | NH(D1-2b) |
| $CH_2C(O)OMe$ | NH{D1-4b($X^{1a}$=Me)} |
| $CH_2C(O)OMe$ | NH(D1-5a) |
| $CH_2C(O)OMe$ | NH(D1-5b) |
| $CH_2C(O)OMe$ | NH(D1-5c) |
| $CH_2C(O)OMe$ | NH(D1-6a) |
| $CH_2C(O)OMe$ | NH(D1-6b) |
| $CH_2C(O)OMe$ | NH(D1-6c) |
| $CH_2C(O)OMe$ | NH(D1-7a) |
| $CH_2C(O)OMe$ | NH{D1-8a($X^{1a}$=Me)} |
| $CH_2C(O)OMe$ | NH{D1-8b($X^{1a}$=Me)} |
| $CH_2C(O)OMe$ | NH{D1-8c($X^{1a}$=Me)} |
| $CH_2C(O)OMe$ | NH(D1-9a) |
| $CH_2C(O)OMe$ | NH(D1-9b) |
| $CH_2C(O)OMe$ | NH(D1-9c) |
| $CH_2C(O)OMe$ | NH(D1-10a) |
| $CH_2C(O)OMe$ | NH(D1-10b) |
| $CH_2C(O)OMe$ | NH(D1-10c) |
| $CH_2C(O)OMe$ | NH(D1-11a) |
| $CH_2C(O)OMe$ | NH{D1-12a($X^{1a}$=Me)} |
| $CH_2C(O)OMe$ | NH{D1-12b($X^{1a}$=Me)} |
| $CH_2C(O)OMe$ | NH{D1-12c($X^{1a}$=Me)} |
| $CH_2C(O)OMe$ | NH(D1-13a) |
| $CH_2C(O)OMe$ | NH(D1-13b) |
| $CH_2C(O)OMe$ | NH(D1-14a) |
| $CH_2C(O)OMe$ | NH(D1-14b) |
| $CH_2C(O)OMe$ | NH(D1-15a) |
| $CH_2C(O)OMe$ | NH(D1-15b) |
| $CH_2C(O)OMe$ | NH(D1-16a) |
| $CH_2C(O)OMe$ | NH(D1-17a) |
| $CH_2C(O)OMe$ | NH(D1-18a) |
| $CH_2C(O)OMe$ | NH(D1-19a) |
| $CH_2C(O)OMe$ | NH(D1-20a) |
| $CH_2C(O)OMe$ | NH{D1-21a($X^{1a}$=Me)} |
| $CH_2C(O)OMe$ | NH(D1-21b($X^{1a}$=Me)) |

(continued)

| $R^a$ | $R^{b-1}$ |
|---|---|
| $CH_2C(O)OMe$ | NH(D1-22a) |
| $CH_2C(O)OMe$ | NH{D1-23a($X^{1a}$=Me)} |
| $CH_2C(O)OMe$ | NH{D1-23b($X^{1a}$=Me)} |
| $CH_2C(O)OMe$ | NH(D1-24a) |
| $CH_2C(O)OMe$ | NH{D1-25a($X^{1a}$=Me)} |
| $CH_2C(O)OMe$ | NH(D1-26a) |
| $CH_2C(O)OMe$ | NH{D1-27a($X^{1a}$=Me)} |
| $CH_2C(O)OMe$ | NH(D1-28a) |
| $CH_2C(O)OMe$ | NH(D1-29a) |
| $CH_2C(O)OMe$ | NH{D1-30a($X^{1a}$=Me)} |
| $CH_2C(O)OMe$ | NH{D1-31a($X^{1a}$=Me)} |
| $CH_2C(O)OMe$ | NH(D1-32a) |
| $CH_2C(O)OMe$ | NH(D1-33a) |
| $CH_2C(O)OMe$ | NH(D1-34a) |
| $CH_2C(O)OMe$ | NH(D1-35a) |
| $CH_2C(O)OMe$ | NH(D1-35b) |
| $CH_2C(O)OMe$ | NH(D1-36a) |
| $CH_2C(O)OMe$ | NH(D1-37a) |
| $CH_2C(O)OMe$ | NH(D1-38a) |
| $CH_2C(O)OMe$ | NH(D1-39a) |
| $CH_2C(O)OMe$ | NH(D1-40a) |
| $CH_2C(O)OMe$ | NH(D1-40b) |
| $CH_2C(O)OMe$ | NH(D1-40c) |
| $CH_2C(O)OMe$ | NH(D1-41a) |
| $CH_2C(O)OMe$ | NH(D1-42a) |
| $CH_2C(O)OMe$ | NH(D1-43a) |
| $CH_2C(O)OMe$ | NH(D1-45a) |
| $CH_2C(O)OMe$ | NH(D1-49a) |
| $CH_2C(O)OMe$ | NH(D1-51a) |
| $CH_2C(O)OMe$ | NH(D1-59a) |
| $CH_2C(O)OMe$ | NH(D1-61a) |
| $CH_2C(O)OMe$ | NH(D1-79a) |
| $CH_2C(O)OMe$ | NH(D1-80a) |
| $CH_2C(O)OMe$ | NH{D1-108b(2-Cl)} |
| $CH_2C(O)OMe$ | NH{D1-108b(3-Cl)} |
| $CH_2C(O)OMe$ | NH{D1-108b(4-Cl)} |
| $CH_2C(O)OMe$ | NH{D1-108b(2-Me)} |
| $CH_2C(O)OMe$ | NH{D1-108b(3-Me)} |
| $CH_2C(O)OMe$ | NH{D1-108b(4-Me)} |
| $CH_2C(O)OMe$ | N(Me)Ph |
| $CH_2C(O)OMe$ | N(Me)(D1-1a) |
| $CH_2C(O)OMe$ | N(Me)(D1-2a) |
| $CH_2C(O)OMe$ | N(Me)(D1-2b) |
| $CH_2C(O)OMe$ | N(Me){D1-4a($X^{1a}$=Me)} |
| $CH_2C(O)OMe$ | N(Me){D1-4b($X^{1a}$=Me)} |
| $CH_2C(O)OMe$ | N(Me)(D1-5a) |
| $CH_2C(O)OMe$ | N(Me)(D1-5b) |
| $CH_2C(O)OMe$ | N(Me)(D1-5c) |
| $CH_2C(O)OMe$ | N(Me)(D1-6a) |

(continued)

| $R^a$ | $R^{b-1}$ |
|---|---|
| $CH_2C(O)OMe$ | N(Me)(D1-6b) |
| $CH_2C(O)OMe$ | N(Me)(D1-6c) |
| $CH_2C(O)OMe$ | N(Me)(D1-7a) |
| $CH_2C(O)OMe$ | N(Me){D1-8a($X^{1a}$=Me)} |
| $CH_2C(O)OMe$ | N(Me){D1-8b($X^{1a}$=Me)} |
| $CH_2C(O)OMe$ | N(Me){D1-8c($X^{1a}$=Me)} |
| $CH_2C(O)OMe$ | N(Me)(D1-9a) |
| $CH_2C(O)OMe$ | N(Me)(D1-9b) |
| $CH_2C(O)OMe$ | N(Me)(D1-9c) |
| $CH_2C(O)OMe$ | N(Me)(D1-10a) |
| $CH_2C(O)OMe$ | N(Me)(D1-10b) |
| $CH_2C(O)OMe$ | N(Me)(D1-10c) |
| $CH_2C(O)OMe$ | N(Me)(D1-11a) |
| $CH_2C(O)OMe$ | N(Me){D1-12a($X^{1a}$=Me)} |
| $CH_2C(O)OMe$ | N(Me){D1-12b($X^{1a}$=Me)} |
| $CH_2C(O)OMe$ | N(Me){D1-12c($X^{1a}$=Me)} |
| $CH_2C(O)OMe$ | N(Me)(D1-13a) |
| $CH_2C(O)OMe$ | N(Me)(D1-13b) |
| $CH_2C(O)OMe$ | N(Me)(D1-14a) |
| $CH_2C(O)OMe$ | N(Me)(D1-14b) |
| $CH_2C(O)OMe$ | N(Me)(D1-15a) |
| $CH_2C(O)OMe$ | N(Me)(D1-15b) |
| $CH_2C(O)OMe$ | N(Me)(D1-16a) |
| $CH_2C(O)OMe$ | N(Me)(D1-17a) |
| $CH_2C(O)OMe$ | N(Me)(D1-18a) |
| $CH_2C(O)OMe$ | N(Me)(D1-19a) |
| $CH_2C(O)OMe$ | N(Me)(D1-20a) |
| $CH_2C(O)OMe$ | N(Me){D1-21a($X^{1a}$=Me)} |
| $CH_2C(O)OMe$ | N(Me){D1-21b($X^{1a}$=Me)} |
| $CH_2C(O)OMe$ | N(Me)(D1-22a) |
| $CH_2C(O)OMe$ | N(Me){D1-23a($X^{1a}$=Me)} |
| $CH_2C(O)OMe$ | N(Me){D1-23b($X^{1a}$=Me)} |
| $CH_2C(O)OMe$ | N(Me)(D1-24a) |
| $CH_2C(O)OMe$ | N(Me){D1-25a($X^{1a}$=Me)} |
| $CH_2C(O)OMe$ | N(Me)(D1-26a) |
| $CH_2C(O)OMe$ | N(Me){D1-27a($X^{1a}$=Me)} |
| $CH_2C(O)OMe$ | N(Me)(D1-28a) |
| $CH_2C(O)OMe$ | N(Me)(D1-29a) |
| $CH_2C(O)OMe$ | N(Me){D1-30a($X^{1a}$=Me)} |
| $CH_2C(O)OMe$ | N(Me){D1-31a($X^{1a}$=Me)} |
| $CH_2C(O)OMe$ | N(Me)(D1-32a) |
| $CH_2C(O)OMe$ | N(Me)(D1-34a) |
| $CH_2C(O)OMe$ | N(Me)(D1-35a) |
| $CH_2C(O)OMe$ | N(Me)(D1-35b) |
| $CH_2C(O)OMe$ | N(Me)(D1-36a) |
| $CH_2C(O)OMe$ | N(Me)(D1-37a) |
| $CH_2C(O)OMe$ | N(Me)(D1-38a) |
| $CH_2C(O)OMe$ | N(Me)(D1-39a) |
| $CH_2C(O)OMe$ | N(Me)(D1-40a) |

(continued)

| $R^a$ | $R^{b-1}$ |
|---|---|
| $CH_2C(O)OMe$ | N(Me)(D1-40b) |
| $CH_2C(O)OMe$ | N(Me)(D1-40c) |
| $CH_2C(O)OMe$ | N(Me)(D1-41a) |
| $CH_2C(O)OMe$ | N(Me)(D1-42a) |
| $CH_2C(O)OMe$ | N(Me)(D1-43a) |
| $CH_2C(O)OMe$ | N(Me)(D1-45a) |
| $CH_2C(O)OMe$ | N(Me)(D1-49a) |
| $CH_2C(O)OMe$ | N(Me)(D1-51 a) |
| $CH_2C(O)OMe$ | N(Me)(D1-59a) |
| $CH_2C(O)OMe$ | N(Me)(D1-61a) |
| $CH_2C(O)OMe$ | N(Me)(D1-79a) |
| $CH_2C(O)OMe$ | N(Me)(D1-80a) |
| $CH_2C(O)OMe$ | N(Me){D1-108b(2-Cl)} |
| $CH_2C(O)OMe$ | N(Me){D1-108b(3-Cl)} |
| $CH_2C(O)OMe$ | N(Me){D1-108b(4-Cl)} |
| $CH_2C(O)OMe$ | N(Me){D1-108b(2-Me)} |
| $CH_2C(O)OMe$ | N(Me){D1-108b(3-Me)} |
| $CH_2C(O)OMe$ | N(Me){D1-108b(4-Me)} |
| $CH_2C(O)OEt$ | $CH_2SMe$ |
| $CH_2C(O)OEt$ | $CH_2S(O)Me$ |
| $CH_2C(O)OEt$ | $CH_2S(O)_2Me$ |
| $CH_2C(O)OEt$ | $CH_2SEt$ |
| $CH_2C(O)OEt$ | $CH_2S(O)Et$ |
| $CH_2C(O)OEt$ | $CH_2S(O)_2Et$ |
| $CH_2C(O)OEt$ | $CH_2SPr$-n |
| $CH_2C(O)OEt$ | $CH_2S(O)Pr$-n |
| $CH_2C(O)OEt$ | $CH_2S(O)_2Pr$-n |
| $CH_2C(O)OEt$ | $CH_2SPr$-i |
| $CH_2C(O)OEt$ | $CH_2S(O)Pr$-i |
| $CH_2C(O)OEt$ | $CH_2S(O)_2Pr$-i |
| $CH_2C(O)OEt$ | $CH_2SPr$-c |
| $CH_2C(O)OEt$ | $CH_2S(O)Pr$-c |
| $CH_2C(O)OEt$ | $CH_2S(O)_2Pr$-c |
| $CH_2C(O)OEt$ | $CH_2SCH_2CH=CH_2$ |
| $CH_2C(O)OEt$ | $CH_2S(O)CH_2CH=CH_2$ |
| $CH_2C(O)OEt$ | $CH_2S(O)_2CH_2CH=CH_2$ |
| $CH_2C(O)OEt$ | $CH_2SCH_2C\equiv CH$ |
| $CH_2C(O)OEt$ | $CH_2S(O)CH_2C\equiv CH$ |
| $CH_2C(O)OEt$ | $CH_2S(O)_2CH_2C\equiv CH$ |
| $CH_2C(O)OEt$ | $CH_2SCH_2CF_3$ |
| $CH_2C(O)OEt$ | $CH_2S(O)CH_2CF_3$ |
| $CH_2C(O)OEt$ | $CH_2S(O)_2CH_2CF_3$ |
| $CH_2C(O)OEt$ | $CH_2SCH_2CH(OMe)2$ |
| $CH_2C(O)OEt$ | $CH_2SCH_2CH(=NOMe)$ |
| $CH_2C(O)OEt$ | CH(Me)SMe |
| $CH_2C(O)OEt$ | CH(Me)S(O)Me |
| $CH_2C(O)OEt$ | $CH(Me)S(O)_2Me$ |
| $CH_2C(O)OEt$ | CH(Me)SEt |
| $CH_2C(O)OEt$ | CH(Me)S(O)Et |

(continued)

| $R^a$ | $R^{b-1}$ |
|---|---|
| $CH_2C(O)OEt$ | $CH(Me)S(O)_2Et$ |
| $CH_2C(O)OEt$ | CH(Me)S(=NCN)Et |
| $CH_2C(O)OEt$ | CH(Me)S(O)(=NCN)Et |
| $CH_2C(O)OEt$ | CH(Me)SPr-n |
| $CH_2C(O)OEt$ | CH(Me)S(O)Pr-n |
| $CH_2C(O)OEt$ | $CH(Me)S(O)_2Pr$-n |
| $CH_2C(O)OEt$ | CH(Me)SPr-i |
| $CH_2C(O)OEt$ | CH(Me)S(O)Pr-i |
| $CH_2C(O)OEt$ | $CH(Me)S(O)_2Pr$-i |
| $CH_2C(O)OEt$ | CH(Me)SBu-t |
| $CH_2C(O)OEt$ | CH(Me)S(O)Bu-t |
| $CH_2C(O)OEt$ | $CH(Me)S(O)_2Bu$-t |
| $CH_2C(O)OEt$ | $CH(Me)SCH_2Pr$-c |
| $CH_2C(O)OEt$ | $CH(Me)S(O)CH_2Pr$-c |
| $CH_2C(O)OEt$ | $CH(Me)S(O)_2CH_2Pr$-c |
| $CH_2C(O)OEt$ | $CH(Me)SCH_2OMe$ |
| $CH_2C(O)OEt$ | $CH(Me)SCH_2SMe$ |
| $CH_2C(O)OEt$ | $CH(Me)S(O)CH_2SMe$ |
| $CH_2C(O)OEt$ | $CH(Me)S(O)_2CH_2SMe$ |
| $CH_2C(O)OEt$ | $CH(Me)SCH_2C=CH_2$ |
| $CH_2C(O)OEt$ | $CH(Me)S(O)CH_2C=CH_2$ |
| $CH_2C(O)OEt$ | $CH(Me)S(O)_2CH_2C=CH_2$ |
| $CH_2C(O)OEt$ | $CH(Me)SCH_2C\equiv CH$ |
| $CH_2C(O)OEt$ | $CH(Me)S(O)CH_2C\equiv CH$ |
| $CH_2C(O)OEt$ | $CH(Me)S(O)_2CH_2C\equiv CH$ |
| $CH_2C(O)OEt$ | $CH(Me)SCH_2C(O)NHMe$ |
| $CH_2C(O)OEt$ | $CH(Me)S(O)CH_2C(O)NHMe$ |
| $CH_2C(O)OEt$ | $CH(Me)S(O)_2CH_2C(O)NHMe$ |
| $CH_2C(O)OEt$ | $CH(Me)SCH_2C(O)OMe$ |
| $CH_2C(O)OEt$ | $CH(Me)S(O)CH_2C(O)OMe$ |
| $CH_2C(O)OEt$ | $CH(Me)S(O)_2CH_2C(O)OMe$ |
| $CH_2C(O)OEt$ | $CH(Me)SCH_2CH_2CH_2Cl$ |
| $CH_2C(O)OEt$ | $CH(Me)S(O)CH_2CH_2CH_2Cl$ |
| $CH_2C(O)OEt$ | $CH(Me)S(O)_2CH_2CH_2CH_2Cl$ |
| $CH_2C(O)OEt$ | $CH(Me)SCH_2CF_3$ |
| $CH_2C(O)OEt$ | $CH(Me)S(O)CH_2CF_3$ |
| $CH_2C(O)OEt$ | $CH(Me)S(O)_2CH_2CF_3$ |
| $CH_2C(O)OEt$ | $CH(Me)SCH_2Ph$ |
| $CH_2C(O)OEt$ | $CH(Me)S(O)CH_2Ph$ |
| $CH_2C(O)OEt$ | $CH(Me)S(O)_2CH_2Ph$ |
| $CH_2C(O)OEt$ | $CH(Me)SCH_2$(D1-34a) |
| $CH_2C(O)OEt$ | $CH(Me)S(O)CH_2$(D1-34a) |
| $CH_2C(O)OEt$ | $CH(Me)S(O)_2CH_2$(D1-34a) |
| $CH_2C(O)OEt$ | $CH(CH_3)SCH_2Si(CH_3)_3$ |
| $CH_2C(O)OEt$ | CH(Me)SCN |
| $CH_2C(O)OEt$ | $CH(Me)SCH_2CN$ |
| $CH_2C(O)OEt$ | CH(Me)SC(O)Me |
| $CH_2C(O)OEt$ | CH(Me)S{D1-12a($X^{1a}$=Me)} |
| $CH_2C(O)OEt$ | CH(Me)S(D1-32a) |

(continued)

| $R^a$ | $R^{b-1}$ |
|---|---|
| $CH_2C(O)OEt$ | CH(Me)S{D1-32b(3-$NO_2$)} |
| $CH_2C(O)OEt$ | CH(Me)S{D1-32b(3-CF3)} |
| $CH_2C(O)OEt$ | CH(Me)S(D1-37a) |
| $CH_2C(O)OEt$ | CH(Me)S(D1-51a) |
| $CH_2C(O)OEt$ | CH(Et)SMe |
| $CH_2C(O)OEt$ | CH(Et)S(O)Me |
| $CH_2C(O)OEt$ | CH(Et)S$(O)_2$Me |
| $CH_2C(O)OEt$ | CH(Et)SEt |
| $CH_2C(O)OEt$ | CH(Et)S(O)Et |
| $CH_2C(O)OEt$ | CH(Et)S$(O)_2$Et |
| $CH_2C(O)OEt$ | CH(Et)$SCH_2CF_3$ |
| $CH_2C(O)OEt$ | CH(Et)S(O)$CH_2CF_3$ |
| $CH_2C(O)OEt$ | CH(Et)S$(O)_2CH_2CF_3$ |
| $CH_2C(O)OEt$ | CH(Pr-n)SMe |
| $CH_2C(O)OEt$ | CH(Pr-n)S(O)Me |
| $CH_2C(O)OEt$ | CH(Pr-n)S$(O)_2$Me |
| $CH_2C(O)OEt$ | CH(Pr-n)SEt |
| $CH_2C(O)OEt$ | CH(Pr-n)S(O)Et |
| $CH_2C(O)OEt$ | CH(Pr-n)S$(O)_2$Et |
| $CH_2C(O)OEt$ | CH(Pr-n)S(=NCN)Et |
| $CH_2C(O)OEt$ | CH(Pr-n)S(O)(=NCN)Et |
| $CH_2C(O)OEt$ | CH(Pr-n)SPr-n |
| $CH_2C(O)OEt$ | CH(Pr-n)S(O)Pr-n |
| $CH_2C(O)OEt$ | CH(Pr-n)S$(O)_2$Pr-n |
| $CH_2C(O)OEt$ | CH(Pr-n)SPr-i |
| $CH_2C(O)OEt$ | CH(Pr-n)S(O)Pr-i |
| $CH_2C(O)OEt$ | CH(Pr-n)S$(O)_2$Pr-i |
| $CH_2C(O)OEt$ | CH(Pr-n)SPr-c |
| $CH_2C(O)OEt$ | CH(Pr-n)S(O)Pr-c |
| $CH_2C(O)OEt$ | CH(Pr-n)S$(O)_2$Pr-c |
| $CH_2C(O)OEt$ | CH(Pr-n)$SCH_2CF_3$ |
| $CH_2C(O)OEt$ | CH(Pr-n)S(O)$CH_2CF_3$ |
| $CH_2C(O)OEt$ | CH(Pr-n)S$(O)_2CH_2CF_3$ |
| $CH_2C(O)OEt$ | CH(Pr-i)SMe |
| $CH_2C(O)OEt$ | CH(Pr-i)S(O)Me |
| $CH_2C(O)OEt$ | CH(Pr-i)S$(O)_2$Me |
| $CH_2C(O)OEt$ | CH(Pr-i)SEt |
| $CH_2C(O)OEt$ | CH(Pr-i)S(O)Et |
| $CH_2C(O)OEt$ | CH(Pr-i)S$(O)_2$Et |
| $CH_2C(O)OEt$ | CH(Pr-i)S(=NCN)Et |
| $CH_2C(O)OEt$ | CH(Pr-i)S(O)(=NCN)Et |
| $CH_2C(O)OEt$ | CH(Pr-i)SPr-n |
| $CH_2C(O)OEt$ | CH(Pr-i)S(O)Pr-n |
| $CH_2C(O)OEt$ | CH(Pr-i)S$(O)_2$Pr-n |
| $CH_2C(O)OEt$ | CH(Pr-i)SPr-i |
| $CH_2C(O)OEt$ | CH(Pr-i)S(O)Pr-i |
| $CH_2C(O)OEt$ | CH(Pr-i)S$(O)_2$Pr-i |
| $CH_2C(O)OEt$ | CH(Pr-i)SPr-c |
| $CH_2C(O)OEt$ | CH(Pr-i)S(O)Pr-c |

(continued)

| $R^a$ | $R^{b-1}$ |
|---|---|
| $CH_2C(O)OEt$ | $CH(Pr-i)S(O)_2Pr-c$ |
| $CH_2C(O)OEt$ | $CH(Pr-i)SCH_2CF_3$ |
| $CH_2C(O)OEt$ | $CH(Pr-i)S(O)CH_2CF_3$ |
| $CH_2C(O)OEt$ | $CH(Pr-i)S(O)_2CH_2CF_3$ |
| $CH_2C(O)OEt$ | CH(Pr-c)SMe |
| $CH_2C(O)OEt$ | CH(Pr-c)S(O)Me |
| $CH_2C(O)OEt$ | $CH(Pr-c)S(O)_2Me$ |
| $CH_2C(O)OEt$ | CH(Pr-c)SEt |
| $CH_2C(O)OEt$ | CH(Pr-c)S(O)Et |
| $CH_2C(O)OEt$ | $CH(Pr-c)S(O)_2Et$ |
| $CH_2C(O)OEt$ | CH(Pr-c)S(=NCN)Et |
| $CH_2C(O)OEt$ | CH(Pr-c)S(O)(=NCN)Et |
| $CH_2C(O)OEt$ | CH(Pr-c)SPr-n |
| $CH_2C(O)OEt$ | CH(Pr-c)S(O)Pr-n |
| $CH_2C(O)OEt$ | $CH(Pr-c)S(O)_2Pr-n$ |
| $CH_2C(O)OEt$ | CH(Pr-c)SPr-i |
| $CH_2C(O)OEt$ | CH(Pr-c)S(O)Pr-i |
| $CH_2C(O)OEt$ | $CH(Pr-c)S(O)_2Pr-i$ |
| $CH_2C(O)OEt$ | CH(Pr-c)SPr-c |
| $CH_2C(O)OEt$ | CH(Pr-c)S(O)Pr-c |
| $CH_2C(O)OEt$ | $CH(Pr-c)S(O)_2$ Pr-c |
| $CH_2C(O)OEt$ | $CH(Pr-c)SCH_2CF_3$ |
| $CH_2C(O)OEt$ | $CH(Pr-c)S(O)CH_2CF_3$ |
| $CH_2C(O)OEt$ | $CH(Pr-c)S(O)_2CH_2CF_3$ |
| $CH_2C(O)OEt$ | CH(Bu-n)SMe |
| $CH_2C(O)OEt$ | CH(Bu-n)S(O)Me |
| $CH_2C(O)OEt$ | $CH(Bu-n)S(O)_2Me$ |
| $CH_2C(O)OEt$ | CH(Bu-n)SEt |
| $CH_2C(O)OEt$ | CH(Bu-n)S(O)Et |
| $CH_2C(O)OEt$ | $CH(Bu-n)S(O)_2Et$ |
| $CH_2C(O)OEt$ | CH(Bu-n)S(=NCN)Et |
| $CH_2C(O)OEt$ | CH(Bu-n)S(O)(=NCN)Et |
| $CH_2C(O)OEt$ | CH(Bu-n)SPr-n |
| $CH_2C(O)OEt$ | CH(Bu-n)S(O)Pr-n |
| $CH_2C(O)OEt$ | $CH(Bu-n)S(O)_2Pr-n$ |
| $CH_2C(O)OEt$ | CH(Bu-n)SPr-i |
| $CH_2C(O)OEt$ | CH(Bu-n)S(O)Pr-i |
| $CH_2C(O)OEt$ | $CH(Bu-n)S(O)_2Pr-i$ |
| $CH_2C(O)OEt$ | CH(Bu-n)SPr-c |
| $CH_2C(O)OEt$ | CH(Bu-n)S(O)Pr-c |
| $CH_2C(O)OEt$ | $CH(Bu-n)S(O)_2Pr-c$ |
| $CH_2C(O)OEt$ | $CH(Bu-n)SCH_2CF_3$ |
| $CH_2C(O)OEt$ | $CH(Bu-n)S(O)CH_2CF_3$ |
| $CH_2C(O)OEt$ | $CH(Bu-n)S(O)_2CH_2CF_3$ |
| $CH_2C(O)OEt$ | CH(F)SMe |
| $CH_2C(O)OEt$ | CH(F)S(O)Me |
| $CH_2C(O)OEt$ | $CH(F)S(O)_2Me$ |
| $CH_2C(O)OEt$ | CH(F)SEt |
| $CH_2C(O)OEt$ | CH(F)S(O)Et |

(continued)

| $R^a$ | $R^{b-1}$ |
|---|---|
| $CH_2C(O)OEt$ | $CH(F)S(O)_2Et$ |
| $CH_2C(O)OEt$ | CH(F)S(=NCN)Et |
| $CH_2C(O)OEt$ | CH(F)S(O)(=NCN)Et |
| $CH_2C(O)OEt$ | $CH(F)SCH_2CF_3$ |
| $CH_2C(O)OEt$ | $CH(F)S(O)CH_2CF_3$ |
| $CH_2C(O)OEt$ | $CH(F)S(O)_2CH_2CF_3$ |
| $CH_2C(O)OEt$ | $C(F)_2SMe$ |
| $CH_2C(O)OEt$ | $C(F)_2S(O)Me$ |
| $CH_2C(O)OEt$ | $C(F)_2S(O)_2Me$ |
| $CH_2C(O)OEt$ | $C(F)_2SEt$ |
| $CH_2C(O)OEt$ | $C(F)_2S(O)Et$ |
| $CH_2C(O)OEt$ | $C(F)_2S(O)_2Et$ |
| $CH_2C(O)OEt$ | $C(F)_2S(=NCN)Et$ |
| $CH_2C(O)OEt$ | $C(F)_2S(O)(=NCN)Et$ |
| $CH_2C(O)OEt$ | $C(F)_2SCH_2CF_3$ |
| $CH_2C(O)OEt$ | $C(F)_2S(O)CH_2CF_3$ |
| $CH_2C(O)OEt$ | $C(F)_2S(O)_2CH_2CF_3$ |
| $CH_2C(O)OEt$ | CH(Cl)SMe |
| $CH_2C(O)OEt$ | CH(Cl)S(O)Me |
| $CH_2C(O)OEt$ | $CH(Cl)S(O)_2Me$ |
| $CH_2C(O)OEt$ | CH(Cl)SEt |
| $CH_2C(O)OEt$ | CH(Cl)S(O)Et |
| $CH_2C(O)OEt$ | $CH(Cl)S(O)_2Et$ |
| $CH_2C(O)OEt$ | CH(Cl)S(=NCN)Et |
| $CH_2C(O)OEt$ | CH(Cl)S(O)(=NCN)Et |
| $CH_2C(O)OEt$ | $CH(Cl)SCH_2CF_3$ |
| $CH_2C(O)OEt$ | $CH(Cl)S(O)CH_2CF_3$ |
| $CH_2C(O)OEt$ | $CH(Cl)S(O)_2CH_2CF_3$ |
| $CH_2C(O)OEt$ | $C(Cl)_2SMe$ |
| $CH_2C(O)OEt$ | $C(Cl)_2S(O)Me$ |
| $CH_2C(O)OEt$ | $C(Cl)_2S(O)_2Me$ |
| $CH_2C(O)OEt$ | $C(Cl)_2$ SEt |
| $CH_2C(O)OEt$ | $C(Cl)_2S(O)Et$ |
| $CH_2C(O)OEt$ | $C(Cl)_2S(O)_2Et$ |
| $CH_2C(O)OEt$ | $C(Cl)_2S(=NCN)Et$ |
| $CH_2C(O)OEt$ | $C(Cl)_2S(O)(=NCN)Et$ |
| $CH_2C(O)OEt$ | $C(Cl)_2SCH_2CF_3$ |
| $CH_2C(O)OEt$ | $C(Cl)_2S(O)CH_2CF_3$ |
| $CH_2C(O)OEt$ | $C(Cl)_2S(O)_2CH_2CF_3$ |
| $CH_2C(O)OEt$ | CH(Br)SMe |
| $CH_2C(O)OEt$ | CH(Br)S(O)Me |
| $CH_2C(O)OEt$ | $CH(Br)S(O)_2Me$ |
| $CH_2C(O)OEt$ | CH(Br)SEt |
| $CH_2C(O)OEt$ | CH(Br)S(O)Et |
| $CH_2C(O)OEt$ | $CH(Br)S(O)_2Et$ |
| $CH_2C(O)OEt$ | CH(Br)S(=NCN)Et |
| $CH_2C(O)OEt$ | CH(Br)S(O)(=NCN)Et |
| $CH_2C(O)OEt$ | $CH(Br)SCH_2CF_3$ |
| $CH_2C(O)OEt$ | $CH(Br)S(O)CH_2CF_3$ |

(continued)

| $R^a$ | $R^{b-1}$ |
|---|---|
| $CH_2C(O)OEt$ | $CH(Br)S(O)_2CH_2CF_3$ |
| $CH_2C(O)OEt$ | CH(I)SMe |
| $CH_2C(O)OEt$ | CH(I)S(O)Me |
| $CH_2C(O)OEt$ | $CH(I)S(O)_2Me$ |
| $CH_2C(O)OEt$ | CH(I)SEt |
| $CH_2C(O)OEt$ | CH(I)S(O)Et |
| $CH_2C(O)OEt$ | $CH(I)S(O)_2Et$ |
| $CH_2C(O)OEt$ | CH(I)S(=NCN)Et |
| $CH_2C(O)OEt$ | CH(I)S(O)(=NCN)Et |
| $CH_2C(O)OEt$ | $CH(I)SCH_2CF_3$ |
| $CH_2C(O)OEt$ | $CH(I)S(O)CH_2CF_3$ |
| $CH_2C(O)OEt$ | $CH(I)S(O)_2CH_2CF_3$ |
| $CH_2C(O)OEt$ | CH{OC(O)Me}SMe |
| $CH_2C(O)OEt$ | CH{OC(O)Me}S(O)Me |
| $CH_2C(O)OEt$ | $CH\{OC(O)Me\}S(O)_2Me$ |
| $CH_2C(O)OEt$ | CH{OC(O)OMe}SEt |
| $CH_2C(O)OEt$ | CH{OC(O)OMe}S(O)Et |
| $CH_2C(O)OEt$ | $CH\{OC(O)OMe\}S(O)_2Et$ |
| $CH_2C(O)OEt$ | CH{OC(O)OMe}S(=NCN)Et |
| $CH_2C(O)OEt$ | CH{OC(O)OMe}S(O)(=NCN)Et |
| $CH_2C(O)OEt$ | CH{OC(O)OEt}SMe |
| $CH_2C(O)OEt$ | CH{OC(O)OEt}S(O)Me |
| $CH_2C(O)OEt$ | $CH\{OC(O)OEt\}S(O)_2Me$ |
| $CH_2C(O)OEt$ | CH{OC(O)OEt}SEt |
| $CH_2C(O)OEt$ | CH{OC(O)OEt}S(O)Et |
| $CH_2C(O)OEt$ | $CH\{OC(O)OEt\}S(O)_2Et$ |
| $CH_2C(O)OEt$ | CH{OC(O)OEt}S(=NCN)Et |
| $CH_2C(O)OEt$ | CH{OC(O)OEt}S(O)(=NCN)Et |
| $CH_2C(O)OEt$ | CH(OMe)SMe |
| $CH_2C(O)OEt$ | CH(OMe)S(O)Me |
| $CH_2C(O)OEt$ | $CH(OMe)S(O)_2Me$ |
| $CH_2C(O)OEt$ | CH(OMe)SEt |
| $CH_2C(O)OEt$ | CH(OMe)S(O)Et |
| $CH_2C(O)OEt$ | $CH(OMe)S(O)_2Et$ |
| $CH_2C(O)OEt$ | CH(OMe)S(=NCN)Et |
| $CH_2C(O)OEt$ | CH(OMe)S(O)(=NCN)Et |
| $CH_2C(O)OEt$ | CH(OEt)SMe |
| $CH_2C(O)OEt$ | CH(OEt)S(O)Me |
| $CH_2C(O)OEt$ | $CH(OEt)S(O)_2Me$ |
| $CH_2C(O)OEt$ | CH(OEt)SEt |
| $CH_2C(O)OEt$ | CH(OEt)S(O)Et |
| $CH_2C(O)OEt$ | $CH(OEt)S(O)_2Et$ |
| $CH_2C(O)OEt$ | CH(OEt)S(=NCN)Et |
| $CH_2C(O)OEt$ | CH(OEt)S(O)(=NCN)Et |
| $CH_2C(O)OEt$ | $CH(SMe)_2$ |
| $CH_2C(O)OEt$ | CH(SMe)S(O)Me |
| $CH_2C(O)OEt$ | $CH(SMe)S(O)_2Me$ |
| $CH_2C(O)OEt$ | CH(SMe)SEt |
| $CH_2C(O)OEt$ | CH(SMe)S(O)Et |

(continued)

| $R^a$ | $R^{b-1}$ |
|---|---|
| $CH_2C(O)OEt$ | $CH(SMe)S(O)_2Et$ |
| $CH_2C(O)OEt$ | CH(SMe)S(=NCN)Et |
| $CH_2C(O)OEt$ | CH(SMe)S(O)(=NCN)Et |
| $CH_2C(O)OEt$ | CH(SEt)SMe |
| $CH_2C(O)OEt$ | CH(SEt)S(O)Me |
| $CH_2C(O)OEt$ | $CH(SEt)S(O)_2Me$ |
| $CH_2C(O)OEt$ | CH(SEt)SEt |
| $CH_2C(O)OEt$ | CH(SEt)S(O)Et |
| $CH_2C(O)OEt$ | $CH(SEt)S(O)_2Et$ |
| $CH_2C(O)OEt$ | CH(SEt)S(=NCN)Et |
| $CH_2C(O)OEt$ | CH(SEt)S(O)(=NCN)Et |
| $CH_2C(O)OEt$ | CH(CN)SMe |
| $CH_2C(O)OEt$ | CH(CN)S(O)Me |
| $CH_2C(O)OEt$ | $CH(CN)S(O)_2Me$ |
| $CH_2C(O)OEt$ | CH(CN)SEt |
| $CH_2C(O)OEt$ | CH(CN)S(O)Et |
| $CH_2C(O)OEt$ | $CH(CN)S(O)_2Et$ |
| $CH_2C(O)OEt$ | CH(CN)S(=NCN)Et |
| $CH_2C(O)OEt$ | CH(CN)S(O)(=NCN)Et |
| $CH_2C(O)OEt$ | CH{C(O)OMe}SMe |
| $CH_2C(O)OEt$ | CH{C(O)OMe}S(O)Me |
| $CH_2C(O)OEt$ | $CH\{C(O)OMe\}S(O)_2Me$ |
| $CH_2C(O)OEt$ | CH{C(O)OMe}SMe |
| $CH_2C(O)OEt$ | CH{C(O)OMe}S(O)Me |
| $CH_2C(O)OEt$ | $CH\{C(O)OMe\}S(O)_2Me$ |
| $CH_2C(O)OEt$ | CH{C(O)OMe}SEt |
| $CH_2C(O)OEt$ | CH{C(O)OMe}S(O)Et |
| $CH_2C(O)OEt$ | $CH\{C(O)OMe\}S(O)_2Et$ |
| $CH_2C(O)OEt$ | CH{C(O)OMe}S(=NCN)Et |
| $CH_2C(O)OEt$ | CH{C(O)OMe}S(O)(=NCN)Et |
| $CH_2C(O)OEt$ | CH{C(O)OEt}SMe |
| $CH_2C(O)OEt$ | CH{C(O)OEt}S(O)Me |
| $CH_2C(O)OEt$ | $CH\{C(O)OEt\}S(O)_2Me$ |
| $CH_2C(O)OEt$ | CH{C(O)OEt}SEt |
| $CH_2C(O)OEt$ | CH{C(O)OEt}S(O)Et |
| $CH_2C(O)OEt$ | $CH\{C(O)OEt\}S(O)_2Et$ |
| $CH_2C(O)OEt$ | CH{C(O)OEt}S(=NCN)Et |
| $CH_2C(O)OEt$ | CH{C(O)OEt}S(O)(=NCN)Et |
| $CH_2C(O)OEt$ | $C(Me)_2SMe$ |
| $CH_2C(O)OEt$ | $C(Me)_2S(O)Me$ |
| $CH_2C(O)OEt$ | $C(Me)_2S(O)_2Me$ |
| $CH_2C(O)OEt$ | $CH_2CH_2SMe$ |
| $CH_2C(O)OEt$ | $CH_2CH_2S(O)Me$ |
| $CH_2C(O)OEt$ | $CH_2CH_2S(O)_2Me$ |
| $CH_2C(O)OEt$ | $CH(Me)CH_2SMe$ |
| $CH_2C(O)OEt$ | $CH(Me)CH_2S(O)Me$ |
| $CH_2C(O)OEt$ | $CH(Me)CH_2S(O)_2Me$ |
| C(O)OMe | $CH_2SMe$ |
| C(O)OMe | $CH_2S(O)Me$ |

(continued)

| $R^a$ | $R^{b-1}$ |
|---|---|
| C(O)OMe | $CH_2S(O)_2Me$ |
| C(O)OMe | $CH_2SEt$ |
| C(O)OMe | $CH_2S(O)Et$ |
| C(O)OMe | $CH_2S(O)_2Et$ |
| C(O)OMe | $CH_2SPr$-n |
| C(O)OMe | $CH_2S(O)Pr$-n |
| C(O)OMe | $CH_2S(O)_2Pr$-n |
| C(O)OMe | $CH_2SPr$-i |
| C(O)OMe | $CH_2S(O)Pr$-i |
| C(O)OMe | $CH_2S(O)_2Pr$-i |
| C(O)OMe | $CH_2SPr$-c |
| C(O)OMe | $CH_2S(O)Pr$-c |
| C(O)OMe | $CH_2S(O)_2Pr$-c |
| C(O)OMe | $CH_2SCH_2CH=CH_2$ |
| C(O)OMe | $CH_2S(O)CH_2CH=CH_2$ |
| C(O)OMe | $CH_2S(O)_2CH_2CH=CH_2$ |
| C(O)OMe | $CH_2SCH_2C\equiv CH$ |
| C(O)OMe | $CH_2S(O)CH_2C\equiv CH$ |
| C(O)OMe | $CH_2S(O)_2CH_2C\equiv CH$ |
| C(O)OMe | $CH_2SCH_2CF_3$ |
| C(O)OMe | $CH_2S(O)CH_2CF_3$ |
| C(O)OMe | $CH_2S(O)_2CH_2CF_3$ |
| C(O)OMe | $CH_2SCH_2CH(OMe)_2$ |
| C(O)OMe | $CH_2SCH_2CH(=NOMe)$ |
| C(O)OMe | CH(Me)SMe |
| C(O)OMe | CH(Me)S(O)Me |
| C(O)OMe | $CH(Me)S(O)_2Me$ |
| C(O)OMe | CH(Me)SEt |
| C(O)OMe | CH(Me)S(O)Et |
| C(O)OMe | $CH(Me)S(O)_2Et$ |
| C(O)OMe | CH(Me)S(=NCN)Et |
| C(O)OMe | CH(Me)S(O)(=NCN)Et |
| C(O)OMe | CH(Me)SPr-n |
| C(O)OMe | CH(Me)S(O)Pr-n |
| C(O)OMe | $CH(Me)S(O)_2Pr$-n |
| C(O)OMe | CH(Me)SPr-i |
| C(O)OMe | CH(Me)S(O)Pr-i |
| C(O)OMe | $CH(Me)S(O)_2Pr$-i |
| C(O)OMe | CH(Me)SBu-t |
| C(O)OMe | CH(Me)S(O)Bu-t |
| C(O)OMe | $CH(Me)S(O)_2Bu$-t |
| C(O)OMe | $CH(Me)SCH_2Pr$-c |
| C(O)OMe | $CH(Me)S(O)CH_2Pr$-c |
| C(O)OMe | $CH(Me)S(O)_2CH_2Pr$-c |
| C(O)OMe | $CH(Me)SCH_2OMe$ |
| C(O)OMe | $CH(Me)SCH_2SMe$ |
| C(O)OMe | $CH(Me)S(O)CH_2SMe$ |
| C(O)OMe | $CH(Me)S(O)_2CH_2SMe$ |
| C(O)OMe | $CH(Me)SCH_2C=CH_2$ |

(continued)

| $R^a$ | $R^{b-1}$ |
|---|---|
| C(O)OMe | $CH(Me)S(O)CH_2C=CH_2$ |
| C(O)OMe | $CH(Me)S(O)_2CH_2C=CH_2$ |
| C(O)OMe | $CH(Me)SCH_2C\equiv CH$ |
| C(O)OMe | $CH(Me)S(O)CH_2C\equiv CH$ |
| C(O)OMe | $CH(Me)S(O)_2CH_2C\equiv CH$ |
| C(O)OMe | $CH(Me)SCH_2C(O)NHMe$ |
| C(O)OMe | $CH(Me)S(O)CH_2C(O)NHMe$ |
| C(O)OMe | $CH(Me)S(O)_2CH_2C(O)NHMe$ |
| C(O)OMe | $CH(Me)SCH_2C(O)OMe$ |
| C(O)OMe | $CH(Me)S(O)CH_2C(O)OMe$ |
| C(O)OMe | $CH(Me)S(O)_2CH_2C(O)OMe$ |
| C(O)OMe | $CH(Me)SCH_2CH_2CH_2Cl$ |
| C(O)OMe | $CH(Me)S(O)CH_2CH_2CH_2Cl$ |
| C(O)OMe | $CH(Me)S(O)_2CH_2CH_2CH_2Cl$ |
| C(O)OMe | $CH(Me)SCH_2CF_3$ |
| C(O)OMe | $CH(Me)S(O)CH_2CF_3$ |
| C(O)OMe | $CH(Me)S(O)_2CH_2CF_3$ |
| C(O)OMe | $CH(Me)SCH_2Ph$ |
| C(O)OMe | $CH(Me)S(O)CH_2Ph$ |
| C(O)OMe | $CH(Me)S(O)_2CH_2Ph$ |
| C(O)OMe | $CH(Me)SCH_2$(D1-34a) |
| C(O)OMe | $CH(Me)S(O)CH_2$(D1-34a) |
| C(O)OMe | $CH(Me)S(O)_2CH_2$(D1-34a) |
| C(O)OMe | $CH(CH_3)SCH_2Si(CH_3)_3$ |
| C(O)OMe | CH(Me)SCN |
| C(O)OMe | $CH(Me)SCH_2CN$ |
| C(O)OMe | CH(Me)SC(O)Me |
| C(O)OMe | CH(Me)S{D1-12a($X^{1a}$=Me)} |
| C(O)OMe | CH(Me)S(D1-32a) |
| C(O)OMe | CH(Me)S{D1-32b(3-$NO_2$)} |
| C(O)OMe | CH(Me)S{D1-32b(3-CF3)} |
| C(O)OMe | CH(Me)S(D1-37a) |
| C(O)OMe | CH(Me)S(D1-51a) |
| C(O)OMe | CH(Et)SMe |
| C(O)OMe | CH(Et)S(O)Me |
| C(O)OMe | $CH(Et)S(O)_2Me$ |
| C(O)OMe | CH(Et)SEt |
| C(O)OMe | CH(Et)S(O)Et |
| C(O)OMe | $CH(Et)S(O)_2Et$ |
| C(O)OMe | $CH(Et)SCH_2CF_3$ |
| C(O)OMe | $CH(Et)S(O)CH_2CF_3$ |
| C(O)OMe | $CH(Et)S(O)_2CH_2CF_3$ |
| C(O)OMe | CH(Pr-n)SMe |
| C(O)OMe | CH(Pr-n)S(O)Me |
| C(O)OMe | $CH(Pr-n)S(O)_2Me$ |
| C(O)OMe | CH(Pr-n)SEt |
| C(O)OMe | CH(Pr-n)S(O)Et |
| C(O)OMe | $CH(Pr-n)S(O)_2Et$ |
| C(O)OMe | CH(Pr-n)S(=NCN)Et |

(continued)

| $R^a$ | $R^{b-1}$ |
|---|---|
| C(O)OMe | CH(Pr-n)S(O)(=NCN)Et |
| C(O)OMe | CH(Pr-n)SPr-n |
| C(O)OMe | CH(Pr-n)S(O)Pr-n |
| C(O)OMe | CH(Pr-n)$S(O)_2$Pr-n |
| C(O)OMe | CH(Pr-n)SPr-i |
| C(O)OMe | CH(Pr-n)S(O)Pr-i |
| C(O)OMe | CH(Pr-n)$S(O)_2$Pr-i |
| C(O)OMe | CH(Pr-n)SPr-c |
| C(O)OMe | CH(Pr-n)S(O)Pr-c |
| C(O)OMe | CH(Pr-n)$S(O)_2$Pr-c |
| C(O)OMe | CH(Pr-n)$SCH_2CF_3$ |
| C(O)OMe | CH(Pr-n)$S(O)CH_2CF_3$ |
| C(O)OMe | CH(Pr-n)$S(O)_2CH_2CF_3$ |
| C(O)OMe | CH(Pr-i)SMe |
| C(O)OMe | CH(Pr-i)S(O)Me |
| C(O)OMe | CH(Pr-i)$S(O)_2$Me |
| C(O)OMe | CH(Pr-i)SEt |
| C(O)OMe | CH(Pr-i)S(O)Et |
| C(O)OMe | CH(Pr-i)$S(O)_2$Et |
| C(O)OMe | CH(Pr-i)S(=NCN)Et |
| C(O)OMe | CH(Pr-i)S(O)(=NCN)Et |
| C(O)OMe | CH(Pr-i)SPr-n |
| C(O)OMe | CH(Pr-i)S(O)Pr-n |
| C(O)OMe | CH(Pr-i)$S(O)_2$Pr-n |
| C(O)OMe | CH(Pr-i)SPr-i |
| C(O)OMe | CH(Pr-i)S(O)Pr-i |
| C(O)OMe | CH(Pr-i)$S(O)_2$Pr-i |
| C(O)OMe | CH(Pr-i)SPr-c |
| C(O)OMe | CH(Pr-i)S(O)Pr-c |
| C(O)OMe | CH(Pr-i)$S(O)_2$Pr-c |
| C(O)OMe | CH(Pr-i)$SCH_2CF_3$ |
| C(O)OMe | CH(Pr-i)$S(O)CH_2CF_3$ |
| C(O)OMe | CH(Pr-i)$S(O)_2CH_2CF_3$ |
| C(O)OMe | CH(Pr-c)SMe |
| C(O)OMe | CH(Pr-c)S(O)Me |
| C(O)OMe | CH(Pr-c)$S(O)_2$Me |
| C(O)OMe | CH(Pr-c)SEt |
| C(O)OMe | CH(Pr-c)S(O)Et |
| C(O)OMe | CH(Pr-c)$S(O)_2$Et |
| C(O)OMe | CH(Pr-c)S(=NCN)Et |
| C(O)OMe | CH(Pr-c)S(O)(=NCN)Et |
| C(O)OMe | CH(Pr-c)SPr-n |
| C(O)OMe | CH(Pr-c)S(O)Pr-n |
| C(O)OMe | CH(Pr-c)$S(O)_2$Pr-n |
| C(O)OMe | CH(Pr-c)SPr-i |
| C(O)OMe | CH(Pr-c)S(O)Pr-i |
| C(O)OMe | CH(Pr-c)$S(O)_2$Pr-i |
| C(O)OMe | CH(Pr-c)SPr-c |
| C(O)OMe | CH(Pr-c)S(O)Pr-c |

(continued)

| $R^a$ | $R^{b-1}$ |
|---|---|
| C(O)OMe | CH(Pr-c)S(O)$_2$Pr-c |
| C(O)OMe | CH(Pr-c)SCH$_2$CF$_3$ |
| C(O)OMe | CH(Pr-c)S(O)CH$_2$CF$_3$ |
| C(O)OMe | CH(Pr-c)S(O)$_2$CH$_2$CF$_3$ |
| C(O)OMe | CH(Bu-n)SMe |
| C(O)OMe | CH(Bu-n)S(O)Me |
| C(O)OMe | CH(Bu-n)S(O)$_2$Me |
| C(O)OMe | CH(Bu-n)SEt |
| C(O)OMe | CH(Bu-n)S(O)Et |
| C(O)OMe | CH(Bu-n)S(O)$_2$Et |
| C(O)OMe | CH(Bu-n)S(=NCN)Et |
| C(O)OMe | CH(Bu-n)S(O)(=NCN)Et |
| C(O)OMe | CH(Bu-n)SPr-n |
| C(O)OMe | CH(Bu-n)S(O)Pr-n |
| C(O)OMe | CH(Bu-n)S(O)$_2$Pr-n |
| C(O)OMe | CH(Bu-n)SPr-i |
| C(O)OMe | CH(Bu-n)S(O)Pr-i |
| C(O)OMe | CH(Bu-n)S(O)$_2$Pr-i |
| C(O)OMe | CH(Bu-n)SPr-c |
| C(O)OMe | CH(Bu-n)S(O)Pr-c |
| C(O)OMe | CH(Bu-n)S(O)$_2$Pr-c |
| C(O)OMe | CH(Bu-n)SCH$_2$CF$_3$ |
| C(O)OMe | CH(Bu-n)S(O)CH$_2$CF$_3$ |
| C(O)OMe | CH(Bu-n)S(O)$_2$CH$_2$CF$_3$ |
| C(O)OMe | CH(F)SMe |
| C(O)OMe | CH(F)S(O)Me |
| C(O)OMe | CH(F)S(O)$_2$Me |
| C(O)OMe | CH(F)SEt |
| C(O)OMe | CH(F)S(O)Et |
| C(O)OMe | CH(F)S(O)$_2$Et |
| C(O)OMe | CH(F)S(=NCN)Et |
| C(O)OMe | CH(F)S(O)(=NCN)Et |
| C(O)OMe | CH(F)SCH$_2$CF$_3$ |
| C(O)OMe | CH(F)S(O)CH$_2$CF$_3$ |
| C(O)OMe | CH(F)S(O)$_2$CH$_2$CF$_3$ |
| C(O)OMe | C(F)$_2$SMe |
| C(O)OMe | C(F)$_2$S(O)Me |
| C(O)OMe | C(F)$_2$S(O)$_2$Me |
| C(O)OMe | C(F)$_2$SEt |
| C(O)OMe | C(F)$_2$S(O)Et |
| C(O)OMe | C(F)$_2$S(O)$_2$Et |
| C(O)OMe | C(F)$_2$S(=NCN)Et |
| C(O)OMe | C(F)$_2$S(O)(=NCN)Et |
| C(O)OMe | C(F)$_2$SCH$_2$CF$_3$ |
| C(O)OMe | C(F)$_2$S(O)CH$_2$CF$_3$ |
| C(O)OMe | C(F)$_2$S(O)$_2$CH$_2$CF$_3$ |
| C(O)OMe | CH(Cl)SMe |
| C(O)OMe | CH(Cl)S(O)Me |
| C(O)OMe | CH(Cl)S(O)$_2$Me |

(continued)

| $R^a$ | $R^{b-1}$ |
|---|---|
| C(O)OMe | CH(Cl)SEt |
| C(O)OMe | CH(Cl)S(O)Et |
| C(O)OMe | $CH(Cl)S(O)_2Et$ |
| C(O)OMe | CH(Cl)S(=NCN)Et |
| C(O)OMe | CH(Cl)S(O)(=NCN)Et |
| C(O)OMe | $CH(Cl)SCH_2CF_3$ |
| C(O)OMe | $CH(Cl)S(O)CH_2CF_3$ |
| C(O)OMe | $CH(Cl)S(O)_2CH_2CF_3$ |
| C(O)OMe | $C(Cl)_2SMe$ |
| C(O)OMe | $C(Cl)_2S(O)Me$ |
| C(O)OMe | $C(Cl)_2S(O)_2Me$ |
| C(O)OMe | $C(Cl)_2SEt$ |
| C(O)OMe | $C(Cl)_2S(O)Et$ |
| C(O)OMe | $C(Cl)_2S(O)_2Et$ |
| C(O)OMe | $C(Cl)_2S(=NCN)Et$ |
| C(O)OMe | $C(Cl)_2S(O)(=NCN)Et$ |
| C(O)OMe | $C(Cl)_2SCH_2CF_3$ |
| C(O)OMe | $C(Cl)_2S(O)CH_2CF_3$ |
| C(O)OMe | $C(Cl)_2S(O)_2CH_2CF_3$ |
| C(O)OMe | CH(Br)SMe |
| C(O)OMe | CH(Br)S(O)Me |
| C(O)OMe | $CH(Br)S(O)_2Me$ |
| C(O)OMe | CH(Br)SEt |
| C(O)OMe | CH(Br)S(O)Et |
| C(O)OMe | $CH(Br)S(O)_2Et$ |
| C(O)OMe | CH(Br)S(=NCN)Et |
| C(O)OMe | CH(Br)S(O)(=NCN)Et |
| C(O)OMe | $CH(Br)SCH_2CF_3$ |
| C(O)OMe | $CH(Br)S(O)CH_2CF_3$ |
| C(O)OMe | $CH(Br)S(O)_2CH_2CF_3$ |
| C(O)OMe | CH(I)SMe |
| C(O)OMe | CH(I)S(O)Me |
| C(O)OMe | $CH(I)S(O)_2Me$ |
| C(O)OMe | CH(I)SEt |
| C(O)OMe | CH(I)S(O)Et |
| C(O)OMe | $CH(I)S(O)_2Et$ |
| C(O)OMe | CH(I)S(=NCN)Et |
| C(O)OMe | CH(I)S(O)(=NCN)Et |
| C(O)OMe | $CH(I)SCH_2CF_3$ |
| C(O)OMe | $CH(I)S(O)CH_2CF_3$ |
| C(O)OMe | $CH(I)S(O)_2CH_2CF_3$ |
| C(O)OMe | CH{OC(O)Me}SMe |
| C(O)OMe | CH{OC(O)Me}S(O)Me |
| C(O)OMe | $CH\{OC(O)Me\}S(O)_2Me$ |
| C(O)OMe | CH{OC(O)OMe}SEt |
| C(O)OMe | CH{OC(O)OMe}S(O)Et |
| C(O)OMe | $CH\{OC(O)OMe\}S(O)_2Et$ |
| C(O)OMe | CH{OC(O)OMe}S(=NCN)Et |
| C(O)OMe | CH{OC(O)OMe}S(O)(=NCN)Et |

(continued)

| $R^a$ | $R^{b-1}$ |
|---|---|
| C(O)OMe | CH{OC(O)OEt}SMe |
| C(O)OMe | CH{OC(O)OEt}S(O)Me |
| C(O)OMe | CH{OC(O)OEt}$S(O)_2$Me |
| C(O)OMe | CH{OC(O)OEt}SEt |
| C(O)OMe | CH{OC(O)OEt}S(O)Et |
| C(O)OMe | CH{OC(O)OEt}$S(O)_2$Et |
| C(O)OMe | CH{OC(O)OEt}S(=NCN)Et |
| C(O)OMe | CH{OC(O)OEt}S(O)(=NCN)Et |
| C(O)OMe | CH(OMe)SMe |
| C(O)OMe | CH(OMe)S(O)Me |
| C(O)OMe | CH(OMe)$S(O)_2$Me |
| C(O)OMe | CH(OMe)SEt |
| C(O)OMe | CH(OMe)S(O)Et |
| C(O)OMe | CH(OMe)$S(O)_2$Et |
| C(O)OMe | CH(OMe)S(=NCN)Et |
| C(O)OMe | CH(OMe)S(O)(=NCN)Et |
| C(O)OMe | CH(OEt)SMe |
| C(O)OMe | CH(OEt)S(O)Me |
| C(O)OMe | CH(OEt)$S(O)_2$Me |
| C(O)OMe | CH(OEt)SEt |
| C(O)OMe | CH(OEt)S(O)Et |
| C(O)OMe | CH(OEt)$S(O)_2$Et |
| C(O)OMe | CH(OEt)S(=NCN)Et |
| C(O)OMe | CH(OEt)S(O)(=NCN)Et |
| C(O)OMe | $CH(SMe)_2$ |
| C(O)OMe | CH(SMe)S(O)Me |
| C(O)OMe | CH(SMe)$S(O)_2$Me |
| C(O)OMe | CH(SMe)SEt |
| C(O)OMe | CH(SMe)S(O)Et |
| C(O)OMe | CH(SMe)$S(O)_2$Et |
| C(O)OMe | CH(SMe)S(=NCN)Et |
| C(O)OMe | CH(SMe)S(O)(=NCN)Et |
| C(O)OMe | CH(SEt)SMe |
| C(O)OMe | CH(SEt)S(O)Me |
| C(O)OMe | CH(SEt)$S(O)_2$Me |
| C(O)OMe | CH(SEt)SEt |
| C(O)OMe | CH(SEt)S(O)Et |
| C(O)OMe | CH(SEt)$S(O)_2$Et |
| C(O)OMe | CH(SEt)S(=NCN)Et |
| C(O)OMe | CH(SEt)S(O)(=NCN)Et |
| C(O)OMe | CH(CN)SMe |
| C(O)OMe | CH(CN)S(O)Me |
| C(O)OMe | CH(CN)$S(O)_2$Me |
| C(O)OMe | CH(CN)SEt |
| C(O)OMe | CH(CN)S(O)Et |
| C(O)OMe | CH(CN)$S(O)_2$Et |
| C(O)OMe | CH(CN)S(=NCN)Et |
| C(O)OMe | CH(CN)S(O)(=NCN)Et |
| C(O)OMe | CH{C(O)OMe}SMe |

(continued)

| $R^a$ | $R^{b-1}$ |
|---|---|
| C(O)OMe | CH{C(O)OMe}S(O)Me |
| C(O)OMe | $CH\{C(O)OMe\}S(O)_2Me$ |
| C(O)OMe | CH{C(O)OMe}SMe |
| C(O)OMe | CH{C(O)OMe}S(O)Me |
| C(O)OMe | $CH\{C(O)OMe\}S(O)_2Me$ |
| C(O)OMe | CH{C(O)OMe}SEt |
| C(O)OMe | CH{C(O)OMe}S(O)Et |
| C(O)OMe | $CH\{C(O)OMe\}S(O)_2Et$ |
| C(O)OMe | CH{C(O)OMe}S(=NCN)Et |
| C(O)OMe | CH{C(O)OMe}S(O)(=NCN)Et |
| C(O)OMe | CH{C(O)OEt}SMe |
| C(O)OMe | CH{C(O)OEt}S(O)Me |
| C(O)OMe | $CH\{C(O)OEt\}S(O)_2Me$ |
| C(O)OMe | CH{C(O)OEt}SEt |
| C(O)OMe | CH{C(O)OEt}S(O)Et |
| C(O)OMe | $CH\{C(O)OEt\}S(O)_2Et$ |
| C(O)OMe | CH{C(O)OEt}S(=NCN)Et |
| C(O)OMe | CH{C(O)OEt}S(O)(=NCN)Et |
| C(O)OMe | $C(Me)_2SMe$ |
| C(O)OMe | $C(Me)_2S(O)Me$ |
| C(O)OMe | $C(Me)_2S(O)_2Me$ |
| C(O)OMe | $CH_2CH_2SMe$ |
| C(O)OMe | $CH_2CH_2S(O)Me$ |
| C(O)OMe | $CH_2CH_2S(O)_2Me$ |
| C(O)OMe | $CH(Me)CH_2SMe$ |
| C(O)OMe | $CH(Me)CH_2S(O)Me$ |
| C(O)OMe | $CH(Me)CH_2S(O)_2Me$ |
| C(O)OEt | $CH_2SMe$ |
| C(O)OEt | $CH_2S(O)Me$ |
| C(O)OEt | $CH_2S(O)_2Me$ |
| C(O)OEt | $CH_2SEt$ |
| C(O)OEt | $CH_2S(O)Et$ |
| C(O)OEt | $CH_2S(O)_2Et$ |
| C(O)OEt | $CH_2SPr$-n |
| C(O)OEt | $CH_2S(O)Pr$-n |
| C(O)OEt | $CH_2S(O)_2Pr$-n |
| C(O)OEt | $CH_2SPr$-i |
| C(O)OEt | $CH_2S(O)Pr$-i |
| C(O)OEt | $CH_2S(O)_2Pr$-i |
| C(O)OEt | $CH_2SPr$-c |
| C(O)OEt | $CH_2S(O)Pr$-c |
| C(O)OEt | $CH_2S(O)_2Pr$-c |
| C(O)OEt | $CH_2SCH_2CH=CH_2$ |
| C(O)OEt | $CH_2S(O)CH_2CH=CH_2$ |
| C(O)OEt | $CH_2S(O)_2CH_2CH=CH_2$ |
| C(O)OEt | $CH_2SCH_2C\equiv CH$ |
| C(O)OEt | $CH_2S(O)CH_2C\equiv CH$ |
| C(O)OEt | $CH_2S(O)_2CH_2C\equiv CH$ |
| C(O)OEt | $CH_2SCH_2CF_3$ |

(continued)

| $R^a$ | $R^{b-1}$ |
|---|---|
| C(O)OEt | $CH_2S(O)CH_2CF_3$ |
| C(O)OEt | $CH_2S(O)_2CH_2CF_3$ |
| C(O)OEt | $CH_2SCH_2CH(OMe)_2$ |
| C(O)OEt | $CH_2SCH_2CH(=NOMe)$ |
| C(O)OEt | CH(Me)SMe |
| C(O)OEt | CH(Me)S(O)Me |
| C(O)OEt | $CH(Me)S(O)_2Me$ |
| C(O)OEt | CH(Me)SEt |
| C(O)OEt | CH(Me)S(O)Et |
| C(O)OEt | $CH(Me)S(O)_2Et$ |
| C(O)OEt | CH(Me)S(=NCN)Et |
| C(O)OEt | CH(Me)S(O)(=NCN)Et |
| C(O)OEt | CH(Me)SPr-n |
| C(O)OEt | CH(Me)S(O)Pr-n |
| C(O)OEt | $CH(Me)S(O)_2Pr$-n |
| C(O)OEt | CH(Me)SPr-i |
| C(O)OEt | CH(Me)S(O)Pr-i |
| C(O)OEt | $CH(Me)S(O)_2Pr$-i |
| C(O)OEt | CH(Me)SBu-t |
| C(O)OEt | CH(Me)S(O)Bu-t |
| C(O)OEt | $CH(Me)S(O)_2Bu$-t |
| C(O)OEt | $CH(Me)SCH_2Pr$-c |
| C(O)OEt | $CH(Me)S(O)CH_2Pr$-c |
| C(O)OEt | $CH(Me)S(O)_2CH_2Pr$-c |
| C(O)OEt | $CH(Me)SCH_2OMe$ |
| C(O)OEt | $CH(Me)SCH_2SMe$ |
| C(O)OEt | $CH(Me)S(O)CH_2SMe$ |
| C(O)OEt | $CH(Me)S(O)_2CH_2SMe$ |
| C(O)OEt | $CH(Me)SCH_2C=CH_2$ |
| C(O)OEt | $CH(Me)S(O)CH_2C=CH_2$ |
| C(O)OEt | $CH(Me)S(O)_2CH_2C=CH_2$ |
| C(O)OEt | $CH(Me)SCH_2C\equiv CH$ |
| C(O)OEt | $CH(Me)S(O)CH_2C\equiv CH$ |
| C(O)OEt | $CH(Me)S(O)_2CH_2C\equiv CH$ |
| C(O)OEt | $CH(Me)SCH_2C(O)NHMe$ |
| C(O)OEt | $CH(Me)S(O)CH_2C(O)NHMe$ |
| C(O)OEt | $CH(Me)S(O)_2CH_2C(O)NHMe$ |
| C(O)OEt | $CH(Me)SCH_2C(O)OMe$ |
| C(O)OEt | $CH(Me)S(O)CH_2C(O)OMe$ |
| C(O)OEt | $CH(Me)S(O)_2CH_2C(O)OMe$ |
| C(O)OEt | $CH(Me)SCH_2CH_2CH_2Cl$ |
| C(O)OEt | $CH(Me)S(O)CH_2CH_2CH_2Cl$ |
| C(O)OEt | $CH(Me)S(O)_2CH_2CH_2CH_2Cl$ |
| C(O)OEt | $CH(Me)SCH_2CF_3$ |
| C(O)OEt | $CH(Me)S(O)CH_2CF_3$ |
| C(O)OEt | $CH(Me)S(O)_2CH_2CF_3$ |
| C(O)OEt | $CH(Me)SCH_2Ph$ |
| C(O)OEt | $CH(Me)S(O)CH_2Ph$ |
| C(O)OEt | $CH(Me)S(O)_2CH_2Ph$ |

(continued)

| $R^a$ | $R^{b-1}$ |
|---|---|
| C(O)OEt | CH(Me)SCH$_2$(D1-34a) |
| C(O)OEt | CH(Me)S(O)CH$_2$(D1-34a) |
| C(O)OEt | CH(Me)S(O)$_2$CH$_2$(D1-34a) |
| C(O)OEt | CH(CH$_3$)SCH$_2$Si(CH$_3$)$_3$ |
| C(O)OEt | CH(Me)SCN |
| C(O)OEt | CH(Me)SCH$_2$CN |
| C(O)OEt | CH(Me)SC(O)Me |
| C(O)OEt | CH(Me)S{D1-12a(X$^{1a}$=Me)} |
| C(O)OEt | CH(Me)S(D1-32a) |
| C(O)OEt | CH(Me)S{D1-32b(3-NO$_2$)} |
| C(O)OEt | CH(Me)S{D1-32b(3-CF$_3$)} |
| C(O)OEt | CH(Me)S(D1-37a) |
| C(O)OEt | CH(Me)S(D1-51a) |
| C(O)OEt | CH(Et)SMe |
| C(O)OEt | CH(Et)S(O)Me |
| C(O)OEt | CH(Et)S(O)$_2$Me |
| C(O)OEt | CH(Et)SEt |
| C(O)OEt | CH(Et)S(O)Et |
| C(O)OEt | CH(Et)S(O)$_2$Et |
| C(O)OEt | CH(Et)SCH$_2$CF$_3$ |
| C(O)OEt | CH(Et)S(O)CH$_2$CF$_3$ |
| C(O)OEt | CH(Et)S(O)$_2$CH$_2$CF$_3$ |
| C(O)OEt | CH(Pr-n)SMe |
| C(O)OEt | CH(Pr-n)S(O)Me |
| C(O)OEt | CH(Pr-n)S(O)$_2$Me |
| C(O)OEt | CH(Pr-n)SEt |
| C(O)OEt | CH(Pr-n)S(O)Et |
| C(O)OEt | CH(Pr-n)S(O)$_2$Et |
| C(O)OEt | CH(Pr-n)S(=NCN)Et |
| C(O)OEt | CH(Pr-n)S(O)(=NCN)Et |
| C(O)OEt | CH(Pr-n)SPr-n |
| C(O)OEt | CH(Pr-n)S(O)Pr-n |
| C(O)OEt | CH(Pr-n)S(O)$_2$Pr-n |
| C(O)OEt | CH(Pr-n)SPr-i |
| C(O)OEt | CH(Pr-n)S(O)Pr-i |
| C(O)OEt | CH(Pr-n)S(O)$_2$Pr-i |
| C(O)OEt | CH(Pr-n)SPr-c |
| C(O)OEt | CH(Pr-n)S(O)Pr-c |
| C(O)OEt | CH(Pr-n)S(O)$_2$Pr-c |
| C(O)OEt | CH(Pr-n)SCH$_2$CF$_3$ |
| C(O)OEt | CH(Pr-n)S(O)CH$_2$CF$_3$ |
| C(O)OEt | CH(Pr-n)S(O)$_2$CH$_2$CF$_3$ |
| C(O)OEt | CH(Pr-i)SMe |
| C(O)OEt | CH(Pr-i)S(O)Me |
| C(O)OEt | CH(Pr-i)S(O)$_2$Me |
| C(O)OEt | CH(Pr-i)SEt |
| C(O)OEt | CH(Pr-i)S(O)Et |
| C(O)OEt | CH(Pr-i)S(O)$_2$Et |
| C(O)OEt | CH(Pr-i)S(=NCN)Et |

(continued)

| $R^a$ | $R^{b-1}$ |
|---|---|
| C(O)OEt | CH(Pr-i)S(O)(=NCN)Et |
| C(O)OEt | CH(Pr-i)SPr-n |
| C(O)OEt | CH(Pr-i)S(O)Pr-n |
| C(O)OEt | CH(Pr-i)$S(O)_2$Pr-n |
| C(O)OEt | CH(Pr-i)SPr-i |
| C(O)OEt | CH(Pr-i)S(O)Pr-i |
| C(O)OEt | CH(Pr-i)$S(O)_2$Pr-i |
| C(O)OEt | CH(Pr-i)SPr-c |
| C(O)OEt | CH(Pr-i)S(O)Pr-c |
| C(O)OEt | CH(Pr-i)$S(O)_2$Pr-c |
| C(O)OEt | CH(Pr-i)$SCH_2CF_3$ |
| C(O)OEt | CH(Pr-i)$S(O)CH_2CF_3$ |
| C(O)OEt | CH(Pr-i)$S(O)_2CH_2CF_3$ |
| C(O)OEt | CH(Pr-c)SMe |
| C(O)OEt | CH(Pr-c)S(O)Me |
| C(O)OEt | CH(Pr-c)$S(O)_2$Me |
| C(O)OEt | CH(Pr-c)SEt |
| C(O)OEt | CH(Pr-c)S(O)Et |
| C(O)OEt | CH(Pr-c)$S(O)_2$Et |
| C(O)OEt | CH(Pr-c)S(=NCN)Et |
| C(O)OEt | CH(Pr-c)S(O)(=NCN)Et |
| C(O)OEt | CH(Pr-c)SPr-n |
| C(O)OEt | CH(Pr-c)S(O)Pr-n |
| C(O)OEt | CH(Pr-c)$S(O)_2$Pr-n |
| C(O)OEt | CH(Pr-c)SPr-i |
| C(O)OEt | CH(Pr-c)S(O)Pr-i |
| C(O)OEt | CH(Pr-c)$S(O)_2$Pr-i |
| C(O)OEt | CH(Pr-c)SPr-c |
| C(O)OEt | CH(Pr-c)S(O)Pr-c |
| C(O)OEt | CH(Pr-c)$S(O)_2$Pr-c |
| C(O)OEt | CH(Pr-c)$SCH_2CF_3$ |
| C(O)OEt | CH(Pr-c)$S(O)CH_2CF_3$ |
| C(O)OEt | CH(Pr-c)$S(O)_2CH_2CF_3$ |
| C(O)OEt | CH(Bu-n)SMe |
| C(O)OEt | CH(Bu-n)S(O)Me |
| C(O)OEt | CH(Bu-n)$S(O)_2$Me |
| C(O)OEt | CH(Bu-n)SEt |
| C(O)OEt | CH(Bu-n)S(O)Et |
| C(O)OEt | CH(Bu-n)$S(O)_2$Et |
| C(O)OEt | CH(Bu-n)S(=NCN)Et |
| C(O)OEt | CH(Bu-n)S(O)(=NCN)Et |
| C(O)OEt | CH(Bu-n)SPr-n |
| C(O)OEt | CH(Bu-n)S(O)Pr-n |
| C(O)OEt | CH(Bu-n)$S(O)_2$Pr-n |
| C(O)OEt | CH(Bu-n)SPr-i |
| C(O)OEt | CH(Bu-n)S(O)Pr-i |
| C(O)OEt | CH(Bu-n)$S(O)_2$Pr-i |
| C(O)OEt | CH(Bu-n)SPr-c |
| C(O)OEt | CH(Bu-n)S(O)Pr-c |

(continued)

| $R^a$ | $R^{b-1}$ |
|---|---|
| C(O)OEt | CH(Bu-n)S(O)$_2$Pr-c |
| C(O)OEt | CH(Bu-n)SCH$_2$CF$_3$ |
| C(O)OEt | CH(Bu-n)S(O)CH$_2$CF$_3$ |
| C(O)OEt | CH(Bu-n)S(O)$_2$CH$_2$CF$_3$ |
| C(O)OEt | CH(F)SMe |
| C(O)OEt | CH(F)S(O)Me |
| C(O)OEt | CH(F)S(O)$_2$Me |
| C(O)OEt | CH(F)SEt |
| C(O)OEt | CH(F)S(O)Et |
| C(O)OEt | CH(F)S(O)$_2$Et |
| C(O)OEt | CH(F)S(=NCN)Et |
| C(O)OEt | CH(F)S(O)(=NCN)Et |
| C(O)OEt | CH(F)SCH$_2$CF$_3$ |
| C(O)OEt | CH(F)S(O)CH$_2$CF$_3$ |
| C(O)OEt | CH(F)S(O)$_2$CH$_2$CF$_3$ |
| C(O)OEt | C(F)$_2$SMe |
| C(O)OEt | C(F)$_2$S(O)Me |
| C(O)OEt | C(F)$_2$S(O)$_2$Me |
| C(O)OEt | C(F)$_2$SEt |
| C(O)OEt | C(F)$_2$S(O)Et |
| C(O)OEt | C(F)$_2$S(O)$_2$Et |
| C(O)OEt | C(F)$_2$S(=NCN)Et |
| C(O)OEt | C(F)$_2$S(O)(=NCN)Et |
| C(O)OEt | C(F)$_2$SCH$_2$CF$_3$ |
| C(O)OEt | C(F)$_2$S(O)CH$_2$CF$_3$ |
| C(O)OEt | C(F)$_2$S(O)$_2$CH$_2$CF$_3$ |
| C(O)OEt | CH(Cl)SMe |
| C(O)OEt | CH(Cl)S(O)Me |
| C(O)OEt | CH(Cl)S(O)$_2$Me |
| C(O)OEt | CH(Cl)SEt |
| C(O)OEt | CH(Cl)S(O)Et |
| C(O)OEt | CH(Cl)S(O)$_2$Et |
| C(O)OEt | CH(Cl)S(=NCN)Et |
| C(O)OEt | CH(Cl)S(O)(=NCN)Et |
| C(O)OEt | CH(Cl)SCH$_2$CF$_3$ |
| C(O)OEt | CH(Cl)S(O)CH$_2$CF$_3$ |
| C(O)OEt | CH(Cl)S(O)$_2$CH$_2$CF$_3$ |
| C(O)OEt | C(Cl)$_2$SMe |
| C(O)OEt | C(Cl)$_2$S(O)Me |
| C(O)OEt | C(Cl)$_2$S(O)$_2$Me |
| C(O)OEt | C(Cl)$_2$SEt |
| C(O)OEt | C(Cl)$_2$S(O)Et |
| C(O)OEt | C(Cl)$_2$S(O)$_2$Et |
| C(O)OEt | C(Cl)$_2$S(=NCN)Et |
| C(O)OEt | C(Cl)$_2$S(O)(=NCN)Et |
| C(O)OEt | C(Cl)$_2$SCH$_2$CF$_3$ |
| C(O)OEt | C(Cl)$_2$S(O)CH$_2$CF$_3$ |
| C(O)OEt | C(Cl)$_2$S(O)$_2$CH$_2$CF$_3$ |
| C(O)OEt | CH(Br)SMe |

(continued)

| $R^a$ | $R^{b-1}$ |
|---|---|
| C(O)OEt | CH(Br)S(O)Me |
| C(O)OEt | CH(Br)S(O)$_2$Me |
| C(O)OEt | CH(Br)SEt |
| C(O)OEt | CH(Br)S(O)Et |
| C(O)OEt | CH(Br)S(O)$_2$Et |
| C(O)OEt | CH(Br)S(=NCN)Et |
| C(O)OEt | CH(Br)S(O)(=NCN)Et |
| C(O)OEt | CH(Br)SCH$_2$CF$_3$ |
| C(O)OEt | CH(Br)S(O)CH$_2$CF$_3$ |
| C(O)OEt | CH(Br)S(O)$_2$CH$_2$CF$_3$ |
| C(O)OEt | CH(I)SMe |
| C(O)OEt | CH(I)S(O)Me |
| C(O)OEt | CH(I)S(O)$_2$Me |
| C(O)OEt | CH(I)SEt |
| C(O)OEt | CH(I)S(O)Et |
| C(O)OEt | CH(I)S(O)$_2$Et |
| C(O)OEt | CH(I)S(=NCN)Et |
| C(O)OEt | CH(I)S(O)(=NCN)Et |
| C(O)OEt | CH(I)SCH$_2$CF$_3$ |
| C(O)OEt | CH(I)S(O)CH$_2$CF$_3$ |
| C(O)OEt | CH(I)S(O)$_2$CH$_2$CF$_3$ |
| C(O)OEt | CH{OC(O)Me}SMe |
| C(O)OEt | CH{OC(O)Me}S(O)Me |
| C(O)OEt | CH{OC(O)Me}S(O)$_2$Me |
| C(O)OEt | CH{OC(O)OMe}SEt |
| C(O)OEt | CH{OC(O)OMe}S(O)Et |
| C(O)OEt | CH{OC(O)OMe}S(O)$_2$Et |
| C(O)OEt | CH{OC(O)OMe}S(=NCN)Et |
| C(O)OEt | CH{OC(O)OMe}S(O)(=NCN)Et |
| C(O)OEt | CH{OC(O)OEt}SMe |
| C(O)OEt | CH{OC(O)OEt}S(O)Me |
| C(O)OEt | CH{OC(O)OEt}S(O)$_2$Me |
| C(O)OEt | CH{OC(O)OEt}SEt |
| C(O)OEt | CH{OC(O)OEt}S(O)Et |
| C(O)OEt | CH{OC(O)OEt}S(O)$_2$Et |
| C(O)OEt | CH{OC(O)OEt}S(=NCN)Et |
| C(O)OEt | CH{OC(O)OEt}S(O)(=NCN)Et |
| C(O)OEt | CH(OMe)SMe |
| C(O)OEt | CH(OMe)S(O)Me |
| C(O)OEt | CH(OMe)S(O)$_2$Me |
| C(O)OEt | CH(OMe)SEt |
| C(O)OEt | CH(OMe)S(O)Et |
| C(O)OEt | CH(OMe)S(O)$_2$Et |
| C(O)OEt | CH(OMe)S(=NCN)Et |
| C(O)OEt | CH(OMe)S(O)(=NCN)Et |
| C(O)OEt | CH(OEt)SMe |
| C(O)OEt | CH(OEt)S(O)Me |
| C(O)OEt | CH(OEt)S(O)$_2$Me |
| C(O)OEt | CH(OEt)SEt |

(continued)

| $R^a$ | $R^{b-1}$ |
|---|---|
| C(O)OEt | CH(OEt)S(O)Et |
| C(O)OEt | CH(OEt)S(O)$_2$Et |
| C(O)OEt | CH(OEt)S(=NCN)Et |
| C(O)OEt | CH(OEt)S(O)(=NCN)Et |
| C(O)OEt | CH(SMe)$_2$ |
| C(O)OEt | CH(SMe)S(O)Me |
| C(O)OEt | CH(SMe)S(O)$_2$Me |
| C(O)OEt | CH(SMe)SEt |
| C(O)OEt | CH(SMe)S(O)Et |
| C(O)OEt | CH(SMe)S(O)$_2$Et |
| C(O)OEt | CH(SMe)S(=NCN)Et |
| C(O)OEt | CH(SMe)S(O)(=NCN)Et |
| C(O)OEt | CH(SEt)SMe |
| C(O)OEt | CH(SEt)S(O)Me |
| C(O)OEt | CH(SEt)S(O)$_2$Me |
| C(O)OEt | CH(SEt)SEt |
| C(O)OEt | CH(SEt)S(O)Et |
| C(O)OEt | CH(SEt)S(O)$_2$Et |
| C(O)OEt | CH(SEt)S(=NCN)Et |
| C(O)OEt | CH(SEt)S(O)(=NCN)Et |
| C(O)OEt | CH(CN)SMe |
| C(O)OEt | CH(CN)S(O)Me |
| C(O)OEt | CH(CN)S(O)$_2$Me |
| C(O)OEt | CH(CN)SEt |
| C(O)OEt | CH(CN)S(O)Et |
| C(O)OEt | CH(CN)S(O)$_2$Et |
| C(O)OEt | CH(CN)S(=NCN)Et |
| C(O)OEt | CH(CN)S(O)(=NCN)Et |
| C(O)OEt | CH{C(O)OMe}SMe |
| C(O)OEt | CH{C(O)OMe}S(O)Me |
| C(O)OEt | CH{C(O)OMe}S(O)$_2$Me |
| C(O)OEt | CH{C(O)OMe}SMe |
| C(O)OEt | CH{C(O)OMe}S(O)Me |
| C(O)OEt | CH{C(O)OMe}S(O)$_2$Me |
| C(O)OEt | CH{C(O)OMe}SEt |
| C(O)OEt | CH{C(O)OMe}S(O)Et |
| C(O)OEt | CH{C(O)OMe}S(O)$_2$Et |
| C(O)OEt | CH{C(O)OMe}S(=NCN)Et |
| C(O)OEt | CH{C(O)OMe}S(O)(=NCN)Et |
| C(O)OEt | CH{C(O)OEt}SMe |
| C(O)OEt | CH{C(O)OEt}S(O)Me |
| C(O)OEt | CH{C(O)OEt}S(O)$_2$Me |
| C(O)OEt | CH{C(O)OEt}SEt |
| C(O)OEt | CH{C(O)OEt}S(O)Et |
| C(O)OEt | CH{C(O)OEt}S(O)$_2$Et |
| C(O)OEt | CH{C(O)OEt}S(=NCN)Et |
| C(O)OEt | CH{C(O)OEt}S(O)(=NCN)Et |
| C(O)OEt | C(Me)$_2$SMe |
| C(O)OEt | C(Me)$_2$S(O)Me |

(continued)

| $R^a$ | $R^{b-1}$ |
|---|---|
| C(O)OEt | $C(Me)_2S(O)_2Me$ |
| C(O)OEt | $CH_2CH_2SMe$ |
| C(O)OEt | $CH_2CH_2S(O)Me$ |
| C(O)OEt | $CH_2CH_2S(O)_2Me$ |
| C(O)OEt | $CH(Me)CH_2SMe$ |
| C(O)OEt | $CH(Me)CH_2S(O)Me$ |
| C(O)OEt | $CH(Me)CH_2S(O)_2Me$ |
| $CH_2CF_3$ | $CH_2SMe$ |
| $CH_2CF_3$ | $CH_2S(O)Me$ |
| $CH_2CF_3$ | $CH_2S(O)_2Me$ |
| $CH_2CF_3$ | $CH_2SEt$ |
| $CH_2CF_3$ | $CH_2S(O)Et$ |
| $CH_2CF_3$ | $CH_2S(O)_2Et$ |
| $CH_2CF_3$ | $CH_2SPr$-n |
| $CH_2CF_3$ | $CH_2S(O)Pr$-n |
| $CH_2CF_3$ | $CH_2S(O)_2$ Pr-n |
| $CH_2CF_3$ | $CH_2SPr$-i |
| $CH_2CF_3$ | $CH_2S(O)Pr$-i |
| $CH_2CF_3$ | $CH_2S(O)_2Pr$-i |
| $CH_2CF_3$ | $CH_2SPr$-c |
| $CH_2CF_3$ | $CH_2S(O)Pr$-c |
| $CH_2CF_3$ | $CH_2S(O)_2Pr$-c |
| $CH_2CF_3$ | $CH_2SCH_2CH{=}CH_2$ |
| $CH_2CF_3$ | $CH_2S(O)CH_2CH{=}CH_2$ |
| $CH_2CF_3$ | $CH_2S(O)_2CH_2CH{=}CH_2$ |
| $CH_2CF_3$ | $CH_2SCH_2C{=}CH$ |
| $CH_2CF_3$ | $CH_2S(O)CH_2C{=}CH$ |
| $CH_2CF_3$ | $CH_2S(O)_2CH_2C{=}CH$ |
| $CH_2CF_3$ | $CH_2SCH_2CF_3$ |
| $CH_2CF_3$ | $CH_2S(O)CH_2CF_3$ |
| $CH_2CF_3$ | $CH_2S(O)_2CH_2CF_3$ |
| $CH_2CF_3$ | $CH_2SCH_2CH(OMe)_2$ |
| $CH_2CF_3$ | $CH_2SCH_2CH(=NOMe)$ |
| $CH_2CF_3$ | CH(Me)SMe |
| $CH_2CF_3$ | CH(Me)S(O)Me |
| $CH_2CF_3$ | $CH(Me)S(O)_2Me$ |
| $CH_2CF_3$ | CH(Me)SEt |
| $CH_2CF_3$ | CH(Me)S(O)Et |
| $CH_2CF_3$ | $CH(Me)S(O)_2Et$ |
| $CH_2CF_3$ | CH(Me)S(=NCN)Et |
| $CH_2CF_3$ | CH(Me)S(O)(=NCN)Et |
| $CH_2CF_3$ | CH(Me)SPr-n |
| $CH_2CF_3$ | CH(Me)S(O)Pr-n |
| $CH_2CF_3$ | $CH(Me)S(O)_2Pr$-n |
| $CH_2CF_3$ | CH(Me)SPr-i |
| $CH_2CF_3$ | CH(Me)S(O)Pr-i |
| $CH_2CF_3$ | $CH(Me)S(O)_2Pr$-i |
| $CH_2CF_3$ | CH(Me)SBu-t |
| $CH_2CF_3$ | CH(Me)S(O)Bu-t |

(continued)

| $R^a$ | $R^{b-1}$ |
|---|---|
| $CH_2CF_3$ | $CH(Me)S(O)_2Bu$-t |
| $CH_2CF_3$ | $CH(Me)SCH_2Pr$-c |
| $CH_2CF_3$ | $CH(Me)S(O)CH_2Pr$-c |
| $CH_2CF_3$ | $CH(Me)S(O)_2CH_2Pr$-c |
| $CH_2CF_3$ | $CH(Me)SCH_2OMe$ |
| $CH_2CF_3$ | $CH(Me)SCH_2SMe$ |
| $CH_2CF_3$ | $CH(Me)S(O)CH_2SMe$ |
| $CH_2CF_3$ | $CH(Me)S(O)_2CH_2SMe$ |
| $CH_2CF_3$ | $CH(Me)SCH_2C=CH_2$ |
| $CH_2CF_3$ | $CH(Me)S(O)CH_2C=CH_2$ |
| $CH_2CF_3$ | $CH(Me)S(O)_2CH_2C=CH_2$ |
| $CH_2CF_3$ | $CH(Me)SCH_2C\equiv CH$ |
| $CH_2CF_3$ | $CH(Me)S(O)CH_2C\equiv CH$ |
| $CH_2CF_3$ | $CH(Me)S(O)_2CH_2C\equiv CH$ |
| $CH_2CF_3$ | $CH(Me)SCH_2C(O)NHMe$ |
| $CH_2CF_3$ | $CH(Me)S(O)CH_2C(O)NHMe$ |
| $CH_2CF_3$ | $CH(Me)S(O)_2CH_2C(O)NHMe$ |
| $CH_2CF_3$ | $CH(Me)SCH_2C(O)OMe$ |
| $CH_2CF_3$ | $CH(Me)S(O)CH_2C(O)OMe$ |
| $CH_2CF_3$ | $CH(Me)S(O)_2CH_2C(O)OMe$ |
| $CH_2CF_3$ | $CH(Me)SCH_2CH_2CH_2Cl$ |
| $CH_2CF_3$ | $CH(Me)S(O)CH_2CH_2CH_2Cl$ |
| $CH_2CF_3$ | $CH(Me)S(O)_2CH_2CH_2CH_2Cl$ |
| $CH_2CF_3$ | $CH(Me)SCH_2CF_3$ |
| $CH_2CF_3$ | $CH(Me)S(O)CH_2CF_3$ |
| $CH_2CF_3$ | $CH(Me)S(O)_2CH_2CF_3$ |
| $CH_2CF_3$ | $CH(Me)SCH_2Ph$ |
| $CH_2CF_3$ | $CH(Me)S(O)CH_2Ph$ |
| $CH_2CF_3$ | $CH(Me)S(O)_2CH_2Ph$ |
| $CH_2CF_3$ | $CH(Me)SCH_2$(D1-34a) |
| $CH_2CF_3$ | $CH(Me)S(O)CH_2$(D1-34a) |
| $CH_2CF_3$ | $CH(Me)S(O)_2CH_2$(D1-34a) |
| $CH_2CF_3$ | $CH(CH_3)SCH_2Si(CH_3)_3$ |
| $CH_2CF_3$ | CH(Me)SCN |
| $CH_2CF_3$ | $CH(Me)SCH_2CN$ |
| $CH_2CF_3$ | CH(Me)SC(O)Me |
| $CH_2CF_3$ | CH(Me)S{D1-12a($X^{1a}$=Me)} |
| $CH_2CF_3$ | CH(Me)S(D1-32a) |
| $CH_2CF_3$ | CH(Me)S{D1-32b(3-$NO_2$)} |
| $CH_2CF_3$ | CH(Me)S{D1-32b(3-$CF_3$)} |
| $CH_2CF_3$ | CH(Me)S(D1-37a) |
| $CH_2CF_3$ | CH(Me)S(D1-51a) |
| $CH_2CF_3$ | CH(Et)SMe |
| $CH_2CF_3$ | CH(Et)S(O)Me |
| $CH_2CF_3$ | $CH(Et)S(O)_2Me$ |
| $CH_2CF_3$ | CH(Et)SEt |
| $CH_2CF_3$ | CH(Et)S(O)Et |
| $CH_2CF_3$ | $CH(Et)S(O)_2Et$ |
| $CH_2CF_3$ | $CH(Et)SCH_2CF_3$ |

(continued)

| $R^a$ | $R^{b-1}$ |
|---|---|
| $CH_2CF_3$ | $CH(Et)S(O)CH_2CF_3$ |
| $CH_2CF_3$ | $CH(Et)S(O)_2CH_2CF_3$ |
| $CH_2CF_3$ | CH(Pr-n)SMe |
| $CH_2CF_3$ | CH(Pr-n)S(O)Me |
| $CH_2CF_3$ | $CH(Pr-n)S(O)_2Me$ |
| $CH_2CF_3$ | CH(Pr-n)SEt |
| $CH_2CF_3$ | CH(Pr-n)S(O)Et |
| $CH_2CF_3$ | $CH(Pr-n)S(O)_2Et$ |
| $CH_2CF_3$ | CH(Pr-n)S(=NCN)Et |
| $CH_2CF_3$ | CH(Pr-n)S(O)(=NCN)Et |
| $CH_2CF_3$ | CH(Pr-n)SPr-n |
| $CH_2CF_3$ | CH(Pr-n)S(O)Pr-n |
| $CH_2CF_3$ | $CH(Pr-n)S(O)_2Pr-n$ |
| $CH_2CF_3$ | CH(Pr-n)SPr-i |
| $CH_2CF_3$ | CH(Pr-n)S(O)Pr-i |
| $CH_2CF_3$ | $CH(Pr-n)S(O)_2Pr-i$ |
| $CH_2CF_3$ | CH(Pr-n)SPr-c |
| $CH_2CF_3$ | CH(Pr-n)S(O)Pr-c |
| $CH_2CF_3$ | $CH(Pr-n)S(O)_2Pr-c$ |
| $CH_2CF_3$ | $CH(Pr-n)SCH_2CF_3$ |
| $CH_2CF_3$ | $CH(Pr-n)S(O)CH_2CF_3$ |
| $CH_2CF_3$ | $CH(Pr-n)S(O)_2CH_2CF_3$ |
| $CH_2CF_3$ | CH(Pr-i)SMe |
| $CH_2CF_3$ | CH(Pr-i)S(O)Me |
| $CH_2CF_3$ | $CH(Pr-i)S(O)_2Me$ |
| $CH_2CF_3$ | CH(Pr-i)SEt |
| $CH_2CF_3$ | CH(Pr-i)S(O)Et |
| $CH_2CF_3$ | $CH(Pr-i)S(O)_2Et$ |
| $CH_2CF_3$ | CH(Pr-i)S(=NCN)Et |
| $CH_2CF_3$ | CH(Pr-i)S(O)(=NCN)Et |
| $CH_2CF_3$ | CH(Pr-i)SPr-n |
| $CH_2CF_3$ | CH(Pr-i)S(O)Pr-n |
| $CH_2CF_3$ | $CH(Pr-i)S(O)_2Pr-n$ |
| $CH_2CF_3$ | CH(Pr-i)SPr-i |
| $CH_2CF_3$ | CH(Pr-i)S(O)Pr-i |
| $CH_2CF_3$ | $CH(Pr-i)S(O)_2Pr-i$ |
| $CH_2CF_3$ | CH(Pr-i)SPr-c |
| $CH_2CF_3$ | CH(Pr-i)S(O)Pr-c |
| $CH_2CF_3$ | $CH(Pr-i)S(O)_2Pr-c$ |
| $CH_2CF_3$ | $CH(Pr-i)SCH_2CF_3$ |
| $CH_2CF_3$ | $CH(Pr-i)S(O)CH_2CF_3$ |
| $CH_2CF_3$ | $CH(Pr-i)S(O)_2CH_2CF_3$ |
| $CH_2CF_3$ | CH(Pr-c)SMe |
| $CH_2CF_3$ | CH(Pr-c)S(O)Me |
| $CH_2CF_3$ | $CH(Pr-c)S(O)_2Me$ |
| $CH_2CF_3$ | CH(Pr-c)SEt |
| $CH_2CF_3$ | CH(Pr-c)S(O)Et |
| $CH_2CF_3$ | $CH(Pr-c)S(O)_2Et$ |
| $CH_2CF_3$ | CH(Pr-c)S(=NCN)Et |

(continued)

| $R^a$ | $R^{b-1}$ |
|---|---|
| $CH_2CF_3$ | CH(Pr-c)S(O)(=NCN)Et |
| $CH_2CF_3$ | CH(Pr-c)SPr-n |
| $CH_2CF_3$ | CH(Pr-c)S(O)Pr-n |
| $CH_2CF_3$ | CH(Pr-c)$S(O)_2$Pr-n |
| $CH_2CF_3$ | CH(Pr-c)SPr-i |
| $CH_2CF_3$ | CH(Pr-c)S(O)Pr-i |
| $CH_2CF_3$ | CH(Pr-c)$S(O)_2$Pr-i |
| $CH_2CF_3$ | CH(Pr-c)SPr-c |
| $CH_2CF_3$ | CH(Pr-c)S(O)Pr-c |
| $CH_2CF_3$ | CH(Pr-c)$S(O)_2$Pr-c |
| $CH_2CF_3$ | CH(Pr-c)$SCH_2CF_3$ |
| $CH_2CF_3$ | CH(Pr-c)S(O)$CH_2CF_3$ |
| $CH_2CF_3$ | CH(Pr-c)$S(O)_2CH_2CF_3$ |
| $CH_2CF_3$ | CH(Bu-n)SMe |
| $CH_2CF_3$ | CH(Bu-n)S(O)Me |
| $CH_2CF_3$ | CH(Bu-n)$S(O)_2$Me |
| $CH_2CF_3$ | CH(Bu-n)SEt |
| $CH_2CF_3$ | CH(Bu-n)S(O)Et |
| $CH_2CF_3$ | CH(Bu-n)$S(O)_2$ Et |
| $CH_2CF_3$ | CH(Bu-n)S(=NCN)Et |
| $CH_2CF_3$ | CH(Bu-n)S(O)(=NCN)Et |
| $CH_2CF_3$ | CH(Bu-n)SPr-n |
| $CH_2CF_3$ | CH(Bu-n)S(O)Pr-n |
| $CH_2CF_3$ | CH(Bu-n)$S(O)_2$ Pr-n |
| $CH_2CF_3$ | CH(Bu-n)SPr-i |
| $CH_2CF_3$ | CH(Bu-n)S(O)Pr-i |
| $CH_2CF_3$ | CH(Bu-n)$S(O)_2$Pr-i |
| $CH_2CF_3$ | CH(Bu-n)SPr-c |
| $CH_2CF_3$ | CH(Bu-n)S(O)Pr-c |
| $CH_2CF_3$ | CH(Bu-n)$S(O)_2$Pr-c |
| $CH_2CF_3$ | CH(Bu-n)$SCH_2CF_3$ |
| $CH_2CF_3$ | CH(Bu-n)S(O)$CH_2CF_3$ |
| $CH_2CF_3$ | CH(Bu-n)$S(O)_2$ $CH_2CF_3$ |
| $CH_2CF_3$ | CH(F)SMe |
| $CH_2CF_3$ | CH(F)S(O)Me |
| $CH_2CF_3$ | CH(F)$S(O)_2$Me |
| $CH_2CF_3$ | CH(F)SEt |
| $CH_2CF_3$ | CH(F)S(O)Et |
| $CH_2CF_3$ | CH(F)$S(O)_2$Et |
| $CH_2CF_3$ | CH(F)S(=NCN)Et |
| $CH_2CF_3$ | CH(F)S(O)(=NCN)Et |
| $CH_2CF_3$ | CH(F)$SCH_2CF_3$ |
| $CH_2CF_3$ | CH(F)S(O)$CH_2CF_3$ |
| $CH_2CF_3$ | CH(F)$S(O)_2CH_2CF_3$ |
| $CH_2CF_3$ | $C(F)_2$SMe |
| $CH_2CF_3$ | $C(F)_2$S(O)Me |
| $CH_2CF_3$ | $C(F)_2S(O)_2$ Me |
| $CH_2CF_3$ | $C(F)_2$SEt |
| $CH_2CF_3$ | $C(F)_2$S(O)Et |

(continued)

| $R^a$ | $R^{b-1}$ |
|---|---|
| $CH_2CF_3$ | $C(F)_2S(O)_2$ Et |
| $CH_2CF_3$ | $C(F)_2S(=NCN)Et$ |
| $CH_2CF_3$ | $C(F)_2S(O)(=NCN)Et$ |
| $CH_2CF_3$ | $C(F)_2SCH_2CF_3$ |
| $CH_2CF_3$ | $C(F)_2S(O)CH_2CF_3$ |
| $CH_2CF_3$ | $C(F)_2S(O)_2$ $CH_2CF_3$ |
| $CH_2CF_3$ | CH(Cl)SMe |
| $CH_2CF_3$ | CH(Cl)S(O)Me |
| $CH_2CF_3$ | $CH(Cl)S(O)_2Me$ |
| $CH_2CF_3$ | CH(Cl)SEt |
| $CH_2CF_3$ | CH(Cl)S(O)Et |
| $CH_2CF_3$ | $CH(Cl)S(O)_2Et$ |
| $CH_2CF_3$ | CH(Cl)S(=NCN)Et |
| $CH_2CF_3$ | CH(Cl)S(O)(=NCN)Et |
| $CH_2CF_3$ | $CH(Cl)SCH_2CF_3$ |
| $CH_2CF_3$ | $CH(Cl)S(O)CH_2$ $CF_3$ |
| $CH_2CF_3$ | $CH(Cl)S(O)_2CH_2CF_3$ |
| $CH_2CF_3$ | $C(Cl)_2SMe$ |
| $CH_2CF_3$ | $C(Cl)_2S(O)Me$ |
| $CH_2CF_3$ | $C(Cl)_2S(O)_2$ Me |
| $CH_2CF_3$ | $C(Cl)_2SEt$ |
| $CH_2CF_3$ | $C(Cl)_2S(O)Et$ |
| $CH_2CF_3$ | $C(Cl)_2S(O)_2Et$ |
| $CH_2CF_3$ | $C(Cl)_2S(=NCN)Et$ |
| $CH_2CF_3$ | $C(Cl)_2S(O)(=NCN)Et$ |
| $CH_2CF_3$ | $C(Cl)_2SCH_2CF_3$ |
| $CH_2CF_3$ | $C(Cl)_2S(O)CH_2CF_3$ |
| $CH_2CF_3$ | $C(Cl)_2S(O)_2CH_2CF_3$ |
| $CH_2CF_3$ | CH(Br)SMe |
| $CH_2CF_3$ | CH(Br)S(O)Me |
| $CH_2CF_3$ | $CH(Br)S(O)_2Me$ |
| $CH_2CF_3$ | CH(Br)SEt |
| $CH_2CF_3$ | CH(Br)S(O)Et |
| $CH_2CF_3$ | $CH(Br)S(O)_2Et$ |
| $CH_2CF_3$ | CH(Br)S(=NCN)Et |
| $CH_2CF_3$ | CH(Br)S(O)(=NCN)Et |
| $CH_2CF_3$ | $CH(Br)SCH_2CF_3$ |
| $CH_2CF_3$ | $CH(Br)S(O)CH_2CF_3$ |
| $CH_2CF_3$ | $CH(Br)S(O)_2CH_2CF_3$ |
| $CH_2CF_3$ | CH(I)SMe |
| $CH_2CF_3$ | CH(I)S(O)Me |
| $CH_2CF_3$ | $CH(I)S(O)_2Me$ |
| $CH_2CF_3$ | CH(I)SEt |
| $CH_2CF_3$ | CH(I)S(O)Et |
| $CH_2CF_3$ | $CH(I)S(O)_2Et$ |
| $CH_2CF_3$ | CH(I)S(=NCN)Et |
| $CH_2CF_3$ | CH(I)S(O)(=NCN)Et |
| $CH_2CF_3$ | $CH(I)SCH_2CF_3$ |
| $CH_2CF_3$ | $CH(I)S(O)CH_2CF_3$ |

(continued)

| $R^a$ | $R^{b-1}$ |
|---|---|
| $CH_2CF_3$ | $CH(I)S(O)_2CH_2CF_3$ |
| $CH_2CF_3$ | CH{OC(O)Me}SMe |
| $CH_2CF_3$ | CH{OC(O)Me}S(O)Me |
| $CH_2CF_3$ | CH{OC(O)Me}S(O)$_2$Me |
| $CH_2CF_3$ | CH{OC(O)OMe}SEt |
| $CH_2CF_3$ | CH{OC(O)OMe}S(O)Et |
| $CH_2CF_3$ | CH{OC(O)OMe}S(O)$_2$Et |
| $CH_2CF_3$ | CH{OC(O)OMe}S(=NCN)Et |
| $CH_2CF_3$ | CH{OC(O)OMe}S(O)(=NCN)Et |
| $CH_2CF_3$ | CH{OC(O)OEt}SMe |
| $CH_2CF_3$ | CH{OC(O)OEt}S(O)Me |
| $CH_2CF_3$ | CH{OC(O)OEt}S(O)$_2$Me |
| $CH_2CF_3$ | CH{OC(O)OEt}SEt |
| $CH_2CF_3$ | CH{OC(O)OEt}S(O)Et |
| $CH_2CF_3$ | CH{OC(O)OEt}S(O)$_2$Et |
| $CH_2CF_3$ | CH{OC(O)OEt}S(=NCN)Et |
| $CH_2CF_3$ | CH{OC(O)OEt}S(O)(=NCN)Et |
| $CH_2CF_3$ | CH(OMe)SMe |
| $CH_2CF_3$ | CH(OMe)S(O)Me |
| $CH_2CF_3$ | $CH(OMe)S(O)_2Me$ |
| $CH_2CF_3$ | CH(OMe)SEt |
| $CH_2CF_3$ | CH(OMe)S(O)Et |
| $CH_2CF_3$ | $CH(OMe)S(O)_2Et$ |
| $CH_2CF_3$ | CH(OMe)S(=NCN)Et |
| $CH_2CF_3$ | CH(OMe)S(O)(=NCN)Et |
| $CH_2CF_3$ | CH(OEt)SMe |
| $CH_2CF_3$ | CH(OEt)S(O)Me |
| $CH_2CF_3$ | $CH(OEt)S(O)_2Me$ |
| $CH_2CF_3$ | CH(OEt)SEt |
| $CH_2CF_3$ | CH(OEt)S(O)Et |
| $CH_2CF_3$ | $CH(OEt)S(O)_2Et$ |
| $CH_2CF_3$ | CH(OEt)S(=NCN)Et |
| $CH_2CF_3$ | CH(OEt)S(O)(=NCN)Et |
| $CH_2CF_3$ | $CH(SMe)_2$ |
| $CH_2CF_3$ | CH(SMe)S(O)Me |
| $CH_2CF_3$ | $CH(SMe)S(O)_2Me$ |
| $CH_2CF_3$ | CH(SMe)SEt |
| $CH_2CF_3$ | CH(SMe)S(O)Et |
| $CH_2CF_3$ | $CH(SMe)S(O)_2Et$ |
| $CH_2CF_3$ | CH(SMe)S(=NCN)Et |
| $CH_2CF_3$ | CH(SMe)S(O)(=NCN)Et |
| $CH_2CF_3$ | CH(SEt)SMe |
| $CH_2CF_3$ | CH(SEt)S(O)Me |
| $CH_2CF_3$ | $CH(SEt)S(O)_2Me$ |
| $CH_2CF_3$ | CH(SEt)SEt |
| $CH_2CF_3$ | CH(SEt)S(O)Et |
| $CH_2CF_3$ | $CH(SEt)S(O)_2Et$ |
| $CH_2CF_3$ | CH(SEt)S(=NCN)Et |
| $CH_2CF_3$ | CH(SEt)S(O)(=NCN)Et |

(continued)

| $R^a$ | $R^{b-1}$ |
|---|---|
| $CH_2CF_3$ | CH(CN)SMe |
| $CH_2CF_3$ | CH(CN)S(O)Me |
| $CH_2CF_3$ | $CH(CN)S(O)_2Me$ |
| $CH_2CF_3$ | CH(CN)SEt |
| $CH_2CF_3$ | CH(CN)S(O)Et |
| $CH_2CF_3$ | $CH(CN)S(O)_2Et$ |
| $CH_2CF_3$ | CH(CN)S(=NCN)Et |
| $CH_2CF_3$ | CH(CN)S(O)(=NCN)Et |
| $CH_2CF_3$ | CH{C(O)OMe}SMe |
| $CH_2CF_3$ | CH{C(O)OMe}S(O)Me |
| $CH_2CF_3$ | $CH\{C(O)OMe\}S(O)_2Me$ |
| $CH_2CF_3$ | CH{C(O)OMe}SMe |
| $CH_2CF_3$ | CH{C(O)OMe}S(O)Me |
| $CH_2CF_3$ | $CH\{C(O)OMe\}S(O)_2Me$ |
| $CH_2CF_3$ | CH{C(O)OMe}SEt |
| $CH_2CF_3$ | CH{C(O)OMe}S(O)Et |
| $CH_2CF_3$ | $CH\{C(O)OMe\}S(O)_2Et$ |
| $CH_2CF_3$ | CH{C(O)OMe}S(=NCN)Et |
| $CH_2CF_3$ | CH{C(O)OMe}S(O)(=NCN)Et |
| $CH_2CF_3$ | CH{C(O)OEt}SMe |
| $CH_2CF_3$ | CH{C(O)OEt}S(O)Me |
| $CH_2CF_3$ | $CH\{C(O)OEt\}S(O)_2Me$ |
| $CH_2CF_3$ | CH{C(O)OEt}SEt |
| $CH_2CF_3$ | CH{C(O)OEt}S(O)Et |
| $CH_2CF_3$ | $CH\{C(O)OEt\}S(O)_2Et$ |
| $CH_2CF_3$ | CH{C(O)OEt}S(=NCN)Et |
| $CH_2CF_3$ | CH{C(O)OEt}S(O)(=NCN)Et |
| $CH_2CF_3$ | $C(Me)_2SMe$ |
| $CH_2CF_3$ | $C(Me)_2S(O)Me$ |
| $CH_2CF_3$ | $C(Me)_2S(O)_2Me$ |
| $CH_2CF_3$ | OBu-t |
| $S(O)_2Me$ | $CH_2SMe$ |
| $S(O)_2Me$ | $CH_2S(O)Me$ |
| $S(O)_2Me$ | $CH_2S(O)_2Me$ |
| $S(O)_2Me$ | $CH_2SEt$ |
| $S(O)_2Me$ | $CH_2S(O)Et$ |
| $S(O)_2Me$ | $CH_2S(O)_2Et$ |
| $S(O)_2Me$ | $CH_2SPr$-n |
| $S(O)_2Me$ | $CH_2S(O)Pr$-n |
| $S(O)_2Me$ | $CH_2S(O)_2Pr$-n |
| $S(O)_2Me$ | $CH_2SPr$-i |
| $S(O)_2Me$ | $CH_2S(O)Pr$-i |
| $S(O)_2Me$ | $CH_2S(O)_2Pr$-i |
| $S(O)_2Me$ | $CH_2SPr$-c |
| $S(O)_2Me$ | $CH_2S(O)Pr$-c |
| $S(O)_2Me$ | $CH_2S(O)_2Pr$-c |
| $S(O)_2Me$ | $CH_2SCH_2CH=CH_2$ |
| $S(O)_2Me$ | $CH_2S(O)CH_2CH=CH_2$ |
| $S(O)_2Me$ | $CH_2S(O)_2CH_2CH=CH_2$ |

(continued)

| $R^a$ | $R^{b-1}$ |
|---|---|
| $S(O)_2Me$ | $CH_2SCH_2C{=}CH$ |
| $S(O)_2Me$ | $CH_2S(O)CH_2C{=}CH$ |
| $S(O)_2Me$ | $CH_2S(O)_2CH_2C{=}CH$ |
| $S(O)_2Me$ | $CH_2SCH_2CF_3$ |
| $S(O)_2Me$ | $CH_2S(O)CH_2CF_3$ |
| $S(O)_2Me$ | $CH_2S(O)_2CH_2CF_3$ |
| $S(O)_2Me$ | $CH_2SCH_2CH(OMe)_2$ |
| $S(O)_2Me$ | $CH_2SCH_2CH(=NOMe)$ |
| $S(O)_2Me$ | CH(Me)SMe |
| $S(O)_2Me$ | CH(Me)S(O)Me |
| $S(O)_2Me$ | $CH(Me)S(O)_2Me$ |
| $S(O)_2Me$ | CH(Me)SEt |
| $S(O)_2Me$ | CH(Me)S(O)Et |
| $S(O)_2Me$ | $CH(Me)S(O)_2Et$ |
| $S(O)_2Me$ | CH(Me)S(=NCN)Et |
| $S(O)_2Me$ | CH(Me)S(O)(=NCN)Et |
| $S(O)_2Me$ | CH(Me)SPr-n |
| $S(O)_2Me$ | CH(Me)S(O)Pr-n |
| $S(O)_2Me$ | $CH(Me)S(O)_2Pr$-n |
| $S(O)_2Me$ | CH(Me)SPr-i |
| $S(O)_2Me$ | CH(Me)S(O)Pr-i |
| $S(O)_2$ Me | $CH(Me)S(O)_2Pr$-i |
| $S(O)_2Me$ | CH(Me)SBu-t |
| $S(O)_2Me$ | CH(Me)S(O)Bu-t |
| $S(O)_2Me$ | $CH(Me)S(O)_2Bu$-t |
| $S(O)_2Me$ | $CH(Me)SCH_2Pr$-c |
| $S(O)_2Me$ | $CH(Me)S(O)CH_2Pr$-c |
| $S(O)_2Me$ | $CH(Me)S(O)_2CH_2Pr$-c |
| $S(O)_2Me$ | $CH(Me)SCH_2OMe$ |
| $S(O)_2Me$ | $CH(Me)SCH_2SMe$ |
| $S(O)_2Me$ | $CH(Me)S(O)CH_2SMe$ |
| $S(O)_2Me$ | $CH(Me)S(O)_2CH_2SMe$ |
| $S(O)_2Me$ | $CH(Me)SCH_2C{=}CH_2$ |
| $S(O)_2Me$ | $CH(Me)S(O)CH_2C{=}CH_2$ |
| $S(O)_2Me$ | $CH(Me)S(O)_2CH_2C{=}CH_2$ |
| $S(O)_2Me$ | $CH(Me)SCH_2C{\equiv}CH$ |
| $S(O)_2Me$ | $CH(Me)S(O)CH_2C{\equiv}CH$ |
| $S(O)_2Me$ | $CH(Me)S(O)_2CH_2C{\equiv}CH$ |
| $S(O)_2Me$ | $CH(Me)SCH_2C(O)NHMe$ |
| $S(O)_2Me$ | $CH(Me)S(O)CH_2C(O)NHMe$ |
| $S(O)_2Me$ | $CH(Me)S(O)_2CH_2C(O)NHMe$ |
| $S(O)_2Me$ | $CH(Me)SCH_2C(O)OMe$ |
| $S(O)_2Me$ | $CH(Me)S(O)CH_2C(O)OMe$ |
| $S(O)_2Me$ | $CH(Me)S(O)_2CH_2C(O)OMe$ |
| $S(O)_2Me$ | $CH(Me)SCH_2CH_2CH_2Cl$ |
| $S(O)_2Me$ | $CH(Me)S(O)CH_2CH_2CH_2Cl$ |
| $S(O)_2Me$ | $CH(Me)S(O)_2CH_2CH_2CH_2Cl$ |
| $S(O)_2Me$ | $CH(Me)SCH_2CF_3$ |
| $S(O)_2Me$ | $CH(Me)S(O)CH_2CF_3$ |

(continued)

| $R^a$ | $R^{b-1}$ |
|---|---|
| $S(O)_2Me$ | $CH(Me)S(O)_2CH_2CF_3$ |
| $S(O)_2Me$ | $CH(Me)SCH_2Ph$ |
| $S(O)_2Me$ | $CH(Me)S(O)CH_2Ph$ |
| $S(O)_2Me$ | $CH(Me)S(O)_2CH_2Ph$ |
| $S(O)_2Me$ | $CH(Me)SCH_2$(D1-34a) |
| $S(O)_2Me$ | $CH(Me)S(O)CH_2$(D1-34a) |
| $S(O)_2Me$ | $CH(Me)S(O)_2CH_2$(D1-34a) |
| $S(O)_2Me$ | $CH(CH_3)SCH_2Si(CH_3)_3$ |
| $S(O)_2Me$ | CH(Me)SCN |
| $S(O)_2Me$ | $CH(Me)SCH_2CN$ |
| $S(O)_2Me$ | CH(Me)SC(O)Me |
| $S(O)_2$ Me | CH(Me)S{D1-12a($X^{1a}$ =Me)} |
| $S(O)_2Me$ | CH(Me)S(D1-32a) |
| $S(O)_2Me$ | CH(Me)S{D1-32b(3-$NO_2$)} |
| $S(O)_2Me$ | CH(Me)S{D1-32b(3-$CF_3$)} |
| $S(O)_2Me$ | CH(Me)S(D1-37a) |
| $S(O)_2Me$ | CH(Me)S(D1-51a) |
| $S(O)_2Me$ | CH(Et)SMe |
| $S(O)_2Me$ | CH(Et)S(O)Me |
| $S(O)_2Me$ | $CH(Et)S(O)_2Me$ |
| $S(O)_2Me$ | CH(Et)SEt |
| $S(O)_2Me$ | CH(Et)S(O)Et |
| $S(O)_2Me$ | $CH(Et)S(O)_2Et$ |
| $S(O)_2Me$ | $CH(Et)SCH_2CF_3$ |
| $S(O)_2Me$ | $CH(Et)S(O)CH_2CF_3$ |
| $S(O)_2Me$ | $CH(Et)S(O)_2CH_2CF_3$ |
| $S(O)_2Me$ | CH(Pr-n)SMe |
| $S(O)_2Me$ | CH(Pr-n)S(O)Me |
| $S(O)_2Me$ | CH(Pr-n)$S(O)_2$Me |
| $S(O)_2Me$ | CH(Pr-n)SEt |
| $S(O)_2Me$ | CH(Pr-n)S(O)Et |
| $S(O)_2Me$ | CH(Pr-n)$S(O)_2$ Et |
| $S(O)_2Me$ | CH(Pr-n)S(=NCN)Et |
| $S(O)_2Me$ | CH(Pr-n)S(O)(=NCN)Et |
| $S(O)_2Me$ | CH(Pr-n)SPr-n |
| $S(O)_2Me$ | CH(Pr-n)S(O)Pr-n |
| $S(O)_2Me$ | CH(Pr-n)$S(O)_2$Pr-n |
| $S(O)_2Me$ | CH(Pr-n)SPr-i |
| $S(O)_2Me$ | CH(Pr-n)S(O)Pr-i |
| $S(O)_2Me$ | CH(Pr-n)$S(O)_2$Pr-i |
| $S(O)_2Me$ | CH(Pr-n)SPr-c |
| $S(O)_2Me$ | CH(Pr-n)S(O)Pr-c |
| $S(O)_2Me$ | CH(Pr-n)$S(O)_2$Pr-c |
| $S(O)_2Me$ | CH(Pr-n)$SCH_2CF_3$ |
| $S(O)_2Me$ | CH(Pr-n)$S(O)CH_2CF_3$ |
| $S(O)_2Me$ | CH(Pr-n)$S(O)_2CH_2CF_3$ |
| $S(O)_2Me$ | CH(Pr-i)SMe |
| $S(O)_2Me$ | CH(Pr-i)S(O)Me |
| $S(O)_2Me$ | CH(Pr-i)$S(O)_2$Me |

(continued)

| $R^a$ | $R^{b-1}$ |
|---|---|
| $S(O)_2Me$ | CH(Pr-i)SEt |
| $S(O)_2Me$ | CH(Pr-i)S(O)Et |
| $S(O)_2Me$ | $CH(Pr\text{-}i)S(O)_2Et$ |
| $S(O)_2Me$ | CH(Pr-i)S(=NCN)Et |
| $S(O)_2Me$ | CH(Pr-i)S(O)(=NCN)Et |
| $S(O)_2Me$ | CH(Pr-i)SPr-n |
| $S(O)_2Me$ | CH(Pr-i)S(O)Pr-n |
| $S(O)_2Me$ | $CH(Pr\text{-}i)S(O)_2Pr\text{-}n$ |
| $S(O)_2Me$ | CH(Pr-i)SPr-i |
| $S(O)_2Me$ | CH(Pr-i)S(O)Pr-i |
| $S(O)_2Me$ | $CH(Pr\text{-}i)S(O)_2Pr\text{-}i$ |
| $S(O)_2Me$ | CH(Pr-i)SPr-c |
| $S(O)_2Me$ | CH(Pr-i)S(O)Pr-c |
| $S(O)_2Me$ | $CH(Pr\text{-}i)S(O)_2Pr\text{-}c$ |
| $S(O)_2Me$ | $CH(Pr\text{-}i)SCH_2CF_3$ |
| $S(O)_2Me$ | $CH(Pr\text{-}i)S(O)CH_2CF_3$ |
| $S(O)_2Me$ | $CH(Pr\text{-}i)S(O)_2CH_2CF_3$ |
| $S(O)_2Me$ | CH(Pr-c)SMe |
| $S(O)_2Me$ | CH(Pr-c)S(O)Me |
| $S(O)_2Me$ | $CH(Pr\text{-}c)S(O)_2Me$ |
| $S(O)_2Me$ | CH(Pr-c)SEt |
| $S(O)_2Me$ | CH(Pr-c)S(O)Et |
| $S(O)_2Me$ | $CH(Pr\text{-}c)S(O)_2Et$ |
| $S(O)_2Me$ | CH(Pr-c)S(=NCN)Et |
| $S(O)_2Me$ | CH(Pr-c)S(O)(=NCN)Et |
| $S(O)_2Me$ | CH(Pr-c)SPr-n |
| $S(O)_2Me$ | CH(Pr-c)S(O)Pr-n |
| $S(O)_2Me$ | $CH(Pr\text{-}c)S(O)_2Pr\text{-}n$ |
| $S(O)_2Me$ | CH(Pr-c)SPr-i |
| $S(O)_2Me$ | CH(Pr-c)S(O)Pr-i |
| $S(O)_2Me$ | $CH(Pr\text{-}c)S(O)_2Pr\text{-}i$ |
| $S(O)_2Me$ | CH(Pr-c)SPr-c |
| $S(O)_2Me$ | CH(Pr-c)S(O)Pr-c |
| $S(O)_2Me$ | $CH(Pr\text{-}c)S(O)_2Pr\text{-}c$ |
| $S(O)_2Me$ | $CH(Pr\text{-}c)SCH_2CF_3$ |
| $S(O)_2Me$ | $CH(Pr\text{-}c)S(O)CH_2CF_3$ |
| $S(O)_2Me$ | $CH(Pr\text{-}c)S(O)_2CH_2CF_3$ |
| $S(O)_2Me$ | CH(Bu-n)SMe |
| $S(O)_2Me$ | CH(Bu-n)S(O)Me |
| $S(O)_2Me$ | $CH(Bu\text{-}n)S(O)_2Me$ |
| $S(O)_2Me$ | CH(Bu-n)SEt |
| $S(O)_2Me$ | CH(Bu-n)S(O)Et |
| $S(O)_2Me$ | $CH(Bu\text{-}n)S(O)_2Et$ |
| $S(O)_2Me$ | CH(Bu-n)S(=NCN)Et |
| $S(O)_2Me$ | CH(Bu-n)S(O)(=NCN)Et |
| $S(O)_2Me$ | CH(Bu-n)SPr-n |
| $S(O)_2Me$ | CH(Bu-n)S(O)Pr-n |
| $S(O)_2Me$ | $CH(Bu\text{-}n)S(O)_2Pr\text{-}n$ |
| $S(O)_2Me$ | CH(Bu-n)SPr-i |

(continued)

| $R^a$ | $R^{b-1}$ |
|---|---|
| $S(O)_2Me$ | CH(Bu-n)S(O)Pr-i |
| $S(O)_2Me$ | $CH(Bu\text{-}n)S(O)_2Pr\text{-}i$ |
| $S(O)_2Me$ | CH(Bu-n)SPr-c |
| $S(O)_2Me$ | CH(Bu-n)S(O)Pr-c |
| $S(O)_2Me$ | $CH(Bu\text{-}n)S(O)_2Pr\text{-}c$ |
| $S(O)_2Me$ | $CH(Bu\text{-}n)SCH_2CF_3$ |
| $S(O)_2Me$ | $CH(Bu\text{-}n)S(O)CH_2CF_3$ |
| $S(O)_2Me$ | $CH(Bu\text{-}n)S(O)_2CH_2CF_3$ |
| $S(O)_2Me$ | CH(F)SMe |
| $S(O)_2Me$ | CH(F)S(O)Me |
| $S(O)_2Me$ | $CH(F)S(O)_2Me$ |
| $S(O)_2Me$ | CH(F)SEt |
| $S(O)_2Me$ | CH(F)S(O)Et |
| $S(O)_2Me$ | $CH(F)S(O)_2Et$ |
| $S(O)_2Me$ | CH(F)S(=NCN)Et |
| $S(O)_2Me$ | CH(F)S(O)(=NCN)Et |
| $S(O)_2Me$ | $CH(F)SCH_2CF_3$ |
| $S(O)_2Me$ | $CH(F)S(O)CH_2CF_3$ |
| $S(O)_2Me$ | $CH(F)S(O)_2CH_2CF_3$ |
| $S(O)_2Me$ | $C(F)_2SMe$ |
| $S(O)_2Me$ | $C(F)_2S(O)Me$ |
| $S(O)_2Me$ | $C(F)_2S(O)_2Me$ |
| $S(O)_2Me$ | $C(F)_2SEt$ |
| $S(O)_2Me$ | $C(F)_2S(O)Et$ |
| $S(O)_2Me$ | $C(F)_2S(O)_2Et$ |
| $S(O)_2Me$ | $C(F)_2S(=NCN)Et$ |
| $S(O)_2Me$ | $C(F)_2S(O)(=NCN)Et$ |
| $S(O)_2Me$ | $C(F)_2SCH_2CF_3$ |
| $S(O)_2Me$ | $C(F)_2S(O)CH_2CF_3$ |
| $S(O)_2Me$ | $C(F)_2S(O)_2CH_2CF_3$ |
| $S(O)_2Me$ | CH(Cl)SMe |
| $S(O)_2Me$ | CH(Cl)S(O)Me |
| $S(O)_2Me$ | $CH(Cl)S(O)_2Me$ |
| $S(O)_2Me$ | CH(Cl)SEt |
| $S(O)_2Me$ | CH(Cl)S(O)Et |
| $S(O)_2Me$ | $CH(Cl)S(O)_2Et$ |
| $S(O)_2Me$ | CH(Cl)S(=NCN)Et |
| $S(O)_2Me$ | CH(Cl)S(O)(=NCN)Et |
| $S(O)_2Me$ | $CH(Cl)SCH_2CF_3$ |
| $S(O)_2Me$ | $CH(Cl)S(O)CH_2CF_3$ |
| $S(O)_2Me$ | $CH(Cl)S(O)_2CH_2CF_3$ |
| $S(O)_2Me$ | $C(Cl)_2SMe$ |
| $S(O)_2Me$ | $C(Cl)_2S(O)Me$ |
| $S(O)_2Me$ | $C(Cl)_2S(O)_2Me$ |
| $S(O)_2Me$ | $C(Cl)_2SEt$ |
| $S(O)_2Me$ | $C(Cl)_2S(O)Et$ |
| $S(O)_2Me$ | $C(Cl)_2S(O)_2Et$ |
| $S(O)_2Me$ | $C(Cl)_2S(=NCN)Et$ |
| $S(O)_2Me$ | $C(Cl)_2S(O)(=NCN)Et$ |

(continued)

| $R^a$ | $R^{b-1}$ |
|---|---|
| $S(O)_2Me$ | $C(Cl)_2SCH_2CF_3$ |
| $S(O)_2Me$ | $C(Cl)_2S(O)CH_2CF_3$ |
| $S(O)_2Me$ | $C(Cl)_2S(O)_2CH_2CF_3$ |
| $S(O)_2Me$ | CH(Br)SMe |
| $S(O)_2Me$ | CH(Br)S(O)Me |
| $S(O)_2Me$ | $CH(Br)S(O)_2Me$ |
| $S(O)_2Me$ | CH(Br)SEt |
| $S(O)_2Me$ | CH(Br)S(O)Et |
| $S(O)_2Me$ | $CH(Br)S(O)_2Et$ |
| $S(O)_2Me$ | CH(Br)S(=NCN)Et |
| $S(O)_2Me$ | CH(Br)S(O)(=NCN)Et |
| $S(O)_2Me$ | $CH(Br)SCH_2CF_3$ |
| $S(O)_2Me$ | $CH(Br)S(O)CH_2CF_3$ |
| $S(O)_2Me$ | $CH(Br)S(O)_2CH_2CF_3$ |
| $S(O)_2Me$ | CH(I)SMe |
| $S(O)_2Me$ | CH(I)S(O)Me |
| $S(O)_2Me$ | $CH(I)S(O)_2Me$ |
| $S(O)_2Me$ | CH(I)SEt |
| $S(O)_2Me$ | CH(I)S(O)Et |
| $S(O)_2Me$ | $CH(I)S(O)_2Et$ |
| $S(O)_2Me$ | CH(I)S(=NCN)Et |
| $S(O)_2Me$ | CH(I)S(O)(=NCN)Et |
| $S(O)_2Me$ | $CH(I)SCH_2CF_3$ |
| $S(O)_2Me$ | $CH(I)S(O)CH_2CF_3$ |
| $S(O)_2Me$ | $CH(I)S(O)_2CH_2CF_3$ |
| $S(O)_2Me$ | CH{OC(O)Me}SMe |
| $S(O)_2Me$ | CH{OC(O)Me}S(O)Me |
| $S(O)_2Me$ | $CH\{OC(O)Me\}S(O)_2Me$ |
| $S(O)_2Me$ | CH{OC(O)OMe}SEt |
| $S(O)_2Me$ | CH{OC(O)OMe}S(O)Et |
| $S(O)_2Me$ | $CH\{OC(O)OMe\}S(O)_2Et$ |
| $S(O)_2Me$ | CH{OC(O)OMe}S(=NCN)Et |
| $S(O)_2Me$ | CH{OC(O)OMe}S(O)(=NCN)Et |
| $S(O)_2Me$ | CH{OC(O)OEt}SMe |
| $S(O)_2Me$ | CH{OC(O)OEt}S(O)Me |
| $S(O)_2Me$ | $CH\{OC(O)OEt\}S(O)_2Me$ |
| $S(O)_2Me$ | CH{OC(O)OEt}SEt |
| $S(O)_2Me$ | CH{OC(O)OEt}S(O)Et |
| $S(O)_2Me$ | $CH\{OC(O)OEt\}S(O)_2Et$ |
| $S(O)_2Me$ | CH{OC(O)OEt}S(=NCN)Et |
| $S(O)_2Me$ | CH{OC(O)OEt}S(O)(=NCN)Et |
| $S(O)_2Me$ | CH(OMe)SMe |
| $S(O)_2Me$ | CH(OMe)S(O)Me |
| $S(O)_2Me$ | $CH(OMe)S(O)_2Me$ |
| $S(O)_2Me$ | CH(OMe)SEt |
| $S(O)_2Me$ | CH(OMe)S(O)Et |
| $S(O)_2Me$ | $CH(OMe)S(O)_2Et$ |
| $S(O)_2Me$ | CH(OMe)S(=NCN)Et |
| $S(O)_2Me$ | CH(OMe)S(O)(=NCN)Et |

(continued)

| $R^a$ | $R^{b-1}$ |
|---|---|
| $S(O)_2Me$ | CH(OEt)SMe |
| $S(O)_2Me$ | CH(OEt)S(O)Me |
| $S(O)_2Me$ | $CH(OEt)S(O)_2Me$ |
| $S(O)_2Me$ | CH(OEt)SEt |
| $S(O)_2Me$ | CH(OEt)S(O)Et |
| $S(O)_2Me$ | $CH(OEt)S(O)_2Et$ |
| $S(O)_2Me$ | CH(OEt)S(=NCN)Et |
| $S(O)_2Me$ | CH(OEt)S(O)(=NCN)Et |
| $S(O)_2Me$ | $CH(SMe)_2$ |
| $S(O)_2Me$ | CH(SMe)S(O)Me |
| $S(O)_2Me$ | $CH(SMe)S(O)_2Me$ |
| $S(O)_2Me$ | CH(SMe)SEt |
| $S(O)_2Me$ | CH(SMe)S(O)Et |
| $S(O)_2Me$ | $CH(SMe)S(O)_2Et$ |
| $S(O)_2Me$ | CH(SMe)S(=NCN)Et |
| $S(O)_2Me$ | CH(SMe)S(O)(=NCN)Et |
| $S(O)_2Me$ | CH(SEt)SMe |
| $S(O)_2Me$ | CH(SEt)S(O)Me |
| $S(O)_2Me$ | $CH(SEt)S(O)_2Me$ |
| $S(O)_2Me$ | CH(SEt)SEt |
| $S(O)_2Me$ | CH(SEt)S(O)Et |
| $S(O)_2Me$ | $CH(SEt)S(O)_2Et$ |
| $S(O)_2Me$ | CH(SEt)S(=NCN)Et |
| $S(O)_2Me$ | CH(SEt)S(O)(=NCN)Et |
| $S(O)_2Me$ | CH(CN)SMe |
| $S(O)_2Me$ | CH(CN)S(O)Me |
| $S(O)_2Me$ | $CH(CN)S(O)_2Me$ |
| $S(O)_2Me$ | CH(CN)SEt |
| $S(O)_2Me$ | CH(CN)S(O)Et |
| $S(O)_2Me$ | $CH(CN)S(O)_2Et$ |
| $S(O)_2Me$ | CH(CN)S(=NCN)Et |
| $S(O)_2Me$ | CH(CN)S(O)(=NCN)Et |
| $S(O)_2Me$ | CH{C(O)OMe}SMe |
| $S(O)_2Me$ | CH{C(O)OMe}S(O)Me |
| $S(O)_2Me$ | $CH\{C(O)OMe\}S(O)_2Me$ |
| $S(O)_2Me$ | CH{C(O)OMe}SMe |
| $S(O)_2Me$ | CH{C(O)OMe}S(O |
| $S(O)_2Me$ | $CH\{C(O)OMe\}S(O)_2Me$ |
| $S(O)_2Me$ | CH{C(O)OMe}SEt |
| $S(O)_2Me$ | CH{C(O)OMe}S(O)Et |
| $S(O)_2Me$ | $CH\{C(O)OMe\}S(O)_2Et$ |
| $S(O)_2Me$ | CH{C(O)OMe}S(=NCN)Et |
| $S(O)_2Me$ | CH{C(O)OMe}S(O)(=NCN)Et |
| $S(O)_2Me$ | CH{C(O)OEt}SMe |
| $S(O)_2Me$ | CH{C(O)OEt}S(O)Me |
| $S(O)_2Me$ | $CH\{C(O)OEt\}S(O)_2Me$ |
| $S(O)_2Me$ | CH{C(O)OEt}SEt |
| $S(O)_2Me$ | CH{C(O)OEt}S(O)Et |
| $S(O)_2Me$ | $CH\{C(O)OEt\}S(O)_2Et$ |

(continued)

| $R^a$ | $R^{b-1}$ |
|---|---|
| $S(O)_2Me$ | $CH\{C(O)OEt\}S(=NCN)Et$ |
| $S(O)_2Me$ | $CH\{C(O)OEt\}S(O)(=NCN)Et$ |
| $S(O)_2Me$ | $C(Me)_2SMe$ |
| $S(O)_2Me$ | $C(Me)_2S(O)Me$ |
| $S(O)_2Me$ | $C(Me)_2S(O)_2Me$ |
| $SC(Cl)_3$ | $CH(Me)SMe$ |
| $SC(Cl)_3$ | $CH(Me)S(O)Me$ |
| $SC(Cl)_3$ | $CH(Me)S(O)_2Me$ |
| $SC(Cl)_3$ | $CH(Me)SEt$ |
| $SC(Cl)_3$ | $CH(Me)S(O)Et$ |
| $SC(Cl)_3$ | $CH(Me)S(O)_2Et$ |
| $SC(Cl)_3$ | $CH(Me)CH_2SMe$ |
| $CH_2CH_2CH_2SMe$ | Me |
| $CH_2CH_2SMe$ | Me |
| $NH_2$ | OBu-t |
| $NHC(O)CH_2SMe$ | OBu-t |
| $N(Me)C(O)CH_2SMe$ | OBu-t |
| $NHC(O)CH(Me)SMe$ | OBu-t |
| $N(Me)C(O)CH(Me)SMe$ | OBu-t |
| $NHC(O)CH_2CH_2SMe$ | OBu-t |
| $N\{C(O)OBu\text{-}t\}_2$ | OBu-t |
| $NHC(O)OBu\text{-}t$ | OBu-t |
| | $-CH_2CH_2CH_2-$ |
| | $-CH_2CH_2C(=CH_2)-$ |
| | $-CH_2CH_2CH(CH_2SMe)-$ |
| | $-CH_2CH_2CH\{CH_2S(O)Me\}-$ |
| | $-CH_2CH_2CH\{CH_2S(O)_2Me\}-$ |
| $-CH_2CH_2CH_2CH_2-$ | |
| $-CH_2CH_2CH=CH-$ | |
| $-CH_2CH=CHCH_2-$ | |
| $-CH_2CH_2CH_2CH(Br)-$ | |
| $-CH_2CH_2CH_2CH(SCH_3)-$ | |
| $-CH_2CH_2CH_2CH\{S(O)CH_3)-$ | |
| $-CH_2CH_2CH_2CH\{S(O)_2CH_3\}-$ | |
| $-CH_2CH_2CH_2C(F)\{S(O)_2CH_3\}-$ | |
| $-CH_2CH_2CH(SCH_3)CH_2-$ | |
| $-CH_2CH_2CH_2CH(CH_2SCH_3)-$ | |
| $-CH_2CH_2CH(CH_2SCH_3)CH_2-$ | |
| $-CH_2CH_2CH_2CH(OCH_2CF_3)-$ | |
| $-CH_2CH_2CH(OCH_2CF_3)CH_2-$ | |
| $-CH_2CH_2SCH_2-$ | |
| $-CH_2OCH(CF_3)CH_2-$ | |
| $-CH_2OCH_2N(CH_2CF_3)-$ | |
| $-CH_2OCH_2N(CH_2SMe)-$ | |
| $-CH_2CH_2N(CH_2CF_3)-$ | |
| $-CH_2CH_2N(CH_2SMe)-$ | |
| $-C(O)CH_2CH_2-$ | |
| $-C(O)CH=C(Me)-$ | |
| $-C(O)CH_2C(=CH_2)-$ | |

(continued)

| $R^a$ | $R^{b-1}$ |
|---|---|
| | $-C(O)NHCH(CH_2SMe)-$ |
| | $-C(O)NHCH\{CH_2S(O)Me\}-$ |
| | $-C(O)NHCH\{CH_2S(O)_2Me\}-$ |
| | $-CH_2C(OCH_3)_2-$ |

[Table 3]

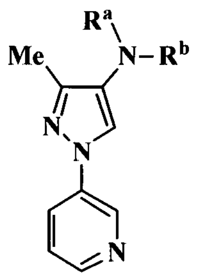
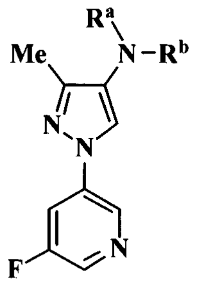
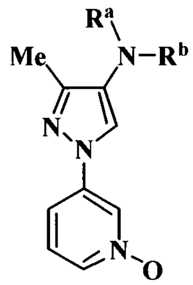
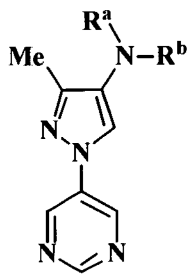
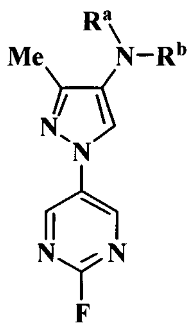
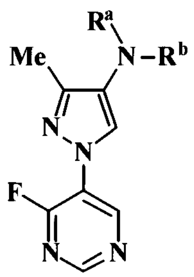
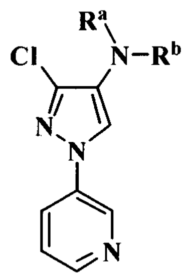
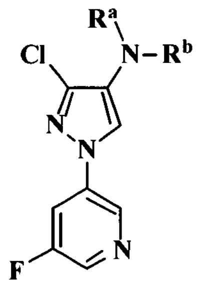
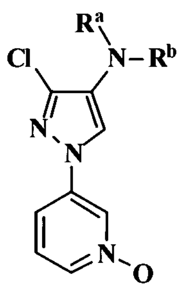
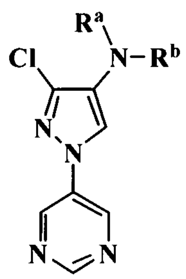
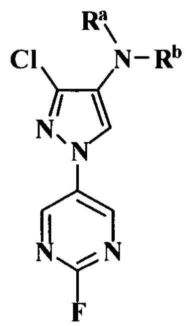
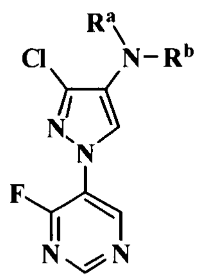
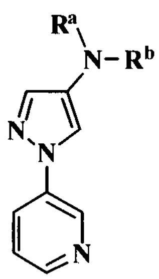

TABLE 3

| $R^a$ | $R^b$ |
|---|---|
| H | D1-51a |
| Me | D1-51a |
| Et | D1-51a |
| =C(Me)OMe | |
| =C(Me)OEt | |
| $=CHNMe_2$ | |
| $=C(Me)N(Me)C(O)CH_2SMe$ | |
| =C(SMe)CH(Me)SEt | |
| $-C(=NOMe)CH_2CH_2CH_2-$ | |
| D1-88b{$X^{1a}=C(O)CH_2SCH_3$} | |

The compounds of the present invention can effectively control insects including so-called agricultural pest insects damaging agricultural crops and trees, so-called livestock pest insects which parasitize livestock and poultry, so-called hygienic insects having harmful effects on houses and living environments and so-called grain-storage insects damaging grains stored in warehouses and any pests which live and give damage in similar settings such as mites, crustaceans, mollusks and nematodes at low doses. The insects, mites, crustaceans, mollusks and nematodes that the compounds of the present invention can control include the following organisms, but the present invention is not restricted thereto.

Insects of the order Lepidoptera such as Adoxophyes honmai, Adoxophyes orana faciata, Archips breviplicanus, Archips fuscocupreanus, Grapholita molesta, Homona magnanima, Leguminivora glycinivorella, Matsumuraeses phaseoli, Pandemis heparana, Bucculatrix pyrivorella, Lyonetia clerkella, Lyonetia prunifoliella malinella, Caloptilia theivora, Phyllonorycter ringoniella, Phyllocnistis citrella, Acrolepiopsis sapporensis, Acrolepiopsis suzukiella, Plutella xylostella, Stathmopoda masinissa, Helcystogramma triannulella, Pectinophora gossypiella, Carposina sasakii, Cydla pomonella, Chilo suppressalis, Cnaphalocrocis medinalis, Conogethes punctiferalis, Diaphania indica, Etiella zinckenella, Glyphodes pyloalis, Hellula undalis, Ostrinia furnacalis, Ostrinia scapulalis, Ostrinia nubilalis, Parapediasia teterrella, Parnara guttata, Pieris brassicae, Pieris rapae crucivora, Ascotis selenaria, Pseudoplusia includens, Euproctis pseudoconspersa, Lymantria dispar, Orgvia thyellina, Hyphantria cunea, Lemyra imparilis, Adris tyrannus, Aedia leucomelas, Agrotis ipsilon, Agrotis segetum, Autographa nigrisigna, Ctenoplusia agnata, Helicoverpa armigera, Helicoverpa assulta, Helicoverpa zea, Heliothis virescens, Mamestra brassicae, Mythimna separata, Naranga aenescens, Spodoptera eridania, Spodoptera exigua, Spodoptera frugiperda, Spodoptera littoralis, Spodoptera litura, Spodoptera depravata, Trichoplusia ni, Endopiza viteana, Manduca quinquemaculata and Manduca sexta.

Insects of the order Thysanoptera such as Frankliniella intonsa, Frankliniella occidentalis, Heliothrips haemorrhoidalis, Scirtothrips dorsalis, Thrips palmi, Thrips tabaci and Ponticulothrips diospyrosi..

Insects of the order Hemiptera such as Dolycoris baccarum, Eurydema rugosum, Eysarcoris aeneus, Eysarcoris lewisi, Eysarcoris ventralis, Glaucias subpunctatus, Halyomorpha halys, Nezara antennata, Nezara viridula, Piezodorus hybneri, Plautia crossota, Scotinophora lurida, Cletus punctiger, Leptocorisa chinensis, Riptortus clavatus, Rhopalus msculatus, Cavelerius saccharivorus, Togo hemipterus, Dysdercus cingulatus, Stephanitis pyrioides, Halticus insularis, Lygus lineolaris, Stenodema sibiricum, Stenotus rubrovittatus, Trigonotylus caelestialium, Arboridia apicalis, Balclutha saltuella, Epiacanthus stramineus, Empoasca fabae, Empoasca nipponica, Empoasca onukii, Empoasca sakaii, Macrosteles striifrons, Nephotettix cinctinceps, Psuedatomoscelis seriatus, Laodelphax striatella, Nilaparvata lugens, Sogatella furcifera, Diaphorina citri, Psylla pyrisuga, Aleurocanthus spiniferus, Bemisia argentifolii, Bemisia tabaci, Dialeurodes citri, Trialeurodes vaporariorum, Viteus vitifolii, Aphis gossypii, Aphis spiraecola, Myzus persicae, Toxoptera aurantii, Drosicha corpulenta, Icerya purchasi, Phenacoccus solani, Planococcus citri, Planococcus kuraunhiae, Pseudococcus comstocki, Ceroplastes ceriferus, Ceroplastes rubens, Aonidiella aurantii, Comstockaspis perniciosa, Fiorinia theae, Pseudaonidia paeoniae, Pseudaulacaspis pentagona, Pseudaulacaspis prunicola, Unaspis euonymi, Unaspis yanonensis and Cimex lectularius.

Insects of the order Coleoptera such as Anomala cuprea, Anomala rufocuprea, Gametis jucunda, Heptophylla picea, Popillia japonica, Lepinotarsa decemlineata, Melanotus fortnumi, Melanotus tamsuyensis, Lasioderma serricorne, Epuraea domina, Epilachna varivestis, Epilachna vigintioctopunctata, Tenebrio molitor, Tribolium castaneum, Anoplophora malasiaca, Monochamus alternatus, Psacothea hilaris, Xylotrechus pyrrhoderus, Callosobruchus chinensis, Aulacophora femoralis, Chaetocnema concinna, Diabrotica undecimpunctata, Diabrotica virgifera, Diabrotica barberi, Oulema oryzae, Phyllotreta striolata, Psylliodes angusticollis, Rhynchites heros, Cylas formicarius, Anthonomus grandis, Echinocnemus squameus, Euscepes postfasciatus, Hypera postica, Lissohoptrus oryzophilus, Otiorhynchus sulcatus,

Sitophilus granarius, Sitophilus zeamais, Sphenophorus venatus vestitus and Paederus fuscipes.

Insects of the order Diptera such as Asphondylia yushimai, Sitodiplosis mosellana, Bactrocera cucurbitae, Bactrocera dorsalis, Ceratitis capitata, Hydrellia oriseota. Drosophila suzukii, Agromyza oryzae, Chromatomyia horticola, Liriomyza bryoniae, Liriomyza chinensis, Liriomyza sativae, Liriomyza trifolii, Delia platura, Pegomya cunicularia, Rhagoletis pomonella, Mayetiola destructor, Musca domestica, Stomoxys calcitrans, Melophagus ovinus, Hypoderma bovis, Hypoderma lineatum, Oestrus ovis, Glossina palpalis, Glossina morsitans, Prosimulium yezoensis, Tabanus trigonus, Telmatoscopus albipunctatus, Leptoconops nipponensis, Culex pipiens pallens, Aedes aegypti, Aedes albopicutus and Anopheles hyracanus sinesis.

Insects of the order Hymenoptera such as Apethymus kuri, Athalia rosae, Arge pagana, Neodiprion sertifer, Dryocosmus kuriphilus, Eciton burchelli, Eciton schmitti, Camponotus japonicus, Vespa mandarina, Myrmecia spp., Solenopsis spp. and Monomorium pharaonis.

Insects of the order Orthoptera such as Teleogryllus emma, Gryllotalpa orientalis, Locusta migratoria, Oxva yezoensis and Schistocerca gregaria.

Insects of the order Collembola such as Onychiurus folsomi, Onychiurus sibiricus and Bourletiella hortensis.

Insects of the order Dyctyoptera such as Periplaneta fuliginosa, Periplaneta japonica and Blattella germanica.

Insects of the order Isoptera such as Coptotermes formosanus, Reticulitermes speratus and Odontotermes formosanus.

Insects of the order Isoptera such as Ctenocephalidae felis, Ctenocephalides canis, Echidnophaga gallinacea, Pulex irritans and Xenopsylla cheopis.

Insects of the order Mallophaga such as Menacanthus stramineus and Bovicola bovis.

Insects of the order Anoplura such as Haematopinus eurysternus, Haematopinus suis, Linognathus vituli and Solenopotes capillatus.

Tarsonemids such as Phytonemus pallidus, Polyphagotarsonemus latus and Tarsonemus bilobatus.

Eupodidae mites such as Penthaleus erythrocephalus and Penthaleus major.

Tetranychids such as Oligonychus shinkajii, Panonychus citri, Panonychus mori, Panonychus ulmi, Tetranychus kanzawai and Tetranychus urticae.

Eriophyids such as Acaphylla theavagrans, Aceria tulipae, Aculops lycopersici, Aculops pelekassi, Aculus schlechtendali, Eriophyes chibaensis and Phyllocoptruta oleivora.

Acarids such as Rhizoglyphus robini, Tyrophagus putrescentiae and Tyrophagus similis.

Bee mites such as Varroa jacobsoni.

Ticks such as Boophilus microplus, Rhipicephalus sanguineus, Haemaphysalis longicornis, Haemophysalis flava, Haemophysalis campanulata, Ixodes ovatus, Ixodes persulcatus, Amblyomma spp.and Dermacentor spp.

Cheyletids such as Cheyletiella yasguri and Cheyletiella blakei.

Demodicids such as Demodex canis and Demodex cacti.

Psoroptic mites such as Psoroptes ovis.

Sarcoptes mites such as Sarcoptes scabiei, Notoedres cati and Knemidocoptes spp.

Crustaceans such as Armadillidium vulgare.

Gastropods such as Pomacea canaliculata, Achatina fulica, Meghimatium bilineatum, Limax Valentiana, Acusta despecta sieboldiana and Euhadra peliomphala.

Nematodes such as Prathylenchus coffeae, Prathylenchus penetrans, Prathylenchus vulnus, Globodera rostochiensis, Heterodera glycines, Metoidogyne hapla, Meloidogyne incognita, Aphelenchoides besseyi and Bursaphelenchus xylophilus.

The internal, livestock, poultry or pet parasites that the compounds of the present invention can control include the following organisms, but the present invention is not restricted thereto.

Nematodes of the genera Haemonchus, Trichostrongylus, Ostertagia, Nematodirus, Cooperia, Ascaris, Bunostomum, Oesophagostomum, Chabertia, Trichuris, Storongylus, Trichonema, Dictyocaulus, Capillaria, Heterakis, Toxocara, Ascaridia, Oxyuris, Ancylostoma, Uncinaria, Toxascaris, Parascaris, and the like.

Nematodes of the family Filariidae such as the genera Wuchereria, Brugia, Onchoceca, Dirofilaria, Loa. and the like.

Nematodes of the family Dracunculidae such as the genus Deacunculus.

Cestodes such as Dipylidium caninum, Taenia taeniaeformis, Taenia solium, Taenia saginata, Hymenolepis diminuta, Moniezia benedeni, Diphyllobothrium latum, Diphyllobothrium erinacei, Echinococcus granulosus and Echinococcus multilocularis.

Trematodes such as Fasciola hepatica, F.gigantica, Paragonimus westermanii, Fasciolopsic bruski, Eurytrema pancreaticum, E.coelomaticum, Clonorchis sinensis, Schistosoma japonicum, Schistosoma haematobium and Schistosoma mansoni.

Eimeria spp. such as Eimeria tenella, Eimeria acervulina, Eimeria brunetti, Eimeria maxima, Eimeria necatrix, Eimeria bovis and Eimeria ovinoidalis.

Trypanosomsa cruzi, Leishmania spp., Plasmodium spp., Babesis spp., Trichomonadidae spp., Histomanas spp., Giardia spp., Toxoplasma spp., Entamoeba histolytica and Theileria spp.

The compounds of the present invention are effective against pests that have acquired resistance to conventional insecticides such as organic phosphorus compounds, carbamate compounds or pyrethroid compounds.

Namely, the compounds of the present invention can effectively control pests such as insects of the order Collembola, the order Dyctyoptera, the order Orthoptera, the order Isoptera, the order Thysanoptera, the order Hemiptera, the order Lepidoptera, the order Coleoptera, the order Hymenoptera, the order Diptera, the order Aphaniptera, the order Anoplura, mites, gastropods and nematodes at low doses. On the other hand, the compounds of the present invention have a quite advantageous feature that they are almost harmless to mammals, fishes, crustaceans and beneficial insects (useful insects such as honey bees and bumblebees and natural enemies such as aphelinids, Aphidiinae, tachina flies, Orius spp., Phytoseiidae spp. etc.).

The compounds of the present invention may be used in any dosage form such as a soluble concentrate, an emulsifiable concentrate, a wettable powder, a water soluble powder, a water dispersible granule, a water soluble granule, a suspension concentrate, a concentrated emulsion, a suspoemulsion, a microemulsion, a dustable powder, a granule, a tablet or an emulsifiable gel usually after mixed with an appropriate solid carrier or a liquid carrier, and if necessary, with a surfactant, a penetrant, a spreader, thickener, an anti-freezing agent, a binder, an anti-caking agent, a disintegrant, an antifoaming agent, a preservative, a stabilizer or the like. A formulation in an arbitrary dosage form may be sealed in water-soluble packaging such as a water-soluble capsule or a water-soluble film, for labor saving or improved safety.

As solid carriers, natural minerals such as quartz, calcite, meerschaum, dolomite, chalk, kaolinite, pyrophyllite, sericite, halloysite, methahalloysite, kibushi clay, gairome clay, pottery stone, zeeklite, allophone, Shirasu, mica, talc, bentonite, activated clay, pumice, attapulgite, zeolite and diatomaceous earth, calcined natural minerals such as calcined clay, pearlite, Shirasu-balloons, vermiculite, attapulgus clay and calcined diatomaceous earth, inorganic salts suchas magnesium carbonate, calcium carbonate, sodium carbonate, sodium hydrogen carbonate, ammonium sulfate, sodium sulfate, magnesium sulfate, diammonium hydrogen phosphate, ammonium dihydrogen phosphate and potassium chloride, saccharides suchas glucose, fructose, sucrose and lactose, polysaccharides such as starch, cellulose powder and dextrin, organic substances such as urea, urea derivatives, benzoic acid and benzoic acid salts, plants such as wood flour, powdered cork, corncob, walnut shell and tobacco stems, fly ash, white carbon (such as hydrated synthetic silica, anhydrous synthetic silica and hydrous synthetic silicate), fertilizers and the like may be mentioned.

As liquid carriers, aromatic hydrocarbons such as xylene, alkyl ($C_9$ or $C_{10}$ etc.) benzene, phenylxylylethane and alkyl($C_1$ or $C_3$ etc.)naphthalene, aliphatic hydrocarbons suchas machine oil, normal paraffin, isoparaffin and naphthene, mixtures of aromatic hydrocarbons and aliphatic hydrocarbons such as kerosene, alcohols such as ethanol, isopropanol, cyclohexanol, phenoxyethanol and benzyl alcohol, polyhydric alcohols suchas ethylene glycol, propylene glycol, diethylene glycol, hexylene glycol, polyethylene glycol and polypropylene glycol, ethers such as propyl cellosolve, butyl cellosolve, phenyl cellosolve, propylene glycol monomethyl ether, propylene glycol monoethyl ether, propylene glycol monopropyl ether, propylene glycol monobutyl ether and propylene glycol monophenyl ether, ketones such as acetophenone, cyclohexanone and $\gamma$-butyrolactone, esters such as fatty acid methyl esters, dialkyl succinates, dialkyl glutamate, dialkyl adipates and dialkyl phthalates, acid amides such as N- alkyl($C_1$, $C_8$ or $C_{12}$ etc.)pyrrolidone, fats and oils suchas soybean oil, linseed oil,rapeseed oil, coconut oil, cottonseed oil and castor oil, dimethyl sulfoxide, water and the like may be mentioned.

These solid and liquid carriers may be used alone or in combinations of two or more.

As surfactants, nonionic surfactants such as polyoxyethylene alkyl ether, polyoxyethylene alkyl(mono or di)phenyl ether, polyoxyethylene(mono, di or tri)styrylphenyl ether, polyoxyethylenepolyoxypropylene block copolymers, polyoxyethylene fatty acid (mono or di)ester, sorbitan fatty acid ester, polyoxyethylene sorbitan fatty acid ester, ethylene oxide adducts of castor oil, acetylene glycol, acetylene alcohol, ethylene oxide adducts of acetylene glycol, ethylene oxide adducts of acetylene alcohol and alkyl glycosides, anionic surfactants such as alkyl sulfate salts, alkylbenzenesulfonic acid salts, lignin sulfonate, alkylsulfosuccinic acid salts, naphthalenesulfonic acid salts, alkylnaphthalenesulfonic acid salts, salts of naphthalenesulfonic acid-formalin condensates, salts of alkylnaphthalenesulfonic acid-formalin condensates, polyoxyethylene alkyl ether sulfate or phosphate salts, polyoxyethylene(mono or di) alkylphenyl ether sulfate or phosphate salts, polyoxyethylene(mono, di or tri)styrylphenyl ether sulfate or phosphate salts, polycarboxylic acid salts (such as polyacrylates, polymaleates and copolymers of maleic acid and an olefin) and polystyrenesulfonic acid salts, cationic surfactants such as alkylamine salts and alkyl quaternary ammonium salts, amphoteric surfactants such as amino acid types and betaine types, silicone surfactants and fluorine surfactants may be mentioned.

The amount of these surfactans is usually preferred to be from 0.05 to 20 parts by weight per 100 parts by weight of the agent of the present invention, though there is no particular restrictions. These surfactants may be used alone or in combination of two or more.

The suitable application dose of compounds of the present invention is generally about from 0.005 to 50 kg per hectare (ha) in terms of the active ingredient, though it varies depending on the application site, the application season, the application method and the cultivated crop.

When the compounds of the present invention are used to control external or internal parasites in and on mammals and birds as farm animals and pet aminals, the compounds of the present invention may be administered in an effective amount together with pharmaceutically acceptable additives orally, parenterally by injection (intramuscular, subcutaneously, intravenously or intraperitoneally); percutaneously by dipping, spraying, bathing, washing, pouring-on and spotting-on and dusting, or intranasally. The compounds of the present invention may be administerd through molded articles suchas chips, plates, bands, collars, ear marks, limb bands and ID tags. The compounds of the present invention are administered in an arbitrary dosage form suitable for the administration route.

The dosage form may be a solid preparation such as a dust, a granule, a wettable powder, a pellet, a tablet, a ball, a capsule and an molded article containing an active ingredeient, a liquid preparation such as an injection fluid, an oral liquid, a liquid preparation applied to the skin or coelom, a pour-on preparation, a spot-on preparation, a flowable, an emulsion, and a semisolid preparation such as an ointment and a gel.

A solid preparation may generally be used by oral administration or by percutaneous or environmental application after dilution with water or the like. A solid preparation can be prepared by mixing an active ingredient with an appropriate vehicle, and with an adjuvant if necessary, and formulating the mixture into a desired dosage form. As the vehicle, an inorganic vehicle such as a carbonate, a hydrogen carbonate, a phosphate, aluminum oxide, silica or clay or an organic vehicle such as a saccharide, cellulose, cereal flour or starch.

An injection fluid may be administered intravenously, intramuscularly or subcutaneously. An injection fluid can be prepared by dissolving an active ingredient in an appropriate solvent and, if necessary, adding additives such as a solubilizer, an acid, a base, a buffering salt, an antioxidant and a protectant. As appropriate solvents, water, ethanol, butanol, benzyl alcohol, glycerine, propylene glycol, polyethylene glycol, N-methylpyrrolidone and mixtures thereof, physiolosically acceptable vegetable oils and synthetic oils suitable for injection may be mentioned. As solubilizers, polyvinylpyrrolidone, polyoxyethylated castor oil, polyoxyethylated sorbitan ester and the like may be mentioned. As protectants, benzyl alcohol, trichlorobutanol, p-hydroxybenzoic acid esters, n-butanol and the like may be mentioned.

An oral liquid may be aministered directly or after dilution and can be prepared in the same manner as an injection fluid.

A flowable, an emulsion or the like may be administerd directly or after dilution percutaneously or by environmental application.

A liquid preparation applied to the skin is administered by dripping, spreading, rubbing, spraying, sprinkling or dipping (soaking, bathing or washing) and can be prepared in the same manner as an injection fluid.

A pour-on preparation and a spot-on preparation are dripped or sprayed to a limited area of the skin so that they permeate through the skin and act systemically. A pour-on preparation and a spot-on preparation can be prepared by dissolving, suspending or emulsifying an active ingredient in an skin-friendlysolvent or solvent mixture. If necessary, additives suchas a surfactant, a colorant, an absorbefacient, an antioxidant, a light stabilizer and an adhesive.

As appropriate solvents, water, alkanol, glycol, polyethylene glycol, polypropylene glycol, glycerine, benzyl alcohol, phenylethanol, phenoxyethanol, ethyl acetate, butyl acetate, benzyl benzoate, dipropylene glycol monomethyl ether, diethylene glycol monobutyl ether, acetone, methyl ethyl ketone, aromatic and/or aliphatic hydrocarbons, vegetable or synthetic oils, DMF, liquid paraffin, light liquid paraffin, silicone, dimethylacetamide, N-methylpyrrolidone or 2,2-dimethyl-4-oxymethylene-1,3-dioxolane may be mentioned. As absorbefacients, DMSO, isopropyl myristate, pelargonic acid dipropylene glycol, silicone oil, fatty acid esters, triglycerides and aliphatic alcohols may be mentioned. As antioxidants, sulfites, metabisulfites, ascorbic acid, butylhydroxytoluene, butylhydroxyanisole and tocopherol may be mentioned.

An emulsion may be administered orally, percutaneously or by injection. An emulsion can be prepared by dissolving an active ingredient in a hydrophobic phase or a hydrophilic phase and homogenizing the resulting solution with another liquid phase together with an appropriate emulsifier, and further with additives such as a colorant, if necessary an absorbefacient, a protectant, an antioxidant, a light screen and a thickner.

As hydrophobic phases (oils), paraffin oil, silicone oil, sesame oil, almond oil, castor oil, synthetic triglycerides, ethyl stearate, di-n-butyryl adipate, hexyl laurate, pelargonic acid dipropylene glycol, esters of branched short-chain fatty acids with C16-C18 saturated fatty acids, isopropyl myristate, isopropyl palmitate, esters of C12-C18 saturated alcohols with caprylic/capric acid, isopropyl stearate, oleyl oleate, decyl oleate , ethyl oleate, ethyl lactate, fatty acid ester waxes, dibutyl phthalate, diisopropyl adipate, isotridecyl alcohol, 2-octyldodecanol, cetylstearyl alcohol and oleyl alcohol may be mentioned.

As hydrophilic phases, water, propylene glycol, glycerine and sorbitol may be mentioned.

As emulsifiers, nonionic surfactants such as polyoxyethylated castor oil, polyoxyethylated sorbitan monoolefinic acid,sorbitan monostearate, glycerine monostearate, polyoxyethyl stearate and alkyl phenol polyglycol ether; amphoteric surfactants such as disodium N-lauryl-β-iminodipropionate and lecithin; and anionic surfactants such as sodium lauryl sulfate, aliphatic alcohol sulfate ether, mono/dialkylpolyglycol orthophosphate monoethanolamine salt may be mentioned.

As other addivies, carboxymethylcellulose, methylcellulose, polyacrylate, alginate, gelatin, gum arabic, polyvinylpyrrolidone, polyvinyl alcohol, methyl vinyl ether, maleic anhydride copolymers, polyethylene glycol, waxes and colloidal silica may be mentioned.

A semisolid preparation is administered by applying or spreading onto the skin or introducing into the coelom. A gel can be prepared by adding a thickner to a solution prepared in the same manner as an injection fluid sufficiently to give a transparent viscous substance like an ointment.

Next, formulation examples of preparations using the compounds of the present invention are given below. However, formulations of the present invention are by no means restricted thereto. In the following Formulation Examples, "parts" means parts by weight.

[Wettable powder]

| | |
|---|---|
| Compound of the present invention | 0.1 to 80 parts |
| Solid carrier | 5 to 98.9 parts |
| Surfactant | 1 to 10parts |
| Others | 0 to 5 parts |

As the others, an anti-caking agent, a stabilizer and the like may be mentioned.

[Emulsion]

| | |
|---|---|
| Compound of the present invention | 0.1 to 30 parts |
| Liquid carrier | 45 to 95 parts |
| Surfactant | 4.9 to 15 parts |
| Others | 0 to 10parts |

As the others, a spreader, a stabilizer and the like may be mentioned.

[Suspension]

| | |
|---|---|
| Compound of the present invention | 0.1 to 70 parts |
| Liquid carrier | 15 to 98.89 parts |
| Surfactant | 1 to 12 parts |
| Others | 0.01 to 30 parts |

As the others, an anti-freezing agent, a thickner and the like may be mentioned.

[Water soluble granule]

| | |
|---|---|
| Compound of the present invention | 0.1 to 90 parts |
| Solid carrier | 0 to 98.9 parts |
| Surfactant | 1 to 20 parts |
| Others | 0 to 10 parts |

As the others, a binder, a stabilizer and the like may be mentioned.

[Sobluble concentrate]

| | |
|---|---|
| Compound of the present invention | 0.01 to 70 parts |
| Liquid carrier | 20 to 99.99 parts |
| Others | 0 to 10 parts |

As the others, an anti-freezing agent, a spreader and the like may be mentioned.

[Granule]

| | |
|---|---|
| Compound of the present invention | 0.01 to 80 parts |
| Solid carrier | 10 to 99.99 parts |
| Others | 0 to 10 parts |

As the others, a bind, a stabilizer and the like may be mentioned.

[Dust]

| | |
|---|---|
| Compound of the present invention | 0.01 to 30 parts |
| Solid carrier | 65 to 99.99 parts |
| Others | 0 to 5 parts |

As the others, an anti-drift agent, a stabilizer and the like may be mentioned.
Next, more specific examples of preparations containing compounds of the present invention as an active ingredient are by no means restricted thereto.
In the following Formulation Examples, "parts" means parts by weight.

[Formulation Example 1] Wettable powder

| | |
|---|---|
| Compound No.1-001 of the present invention | 20 parts |
| Pyrophyllite | 74 parts |
| Sorpol 5039 | 4 parts |
| (trade name for a mixture of a nonionic surfactant and an anionic surfactant: manufactured by TOHO Chemical Industry Col., Ltd.) | |
| CARPLEX #80D | 2 parts |

(hydrous synthetic silicic acid: trade name manufactured by Shionogi & Co., Ltd.)

The above ingredients are mixed and pulverized homogenously to obtain a wettable powder.

[Formulation Example 2] Emulsifiable concentrate

| | |
|---|---|
| Compound No.1-001 of the present invention | 5 parts |
| xylene | 75 parts |
| N-methylpyrrolidone | 15 parts |
| Sorpol 2680 | 5 parts |

(trade name for a mixture of a nonionic surfactant and an anionic surfactant: manufactured by TOHO Chemical Industry Co., Ltd.)

The above ingredients are mixed homogenously to obtain an emulsifiable concentrate.

[Formulation Example 3] Suspension concentrate

| | |
|---|---|
| Compound No.1-001 of the present invention | 25 parts |
| AGRISOL S-710 | 10 parts |
| (trade name for a nonionic surfactant: manufactured by Kao Corporation) | |
| Lunox 1000C | 0.5 part |
| (trade name for an anionic surfactant: manufactured by TOHO Chemical Industry Co., Ltd.) | |
| Xanthan gum | 0.2 part |
| Water | 64.3 parts |

The above ingredients are mixed homogenously and wet-pulverized to obtain a suspension concentration.

[Formulation Example 4] Water soluble granule

| | |
|---|---|
| Compound No.1-001 of the present invention | 75 parts |
| HITENOL NE-15 | 5 parts |
| (trade name for an anionic surfactant: manufactured by Dai-ichi Kogyo Seiyaku Co., Ltd.) | |
| VANILLEX N | 10 parts |
| (trade name for an anionic surfactant: manufactured by Nippon Paper Industries Co., LTD.) | |
| CARPLEX #80D | 10 parts |

(trade name for hydrous synthetic silicic acid :manufactured by Shionogi & Co., Ltd.)

The above ingredients are mixed and pulverized homogenously, then kneaded with a small amount of water, granulated through an extrusion granulator and dried to obtain a water soluble granule.

[Formulation Example 5] Granule

| | |
|---|---|
| Compound No.1-001 of the present invention | 5 parts |
| Bentonite | 50 parts |
| Talc | 45 parts |

The above ingredients are mixed and pulverized homogenously, then kneaded with a small amount of water, granulated through an extrusion granulator and dried to obtain a granule.

[Formulation Example 6] Dust

| | |
|---|---|
| Compound No.1-001 of the present invention | 3 parts |
| CARPLEX #80D | 0.5 part |
| (trade name for a hydrous synthetic silicic acid: manufactured by Shionogi & Co., Ltd.) | |
| Kaolinite | 95 parts |
| Diisopropyl phosphate | 1.5 parts |

The above ingredients are mixed and pulverized homogeneously to obtain a dust.

It is applied after diluted with water by a factor of from 1 to 10000 or directly without dilution.

[Formulation Example 7] Wettable powder preparation

| | |
|---|---|
| Compound No.1-001 of the present invention | 25 parts |
| Sodium diisobutylnaphthalenesulfonate | 1 part |
| Calcium n-dodecylbenzenesulfonate | 10 parts |
| Alkyl aryl polyglycol ether | 12 parts |
| Naphthalenesulfonic acid-formalin condensate sodium salt | 3 parts |
| Silicone emulsion | 1 part |
| Silicon dioxide | 3 parts |
| Kaolin | 45 parts |

[Formulation Example 8] Water-soluble concentrate preparation

| | |
|---|---|
| Compound No.1-001 of the present invention | 20 parts |
| Polyoxyethylenelauryl ether | 3 parts |
| Sodium dioctylsulfosuccinate | 3.5 parts |
| Dimethyl sulfoxide | 37 parts |
| 2-Propanol | 36.5 parts |

[Formulation Example 9] Liquid preparation for spraying

| | |
|---|---|
| Compound No.1-001 of the present invention | 2 parts |
| Dimethyl sulfoxide | 10 parts |
| 2-Propanol | 35 parts |
| Acetone | 53 parts |

[Formulation Example 10] Liquid preparation for percutaneous administration

| | |
|---|---|
| Compound No.1-001 of the present invention | 5 parts |
| Hexylene glycol | 50 parts |
| Isopropanol | 45 parts |

[Formulation Example 11] Liquid preparation for percutaneous administration

| | |
|---|---|
| Compound No.1-001 of the present invention | 5 parts |
| Propylene glycol monomethyl ether | 50 parts |
| Dipropylene glycol | 45 parts |

[Formulation Example 12] Liquid preparation for percutaneous administration (by dripping)

| | |
|---|---|
| Compound No.1-001 of the present invention | 2 parts |
| Light liquid paraffin | 98 parts |

[Formulation Example 13] Liquid preparation for percutaneous administration (by dripping)

| | |
|---|---|
| Compound No.1-001 of the present invention | 2 parts |
| Light liquid paraffin | 58 parts |
| Olive oil | 30 parts |
| ODO-H | 9 parts |
| Shin-etsu silicone | 1 part |

For use as agrochemicals, if necessary, the compounds of the present invention may be mixed with other herbicides, various insecticides, miticides, nematocides, plant growth regulators, synersists, fertilizers, soil conditioners and the like at the time of formulation or application.

Particularly, the combined use with other agrochemicals or plant hormones is expected to reduce the application cost by enabling control at lower doses and lead to a broader insecticidal spectrum and higher insecticidal effect due to the synergistic effect of the other agrochemicals. In such cases, they may be combined with a plurality of known agrochemicals. The agrochemicals to be used in combination with the compounds of the present invention include, for example, the compounds disclosed in The Pesticide Manual, 15th edition, 2009, having the generic names recited below, but are not necessarily restricted thereto.

Fungicide: such as acibenzolar-S-methyl, acylaminobenzamide, acypetacs, aldimorph , ametoctradin, amisulbrom, amobam, ampropyfos, anilazine, azaconazole, azithiram, azoxystrobin, barium polysulfide, benalaxyl, benalaxyl-M, benodanil, benomyl, benquinox, bentaluron, benthiavalicarb, benthiazole, benzamacril, benzamorf, bethoxazine, binapacryl, biphenyl, bitertanol, blasticidin-S, bixafen, bordeaux mixture, boscalid, bromoconazole, bupirimate, buthiobate, calcium polysulfide, calcium polysulfide, captafol, captan, carpropamid, carbamorph, carbendazim, carboxin, carvone, cheshunt mixture, chinomethionat, chlobenthiazone, chloraniformethane, chloranil, chlorfenazol, chloroneb, chloropicrin, chlorothalonil, chlorquinox, chlozolinate, climbazole, clotrimazole, copper acetate, copper carbonate, basic, copper hydroxide, copper naphthenate, copper oleate, copper oxychloride, sulfate, copper sulfate, basic, copper zinc chromate, cufraneb, cuprobam, cyazofamid, cyclafuramid, cycloheximide, cyflufenamid, cymoxanil, cypendazole, cyproconazol, cyprodinil, cyprofuram, dazomet, debacarb, decafentin, dehydroacetic acid, dichlofluanid, dichlone, dichlorophen, dichlozoline, diclobutrazol, diclocymet, diclomedine and dicloran.

Fungicide (continued): such as diethofencarb, difenoconazole, diflumetorim, dimethirimol, dimethomorph, dimoxystrobin, diniconazole, diniconazole-M, dinobuton, dinocap, dinocap-4, dinocap-6, dinocton, dinosulfon, dinoterbon, diphenylamine, dipyrithione, ditalimfos, dithianon, dodemorph, dodine, drazoxolon, edifenphos, epoxiconazole, etaconazole, ethaboxam, etem, ethirimol, ethoxyquin, etridiazole, famoxadone, fenarimol, febuconazole, fenamidone, fenaminosulf, fenapanil, fendazosulam, fenfuram), fenhexamid, fenitropan, fenoxanil, fenpiclonil, fenpropidin, fenpyrazamine, fenpropimorph, fentin, ferbam, ferimzone, fluazinam, fludioxonil, flumetover, flumorph, fluopicolide, fluopyram, fluoroimide, fluotrimazole, fluoxastrobin, fluquinconazole, flusilazole, flusulfamide, flutianil, flutolanil, flutriafol, fluxapyroxad, folpe), fosetyl-aluminium, fuberidazole, furalaxyl, furametpyr, furcarbanil, furconazole, furconazole-cis, furmecyclox, furphanate, glyodin, griseofulvin, guazatine, halacrinate, hexachlorobenzene, hexaconazole, hexylthiofos, 8-hydroxyquinoline sulfate, hymexazol, imazalil, imibenconazole, iminoctadine, ipconazole, iprobenfos, iprodione, iprovalicarb, isoprothiolane and isovaledione.

Fungicide (continued): such as kasugamycin, kresoxim-methyl, mancopper, mancozeb, mandipropamid, maneb, mebenil, mecarbinzid, mepanipyrim, mepronil, metalaxyl, metalaxyl-M, metam, metazoxolon, metconazole, methasulfocarb, methfuroxam, methyl isothiocyanate, metiram, metominostrobin, metrafenone, metsulfovax, milneb, myclobutanil, myclozolin, nabam, natamycin, nickel bis(dimethyldithiocarbamate), nitrostyrene, nitrothal-isopropyl, nuarimol, OCH, octhilinone, ofurace, orysastrobin, oxadixyl, oxine copper, oxycarboxin, (oxpoconazole fumarate, pefurzoate, penconazole, penflufen, pencycuron, penthiopyrad, o-phenylphenol, phosdiphen, phthalide, picoxystrobin, piperalin, polycarbamate, polyoxins, polyoxorim, potassium azide, potassium hydrogen carbonate, proquinazid, probenazole, prochloraz, procymidone, propamocarb hydrochloride, propiconazole, propineb, prothiocarb, prothioconazole, pyracarbolid, pyraclostrobin, pyrazophos, pyridinitril, pyrifenox, pyrimethanil, pyriofenone, pyroquilon, pyroxychlor, pyroxyfur, quinomethionate, quinoxyfen, quintozene, quinacetol-sulfate, quinazamid, quinconazole and rabenzazole.

Fungicide (continued): such as sodium azide, sodium hydrogen carbonate, sodium hypochlorite, sulfur, spiroxamine, salycylanilide, silthiofam, simeconazole, tebuconazole, tecnazene, tecoram, tetraconazole, thiabendazole, thiadifluor, thicyofen, thifluzamide, thiochlorfenphim, thiophanate, thiophanate-methyl, thioquinox, thiram, tiadinil, tioxymid,

tolclofos-methyl, tolylfluanid, triadimefon, toriadimenol, triamiphos, triarimol, triazoxide, triazbutil, tributyltin oxide, trichlamide, tricyclazole, tridemorph, trifloxystrobin, triflumizole, triforine, triticonazole, validamycin, valifenalate, vinclozolin, zarilamide, zinc sulfate, zineb, ziram, zoxamide and shiitake mushroom mycelium extracts.

Bacteriocides: such as benzalkonium chloride, bithionol, bronopol, cresol, formaldehyde, nitrapyrin, oxolinic acid, oxyterracycline, streptomycin and tecloftalam.

Nematocides: such as aldoxycarb, cadusafos, DBCP, dichlofenthion, DSP, ethoprophos, fenamiphos, fensulfothion, fluensulfone, fosthiazate, fosthietan, imicyafos, isamidofos, isazofos, oxamyl and thionazin.

Miticides: such as acequinocyl, acrinathrin, amitraz, BCI-033 (experimental name), bifenazate, bromopropylate, chinomethionat, chlorobezilate, clofentezine, cyenopyrafen, cyflumetofen, cyhexatine, dicofol, dienochlor, DNOC, etoxazole, fenazaquin, fenbutatin oxide, fenothiocarb, fenpropathrin, fenpyroximate, fluacrypyrim, halfenprox, hexythiazox, milbemectin, propargite, pyridaben, pyrimidifen, S-1870 (experimental name), spirodiclofen, spyromesifen, NNI-0711 (experimental name), CL900167 (experimental name) and tebufenpyrad.

Insecticides: such as abamectin, acephate, acetamipirid, alanycarb, aldicarb, allethrin, azamethiphos, azinphos-methyl, bacillus thuringiensis, bendiocarb, benfluthrin, benfuracarb, bensultap, bifenthrin, bioallethrin, bioresmethrin, bistrifluron, buprofezin, butocarboxim, carbaryl, carbofuran, carbosulfan, cartap, chlorantraniliprole, chlorethxyfos, chlorfenapyr, chlorfenvinphos, chlorfluazuron, chlormephos, chlorpyrifos, chlorpyrifos-methyl, chromafenozide, clothianidin, cyantraniliprole, cycloprothrin, cyflumetofen, cyfluthrin, beta-cyfluthrin, cyhalothrin, lambda-cyhalothrin, cypermethrin, alpha-cypermethrin, beta-cypermethrin, zeta-cypermethrin, cyromazine, deltamethrin, diacloden, diafenthiuron, diazinon, dichlorvos, diflubenzuron, dimethylvinphos, dinotefuran, diofenolan, disulfoton, dimethoate, emamectin-benzoate, empenthrin, endosulfan, alpha-endosulfan, EPN, esfenvalerate, ethiofencarb, ethiprole, etofenprox, etrimfos, fenitrothion, fenobucarb, fenoxycarb, fenpropathrin, fenthion, fenvalerate, fipronil, flonicamid, flubendiamide, flucythrinate, flufenerim, flufenoxuron, flufenprox, flumethrin, fluvalinate, tau-fluvalinate, fonophos, formetanate, formothion, furathiocarb, flufiprole, flupyradifurone and flometoquin.

Insecticides (continued): such as halofenozide, hexaflumuron, hydramethylnon, imidacloprid, isofenphos, indoxacarb, isoprocarb, isoxathion, lepimectin, lufenuron, malathion, meperfluthrin, metaflumizone, metaldehyde, methamidophos, methidathion, methacrifos, metaflumizone, metalcarb, methomyl, methoprene, methoxychlor, methoxyfenozide, methyl bromide, monocrotophos, muscalure, nitenpyram, novaluron, noviflumuron, omethoate, oxamyl, oxydemeton-methyl, oxydeprofos, parathion, parathion-methyl, pentachlorophenol(PCP), permethrin, phenothrin, phenthoate, phoxim, phorate, phosalone, phosmet, phosphamidon, pirimicarb, pirimiphos-methyl, profenofos, prothiofos, propaphos, protrifenbute, pymetrozine, pyraclofos, pyrethrins, pyridalyl, pyrifluquinazon, pyriprole, pyrafluprole, pyriproxyfen, resmethrin, rotenone, SI-0405 (experimental name), sulprofos, silafluofen, spinetoram, spinosad, spirotetramat, sulfoxaflor, sulfotep, SYJ-159 (experimental name), tebfenozide, teflubenzuron, tefluthorin, terbufos, tetrachlorvinphos, tetramethrin, d-tetramethrin, tetramethylfluthrin, thiacloprid, thiocyclam, thiodicarb, thiamethoxam, thiofanox, thiometon, tolfenpyrad, tralomethrin, trichlorfon, triazuron, triflumuron, vamidothion and ME-5343 (experimental name).

## EXAMPLES

Now, the present invention will be described in further detail by referring to the following specific Examples of syntheses of and tests on the compounds of the present invention. However, the present invention is by no means restricted thereto.

For the preparative medium pressure liquid chromatography, the preparative medium pressure chromatograph; YFLC-Wprep manufactured by Yamazen Science, Inc., (flow rate 18ml/min, 40-μm silica gel column) was used.

## [SYNTHETIC EXAMPLES]

### SYNTHETIC EXAMPLE 1

Preparation of tert-butyl 3-methyl-1-(pyridin-3-yl)-1H-pyrazol-4-ylcarbamate (Compound No.1-011 of the present invention)

#### Step 1

Preparation of ethyl 3-methyl-1-(pyridin-3-yl)-1H-pyrazole-4-carboxylate

3.0 g of ethyl 3-methyl-1 H-pyrazole-4-carboxylate in 10 ml of N,N-dimethylformamide was mixed with 5.3 g of 3-iodopyridine, 990 mg of copper (I) iodide and 17.0 g of cesium carbonate successively. After the atmosphere in the reaction vessel was replaced by nitrogen gas, the mixture was stirred at 120°C for 4 hours. After the reaction, the reaction mixture was mixed with 100 ml of water and extracted with ethyl acetate (150 ml × 1). The resulting organic layer was

dried over anhydrous sodium sulfate, and the solvent was evaporated under reduced pressure. The resulting residue was purified by silica gel column chromatography using n-hexane - ethyl acetate {1:1 (volume ratio, hereinafter the same applies)} as the eluent to give 2.4 g of the desired product as white crystals.
$^1$H NMR($CDCl_3$ ,$Me_4$ Si,300MHz)δ8.98(d,J=2.4Hz,1H),8.58(d,J=3.9Hz,1H), 8.38(s,1H),8.00-8.10(m, 1H),7.42(dd, J=4.8,8.1Hz,1H),4.34(q,J=7.2Hz,2H),2.57(s,3H), 1.38(t,J=7.2Hz,3H),(no detectable proton peaks for $CO_2$ H)

Step 2

Preparation of 3-methyl-1-(pyridin-3-yl)-1H-pyrazole-4-carboxylic acid

3.9 g of ethyl 3-methyl-1-(pyridin-3-yl)-1H-pyrazole-4-carboxylate was dissolved in 20 mL of ethanol, and 1.9 g of potassium hydroxide and 10 mL of water were added successively. After the addition, the reaction mixture was stirred at 50°C for 1 hour. After the reaction, the solvent was evaporated under reduced pressure, and the resulting residue was neutralized with aqueous hydrochloric acid and extracted with ethyl acetate (50 ml × 2). The resulting organic layer was dried over anhydrous sodium sulfate, and the solvent was evaporated under reduced pressure to give 2.1 g of the desired product as a white solid.
$^1$H NMR(DMSO-d6,$Me_4$ Si,300MHz)δ9.03(m,1H),8.71(s,1H),8.46(d,J=2.1Hz,1H), 8.18(d,J=8.4Hz,1H),7.50(dd, J=8.4Hz,4.8Hz,1H),2.40(s,3H)
m.p.;200-210°C

Step 3

Preparation of tert-butyl 3-methyl-1-(pyridin-3-yl)-1H-pyrazol-4-ylcarbamate

To 4.98 g of 3-methyl-1-(pyridin-3-yl)-1H-pyrazole-4-carboxylic acid in 50 ml of 2-methyl-2-propanol, 7.44 g of triethylamine and 8.78 g of diphenylphosphoryl azide were added at room temperature successively. After the addition, the reaction mixture was refluxed with 2-methyl-2-propanol for 4 hours with stirring. After the reaction, the reaction mixture was allowed to cool to room temperature and poured into ice-water and extracted with 50 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:1) as the eluent to give 3.67 g of the desired product as a white solid.
$^1$H NMR($CDCl_3$,$Me_4$Si,300MHz)δ8.94(d,J=1.2Hz,1H),8.46(dd,J=4.5,2.7Hz,1H), 8.25(brs,1H),7.95(ddd,J=8.4,2.7, 1.2Hz,1H),7.33(dd,J=8.4,4.5Hz,1H),6.18(brs,1H), 2.28(s,3H),1.52(s,9H)

SYNTHETIC EXAMPLE 2

Preparation of tert-butyl methyl{3-methyl-1-(pyridin-3-yl)-1H-pyrazol-4-yl}carbamate

(Compound No.1-012 of the present invention)

To 83 mg of 60 wt% sodium hydride (dispersed in mineral oil) in 5 ml of N,N-dimethylformamide, 500 mg of tert-butyl 3-methyl-1-(pyridin-3-yl)-1H-pyrazol-4-ylcarbamate was added under cooling with ice. After the addition, the reaction mixture was allowed to warm to room temperature and stirred at room temperature for 30 minutes. After the stirring, the reaction mixture was mixed with 284 mg of methyl iodide and stirred at room temperature for other 30 minutes. After the reaction, the reaction mixture poured into ice-water and extracted with 10 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (2:1) as the eluent to give 0.44 g of the desired product as a white solid.
$^1$H NMR($CDCl_3$,$Me_4$Si,300MHz)δ8.89(d,J=2.7Hz,1H),8.49(dd,J=4.8,1.2Hz,1H),8.03-7.95(m,1H),7.82(brs,1H),7.36(dd, J=8.1,4.8Hz,1H),3.19(s,3H),2.26(s,3H),1.44(brs,9H)

SYNTHETIC EXAMPLE 3

Preparation of N-methyl-N-{3-methyl-1-(pyridin-3-yl)-1H-pyrazol-4-yl}-3-(methylthio)propanamide (Compound No. 1-018 of the present invention)

Step 1

Preparation of N,3-dimethyl-1-(pyridin-3-yl)-1H-pyrazole-4-amine dihydrochloride

(Compound No. A-001)

0.35 g of tert-butyl methyl{3-methyl-1-(pyridin-3-yl)-1H-pyrazol-4-yl}carbamate in 5 ml of 1,4-dioxane was mixed with 1.2 ml of about 4 M hydrogen chloride in 1,4-dioxane and stirred at room temperature for 2 days. After the reaction, the impurities in the reaction mixture were filtered off under reduced pressure, and the filtrate was evaporated under reduced pressure to remove the solvent to give 0.34 g of the desired product as a white solid.
m. p.; 174-179°C

Step 2

Preparation of N-methyl-N-{3-methyl-1-(pyridin-3-yl)-1H-pyrazol-4-yl}-3-(methylthio)propanamide

150 mg of N,3-dimethyl-1-(pyridin-3-yl)-1H-pyrazole-4-amine dihydrochloride in 5 ml of dichloromethane was mixed with 174 mg of triethylamine, 103 mg of 3-(methylthio)propanoic acid, 165 mg of 1-ethyl-3-(3-dimethylaminopropyl)carbodiimide hydrochloride and 10 mg of 4-dimethylaminopyridine successively and stirred at room temperature for 12 hours. After the reaction, the reaction mixture was mixed with 10 ml of water and extracted with 10 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:2) as the eluent t to give 78 mg of the desired product as a yellow oil.
$^1$H NMR($CDCl_3$,$Me_4Si$,300MHz)δ8.93(d,J=2.7Hz,1H),8.56(dd,J=4.8,1.2Hz,1H), 8.01(ddd,J=8.4,2.7,1.2Hz,1H),7.90(s, 1H),7.41(dd,J=8.4,4.8Hz,1H),3.22(s,3H), 2.77(t,J=7.2Hz,2H),2.43(t,J=7.2Hz,2H),2.27(s,3H),2.04(s,3H)

SYNTHETIC EXAMPLE 4

Preparation of 2,5-dibromo-N-{1-(pyridin-3-yl)-1H-pyrazol-4-yl}pentanamide (Compound No. 1-025 of the present invention)

To 1.5 g of 1-(pyridin-3-yl)-1H-pyrazole-4-amine dihydrochloride in 20 ml of dichloromethane, 2.3 g of triethylamine and 2.5 g of 2,5-dibromovaleryl bromide were added under cooling with ice successively. After the addition, the reaction mixture was stirred at room temperature for 3 hours. After the reaction, the reaction mixture was mixed with 20 ml of water and extracted with 20 ml of chloroform. The resulting organic layer was dried over anhydrous sodium sulfate, and the solvent was evaporated under reduced pressure. The resulting residue was purified by preparative medium pressure liquid chromatography using n-hexane - ethyl acetate (with a gradient from 9:1 to 1:3) as the eluent to give 1.7 g of the desired product as a white solid. $^1$H NMR($CDCl_3$ ,$Me_4$ Si,300MHz)δ9.01 (d,J=2.4Hz,1H),8.56(s,1H), 8.56(dd, J=4.8,1.2Hz,1H),8.14(brs,1H),8.03(ddd,J=8.1,2.4,1.2Hz,1H),7.75(s,1H), 7.41 (dd,J=8.1,4.8Hz,1H),4.53(dd,J=7.8, 4.8Hz,1H),3.47(t,J=6.3Hz,2H),2.38-2.50(m,1H),2.24-2.36(m,1H),2.07-2.18(m,2H)

SYNTHETIC EXAMPLE 5

Preparation of 3-bromo-1-{1-(pyridin-3-yl)-1H-pyrazol-4-yl}piperidin-2-one (Compound No. 1-027 of the present invention)

To 1.54 g of 2,5-dibromo-N-{1-(pyridin-3-yl)-1H-pyrazol-4-yl}pentanamide in 30 ml of tetrahydrofuran, 0.29 g of 60 wt% sodium hydride (dispersed in mineral oil) was added under cooling with ice. After the addition, the reaction mixture was stirred at room temperature for 4 hours. After the reaction, the reaction mixture was mixed with saturated aqueous ammonium chloride and extracted with 40 ml of ethyl acetate. The resulting organic layer was dried over anhydrous sodium sulfate, and the solvent was evaporated under reduced pressure. The precipitated solid was washed with diisopropyl ether to give 1.11 g of the desired product as a pale yellow solid. $^1$H NMR($CDCl_3$,$Me_4$Si,300MHz)δ9.02(d, J=2.7Hz,1H),8.72(s,1H), 8.56(dd,J=4.8,1.5Hz,1H),8.03(ddd,J=8.4,2.7,1.5Hz,1H),7.82(s,1H), 7.41 (dd,J=8.4,4.8Hz,1H),4.77(dt,J=4.2,1.2Hz,1H),3.80-3.94(m,2H),2.43-2.50(m,1H),2.36-2.41(m,2H),2.02-2.10(m,1H)

SYNTHETIC EXAMPLE 6

Preparation of 3-(methylthio)-1-{1-(pyridin-3-yl)-1H-pyrazol-4-yl}piperidin-2-one

(Compound No. 1-028 of the present invention)

400 mg of 3-bromo-1-(1-(pyridin-3-yl)-1H-pyrazol-4-yl)piperidin-2-one in 2 ml of dimethyl sulfoxide was mixed with 105 mg of sodium methylmercaptan and stirred at room temperature for 5 hours. After the reaction, the reaction mixture was mixed with 5 ml of water and extracted with 10 ml of ethyl acetate. The resulting organic layer was dried over anhydrous sodium sulfate, and the solvent was evaporated under reduced pressure. The precipitated solid was washed with diisopropyl ether to give 304 mg of the desired product as a pale yellow solid.
$^1$H NMR($CDCl_3$ ,$Me_4$ Si,300MHz)δ9.02(d,J=2.7Hz,1H),8.67(s,1H), 8.55(dd,J=4.8,1.5Hz,1H),8.04(ddd,J=8.1,2.7,1.5Hz, 1H),7.81 (s,1H), 7.40(dd,J=8.1,4.8Hz,1H),3.71-3.88(m,2H),3.53(t,J=5.1 Hz,1H),2.38(s,3H),2.20-2.32(m, 2H), 2.02-2.10(m,2H)

SYNTHETIC EXAMPLE 7

Preparation of tert-butyl 3-chloro-1-(pyridin-3-yl)-1H-pyrazol-4-ylcarbamate (Compound No.1-046 of the present invention)

Step 1

Preparation of ethyl 3-chloro-1 H-pyrazole-4-carboxylate

9.71 g of ethyl 1-tert-butyl-3-chloro-1H-pyrazole-4-carboxylate was mixed with 30 ml of concentrated hydrochloric acid under cooling with ice and stirred overnight at room temperature. After the stirring, the reaction mixture was mixed with 10 ml of concentrated hydrochloric acid and stirred for one day and night. After the reaction, the reaction mixture was cooled to 0°C, neutralized with aqueous sodium hydroxide and extracted with 100 ml of ethyl acetate. The resulting organic layer was dried over anhydrous sodium sulfate, and the solvent was evaporated under reduced pressure. The resulting solid was washed with n-hexane to give 6.28 g of the desired product as a white solid.
$^1$H NMR($CDCl_3$ ,$Me_4$ Si,300MHz)δ11.2(brs,1H),8.10(s,1H),4.33(q,J=7.2Hz,2H), 1.36(t,J=7.2Hz,3H)

Step 2

Preparation of ethyl 3-chloro-1-(pyridin-3-yl)-1H-pyrazole-4-carboxylate

To 10.2 g of ethyl 3-chloro-1 H-pyrazole-4-carboxylate in 150 ml of N,N-dimethylformamide, 15.6 g of 3-iodopyridine, 2.23 g of copper (I) iodide and 33.0 g of cesium carbonate were added successively. After the addition, the atmosphere in the reaction vessel was replaced by nitrogen gas, and the reaction mixture was stirred at 130°C for 5 hours. After the reaction, the reaction mixture was allowed to cool to room temperature and mixed with 100 ml of 1 N aqueous sodium hydroxide. The impurities in the reaction mixture were filtered off through Celite under reduced pressure, and the filtrate was extracted with 100 ml of ethyl acetate. The resulting organic layer was washed with saturated aqueous sodium chloride and then dried over anhydrous sodium sulfate, and the solvent was evaporated under reduced pressure. The resulting residue was purified by preparative medium pressure liquid chromatography using n-hexane - ethyl acetate (with a gradient of 1:1) as the eluent to give 7.56 g of the desired product as a white solid. m. p.; 90-93°C.

Step 3

Preparation of 3-chloro-1-(pyridin-3-yl)-1H-pyrazole-4-carboxylic acid

1.2 g of ethyl 3-chloro-1-(pyridin-3-yl)-1H-pyrazole-4-carboxylate was dissolved in 15 ml of ethanol, mixed with 0.6 g of potassium hydroxide and 10 ml of water successively and stirred at room temperature for 1 hour. After the reaction, the solvent was evaporated under reduced pressure. The resulting residue was acidified to pH 3 with 1 N aqueous hydrogen chloride, and the solvent was evaporated under reduced pressure to give 2.4 g of the desired product containing potassium chloride as a white solid. m. p.; 257-263°C.

Step 4

Preparation of tert-butyl 3-chloro-1-(pyridin-3-yl)-1H-pyrazol-4-ylcarbamate

2.40 g of 3-chloro-1-(pyridin-3-yl)-1H-pyrazole-4-carboxylic acid in 20 ml of 2-methyl-2-propanol was mixed with 2.45 g of triethylamine and 2.00 g of diphenylphosphoryl azide at room temperature successively and refluxed with 2-methyl-2-propanol for 4 hours with stirring. After the reaction, the reaction mixture was allowed to cool to room temperature, poured into ice-water and extracted with 50 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:1) as the eluent to give 0.86 g of the desired product as a white solid. m. p.;125-128°C.

SYNTHETIC EXAMPLE 8

Preparation of 1-{1-(pyridin-3-yl)-1H-pyrazol-4-yl}-5,6-dihydropyridin-2(1H)-one and 1-{1-(pyridin-3-yl)-1H-pyrazol-4-yl}-1,6-dihydropyridin-2(1H)-one (Compound No. 1-047 of the present invention)

0.30 g of 3-bromo-1-{1-(pyridin-3-yl)-1H-pyrazol-4-yl}piperidin-2-one in 4.0 ml of 1,4-dioxane was mixed with 0.19 g of 1,8-diazabicyclo[5.4.0]-7-undecene and stirred at 60°C for 2 hours. After the reaction, the reaction mixture was mixed with 10 ml of water and extracted with 20 ml of chloroform. The resulting organic layer was dried over anhydrous sodium sulfate, and the solvent was evaporated under reduced pressure. The resulting residue was purified by preparative medium pressure liquid chromatography using n-hexane - ethyl acetate (with a gradient of from 9:1 to 1:3) as the eluent to give 0.14 g of a 5:1 mixture of 1-{1-(pyridin-3-yl)-1H-pyrazol-4-yl}-5,6-dihydropyridin-2(1H)-one and 1-{1-(pyridin-3-yl)-1H-pyrazol-4-yl}-1,6-dihydropyridin-2(1 H)-one as the desired product in the form of a white solid.
$^1$H NMR($CDCl_3$ ,$Me_4$ Si,300MHz) of 1-{1-(pyridin-3-yl)-1H-pyrazol-4-yl}-5,6-dihydropyridin-2(1H)-one
δ9.02(d,J=2.4Hz,1H),8.61 (s, 1H),8.55(dd,J=4.8,1.2Hz,1H),8.05(ddd,J=8.1,2.4,1.2Hz, 1 H),7.81 (s,1H),7.41(dd,J=8.1, 4.8Hz,1H),6.69-6.74(m,1H),6.10(d,J=9.9Hz,1H), 3.92(t,J=6.9Hz,2H),2.57-2.63(m,2H)
$^1$H NMR($CDCl_3$ ,$Me_4$ Si,300MHz) of 1-{1-(pyridin-3-yl)-1H-pyrazol-4-yl}-1,6-dihydropyridin-2(1H)-one
δ9.02(d,J=2.4Hz,1H),8.72(s,H),8.55(dd,J=4.8,1.2Hz,1H),8.05(ddd,J=8.1,2.4,1.2Hz, 1 H),7.84(s,1H),7.41(dd,J=8.1, 4.8Hz,1H),6.69-6.74(m,1H),5.88-5.90(m,1H),4.37-4.40(m,1H),3.17-3.19(m,2H)

SYNTHETIC EXAMPLE 9

Preparation of 1-{1-(pyridin-3-yl)-1H-pyrazol-4-yl}piperidin-2-one (Compound No. 1-034 of the present invention)

To 108 mg of a mixture of 1-{1-(pyridin-3-yl)-1H-pyrazol-4-yl}-5,6-dihydropyridin-2(1 H)-one and 1-{1-(pyridin-3-yl)-1H-pyrazol-4-yl}-1,6-dihydropyridin-2(1H)-one in 2.5 ml of tetrahydrofuran, 12.3 mg of 5 wt% palladium-activated carbon was added. After the addition, the atmosphere in the reaction vessel was replaced by hydrogen gas, and the reaction mixture was stirred at room temperature for 2 days. After the reaction, the palladium-activated carbon in the reaction mixture was filtered off through Celite, and the filtrate was evaporated under reduced pressure to remove the solvent to give 92.5 mg of the desired product as a white solid.
$^1$H NMR($CDCl_3$,$Me_4$Si,300MHz)δ9.02(d, J=2.4Hz,1H),8.65(s, 1H),8.54(dd,J=4.8, 1.5Hz,1H),8.05(ddd,J=8.4,2.4,1.5Hz, 1H),7.82(s,1H),7.41(dd,J=8.4,4.8Hz,1H), 3.76(t,J=6.3Hz,2H),2.61 (t,J=6.3Hz,2H),2.06-1.98(m,2H),1.95-1.87(m,2H)

SYNTHETIC EXAMPLE 10

Preparation of N,2-dimethyl-3-(methylthio)-N-{1-(pyridin-3-yl)-1H-pyrazol-4-yl}propanamide (Compound No. 1-043 of the present invention)

170 mg of N-methyl-1-(pyridin-3-yl)-1H-pyrazole-4-amine dihydrochloride in 5 ml of dichloromethane was mixed with 348 mg of triethylamine, 206 mg of 2-methyl-3-(methylthio)propanoic acid, 265 mg of 1-ethyl-3-(3-dimethylaminopropyl)carbodiimide hydrochloride and 10 mg of 4-dimethylaminopyridine successively and stirred at room temperature for 12 hours. After the reaction, the reaction mixture was mixed with 5 ml of water and extracted with 5 ml of dichloromethane. The resulting organic layer was washed with saturated aqueous sodium chloride and dried over anhydrous sodium sulfate, and the solvent was evaporated under reduced pressure. The resulting residue was purified by preparative medium pressure liquid chromatography using n-hexane - ethyl acetate (1:1) as the eluent to give 173 mg of the desired product (a 3:1 mixture of two isomers having the same structure) as a yellow oil.
(The compound contained in a ratio of 3)$^1$H

NMR($CDCl_3$ ,$Me_4$ Si,300MHz)δ8.97(d,J=2.4Hz,1H),8.59(d,J=4.8Hz,1H),8.08-7.99(m,1 H),8.01 (s,1H),7.72(s,1H), 7.44(dd,J=4.8,8.1Hz,1H),3.29(s,3H),3.06-2.82(m,2H),2.48-2.40(m,1H),2.01(s,3H),1.12(d,J=6.3Hz,3H).
(The compound contained in a ratio of 1)$^1$H NMR($CDCl_3$,$Me_4$Si,300MHz)δ9.02-8.96(m,1H),8.66(s,1H),8.57-8.51 (m, 1H),8.08-7.99(m,1H),7.78(s,1 H),7.42-7.35(m,1H),3.51 (s,3H),3.19-2.82(m,2H),2.60(dd,J=6.0,13.2Hz,1H),2.14(s,3H), 1.30(d,J=6.9Hz,3H).

SYNTHETIC EXAMPLE 11

Preparation of N-methyl-N-{3-chloro-1-(pyridin-3-yl)-1H-pyrazol-4-yl}ethenesulfonamide (Compound No. 2-003 of the present invention)

To 200 mg of 3-chloro-N-methyl-1-(pyridin-3-yl)-1 H-pyrazole-4-amine dihydrochloride in 3 ml of ethylene dichloride, 281 mg of pyridine and 139 mg of 2-chloroethanesulfonyl chloride in 1 ml of ethylene dichloride were added dropwise at 0°C, and the reaction mixture was stirred at room temperature for 16 hours. After the reaction, the reaction mixture was mixed with 5 ml of water and extracted with 10 ml of ethyl acetate. The resulting organic layer was washed with saturated aqueous sodium chloride and dried over anhydrous sodium sulfate, and the solvent was evaporated under reduced pressure to give 150m g of the desired product as a yellow solid. $^1$H NMR($CDCl_3$ ,$Me_4$ Si,300MHz)δ8.94(d, J=2.7Hz,1H),8.58(dd,J=4.5Hz,1.5Hz,1H), 8.15(s,1H),7.99(ddd,J=8.4Hz,4.5Hz,1.5Hz,1H),7.42(dd,J=8.4Hz,4.5Hz,1H), 6.63(dd,J=1 6.5Hz,9.9Hz,1 H),6.28(d,J=16.5Hz,1H),6.09(d,J=9.9Hz,1H),3.28(s,3H)

SYNTHETIC EXAMPLE 12

Preparation of N-methyl-N-{3-chloro-1-(pyridin-3-yl)-1H-pyrazol-4-yl}-2-methylthioethanesulfonamide (Compound No. 2-002 of the present invention)

150 mg of N-methyl-N-{3-chloro-1-(pyridin-3-yl)-1H-pyrazol-4-yl}ethenesulfonamide was dissolved in 2 ml of 4 M aqueous potassium hydroxide and 1.5 ml of tetrahydrofuran, mixed with 220 mg of S,S'-dimethyl dithiocarbonate and stirred at room temperature for 8 hours. After the reaction, the reaction mixture was mixed with 5 ml of water and extracted with 10 ml of ethyl acetate. The resulting organic layer was washed with saturated aqueous sodium chloride and dried over anhydrous sodium sulfate, and the solvent was evaporated under reduced pressure. The resulting residue was purified by preparative medium pressure liquid chromatography using n-hexane - ethyl acetate (with a gradient of from 1:1 to 0: 1) as the eluent to give 120 mg of the desired product as a white solid.
$^1$H NMR($CDCl_3$,$Me_4$Si,300MHz)δ8.94(d,J=2.7Hz,1H),8.60-8.55(m,1H),8.15(s,1H),8.05-7.95(m,1H),7.50-7.35(m,1H), 3.36(s,3H),3.40-3.30(m,2H),3.00-2.85(m,2H),2.16(s,3H) m. p.;101-103°C

SYNTHETIC EXAMPLE 13

Preparation of N-{3-chloro-1-(pyridin-3-yl)-1H-pyrazol-4-yl}-N-methyl-4,5-dihydrothiazole-2-amine (Compound No. 3-001 of the present invention)

160 mg of 3-chloro-N-methyl-1-(pyridin-3-yl)-1 H-pyrazole-4-amine was dissolved in 5 ml of acetonitrile, mixed with 159 mg of potassium carbonate and 140 mg of 2-chloroethyl isothiocyanate successively and refluxed with acetonitrile for 2 hours with stirring. After the reaction, the reaction mixture was mixed with 5 ml of water and extracted with 10 ml of ethyl acetate. The resulting organic layer was washed with saturated aqueous sodium chloride and dried over anhydrous sodium sulfate. The resulting residue was purified by preparative medium pressure liquid chromatography using ethyl acetate - methanol (with a gradient of from 1:0 to 4:1) to give 140 mg of the desired product as a white solid.
$^1$H NMR($CDCl_3$ ,$Me_4$ Si,300MHz)δ8.92(d,J=2.4Hz,H),8.57(d,J=2.4Hz,1H),8.05(s,1H), 8.05-7.95(m,1H),7.45-7.35(m, 1H),4.13(t,J=7.2Hz,2H),3.36(s,3H),3.35(t,J=7.2Hz,2H)

SYNTHETIC EXAMPLE 14

Preparation of methyl N-3-methyl-1-(pyridin-3-yl)-1H-pyrazol-4-yl acetimidate (Compound No. 3-002 of the present invention)

1.5 g of 1-(pyridin-3-yl)-1H-pyrazole-4-amine dihydrochloride in 10 ml of trimethyl orthoformate was mixed with 0.01 g of p-toluenesulfonic acid and stirred at 120°C for 2 hours. After the reaction, the solvent was evaporated under reduced pressure, and the resulting residue was purified by preparative medium pressure liquid chromatography using ethyl acetate - methanol (with a gradient of from 1:0 to 4:1) as the eluent to obtain 180 mg of the desired product as a

white solid.
m.p.; 90-92°C

SYNTHETIC EXAMPLE 15

Preparation of N-methyl-N-[1-{3-methyl-1-(pyridin-3-yl)-1H-pyrazol-4-ylimino}ethyl]-2-(methylthio)acetamide (Compound No. 3-004 of the present invention)

Step 1

Preparation of N-methyl-N'-{3-methyl-1-(pyridin-3-yl)-1H-pyrazol-4-yl) acetimidate

160 mg of methyl N-3-methyl-1-(pyridin-3-yl)-1H-pyrazol-4-yl acetimidate was mixed with 4 ml of 9.8 mol/L methylamine in methanol, and the mixture was stirred in a sealed tube at 100°C for 2 hours. After the reaction, the solvent was evaporated under reduced pressure to obtain 160 mg of the desired product as a brown oil. The oil was used in the next step without purification.

Step 2

Preparation of N-methyl-N-[1-{3-methyl-1-(pyridin-3-yl)-1H-pyrazol-4-ylimino}ethyl]-2-(methylthio)acetamide

160 mg of N-methyl-N'-{3-methyl-1-(pyridin-3-yl)-1H-pyrazol-4-yl) acetimidate in 6ml of dichloromethane 6 ml was mixed with 104 mg of triethylamine, 148 mg of 3-(methylthio)acetic acid, 200 mg of 1-ethyl-3-(3-dimethylaminopropyl)carbodiimide hydrochloride and 10 mg of 4-dimethylaminopyridine successively, and the reaction mixture was stirred at room temperature for 20 hours. After the reaction, the reaction mixture was mixed with 10 ml of water and extracted with 10 ml of ethyl acetate. The resulting organic layer was dried over anhydrous sodium sulfate, and the solvent was evaporated under reduced pressure. The resulting residue was purified by preparative medium pressure liquid chromatography using ethyl acetate - methanol (with a gradient of from 1:0 to 4:1) as the eluent to obtain 98 mg of the desired product as light brown crystals.
m. p.; 54-57°C

SYNTHETIC EXAMPLE 16

Preparation of 4-chloro-N-{3-chloro-1-(pyridin-3-yl)-1H-pyrazol-4-yl}butanamide (Compound No. 1-131 of the present invention)

To 1.5 g of 3-chloro-1-(pyridin-3-yl)-1H-pyrazole-4-amine dihydrochloride in 25 ml of dichloromethane, 2.0 g of triethylamine and 0.95 g of 4-chlorobutanoyl chloride were added at 0°C dropwise successively, and the resulting reaction mixture was stirred at room temperature for 1 hour. After the reaction, the reaction mixture was mixed with 25 ml of water and extracted with 50 ml of chloroform. The resulting organic layer was washed with saturated aqueous sodium chloride and then dried over anhydrous sodium sulfate. The solvent was evaporated under reduced pressure, and the resulting residue was purified by preparative medium pressure liquid chromatography using n-hexane - ethyl acetate (with a gradient of from 4:1 to 1:3) as the eluent to obtain 1.47 g of the desired product as a white solid.
m. p.; 97-98°C

SYNTHETIC EXAMPLE 17

Preparation of 1-{3-chloro-1-(pyridin-3-yl)-1H-pyrazol-4-yl}pyrrolidin-2-one (Compound No. 1-132 of the present invention)

To 1.45 g of 4-chloro-N-{3-chloro-1-(pyridin-3-yl)-1H-pyrazol-4-yl}butanamide in 20 ml of tetrahydrofuran, 0.22 g of 60 wt% sodium hydride (dispersed in mineral oil) was added at 0°C. After the addition, the reaction mixture was stirred at room temperature for 2 hours. After the reaction, the reaction mixture was mixed with 20 ml of water and extracted with 20 ml of ethyl acetate. The resulting organic layer was washed with saturated aqueous sodium chloride and then dried over anhydrous sodium sulfate, and the solvent was evaporated under reduced pressure. The resulting solid was washed with diisopropyl ether to obtain 1.14 g of the desired product as a yellow solid.
m. p.;109-110°C

SYNTHETIC EXAMPLE 18

Preparation of 1-{3-chloro-1-(pyridin-3-yl)-1H-pyrazol-4-yl}-3-methylenepyrrolidin-2-one (Compound No. 1-133 of the present invention)

500 mg of 1-{3-chloro-1-(pyridin-3-yl)-1H-pyrazol-4-yl}pyrrolidin-2-one in 5 ml of dichloromethane was mixed with 290 mg of triethylamine and 550 mg of trimethylsilyl trifluoromethansuflonate at 0°C, and the reaction mixture was stirred 45 minutes. After the stirring, the reaction mixture was mixed with 460 mg of dimethylmethyleneammonium iodide and stirred at room temperature overnight. After the stirring, the reaction mixture was stirred with 10 ml of dichloromethane and 11 ml of 1 N aqueous hydrochloric acid for 10 minutes, then adjusted to pH 14 with 2 N aqueous sodium hydroxide and extracted with 15 ml of dichloromethane. The resulting organic layer was washed with saturated aqueous sodium chloride and then dried over anhydrous sodium sulfate, and the solvent was evaporated under reduced pressure. The resulting residue was purified by preparative medium pressure liquid chromatography using n-hexane - ethyl acetate (with a gradient of from 1:1 to 0:1) as the eluent to obtain 230 mg of the desired product as a white solid.
m. p.; 117-118°C

SYNTHETIC EXAMPLE 19

Preparation of 1-{3-chloro-1-(pyridin-3-yl)-1H-pyrazol-4-yl}-3-(methylthiomethyl)pyrrolidin-2-one (Compound No. 1-141 of the present invention)

187 mg of 1-{3-chloro-1-(pyridin-3-yl)-1H-pyrazol-4-yl}-3-methylenepyrrolidin-2-one in 3 ml of tetrahydrofuran was mixed with 1 ml of 30% aqueous potassium hydroxide and then with 58 mg of S,S'-dimethyl dithiocarbonate and refluxed with tetrahydrofuran for 3 hours with stirring. After the reaction, the reaction mixture was mixed with 10 ml of water and extracted with 20 ml of chloroform. The resulting organic layer was washed with saturated aqueous sodium chloride and then dried over anhydrous sodium sulfate, and the solvent was evaporated under reduced pressure. The resulting residue was purified by preparative medium pressure liquid chromatography using n-hexane - ethyl acetate (with a gradient of from 3:1 to 0:1) to obtain 214 mg of the desired product as a white solid.
m. p.; 83-84°C

SYNTHETIC EXAMPLE 20

Preparation of N-{3-chloro-1-(pyridin-3-yl)-1H-pyrazol-4-yl}-N-ethyl-2-(methoxyimino)-3-(methylthio)propanamide (Compound No. 1-143 of the present invention)

Step 1

Preparation of ethyl 2-(methoxyimino)-3-(methylthio)propanoate (Compound No. D-001)

2.0 g of ethyl 3-bromo-2-(methoxyimino)propanoate in 30 ml of N,N-dimethylformamide was mixed with 750 mg of sodium methylmercaptan and stirred at room temperature for 2 hours. After the stirring, the reaction mixture was heated to 80°C and stirred at the same temperature for another one hour. After the reaction, the reaction mixture was mixed with 30 ml of water and extracted with 50 ml of ethyl acetate. The resulting organic layer was washed with saturated aqueous sodium hydrogen carbonate and then with saturated aqueous sodium chloride and dried over anhydrous sodium sulfate. The solvent was evaporated under reduced pressure to obtain 2 g of the desired product as a pale yellow oil.
$^1$H NMR($CDCl_3$,$Me_4Si$,300MHz)δ4.34(q,J=7.2Hz,2H),4.04(s,3H),3.55(s,2H), 2.10(s,3H),1.35(t,J=7.2Hz,3H)

Step 2

Preparation of 2-(methoxyimino)-3-(methylthio)propanoic acid (Compound No. E-001)

1.84 g of ethyl 2-(methoxyimino)-3-(methylthio)propanoate in a mixture of 10 ml of water and 10 ml of ethanol was mixed with 430 mg of sodium hydroxide and stirred at room temperature for 2 days. After the reaction, the reaction solution was adjusted to pH 2 with 1 N aqueous hydrochloric acid and extracted with 20 ml of ethyl acetate twice. The resulting organic layer was washed with saturated aqueous sodium chloride and dried over anhydrous sodium sulfate, and the solvent was evaporated under reduced pressure. The resulting solid was washed with 6 ml of a mixture of hexane : isopropyl ether (5:1) to obtain 0.4 g of the desired product as a pale yellow solid.
$^1$H NMR($CDCl_3$,$Me_4Si$,300MHz)δ4.08(s,3H),3.53(s,2H),2.14(s,3H)

Step 3

Preparation of N-{3-chloro-1-(pyridin-3-yl)-1H-pyrazol-4-yl}-N-ethyl-2-(methoxyimino)-3-(methylthio)propanamide

415 mg of 2-(methoxyimino)-3-(methylthio)propanoic acid in 3 ml of dichloromethane was mixed with 429 mg of oxalyl chloride and 10 mg of N,N-dimethylformamide successively and stirred at room temperature for 1 hour. After the stirring, the reaction solution was added dropwise to a separately prepared solution of 500 mg of 3-chloro-N-ethyl-1-(pyridin-3-yl)-1H-pyrazole-4-amine dihydrochloride and 668 mg of pyridine in 3 ml of ethylene dichloride, and the resulting reaction mixture was stirred at room temperature for 30 minutes. After the reaction, the reaction mixture was washed with 10 ml of water and 10 ml of saturated aqueous sodium hydrogen carbonate and dried over anhydrous sodium sulfate, and the solvent was evaporated under reduced pressure. The resulting residue was purified by preparative medium pressure liquid chromatography using n-hexane - ethyl acetate (1:4) as the eluent to obtain 534 mg of the desired product as a pale yellow oil.
$^1$H NMR($CDCl_3$ ,$Me_4$ Si,300MHz)δ8.90(d,J=2.4Hz,1H),8.58(dd,J=4.8,1.2Hz,1H),8.04-7.95(m,1 H),7.98(s,1H),7.43(dd, J=8.1,4.8Hz,1H),3.78(q,J=7.2Hz,2H),3.68(s,3H),3.52(s,2 H),1.90(s,3H),1.22(t,J=7.2Hz,3H)

SYNTHETIC EXAMPLE 21

Preparation of tert-butyl 3-chloro-1-(pyridin-3-yl)-1H-pyrazol-4-yl(methyl)carbamate (Compound No. 1-037 of the present invention)

To 0.34 g of 60 wt% sodium hydride (dispersed in mineral oil) in 25 ml of N,N-dimethylformamide, 2.2 g of tert-butyl3-chloro-1-(pyridin-3-yl)-1H-pyrazol-4-ylcarbamate was added under cooling with ice. After the addition, the reaction mixture was warmed to room temperature and stirred at the same temperature for 30 minutes. After the stirring, the reaction mixture was mixed with 1.17 g of methyl iodide and stirred at room temperature for another 30 minutes. After the reaction, the reaction mixture was poured into ice-water and extracted with 100 ml of ethyl acetate. The resulting organic layer was dried over anhydrous sodium sulfate , and the solvent was evaporated under reduced pressure. The resulting residue was purified by preparative medium pressure liquid chromatography using n-hexane - ethyl acetate (with a gradient of from 9:1 to 1:1) as the eluent to obtain 2.01 g of the desired product as a yellow oil.
$^1$ H NMR($CDCl_3$,$Me_4$Si,300MHz)δ8.90(d,J=2.4Hz,1H),8.56(dd,J=4.8,1.5Hz,1H), 8.00(ddd,J=8.1,2.4,1.5Hz,1H),7.90(s, 1H),7.40(dd,J=8.1,4.8Hz,1H),3.22(s,3H), 1.46(s,9H)

SYNTHETIC EXAMPLE 22

Preparation of N-{3-chloro-1-(pyridin-3-yl)-1H-pyrazol-4-yl}-N-methyl-3-(methylthio)propanamide (Compound No. 1-038 of the present invention)

Step 1

Preparation of 3-chloro-N-methyl-1-(pyridin-3-yl)-1 H-pyrazole-4-amine dihydrochloride (Compound No.A-003)

1.93 g of tert-butyl 3-chloro-1-(pyridin-3-yl)-1H-pyrazol-4-yl(methyl)carbamate in 25 ml of 1,4-dioxane was mixed with 10 ml of about 4 M hydrogen chloride in 1,4-dioxane and stirred at room temperature for 2 days. After the reaction, the solid precipitated in the reaction solution was collected by filtration under reduced pressure and dried under reduced pressure to obtain1.63 g of the desired product as a white solid.
m. p.; 159-160°C

Step 2

Preparation of N-{3-chloro-1-(pyridin-3-yl)-1H-pyrazol-4-yl}-N-methyl-3-(methylthio)propanamide

800 mg of 3-chloro-N-methyl-1-(pyridin-3-yl)-1 H-pyrazole-4-amine dihydrochloride in 10 ml of dichloromethane was mixed with 720 mg of triethylamine, 685 mg of 3-(methylthio)propanoic acid, 1.63 of g 1-ethyl-3-(3-dimethylaminopropyl)carbodiimide hydrochloride and 695 mg of 4-dimethylaminopyridine successively and stirred at room temperature for 12 hours. After the reaction, the reaction mixture was mixed with 20 ml of water and extracted with 30 ml of chloroform. The resulting organic layer was dried over anhydrous sodium sulfate, and the solvent was evaporated under reduced pressure. The resulting residue was purified by preparative medium pressure liquid chromatography using n-hexane - ethyl acetate (with a gradient of from 3:1 to 0:1) as the eluent to obtain 463 mg of the desired product as a colorless oil.

$^1H$ NMR($CDCl_3$ ,$Me_4$ Si,300MHz)δ8.94(d,J=2.7Hz,1H),8.63(dd,J=4.5,1.2Hz,1H), 8.04(ddd,J=8.1,2.7,1.2Hz,1 H), 8.00(s, 1H),7.46(dd,J=8.1,4.5Hz,1H),3.25(s,3H), 2.80(t,J=7.2Hz,2H),2.48(t,J=7.2Hz,2H),2.07(s,3H)

SYNTHETIC EXAMPLE 23

Preparation of N-{3-chloro-1-(pyridin-3-yl)-1H-pyrazol-4-yl}-N-methyl-3-(methylsulfinyl)propanamide (Compound No. 1-051 of the present invention)

153 mg of N-{3-chloro-1-(pyridin-3-yl)-1H-pyrazol-4-yl}-N-methyl-3-(methylthio)propanamide in 3 ml of acetic acid wax mixed with 56 mg of 30 wt% aqueous hydrogen peroxide and stirred at room temperature for 12 hours. After the reaction, the reaction mixture was mixed with 1 ml of saturated aqueous sodium hydrogensulfite, and the solvent was evaporated under reduced pressure. The resulting residue was purified by preparative medium pressure liquid chromatography using ethyl acetate - methanol (5:1) as the eluent to obtain 145 mg of the desired product as a colorless oil.
$^1H$ NMR($CDCl_3$,$Me_4$ Si,300MHz)δ8.96(d,J=2.4Hz,1H),8.63(dd,J=4.8,1.2Hz,1H), 8.07(s,1H),8.03(ddd,J=8.4,2.4,1.2Hz, 1H),7.46(dd,J=8.4,4.8Hz,1H),3.27(s,3H),3.11-3.21 (m,1H),2.84-2.93(m,1H),2.66-2.76(m,2H),2.60(s,3H)

SYNTHETIC EXAMPLE 24

Preparation of N-{3-chloro-1-(pyridin-3-yl)-1H-pyrazol-4-yl}-N-methyl-3-(methylsulfonyl)propanamide (Compound No. 1-052 of the present invention)

158 mg of N-{3-chloro-1-(pyridin-3-yl)-1H-pyrazol-4-yl}-N-methyl-3-(methylthio)propanamide in 3 ml of acetic acid was mixed with 56 mg of 30 wt% aqueous hydrogen peroxide and 8 mg of sodium tungstate dihydride and stirred at room temperature for 12 hours. After the reaction, the reaction mixture was mixed with 1 ml of aqueous saturated sodium hydrogensulfite, and the solvent was evaporated under reduced pressure. The resulting residue was purified by preparative medium pressure liquid chromatography using n-hexane - ethyl acetate (with a gradient of from 1:1 to 0:1) as the eluent to obtain 160 mg of the desired product as a white solid.
m. p.; 90-91°C

SYNTHETIC EXAMPLE 25

Preparation of N-{3-chloro-1-(pyridin-3-yl)-1H-pyrazol-4-yl}-N,2,2-trimethyl-3-(methylthio)propanamide (Compound No. 1-134 of the present invention)

Step 1

Preparation of 3-chloro-N-methyl-1-(pyridin-3-yl)-1 H-pyrazole-4-amine (Compound No. B-003)

17.3 g of 3-chloro-N-methyl-1-(pyridin-3-yl)-1 H-pyrazole-4-amine dihydrochloride in 200 ml of chloroform and 100 ml of water was mixed with 25.3 g of potassium carbonate and stirred at room temperature for 15 minutes. After the reaction, the reaction solution was separated, and the resulting organic layer was washed with saturated aqueous sodium chloride and then dried over anhydrous sodium sulfate. The solvent was evaporated under reduced pressure, and the resulting solid was washed with diisopropyl ether to obtain 11.1 g of the desired product as a pale yellow solid.
$^1H$ NMR($CDCl_3$,$Me_4$Si,300MHz)δ8.87(d,J=2.7Hz,1H),8.46(dd,J=4.5Hz,1.8Hz,1H), 7.94(ddd,J=8.1 Hz,2.7Hz,1.8Hz, 1H),7.34(dd,J=8.1Hz,4.5Hz,1H),7.32(s,1H), 3.11(brs,1H),2.85(s,3H)
m. p. 105-106°C

Step 2

Preparation of N-{3-chloro-1-(pyridin-3-yl)-1H-pyrazol-4-yl}-N,2,2-trimethyl-3-(methylthio)propanamide

500 mg of 3-chloro-N-methyl-1-(pyridin-3-yl)-1 H-pyrazole-4-amine in 8 ml of dichloromethane was mixed with 484 mg of triethylamine and cooled to 0°C. After the cooling, 622 mg of 2,2-dimethyl-3-(methylthio)propanoyl chloride was added dropwise to the reaction solution, and the reaction solution was stirred at room temperature for 12 hours. After the reaction, the reaction mixture was mixed with 5 ml of water and extracted with 10 ml of chloroform. The resulting organic layer was dried over anhydrous sodium sulfate , and the solvent was evaporated under reduced pressure. The resulting residue was purified by preparative medium pressure liquid chromatography using n-hexane - ethyl acetate (with a gradient of from 1:1 to 1:4) as the eluent to obtain 180 mg of the desired product as a colorless oil. $^1H$ NMR($CDCl_3$ ,

$Me_4$ Si,300MHz)δ8.95(d,J=1.5Hz,1H),8.60(d,J=4.8Hz,1H),8.14(s,1H), 8.04(d,J=8.4Hz,1H),7.45(dd,J=8.4Hz,4.8Hz, 1H),3.21 (s,3H),2.72(s,2H),2.14(s,3H), 1.21(s,6H)

SYNTHETIC EXAMPLE 26

Preparation of tert-butyl 3-chloro-1-(pyridin-3-yl)-1H-pyrazol-4-yl(ethyl)carbamate (Compound No. 1-056 of the present invention)

To 0.15 g of 60 wt% sodium hydride (dispersed in mineral oil) in 10 ml of N,N-dimethylformamide, 0.95g of tert-butyl 3-chloro-1-(pyridin-3-yl)-1H-pyrazol-4-ylcarbamate was added under cooling with ice. After the addition, the reaction mixture was warmed to room temperature and stirred at the same temperature for 30 minutes. After the stirring, the reaction mixture was mixed with 0.55 g of ethyl iodide and stirred at room temperature for another 1 hour. After the reaction, the reaction mixture was poured into ice-water and extracted with 100 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (2:1) as the eluent to obtain 1.06 g of the desired product as a yellow solid.
m. p.; 55-58°C

SYNTHETIC EXAMPLE 27

Preparation of N-{3-chloro-1-(pyridin-3-yl)-1H-pyrazol-4-yl)-N-ethylacrylamide (Compound No. 1-152 of the present invention)

Step 1

Preparation of 3-chloro-N-ethyl-1-(pyridin-3-yl)-1H-pyrazole-4-amine dihydrochloride (Compound No. A-004)

1.0 g of tert-butyl 3-chloro-1-(pyridin-3-yl)-1H-pyrazol-4-yl(ethyl)carbamate in 20 ml of 1,4-dioxane was mixed with 8.1 ml of about 4 M hydrogen chloride in 1,4-dioxane and stirred at room temperature for 12 hours. After the reaction, the solid precipitated in the reaction solution was collected by filtration under reduced pressure and dried under reduced pressure to obtain 0.85 g of the desired product as a white solid.
m. p.;132-133°C

Step 2

Preparation of 3-chloro-N-ethyl-1-(pyridin-3-yl)-1H-pyrazole-4-amine (Compound No. B-004)

7.4 ml of 1 N aqueous sodium hydroxide was mixed with 1 g of 3-chloro-N-ethyl-1-(pyridin-3-yl)-1 H-pyrazole-4-amine dihydrochloride and stirred at room temperature for 3 hours. After the reaction, the reaction mixture was extracted with dichloromethane (20 ml × 3). The resulting organic layer was dried over anhydrous sodium sulfate, and the solvent was evaporated under reduced pressure to obtain 660 mg of the desired product as a white solid.
$^1H$ NMR($CDCl_3$,$Me_4Si$,300MHz)δ8.86(d,J=2.7Hz,1H),8.46(d,J=4.8,1.2Hz,1H),8.00-7.90(m,1 H),7.45(ddd,J=8.1,4.8, 1.2Hz,1H),7.33(s,1H),3.01(q,J=7.2Hz,2H),3.00(brs,1H), 1.31(t,J=7.2Hz,3H)
m. p.; 89-90°C

Step 3

Preparation of N-{3-chloro-1-(pyridin-3-yl)-1H-pyrazol-4-yl}-N-ethylacrylamide

380 mg of 3-chloro-N-ethyl-1-(pyridin-3-yl)-1H-pyrazole-4-amine in 8.5 ml of dichloromethane was mixed with 8.5 ml of saturated aqueous sodium hydrogencarbonate and then with 230 mg of acryloyl chloride and stirred at room temperature for 20 minutes. After the reaction, the reaction mixture was extracted with dichloromethane (20 ml × 2). The resulting organic layer was dried over anhydrous sodium sulfate, and the solvent was evaporated under reduced pressure. The resulting residue was purified by preparative medium pressure liquid chromatography using n-hexane - ethyl acetate (with a gradient of from 9:1 to 1:1) to obtain 320 mg of the desired product as a white solid.
$^1H$ NMR($CDCl_3$,$Me_4Si$,300MHz)δ8.94(d,J=2.7Hz,1H),8.61(dd,J=4.8,1.5Hz,1H),8.00-8.10(m,1 H),7.95(s,1H),7.45(dd, J=8.6,4.8Hz,1H),6.43(dd,J=16.8,1.8Hz,1H), 6.17(dd,16.8,10.4Hz,1H),5.63(dd,10.4,1.8Hz,1H),3.77(q,7.1Hz, 2H).1.20(t,7.1 Hz,3H)

m. p.; 67-68°C

SYNTHETIC EXAMPLE 28

Preparation of N-{3-chloro-1-(pyridin-3-yl)-1H-pyrazol-4-yl}-N-{3-(methylthio)propyl}acetamide (Compound No. 1-153 of the present invention)

Step 1

Preparation of 3-chloro-N-{3-(methylthio)propyl}-1-(pyridin-3-yl)-1H-pyrazole-4-amine (Compound No. C-001)

500 mg of N-{3-chloro-1-(pyridin-3-yl)-1H-pyrazol-4-yl}-3-(methylthio)propanamide in 15 ml of tetrahydrofuran was mixed with 3.3 ml of 0.98M borane-tetrahydrofuran complex in tetrahydrofuran at room temperature and refluxed with tetrahydrofuran for 2 hours with stirring. After the reaction, the reaction solution was cooled to 0°C, mixed with 15 ml of water and then with 10 ml of saturated aqueous sodium hydrogencarbonate and extracted with 150 ml of ethyl acetate. The resulting organic layer was dried over anhydrous sodium sulfate. The resulting residue was purified by silica gel column chromatography using n-hexane - ethyl acetate (1:1) as the eluent to obtain 256 mg of the desired product as a yellow solid.
$^1$H NMR($CDCl_3$ ,$Me_4$ Si,300MHz)δ8.82(d, J=1.8Hz, 1 H), 8.42(d, J=5.4Hz, 1 H), 8.21 (dd, J=8.4, 1.8Hz, 1 H), 7.54(dd, J=8.4, 5.4Hz, 1 H), 7.34(s, 1H), 3.23(brs, 1 H), 3.21 (t, J=6.9Hz, 2H), 2.65(t, J=6.9Hz, 2H), 2.15(s, 3H), 1.96(tt, J=6.9, 6.9Hz, 2H)
m. p.; 80-81 °C

Step 2

Preparation of N-{3-chloro-1-(pyridin-3-yl)-1H-pyrazol-4-yl}-N-{3-(methylthio)propyl}acetamide

150 mg of 3-chloro-N-{3-(methylthio)propyl}-1-(pyridin-3-yl)-1H-pyrazole-4-amine in 2 ml of dichloromethane was mixed with 1 ml of pyridine and cooled to 0°C. Then, to the reaction solution, 163 mg of acetic anhydride was added dropwise, and the reaction solution was stirred at room temperature for 12 hours. After the reaction, the reaction mixture was mixed with 30 ml of water and extracted with 150 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:4) as the eluent to obtain 145 mg of the desired product as a colorless oil.
$^1$H NMR($CDCl_3$ ,$Me_4$ Si,300MHz)δ8.95(d, J=2.7Hz, 1 H), 8.63(dd, J=4.8, 1.5Hz, 1 H), 8.05(ddd, J=8.4, 2.7, 1.5Hz, 1 H), 7.97(s, 1 H), 7.46(dd, J=8.4, 4.8Hz, 1 H), 3.73(t, J=7.5Hz, 2H), 2.54(t, J=7.2Hz, 2H), 2.10(s, 3H), 1.97(s, 3H), 1.95-1.75(m, 2H)

SYNTHETIC EXAMPLE 29

Preparation of N-{3-chloro-1-(pyridin-3-yl)-1H-pyrazol-4-yl}-N-ethyl-2-methoxy-3-(methylthio)propanamide (Compound No. 1-160 of the present invention)

Step 1

Preparation of methyl 2-methoxy-3-(methylthio)propanoate

2.3 g of methyl 2-methoxyacrylate in 10 ml of acetonitrile was mixed with 1.39 g of sodium methylmercaptan and stirred at room temperature for 1 hour. After the reaction, the reaction mixture was mixed with 10 ml of water and extracted with 10 ml of ethyl acetate. The resulting organic layer was dried over anhydrous sodium sulfate, and the solvent was evaporated under reduced pressure to obtain 1.92 g of the desired product as a pale yellow oil.
$^1$ H NMR($CDCl_3$,$Me_4$Si,300MHz)δ3.98(dd,J=6.6,5.4Hz,1H),3.79(s,3H),3.45(s,3H),3.95-3.75(m,2H),2.19(s,3H)

Step 2

Preparation of 2-methoxy-3-(methylthio)propanoic acid

1.92 g of methyl 2-methoxy-3-(methylthio)propanoate in 5 ml of methanol was mixed with 0.94 g of sodium

hydroxide and 5 ml of water and stirred at room temperature for 1 hour. After the reaction, the reaction mixture was mixed with 10 ml of water, and the aqueous layer was washed with 10 ml of ethyl acetate, then adjusted to pH 2 with 1 N aqueous hydrogen chloride and extracted with 10 ml of ethyl acetate twice. The resulting organic layer was washed with saturated aqueous sodium chloride and dried over anhydrous sodium sulfate, and the solvent was evaporated under reduced pressure to obtain 270 mg of the desired product as a pale yellow oil.
$^1$ H NMR($CDCl_3$,$Me_4Si$,300MHz)$\delta$3.98(dd,J=6.6,4.2Hz,1H),3.52(s,3H),3.00-2.80(m,2H),2.21 (s,3H)

Step 3

Preparation of N-{3-chloro-1-(pyridin-3-yl)-1H-pyrazol-4-yl}-N-ethyl-2-methoxy-3-(methylthio)propanamide

420 mg of 3-chloro-N-methyl-1-(pyridin-3-yl)-1 H-pyrazole-4-amine dihydrochloride in 2 ml of dichloromethane was mixed with 725 mg of pyridine, 240 mg of 2-methoxy-3-(methylthio)propanoic acid, 555 mg of 1-ethyl-3-(3-dimethylaminopropyl)carbodiimide hydrochloride and 10 mg of 4-dimethylaminopyridine successively and stirred at room temperature for 1 hour. After the reaction, the reaction mixture was mixed with 10 ml of water and extracted with 10 ml of chloroform. The resulting organic layer was dried over anhydrous sodium sulfate, and the solvent was evaporated under reduced pressure. The resulting residue was purified by preparative medium pressure liquid chromatography using n-hexane - ethyl acetate (with a gradient of from 3:1 to 3:7) as the eluent to obtain 245 mg of the desired product as a colorless oil.

SYNTHETIC EXAMPLE 30

Preparation of tert-butyl 3-bromo-1-(pyridin-3-yl)-1H-pyrazol-4-ylcarbamate (Compound No. 1-188 of the present invention)

Step 1

Preparation of ethyl 3-amino-1-(pyridin-3-yl)-1H-pyrazole-4-carboxylate

To 20 g of ethyl 3-amino-1H-pyrazole-4-carboxylate in 200 ml of N,N-dimethylformamide, 29.1 g of 3-iodopyridine, 7.4 g of copper (I) iodide and 72.7 g of cesium carbonate were added successively. After the addition, the atmosphere in the reaction vessel was replaced by nitrogen gas, and the reaction mixture was stirred at 100°C for 10 hours. After the reaction, the reaction mixture was mixed with 800 ml of water and 150 g of sodium chloride, and the precipitated solid was filtered off. The filtrate was extracted with ethyl acetate (800 ml $\times$ 3) and then with 500 ml of n-butanol. The resulting organic layer was dried over anhydrous sodium sulfate, and the solvent was evaporated under reduced pressure. 200 ml of ethyl acetate was added to the residue, and the precipitated solid was filtered off. The filtrate was evaporated to remove the solvent. 15 ml of chloroform was added to the residue, and the precipitated solid was collected by filtration to obtain 3.1 g of the desired product as a brown solid. The filtrate was purified by silica gel column chromatography using n-hexane - ethyl acetate (with a gradient of from 2:3 to 1:4) as the eluent to obtain 3.0 g of the desired product as a brown solid.
m. p.; 140-141°C

Step 2

Preparation of ethyl 3-bromo-1-(pyridin-3-yl)-1H-pyrazole-4-carboxylate

6.1 g of ethyl 3-amino1-(pyridin-3-yl)-1H-pyrazole-4-carboxylate in 50 ml of acetonitrile was mixed with 11.8 g of copper (II) bromide and stirred at 60°C. After the stirring, 4.1 g of tert-butyl nitrite was added dropwise to the reaction solution, and the reaction solution was stirred at 60°C for 3 hours. After the reaction, the reaction solution was cooled to 0°C, adjusted to pH 4 with 1 N aqueous sulfuric acid and stirred at room temperature for 1 hour. The reaction solution was further adjusted to pH 7 with 1 N aqueous sodium hydroxide, mixed with 150 ml of water and extracted with 300 ml of ethyl acetate. The resulting organic layer was dried over anhydrous sodium sulfate, and the solvent was evaporated under reduced pressure. The resulting residue was purified by silica gel column chromatography using n-hexane - ethyl acetate (with a gradient of from 3:1 to 1:4) as the eluent to obtain 4.5 g of the desired product as a pale yellow solid.
m. p.; 94-95°C

Step 3

Preparation of 3-bromo-1-(pyridin-3-yl)-1H-pyrazole-4-carboxylic acid

4.5 g of ethyl 3-bromo-1-(pyridin-3-yl)-1H-pyrazole-4-carboxylate in 75 ml of methanol was mixed with 1.7 g of potassium hydroxide and 10 ml of water and stirred at room temperature for 11 hours. After the reaction, the reaction solution was adjusted to pH 4 with 2 N aqueous hydrogen chloride, and the solvent was evaporated under reduced pressure. The resulting residue was mixed with 150 ml of water and extracted with ethyl acetate (200 ml × 3). The resulting organic layer was dried over anhydrous sodium sulfate, and the solvent was evaporated under reduced pressure to obtain 4 g of the desired product as a white solid.
m. p.; 270-278°C

Step 4

Preparation of tert-butyl 3-bromo-1-(pyridin-3-yl)-1H-pyrazol-4-ylcarbamate

To 4.0 g of 3-bromo-1-(pyridin-3-yl)-1H-pyrazole-4-carboxyli acid and 15 ml of 2-methyl-2-propanol in 15 ml of toluene, 3.77 g of triethylamine and 6.16 g of diphenylphosphoryl azide were added dropwise at room temperature successively. After the addition, the reaction mixture was refluxed with toluene for 4 hours with stirring. After the reaction, the reaction mixture was allowed to cool to room temperature, poured into ice-water and extracted with 300 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:1) as the eluent to obtain 4.0 g of the desired product as a pale yellow solid.
m. p.; 109-112°C

SYNTHETIC EXAMPLE 31

Preparation of tert-butyl 5-methyl-1-(pyridin-3-yl)-1H-pyrazol-4-ylcarbamate (Compound No. 5-001 of the present invention)

Step 1

Preparation of tert-butyl-1-(pyridin-3-yl)hydrazinecarboxylate

To 25 g of 3-iodopyridine in 200 ml of N,N-dimethylformamide, 219.3 g of tert-butyl carbazate, 64.3 g of cesium carbonate, 4.32 g of 1,10-phenanthroline, 1.16 g of copper (I) iodide were added successively. After the addition, the atmosphere in the reaction vessel was replaced by nitrogen gas, and the reaction mixture was stirred at 100°C for 4 hours. After the reaction, the reaction mixture was allowed to cool to room temperature, and the insolubles in the reaction mixture were filtered off through celite under reduced pressure. After addition of 100 ml of 1 N aqueous sodium hydroxide, the filtrate was extracted with ethyl acetate (200 ml × 1). The resulting organic layer was dried over anhydrous sodium sulfate, and the solvent was evaporated under reduced pressure to obtain 25.5 g of the desired product as a brown oil.
$^1$H NMR($CDCl_3$ ,$Me_4$ Si,300MHz)δ8.81(d,J=2.5Hz,1H),8.32(dd,J=4.8,1.2Hz,1H),7.90-7.80(m,1H),7.23(dd,J=8.4,4.8Hz,1H),4.43(brs,2H),1.52(s,9H).

Step 2

Preparation of 3-hydrazinylpyridine hydrochloride

25.5 g of tert-butyl-1-(pyridin-3-yl)hydrazinecarboxylate in 200 ml of 1,4-dioxane was mixed with 122 ml of about 4 M hydrogen chloride in 1,4-dioxane and stirred at room temperature for 12 hours. After the reaction, the crystals precipitated in the reaction solution were collected by filtration to obtain 26.5 g of the desired product as a white solid.
m. p.; 169-175°C

Step 3

Preparation of ethyl 5-methyl-1-(pyridin-3-yl)-1H-pyrazole-4-carboxylate

1.0 g of 3-hydrazinylpyridine in 15 ml of ethanol was mixed with 1.27 g of ethyl 2-dimethylaminomethylene

3-oxobutanoate and refluxed with ethanol for 30 minutes with stirring. After the reaction, the ethanol was evaporated from the reaction mixture, and the resulting residue was diluted with ethyl acetate and washed with water. The resulting organic layer was dried over anhydrous sodium sulfate, and the solvent was evaporated under reduced pressure. The resulting residue was purified by eluting a silica gel column with ethyl acetate to obtain 0.94 g of the desired product as a brown oil.
$^1H$ NMR($CDCl_3$ ,$Me_4$ Si,300MHz)δ8.73(d,J=2.4Hz,1H),8.68(dd,J=4.8,1.2Hz,11H), 8.06(s,1H),7.80(ddd,J=8.1,2.4, 1.2Hz,1H),7.46(dd,J=8.1,4.8Hz,1H), 4.33(q,J=7.2Hz,2H),2.59(s,3H),1.37(t,J=7.2Hz,3H)

Step 4

Preparation of 5-methyl-1-(pyridin-3-yl)-1H-pyrazole-4-carboxylic acid

3.88 g of ethyl 5-methyl-1-(pyridin-3-yl)-1H-pyrazole-4-carboxylate was dissolved in 30 ml of ethanol, then mixed with 1.41 g of potassium hydroxide and 30 ml of water successively and stirred overnight at room temperature. After the reaction, the ethanol was evaporated, and the resulting residue was adjusted to pH 4 with concentrated hydrochloric acid. The crystals precipitated in the reaction mixture were collected by filtration to obtain 2.82 g of the desired product as a pale yellow solid.
m. p.; 192-212°C

Step 5

Preparation of tert-butyl 5-methyl-1-(pyridin-3-yl)-1H-pyrazol-4-ylcarbamate

To 2.7 g of 5-methyl-1-(pyridin-3-yl)-1H-pyrazole-4-carboxylic acid and 15 ml of 2-methyl-2-propanol in 45 ml of toluene, 3.4 g of triethylamine and 5.5 g of diphenylphosphoryl azide were added at room temperature successively. After the addition, the reaction mixture was stirred at 100°C for 2 hours. After the reaction, the reaction mixture was allowed to cool to room temperature, poured into ice-water and extracted with 50 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (1:3) as the eluent to obtain 2.9 g of the desired product as a pale yellow solid.
m. p.; 89-91°C

SYNTHETIC EXAMPLE 32

Preparation of N-{3-chloro-1-(pyridin-3-yl)-1H-pyrazol-4-yl}-N-ethyl-2-(ethylthio)propanamide (Compound No. 1-118 of the present invention)

Step 1

Preparation of 1-tert-butyl-5-chloro-1H-pyrazole-4-carboxylic acid

10 g of ethyl 1-tert-butyl-5-chloro-1H-pyrazole-4-carboxylate was dissolved in 50 ml of ethanol, and 2.6 g of sodium hydroxide and 20 ml of water were added successively. After the addition, the reaction mixture was stirred at room temperature overnight. After the reaction, the solvent was evaporated under reduced pressure, and the resulting residue was adjusted to pH 3 with aqueous hydrochloric acid. The solid precipitated in the reaction solution was collected by filtration, washed with water and dried under reduced pressure to obtain 6.8 g of the desired product as a white solid.
m. p.; 132-134°C

Step 2

Preparation of tert-butyl 1-tert-butyl-5-chloro-1H-pyrazol-4-ylcarbamate (Compound No. F-001)

To 5.0 g of 1-tert-butyl-5-chloro-1H-pyrazole-4-carboxylic acid and 40 ml of 2-methyl-2-propanol in 40 ml of toluene, 3.25 g of triethylamine and 7.47 g of diphenylphosphoryl azide were added at room temperature successively. After the addition, the reaction mixture was heated with stirring for 1.5 hours under reflux. After the reaction, the reaction mixture was allowed to cool to room temperature, poured into ice-water and extracted with 100 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate

(6:1) as the eluent to obtain 6.25 g of the desired product as a pale yellow oil.
$^1$H NMR($CDCl_3$,$Me_4$Si,300MHz)δ7.74(s,1H),6.00(brs,1H),1.72(s,9H),1.50(s,9H)

Step 3

Preparation of tert-butyl 1-tert-butyl-5-chloro-1H-pyrazol-4-yl(ethyl)carbamate (Compound No.G-001)

To 136 mg of 60 wt% sodium hydride (dispersed in mineral oil) in 5 ml of tetrahydrofuran, 850 mg of tert-butyl 1-tert-butyl-5-chloro-1H-pyrazol-4-ylcarbamate was added under cooling with ice. After the addition, the reaction mixture was warmed to room temperature and stirred for 30 minutes. After the stirring, the reaction mixture was mixed with 532 mg of ethyl iodide and stirred at room temperature for another 1 hour. Then, the reaction solution was mixed with 10 ml of N,N-dimethylformamide and stirred for another 2 hours. After the reaction, the reaction mixture was poured into ice-water and extracted with 40 ml of ethyl acetate. The resulting organic layer was dried over anhydrous sodium sulfate, and the solvent was evaporated under reduced pressure. The resulting residue was purified by preparative medium pressure liquid chromatography using n-hexane - ethyl acetate (9:1) as the eluent to obtain 680 mg of the desired product as a white solid.
m. p.; 50-52°C

Step 4

Preparation of 1-tert-butyl-5-chloro-N-ethyl-1H-pyrazole-4-amine hydrochloride (Compound No. H-001)

0. 3 g of tert-butyl 1-tert-butyl-5-chloro-1H-pyrazol-4-yl(ethyl)carbamate in 2 ml of 1,4-dioxane was mixed with 2 ml of about 4 M hydrogen chloride in 1,4-dioxane and stirred at room temperature overnight. After the reaction, the solvent was evaporated under reduced pressure to obtain 0.27 g of the desired product as a white solid. $^1$H NMR($CDCl_3$, $Me_4$Si,300MHz)δ7.78(s,1H),3.69(s,1H),3.41(q,J=7.2Hz,2H), 1.69(s,9H),1.42(t,J=7.2Hz,3H)

Step 5

Preparation of N-(1-tert-butyl-5-chloro-1H-pyrazol-4-yl)-N-ethyl-2-(ethylthio)propanamide (Compound No. I-001)

236 mg of 2-(ethylthio)propanoic acid in 4 ml of dichloromethane was mixed with 300 mg of oxalyl chloride and 10 mg of N,N-dimethylformamide successively and stirred at room temperature for 1 hour. After the stirring, the reaction solution was added dropwise to a separately prepared solution of 280 mg of 1-tert-butyl-5-chloro-N-ethyl-1 H-pyrazole-4-amine hydrochloride and 140 mg of pyridine in 4 ml of dichloromethane, and the reaction was stirred at room temperature for 30 minutes. After the reaction, the reaction solution was washed with 5 ml of water. The resulting organic layer was washed with saturated aqueous sodium chloride and dried over anhydrous sodium sulfate, and the solvent was evaporated under reduced pressure. The resulting residue was purified by preparative medium pressure liquid chromatography using n-hexane - ethyl acetate (4:1) as the eluent to obtain 300 mg of the desired product as a colorless oil.
$^1$H NMR($CDCl_3$ ,$Me_4$ Si,300MHz)δ7.48(brs,1H),3.90-3.50(m,2H),3.30-3.20(m,1H), 2.52(q,J=7.2Hz,2H),1.72(s,9H), 1.42(d,J=7.2Hz,3H),1.14(t,J=7.2Hz,3H), 1.10(t,J=7.2Hz,3H)

Step 6

Preparation of N-(3-chloro-1H-pyrazol-4-yl)-N-ethyl-2-(ethylthio)propanamide (Compound No. J-001)

0.2 g of N-(1-tert-butyl-5-chloro-1H-pyrazol-4-yl)-N-ethyl-2-(ethylthio)propanamide was mixed with 2 ml of concentrated hydrochloric acid under cooling with ice and stirred at room temperature overnight. After the reaction, the reaction mixture was cooled to 0°C, neutralized with aqueous sodium hydroxide and extracted with 10 ml of chloroform. The resulting organic layer was dried over anhydrous sodium sulfate, and the solvent was evaporated under reduced pressure to obtain 0.1 g of the desired product as a white solid.
m. p.; 78-80°C

Step 7

Preparation of N-{3-chloro-1-(pyridin-3-yl)-1H-pyrazol-4-yl}-N-ethyl-2-(ethylthio)propanamide

To 100 mg of N-(3-chloro-1H-pyrazol-4-yl)-N-ethyl-2-(ethylthio)propanamide in 2 ml of N,N-dimethylformamide,

72 mg of 3-bromopyridine, 11 mg of copper (I) iodide, 215 mg of cesium carbonate and 16 mg of trans-N,N'-dimethylcyclohexane-1,2-diamine were added successively. After the addition, the atmosphere in the reaction vessel was replaced by nitrogen gas, and the reaction mixture was stirred at 140°C for 20 hours. After the reaction, the reaction mixture was mixed with 20 ml of ethyl acetate, and the solid precipitated in the reaction mixture was filtered off. The filtrate was washed with 10 ml of water and then with saturated aqueous sodium chloride and dried over anhydrous sodium sulfate, and the solvent was evaporated under reduced pressure. The resulting residue was purified by silica gel column chromatography using n-hexane - ethyl acetate (with a gradient of from 7:3 to 3:7) as the eluent to obtain 21 mg of the desired product as a white solid.
m. p.; 65-66°C

SYNTHETIC EXAMPLE 33

Preparation of N-{3-chloro-1-(pyridin-3-yl)-1H-pyrazol-4-yl}-2-(ethylthio)propanethioamide (Compound No. 1-353 of the present invention)

800 mg of N-{3-chloro-1-(pyridin-3-yl)-1H-pyrazol-4-yl}-2-(ethylthio)propanamide in 12 ml of toluene was mixed with 1.04 g of Lawesson's Reagent and refluxed with toluene for 3 hours with stirring. After the reaction, the reaction mixture was allowed to cool to room temperature, mixed with 15 ml of saturated aqueous sodium hydrogen carbonate and extracted with 40 ml of ethyl acetate. The resulting organic layer was with saturated aqueous sodium chloride and dried over anhydrous sodium sulfate, and the solvent was evaporated under reduced pressure. The resulting residue was purified by preparative medium pressure liquid chromatography using n-hexane - ethyl acetate (with a gradient of from 9:1 to 1:1) as the eluent to obtain 598 mg of the desired product as a yellow oil.
$^1$H NMR($CDCl_3$,$Me_4$Si,300MHz)δ10.61(brs,1H),9.52(s,1H),8,99(d,J=2.7Hz,1H), 8.58(dd,J=4.8,1.2Hz,1H),8.01(ddd, J=8.4,2.7,1.2Hz,1H),7.42(dd,J=8.4,4.8Hz,1H), 4.21 (q,J=7.2Hz,1 H),2.67-2.49(m,2H),1.64(d,J=7.2Hz,3H),1.29(t, J=7.5Hz,3H)

SYNTHETIC EXAMPLE 34

Preparation of methyl N-3-chloro-1-(pyridin-3-yl)-1H-pyrazol-4-yl-2-(ethylthio)propanimidothioate (Compound No. 3-006 of the present invention)

540 mg of N-{3-chloro-1-(pyridin-3-yl)-1H-pyrazol-4-yl)-2-(ethylthio)propanethioamide in 4.5 ml of acetonitrile was mixed with 230 mg of potassium carbonate and 350 mg of methyl iodide and stirred at 50°C for 1 hour. After the reaction, the reaction mixture was mixed with 10 ml of water and extracted with 20 ml of ethyl acetate. The resulting organic layer was with saturated aqueous sodium chloride and dried over anhydrous sodium sulfate, and the solvent was evaporated under reduced pressure. The resulting residue was purified by preparative medium pressure liquid chromatography using n-hexane - ethyl acetate (with a gradient of from 9:1 to 1:1) as the eluent to obtain 391 mg of the desired product as a yellow oil. The desired product was a 2:1 mixture of two geometrical isomers.
The isomer contained in a ratio of 2
$^1$H NMR($CDCl_3$,$Me_4$Si,300MHz)δ8.89(d,J=2.1Hz,1H),8.54(dd,J=4.8,1.2Hz,1H),8.01-7.99(m,1 H),7.59(s,1H),7.40(dd, J=8.4,4.8Hz,1H),4.12(q,J=7.2Hz,1H),2.71-2.53(m,2H),2.42(s,3H),1.51(d,J=7.2Hz,3H),1.19(t,J=7.2Hz,3H)
The isomer contained in a ratio of 1
$^1$H NMR($CDCl_3$ ,$Me_4$ Si,300MHz)δ8.92-8.89(m,1H),8.54(dd,J=4.8,1.2Hz,1H),8.01-7.99(m,1H),7.93(s,1H),7.40(dd, J=8.4,4.8Hz,1H),3.89(q,J=6.9Hz,1H),2.71-2.53(m,2H),2.65(s,3H),1.69(d,J=6.9Hz,3H),1.27(t,J=7.2Hz,3H)

SYNTHETIC EXAMPLE 35

Preparation of N-{3-chloro-1-(pyridin-3-yl)-1H-pyrazol-4-yl}-2-(ethylthio)-N'-methoxypropanimidamide (Compound No. 1-356 of the present invention)

284 mg of methyl N-3-chloro-1-(pyridin-3-yl)-1H-pyrazol-4-yl-2-(ethylthio)propanimidothioate in 4 ml of ethanol was mixed with 110 mg of O-methylhydroxylamine hydrochloride and 132 mg of triethylamine successively and stirred at 50°C for 3 hours. After the stirring, 110 mg of O-methylhydroxylamine hydrochloride and 132 mg of triethylamine were added again, and the reaction solution was stirred at 50°C for another 4 hours. After the stirring, 220 mg of O-methylhydroxylamine hydrochloride and 265 mg of triethylamine were further added, and the reaction solution was stirred at 50°C for another 3 hours. After the reaction, the reaction solution was mixed with 10 ml of water and extracted with 20 ml of ethyl acetate. The resulting organic layer was with saturated aqueous sodium chloride and dried over anhydrous sodium sulfate, and the solvent was evaporated under reduced pressure. The resulting residue was purified by preparative

medium pressure liquid chromatography using n-hexane - ethyl acetate (with a gradient of from 9:1 to 1:1) as the eluent to obtain 237 mg of the desired product as a yellow oil. The desired product was a 5:2 mixture of two isomers.
The isomer contained in a ratio of 5
$^1$H NMR($CDCl_3$ ,$Me_4$ Si,300MHz)δ8.93(d,J=2.7Hz,1H),8.52(dd,J=5.1,1.2Hz,1H), 8.51 (s,1H),8.00(ddd,J=8.4,2.7,1.2Hz, 1H),7.39(dd,J=8.4,5.1Hz,1H),6.92(brs,1H), 4.64(q,J=7.2Hz,1H),3.88(s,3H),2.65-2.44(m,2H),1.45(d,J=7.2Hz,3H), 1.26(t,J=7.5Hz,3H) The isomer contained in a ratio of 2
$^1$H NMR($CDCl_3$ ,$Me_4$ Si,300MHz)δ8.92(d,J=2.7Hz,1H),8.58(dd,J=4.5,1.2Hz,1H), 8.08(s,1H),8.00(ddd,J=8.4,2.7,1.2Hz, 1H),7.57(dd,J=8.4,4.5Hz,1H),6.38(brs,1H), 3.43(q,J=7.2Hz,1 H),3.87(s,3H),2.65-2.44(m,2H),1.47(d,J=7.2Hz,3H), 1.24(t,J=7.5Hz,3H)

SYNTHETIC EXAMPLE 36

Preparation of N-{3-chloro-1-(pyridin-3-yl)-1H-pyrazol-4-yl}-2-(ethylthio)-N-(pyop-2-ynyl)propanamide (Compound No. 1-289 of the present invention)

0.15 g of 60 wt% sodium hydride (dispersed in mineral oil) in 5 ml of tetrahydrofuran was mixed with 0.48 g of N-{3-chloro-1-(pyridin-3-yl)-1H-pyrazol-4-yl)-2-(ethylthio)propanamide under cooling with ice and stirred for 30 minutes. After the stirring, the reaction mixture was mixed with 63 μL of about 9.2 mol/L propargyl bromide in toluene and stirred at room temperature for another 30 minutes. After the reaction, the reaction mixture was poured into 10 ml of ice-water and extracted with 10 ml of ethyl acetate. The resulting organic layer was with saturated aqueous sodium chloride and dried over anhydrous sodium sulfate, and the solvent was evaporated under reduced pressure. The resulting residue was purified by preparative medium pressure liquid chromatography using n-hexane - ethyl acetate (2:1) as the eluent to obtain 0.13 g of the desired product as a pale yellow amorphous substance.
$^1$H NMR($CDCl_3$ ,$Me_4$ Si,300MHz)δ8.95(d,J=2.4Hz,1H),8.62(dd,J=4.8,1.2Hz,1H), 8.22(s,1H),8.04(ddd,J=8.1,2.4,1.2Hz, 1H),7.45(dd,J=8.1,4.8Hz,1H),4.97-4.60(m,1H), 4.30-3.95(m,1H),3.39-3.27(m,1H),2.66-2.52(m,2H),2.25(t,J=2.1Hz, 11H), 1.48(d,J=6.9Hz,3H),1.17(t,J=7.2Hz,3H)

SYNTHETIC EXAMPLE 37

Preparation of (S)-(-)-N-{3-chloro-1-(pyridin-3-yl)-1H-pyrazol-4-yl}-N-ethyl-2-methyl-3-(methylthio)propanamide [Compound No. 1-048(S)-(-) of the present invention]

To a solution of 0.905 g of (S)-2-methyl-3-(methylthio)propanoic acid synthesized in accordance with US-A-2005-146823 in 20 ml of dichloromethane, 1.14 g of oxalyl chloride and 10 mg of N,N-dimethylformamide were added successively, and the reaction mixture was stirred at room temperature for 2 hours. After the reaction, the solvent was evaporated under reduced pressure, and the resulting residue was mixed with 1 g of 3-chloro-N-ethyl-1-(pyridin-3-yl)-1H-pyrazole-4-amine and 1.07 g of pyridine in 20 ml of dichloromethane and stirred at room temperature for 1 hour. After the reaction, the reaction solution was washed with 20 ml of water. The resulting organic layer was washed with saturated aqueous sodium chloride and dried over anhydrous sodium sulfate, and the solvent was evaporated under reduced pressure. The resulting residue was purified by preparative medium pressure liquid chromatography using n-hexane - ethyl acetate (1:1) as the eluent and recrystallized from a solvent mixture of n-hexane and ethyl acetate (5:1) to obtain 0.4 g of the desired product as a white solid. The desired product was optically resolved by high performance liquid chromatography using an optically active column under the conditions described later, and 99% of it, in terms of peak area percentage, was detected as a fraction with a retention time of 18.4min {$[\alpha]_D^{23.9}$-10.00° ($CHCl_3$, c=0.510)}.

[REFERENCE EXAMPLES]

REFERENCE EXAMPLE 1

The following intermediates were synthesized in the same manner as in Step 1 in Synthetic Example 3.
A-002; N-ethyl-3-methyl-1-(pyridin-3-yl)-1H-pyrazole-4-amine dihydrochloride
m. p.; 113-116°C
A-005; N-allyl-3-chloro-1-(pyridin-3-yl)-1H-pyrazole-4-amine
$^1$H NMR($D_2O$,$Me_4$Si,300MHz)δ9.05(d,J=2.7Hz,1H),8.66(ddd,J=8.4,2.4,1.2Hz,1H), 8.59(ddd,J=5.7,0.9,0.6Hz,1 H), 8.32(brs,1H),8.02(ddd,J=9.3,5.7, 0.6Hz,1 H),5.33-5.25(m,1H),5.89-5.76(m,2H),3.79(d,J=6.3Hz,2H) (no detectable peaks for NH) A-009; 3-methyl-1-(pyridin-3-yl)-1H-pyrazole-4-amine dihydrochloride
m. p.; 198-206°C
A-010; 1-(pyridin-3-yl)-1H-pyrazole-4-amine dihydrochloride

m. p.; 195-196°C (decomposition)

REFERENCE EXAMPLE 2

The following intermediates were synthesized in the same manner as in Step 1 in Synthetic Example 25.
B-001; N,3-dimethyl1-(pyridin-3-yl)-1H-pyrazole-4-amine
m. p.; 74-76°C
B-002; N-ethyl-3-methyl-1-(pyridin-3-yl)-1H-pyrazole-4-amine
m. p.; 80-81°C
B-006; 3-chloro-N-n-propyl-1-(pyridin-3-yl)-1H-pyrazole-4-amine
$^1$H NMR($CDCl_3$ ,$Me_4$ Si,300MHz)δ8.86(d,J=2.7Hz,1H),8.46(d,J=3.6Hz,1H), 7.94(ddd,J=9.6,2.7,1.2Hz,1H), 7.37-7.32(m,2H),3.15(brs,1H),3.02(t,J=7.2Hz,2H), 1.69(q,J=7.2Hz,2H),1.02(t,J=7.5Hz,3H)
B-007;3-chloro-N-isopropyl-1-(pyridin-3-yl)-1H-pyrazole-4-amine
$^1$H NMR($CDCl_3$ ,$Me_4$ Si,300MHz)δ8.87(d,J=2.7Hz,1H),8.48(dd,J=4.8,1.5Hz,1H), 7.96(ddd,J=8.4,2.7,1.5Hz,1H), 7.38-7.34(m,2H),3.34(qq,J=4.8,4.8Hz,1H),2.85(brs,1H), 1.25(d,J=6.6Hz,6H)
B-008; 3-chloro-N-(prop-2-ynyl)-1-(pyridin-3-yl)-1H-pyrazole-4-amine m. p.; 123-124°C
B-009; 3-methyl-1-(pyridin-3-yl)-1H-pyrazole-4-amine
m. p.; 120-122°C
B-010; 3-bromo-N-ethyl-1-(pyridin-3-yl)-1H-pyrazole-4-amine
m. p.; 109-110
B-011; 1-(pyridin-3-yl)-1H-pyrazole-4-amine
m. p.; 88-89°C
B-012; 3-chloro-1-(pyridin-3-yl)-N-(2,2,2-trifluoroethyl)-1H-pyrazole-4-amine
m. p.; 79-82°C
B-013; N-ethyl-5-methyl-1-(pyridin-3-yl)-1H-pyrazole-4-amine
$^1$ H NMR($CDCl_3$,$Me_4$Si,300MHz)δ8.76-8.70(m,1H),8.57(dd,J=4.8,1.2Hz,1H), 7.81 (ddd,J=8.1,2.4,1.2Hz,1H), 7.44-7.36(m,1H),7.42(s,1H),3.11(q,J=7.2Hz,2H), 2.26(s,3H),1.25(t,J=7.2Hz,3H) (no detectable peaks for NH)
B-014; 3-chloro-N-(cyclopropylmethyl)-1-(pyridin-3-yl)-1H-pyrazole-4-amine
m. p.; 72-78°C
B-015; 3-chloro-N-(2-methoxyethyl)-1-(pyridin-3-yl)-1H-pyrazole-4-amine
m. p.; 68-69°C
B-016; 3-chloro-1-(pyridin-3-yl)-N-{(trimethylsilyl)methyl}-1H-pyrazole-4-amine
m. p.; 105-111°C
B-017; N-ethyl-3-methoxy-1-(pyridin-3-yl)-1H-pyrazole-4-amine
$^1$H NMR($CDCl_3$,$Me_4$Si,300MHz)δ8.79(d,J=2.7Hz,1H),8.31(dd,J=4.8,1.2Hz,1H), 7.81 (ddd,J=8.4,2.7,1.2Hz,1H), 7.27(dd,J=8.4,4.8Hz,1H),7.21(s,1H),4.01(s,3H), 3.05(q,J=7.2Hz,2H),2.63(brs,1H),1.27(t,J=7.2Hz,3H)
B-018; N-ethyl-1-(pyridin-3-yl)-3-(trifluoromethyl)-1H-pyrazole-4-amine
m. p.; 88-89°C
B-020; N-ethyl-3-(methylsulfonyl)-1-(pyridin-3-yl)-1H-pyrazole-4-amine
m. p.; 139-141°C
B-020; 3-bromo-N-methyl-1-(pyridin-3-yl)-1H-pyrazole-4-amine
m. p.; 107-109°C
B-021; 3-chloro-1-(pyridin-3-yl)-1H-pyrazole-4-amine
m. p.; 70-72°C
B-022; 3-bromo-1-(pyridin-3-yl)-1H-pyrazole-4-amine
m. p.; 138-140°C

REFERENCE EXAMPLE 3

The following intermediate was synthesized in the same manner as in Step 1 in Synthetic Example 28.
C-002;3-chloro-N-{2-(methylthio)ethyl}-1-(pyridin-3-yl)-1H-pyrazole-4-amine
m. p.; 66-68 °C

REFERENCE EXAMPLE 4

The following intermediates were synthesized in the same manner as in Step 1 in Synthetic Example 20.
D-002; ethyl 2-(ethoxyimino)-3-(methylthio)propanoate
$^1$H NMR($CDCl_3$,$Me_4$Si,300MHz)δ4.40-4.25(m,4H),3.57(s,2H),2.11(s,3H), 1.38(t,J=7.1 Hz,3H),1.31 (t,J=6.3Hz,3H)

D-003; ethyl 2-(isopropoxyimino)-3-(methylthio)propanoate
$^1$H NMR($CDCl_3$,$Me_4Si$,300MHz)δ4.56(sep,J=6.3Hz,1H),4.34(q,J=7.1Hz,2H), 3.57(s,2H),2.10(s,3H),1.35(t,J=7.1Hz, 3H),1.30(d,J=6.3Hz,6H) D-004; ethyl 3-(methylthio)-2-(n-propoxyimino)propanoate
$^1$H NMR($CDCl_3$,$Me_4Si$,300MHz)δ4.35(q,J=7.1Hz,2H),4.22(t,J=7.1 Hz,2H),3.58(s,2H), 2.11(s,3H),1.72(m,2H),1.35(t, J=7.1Hz,3H),0.96(t,J=7.1Hz,3H) D-005; ethyl 2-(benzyloxyimino)-3-(methylthio)propanoate
$^1$H NMR($CDCl_3$,$Me_4Si$,300MHz)δ7.40-7.35(m,5H),5.28(s,2H),4.33(q,J=7.1Hz,2H), 3.58(s,2H),2.05(s,3H),1.35(t, J=7.1Hz,3H)
D-006; ethyl 2-(butoxyimino)-3-(methylthio)propanoate
$^1$H NMR($CDCl_3$,$Me_4Si$,300MHz)δ4.35(q,J=7.1Hz,2H),4,27(t,J=6.5Hz,2H),3.57(s,2H), 2.13(s,3H),1.69(tt,J=7.1,6.5Hz, 2H),1.39(sxt,J=7.1Hz,2H),1.35(t,J=7.1Hz,3H), 0.94(t,J=7.1Hz,3H)
D-007; ethyl 2-(methoxyimino)-3-(2,2,2-trifluoroethylthio)propanoate
$^1$H NMR($CDCl_3$,$Me_4Si$,300MHz)δ4.37(q,J=7.1Hz,2H),4.09(s,3H),3.69(s,2H), 3.15(q,J=9.9Hz,2H),1.36(t,J=7.1Hz,3H)
D-008; ethyl (2-methoxyethoxyimino)-3-(methylthio)propanoate
$^1$H NMR($CDCl_3$,$Me_4Si$,300MHz)δ4.40(t,J=4.8Hz,2H),4.34(q,J=7.1Hz,2H), 3.66(t,J=4.8Hz,2H),3.60(s,2H),3.37(s,3H), 2.11(s,3H),1.35(t,J=7.1Hz,3H) D-009; ethyl 2-(allyloxyimino)-3-(methylthio)propanoate
$^1$H NMR($CDCl_3$,$Me_4Si$,300MHz)δ5.98(ddt,J=17.1,10.5,5.7Hz,1H), 5.33(dd,J=17.1,1.5Hz,1H),5.26(dd,J=10.5,1.5Hz, 1H),4.76(d,J=5.7Hz,2H), 4.34(q,J=7.1Hz,2H),3.59(s,2H),2.11(s,3H),1.34(t,J=7.1Hz,3H)
D-010; ethyl 2-(methoxyimino)-3-(trifluoromethylsulfonyl)propanoate
$^1$H NMR($CDCl_3$,$Me_4Si$,300MHz)δ4.64(s,2H),4.38(q,J=7.2Hz,2H),4.19(s,3H), 1.36(t,J=7.2Hz,3H)

REFERENCE EXAMPLE 5

The following intermediates were synthesized in the same manner as in Step 2 in Synthetic Example 20.
E-002; 2-(ethoxyimino)-3-(methylthio)propanoic acid
$^1$H NMR($CDCl_3$,$Me_4Si$,300MHz)δ4.34(q,J=7.1Hz,2H),3.54(s,2H),2.14(s,3H), 1.34(t,J=7.1Hz,3H)(no detectable peaks for $CO_2H$)
E-003; 2-(isopropoxyimino)-3-(methylthio)propanoic acid
$^1$H NMR($CDCl_3$,$Me_4Si$,300MHz)δ4.54(sep,J=6.5Hz,1H),3.53(s,2H),2.12(s,3H), 1.31 (d,J=6.5Hz,6H)(no detectable peaks for $CO_2H$)
E-004; 3-(methylthio)-2-(n-propoxyimino)propanoic acid
$^1$H NMR($CDCl_3$,$Me_4Si$,300MHz)δ4.22(t,J=7.1Hz,2H),3.54(s,2H),2.13(s,3H), 1.74(m,2H),0.96(t,J=7.1Hz,3H)(no detectable peaks for $CO_2H$)
E-005; 2-(benzyloxyimino)-3-(methylthio)propanoic acid
$^1$H NMR($CDCl_3$,$Me_4Si$,300MHz)δ7.40-7.30(m,5H),5.23(s,2H),3.47(s,2H),1.91(s,3H) (no detectable peaks for $CO_2H$)
E-006; 2-(butoxyimino)-3-(methylthio)propanoic acid
$^1$H NMR($CDCl_3$,$Me_4Si$,300MHz)δ4.10(t,J=6.5Hz,2H),3.47(s,2H),2.01(s,3H), 1.57(tt,J=7.1,6.5Hz,2H),1.35(sxt,J=7.1Hz, 2H),0.90(t,J=7.1Hz,3H) (no detectable peaks for $CO_2H$)
E-007; 2-(methoxyimino)-3-(2,2,2-trifluoroethylthio)propanoic acid
$^1$H NMR($CDCl_3$,$Me_4Si$,300MHz)δ3.90(s,3H),3.57(s,2H),3.12(q,J=9.9Hz,2H) (no detectable peaks for $CO_2H$)
E-008; (2-methoxyethoxyimino)-3-(methylthio)propanoic acid
$^1$H NMR($CDCl_3$,$Me_4Si$,300MHz)δ4.43(t,J=4.8Hz,2H),3.68(t, J=4.8Hz,2H), 3.57(s,2H),3.39(s,3H),2.15(s,3H)( no detectable peaks for $CO_2H$) E-009; 2-(allyloxyimino)-3-(methylthio)propanoic acid
$^1$H NMR($CDCl_3$,$Me_4Si$,300MHz)δ5.95(ddt,J=17.1,10.5,5.7Hz,1H),5.40-5.30(m,1H),5.30-5.20(m,1H),4.73(d,J=5.7Hz, 2H),3.54(s,2H),2.13(s,3H) (no detectable peaks for $CO_2H$) E-010; 2-(methoxyimino)-3-(trifluoromethylsulfonyl)propanoic acid
$^1$H NMR($CDCl_3$,$Me_4Si$,300MHz)δ4.64(s,2H),4.26(s,3H) (no detectable peaks for $CO_2$ H)

REFERENCE EXAMPLE 6

Synthesis of methyl 4-amino-1-(pyridin-3-yl)-1H-pyrazole-3-carboxylate

3-Bromopyridine and methyl 4-amino-1H-pyrazole-3-carboxylate were reacted in the same manner as in Step 7 in Synthetic Example 32 to give methyl 4-amino-1-(pyridin-3-yl)-1H-pyrazole-3-carboxylate.
m. p.; 102-108 °C

REFERENCE EXAMPLE 7

The following intermediates were synthesized in the same manner as in Step 3 in Synthetic Example 32.

G-002; tert-butyl 1-tert-butyl-5-chloro-1H-pyrazol-4-yl(methyl)carbamate
m. p.; 30-31 °C

REFERENCE EXAMPLE 8

The following intermediates were synthesized in the same manner as in Step 4 in Synthetic Example 32.
H-002; 1-tert-butyl-5-chloro-N-methyl-1H-pyrazole-4-amine
$^1H$ NMR($CDCl_3$,$Me_4Si$,300MHz)δ7.14(s,1H),2.81(s,3H),1.66(s,9H) (no detectable peaks for NH)

REFERENCE EXAMPLE 9

The following intermediates were synthesized in the same manner as in Step 5 in Synthetic Example 32.
I-002; N-(1-tert-butyl-5-chloro-1H-pyrazol-4-yl)-N,2-dimethyl-3-(methylthio)propanamide
m. p.; 41-42°C
I-003; N-(1-tert-butyl-5-chloro-1H-pyrazol-4-yl)-N-ethyl-2-methyl-3-(methylthio)propanamide
m. p.; 52-53°C

REFERENCE EXAMPLE 10

The following intermediates were synthesized in the same manner as in Step 6 in Synthetic Example 32.
J-002; N-(3-chloro-1H-pyrazol-4-yl)-N,2-dimethyl-3-(methylthio)propanamide
m. p.; 91-93°C
J-003; N-(3-chloro-1H-pyrazol-4-yl)-N-ethyl-2-methyl-3-(methylthio)propanamide
m. p.; 111-112°C

REFERENCE EXAMPLE 11

Synthesis of 3-chloro-1-(pyridin-3-yl)-1H-pyrazole-4-amine (Compound No. B-021)

3-Bromopyridine and 3-chloro-1 H-pyrazole-4-amine were reacted in the same manner as in Step 7 in Synthetic Example 32 to give 3-chloro-1-(pyridin-3-yl)-1H-pyrazole-4-amine.
m. p.; 70-72 °C

The compounds of the present invention can be produced in accordance with the processes and Examples previously described. Examples of the compounds of the present invention in the same manners as in Synthetic Examples 1 to 37 are recited in Tables 4 to 10 and their physical properties are shown in Tables 11 and 12. However, the present invention is by no means restricted thereto. In the Tables, D1-1a, D1-1b, D1-2a, D1-2c, D1-4a, D1-5e, D1-5f, D1-6d, D1-7b, D1-8c, D1-8f, D1-9f, D1-10e, D1-10f, D1-12a, D1-12f, D1-13d, D1-19a, D1-28a, D1-32a, D1-32b, D1-33a, D1-33b, D1-34a, D1-35b, D1-37a, D1-37b, D1-38b, D1-45d, D1-51a, D1-80a, D1-81a, D1-81b, D1-82a, D1-82b, D1-82c, D1-84d, D1-85d, D1-87a, D1-88a, D1-88b, D1-92c, D1-93a, D1-94b, D1-94c, D1-98a, D1-103b, D1-103c, D1-103d, D1-103e, D1-103f, D1-103g, D1-103h, D1-103i, D1-108a and D1-108b represent the following structures, and the numbers in the structural formulae D1-1b, D1-2c, D1-5e, D1-5f, D1-6d, D1-7b, D1-8f, D1-9f, D1-10e, D1-10f, D1-12e, D1-12f, D1-32b, D1-33b, D1-37b and D1-38b indicate the positions of the substituent $X^1$, the numbers in the structural formula D1-45d indicate the positions of the substituent $X^{1b}$, and the numbers in the structural formula D1-108b indicate the positions of the substituent Z. The numbers in the structural formulae in Table 10 indicate the positions of the substituent $R^2$.

*1 means resinous. *2 means a resinous mixture of two isomers having the same structure as in Synthetic Example 10, and, if known, the ratio of the isomers is shown in Table 11. *3 means the melting point of a mixture of two compounds having different structures, as obtained in Synthetic Example 8. *4 means the geometrical isomer of the oxime 1-170.

The symbols in Tables 11 and 12 have the following meanings.
s: singlet, d: doublet, t: triplet, q: quartet, qui: quintet, sxt: sextet, sep: septet, m: multiplet, brs: broad peak.

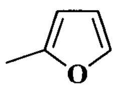
D1-1a

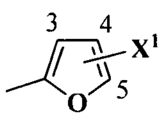
D1-1b

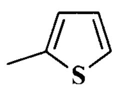
D1-2a

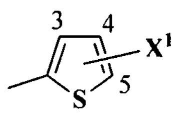
D1-2c

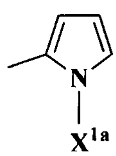
D1-4a

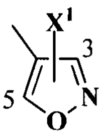
D1-5e

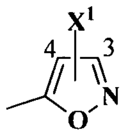
D1-5f

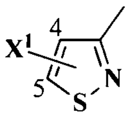
D1-6d

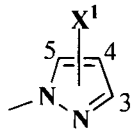
D1-7b

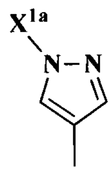
D1-8c

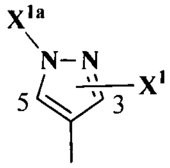
D1-8f

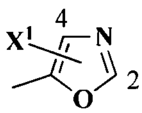
D1-9f

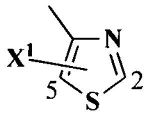
D1-10e

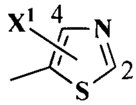
D1-10f

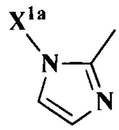
D1-12a

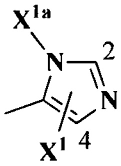
D1-12f

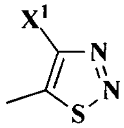
D1-13d

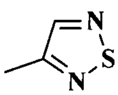
D1-19a

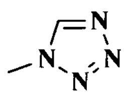
D1-28a

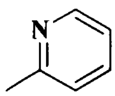
D1-32a

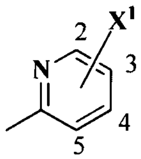
D1-32b

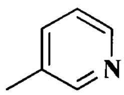
D1-33a

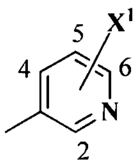
D1-33b

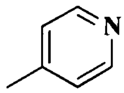
D1-34a

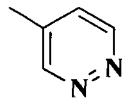
D1-35b

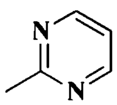
D1-37a

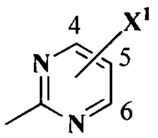
D1-37b

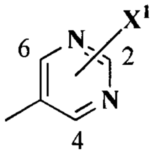
D1-38b

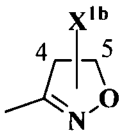
D1-45d

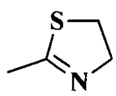
D1-51a

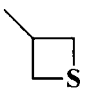
D1-80a

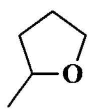
D1-81a

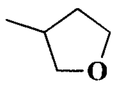
D1-81b

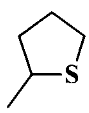
D1-82a

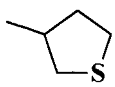
D1-82b

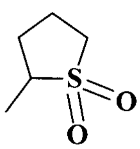
D1-82c

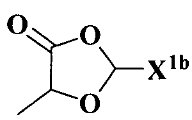
D1-84d

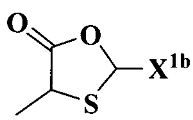
D1-85d

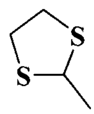
D1-87a

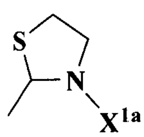
D1-88a

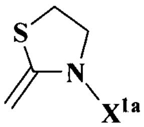

D1-88b

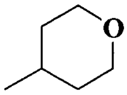

D1-92c

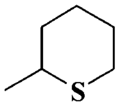

D1-93a

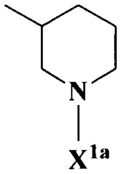

D1-94b

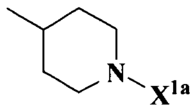

D1-94c

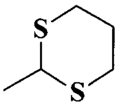

D1-98a

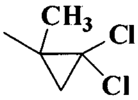

D1-103b

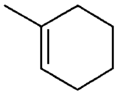

D1-103c

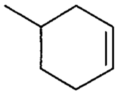

D1-103d

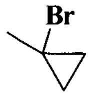

D1-103e

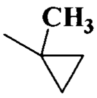

D1-103f

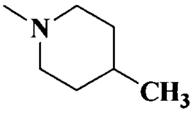

D1-103g

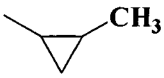

D1-103h

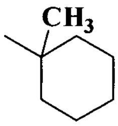

D1-103i

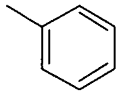

D1-108a

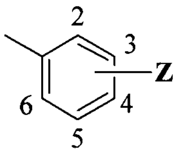

D1-108b

[Table 4]

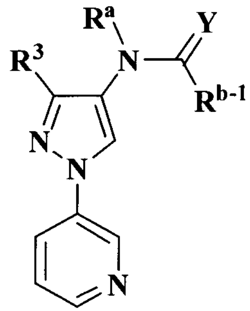

| No. | $R^3$ | Y | $R^a$ | $R^{b-1}$ | m.p.(°C) |
|---|---|---|---|---|---|
| 1-001 | H | O | H | $CH_2CF_3$ | 178-183 |
| 1-002 | H | O | H | $CH_2S(O)_2CH_3$ | 202-205 |
| 1-003 | H | O | H | $CH_2SCH_3$ | 173-176 |
| 1-004 | H | O | H | $NHCH_2CF_3$ | *1 |
| 1-005 | H | O | H | $NHCH_2CH_3$ | 145-151 |
| 1-006 | H | O | H | $CH_2CH_2SCH_3$ | 134-138 |
| 1-007 | H | O | H | $CH=C(CH_3)CF_3$ | 210-216 |
| 1-008 | H | O | H | $CH=CHCF_3$ | 248-249 |
| 1-009 | H | O | H | CH=CH{D1-108b(4-CF3)} | *1 |
| 1-010 | H | NCN | H | $NHCH_2CH_3$ | 190-197 |
| 1-011 | Me | O | H | $OC(CH_3)_3$ | 112-116 |
| 1-012 | Me | O | $CH_3$ | $OC(CH_3)_3$ | 65-70 |
| 1-013 | Me | O | $CH_2CH_3$ | $OC(CH_3)_3$ | 73-76 |
| 1-014 | H | O | H | $CH_2CH(OH)CF_3$ | 157-158 |
| 1-015 | H | NCN | H | $NHCH_2CF_3$ | 205-210 |
| 1-016 | Me | NCN | H | $NHCH_2CH_3$ | 170-175 |
| 1-017 | Me | NCN | H | $NHCH_2CF_3$ | 215-223 |
| 1-018 | Me | O | $CH_3$ | $CH_2CH_2SCH_3$ | *1 |
| 1-019 | Me | O | $CH_2CH_3$ | $CH_2CH_2SCH_3$ | *1 |
| 1-020 | H | O | H | $OC(CH_3)_3$ | 110-115 |
| 1-021 | H | O | $CH_3$ | $OC(CH_3)_3$ | *1 |
| 1-022 | H | O | $CH_2CH_3$ | $OC(CH_3)_3$ | *1 |
| 1-023 | Me | O | $CH_3$ | $CH(CH_3)CH_2SCH_3$ | *1 |

(continued)

| No. | $R^3$ | Y | $R^a$ | $R^{b-1}$ | m.p.(°C) |
|---|---|---|---|---|---|
| 1-024 | H | O | H | D1-8c($CH_2CF_3$) | 145-155 |
| 1-025 | H | O | H | $CH(Br)CH_2CH_2CH_2Br$ | 114-115 |
| 1-026 | H | O | $-CH_2CH_2CH_2-$ | | *1 |
| 1-027 | H | O | $-CH_2CH_2CH_2CH(Br)-$ | | 120-121 |
| 1-028 | H | O | $-CH_2CH_2CH_2CH(SCH_3)-$ | | 107-108 |
| 1-029 | H | O | $-CH_2OCH(CF_3)CH_2-$ | | 147-148 |
| 1-030 | H | O | $-CH_2OCH_2N(CH_2CF_3)-$ | | 138-141 |
| 1-031 | Me | O | $CH_2CH_3$ | $CH(CH_3)CH_2SCH_3$ | *1 |
| 1-031(S)-(+) | | 99%e.e. | $[\alpha]_D^{24.4}$+32.20° ($CHCl_3$,c=0.682) | | *1 |
| 1-032 | Me | O | H | $NHCH_2CF_3$ | 228-230 |
| 1-033 | Cl | O | $CH_2CH_3$ | $CH_2CH_2SCH_3$ | *1 |
| 1-034 | H | O | $-CH_2CH_2CH_2CH_2-$ | | 123-124 |
| 1-035 | H | O | $-CH_2CH_2CH_2CH\{S(O)_2CH_3\}-$ | | 163-164 |
| 1-036 | H | O | $-CH_2CH_2CH_2C(F)\{S(O)_2CH_3\}-$ | | 173-174 |
| 1-037 | Cl | O | $CH_3$ | $OC(CH_3)_3$ | 83-84 |
| 1-038 | Cl | O | $CH_3$ | $CH_2CH_2SCH_3$ | *1 |
| 1-039 | Cl | O | $CH_3$ | $CH(CH_3)CH_2SCH_3$ | *1 |
| 1-039(S)-(-) | | 99%e.e. | $[\alpha]_D^{24.0}$-10.76° ($CHCl_3$,c=0.502) | | 76-77 |
| 1-040 | Cl | O | $CH_2CH_3$ | $CH_2CH_2S(O)CH_3$ | *1 |
| 1-041 | Cl | O | $CH_2CH_3$ | $CH_2CH_2S(O)_2CH_3$ | 103-108 |
| 1-042 | H | O | $CH_3$ | $CH_2CH_2SCH_3$ | *2(~3:2) |
| 1-043 | H | O | $CH_3$ | $CH(CH_3)CH_2SCH_3$ | *2(~3:1) |
| 1-044 | H | O | $CH_2CH_3$ | $CH_2CH_2SCH_3$ | *2(~3:1) |
| 1-045 | H | O | $CH_2CH_3$ | $CH(CH_3)CH_2SCH_3$ | *2(~5:1) |
| 1-046 | Cl | O | H | $OC(CH_3)_3$ | 125-128 |
| 1-047 | H | O | $(-CH_2CH_2CH=CH-)\&(-CH_2CH=CHCH_2-)$ | | 128-129(*3) |
| 1-048 | Cl | O | $CH_2CH_3$ | $CH(CH_3)CH_2SCH_3$ | 64-65 |
| 1-048(R)-(+) | | 84%e.e. | $[\alpha]_D^{24.1}$+8.95° | ($CHCl_3$,c=0.503) | 64-65 |
| 1-048(S)-(-) | | 99%e.e. | $[\alpha]_D^{23.9}$-10.00° | ($CHCl_3$,c=0.510) | 64-65 |
| 1-049 | Cl | O | $CH_2CH_3$ | $CH(CH_3)CH_2S(O)CH_3$ | *2(~10:7) |
| 1-050 | Cl | O | $CH_2CH_3$ | $CH(CH_3)CH_2S(O)_2CH_3$ | *1 |
| 1-050(S)-(+) | | 99%e.e. | $[\alpha]_D^{24.4}$+32.20° ($CHCl_3$,c=0.682) | | *1 |
| 1-051 | Cl | O | $CH_3$ | $CH_2CH_2S(O)CH_3$ | *1 |

(continued)

| No. | $R^3$ | Y | $R^a$ | $R^{b-1}$ | m.p.(°C) |
|---|---|---|---|---|---|
| 1-052 | Cl | O | $CH_3$ | $CH_2CH_2S(O)_2CH_3$ | 90-91 |
| 1-053 | Cl | O | $CH_3$ | $CH(CH_3)CH_2S(O)CH_3$ | *2(~4:1) |
| 1-054 | Cl | O | $CH_3$ | $CH(CH_3)CH_2S(O)_2CH_3$ | 153-154 |
| 1-054(S)-(+) | | 96%e.e. | $[\alpha]_D^{23.8}$+23.20°($CHCl_3$,c=0.500) | | 115-116 |
| 1-055 | H | O | H | $CH_2CH_2CH_2Cl$ | 131-132 |
| 1-056 | Cl | O | $CH_2CH_3$ | $OC(CH_3)_3$ | 55-58 |
| 1-057 | H | O | $CH_3$ | $CH_2S(O)_2CH_3$ | 152-159 |
| 1-058 | Cl | O | H | $CH_2CH_2SCH_3$ | 105-115 |
| 1-059 | Me | O | $CH_2CH_3$ | O{D1-108b(4-$NO_2$)} | *1 |
| 1-060 | Me | O | $CH_2CH_3$ | $NHCH_2CH_2SCH_3$ | *1 |
| 1-061 | Me | O | $CH_3$ | $NHCH_2CH_2SCH_3$ | 82-86 |
| 1-062 | Me | O | H | $NHCH_2CH_2SCH_3$ | 145-147 |
| 1-063 | Cl | O | $CH_2CH_2CH_3$ | $CH_2CH_2SCH_3$ | *1 |
| 1-064 | Cl | O | $CH_2CH{=}CH_2$ | $CH_2CH_2SCH_3$ | *1 |
| 1-065 | Cl | O | $CH_2CH_3$ | $CH_2CH(CH_3)SCH_3$ | *1 |
| 1-066 | Cl | O | $CH_2CH_2CH_3$ | $CH(CH_3)CH_2SCH_3$ | *1 |
| 1-067 | Cl | O | $CH_2CH_3$ | $CH_2OCH_2SCH_3$ | *1 |
| 1-068 | Cl | O | $CH_2OCH_3$ | $CH_2CH_2SCH_3$ | *1 |
| 1-069 | Me | O | $CH_3$ | $NHCH(CH_3)_2$ | 130-132 |
| 1-070 | Me | S | $CH_3$ | $NHCH(CH_3)_2$ | 148-150 |
| 1-071 | Cl | O | $CH_2CH_3$ | $CH{=}CHSCH_3$ | 117-118 |
| 1-072 | Me | O | H | $NHCH_2CH_2CH_2SCH_3$ | 60-70 |
| 1-073 | Cl | O | $CH_2CH_3$ | $CH_2CH(SCH_3)_2$ | 76-77 |
| 1-074 | Cl | O | H | $CH(CH_3)CH_2SCH_3$ | 100-110 |
| 1-075 | Cl | O | $CH(CH_3)_2$ | $CH(CH_3)CH_2SCH_3$ | 110-111 |
| 1-076 | Cl | O | $CH_2CH{=}CH_2$ | $CH(CH_3)CH_2SCH_3$ | *1 |
| 1-077 | Cl | O | $CH_2CH_3$ | $CH_2CH_2SC(O)CH_3$ | *1 |
| 1-078 | Cl | O | $CH(CH_3)_2$ | $CH_2CH_2SCH_3$ | *1 |
| 1-079 | Cl | O | $CH_2CH_3$ | $CH_2SCH_3$ | *1 |
| 1-080 | Cl | O | $CH_2CH_3$ | $CH_2S(O)_2CH_3$ | 107-108 |
| 1-081 | Me | O | $CH_3$ | $NHCH_2C(O)OCH_3$ | 129-132 |
| 1-082 | Cl | O | $CH_2CH_3$ | $CH_2OC(O)CH_3$ | 114-116 |
| 1-083 | Cl | O | $CH_2CH_3$ | D1-82b | *1 |

(continued)

| No. | $R^3$ | Y | $R^a$ | $R^{b-1}$ | m.p.(°C) |
|---|---|---|---|---|---|
| 1-084 | Cl | O | $CH_3$ | $CH(CH_2CH_3)CH_2SCH_3$ | 62-63 |
| 1-085 | Cl | O | $CH_2CH_3$ | $CH_2CH_2SCH_2CH_3$ | *1 |
| 1-086 | Cl | O | $CH_2OCH_3$ | $CH(CH_3)CH_2SCH_3$ | *1 |
| 1-087 | Me | O | $CH_3$ | $CH_2CH_2S(O)CH_3$ | *1 |
| 1-088 | Me | O | $CH_3$ | $CH_2CH_2S(O)_2CH_3$ | *1 |
| 1-089 | Me | O | $CH_3$ | $NHC(O)OCH_2CH_3$ | 144-147 |
| 1-090 | Me | O | $CH_3$ | $NHC(Cl)_3$ | 110-112 |
| 1-091 | Me | O | $CH_3$ | $NHS(O)_2$(D1-108a) | 130-133 |
| 1-092 | Cl | O | $CH_2CH_3$ | $C(O)CH_3$ | 73-77 |
| 1-093 | Cl | O | {-C(O)CH=C($CH_3$)-}&{-C(O)$CH_2$C(=$CH_2$)-} | | 115-125(*3) |
| 1-094 | Me | O | $CH_3$ | $NH_2$ | 169-171 |
| 1-095 | Me | O | $CH_3$ | $NHC(O)CH_2SCH_3$ | *1 |
| 1-096 | Cl | O | $CH_2CH_3$ | $CH_2S(O)CH_2CH_3$ | 145-147 |
| 1-097 | Cl | O | $CH_2CH_3$ | $CH_2S(O)_2CH_2CH_3$ | *1 |
| 1-098 | Cl | O | $CH_2CH_3$ | $SCH_3$ | *1 |
| 1-099 | Cl | O | $CH_2CH_3$ | $CH(CH_3)_2$ | 107-111 |
| 1-100 | Cl | O | $CH_2CH_3$ | $CH_2S(O)CH_3$ | 115-116 |
| 1-101 | Cl | O | $CH_2CH_3$ | $CH(CH_3)SCH_3$ | 94-96 |
| 1-102 | Cl | O | $CH_3$ | $CH_2SCH_3$ | *1 |
| 1-103 | Cl | S | $CH_2CH_3$ | $CH(CH_3)CH_2SCH_3$ | *1 |
| 1-104 | Cl | O | $CH_2CH_2CH_3$ | $CH_2CH_2S(O)CH_3$ | *1 |
| 1-105 | Cl | O | $CH_2CH_2CH_3$ | $CH_2CH_2S(O)_2CH_3$ | *1 |
| 1-106 | Me | O | $CH_2CH_3$ | $CH_2SCH_3$ | *1 |
| 1-107 | Cl | O | $CH_2CH_3$ | $CH_2SCH_2CH_3$ | *1 |
| 1-108 | Cl | O | $CH_2CH_3$ | $CH_2Br$ | 220-222 |
| 1-109 | Cl | O | $CH_2CH_3$ | $CH_2SCH_2CH=CH_2$ | *1 |
| 1-110 | Me | O | $CH_2CH_3$ | $CH_2CH_2S(O)CH_3$ | *1 |
| 1-111 | Me | O | $CH_2CH_3$ | $CH_2CH_2S(O)_2CH_3$ | *1 |
| 1-112 | Cl | O | $CH_2CH_3$ | $CH(CH_3)S(O)CH_3$ | *1 |
| 1-113 | Cl | O | $CH_2CH_3$ | $CH(CH_3)S(O)_2CH_3$ | *1 |
| 1-114 | Cl | O | $CH_3$ | $CH_2S(O)CH_3$ | 103-105 |
| 1-115 | Cl | O | $CH_3$ | $CH_2S(O)_2CH_3$ | 140-141 |
| 1-116 | Cl | O | $CH_2CH_3$ | $CH(SCH_3)_2$ | 93-95 |

(continued)

| No. | $R^3$ | Y | $R^a$ | $R^{b-1}$ | m.p.(°C) |
|---|---|---|---|---|---|
| 1-117 | Cl | O | $CH_2CH_3$ | $CH_2CH_2SH$ | *1 |
| 1-118 | Cl | O | $CH_2CH_3$ | $CH(CH_3)SCH_2CH_3$ | 65-66 |
| 1-118(+) | 99%e.e. | | $[\alpha]_D^{23.9}$+11.93°($CHCl_3$,c=0.503) | | 65-66 |
| 1-118(-) | 94.7%e.e. | | $[\alpha]_D^{23.8}$-11.18°($CHCl_3$,c=0.340) | | 65-66 |
| 1-119 | Me | O | $CH_2CH_3$ | $N(CH_3)CH_2CH_2SCH_3$ | *1 |
| 1-120 | Cl | O | $CH_2CH_3$ | $CH(CH_2CH_3)SCH_3$ | *2(~1:1) |
| 1-121 | Cl | O | $CH_2CH_3$ | $CH(F)SCH_3$ | *2(~3:2) |
| 1-122 | Cl | O | $CH_2CH_3$ | $CH_2CH_2SCH_2CH_2CH_3$ | *1 |
| 1-123 | Cl | O | $CH_2CH_3$ | $CH_2CH_2SCH(CH_3)_2$ | *1 |
| 1-124 | Cl | O | $CH_2CH_3$ | $CH_2CH_2SCH_2CF_3$ | *1 |
| 1-125 | Cl | O | $CH_2CH_3$ | $CH\{OC(O)CH_3\}S(O)CH_3$ | *2(~3:1) |
| 1-126 | Cl | O | $CH_2CH_3$ | $CH(F)S(O)_2CH_3$ | 133-135 |
| 1-127 | Cl | O | $CH_2CH_3$ | $CH\{OC(O)CH_3\}S(O)_2CH_3$ | *2(~3:2) |
| 1-128 | Cl | O | H | $CH(Br)CH_2CH_2CH_2Br$ | 119-120 |
| 1-129 | Cl | O | $-CH_2CH_2CH_2CH(Br)-$ | | 211-212 |
| 1-130 | Cl | O | $-CH_2CH_2CH_2CH(SCH_3)-$ | | 118-119 |
| 1-131 | Cl | O | H | $CH_2CH_2CH_2Cl$ | 97-98 |
| 1-132 | Cl | O | $-CH_2CH_2CH_2-$ | | 109-110 |
| 1-133 | Cl | O | $-CH_2CH_2C(=CH_2)-$ | | 117-118 |
| 1-134 | Cl | O | $CH_3$ | $C(CH_3)_2CH_2SCH_3$ | *1 |
| 1-135 | Cl | O | $CH_3$ | $C(=CH_2)CH_2C(O)OH$ | 128-132 |
| 1-136 | Me | O | $-C(O)CH(CH_2SCH_3)NH-$ | | 231-233 |
| 1-137 | Me | O | $-C(O)CH\{CH_2S(O)_2CH_3\}NH-$ | | 200-205 |
| 1-138 | Me | O | $CH_3$ | $NHCH_2CH_3$ | 148-150 |
| 1-139 | Cl | O | $CH_3$ | $C(CH_3)_2CH_2S(O)_2CH_3$ | 130-135 |
| 1-140 | Cl | O | $CH_2CH_3$ | $C(CH_3)_2SCH_3$ | 116-117 |
| 1-141 | Cl | O | $-CH_2CH_2CH(CH_2SCH_3)-$ | | 83-84 |
| 1-142 | Cl | O | $CH_3$ | $C(=NOCH_3)CH_2SCH_3$ | *1 |
| 1-143 | Cl | O | $CH_2CH_3$ | $C(=NOCH_3)CH_2SCH_3$ | *1 |
| 1-144 | Cl | O | $CH_2C\equiv CH$ | $CH_2CH_2SCH_3$ | *1 |
| 1-145 | Cl | O | $-CH_2CH_2CH\{CH_2S(O)_2CH_3\}-$ | | 198-199 |
| 1-146 | Cl | O | $CH_2C\equiv CH$ | $CH_2CH_2S(O)_2CH_3$ | *1 |
| 1-147 | Cl | O | $CH_2C\equiv CH$ | $CH(CH_3)CH_2SCH_3$ | *1 |

(continued)

| No. | $R^3$ | Y | $R^a$ | $R^{b-1}$ | m.p.(°C) |
|---|---|---|---|---|---|
| 1-148 | Cl | O | $CH_3$ | $CH_3$ | 55-58 |
| 1-149 | Cl | O | $CH_2CH_3$ | $CH_3$ | *1 |
| 1-150 | Cl | O | $-CH_2CH_2CH_2CH\{S(O)_2CH_3\}-$ | | 207-208 |
| 1-151 | Cl | O | $CH_2CH_3$ | $CH_2CH_2CH_3$ | *1 |
| 1-152 | Cl | O | $CH_2CH_3$ | $CH=CH_2$ | 67-68 |
| 1-153 | Cl | O | $CH_2CH_2CH_2SCH_3$ | $CH_3$ | *1 |
| 1-154 | Cl | O | $CH_2CH_3$ | $CH_2CH_2S(O)CH_2CF_3$ | 96-97 |
| 1-155 | Cl | O | $CH_2CH_3$ | $CH_2CH_2S(O)_2CH_2CF_3$ | *1 |
| 1-156 | Cl | O | $CH_2CH_3$ | $CH(SCH_3)CH_2SCH_3$ | *1 |
| 1-157 | Cl | O | $CH_2CH_3$ | $C(=NOCH_2CH_3)CH_2SCH_3$ | *1 |
| 1-158 | Cl | O | H | $NHCH_2CF_3$ | 228-230 |
| 1-159 | Cl | O | $CH_2CH_3$ | $C(=CH_2)CH_2SCH_3$ | *2(~3:2) |
| 1-160 | Cl | O | $CH_2CH_3$ | $CH(OCH_3)CH_2SCH_3$ | *2(~3:2) |
| 1-161 | Cl | O | $CH_2CH_3$ | $C(=CH_2)CH_2SCH_2CH_3$ | *1 |
| 1-162 | Cl | O | $CH_2CH_3$ | $C(=NOCH_3)CH_2S(O)CH_3$ | *2(~4:1) |
| 1-163 | Cl | O | $CH_2CH_3$ | $C(=NOCH_3)CH_2S(O)_2CH_3$ | 130-133 |
| 1-164 | Cl | O | H | $CH_2SCH_3$ | 183-184 |
| 1-165 | H | O | $NH_2$ | $OC(CH_3)_3$ | 120-123 |
| 1-166 | H | O | $NHC(O)CH_2SCH_3$ | $OC(CH_3)_3$ | *1 |
| 1-167 | Cl | O | $CH_3$ | $CH(SCH_3)CH_2SCH_3$ | *1 |
| 1-168 | H | S | | $-CH_2CH_2CH_2-$ | 83-84 |
| 1-169 | Me | O | $CH_3$ | $N(CH_2CH_3)C(O)CH_2SCH_3$ | *1 |
| 1-170 | H | $NOCH_3$ | | $-CH_2CH_2CH_2-$ | 75-76 |
| 1-171 | H | $NOCH_3$ | | $-CH_2CH_2CH_2-$ | 55-56(*4) |
| 1-172 | Cl | O | $CH_2CH_3$ | $C\{=NOCH(CH_3)_2\}CH_2SCH_3$ | *1 |
| 1-173 | Cl | O | $CH_2CH_3$ | $C(=NOCH_2CH_2CH_3)CH_2SCH_3$ | *1 |
| 1-174 | Cl | O | $CH_2CH_3$ | $CH_2C(O)OCH_3$ | 102-103 |
| 1-175 | Cl | O | $CH_3$ | $CH_2CN$ | 145-154 |
| 1-176 | Cl | O | $CH_3$ | $CH(Cl)CH_2SCH_3$ | 113-114 |
| 1-177 | Cl | O | $CH_3$ | $CH_2CH_2SCH_2CF_3$ | *1 |
| 1-178 | Cl | O | $CH_3$ | $CH_2CH_2S(O)CH_2CF_3$ | *1 |
| 1-179 | Cl | O | $CH_3$ | $CH_2CH_2S(O)_2CH_2CF_3$ | *1 |
| 1-180 | Me | O | | $-C(O)CH_2CH_2-$ | 155-156 |

(continued)

| No. | $R^3$ | Y | $R^a$ | $R^{b-1}$ | m.p.(°C) |
|---|---|---|---|---|---|
| 1-181 | Cl | O | $CH_2CH_3$ | $CH\{C(O)OCH_3\}CH_2SCH_2CH_3$ | *1 |
| 1-182 | Cl | O | $CH_2CH_3$ | $CH(CH_2SCH_3)CH_2SCH_3$ | *1 |
| 1-183 | Cl | O | $CH_2CH_3$ | $C(=NOCH_2Ph)CH_2SCH_3$ | *1 |
| 1-184 | Cl | O | $CH_2CH_3$ | $C(=NOCH_2CH_2CH_2CH_3)CH_2SCH_3$ | *1 |
| 1-185 | Cl | O | $CH_3$ | $CH(CN)CH_2SCH_2CH_3$ | *1 |
| 1-186 | Cl | O | $CH_2CH_2SCH_3$ | $CH_3$ | *1 |
| 1-187 | Cl | O | | $-CH_2C(OCH_3)_2-$ | 135-138 |
| 1-188 | Br | O | H | $OC(CH_3)_3$ | 109-112 |
| 1-189 | Br | O | $CH_2CH_3$ | $OC(CH_3)_3$ | 84-86 |
| 1-190 | Cl | O | $CH_3$ | $N(CH_3)CH_2CF_3$ | *1 |
| 1-191 | Cl | O | $CH_3$ | $C(=NOCH_3)CH_3$ | *1 |
| 1-192 | Cl | O | $CH_2CH_3$ | $CH(Cl)CH_2S(O)_2CH_3$ | 162-163 |
| 1-193 | Me | O | $CH_3$ | $C(=NOCH_3)CH_2SCH_3$ | *1 |
| 1-194 | Me | O | $CH_2CH_3$ | $C(=NOCH_3)CH_2SCH_3$ | 71-73 |
| 1-195 | Cl | O | $CH_2CH_3$ | $CH(CN)CH_2SCH_2CH_3$ | *1 |
| 1-196 | Cl | O | $CH_3$ | $NHCH_2CF_3$ | 199-201 |
| 1-197 | H | O | $N(CH_3)C(O)CH(CH_3)SCH_3$ | $OC(CH_3)_3$ | *2(~10:7) |
| 1-198 | Br | O | $CH_2CH_3$ | $CH_2CH_2SCH_3$ | *1 |
| 1-199 | Cl | O | $CH_2CH_3$ | $CH(OCH_3)CH_2S(O)CH_3$ | *2(~3:1) |
| 1-200 | Cl | O | $CH_2CH_3$ | $CH(OCH_3)CH_2S(O)_2CH_3$ | 174-176 |
| 1-201 | Cl | O | $CH_2CH_3$ | $CH(CH_3)CH_2SCH_2CF_3$ | *1 |
| 1-202 | Br | O | $CH_2CH_3$ | $CH(CH_3)CH_2SCH_3$ | *1 |
| 1-203 | Br | O | $CH_2CH_3$ | $CH_2CH_2S(O)CH_3$ | *1 |
| 1-204 | Br | O | $CH_2CH_3$ | $CH_2CH_2S(O)_2CH_3$ | *1 |
| 1-205 | Br | O | $CH_2CH_3$ | $CH(CH_3)CH_2S(O)CH_3$ | *2(~1:1) |
| 1-206 | Br | O | $CH_2CH_3$ | $CH(CH_3)CH_2S(O)_2CH_3$ | *1 |
| 1-207 | H | O | $N(CH_3)C(O)CH_2SCH_3$ | $OC(CH_3)_3$ | 91-92 |
| 1-208 | Cl | O | $CH_2CH_3$ | $C(=NOCH_2CH_3)CH_2S(O)CH_3$ | *1 |
| 1-209 | Cl | O | $CH_2CH_3$ | $C(=NOCH_2CH_3)CH_2S(O)_2CH_3$ | *1 |
| 1-210 | Cl | O | $CH_2CH_3$ | $CH(CH_3)SCH_2CF_3$ | *1 |
| 1-211 | Cl | O | $CH_2CH_3$ | $C(=NOCH_3)CH_3$ | *1 |
| 1-212 | Cl | O | $CH_2CH_3$ | Pr-c | 89-91 |
| 1-213 | Cl | O | $CH_2CH_3$ | D1-103b | *1 |

(continued)

| No. | $R^3$ | Y | $R^a$ | $R^{b-1}$ | m.p.(°C) |
|---|---|---|---|---|---|
| 1-214 | Cl | O | $CH_2CH_3$ | D1-103c | *1 |
| 1-215 | Cl | O | $CH_2CH_3$ | D1-103d | *1 |
| 1-216 | Cl | O | $CH_2CH_3$ | $CH(Ph)CH_2SCH_3$ | *1 |
| 1-217 | Cl | O | $CH_2CH_3$ | $CH(Ph)CH_2S(O)CH_3$ | *2(~1:1) |
| 1-218 | Cl | O | $CH_2CH_3$ | $CH(Ph)CH_2S(O)_2CH_3$ | *1 |
| 1-219 | Cl | O | $CH_2CH_3$ | $CH(CH_3)CH_2SC(O)CH_3$ | *1 |
| 1-220 | Cl | O | $CH_2CH_3$ | $CH_2OCH_2CH_3$ | 93-96 |
| 1-221 | Cl | O | $CH_2CH_3$ | $C(=NOCH_3)CH_2SCH_2CF_3$ | *1 |
| 1-222 | Cl | O | $CH_2CH_3$ | $C(=NOCH_2CH=CH_2)CH_2SCH_3$ | *1 |
| 1-223 | Cl | O | $CH_2CH_3$ | $C(=NOCH_2CH_2OCH_3)CH_2SCH_3$ | *1 |
| 1-224 | Cl | O | $CH_2CH_3$ | $CH(Br)CH_3$ | 224-225 |
| 1-225 | Cl | O | $CH_2CH_3$ | $CH(CH_3)S(O)CH_2CH_3$ | *2(~3:1) |
| 1-226 | Cl | O | $CH_2CH_3$ | $CH(CH_3)S(O)_2CH_2CH_3$ | *1 |
| 1-227 | Cl | O | $CH_3$ | $C(=NOCH_3)CH_2S(O)_2CF_3$ | *1 |
| 1-228 | Cl | O | $CH_2CH_3$ | $C(=NOCH_3)CH_2S(O)_2CF_3$ | *1 |
| 1-229 | Cl | O | $CH_2CH_3$ | $CH(CH_3)CH_2S(O)CH_2CF_3$ | *2(~3:1) |
| 1-230 | Cl | O | $CH_2CH_3$ | $CH(CH_3)CH_2S(O)_2CH_2CF_3$ | *1 |
| 1-231 | Cl | O | $CH_3$ | $CH(CH_3)CH_2SCH_2CF_3$ | *1 |
| 1-232 | Cl | O | $CH_2CH_3$ | $CH_2SCH_2CF_3$ | *1 |
| 1-233 | Cl | O | $CH_2CH_3$ | $C(=NOCH_3)CH_2S(O)CH_2CF_3$ | *1 |
| 1-234 | Cl | O | $CH_2CH_3$ | $C(=NOCH_3)CH_2S(O)_2CH_2CF_3$ | *1 |
| 1-235 | Cl | O | $CH_3$ | $C(=NOCH_3)CH_2SCH_2CF_3$ | *1 |
| 1-236 | Cl | O | $CH_3$ | $C(=NOCH_3)CH_2S(O)CH_2CF_3$ | *1 |
| 1-237 | Cl | O | $CH_3$ | $C(=NOCH_3)CH_2S(O)_2CH_2CF_3$ | *1 |
| 1-238 | H | O | | $-N=C(CH_2SCH_3)O-$ | 130-131 |
| 1-239 | Cl | O | $CH_3$ | $CH(CH_3)SCH_2CH_3$ | 108-109 |
| 1-240 | Cl | O | $CH_3$ | $CH(CH_3)SCH_2CF_3$ | *1 |
| 1-241 | Cl | O | $CH_3$ | $CH(CH_3)S(O)_2CH_2CF_3$ | *1 |
| 1-242 | Cl | O | $CH_3$ | $CH_2SCH_2CH_3$ | *1 |
| 1-243 | Cl | O | $CH_3$ | $CH_2S(O)CH_2CH_3$ | *1 |
| 1-244 | Cl | O | $CH_3$ | $CH_2S(O)_2CH_2CH_3$ | 155-156 |
| 1-245 | Cl | O | $CH_2CH_3$ | $CH(CH_3)SC(O)CH_3$ | *1 |
| 1-246 | Cl | O | $CH_2CH_3$ | $CH(CH_3)SCH_2CH_2CH_3$ | *1 |

(continued)

| No. | $R^3$ | Y | $R^a$ | $R^{b-1}$ | m.p.(°C) |
|---|---|---|---|---|---|
| 1-247 | Cl | O | $CH_2CH_3$ | $CH(CH_3)S(O)_2CH(CH_3)_2$ | 137-138 |
| 1-248 | Cl | O | $CH_2CH_3$ | $CH(CH_3)SCH(CH_3)_2$ | *1 |
| 1-249 | Cl | O | $CH_2CH_3$ | $CH(CH_3)S(O)_2CH_2CH_2CH_3$ | 114-115 |
| 1-250 | Cl | O | $CH_2CH_3$ | $CH(CH_3)S$(D1-37a) | 126-128 |
| 1-251 | Cl | O | $CH_2CH_3$ | $CH(CH_3)SCN$ | *1 |
| 1-252 | Cl | O | $CH_2CH_3$ | $CH(CH_3)SCH_2CN$ | *1 |
| 1-253 | Cl | O | $CH_2CH_3$ | $CH(CH_3)S(O)CH_2CF_3$ | *2(~54:46) |
| 1-254 | Cl | O | $CH_2CH_3$ | $CH(CH_3)S(O)_2CH_2CF_3$ | 119-121 |
| 1-255 | Cl | O | $CH_3$ | $CH(CH_3)SCH_3$ | 111-112 |
| 1-256 | H | O | $N\{C(O)OC(CH_3)_3\}_2$ | $OC(CH_3)_3$ | *1 |
| 1-257 | H | O | $NHC(O)OC(CH_3)_3$ | $OC(CH_3)_3$ | *1 |
| 1-258 | H | O | $NHC(O)CH_2CH_2SCH_3$ | $OC(CH_3)_3$ | 129-130 |
| 1-259 | Cl | O | $CH_3$ | $C(=NOCH_3)CH_2SCH_2CH_3$ | *1 |
| 1-260 | Cl | O | $CH_3$ | $C(=NOCH_3)CH_2S(O)CH_2CH_3$ | *2(~3:2) |
| 1-261 | Cl | O | $CH_3$ | $C(=NOCH_3)CH_2S(O)_2CH_2CH_3$ | 105-110 |
| 1-262 | Cl | O | $CH_2CH_3$ | $C(=NOCH_3)CH_2SCH_2CH_3$ | *1 |
| 1-263 | Cl | O | $CH_2CH_3$ | $C(=NOCH_3)CH_2S(O)CH_2CH_3$ | *2(~4:1) |
| 1-264 | Cl | O | $CH_2CH_3$ | $C(=NOCH_3)CH_2S(O)_2CH_2CH_3$ | 110-115 |
| 1-265 | Cl | O | $CH_3$ | $CH(CH_3)S(O)CH_2CH_3$ | 129-130 |
| 1-266 | Cl | O | $CH_3$ | $CH(CH_3)S(O)_2CH_2CH_3$ | 167-168 |
| 1-267 | Cl | O | $CH_2CH_3$ | $C(=NOCH_2OCH_3)CH_2SCH_3$ | *1 |
| 1-268 | Cl | O | $CH_2CH_3$ | $C\{=NOCH_2CH_2Si(CH_3)_3\}CH_2SCH_3$ | *1 |
| 1-269 | Cl | O | $CH_2CH_3$ | $CH(CH_2CH_3)S(O)CH_3$ | 105-106 |
| 1-270 | Cl | O | $CH_3$ | $CH(CH_3)S(O)CH_3$ | *2(~54:46) |
| 1-271 | Cl | O | $CH_3$ | $CH(CH_3)S(O)_2CH_3$ | 151-152 |
| 1-272 | Cl | O | $CH_2CH_3$ | $CH(CH_3)OCH_2CF_3$ | 99-101 |
| 1-273 | Cl | O | $CH_3$ | $C(=NOCH_3)CH_2OCH_2CF_3$ | *1 |
| 1-274 | Cl | O | $CH_2CH_3$ | $C(=NOCH_3)CH_2OCH_2CF_3$ | *1 |
| 1-275 | Cl | O | H | $CH(CH_3)SCH_2CH_3$ | 104-105 |
| 1-276 | Cl | O | $CH_2OCH_3$ | $CH(CH_3)SCH_2CH_3$ | 68-69 |
| 1-277 | Cl | O | $CH_2CH_3$ | $CH(CH_3)SCH_2OCH_3$ | *1 |
| 1-278 | Cl | O | $CH_2CH_3$ | $CH(CH_3)SCH_2$Pr-c | *1 |
| 1-279 | Cl | O | $CH_2CH_3$ | $CH(CH_3)ON=CHCH_3$ | *1 |

(continued)

| No. | $R^3$ | Y | $R^a$ | $R^{b-1}$ | m.p.(°C) |
|---|---|---|---|---|---|
| 1-280 | Cl | O | $CH_2CH_3$ | $CH(CH_2CH_3)S(O)_2CH_3$ | 116-117 |
| 1-281 | Cl | O | $CH_2CH_3$ | $CH(CH_2CH_3)SCH_2CH_3$ | 67-68 |
| 1-282 | Cl | O | $CH_2CH_3$ | $CH(CH_2CH_3)S(O)CH_2CH_3$ | 109-110 |
| 1-283 | Cl | O | $CH_2CH_3$ | $CH(CH_2CH_3)S(O)_2CH_2CH_3$ | 89-90 |
| 1-284 | Cl | O | $CH_2CN$ | $CH(CH_3)SCH_2CH_3$ | *1 |
| 1-285 | Cl | O | $CH_2CH_3$ | $CH_2S(O)_2CH_2CF_3$ | *1 |
| 1-286 | Cl | O | $CH_2CH_3$ | $CH(CH_3)SCH_2SCH_3$ | *1 |
| 1-287 | Cl | O | $CH_2CH_3$ | $CH(CH_3)SCH_2C\equiv CH$ | 82-83 |
| 1-288 | Cl | O | $CH_3$ | $CH_2SCH_2CF_3$ | *1 |
| 1-289 | Cl | O | $CH_2C\equiv CH$ | $CH(CH_3)SCH_2CH_3$ | *1 |
| 1-290 | Br | O | $CH_2CH_3$ | $CH(CH_3)SCH_3$ | 93-95 |
| 1-291 | Br | O | $CH_2CH_3$ | $CH(CH_3)SCH_2CH_3$ | *1 |
| 1-292 | Cl | O | $CH_2CH_3$ | $CH(CH_3)SCH_2C(O)NHCH_3$ | *1 |
| 1-293 | Cl | O | $CH_2CH_3$ | $CH(CH_3)SCH_2C(O)OCH_3$ | *1 |
| 1-294 | Cl | O | $CH_3$ | $CH(CH_2CH_3)SCH_3$ | 86-87 |
| 1-295 | Cl | O | $CH_3$ | $CH(CH_2CH_3)SCH_2CH_3$ | 76-77 |
| 1-296 | Cl | O | $CH_2CH_3$ | $CH_2S(O)CH_2CF_3$ | 114-116 |
| 1-297 | Cl | O | $CH_3$ | $CH(CH_2CH_3)S(O)CH_3$ | 84-86 |
| 1-298 | Cl | O | $CH_3$ | $CH(CH_2CH_3)S(O)_2CH_3$ | 168-170 |
| 1-299 | Cl | O | $CH_3$ | $CH(CH_2CH_3)S(O)_2CH_2CH_3$ | 93-95 |
| 1-300 | Cl | O | $CH_2C(O)OCH_2CH_3$ | $CH(CH_3)SCH_2CH_3$ | *1 |
| 1-301 | Cl | O | $S(O)_2CH_3$ | $CH(CH_3)SCH_2CH_3$ | 125-127 |
| 1-302 | Cl | O | $C(O)OCH_3$ | $CH(CH_3)SCH_2CH_3$ | *1 |
| 1-303 | Cl | O | $CH_2CH_3$ | $CH_2C(=NOMe)CF_3$ | *1 |
| 1-304 | Cl | O | $CH_3$ | $CH_2S(O)_2CH_2CF_3$ | *1 |
| 1-305 | Cl | O | $CH_2CH_3$ | $CH(CH_3)S(=NCN)CH_2CH_3$ | *1 |
| 1-306 | Cl | O | $CH_2CH_3$ | D1-82a | 107-109 |
| 1-307 | Cl | O | $CH_2CH_3$ | D1-103e | *1 |
| 1-308 | Cl | O | $CH_3$ | $CH_2NHC(O)OC(CH_3)_3$ | *1 |
| 1-309 | Cl | O | $CH_2OCH_3$ | $CH(CH_3)S(O)CH_2CH_3$ | *1 |
| 1-310 | Cl | O | $CH_2OCH_3$ | $CH(CH_3)S(O)_2CH_2CH_3$ | *1 |
| 1-311 | Cl | O | $CH_3$ | $CH_2S(O)CH_2CF_3$ | 55-58 |
| 1-312 | Cl | O | $CH_3$ | $CH_2NH_2$ HCl | 168-180 |

(continued)

| No. | $R^3$ | Y | $R^a$ | $R^{b-1}$ | m.p.(°C) |
|---|---|---|---|---|---|
| 1-313 | Cl | O | $CH_3$ | $CH_2NHS(O)_2CH_3$ | *1 |
| 1-314 | Cl | O | $CH_3$ | $CH(CH_2CH_3)S(O)CH_2CH_3$ | *2(~52:48) |
| 1-315 | Cl | O | $CH_2CH_3$ | $CH(CH_3)SC(CH_3)_3$ | *1 |
| 1-316 | Cl | O | $CH_2CH=CH_2$ | $CH(CH_3)SCH_2CH_3$ | 70-74 |
| 1-317 | Cl | O | $CH_2CH_2CH_3$ | $OC(CH_3)_3$ | 85-87 |
| 1-318 | Cl | S | $CH_2CH_3$ | $CH(CH_3)SCH_2CH_3$ | *1 |
| 1-319 | Cl | O | $SC(Cl)_3$ | $CH(CH_3)SCH_2CH_3$ | *1 |
| 1-320 | Cl | O | $CH_2CH_3$ | $CH(CH_3)SCH_2$(D1-34a) | *1 |
| 1-321 | Cl | O | $CH_2CH_3$ | $CH(CH_3)SCH_2Si(CH_3)_3$ | 98-100 |
| 1-322 | Cl | O | $CH_2CH_3$ | D1-82c | 192-195 |
| 1-323 | Cl | O | $CH_2CH_2CH_3$ | $CH(CH_3)SCH_2CH_3$ | 73-76 |
| 1-324 | Cl | O | $CH_2CF_3$ | $OC(CH_3)_3$ | *1 |
| 1-325 | Cl | O | $CH_3$ | $CH_2N(CH_3)S(O)_2CH_3$ | 156-158 |
| 1-326 | Cl | O | $CH_3$ | D1-10e(2-$CF_3$) | 151-153 |
| 1-327 | Cl | O | $CH_3$ | D1-19a | 117-119 |
| 1-328 | Cl | O | $CH_3$ | D1-32b(2-Br) | *1 |
| 1-329 | Cl | O | $CH_3$ | $CH_2$(D1-2a) | 96-98 |
| 1-330 | Cl | O | $CH_3$ | $CH_2${D1-8f[$X^{1a}$=$CH_3$,$X^1$=3,5-$(CH_3)_2$]} | 150-152 |
| 1-331 | Cl | O | $CH_3$ | $CH_2${D1-33b(6-Cl)} | *1 |
| 1-332 | Cl | O | H | $CH_2$(D1-32a) | 154-155 |
| 1-333 | Cl | O | $CH_3$ | D1-1b(5-Br) | *1 |
| 1-334 | Cl | O | $CH_3$ | D1-33b(2-$OCH_3$) | 164-166 |
| 1-335 | Cl | O | $CH_3$ | D1-10f{2-($CH_2SCH_3$),4-$CH_3$} | *1 |
| 1-336 | Cl | O | $CH_3$ | D1-38b(4-$SCH_3$) | *1 |
| 1-337 | Cl | O | $CH_3$ | $CH_2$(D1-33a) | *1 |
| 1-338 | Cl | O | $CH_3$ | $CH_2$(D1-34a) | 121-125 |
| 1-339 | Cl | O | $CH_3$ | $CH_2$(D1-28a) | 190-198 |
| 1-340 | Cl | O | $CH_2CH_3$ | $CH(CH_3)SCH_2CH_2CH_2Cl$ | *1 |
| 1-341 | Cl | O | $CH_2CF_3$ | $CH(CH_3)CH_2SCH_3$ | *1 |
| 1-342 | Cl | O | $CH_2CF_3$ | $CH(CH_3)SCH_2CH_3$ | *1 |
| 1-343 | Cl | O | $CH_2$Pr-c | $OC(CH_3)_3$ | *1 |
| 1-344 | Cl | O | $CH_3$ | D1-2c(5-$SCH_3$) | *1 |
| 1-345 | Cl | O | $CH_3$ | D1-12f($X^{1a}$=$CH_3$,$X^1$=4-$NO_2$) | 125-131 |

(continued)

| No. | $R^3$ | Y | $R^a$ | $R^{b-1}$ | m.p.(°C) |
|---|---|---|---|---|---|
| 1-346 | Cl | O | $CH_3$ | D1-35b | *1 |
| 1-347 | Cl | O | $CH_3$ | $CH_2${D1-5f(3-$CH_3$)} | 157-159 |
| 1-348 | Cl | O | $CH_3$ | D1-5e{3,5-$(CH_3)_2$} | *1 |
| 1-349 | Cl | O | $CH_2CH_3$ | D1-13d($CH_3$) | *1 |
| 1-350 | Cl | O | $CH_2CH_3$ | $CH_2${D1-8f[$X^{1a}$=$CH_3$,$X^1$=3,5-$(Cl)_2$]} | *1 |
| 1-351 | Cl | O | $CH_2CH_3$ | $CH_2CH_2${D1-7b(3-$CF_3$,5-Pr-c)} | *1 |
| 1-352 | Cl | O | $CH_2CH_3$ | $CH_2P(=O)(OCH_2CH_3)_2$ | *1 |
| 1-353 | Cl | S | H | $CH(CH_3)SCH_2CH_3$ | *1 |
| 1-354 | Cl | O | $CH_2CH_3$ | $CH(CH_3)S$(D1-51a) | *1 |
| 1-355 | Cl | O | $CH_2CH_3$ | D1-45d{5,5-$(CH_3)_2$} | *1 |
| 1-356 | Cl | $NOCH_3$ | H | $CH(CH_3)SCH_2CH_3$ | *2(~5:2) |
| 1-357 | Cl | O | $CH_2CH_3$ | $C{\equiv}CSi(CH_3)_3$ | 94-95 |
| 1-358 | Cl | O | $CH_2CH_3$ | D1-88a{$X^{1a}$=$C(O)OC(CH_3)_3$} | 54-55 |
| 1-359 | Cl | O | $CH_2CH_3$ | $C{\equiv}CH$ | *1 |
| 1-360 | Me | O | $CH_2CH_3$ | $CH(CH_3)SCH_2CH_3$ | *1 |
| 1-361 | Cl | O | $CH_2CH_3$ | D1-6d(4,5-$Cl_2$) | *1 |
| 1-362 | Cl | O | $CH_2CH_3$ | D1-2c{5-$C(O)CH_3$} | *1 |
| 1-363 | Cl | O | $CH_2CH_3$ | $CH_2CH_2${D1-7b(3-$CF_3$,5-$CH_3$)} | *1 |
| 1-364 | H | O | $CH_3$ | Pr-c | 62-66 |
| 1-365 | Cl | O | $CH_2CH_3$ | $CH_2NHC(O)OC(CH_3)_3$ | 67-69 |
| 1-366 | Cl | O | $CH_2CH_3$ | D1-88a{$X^{1a}$=$C(O)CH_3$} | 140-141 |
| 1-367 | Cl | O | $CH_2CH_3$ | $CH_2C(=NOCH_3)CH_2SCH_3$ | *2(~65:35) |
| 1-368 | Cl | O | $CH_2CH_3$ | D1-87a | 149-150 |
| 1-369 | Cl | O | $CH_2$Pr-c | $CH(CH_3)SCH_2CH_3$ | 75-80 |
| 1-370 | Me | O | $CH_2CH_3$ | $CH(CH_3)S(O)_2CH_2CH_3$ | *1 |
| 1-371 | Cl | O | $CH_2$Pr-c | $CH(CH_3)CH_2SCH_3$ | *1 |
| 1-372 | Cl | O | $CH_2CH_3$ | $CH_2C(O)NHCH_3$ | 91-93 |
| 1-373 | Cl | O | $CH_2CH_3$ | D1-2c{5-$C(=NOCH_3)CH_3$} | *2(~4:3) |
| 1-374 | $OCH_3$ | O | H | $OC(CH_3)_3$ | 141-143 |
| 1-375 | Cl | O | $CH_2CH_3$ | $CH_2NHC(O)NHCH_2CH_3$ | 160-162 |
| 1-376 | Cl | O | $CH_2CH_3$ | $CH_2NHC(O)CH_2OCH_3$ | *1 |
| 1-377 | Cl | O | $CH_2CH_3$ | D1-33b(6-CN) | *1 |
| 1-378 | Cl | O | $CH_2CH_3$ | C(O)(D1-2a) | *1 |

(continued)

| No. | $R^3$ | Y | $R^a$ | $R^{b-1}$ | m.p.(°C) |
|---|---|---|---|---|---|
| 1-379 | Cl | O | $CH_2CH_3$ | D1-81a | 81-83 |
| 1-380 | Cl | O | $CH_2CH_3$ | D1-81b | *1 |
| 1-381 | Cl | O | $CH_2CH_3$ | $CH_2CH_2CH_2CH_2Br$ | 109-110 |
| 1-382 | Cl | O | | $-CH_2CH_2CH_2CH_2-$ | 125-126 |
| 1-383 | Cl | O | $CH_2OCH_2CH_3$ | $CH(CH_3)SCH_2CH_3$ | *1 |
| 1-384 | Cl | O | $CH_2OCH_2CH_3$ | $CH(CH_3)S(O)_2CH_2CH_3$ | 128-129 |
| 1-385 | Cl | O | $CH_2CH_2OCH_3$ | $OC(CH_3)_3$ | 75-76 |
| 1-386 | Cl | O | $CH_2CH_2OCH_3$ | $CH(CH_3)SCH_2CH_3$ | *1 |
| 1-387 | Cl | O | $CH_2CH_2OCH_3$ | $CH(CH_3)S(O)_2CH_2CH_3$ | *1 |
| 1-388 | Cl | O | $CH_2CH_3$ | $CH(CH_3)SCH_2Ph$ | *1 |
| 1-389 | Cl | O | $CH_2CH_3$ | $CH_2$(D1-87a) | 117-118 |
| 1-390 | Cl | O | $CH_2CH_3$ | $CH_2$(D1-93a) | *1 |
| 1-391 | Cl | O | $CH_2CH_3$ | $CH_2NHC(O)CH_2SCH_3$ | 125-126 |
| 1-392 | Cl | O | $CH_2CH_3$ | $CH_2NH_2$ | 140-143 |
| 1-393 | Cl | O | $CH_2CH_3$ | D1-88a{$X^{1a}$=C(O)$CF_3$} | 135-137 |
| 1-394 | Cl | O | $CH_2CH_3$ | $CH_2NHC(O)CH_3$ | 128-129 |
| 1-395 | $OCH_3$ | O | $CH_2CH_3$ | $OC(CH_3)_3$ | *1 |
| 1-396 | Cl | O | $CH_2Si(CH_3)_3$ | $OC(CH_3)_3$ | 80-83 |
| 1-397 | Cl | O | $CH_2CH_3$ | $CH(CH_3)S${D1-32b(3-$CF_3$)} | *1 |
| 1-398 | Cl | O | $CH_2C(=NOCH_3)CH_3$ | $OC(CH_3)_3$ | 97-98 |
| 1-399 | Cl | O | $CH_2C(=NOCH_3)CH_3$ | $CH(CH_3)CH_2SCH_3$ | *2(~9:1) |
| 1-400 | Cl | O | $CH_2Si(CH_3)_3$ | $CH(CH_3)CH_2SCH_3$ | *1 |
| 1-401 | Cl | O | $CH_2Si(CH_3)_3$ | $CH(CH_3)SCH_2CH_3$ | 66-70 |
| 1-402 | Cl | O | $CH_2CH_3$ | D1-94c{$X^{1a}$=C(O)OC$(CH_3)_3$} | *1 |
| 1-403 | Cl | O | $CH_2CH_3$ | D1-94b{$X^{1a}$=C(O)OC$(CH_3)_3$} | *1 |
| 1-404 | Cl | O | $CH_2CH_3$ | D1-10f{2-$CH_2OCH_3$,4-$CF_3$} | *1 |
| 1-405 | Cl | O | $CH_2CH_3$ | D1-8f{$X^{1a}$=$CH_3$,$X^1$=3-Cl,5-$S(O)_2NH_2$} | *1 |
| 1-406 | Cl | O | $CH_2CH_3$ | D1-4a($X^{1a}$=$CH_3$) | *1 |
| 1-407 | Cl | O | $CH_2CH_3$ | $CH_2NHS(O)_2N(CH_3)_2$ | *1 |
| 1-408 | Cl | O | $CH_2CH_3$ | $CH_2NHC(S)NHCH_2CH_3$ | 162-164 |
| 1-409 | Cl | O | $CH_2CH_3$ | $CH_2NHC(O)CF_3$ | *1 |
| 1-410 | Cl | O | $CH_2CH_3$ | $CH_2NHS(O)_2CF_3$ | 140-145 |
| 1-411 | Cl | O | $CH_2CH_3$ | $CH_2N(CH_3)_2$ | *1 |

(continued)

| No. | $R^3$ | Y | $R^a$ | $R^{b-1}$ | m.p.(°C) |
|---|---|---|---|---|---|
| 1-412 | Cl | O | | $-CH_2CH_2CH=CH-$ | 109-110 |
| 1-413 | Cl | O | H | $NHCH=NOCH_3$ | >280 |
| 1-414 | Cl | O | H | $N=CHN(CH_3)_2$ | 105-110 |
| 1-415 | Cl | O | H | $NH_2$ | >280 |
| 1-416 | Cl | O | $CH_2CH_3$ | $CH(CH_3)NHC(O)OC(CH_3)_3$ | 193-195 |
| 1-417 | Cl | O | $CH_2CH_3$ | $CH_2N(CH_3)C(O)OC(CH_3)_3$ | *1 |
| 1-418 | Cl | O | $CH_2CH_3$ | $CH_2CH_2C(F)=CF_2$ | *1 |
| 1-419 | Cl | O | $CH_2CH_3$ | $CH_2${D1-108b(2,3-$OCH_2O$-)} | 85-89 |
| 1-420 | Cl | O | $CH_2CH_3$ | $CH_2CH_2NHC(O)OC(CH_3)_3$ | *1 |
| 1-421 | Cl | O | $CH_2CH_3$ | CH=CH(D1-33a) | *1 |
| 1-422 | Cl | O | $CH_2CH_3$ | CH=CH(D1-1a) | *1 |
| 1-423 | Cl | O | $CH_2CH_3$ | CH=CH(D1-2a) | *1 |
| 1-424 | Cl | O | $CH_2CH_3$ | $C(O)OCH_2CH_3$ | *1 |
| 1-425 | Cl | O | $CH_2CH_3$ | Pen-c | 76-78 |
| 1-426 | Cl | O | $CH_2CH_3$ | $C(CH_3)_2OC(O)CH_3$ | *1 |
| 1-427 | Cl | O | $CH_2CH_3$ | $CF_2CF_2Cl$ | *1 |
| 1-428 | $CF_3$ | O | H | $OC(CH_3)_3$ | 86-88 |
| 1-429 | $CF_3$ | O | $CH_2CH_3$ | $OC(CH_3)_3$ | *1 |
| 1-430 | Cl | O | $CH_2SCH_3$ | $CH(CH_3)SCH_2CH_3$ | *1 |
| 1-431 | Cl | O | $CH_2CH_3$ | $CH(CH_3)S${D1-12a($X^{1a}=CH_3$)} | *1 |
| 1-432 | Cl | O | $CH_2CH_3$ | $CH(CH_3)S$(D1-32a) | *1 |
| 1-433 | Cl | O | $CH_2CH_3$ | $CH(CH_3)S${D1-32b(3-$NO_2$)} | *1 |
| 1-434 | Cl | O | $CH_2CH_3$ | $CH_2SCH_2CH(OCH_3)_2$ | *1 |
| 1-435 | $CH_2CH_3$ | O | H | $OC(CH_3)_3$ | *1 |
| 1-436 | $C(O)OCH_3$ | O | H | $CH(CH_3)SCH_2CH_3$ | 102-104 |
| 1-437 | $CH_2OH$ | O | H | $CH(CH_3)SCH_2CH_3$ | 87-96 |
| 1-438 | Cl | O | $CH_2CH_3$ | D1-88a($X^{1a}=C(O)CH_2SCH_3$} | 45-47 |
| 1-439 | Cl | O | $CH_2CH_3$ | $CH_2OC(CH_3)_3$ | *1 |
| 1-440 | Cl | O | $CH_2CH_3$ | D1-92c | *1 |
| 1-441 | Cl | O | $CH_2CH_3$ | $CH(CH_3)OCH_2CH=CH_2$ | 91-93 |
| 1-442 | Cl | O | $CH_2CH_3$ | $CH(CH_3)OCH_2C{\equiv}CH$ | 105-107 |
| 1-443 | Cl | O | $CH_2CH_3$ | D1-103h | *1 |
| 1-444 | Cl | O | $CH_2CH_3$ | $CH_2C(CH_3)_3$ | *1 |

(continued)

| No. | $R^3$ | Y | $R^a$ | $R^{b-1}$ | m.p.(°C) |
|---|---|---|---|---|---|
| 1-445 | Cl | O | $CH_2CH_3$ | $C(CH_3)=CH_2$ | *1 |
| 1-446 | Cl | O | $CH_2CH_3$ | $CHCl_2$ | 81-83 |
| 1-447 | Cl | O | $CH_2CH_3$ | D1-103i | 68-70 |
| 1-448 | Cl | O | $CH_2CH_3$ | $CH(Br)C(CH_3)_3$ | 99-101 |
| 1-449 | Cl | O | $CH_2CH_3$ | NHPh | 163-165 |
| 1-450 | Cl | S | H | $NHCH_2CH=CH_2$ | 168-170 |
| 1-451 | Cl | S | H | NHPr-c | 192-195 |
| 1-452 | Cl | O | $CH_2CH_3$ | $CH_2$(D1-98a) | *1 |
| 1-453 | $CH_2OCH_3$ | O | $CH_3$ | $CH(CH_3)SCH_2CH_3$ | *1 |
| 1-454 | Cl | O | $C(O)CH_3$ | $CH_3$ | 106-107 |
| 1-455 | Cl | O | $CH_2CH_3$ | $C(=NOCH_3)C(=NOCH_3)CH_3$ | 130-131 |
| 1-456 | Cl | O | $CH_2CH_3$ | $CH_2CH_2CH_2CH_2CH_2CH_2CH_2CH_3$ | *1 |
| 1-457 | Cl | O | $CH_2CH_3$ | $CH_2CH_2CH_2CH_2CH_2CH_2CH_2CH_2CH_2CH_2CH_3$ | *1 |
| 1-458 | Cl | O | $CH_2CH_3$ | D1-103f | *1 |
| 1-459 | Cl | O | $CH_2CH_3$ | Bu-c | *1 |
| 1-460 | Cl | O | $CH_2CH_3$ | $CH_2CH(CF_3)_2$ | 74-76 |
| 1-461 | Cl | O | $CH_2CH_3$ | $CH_2$Pr-c | *1 |
| 1-462 | Cl | O | $CH_2CH_3$ | $CH_2${D1-85d[$X^{1b}$=C(Cl)$_3$] | *1 |
| 1-463 | Cl | O | $CH_2CH_3$ | $CH_2${D1-84d[$X^{1b}$=C(Cl)$_3$] | *1 |
| 1-464 | Cl | O | $CH_2CH_3$ | C(O)NHPh | 156-158 |
| 1-465 | Cl | O | $CH_2CH_3$ | $CH_2SCH_2CH(=NOCH_3)$ | *2(~2:1) |
| 1-466 | Cl | O | $CH_2CH_3$ | NH(D1-33a) | 184-186 |
| 1-467 | $SCH_3$ | O | H | $OC(CH_3)_3$ | *1 |
| 1-468 | Cl | O | $CH_2CH_3$ | D1-103g | 57-60 |
| 1-469 | Cl | O | $CH_3$ | D1-108b(2-Cl) | *1 |
| 1-470 | Cl | O | $CH_3$ | D1-108b(3-Cl) | 121-131 |
| 1-471 | Cl | O | $CH_3$ | D1-108b(4-Cl) | 160-161 |
| 1-472 | Cl | O | $CH_2CH_3$ | D1-108b(2-$SCH_3$) | *1 |
| 1-473 | Cl | O | $CH_2CH_3$ | D1-108b(3-$SCH_3$) | *1 |
| 1-474 | Cl | O | $CH_2CH_3$ | D1-108b(4-$SCH_3$) | *1 |
| 1-475 | Cl | O | $CH_2CH_3$ | D1-108b(3-$SCF_3$) | *1 |
| 1-476 | Cl | O | $CH_2CH_3$ | D1-108b(3,4-$OCF_2O$-) | *1 |
| 1-477 | Cl | O | $CH=NOCH_3$ | $CH(CH_3)CH_2SCH_3$ | *2(~4:1) |

(continued)

| No. | $R^3$ | Y | $R^a$ | $R^{b-1}$ | m.p.(°C) |
|---|---|---|---|---|---|
| 1-478 | Cl | S | H | $NHCH_2CH_3$ | 107-108 |
| 1-479 | H | O | H | $CH(CH_3)SCH_2CH_3$ | 92-93 |
| 1-480 | H | S | H | $CH(CH_3)SCH_2CH_3$ | 143-144 |
| 1-481 | H | $NOCH_3$ | H | $CH(CH_3)SCH_2CH_3$ | *2(~11:9) |
| 1-482 | $CH_2Cl$ | O | H | $CH(CH_3)SCH_2CH_3$ | 88-90 |
| 1-483 | Cl | O | $CH_2CH_3$ | $CH(CH_3)CH_2CH_2CH_3$ | 60-61 |
| 1-484 | $CH_2CH_3$ | O | $CH_2CH_3$ | $CH(CH_3)CH_2SCH_3$ | *1 |
| 1-485 | Cl | S | $CH_2CH_3$ | NHPr-c | 142-144 |
| 1-486 | H | $NOCH_3$ | $CH_3$ | $CH(CH_3)SCH_2CH_3$ | *2(~7:1) |
| 1-487 | $S(O)_2CH_3$ | O | $CH_2CH_3$ | $OC(CH_3)_3$ | *1 |
| 1-488 | Cl | O | $CH_2CH_3$ | $CH(CH_3)CH_2S(=NCN)CH_3$ | *1 |
| 1-489 | $CH_3$ | O | $CH_2CH_3$ | $CH(CH_3)CH_2S(O)CH_3$ | *1 |
| 1-490 | $CH_3$ | O | $CH_2CH_3$ | $CH(CH_3)CH_2S(O)_2CH_3$ | *1 |
| 1-491 | Cl | O | $CH_2CH_3$ | $CH(CH_3)CH_2S(O)(=NCN)CH_3$ | *1 |
| 1-492 | $S(O)_2CH_3$ | O | H | $OC(CH_3)_3$ | 155-157 |
| 1-493 | Cl | O | H | $CH(CH_3)CH_2S(O)CH_3$ | 171-172 |
| 1-494 | Cl | O | H | $CH(CH_3)CH_2S(O)_2CH_3$ | 180-181 |
| 1-495 | Cl | O | $SC(Cl)_3$ | $CH(CH_3)CH_2SCH_3$ | *1 |
| 1-496 | Cl | O | $CH=NOCH_3$ | $CH_2CH_2SCH_3$ | *1 |
| 1-497 | Cl | $NCH_2CH_3$ | $CH_3$ | $SCH_3$ | *1 |
| 1-498 | Cl | O | $CH_2CH_3$ | D1-9f(4-$CH_3$) | *1 |
| 1-499 | Cl | O | $CH_2CH_3$ | NHPr-c | 180-182 |
| 1-500 | Cl | O | $CH_2CH_3$ | $N(CH_3)$Pr-c | *1 |
| 1-501 | Cl | O | $CH_2CH_3$ | $NHCH_2CF_3$ | 152-153 |
| 1-502 | CN | O | $CH_2CH_3$ | $OC(CH_3)_3$ | *1 |
| 1-503 | Br | O | $CH_3$ | $OC(CH_3)_3$ | *1 |
| 1-504 | Cl | O | $CH_2CH_3$ | $N(CH_3)CH_2CF_3$ | *1 |
| 1-505 | Cl | O | $CH_2$Pr-c | $CH_2CH_2SCH_3$ | *1 |
| 1-506 | Cl | O | $CH_2$Pr-c | $CH(CH_3)CH_2S(O)CH_3$ | *2(~10:4) |
| 1-507 | Cl | O | $CH_2$Pr-c | $CH_2CH_2S(O)CH_3$ | *2(~10:3) |
| 1-508 | Cl | O | $CH_2$Pr-c | $CH(CH_3)S(O)CH_2CH_3$ | *2(~10:1) |
| 1-509 | Cl | O | $CH_2$Pr-c | $CH(CH_3)CH_2S(O)_2CH_3$ | *1 |
| 1-510 | Cl | O | $CH_2$Pr-c | $CH_2CH_2S(O)_2CH_3$ | *1 |

(continued)

| No. | $R^3$ | Y | $R^a$ | $R^{b-1}$ | m.p.(°C) |
|---|---|---|---|---|---|
| 1-511 | Cl | O | $CH_2Pr$-c | $CH(CH_3)S(O)_2CH_2CH_3$ | 123-125 |
| 1-512 | Cl | O | $CH_2CH_3$ | NH(D1-80a) | 236-237 |
| 1-513 | Cl | O | $CH_3$ | $CH(CH_3)CH_2SCH_2CH_3$ | 50-54 |
| 1-514 | Cl | O | $CH_2CH_3$ | $CH(CH_3)CH_2SCH_2CH_3$ | *1 |
| 1-515 | Cl | O | $CH_3$ | $CH(CH_3)CH_2S(O)CH_2CH_3$ | *2(~2:1) |
| 1-516 | Cl | O | $CH_2CH_3$ | $CH(CH_3)CH_2S(O)CH_2CH_3$ | *2(~2:1) |
| 1-517 | Cl | O | $CH_3$ | $CH(CH_3)CH_2S(O)_2CH_2CH_3$ | *1 |
| 1-518 | Cl | O | $CH_2CH_3$ | $CH(CH_3)CH_2S(O)_2CH_2CH_3$ | *1 |
| 1-519 | Cl | O | $CH_2CH_3$ | $NHC(O)CF_3$ | 175-179 |
| 1-520 | Cl | O | $CH_2CH_3$ | $CH_2CH_2CH_2SCH_3$ | *1 |
| 1-521 | Cl | O | $CH_2CH_3$ | $CH_2C(O)NHCH_2CF_3$ | 115-125 |
| 1-522 | Cl | O | $CH_2CN$ | $OC(CH_3)_3$ | 77-79 |
| 1-523 | $CF_3$ | O | $CH_2CH_3$ | $CH(CH_3)CH_2SCH_3$ | *1 |
| 1-524 | $S(O)_2CH_3$ | O | $CH_2CH_3$ | $CH(CH_3)CH_2SCH_3$ | *1 |
| 1-525 | CN | O | $CH_2CH_3$ | $CH(CH_3)CH_2SCH_3$ | *1 |
| 1-526 | $OCH_3$ | O | $CH_2CH_3$ | $CH(CH_3)CH_2SCH_3$ | *1 |
| 1-527 | Cl | O | $CH_2CH_3$ | $CH_2CH_2CH_2S(O)CH_3$ | *1 |
| 1-528 | Cl | O | $CH_2CH_3$ | $CH_2CH_2CH_2S(O)_2CH_3$ | *1 |
| 1-529 | Cl | O | $CH_2CH_3$ | $CH_2C(O)N(CH_3)S(O)_2CH_3$ | 65-72 |
| 1-530 | Cl | O | $CH_2CH_3$ | $N(CH_2CH_3)CH_2CF_3$ | *1 |
| 1-531 | Cl | O | $CH_2CH_3$ | $CH(CH_3)S${D1-108b(4-F)} | *1 |
| 1-532 | Cl | O | $CH_2CH_3$ | $CH(CH_3)S${D1-108b(3-F)} | *1 |
| 1-533 | Cl | O | $CH_2CH_3$ | $CH(CH_3)S${D1-108b(2-F)} | *1 |
| 1-534 | Cl | O | $CH_2CH_3$ | $CH(CH_3)S${D1-108b(4-$CH_3$)} | *1 |
| 1-535 | Cl | O | $CH_2CH_3$ | $CH(CH_3)S${D1-108b(4-$CF_3$)} | *1 |
| 1-536 | Cl | O | $CH_2CH_3$ | $N(CH_2SCH_3)CH_2CF_3$ | *1 |
| 1-537 | Cl | O | $CH_2CN$ | $CH(CH_3)S(O)CH_2CH_3$ | 44-47 |
| 1-538 | Cl | O | $CH_2CN$ | $CH(CH_3)S(O)_2CH_2CH_3$ | 136-138 |
| 1-539 | Cl | O | $CH_2CH_3$ | $CH=CHCH_2SCH_3$ | *2(~78:22) |
| 1-540 | Cl | O | $CH_2CH_3$ | $N\{CH_2S(O)_2CH_3\}CH_2CF_3$ | 162-163 |
| 1-541 | Cl | S | $CH_2CH_3$ | $N(CH_3)CH_2CF_3$ | *1 |
| 1-542 | Cl | O | $CH_2CN$ | $CH_2CH_2SCH_3$ | *1 |
| 1-543 | Cl | O | $CH_2CN$ | $CH_2CH_2S(O)CH_3$ | *1 |

(continued)

| No. | $R^3$ | Y | $R^a$ | $R^{b-1}$ | m.p.(°C) |
|---|---|---|---|---|---|
| 1-544 | Cl | O | $CH_2CN$ | $CH_2CH_2S(O)_2CH_3$ | *1 |
| 1-545 | Br | O | $CH_3$ | $CH(CH_3)CH_2SCH_3$ | *1 |
| 1-546 | Br | O | $CH_3$ | $C(=NOCH_3)CH_2SCH_3$ | *1 |
| 1-547 | Cl | O | $CH_2CN$ | $CH(CH_3)CH_2SCH_3$ | 126-127 |
| 1-548 | Me | O | H | $NHCH_2CH_3$ | 199-200 |
| 1-549 | Me | S | H | $NHCH_2CH_3$ | 120-122 |
| 1-550 | Cl | O | H | $NHCH_2CH_3$ | 215-217 |
| 1-551 | Cl | O | $CH_3$ | $NHCH_2CH_3$ | 167-168 |
| 1-552 | Cl | S | H | $NHCH_3$ | 180-183 |
| 1-553 | Cl | S | H | $NHCH_2CH_2CH_3$ | 167-169 |
| 1-554 | Cl | S | H | $NHCH(CH_3)_2$ | 176-178 |
| 1-555 | Cl | S | H | NH{D1-108b(4-$SCH_3$)} | 148-150 |
| 1-556 | Cl | S | H | NHPh | 161-163 |
| 1-557 | Cl | S | H | NH{D1-108b(4-$CF_3$)} | 216-218 |
| 1-558 | Cl | S | H | NH{D1-108b(3-$CF_3$)} | 153-155 |
| 1-559 | Cl | S | H | NH{D1-108b(2-$CF_3$)} | 130-132 |
| 1-560 | Cl | S | H | $NHCH_2$(D1-108a) | 147-149 |
| 1-561 | Cl | S | H | $NHC(O)OCH_2CH_3$ | 240-242 |
| 1-562 | Cl | S | $CH_3$ | $NHCH_2CH_3$ | 141-142 |
| 1-563 | Cl | O | $CH_2CH_3$ | $NHCH_2CH_3$ | 169-171 |
| 1-564 | Cl | S | $CH_2CH_3$ | $NHCH_2CH_3$ | 127-128 |
| 1-565 | Me | O | $CH_2CH_3$ | $NHCH_2CH_3$ | 115-120 |
| 1-566 | Me | S | $CH_2CH_3$ | $NHCH_2CH_3$ | 129-131 |
| 1-567 | Me | S | $CH_3$ | $NHCH_2CH_3$ | 167-169 |
| 1-568 | Cl | O | $CH_2CN$ | $CH(CH_3)CH_2S(O)CH_3$ | *2(~3:2) |
| 1-569 | Cl | O | $CH_2CN$ | $CH(CH_3)CH_2S(O)_2CH_3$ | *1 |
| 1-570 | Cl | O | $CH_2CH_3$ | $NHCH_3$ | 168-170 |
| 1-571 | Me | O | $CH_2CH_3$ | $NHCH_2CF_3$ | 112-113 |
| 1-572 | Cl | O | $CH_2CH_3$ | $NH_2$ | 204-206 |
| 1-573 | Cl | O | $CH_2CH_3$ | $N(CH_2CH_3)CH_2CF_3$ | *1 |
| 1-574 | Cl | O | $CH_2CH_3$ | $NCH_2CH_2Cl$ | 130-132 |
| 1-575 | Cl | O | $CH_2CH_3$ | $NCH_2CH_2CH_3$ | 124-128 |
| 1-576 | Cl | O | $CH_2CH_3$ | $NCH(CH_3)_2$ | 130-134 |

(continued)

| No. | $R^3$ | Y | $R^a$ | $R^{b-1}$ | m.p.(°C) |
|---|---|---|---|---|---|
| 1-577 | Cl | S | H | $NHCH_2C{\equiv}CH$ | 210-212 |
| 1-578 | Cl | S | H | NH(D1-33a) | 100-102 |
| 1-579 | Cl | S | H | $NHCH_2CF_3$ | 177-178 |
| 1-580 | Cl S | | H | $N(CH_3)CH_2CH_3$ | 134-135 |
| 1-581 | Br | O | $CH_3$ | $CH(CH_3)CH_2S(O)CH_3$ | *2(~7:5) |
| 1-582 | Br | O | $CH_3$ | $CH(CH_3)CH_2S(O)_2CH_3$ | *1 |
| 1-583 | Br | O | $CH_3$ | $C(=NOCH_3)CH_2S(O)CH_3$ | *1 |
| 1-584 | Br | O | $CH_3$ | $C(=NOCH_3)CH_2S(O)_2CH_3$ | *1 |
| 1-585 | Br | O | H | $NHCH_2CF_3$ | >280 |
| 1-586 | Br | O | $CH_3$ | $NHCH_2CF_3$ | 209-210 |
| 1-587 | Br | O | $CH_2CH_3$ | $NHCH_2CF_3$ | 145-147 |
| 1-588 | Cl | O | $CH_2CH_3$ | $CH(CH_3)$(D1-87a) | *1 |
| 1-589 | Cl | O | $CH_2CH_3$ | D1-108b{2-C(O)OH} | 177-179 |
| 1-590 | Cl | O | $CH_2CH_3$ | D1-108b{2-C(O)NHS(O)$_2$CH$_3$} | *1 |
| 1-591 | Cl | O | $-CH_2CH_2N(CH_2CF_3)-$ | | 130-132 |
| 1-592 | Cl | O | $-CH_2CH_2NH-$ | | 170-172 |
| 1-593 | Cl | O | H | $NHCH_2CH_2Cl$ | 182-184 |
| 1-594 | Cl | S | $CH_2CH_3$ | NH(D1-34a) | 96-98 |
| 1-595 | Cl | O | $CH_2CH_3$ | $NHCH_2CH=CH_2$ | 145-147 |
| 1-596 | Cl | O | $CH_2CH_3$ | $CH(CH_3)CH_2S(O)_2N(CH_3)_2$ | *1 |
| 1-597 | Cl | S | $CH_2CH_3$ | NH(D1-32a) | 241-242 |
| 1-598 | Cl | O | $CH_2CH_3$ | $NHCH_2CN$ | *1 |
| 1-599 | Cl | O | $CH_2CH_3$ | $NHCH_2$(D1-32a) | *1 |
| 1-600 | Cl | S | $CH_2CH_3$ | $NHS(O)_2CF_3$ | 249-250 |
| 1-601 | Cl | O | $CH_2Ph$ | $CH(CH_3)CH_2SCH_3$ | *1 |
| 1-602 | Cl | O | $CH_2Ph$ | $CH_2CH_2SCH_3$ | *1 |
| 1-603 | Cl | O | CH$_2$[D1-37b{4,6-(OCH$_3$)$_2$}] | $CH(CH_3)SCH_2CH_3$ | *1 |
| 1-604 | Cl | O | $CH_2Ph$ | $CH(CH_3)SCH_2CH_3$ | *1 |
| 1-605 | Cl | O | $-CH_2CH_2N\{CH(CF_3)OCH_2CF_3\}-$ | | 92-94 |
| 1-606 | Cl | O | $-CH_2CH_2N(CH_2SCH_3)-$ | | 101-103 |
| 1-607 | Cl | O | $CH_2CH_3$ | $NHNHC(O)OC(CH_3)_3$ | 185-187 |
| 1-608 | Cl | O | $CH_2CH_3$ | $NHNH_2$ 2HCl | 172-174 |

Compound No. 1-047 of the present invention is a mixture of Compounds Nos. 1-047a and 1-047b, and Compound No. 1-093 of the present invention is a mixture of Comopunds Nos.1-093a and 1-093b.

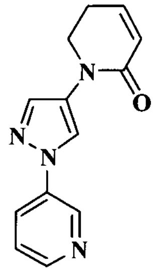

1-047a

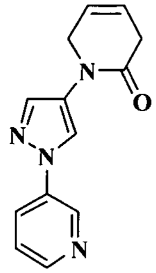

1-047b

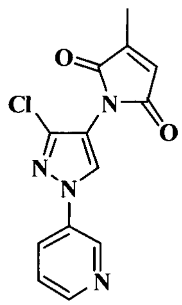

1-093a

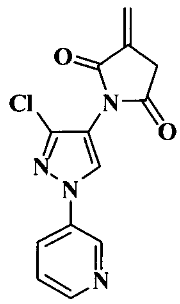

1-093b

[Table 5]

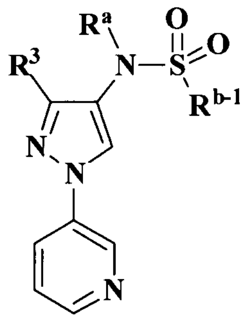

| No. | $R^3$ | $R^a$ | $R^{b-1}$ | m.p.(°C) |
|---|---|---|---|---|
| 2-001 | H | H | $CH_3$ | 140-150 |
| 2-002 | Cl | $CH_3$ | $CH_2CH_2SCH_3$ | 101-103 |
| 2-003 | Cl | $CH_3$ | $CH=CH_2$ | *1 |

[Table 6]

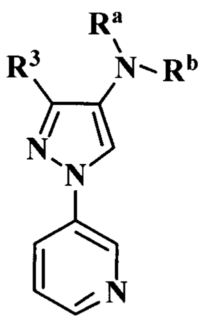

| No. | $R^3$ | $R^a$ | $R^b$ | m.p.(°C) |
|---|---|---|---|---|
| 3-001 | Cl | $CH_3$ | D1-51a | 125-127 |
| 3-002 | Me | | $=C(CH_3)OCH_3$ | 90-92 |
| 3-003 | Me | | $=C(CH_3)OCH_2CH_3$ | 56-58 |
| 3-004 | Me | | $=C(CH_3)N(CH_3)C(O)CH_2SCH_3$ | 54-57 |
| 3-005 | Cl | | $=CHN(CH_3)_2$ | 82-85 |
| 3-006 | Cl | | $=C(SCH_3)CH(CH_3)SCH_2CH_3$ | *2(~2:1) |
| 3-007 | Cl | | D1-88b{$X^{1a}=C(O)CH_2SCH_3$} | 126-129 |
| 3-008 | Cl | H | D1-51a | 202-204 |

(continued)

| No. | $R^3$ | $R^a$ | $R^b$ | m.p.(°C) |
|---|---|---|---|---|
| 3-009 | H | | $=C(SCH_3)CH(CH_3)SCH_2CH_3$ | *2(~2:1) |
| 3-010 | Cl | | $=CHN(OCH_3)C(O)CH(CH_3)CH_2SCH_3$ | 58-59 |
| 3-011 | Cl | | $=CHN(OCH_3)C(O)$Pr-c | 215-220 |
| 3-012 | Cl | | $=CHN(OCH_3)C(O)CH(CH_3)SCH_2CH_3$ | *2(~77:23) |
| 3-013 | Cl | | $=CHN(OCH_3)C(O)CH_2CH_2SCH_3$ | 99-100 |

[Table 7]

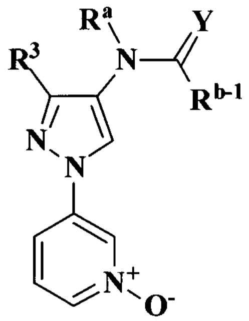

| No. | $R^3$ | Y | $R^a$ | $R^{b-1}$ | m.p.(°C) |
|---|---|---|---|---|---|
| 4-001 | Cl | O | $CH_3$ | $CH(CH_3)CH_2S(O)_2CH_3$ | 154-156 |
| 4-002 | Cl | O | $CH_2CH_3$ | $OC(CH_3)_3$ | 146-147 |
| 4-003 | Cl | O | $CH_2CH_3$ | $N\{CH_2S(O)_2CH_3\}CH_2CF$ | *1 |

[Table 8]

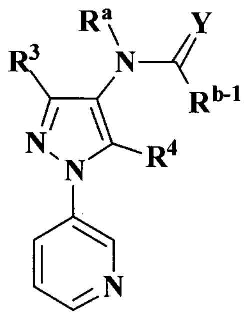

| No. | $R^3$ | $R^4$ | Y | $R^a$ | $R^{b-1}$ | m.p.(°C) |
|---|---|---|---|---|---|---|
| 5-001 | H | $CH_3$ | O | H | $OC(CH_3)_3$ | 89-91 |
| 5-002 | H | $CH_3$ | O | $CH_2CH_3$ | $OC(CH_3)_3$ | 80-83 |
| 5-003 | H | $CH_3$ | O | $CH_2CH_3$ | $CH(CH_3)CH_2SCH_3$ | *1 |
| 5-004 | H | $CH_3$ | O | $CH_2CH_3$ | $CH(CH_3)SCH_2CH_3$ | *1 |
| 5-005 | H | Cl | O | H | $OC(CH_3)_3$ | *1 |
| 5-006 | H | Cl | O | H | $OC(CH_3)_3$ | 87-89 |
| 5-007 | Cl | $SCH_2CH_3$ | O | $CH_2CH_3$ | $CH(CH_3)SCH_2CH_3$ | *1 |

[Table 9]

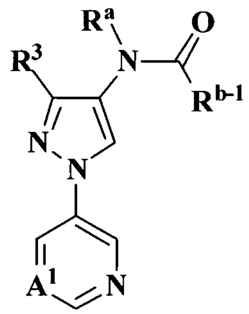

| No. | $A^1$ | $R^3$ | $R^a$ | $R^{b-1}$ | m.p.(°C) |
|---|---|---|---|---|---|
| 6-001 | N | Cl | $CH_2CH_3$ | $CH(CH_3)SCH_2CH_3$ | 154-156 |
| 6-002 | $C-CH_3$ | Cl | $CH_2CH_3$ | $CH(CH_3)SCH_2CH_3$ | *1 |
| 6-003 | $C-OCH_3$ | Cl | $CH_2CH_3$ | $CH(CH_3)SCH_2CH_3$ | *1 |
| 6-004 | C-F | Cl | H | $OC(CH_3)_3$ | 199-201 |
| 6-005 | C-F | Cl | $CH_2CH_3$ | $OC(CH_3)_3$ | 108-110 |
| 6-006 | C-CN | Cl | $CH_2CH_3$ | $CH(CH_3)SCH_2CH_3$ | 94-98 |
| 6-007 | $C-CF_3$ | Cl | $CH_2CH_3$ | $CH(CH_3)SCH_2CH_3$ | 100-102 |
| 6-008 | C-F | Cl | $CH_2CH_3$ | $CH(CH_3)SCH_2CH_3$ | 83-84 |
| 6-009 | C-F | Cl | $CH_2CH_3$ | $CH(CH_3)CH_2SCH_3$ | 80-81 |
| 6-010 | $C-NH_2$ | H | $CH_3$ | c-Pr | *1 |

[Table 10]

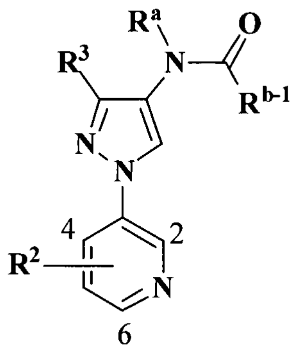

| No. | $R^2$ | $R^3$ | $R^a$ | $R^{b-1}$ | m.p.(°C) |
|---|---|---|---|---|---|
| 7-001 | $6-CH_3$ | H | $CH_3$ | c-Pr | 198-199 |
| 7-002 | $6-CH_3$ | Cl | $CH_2CH_3$ | $CH(CH_3)SCH_2CH_3$ | 57-60 |
| 7-003 | $4-CH_3$ | Cl | $CH_2CH_3$ | $CH(CH_3)SCH_2CH_3$ | *1 |
| 7-004 | $2-CH_3$ | Cl | $CH_2CH_3$ | $CH(CH_3)SCH_2CH_3$ | *1 |

[Table 11]

| No.<br>Proton NMR chemical shift (in $CDCl_3$): σ(ppm) |
|---|
| 1-018;<br>8.93(d,J=2.7Hz,1H),8.56(dd,J=4.8,1.2Hz,1H),8.01(ddd,J=8.4,2.7,1.2Hz,1H),7.90(s,1H), 7.41(dd,J=8.4,4.8Hz,1H), 3.22(s,3H),2.77(t,J=7.2Hz,2H),2.43(t,J=7.2Hz,2H),2.27(s,3H), 2.04(s,3H) |
| 1-019;<br>8.94(d,J=2.4Hz,1H),8.56(dd,J=1.2,4.8Hz,1H),8.02(ddd,J=1.2,2.4,8.1Hz,1H),7.86(s,1H), 7.42(dd,J=4.8,8.1Hz,1H), 3.74-3.62(m,2H),2.77(t,J=7.2Hz,2H),2.39(t,J=7.2Hz,2H),2.26(s ,3H),2.04(s,3H),1.14(t,J=7.2Hz,3H) |

(continued)

| No. Proton NMR chemical shift (in $CDCl_3$): σ(ppm) |
|---|
| 1-021; |
| 9.02-8.89(m,1H),8.51(dd,J=1.2,4.8Hz,1H),8.30(brs,1H),8.01(ddd,J=1.2,2.7,9.0Hz,1H),7. 72(s,1H),7.37(dd,J=4.8, 9.0Hz,1H),3.28(s,3H),1.51(s,9H) |
| 1-022; |
| 9.01-8.92(m,1H).8.51(dd,J=1.2,4.8Hz,1H),8.30(brs,1H),8.01(ddd,J=1.2,2.7,9.0Hz.1H),7. 68(s,1H),7.37(dd,J=4.8, 9.0Hz,1H),3.71(q,J=7.2Hz,2H),1.58(s,9H),1.25(t,J=7.2Hz,3H) |
| 1-023; |
| 8.93(d,J=2.7Hz,1H),8.55(dd,J=1.2,4.8Hz,1H),8.01(ddd,J=1.2,2.7,8.1 Hz,1H),7.92(s,1H), 7.41 (dd,J=4.8,8.1 Hz,1H), 3.23(s,3H),2.89-2.71(m,1H),2.85(d,J=12.3Hz,1H),2.42(dd,J=4. 5,12.3Hz,1H),2.30(s,3H),1.99(s,3H),1.12(d, J=6.6Hz,3H) |
| 1-026; |
| 9.02(d,J=2.7Hz,1H),8.58(s,1H),8.54(dd,J=1.5,4.8Hz,1H),8.00-8.10(m,1H),7.77(s,1H),7. 40(dd,J=4.8,8.4Hz,1H), 3.83(t,J=7.2Hz,2H),2.60(t,J=8.1Hz,2H),2.20-2.35(m,2H) |
| 1-031; |
| 8.99-8.91 (m,1H),8.56(d,J=4.8Hz,1H),8.02(ddd,J=1.2,2.4,8.1 Hz,1H),7.90(s,1H),7.41 (dd, J=4.8,8.1 Hz,1H), 3.90-3.40(m,2H),2.87(dd,J=9.3,12.3Hz,1H),2.79-2.62(m,1H),2.41(dd,J =5.1,12.3Hz,1H),2.29(s,3H),1.99(s,3H), 1.12(t,J=6.3Hz,3H)1.11(d,J=6.6Hz,3H) |
| 1-033; |
| 8.94(d,J=2.4Hz,1H),8.62(dd,J=1.2,4.8Hz,1H),8.04(ddd,J=1.2,2.4,8.4Hz,1H),7.95(s,1H), 7.46(dd,J=4.8,8.4Hz,1H), 3.71(q,J=7.2Hz,2H),2.79(dd,J=7.2,7.5Hz,2H),2.44(dd,J=7.2,7. 5Hz,2H),2.06(s,3H),1.16(t,J=7.2Hz,3H) |
| 1-037; |
| 8.90(d,J=2.4Hz,1H),8.56(dd,J=4.8,1.5Hz,1H),8.00(ddd,J=8.1,2.4,1.5Hz,1H),7.90(s,1H), 7.40(dd,J=8.1,4.8Hz,1H), 3.22(s,3H),1.46(s,9H) |
| 1-038; |
| 8.94(d,J=2.7Hz,1H),8.63(dd,J=4.5,1.2Hz,1H),8.04(ddd,J=8.1,2.7,1.2Hz,1H),8.00(s,1H) ,7.46(dd,J=8.1,4.5Hz,1H), 3.25(s,3H),2.80(t,J=7.2Hz,2H),2.48(t,J=7.2Hz,2H),2.07(s,3H) |
| 1-039; |
| 8.95(d,J=2.4Hz,1H),8.63(d,J=4.5Hz,1H),8.04(ddd,J=8.1,2.4,1.2Hz,1H),8.04(s,1H),7.46( dd,J=8.1,4.5Hz,1H), 3.27(s,3H),2.74-2.90(m,2H),2.48(dd,J=12.6,4.8Hz,1H),2.03(s,3H),1. 17(d,J=6.3Hz,3H) |
| 1-040; |
| 8.95(d,J=2.4Hz,1H),8.61(dd,J=1.8,4.8Hz,1H),8.02(ddd,J=1.2,2.4,8.4Hz,1H),8.02(s,1H), 7.44(dd,J=4.8,8.4Hz,1H), 3.76(td,J=7.2,13.8Hz,1H),3.67(td,J=7.2,13.8Hz,1H),3.14(td,J= 7.8,12.9Hz,1H),2.86(td,J=6.3,12.9Hz,1H), 2.72-2.60(m,2H),2.58(s,3H),1.16(t,J=7.2Hz,3 H) |
| 1-042; |
| (the isomer contained in a ratio of 3)8.97(d,J=2.1Hz,1H),8.63-8.58(m,1H),8.08-7.99( m,1H),7.96(s,1H),7.69(s,1H), 7.44(dd,J=4.8,8.1Hz,1H),3.27(s,3H),2.78(t,J=7.2Hz,2H),2. 53(t,J=7.2Hz,2H),2.05(s,3H) |
| (the isomer contained in a ratio of 2)9.02-8.97(m,1H),8.61(s,1H),8.53(d,J=3.9Hz,1H) ,8.08-7.99(m,1H),7.77(s,1H), 7.38(dd,J=4.8,8.1Hz,1H),3.43(s,3H),2.94-2.80(m,4H),2.18( s,3H) |
| 1-043; |
| (the isomer contained in a ratio of 3)8.97(d,J=2.4Hz,1H),8.59(d,J=4.8Hz,1H),8.08-7. 99(m,1H),8.01(s,1H),7.72(s, 1H),7.44(dd,J=4.8,8.1Hz,1H),3.29(s,3H),3.06-2.82(m,2H),2. 48-2.40(m,1H),2.01(s,3H),1.12(d,J=6.3Hz,3H) |
| (the isomer contained in a ratio of 1)9.02-8.96(m,1H),8.66(s,1H),8.57-8.51(m,1H),8. 08-7.99(m,1H),7.78(s,1H), 7.42-7.35(m,1H),3.51(s,3H),3.19-2.82(m,2H),2.60(dd,J=6.0,1 3.2Hz,1H),2.14(s,3H),1.30(d,J=6.9Hz,3H) |
| 1-044; |
| (the isomer contained in a ratio of 3)8.98(d,J=2.4Hz,1H),8.60(d,J=4.8Hz,1H),8.10-8. 02(m,1H),7.94(s,1H),7.73(s, 1H),7.45(dd,J=4.8,8.1Hz,1H),3.71(q,J=7.2Hz,2H),2.77(t,J= 7.2Hz,2H),2.48(t,J=7.2Hz,2H),2.03(s,3H),1.16(t, J=7.2Hz,3H) |
| (the isomer contained in a ratio of 1)8.98(d,J=2.4Hz,1H),8.62(s,1H),8.53(d,J=4.8Hz, 1H),8.06-7.99(m,1H),7.66(s, 1H),7.39(dd,J=4.8,8.1Hz,1H),3.80(q,J=7.2Hz,2H),2.95-2.79 (m,4H),2.18(s,3H),1.36(t,J=7.2Hz,3H) |
| 1-045; |

(continued)

| No. |
| --- |
| Proton NMR chemical shift (in $CDCl_3$): σ(ppm) |
| (the isomer contained in a ratio of 5)8.98(d,J=2.4Hz,1H),8.60(dd,J=1.2,4.8Hz,1H),8. 06(ddd,J=1.2,2.4,8.4Hz,1H), 7.99(s,1H),7.70(s,1H),7.45(dd,J=4.8,8.4Hz,1H),3.94-3.58( m,2H),2.93-2.75(m,2H),2.49-2.35(m,1H),2.01(s,3H), 1.16(t,J=7.2Hz,3H),1.11(d,J=6.6Hz, 3H) |
| (the isomer contained in a ratio of 1)8.98(d,J=2.4Hz,1H),8.66(s,1H),8.58-8.54(m,1H) ,8.06-7.98(m,1H),7.74(s,1H), 7.45-7.37(m,1H),3.94-3.58(m,2H),3.16-2.94(m,2H),2.67-2. 57(m,1H),2.14(s,3H),1.40(t,J=7.2Hz,3H),1.31(d, J=6.9Hz,3H) |
| 1-048; |
| 8.95(d,J=2.7Hz,1H),8.61(d,J=4.8Hz,1H),8.07-8.01(m,1H),8.00(s,1H),7.45(dd,J=4.8,8.4 Hz,1H),3.92-3.71(m,1H), 3.69-3.50(m,1H),2.85(dd,J=9.0,12.6Hz,1H),2.75-2.61(m,1H),2. 45(dd,J=5.1,12.6Hz,1H),2.01(s,3H),1.16(t,J=7.8Hz, 3H),1.15(d,J=6.9Hz,3H) |
| 1-049; |
| (the isomer contained in a ratio of 10)9.02(d,J=2.7Hz,1H),8.59(dd,J=1.2,4.8Hz,1H),8 .22(s,1H),8.06-7.98(m,1H), 7.40(dd,J=4.8,8.1Hz,1H),4.18-3.87(m,1H),3.52-3.07(m,3H),2 .74-2.51(m,1H),2.58(s,3H),1.18(d,J=6.9Hz,3H), 1.15(t,J=7.2Hz,3H) |
| (the isomer contained in a ratio of 7)8.98(d,J=2.7Hz,1H),8.61(dd,J=1.2,4.8Hz,1H),8. 15(s,1H),8.06-7.98(m,1H), 7.43(dd,J=4.8,8.1Hz,1H),4.18-3.87(m,1H),3.52-3.07(m,3H),2. 74-2.51(m,1H),2.58(s,3H),1.25(d,J=6.9Hz,3H), 1.20(t,J=7.2Hz,3H) |
| 1-050; |
| 9.01(d,J=2.7Hz,1H),8.61(dd,J=1.2,4.8Hz,1H),8.18(s,1H),8.00(ddd,J=1.2,2.7,8.1Hz,1H), 7.43(dd,J=4,8,8.1Hz,1H), 4.17-4,01(m,1H),3,81(dd,J=10.8,13.5Hz,1H),3.39-3.19(m,2H), 2.93(s,3H),2.87(dd,J=2.7,13.5Hz,1H),1.20(d, J=7.2Hz,3H),1.16(t,J=7.2Hz,3H) |
| 1-051; |
| 8.96(d,J=2.4Hz,1H),8.63(dd,J=4.8,1.2Hz,1H),8.07(s,1H),8.03(ddd,J=8.4,2.4,1.2Hz,1H), 7.46(dd,J=8.4,4.8Hz,1H), 3.27(s,3H),3.11-3.21(m,1H),2.84-2.93(m,1H),2.66-2.76(m,2H), 2.60(s,3H) |
| 1-052; |
| 8.96(d,J=2.4Hz,1H),8.64(d,J=4.8Hz,1H),8.06(s,1H),8.04(ddd,J=8.4,2.4,1.5Hz,1H),7.47( dd,J=8.4,4.8Hz,1H),3.43(t, J=7.2Hz,2H),3.27(s,3H),2.97(s,3H),2.75(t,J=7.2Hz,2H) |
| 1-053; |
| (the isomer contained in a ratio of 4)8.99-9.03(m,1H),8.60(d,J=4.5Hz,1H),8.28(s,1H) ,8.00(dd,J=8.1,1.BHz,1H), 7.41(dd,J=8.1,4.5Hz,1H),3.30(s,3H),3.13-3.21(m,2H),2.57-2.6 2(m,1H),2.61 (s,3H),1.21 (d,J=6.9Hz,3H) |
| (the isomer contained in a ratio of1)8.99-9.03(m,1H),8.60(d,J=4.5Hz,1H),8.20(s,1H), 8.00(dd,J=8.1,1.8Hz,1H), 7.4(dd,J=8.1,4.5Hz,1H),3.26(s,3H),3.13-3.21(m,2H),2.57-2.6 2(m,1H),2.61(s,3H),1.21(d,J=6.9Hz,3H) |
| 1-054; |
| 9.00(d,J=2.7Hz,1H),8.62(dd,J=4.5,1.2Hz,1H),8.22(s,1H),8.00(ddd,J=8.1,2.7,1.2Hz,1H), 7.43(dd,J=8.1,4.5Hz,1H), 3.83(dd,J=13.8,10.5Hz,1H),3.33-3.40(m,1H),3.28(s,3H),2.95( s,3H),2.89(dd,J=13.8,3.0Hz,1H),1.16(d,J=6.9Hz,3H) |
| 1-059; |
| 9.20-8.90(m,1H),8.70-8.45(m,1H),8.20(d,J=9.0Hz,2H),8.04(d,J=9.0Hz,2H),8.03(s,1H),7. 60-7.40(m,1H), 7.25-7.20(m,1H),3.74(q,J=6.9Hz,2H),2.36(s,3H),1.28(t,J=6.9Hz,3H) |
| 1-060; |
| 8.92(d,J=3.0Hz,1H),8.51(dd,J=4.8Hz,1.8Hz,1H),7.99(ddd,J=8.4Hz,3.0Hz,1.8Hz,1H),7.9 5(s,1H),7.38(dd,J=8.4Hz, 3.0Hz,1H),5.06(brs,1H),3.65(q,J=7.2Hz,2H),3.39(q,J=7.2Hz,2 H),2.60(t,J=7.2Hz,2H),2.28(s,3H),2.57(s,3H),1.26(t, J=7.2Hz,3H) |
| 1-063; |
| 8.95(d,J=2.7Hz,1H),8.62(dd,J=4.8,1.2Hz,1H),8.05(ddd,J=8.4,2.7,1.2Hz,1H),7.96(s,1H), 7.46(dd,J=8.1,4.8Hz,1H), 3.61(m,2H),2.79(m,2H),2.45(m,2H),2.06(s,3H),1.57(m,2H),0.9 3(t,J=7.2Hz,3H) |
| 1-064; |
| 8.94(d,J=2.7Hz,1H),8.63(dd,J=4.5,1.2Hz,1H),8.04(ddd,J=8.7,2.4,1.2Hz,1H),7.93(s,1H), 7.46(dd,J=8.7,4.5Hz,1H), 5.92-5.79(m,1H),5.21-5.12(m,2H),4.26(brs,2H),2.81(t,J=7.5Hz, 2H),2.49(t,J=7.5Hz,2H),2.07(s,3H) |
| 1-065; |

(continued)

| No. Proton NMR chemical shift (in $CDCl_3$): σ(ppm) |
|---|
| 8.95(d,J=2.7Hz,1H),8.62(dd,J=4.8,1.5Hz,1H),8.05(ddd,J=8.4,2.7,1.5Hz,1H),7.95(s,1H), 7.46(dd,J=8.4,4.8Hz,1H), 3.65-3.79(m,2H),3.18-3.34(m,1H),2.48(dd,J=15.9,6.3Hz,1H),2. 26(dd,J=15.9,7.8Hz,1H),2.06(s,3H), 1.28(d.J=6.9Hz,3H),1.17(t,J=7.2Hz,3H) |
| 1-066; |
| 8.96(d,J=2.7Hz,1H),8.63(dd,J=4.5,1.2Hz,1H),8.07-8.01(m,1H),8.01(s,1H),7.46(dd,J=8.1 ,4.5Hz,1H),3.76(brs,1H), 3.50(brs,1H),2.86(dd,J=12.6,9.0Hz,1H),2.74-2.70(m,1H),2.47(d d,J=12.6,5.1Hz,1H),2.02(s,3H),1.58(tq,J=7.8, 7.8Hz,2H),1.16(d,J=6.6Hz,3H),0.94(t,J=7. 5Hz,3H) |
| 1-067; |
| 8.95(d,J=2.7Hz,1H),8.62(d,J=4.8Hz,1H),8.00-8.10(m,1H),8.00(s,1H),7.46(dd,J=4.8,8.1 Hz,1H),4.74(s,2H),4.06(s, 2H),3.72(q,J=7.5Hz,2H),2.10(s,3H),1.18(t,J=7.5Hz,3H) |
| 1-068; |
| 8.95(d,J=2.7Hz,1H),8.65-8.59(m,1H),8.07-8.00(m,1H),7.99(s,1H),7.45(dd,J=4.8,8.1Hz,1 H),5.20-4.80(m,2H), 3.44(s,3H),2.80(t,J=7.2Hz,2H),2.54(t,J=7.2Hz,2H),2.06(s,3H) |
| 1-076; |
| 8.94(d,J=2.4Hz,1H),8.63-8.62(m,1H),8.04(ddd,J=8.4,2.7,1.2Hz,1H),7.99(s,1H),7.46(dd, J=8.4,4.8Hz,1H), 5.92-5.80(m,1H),5.21-5.15(m,2H),4.57(brs,1H),4.00(brs,1H),2.87(m,1 H),2.78-2.71(m,1H),2.48(m,1H),2.02(s,3H), 1.18(d,J=6.6Hz,3H) |
| 1-077; |
| 8.96(d,J=2.7Hz,1H),8.63(d,J=4.5Hz,1H),8.08-8.03(m,1H),7.96(d,J=1.2Hz,1H),7.46(dd,J =8.4,4.8Hz,1H),3.71(q, J=7.2Hz,2H),3.1(t,J=7.2Hz,2H),2.45(t,J=7.2Hz,2H),2.28(s,3H),1 .16(t,J=7.2Hz,3H) |
| 1-078; |
| 8.96(d,J=2.1Hz,1H),8.65-8.63(m,1H),8.07(ddd,J=8.7,3.0,1.5Hz,1H),7.90(s,1H),7.47(dd, J=8.4,4.8Hz,1H),5.04(qq,J=6.6,6.6Hz,1H),2.82-2.72(m,2H),2.46-2.29(m,2H),2.06(s,3H), 1.56(d,J=6.6Hz,3H), 1.06(d,J=6.6Hz,3H) |
| 1-079; |
| 8.95(d,J=2.7Hz,1H),8.62(d,J=4.8Hz,1H),8.08(s,1H),8.00-8.10(m,1H),7.45(dd,J=4.8,8.1 Hz,1H),3.71(q,J=7.2Hz, 2H),3.08(s,2H),2.22(s,3H),1.18(t,J=7.2Hz,3H) |
| 1-083; |
| 8.95(d,J=2.4Hz,1H),8.62(dd,J=1.5,4.8Hz,1H),8.00-8.10(m,1H),7.96(s,1H),7.45(dd,J=4.8 ,8.1Hz,1H),3.72(q, J=7.2Hz,2H),2.70-3.15(m,5H),2.15-2.30(m,2H),1.16(t,J=7.2Hz,3H) |
| 1-085; |
| 8.95(d,J=2.7Hz,1H),8.63(dd,J=4.8, 1.5Hz,H),8.05(ddd,J=8.1,2.3,1.2Hz,1H),7.96(s,1H), 7.47(dd,J=8.1,4.5Hz,1H), 3.72(q,J=7.2Hz,2H),2.82(t,J=7.2Hz,2H),2.45(q,J=7.2Hz,2H),2. 44(t,J=7.5Hz,2H),1.22(t,J=7.5Hz,3H),1.16(t, J=7.2Hz,3H) |
| 1-086; |
| 8.95(d,J=2.4Hz,1H),8.61(d,J=4.8Hz,1H),8.06-8.00(m,1H),8.01(s,1H),7.44(dd,J=4.8,8.1 Hz,1H),5.60-4.40(m,2H), 3.46(s,3H),2.91-2.70(m,2H),2.52-2.44(m,1H),2.00(s,3H),1.20(d ,J=6.3Hz,3H) |
| 1-087; |
| 8.95(m,1H),8.56(m,1H),8.05-7.95(m,2H),7.45-7.35(m,1H),3.20(s,3H),3.20-3.25(m,H),2 .90-2.80(m,1H), 2.80-2.65(m,2H),2.59(s,3H),2.28(s,3H) |
| 1-088; |
| 8.95(m,1H),8.56(m,1H),8.05-7.95(m,2H),7.45-7.35(m,1H),3.41(t,J=7.2Hz,2H),3.24(s,3H ),2.96(s,3H),2.69(t, J=7.2Hz,2H),2.28(s,3H) |
| 1-095; |
| 8.93(d,J=1.5Hz,1H),8.55(dd,J=4.5,1.5Hz,1H),8.03(s,1H),7.99(dd,J=4.5,1.5Hz,1H),7.41( dd,J=8.1,4.5Hz,1H), 3.43(s,3H),3.09(s,2H),2.30(s,3H),2.24(s,3H) |
| 1-097; |
| 8.99(d,J=1.5Hz,1H),8.63(dd,J=4.5,1.5Hz,1H),8.22(s,1H),7.99(ddd,J=8.1,4.5,1.5Hz,1H), 7.44(ddd,J=8.1,4.5,1.5Hz, lH),3.89(s,2H),3.80-3.50(m,2H),3.56(m,2H),1.44(t,J=7.5Hz,3 H),1.20(t,J=7.5Hz,3H) |
| 1-098; |

(continued)

| No. |
|---|
| Proton NMR chemical shift (in $CDCl_3$): σ(ppm) |
| 8.95(d,J=2.7Hz,1H),8.61(d,J=4.8Hz,1H),8.00-8.10(m,1H),8.01(s,1H),7.45(dd,J=4.8,8.1 Hz,1H),3.75(q,J=7.2Hz, 2H),2.30(s,3H),1.20(t,J=7.2Hz,3H) |
| 1-102; |
| 8.94(d,J=2.4Hz,1H),8.62(d,J=4.8Hz,1H),8.13(s,1H),7.95-8.10(m,1H),7.35-7.55(m,1H),3. 26(s,3H),3.12(s,2H),2.23(s,3H) |
| 1-103; |
| 8.97(d,J=2.7Hz,1H),8.65(dd,J=4.8,0.9Hz,1H),8.06-8.05(m,2H),7.48(dd,J=8.1,4.5Hz,1H), 4.61(brs,1H),4.02(brs, 1H),3.10-2.57(m,3H),2.01(s,3H),1.32-1.24(m,6H) |
| 1-104; |
| 8.98(d,J=2.7Hz,1H),8.62(dd,J=5.1,1.5Hz,1H),8.08(s,1H),8.05(ddd,J=8.4,2.4,1.5Hz,1H), 7.46(dd,J=8.4,5.1Hz,1H), 3.69-3.66(m,2H),3.16(m,1H),2.88(m,1H),2.72-2.65(m,2H),2.60 (s,3H),1.58(m,2H),0.93(t,J=7.5Hz,3H) |
| 1-105; |
| 8.97(d,J=2.7Hz,1H),8.64(dd,J=5.1,0.9Hz,1H),8.04(ddd,J=8.7,2.7,1.5Hz,1H),8.02(s,1H), 7.46(dd,J=8.1,4.5Hz,1H), 3.63(brs,1H),3.43(brs,1H),2.96(s,3H),2.72(m,2H),1.64-1.52(m, 4H),0.94(t,J=7.2Hz,3H) |
| 1-106; |
| 8.99(d,J=3.0Hz,1H),8.56(d,J=5.1Hz,1H),8.07(dd,J=6.0Hz,1.5Hz,1H),8.06(s,1H),7.50-7. 40(m,1H),3.69(brs,2H), 3.08(s,2H),2.30(s,3H),2.22(s,3H),1.20-1.05(m,3H) |
| 1-107; |
| 8.94(d,J=1.5Hz,1H),8.61(dd,J=4.8Hz,1.5Hz,1H),8.07(s,1H),8.02(ddd,J=8.7Hz,4.8Hz,1.5 Hz,1H),7.44(dd,J=8.7Hz, 4.8Hz,1H),3.69(brs,2H),3.10(s,2H),2.67(q,J=7.5Hz,2H),1.25(t,J =7.5Hz,3H),1.17(t,J=7.5Hz,3H) |
| 1-109; |
| 8.95(d,J=1.5Hz,1H),8.62(dd,J=4.8,1.5Hz,1H),8.06(s,1H),8.03(ddd,J=8.1,4.8,1.5Hz,1H), 7.46(dd,J=8.1,4.8Hz,1H), 5.80-5.65(m,1H),5.17(dd,J=16.8Hz,J=1.SHz,1H),5.11(dd,J=10. 8,1.5Hz,1H),3.60(q,J=7.2Hz,2H),3.26(d,J=7.2Hz, 2H),3.06(s,2H),1.17(t,J=7.2Hz,3H) |
| 1-110; |
| 8.96(d,J=1.5Hz,1H),8.55(dd,J=4.8,1.5Hz,1H),8.04(ddd,J=8.4,4.8,1.5Hz,1H),7.98(s,1H), 7.42(dd,J=8.4,4.8Hz,1H), 3.69(brs,2H),3.20-3.05(m,1H),2.95-2.80(m,1H),2.70-2.60(m,2 H),2.59(s,3H),2.28(s,3H),1.15(t,J=7.2Hz,3H) |
| 1-111; |
| 8.96(d,J=1.5Hz,1H),8.55(dd,J=4.8,1.5Hz,1H),8.04(ddd,J=8.4,4.8,1.5Hz,1H),7.97(s,1H), 7.43(dd,J=8.4,4.8Hz,1H), 3.70(brs,2H),3.41(t,J=7.2Hz,2H),2.96(s,3H),2.65(t,J=7.2Hz,2H ),2.28(s,3H),1.15(t,J=7.2Hz,3H) |
| 1-112; |
| 8.98(d,J=2.7Hz,1H),8.62(d,J=4.8Hz,1H),8.20(s,1H),7.95-8.10(m,1H),7.40-7.55(m,1H),3. 87(q,J=7.2Hz,2H), 3.55-3.75(m,1H),2.54and2.64(s,3H),1.43(d,J=6.9Hz,3H),1.18and1.21 (t,J=7.2Hz,3H) |
| 1-113; |
| 8.97(d,J=2.7Hz,1H),8.62(dd,J=1.5,4.8Hz,1H),8.19(s,1H),7.95-8.05(m,1H),7.44(dd,J=4.8 ,9.0Hz,1H),3.98(q, J=7.2Hz,2H),3.45-3.60(m,1H),3.00(s,3H),1.65(d,J=7.2Hz,3H),1.21(t,J =7.2Hz,3H) |
| 1-117; |
| 8.96(d,J=2.4Hz,1H),8.63(dd,J=4.5,1.5Hz,1H),8.06(ddd,J=8.4,2.7,1.5Hz,1H),7.99(s,1H), 7.47(dd,J=8.7,4.5Hz,1H), 3.72(q,J=7.2Hz,2H),2.79(dt,J=8.4,6.6Hz,2H),2.49(t,J=6.6Hz,2 H),1.68(t,J=8.4Hz,1H),1.17(t,J=7.2Hz,3H) |
| 1-118; |
| 8.96(d,J=2.7Hz,1H),8.61(d,J=3.6Hz,1H),8.14(s,1H),8.00-8.10(m,1H),7.46(dd,J=4.8,8.1 Hz,1H),3.63(brs,1H), 3.77(brs,1H),3.30(q,J=6.9Hz,1H),2.50-2.70(m,2H),1.47(d,J=6.9Hz, 3H),1.17(t,J=7.5Hz,3H),1.16(t,J=7.5Hz,3H) |
| 1-119; |
| 8.93(d,J=2.7Hz,1H),8.51(dd,J=4.5Hz,1.5Hz,1H),8.05-7.95(m,2H),7.38(dd,J=8.4Hz,4.8H z,1H),3.50(q,J=7.2Hz,2H), 3.39(t,J=7.2Hz,2H),2.64(s,3H),2.54(t,J=7.2Hz,2H),2.25(s,3H), 2.09(s,3H),1.28(t,J=7.2Hz,3H) |
| 1-120; |
| (the isomer contained in a ratio of 1)8.95(d,J=2.7Hz,1H),8.61(d,J=3.6Hz,1H),8.11(s, 1H),8.10-8.00(m,1H),7.45(dd, J=4.8,8.1Hz,1H),3.85-3.55(brs,2H),2.93(t,J=7.2Hz,1H),2.0 6(s,3H),1.65(qui,J=7.2Hz,2H),1.17(t,J=7.2Hz,3H), 0.94(t,J=7.5Hz,3H) |

(continued)

| No. Proton NMR chemical shift (in $CDCl_3$): σ(ppm) |
|---|
| (the isomer contained in a ratio of 1)8.95(d,J=2.7Hz,1H),8.61(d,J=3.6Hz,1H),8.11(s, 1H),8.10-8.00(m,1H),7.45(dd, J=4.8,8.1Hz,1H),3.85-3.55(brs,2H),2.93(t,J=7.2Hz,1H),2.0 6(s,3H),2.06(qui,J=7.2Hz,2H),1.17(t,J=7.2Hz,3H), 0.94(t,J=7.5Hz,3H) |
| 1-121; |
| (the isomer contained in a ratio of 3)8.95(d,J=2.7Hz,1H),8.63(d,J=3.6Hz,1H),8.05(s, 1H),8.10-8.00(m,1H),7.46(dd, J=8.1,4.8Hz,1H),5.86(s,1H),3.75(brs,2H),2.23(d,J=2.4Hz, 3H),1.20(t,J=7.2Hz,3H) |
| (the isomer contained in a ratio of 2) |
| 8.95(d,J=2.7Hz,1H),8.63(d,J=3.6Hz,1H),8.05(s,1H),8.10-8.00(m,1H),7.46(dd,J=8.1,4.8 Hz,1H),5.69(s,1H),3.75(brs, 2H),2.23(d,J=2.4Hz,3H),1.20(t,J=7.2Hz,3H) |
| 1-122; |
| 8.94(d,J=2.7Hz,1H),8.62(dd,J=4.8,1.2Hz,1H),8.04(ddd,J=8.4,2.7,1.5Hz,1H),7.95(s,H), 7.46(dd,J=8.4,4.8Hz,1H), 3.71(q,J=7.2Hz,2H),2.80(m,2H),2.45(m,2H),2.42(m,2H),1.57( m,2H),1.16(t,J=7.5Hz,3H),0.94(t,J=7.5Hz,3H) |
| 1-123; |
| 8.94(d,J=2.4Hz,1H),8.62(dd,J=4.8,1.2Hz,1H),8.04(ddd,J=8.4,3.0,1.SHz,1H),7.95(s,1H), 7.46(dd,J=7.8,4.8Hz,1 H), 3.71 (q,J=7.2Hz,2H),2.86(m,1H),2.82(t,J=7.8Hz,2H),2.42(t,J=7. 8Hz,2H),1.23(d,J=6.9Hz,6H),1.16(t,J=7.2Hz,3H) |
| 1-124; |
| 8.95(d,J=2.7Hz,1H),8.63(dd,J=4.5,0.9Hz,1H),8.04(ddd,J=8.4,2.7,1.2Hz,1H),7.94(s,1H), 7.46(dd,J=8.4,4.8Hz,1H), 3.71(q,J=7.2Hz,2H),3.10(q,J=9.9Hz,2H),2.96(t,J=6.9Hz,2H),2. 47(t,J=6.9Hz,2H),1.17(t,J=7.2Hz,3H) |
| 1-125; |
| (the isomer contained in a ratio of 3)8.95(d,J=2.7Hz,1H),8.61(d,J=4.8Hz,1H),8.16(s, 1H),8.10-7.95(m,1H),7.45(dd, J=4.8,8.7Hz,1H),6.17(s,1H),2.72(brs,2H),2.21(s,3H),1.61( s,3H),1.18(t,J=7.2Hz,3H) |
| (the isomer contained in a ratio of 1)8.95(d,J=2.7Hz,1H),8.61(d,J=4.8Hz.1H),8.26(s, 1H),8.10-7.95(m,1H),7.45(dd, J=4.8,8.7Hz,1H),6.17(s,1H),2.75(brs,2H),2.16(s,3H),1.61( s,3H),1.18(t,J=7.2Hz,3H) |
| 1-127; |
| (the isomer contained in a ratio of 3)8.97(d,J=2.7Hz,1H),8.62(q,J=1.2Hz,1H),8.27(s, 1H),8.02(d,J=8.1Hz,1H),7.45(dd,J=8.1,4.8Hz,1H),5.94(s,1H),3.12(brs,2H),3.12(s,3H),2. 24(s,3H),1.20(d,J=2.4Hz, 3H) |
| (the isomer contained in a ratio of 2)8.97(d,J=2.7Hz,1H),8.62(q,J=1.2Hz,1H),8.27(s, 1H),8.02(d,J=8.1Hz,1H), 7.45(dd,J=8.1,4.8Hz,1H),5.94(s,1H),4.17(brs,2H),3.12(s,3H),2. 24(s,3H),1.20(d,J=2.4Hz,3H) |
| 1-134; |
| 8.95(d,J=1.5Hz,1 H),8.60(d,J=4.8Hz,1H),8.14(s,1H),8.04(d,J=8.4Hz,1H),7.45(dd,J=8.4H z,4.8Hz,1H),3.21(s,3H), 2.72(s,2H),2.14(s,3H),1.21(s,6H) |
| 1-138; |
| 8.93(d,J=1.5Hz,1H),8.53(dd,J=4.8Hz,1.5Hz,1H),8.00(ddd,J=8.4Hz,4.8Hz,1.5Hz,1H),7.9 5(s,1H),7.41 (dd,J=8.4Hz, 4.8Hz,1H),4.60(brs,1H),3.25(q,J=7.2Hz,2H),3.21 (s,3H),2.27(s, 3H),1.08(t,J=7.2Hz,3H) |
| 1-139; |
| 8.97(d,J=1.5Hz,1H),8.60(dd,J=4.8Hz,1.5Hz,1H),8.19(s,1H),7.99(dd,J=8.4Hz,4.8,1.5Hz, 1H),7.44(dd,J=8.4Hz, 4.8Hz,1H),3.47(brs,2H),3.24(s,3H),3.01(s,3H),1.26(s,6H) |
| 1-142; |
| 8.89(d,J=2.1Hz,1H),8.58(dd,J=4.8,1.2Hz,1H),8.04-7.95(m,1H),8.01(s,1H),7.43(dd,J=8.1 ,4.8Hz,1H),3.69(s,3H), 3.53(s,2H),3.33(s,3H),1.90(s,3H) |
| 1-143; |
| 8.90(d,J=2.4Hz,1H),8.58(dd,J=4.8,1.2Hz,1H),8.04-7.95(m,1H),7.98(s,1H),7.43(dd,J=8.1 ,4.8Hz,1H),3.78(q, J=7.2Hz,2H),3.68(s,3H),3.52(s,2H),1.90(s,3H),1.22(t,J=7.2Hz,3H) |
| 1-144; |
| 8.96(d,J=2.1Hz,1H),8.39(d,J=4.2Hz,1H),8.12(s,1H),8.10-8.00(m,H),7.47(dd,J=4.8,3.5 Hz,1H),4.48(brs,2H),2.80(t, J=7.5Hz,2H),2.50(t,J=7.5Hz,2H),2.26(t,J=2.7Hz,1H),2.08(s,3 H) |
| 1-146; |
| 8.97(d,J=2.7Hz,1H),8.63(d,J=4.8Hz,1H),8.16(s,1H),8.05-8.00(m,1H),7.46(dd,J=4.8,3.9 Hz,1H),4.45(brs,2H),3.43(t, J=7.0Hz,2H),2.97(s,3H),2.76(t,J=7.0Hz,2H),2.29(t,J=2.4Hz,1 H) |

(continued)

| No. Proton NMR chemical shift (in $CDCl_3$): σ(ppm) |
|---|
| 1-147; |
| 8.97(d,J=2.7Hz,1H),8.63(d,J=3.6Hz,H),8.15(s,1H),8.06-8.03(m,1H),7.46(dd,J=8.1,4.8 Hz,1H),4.65(brs,2H), 2.90-2.65(m,2H),2.55-2.45(m,1H),2.25(t,J=2.4Hz,1H),2.03(s,3H),1. 18(d,J=6.8Hz,3H) |
| 1-149; |
| 8.98(d,J=2.7Hz,1H),8.63(dd,J=4.8,1.5Hz,1H),8.07(ddd,J=8.4,2.7,1.5Hz,1H),8.04(s,1H), 7.47(dd,J=8.4,4.8Hz,1H), 3.71(q,J=7.2Hz,2H),1.97(s,3H),1.17(t,J=7.2Hz,3H) |
| 1-151; |
| 8.95(d,J=2.4Hz,1H),8.61 (dd,J=4.8,1.5Hz,1H),8.18(ddd,J=8.1,2.4,1.5Hz,1H),7.92(s,1H), 7.45(dd,J=8.4,4.8Hz,1H), 3.70(q,J=7.2Hz,2H),2.12(t,J=7.2Hz,2H),1.70-1.59(m,2H),1.15( t,J=7.2Hz,3H),0.89(t,J=7.5Hz,3H) |
| 1-153; |
| 8.95(d,J=2.7Hz,1 H),8.63(dd,J=4.8,1.5Hz,1H),8.05(ddd,J=8.4,2.7,1.5Hz,1H),7.97(s,1H), 7.46(dd,J=8.4,4.8Hz,1H), 3.73(t,J=7.5Hz,2H),2.54(t,J=7.2Hz,2H),2.10(s,3H),1.97(s,3H), 1.95-1.75(m,2H) |
| 1-155; |
| 8.96(s,1H),8.63(d,J=4.7Hz,1H),8.03(s,1H),8.05-8.00(m,1H),7.46(dd,J=8.3,4.8Hz,1H),3. 95(q,J=9.2Hz,2H),3.73(q, J=7.1Hz,2H),3.56(t,J=5.7Hz,2H),2.75(t,J=6.3Hz,2H),1.19(t,7.1 Hz,3H) |
| 1-156; |
| 8.95(d,J=2.4Hz,1H),8.62(dd,J=4.8,1.5Hz,1H),8.14(s,1H),8.04(ddd,J=8.4,2.4,1.5Hz,1H), 7.45(dd,J=8.4,4.8Hz,1H), 4.00-3.70(m,1H),3.70-3.40(m,1H),3.36-3.24(m,1H),3.21 (dd,J= 12.9,9.9Hz,1H),2.71(dd,J=12.9,4.2Hz,1H),2.11(s, 3H),2.10(s,3H),1.19(t,J=7.2Hz,3H) |
| 1-157; |
| 8.91(d,2.4Hz,1H),8.59(dd,J=4.8,1.2Hz,1H),8.05-8.00(m,1H),7.99(s,1H),7.43(dd,J=8.0,4. 8Hz,1H),3.93(q,7.1 Hz, 2H),3.80(q,J=7.1Hz,2H),3.56(s,2H),1.94(s,3H),1.25(t,7.1Hz,3H),1 .02(t,7.1Hz,3H) |
| 1-159; |
| (the isomer contained in a ratio of 3)8.92(d,J=3.0Hz,1H),8.61(dd,J=4.8,1.2Hz,1H),8. 04(s,1H),8.10-7.90(m,1H), 7.43(dd,J=8.1,4.8Hz,1H),5.20(s.2H),3.77(q,J=7.2Hz,2H),3.34 (s,2H),1.91(s,3H),1.21(t,J=7.2Hz,3H) |
| (the isomer contained in a ratio of 2)8.92(d,J=3.0Hz,1H),8.61(dd,J=4.8,1.2Hz,1H),8. 04(s,1H),8.10-7.90(m,1H), 7.43(dd,J=8.1,4.8Hz,1H),5.14(s,2H),3.77(q,J=7.2Hz,2H),3.34 (s,2H),1.91(s,3H),1.21(t,J=7.2Hz,3H) |
| 1-160; |
| (the isomer contained in a ratio of 3)8.95(d,J=2.7Hz,1H),8.63(dd,J=4.8,1.2Hz,1H),8. 05(s,1H),8.10-7.90(m,1H), 7.46(dd,J=8.1,4.8Hz,1H),3.93(t,J=6.6Hz,1H),3.82(brs,2H),3.3 2(s,3H),2.90-2.65(m,2H),2.06(s,3H),1.19(t,J=7.2Hz, 3H) |
| (the isomer contained in a ratio of 2)8.95(d,J=2.7Hz,1H),8.63(dd,J=4.8,1.2Hz,1H),8. 05(s,1H),8.10-7.90(m,1H), 7.46(dd,J=8.1,4.8Hz,1H),3.82(brs,2H),3.72(t,J=6.6Hz,1H),3.3 2(s,3H),2.90-2.65(m,2H),2.06(s,3H),1.19(t,J=7.2Hz, 3H) |
| 1-161; |
| 8.92(d,J=2.7Hz,1H),8.59(dd,J=4.8,1.2Hz,1H),8.05(s,1H),8.00(ddd,J=8.1,2.7,1.2Hz,1H), 7.43(dd,J=8.1,4.8Hz,1H), 5.17(s,2H),3.77(q,J=7.2Hz,2H),3.39(s,2H),3.37(q,J=7.2Hz,2H) ,1.20(t,J=7.2Hz,3H),1.18(t,J=7.2Hz,3H) |
| 1-162; |
| (the isomer contained in a ratio of 4)8.97(d,J=2.4Hz,1H),8.58(dd,J=4.8,1.2Hz,1H),8. 18(s,1H),8.00(ddd,J=8.4,2.4, 1.2Hz,1H),7.41(dd,J=8.4,4.8Hz,1H),4.05(d,J=12.4Hz,1H),3 .92(d,J=12.4Hz,1 H)3.82-3.72(m,2H),3.75(s,3H), 2.58(s,3H),1.22(t,J=7.2Hz,3H) |
| (the isomer contained in a ratio of 1)9.00-8.95(m,1H),8.61-8.55(m,1H),8.40(s,1H),8. 06-8.00(m,1H),7.45-7.37(m,1H), 4.05(d,J=12.4Hz,1H),3.92(d,J=12.4Hz,1H)3.82-3.72(m, 2H),3.79(s,3H),2.69(s,3H),1.17(t,J=7.2Hz,3H) |
| 1-166; |
| 8.90(m,2H),8.52(d,J=4.8Hz,1H),8.25(bs,1H),7.95-8.05(m,1H),7.70(s,1H),7.38(m,1H),3.3 2(s,2H),2.29(s,3H),1.54(s, 9H) |
| 1-167; |
| 8.94(d,J=2.7Hz,1H),8.62(dd,J=4.8,1.5Hz,1 H),8.17(s,1H),8.03(ddd,J=8.1,2.7,1.5Hz,1H), 7.45(dd,J=8.1,4.8Hz,1H), 3.42-3.34(m,1H),3.32-3.17(m,1H),3.29(s,3H),2.73(dd,J=13.2,4 .5Hz,1H),2.12(s,3H),2.10(s,3H) |

(continued)

| No. Proton NMR chemical shift (in $CDCl_3$): σ(ppm) |
| --- |
| 1-169; |
| 8.89(d,J=2.4Hz,1H),8.53(dd,J=4.8Hz,1.5Hz,1H),8.08(s,1H),7.97(ddd,J=9.0Hz,2.4Hz,1.5 Hz,1H),7.39(dd,J=9.0Hz, 4.8Hz,1H),3.49(s,2H),3.43(q,J=6.6Hz,2H),3.30(s,3H),2.34(s,3 H),2.13(s,3H),1.11(t,J=6.6Hz,3H) |
| 1-172; |
| 8.90(d,J=2.7Hz,1H),8.59(dd,J=4.8,1.2Hz,1H),8.05-8.00(m,1H),7.98(s,1H),7.43(dd,J=8.3 ,4.8Hz,1H),4.55(sep, J=6.3Hz,1H),3.79(q,J=6.8Hz,2H),3.57(s,2H),1.95(s,3H),1.23(t,J=6. 8Hz,3H),1.01(d,J=6.3Hz,6H) |
| 1-173; |
| 8.92(d,2.4Hz,1H),8.59(dd,J=4.8,1.2Hz,1H),8.05-7.95(m,1H),7.98(s,1H),7.44(dd,J=8.3,4. 8Hz,1H),4.25(m,2H), 3.85-3.75(m,2H),3.53(s,2H),1.95(s,3H),1.40(m,2H),1.23(t,J=7.1Hz, 3H),0.72(t,J=7.1Hz,3H) |
| 1-176; |
| 8.95(d,J=2.7Hz,1H),8.63(dd,J=4.8,1.5Hz,1H),8.14(s,1H),8.04(ddd,J=8.7,2.7,1.5Hz,1H), 7.46(dd,J=8.7,4.8Hz,1H), 4.12(t,J=10.2Hz,1H),3.61(dd,J=10.2,4.2Hz,1H),3.54-3.46(m,1 H),3.31(s,3H),2.12(s,3H) |
| 1-177; |
| 8.94(d,J=2.4Hz,1H),8.63(dd,J=4.2,1.2Hz,1H),8.03(ddd,J=8.1,2.4,1.2Hz,1H),7.97(s,1H), 7.46(dd,J=8.1,4.2Hz,1H), 3.25(s,3H),3.11(q,J=9.9Hz,2H),2.96(t,J=7.2Hz,2H),2.50(t,J=7. 2Hz,2H) |
| 1-178; |
| 8.95(d,J=2.7Hz,1H),8.62(dd,J=4.8,1.2Hz,1H),8.05(s,1H),8.02(ddd,J=8.7,2.7,1.2Hz,1H), 7.45(dd,J=8.7,4.8Hz,1H), 3.59-3.40(m,2H),3.32-3.24(m,1H),3.22(s,3H),3.14-3.06(m,1H), 2.75(t,J=6.6Hz,2H) |
| 1-179; |
| 8.95(d,J=2.4Hz,1H),8.63(dd,J=4.8,1.2Hz,1H),8.04(s,1H),8.02(ddd,J=8.7,2.4,1.2Hz,1H), 7.46(dd,J=8.7,4.8Hz,1H), 3.97(q,J=9.0Hz,2H),3.57(t,J=6.6Hz,2H),3.27(s,3H),2.79(t,J=6. 6Hz,2H) |
| 1-181; |
| 8.95(d,J=2.4Hz,1H),8.63(dd,J=4.8,1.2Hz,1H),8.04(s,1H),8.05-8.00(m,1H),7.47(dd,J=8.1 ,4.8Hz,1H),3.90-3.60(m, 6H),3.20-3.00(m,2H),2.60-2.40(m,2H),1.22(t,J=7.2Hz,3H),1.19( t,J=7.2Hz,3H) |
| 1-182; |
| 8.97(d,J=2.7Hz,1H),8.63(dd,J=4.8,1.2Hz,1H),8.13(s,1H),8.05(ddd,J=8.1,2.7,1.2Hz,1H), 7.46(dd,J=8.1,4.8Hz,1H), 3.78(brs,2H),2.90-2.60(m,5H),2.01(s,6H),1.19(t,J=7.2Hz,3H) |
| 1-183; |
| 8.81(d,J=2.7Hz,1H),8.59(d,J=4.8Hz,1H),7.95-7.85(m,1H),7.88(s,1H),7.41(dd,J=8.3,4.8 Hz,1H),7.15-7.10(m,5H), 4.96(s,2H),3.76(q,J=7.1Hz,2H),3.57(s,2H),1.95(s,3H),1.23(t,J= 7.1Hz,3H) |
| 1-184; |
| 8.92(d,J=2.7Hz,1H),8.59(d,J=4.8Hz,1H),8.05-7.95(m,1H),7.99(s,1H),7.44(dd,J=8.0,4.8 Hz,1H),3.87(t,J=6.5Hz,2H), 3.80(q,J=7.1Hz,2H),3.55(s,2H),1.95(s,3H),1.40(tt,J=7.1,6.5H z,2H),1.23(t,J=7.1Hz,3H),1.19(sxt,J=7.1Hz,2H), 0.75(t,J=7.1Hz,3H) |
| 1-185; |
| 8.96(d,J=2.7Hz,1H),8.64(dd,J=4.8,1.2Hz,1H),8.16(s,1H),8.01(ddd,J=8.1,2.7,1.2Hz,1H), 7.46(dd,J=8.1,4.8Hz,1H), 3.58(dd,J=7.8,7.2Hz,1H),3.31(s,3H),3.14(dd,J=14.4,7.8Hz,1H) ,2.97(dd,J=14.4,7.2Hz,1H),2.56(q,J=7.5Hz,2H), 1.23(t,J=7.5Hz,3H) |
| 1-186; |
| 8.95(d,J=2.7Hz,1H),8.63(d,J=4.8Hz,1H),8.10-8.00(ddd,J=8.1,2.7,1.8Hz,1H),8.05(s,1H), 7.46(dd,J=8.1,4.8Hz,1H), 3.85(t,J=7.5Hz,2H),2.70(t,J=7.5Hz,2H),2.13(s,3H),1.98(s,3H) |
| 1-190; |
| 8.91(d,J=2.4Hz,1H),8.59(dd,J=4.8,1.5Hz,1H),7.95(ddd,J=8.4,2.4,1.5Hz,1H),7.81(s,1H), 7.44(dd,J=8.4,4.8Hz,1H), 4.02(q,J=9.0Hz,2H),3.16(s,3H),2.75(s,3H) |
| 1-191; |
| 9.00-8.90(m,1H),8.65-8.55(m,1H),8.00(dd,J=8.1,1.SHz,1H),7.90(s,1H),7.50-7.40(m,1H), 3.70(s,3H),3.33(s,3H), 1.95(s,3H) |
| 1-193; |
| 8.88(d,J=2.4Hz,1H),8.52(d,J=4.5Hz,1H),8.00-7.95(m,1H),7.94(s,1H),7.39(dd,J=8.4,4.5 Hz,1H),3.68(s,3H),3.51(s, 2H),3.30(s,3H),2.34(s,3H),1.82(s,3H) |

(continued)

| No. Proton NMR chemical shift (in $CDCl_3$): σ(ppm) |
|---|
| 1-195; 8.97(d,J=2.7Hz,1H),8.65(dd,J=4.8,1.2Hz,H),8.12(s,1H),8.02(ddd,J=8.1,2.7,1.2Hz,1H), 7.46(dd,J=8.1,4.8Hz,1H), 4.00-3.41(m,3H),3.14(dd,J=13.5,7.5Hz,1H)2.97(dd,J=13.5,7.2 Hz,1H),2.97(q,J=7.5Hz,2H),1.23(t,J=7.5Hz,3H), 1.21(t,J=7.5Hz,3H) |
| 1-197; (the isomer contained in a ratio of 10)8.97(d,J=2.1Hz,1H),8.56-8.53(m,1H),8.25(brs, 1H),8.04-8.00(m,1H),7.81(s, 1H),7.39(dd,J=8.4,4.8Hz,1H),3.64-3.45(m,1H),3.17(s,3H),2. 10(s,3H),1.59(s,9H),1.49(d,J=7.2Hz,3H) (the isomer contained in a ratio of 7)8.97(d,J=2.1Hz,1H),8.56-8.53(m,1H),8.25(brs,1 H),8.04-8.00(m,1H),7.54(s,1H), 7.39(dd,J=8.4,4.8Hz,1H),3.64-3.45(m,1H),3.26(s,3H),2.1 1(s,3H),1.56(s,9H),1.42(d,J=7.2Hz,3H) |
| 1-198; 8.96(d,J=2.7Hz,1H),8.63(dd,J=4.8,1.2Hz,1H),8.06(ddd,J=8.4,2.7,1.2Hz,1H),7.93(s,1H), 7.47(dd,J=8.4,4.8Hz,1H), 3.72(q,J=7.2Hz,2H),2.80(t,J=7.5Hz,2H),2.44(t,J=7.5Hz,2H),2.0 7(s,3H),1.17(t,J=7.2Hz,3H) |
| 1-199; (the isomer contained in a ratio of 3)8.98(d,J=2.7Hz,1H),8.62(dd,J=4.8,1.2Hz,1H),8. 06(s,1H),8.10-8.00(m,1H), 7.43(dd,J=8.1,4.8Hz,1H),4.50-4.35(m,1H),4.10-3.80(m,1H),3. 75-3.50(m,1H),3.34(s,3H),3.25-3.05(m,1H), 2.90-3.30(m,1H),2.61(s,3H),1.19(t,J=7.2Hz, 3H) (the isomer contained in a ratio of 1)9.01(d,J=2.7Hz,1H),8.62(dd,J=4.8,1.2Hz,1H),8. 18(s,1H),8.10-8.00(m,1H), 7.44(dd,J=8.1,4.8Hz,1H),4.50-4.35(m,1H),4.10-3.80(m,1H),3. 75-3.50(m,1H),3.30(s,3H),3.25-3.05(m,1H), 2.90-3.30(m,1H),2.64(s,3H),1.20(t,J=7.2Hz, 3H) |
| 1-201; 8.96(d,J=2.7Hz,1H),8.63(dd,J=4.8,1.2Hz,1H),8.07(s,1H),8.03(ddd,J=8.1,2.7,1.2Hz,1H), 7.44(dd,J=8.1,4.8Hz,1H), 3.90-3.50(brs,2H),3.45(q,J=6.9Hz,1H),3.25(q,J=9.9Hz,2H),1.4 8(d,J=6.9Hz,3H),1.17(t,J=7.2Hz,3H) |
| 1-202; 8.97(d,J=2.7Hz,1H),8.63(dd,J=4.8,1.5Hz,1H),8.06(ddd,J=8.4,2.7,1.5Hz,1H),7.98(s,1 H), 7.46(dd,J=8.4,4.8Hz,1H), 4.10-3.30(m,2H),2.86(dd,J=12.6,8.7Hz,1H),2.75-2.55(m,1H)2. 47(dd,J=12.6,5.4Hz,1H),2.02(s,3H),1.18(t,J=6.9Hz, 3H),1.20-1.10(m,3H) |
| 1-203; 8.97(d,J=2.7Hz,1H),8.63(dd,J=4.8,1.5Hz,1H),8.06(ddd,J=8.4,2.7,1.5Hz,1H),8.02(s,1H), 7.46(dd,J=8.4,4.8Hz,1H), 3.85-3.60(m,2H),3.15(dt,J=13.5,7.2Hz,1H),2.95-2.80(m,1H)2.7 5-2.60(m,2H),2.59(s,3H),1.18(t,J=7.2Hz,3H) |
| 1-204; 8.97(d,J=3.0Hz,1H),8.64(dd,J=4.8,1.5Hz,1H),8.06(ddd,J=8.7,3.0,1.5Hz,1H),7.99(s,1H), 7.46(dd,J=8.7,4.8Hz,1H), 3.80-3.65(m,2H),3.50-3.35(m,2H),2.96(s,3H),2.71(t,J=6.9Hz,2 H),1.18(t,J=7.2Hz,3H) |
| 1-205; (the isomer contained in a ratio of 1)9.05(d,J=2.7Hz,1H),8.61(dd,J=4.8,1.5Hz,1H),8. 21 (s,1H),8.04(ddd,J=8.1,2.7, 1.5Hz,1H),7.44(dd,J=8.1,4.8Hz,1H),4.30-3.25(m,2H),4.10-3.25(m,1H),3.25-3.10(m,2H)2.59(s,3H),1.27(d,J=6.9Hz, 3H),1.18(t,J=7.2Hz,3H) (the isomer contained in a ratio of 1)9.00(d,J=2.7Hz,1H),8.60(dd,J=4.8,1.5Hz,1H),8. 15(s,11H),8.03(ddd,J=8.1,2.7, 1.5Hz,1H),7.41(dd,J=8.1,4.8Hz,1H),4.30-3.25(m,2H),4.10-3.25(m,1H),3.25-3.10(m,2H)2.59(s,3H),1.23(d,J=6.9Hz, 3H),1.16(t,J=7.2Hz,3H) |
| 1-206; 9.02(d,J=2.7Hz,1H),8.62(dd,J=4.8,1.2Hz,1H),8.17(s,1H),8.02(ddd,J=8.7,2.7,1.2Hz,1H), 7.43(dd,J=8.7,4.8Hz,1H), 4.25-4.05(m,H),3.81(dd,J=13.5,11.4Hz,1H)3.40-3.15(m,2H),2 .94(s,3H),2.87(dd,J=13.5,5.4Hz,1H),1.22(d,J=6.9Hz, 3H),1.17(t,J=7.2Hz,3H) |
| 1-208; 8.96(d,J=2.7Hz,1H),8.58(d,J=4.8Hz,1H),8.19(s,1H),8.05-7.95(m,1H),7.40(dd,J=8.3,4.8 Hz,1H),4.48(s,2H), 4.10-4.00(m,4H),2.59(s,3H),1.21(t,J=7.1Hz,3H),1.03(t,J=7.1Hz,3H) |
| 1-209; 8.96(d,J=2.7Hz,1H),8.59(d,J=4.8Hz,1H),8.08(s,1H),8.00-7.95(m,1H),7.42(dd,J=8.1,4.8 Hz,1H),4.48(s,2H),4.05(q, J=7.1Hz,2H),3.80-3.70(m,2H),2.86(s,3H),1.23(t,J=7.1Hz,3H), 1.05(t,J=7.1 Hz,3H) |

(continued)

| No. Proton NMR chemical shift (in $CDCl_3$): σ(ppm) |
|---|
| 1-210; 8.96(d,J=2.7Hz,1 H),8.63(dd,J=4.8,1.2Hz,1H),8.07(s,1H),8.03(ddd,J=8.1,2.7,1.2Hz,1H), 7.44(dd,J=8.1,4.8Hz,1H), 3.90-3.50(brs,2H),3.45(q,J=6.9Hz,1H),3.25(q,J=9.9Hz,2H),1.4 8(d,J=6.9Hz,3H),1.17(d,J=7.2Hz,3H) |
| 1-211; 8.90(d,J=2.7Hz,1H),8.59(d,J=4.8Hz,1H),8.01(ddd,J=8.1,2.7,1.5Hz,1H),7.88(s,1H),7.43( dd,J=8.1,4.8Hz,1H), 3.78(q,J=7.2Hz,2H),3.68(s,3H),1.94(s,3H),1.22(t,J=7.2Hz,3H) |
| 1-213; 9.04(d,J=2.4Hz,1 H),8.62(dd,J=4.8,1.5Hz,1 H),8.44(s,1H),8.04(ddd,J=8.4,2.4,1.5Hz,1H), 7.45(dd,J=8.4,4.8Hz,1H), 4.40-4.25(m,1H),3.25-3.10(m,1H),2.34(d,J=7.2Hz,1H),1.56(s,3 H),1.33(d,J=7.2Hz,1H),1.14(t,J=7.2Hz,3H) |
| 1-214; 8.90(d,J=2.4Hz,1H),8.59(dd,J=8.1,1.5Hz,1H),8.01(ddd,J=8.1,2.4,1.5Hz,1H),7.83(s,1H), 7.43(dd,J=8.1,4.8Hz,1H), 6.00-5.90(m,1H),3.74(q,J=7.2Hz,2H),2.25-2.05(m,2H),2.05-1.9 0(m,2H),1.80-1.40(m,4H),1.18(t,J=7.2Hz,3H) |
| 1-215; 8.94(d,J=2.4Hz,1H),8.65-8.55(m,1H),8.10-8.00(m,1H),7.92(s,1H),7.44(dd,J=8.4,4.8Hz,1 H),5.70-5.65(m,2H), 3.80-3.60(m,2H),2.55-2.25(m,2H),2.15-1.90(m,3H),1.90-1.70(m,2H) ,1.15(t,J=7.2Hz,3H) |
| 1-216; 8.74-8.60(m,1H),8.60(dd,J=4.8,1.5Hz,1H),7.95-7.85(m,1H),7.43(dd,J=8.4,4.8Hz,1H),7.2 6-7.24(m,4H), 7.09-7.03(m,2H),3.90-3.50(m,3H),3.30(dd.J=13.2,9.0Hz,1H),2.70(dd,J=13 .2,6.0Hz,1H),2.05(s,3H),1.12(t,J=7.2Hz, 3H) |
| 1-217; (the isomer contained in a ratio of 1)8.83-8.74(m,1H),8.60(dd,J=4.5,1.2Hz,1H),8.05-7.95(m,1H),7.42(dd,J=8.1,4.5Hz, 1H),7.29-7.26(m,4H),7.10-7.05(m,2H),4.25-4.05(m,2H) ,3.80-3.60(m,2H),2.73(dd,J=12.0,2.7Hz,1H),2.55(s,3H), 1.12(t,J=7.2Hz,3H)<br>(the isomer contained in a ratio of 1)8.83-8.74(m,1H),8.60(dd,J=4.5,1.2Hz,1H),8.05-7.95(m,1H),7.42(dd,J=8.1,4.5Hz, 1H),7.29-7.26(m,4H),7.10-7.05(m,2H),4.25-4.05(m,2H) ,3.80-3.60(m,1H),3.46(dd,J=12.9,5.4Hz,1H),3.08(dd, J=12.9,8.7Hz,1H),2.62(s,3H),1.15(t ,J=7.2Hz,3H) |
| 1-218; 9.00-8.88(m,1H),8.62(d,J=3.9Hz,1H),8.10-7.95(m,1H),7.44(dd,J=9.0,5.4Hz,1H),7.30-7.2 6(m,4H),7.08-7.05(m,2H), 4.40-4.00(m,4H),3.16-3.10(m,1H),2.89(s,3H),1.14(t,J=7.2Hz,3 H) |
| 1-219; 8.98(s,1H),8.61 (d,J=4.8Hz,1H),8.06(d,J=8.1 Hz,1H),8.01(s,1H),7.44(dd,J=8.1,4.8Hz,1H) ,3.92-3.70(m,1H), 3.66-3.48(m,1H),3.12(dd,J=13.2,6.6Hz,1H)2.84(dd,J=13.2,7.2Hz,1H), 2.64(dqd,J=7.2,6.9,6.6Hz,1H),2.26(s,3H), 1.19-1.10(m,6H) |
| 1-221; 8.91(d,J=2.7Hz,1H),8.60(d,J=4.8,0.9Hz,1H),8.05-7.95(m,1H),7.91(s,1H),7.44(dd,J=8.3, 4.8Hz,1H),3.78(q,J=7.1Hz, 2H),3.71(s,3H),3.64(s,2H),3.14(q,J=9.9Hz,2H),1.23(t,J=7.1H z,3H) |
| 1-222; 8.91(d,J=2.7Hz,1H),8.59(dd,J=4.8,1.2Hz,1H),8.05-7.95(m,1H),7.97(s,1H),7.43(dd,J=8.3 ,4.8Hz,1H),5.68(ddt, J=17.4,10.5,5.7Hz,1H),5.11(dd,J=17.4,1.2Hz,1H),5.00(dd,J=10.5,1. 2Hz,1H),4.37(d,J=5.7Hz,2H),3.78(q,J=7.1Hz, 2H),3.57(s,2H),1.96(s,3H),1.23(t,J=7.1Hz, 3H) |
| 1-223; 8.92(d,J=2.7Hz,1H),8.58(dd,J=4.8,0.9Hz,1H),8.05-7.95(m,1H),8.00(s,1H),7.43(dd,J=8.3 ,4.8Hz,1H),4.02(t, J=4.8Hz,2H),3.79(q,J=7.1Hz,2H),3.58(s,2H),3.35(t,J=4.8Hz,2H),3.14( s,3H),1.93(s,3H),1.23(t,J=7.1Hz,3H) |
| 1-225; (the isomer contained in a ratio of 3)8.97(d,J=2.7Hz,1H),8.62(dd,J=4.8,1.2Hz,1H),8. 17(s,1H),8.10-7.95(m,1H), 7.44(dd,J=8.1,4.8Hz,1H),3.91(q,J=6.9Hz,1H),4.05-3.55(m,2H ),2.90-2.70(m,2H),1.55-1.10(m,9H)<br>(the isomer contained in a ratio of 1)8.97(d,J=2.7Hz,1H),8.62(dd,J=4.8,1.2Hz,1H),8. 14(s,1H),8.10-7.95(m,1H), 7.44(dd,J=8.1,4.8Hz,1H),3.91(q,J=6.9Hz,1H),4.05-3.55(m,2H ),2.60-2.45(m,2H),1.55-1.10(m,9H) |
| 1-226; |

(continued)

| No. Proton NMR chemical shift (in $CDCl_3$): σ(ppm) |
|---|
| 8.97(d,J=2.7Hz,1 H),8.62(dd,J=4.8,1.2Hz,1H),8.20(s,1H),7.99(ddd,J=8.1,2.4,1.2Hz,1H), 7.43(dd,J=8.1,4.8Hz,1H), 4.03(q,J=7.2Hz,1H),4.10-3.35(m,2H),3.45-3.10(m,2H),1.64(d,J =7.2Hz,3H),1.38(t,J=7.5Hz,3H),1.21(t,J=7.2Hz,3H) |
| 1-227; |
| 8.91(d,J=2.4Hz,1H),8.59(dd,J=4.8,1.2Hz,1H),7.96-7.89(m,1H),7.91(s,1H),7.42(dd,J=8.1 ,4.8Hz,1H),4.73(s,2H), 3.80(s,3H)3.36(s,3H) |
| 1-228; |
| 8.92(d,J=2.4Hz,1H),8.59(dd,J=4.8,1.2Hz,1H),7.94(ddd,J=8.1,2.4,1.2Hz,1H),7.88(s,1H), 7.42(dd,J=8.1,4.8Hz,1H), 4.73(s,2H),3.86-3.73(m,2H)3.81(s,3H)1,23(t,J=7.2Hz,3H) |
| 1-229; |
| (the isomer contained in a ratio of 3)9.00(d,J=2.7Hz,1H),8.60(dd,J=4.8,1.2Hz,1H),8. 17(s,1H),8.10-7.95(m,1H), 7.50-7.35(m,1H),4.20-3.90(m,1H),3.80-3.15(m,5H),2.95-2.75( m,1H),1.30-1.10(m,6H) |
| (the isomer contained in a ratio of 1)8.99(d,J=2.7Hz,1H),8.60(dd,J=4.8,1.2Hz,1H),8. 19(s,1H),8.10-9.95(m,1H), 7.50-7.35(m,1H),4.20-3.90(m,IH),3.80-3.15(m,5H),2.95-2.75( m,1H),1.30-1.10(m,6H) |
| 1-230; |
| 8.99(d,J=2.4Hz,1H),8.62(dd,J=4.8,1.2Hz,1H),8.13(s,1H),8.00(ddd,J=8.1,2.4,1.2Hz,1H), 7.43(dd,J=8.1,4.8Hz,1H), 4.20-3.90(m,3H),3.90-3.70(m,1H),3.40-3.20(m,2H),3.20(dd,J= 14.1,2.7Hz,1H),1.22(d,J=7.2Hz,3H),1.17(t,J=7.2H, 3H) |
| 1-231; |
| 8.95(d,J=2.4Hz,1H),8.63(dd,J=4.8,1.2Hz,1H),8.03(ddd,J=8.1,2.4,1.2Hz,1H),8.00(s,1H), 7.46(dd,J=8.1,4.8Hz,1H), 3.26(s,3H),3.20-2.95(m,3H),2.85-2.70(m,1H),2.60-2.70(m,1H), 1.16(d,J=6.9Hz,3H) |
| 1-232; |
| 8.94(d,J=2.7Hz,1H),8.63(dd,J=4.8,1.2Hz,1H),8.04(ddd,J=8.1,2.7,1.2Hz,1H),8.01(s,1H), 7.46(dd,J=8.1,4.8Hz,1H), 3.71(q,J=7.2Hz,2H),3.32(q,J=9.9Hz,2H),3.25(s,2H),1.18(t,J=7. 2Hz,3H) |
| 1-233; |
| 8.96(d,J=2.7Hz,1H),8.59(dd,J=4.8,1.2Hz,1H),8.04(s,1H),8.00(ddd,J=8.1,2.7,1.2Hz,1H), 7.43(dd,J=8.1,4.8Hz,1H), 4.25-4.05(m,2H),3.85-3.45(m,7H),1.23(t,J=7.1Hz,3H) |
| 1-234; |
| 8.94(d,J=2.7Hz,1H),8.58(dd,J=4.8,1.2Hz,1H),8.05-7.95(m,1H),7.97(s,1H),7.41(dd,J=8.1 ,4.8Hz,1H),3.88(q, J=9.9Hz,2H),3.85-3.75(m,7H),1.24(t,J=7.1Hz,3H) |
| 1-235; |
| 8.89(d,J=2.7Hz,1H),8.59(dd,J=4.8,1.2Hz,1H),7.99(ddd,J=8.1,2.7,1.2Hz,1H),7.93(s,1H), 7.43(dd,J=8.1,4.8Hz,1H), 3.71(s,3H),3.65(s,2H),3.34(s,3H),3.11(q,J=9.9Hz,2H) |
| 1-236; |
| 8.94(d,J=2.7Hz,1 H),8.59(dd,J=4.8,1.2Hz,1H),8.07(s,1H),8.00-7.95(m,1H),7.42(dd,J=8.1 ,4.8Hz,1H),4.25(m,2H), 3.80-3.30(m,8H) |
| 1-237; |
| 8.93(d,J=2.7Hz,1H),8.58(dd,J=4.8,1.2Hz,1H),7.99(s,1H),8.00-7.90(m,1H),7.42(dd,J=8.1 ,4.8Hz,1H),4.59(s,2H), 3.95(q,J=8.7Hz,2H),3.79(s,3H),3.35(s,3H) |
| 1-240; |
| 8,94(d,J=2.4Hz,1 H),8.63(dd,J=4.8,1.2Hz,1H),8.08(s,1H),8.01(ddd,J=8.1,2.4,1.2Hz,1H), 7.45(dd,J=8.1,4.8Hz,1H), 3.53(q,J=7.2Hz,1H),3.30-3.19(m,2H),3.25(s,3H),1.48(d,J=7.2 Hz,3H) |
| 1-241; |
| 8,96(d,J=2.4Hz,1H),8.64(dd,J=4.8,1.2Hz,1H),8.18(s,1H),7.99(ddd,J=8.7,2.4,1.2Hz,1H), 7.45(dd,J=8.7,4.8Hz,1H), 4.31-4.00(m,3H),3.34(s,3H),1.67(d,J=7.2Hz,3H) |
| 1-242; |
| 8,94(d,J=2.7Hz,1H),8.63(dd,J=4.5,1.5Hz,1H),8.12(s,1H),8.03(ddd,J=8.4,2.7,1.5Hz,1H), 7.46(dd,J=8.4,4.5Hz,1H), 3.25(s,3H),3.14(s,2H),2.70(q,J=7.2Hz,2H),1.27(t,J=7.2Hz,3H) |
| 1-243; |
| 8,97(d,J=2.4Hz,1H),8.62(dd,J=4.8,1.2Hz,1H),8.22(s,1H),8.00(ddd,J=8.1,2.4,1.2Hz,1H), 7.44(dd,J=8.1,4.8Hz,1H), 3.69(d,J=13.5Hz,1H),3.56(d,J=13.5Hz,1H),3.30(s,3H),3.03-2.8 |

(continued)

| No. Proton NMR chemical shift (in $CDCl_3$): σ(ppm) |
|---|
| 8(m,2H),1.35(t,J=7.5Hz,3H) |
| 1-245; 8.98(d,J=2.7Hz,1 H),8.62(dd,J=4.8,1.2Hz,1H),8.12(s,1H),8.05(ddd,J=8.1,2.7,1.2Hz,1H), 7.45(dd,J=8.1,4.8Hz,1H), 4.28(q,J=7.2Hz,1H),3.95-3.50(m,2H),2.25(s,3H),1.43(d,J=7.2 Hz,3H),1.16(t,J=7.2Hz,H) |
| 1-246; 8.95(d,J=2.4Hz,1 H),8.61 (dd,J=4.8,1.2Hz,1H),8.11(s,1H),8.03(ddd,J=8.1,2.4,1.2Hz,1H), 7.45(dd,J=8.1,4.8Hz,1H), 3.95-3.50(m,2H),3.28(q,J=6.9Hz,1H),2.65-2.45(m,2H),1.52(t,J =7.2Hz,2H),1.46(d,J=6.9Hz,3H),1.16(t,J=7.2Hz,3H), 0.94(t,J=7.2Hz,3H) |
| 1-248; 8.95(d,J=2.4Hz,1H),8.62(dd,J=4.8,1.2Hz,1H),8.10(s,1H),8.03(ddd,J=8.1,2.4,1.2Hz,1H), 7.45(dd,J=8.1,4.8Hz,1H), 3.90-3.50(m,2H),3.34(q,J=6.9Hz,1H),2.96(qui,J=6.9Hz,1H),1.4 9(d,J=6.9Hz,3H),1.25-1.05(m,9H) |
| 1-251; 8.97(d,J=2.4Hz,1H),8.59(dd,J=4.8,1.2Hz,1H),8.16(s,1H),8.02(ddd,J=8.1,2.4,1.2Hz,1H), 7.44(dd,J=8.1,4.8Hz,1H), 4.07(q,J=6.9H,1H),3.95-3.55(m,2H),1.60(d,J=6.9Hz,3H),1.18(t ,J=7.2Hz,3H) |
| 1-252; 8.97(d,J=2.4Hz,1H),8.62(dd,J=4.8,1.2Hz,1H),8.14(s,1H),8.03(ddd,J=8.1,2.4,1.2Hz,1H), 7.45(dd,J=8.1,4.8Hz,1H), 3.95-3.30(m,5H),1.54(d,J=6.9Hz,3H),1.19(t,J=7.2Hz,3H) |
| 1-253; (the isomer contained in a ratio of 54)8.98(d,J=2.4Hz,1H),8.64(dd,J=4.8,1.2Hz,1H),8 .10(s,1H),8.04(ddd,J=8.1,2.4, 1.2Hz,1H),7.47(dd,J=8.1,4.8Hz,1H),4.35-3.55(m,4H),3.55-3.30(m,1H),1.54(d,J=6.9Hz,3H),1.18(t,J=7.2Hz,3H) (the isomer contained in a ratio of 46)8.98(d,J=2.4Hz,1H),8.61(dd,J=4.8,1.2Hz,1H),8 .17(s,1H),7.99(ddd,J=8.1,2.4, 1.2Hz,1H),7.43(dd,J=8.1,4.8Hz,1H),4.35-3.55(m,4H),3.30-3.05(m,1 H),1.37(d,J=6.9Hz,3H),1.22(t,J=7.2Hz,3H) |
| 1-256; 8.97(d,J=2.4Hz,1H),8.52(dd,J=4.8,1.5Hz,1H),8.21(brs,1H),8.02(ddd,J=8.4,2.4,1.5Hz,1H ),7.66(s,1H),7.38(dd, J=8.4,4.8Hz,1H),1.55-1.50(m,27H) |
| 1-257; 8.97(brs,1H),8.51(brs,1H),8.22(brs,1H),8.05-8.95(m,1H),7.75(s,1H),7.45-7.30(m,1H),6.7 3(brs,1H),1.60-1.1.50(m, 18H) |
| 1-259; 8.90(d,J=2.1 Hz,1H),8.59(dd,J=4.8,1.2Hz,1H),8.04-7.94(m,1H),7.99(s,1H),7.42(dd,J=8.1 ,4.8Hz,1H),3.69(s,3H), 3.56(s,2H),3.33(s,3H),2.33(q,J=7.2Hz,2H),1.16(t,J=7.2Hz,3H) |
| 1-260; (the isomer contained in a ratio of 3)8.96(d,J=2.4Hz,1H),8.60-8.54(m,1H),8.24(s,1H) ,7.99(ddd,J=8.1,2.4,1.2Hz,1H), 7.44-7.37(m,1H),3.98(d,J=12.6Hz,1H),3.93(d,J=12.6Hz, 1H),3.74(s,3H),3.32(s,3H),2.95-2.68(m,2H),1.32(t, J=7.2Hz,3H) (the isomer contained in a ratio of 2)8.99(d,J=2.7Hz,1H),8.60-8.54(m,1H),8.52(s,1H) ,8.07-7.99(m,1H),7.44-7.37(m, 1H),3.98(d,J=12.6Hz,1H),3.93(d,J=12.6Hz,1H),3.76(s,3H ),3.33(s,3H),2.95-2.68(m,2H),1.36(t,J=7.2Hz,3H) |
| 1-262; 8.90(d,J=2.4Hz,1H),8.59(dd,J=4.8,1.2Hz,1H),8.04-7.94(m,1H),7.97(s,1H),7.43(dd,J=8.1 ,4.8Hz,1H),3.78(q, J=7.2Hz,2H),3.68(s,3H),3.55(s,2H),2.33(q,J=7.2Hz,2H),1.22(t,J=7.2H z,3H),1.16(t,J=7.2Hz,3H) |
| 1-263; (the isomer contained in a ratio of 4)8.97(d,J=2.4Hz,1H),8.57(dd,J=4.8,1.2Hz,1H),8. 20(s,1H),8.00(ddd,J=8.4,2.4, 1.2Hz,1H),7.40(dd,J=8.4,4.8Hz,1H),3.98(d,J=12.3Hz,1H),3 .90(d,J=12.3Hz,1H),3.83-3.69(m,2H),3.74(s,3H), 2.95-2.64(m,2H),1.25(t,J=7.2Hz,3H),1. 21(t,J=7.2Hz,3H) (the isomer contained in a ratio of 1)8.99(d,J=2.7Hz,1H),8.60-8.54(m,1H),8.46(s,1H) ,8.07-8.00(m,1H),7.46-7.37(m, 1H),3.98(d,J=12.3Hz,1H),3.90(d,J=12.3Hz,1H),3.83-3.69 (m,2H),3.74(s,3H),2.95-2.64(m,2H),1.36(t,J=7.2Hz,3H), 1.17(t,J=7.2Hz,3H) |
| 1-267; 8.95(d,J=2.7Hz,1H),8.58(dd,J=4.8,1.5Hz,1H),8.04(s,1H),8.05-7.95(m,1H),7.42(dd,J=8.3 ,4.8Hz,1H),4.93(s,2H), 3.81(q,J=7.1Hz,2H),3.57(s,2H),3.10(s,3H),1.94(s,3H),1.22(t,J=7. 1Hz,3H) |

(continued)

| No. Proton NMR chemical shift (in $CDCl_3$): σ(ppm) |
|---|
| 1-268; |
| 8.90(d,J=3.0Hz,1H),8.59(dd,J=4.8,1.5Hz,1H),7.98(s,1H),8.00-7.95(m,1H),7.41(dd,J=8.3 ,4.8Hz,1H),3.94(t, J=8.3Hz,2H),3.78(q,J=7.1Hz,2H),3.55(s,2H),1.97(s,3H),1.23(t,J=7.1H z,3H),0.73(t,J=8.3Hz,2H),-0.075(s,9H) |
| 1-270; |
| (the isomer contained in a ratio of 54)8.96(d,J=3.0Hz,1H),8.70-8.55(m,1H),8.19(s,1 H),8.10-7.95(m,1H),7.45(dd, J=8.7,4.8Hz,1H),4.10-3.90(m,1H),3.32(s,3H),2.65(s,3H),1.4 3(d,J=6.9Hz,3H) |
| (the isomer contained in a ratio of 46)8.96(d,J=3.0Hz,1H),8.70-8.55(m,1H),8.22(s,1 H),8.10-7.95(m,1H),7.43(dd, J=8.7,4.8Hz,1H),4.10-3.90(m,1H),3.29(s,3H),2.55(s,3H),1.3 8(d,J=6.9Hz,3H) |
| 1-273; |
| 8.90(d,J=2.4Hz,1H),8.63-8.57(m,1H),7.98(ddd,J=8.1,2.4,1.2Hz,1H),7.92(s,1H),7.43(dd, J=8.1,4.5Hz,1H),4.45(s, 2H),3.91(q,J=8.4Hz,2H),3.76(s,3H),3.33(s,3H) |
| 1-274; |
| 8.91(d,J=2.7Hz,1H),8.60(dd,J=4.8,1.2Hz,1H),7.98(ddd,J=8.4,2.7,1.2Hz,1H),7.90(s,1H), 7.43(dd,J=8.4,4.8Hz,1H), 4.43(s,2H),3.91(q,J=8.5Hz,2H),3.78(q,J=7.2Hz,2H),3.75(s,3H) ,1.21(t,J=7.2Hz,3H) |
| 1-276; |
| 8,95(d,J=2.4Hz,1H),8.61 (dd,J=4.8,1.2Hz,1H),8.11(s,1H),8.03(ddd,J=8.1,2.4,1.2Hz,1H), 7.44(dd,J=8.1,4.8Hz,1H), 5.60-5.20(m,1H),4.80-4.40(m,1H),3.46(s,3H),3.37(q,J=6.9Hz,1 |
| H),2.66-2.51(m,2H),1.50(d,J=6.9Hz,3H),1.16(t,J=7.5Hz,3H) |
| 1-277; |
| 8.95(d,J=2.4Hz,1H),8.62(dd,J=4.8,1.2Hz,1H),8.11(s,1H),8.05(ddd,J=8.1,2.4,1.2Hz,1H), 7.46(dd,J=8.1,4.8Hz,1H), 4.71(d,J=12.0Hz,1H),4.55(d,J=12.0Hz,1H),4.00-3.75(m,1H),3. 55(q,J=7.2Hz,2H),3.27(s,3H),1.49(d,J=6.9Hz,3H), 1.17(t,J=7.2Hz,3H) |
| 1-278; |
| 8.93(d,J=2.4Hz,1H),8.58(dd,J=4.8,1.2Hz,1H),8.13(s,1H),8.01 (ddd,J=8.1,2.4,1.2Hz,1H), 7.43(dd,J=8.1,4.8Hz,1H), 3.90-3.45(m,2H),3.32(q,J=6.9Hz,1H),2.55-2.35(m,2H),1.44(d,J =6.9Hz,3H),1.14(t,J=7.2Hz.3H),0.90-0.70(m,1H), 0.60-0.30(m,2H),0.30-0.60(m,2H) |
| 1-279; |
| 8.92(d,J=2.7Hz,1H),8.61(dd,J=4.8,1.SHz,1H),8.10-8.00(m,1H),7.97(s,1H),7.50-7.40(m,1 H),7.30-7.10(m,1H), 4.80-4.65(m,1H),3.95-3.75(m,1H),3.75-3.50(m,1H),1.81(m,3H),1.36 (t,J=7.2Hz,3H),1.25-1.10(m,3H) |
| 1-284; |
| 8,98(d,J=2.7Hz,1H),8.65(dd,J=4.8,1.2Hz,1H),8.32(s,1H),8.02(ddd,J=8.4,2.7,1.2Hz,1H), 7.47(dd,J=8.4,4.8Hz,1H), 5.10-4.80(m,1H),4.40-4.10(m,1H),3.33(q,J=6.9Hz,1H),2.67-2.5 3(m,2H),1.49(d,J=6.9Hz,3H),1.20(t,J=7.2Hz,3H) |
| 1-285; |
| 8.96(d,J=2.7Hz,1H),8.63(dd,J=4.8,1.2Hz,1H),8.04(s,1H),8.01(ddd,J=8.3,2.7,1.2Hz,1H), 7.45(dd,J=8.3,4.8Hz,1H), 4.75-4.10(m,2H),4.05(s,2H),3.95-3.70(m,2H),1.20(t,J=7.1Hz,3 H) |
| 1-286; |
| 8.96(d,J=2.4Hz,1H),8.62(dd,J=4.8,1.2Hz,1H),8.08(s,1H),8.03(ddd,J=8.3,2.4,1.2Hz,1H), 7.41(dd.J=8.3,4.8Hz,1H), 3.90-3.50(m,5H),2.08(s,3H),1.49(d,J=7.1Hz,3H),1.17(t,J=7.1H z,3H) |
| 1-288; |
| 8.93(m,1H),8.63-8.59(m,1H),8.05(s,1H),8.02(ddd,J=8.1,2.7,1.2Hz,1H),7.46(dd,J=8.1,4. 8Hz,1H),3.32(q,J=9.9Hz, 2H),3.28(s,2H),3.26(s,3H) |
| 1-289; |
| 8.95(d,J=2.4Hz,1H),8.62(dd,J=4.8,1.2Hz,1H),8.22(s,1H),8.04(ddd,J=8.1,2.4.1.2Hz,1H), 7.45(dd,J=8.1,4.8Hz,1H), 4.97-4.60(m,1H),4.30-3.95(m,1H),3.39-3.27(m,1H),2.66-2.52( m,2H),2.25(t,J=2.1Hz,1H),1.48(d,J=6.9Hz,3H), 1.17(t,J=7.2Hz,3H) |
| 1-291; |
| 8.95(d,J=2.4Hz,1H),8.61(dd,J=4.8,1.5Hz,1H),8.10-8.00(m,2H),7.44(dd,J=8.3,2.4Hz,1H), 3.35-3.20(m,1H), 3.40-3.20(m,2H),2.65-2.50(m,2H),1.47(d,J=7.1Hz,3H),1.17(t,J=7.1Hz, 3H),1.16(t,J=7.1Hz,3H) |
| 1-292; |

(continued)

| No. Proton NMR chemical shift (in $CDCl_3$): σ(ppm) |
|---|
| 9.06(d,J=2.7Hz,1H),8.62(dd,J=4.8,1.2Hz,1H),8.25(s,1H),8.08(ddd,J=8.0,2.7,1.2Hz,1H), 7.44(dd,J=8.0,4.8Hz,1H), 6.75(brs,1H),3.75-3.65(m,2H),3.54(q,J=7.1Hz,1H),3.25(d,J=17 .0Hz,1H),3.18(d,J=17.0Hz,1H),2.79(d,J=5.1Hz,3H), 1.44(d,J=7.1Hz,3H),1.15(t,J=7.1Hz, 3H) |
| 1-293; |
| 9.00(d,J=2.7Hz,1H),8.61 (dd,J=4.8,1.2Hz,1H),8.25(s,1H),8.05(ddd,J=8.6,2.7,1.2Hz,1H), 7.44(dd,J=8.6,4.8Hz,1H), 3.66(s,3H),3.60-3.30(m,5H),1.45(d,J=6.8Hz,3H),1.16(t,J=7.2H z,3H) |
| 1-300; |
| 8.94(d,J=2.7Hz,1H),8.60(dd,J=4.8,1.2Hz,1H),8.31(s,1H),8.00(ddd,J=8.4,2.7,1.2Hz,1H), 7.42(dd,J=8.4,4.8Hz,1H), 4.80-4.50(m,1H),4.30-3.80(m,1H),4.19(q,J=7.2Hz,2H),3.41(q,J =6.6Hz,1H),2.66-2.49(m,2H),1.46(d,J=6.6Hz,3H), 1.27(t,J=7.2Hz,3H),1.17(t,J=7.2Hz,3H ) |
| 1-302; |
| 8.88(d,J=2.4Hz,1H),8.56-8.51(m,1H),7.98(ddd,J=8.1,2.4,1.5Hz,1H),7.93(s,1H),7.42-7.3 4(m,1H),4.82(q,J=6.9Hz, 1H),3.77(s,3H),2.69-2.44(m,2H),1.52(d,J=6.9Hz,3H),1.20(t,J=7 .2Hz,3H) |
| 1-303; |
| 8.95(d,J=2.7Hz,1H),8.63(dd,J=4.8,1.2Hz,1H),8.05(ddd,J=8.1,2.7,1.2Hz,1H),7.96(s,1H), 7.46(dd,J=8.1,4.8Hz,1H), 3.98(s,3H),3.70(q,J=7.2Hz,2H),3.33(s,2H),1.16(t,J=7.2Hz,3H) |
| 1-304; |
| 8.95(d,J=2.7Hz,1H),8.68-8.61(m,1H),8.15(s,1H),8.00(ddd,J=8.1,2.7,1.2Hz,1H),7.46(dd, J=8.1,4.8Hz,1H), 4.42-4.20(m,2H),4.08(s,2H),3.33(s,3H) |
| 1-305; |
| 8.85-9.15(m,1H),8.55-8.65(m,1H),7.95-8.55(m,2H),7.35-7.50(m,1H),3.45-4.25(m,3H),2. 45-3.35(m,2H), 1.10-1.65(m,9H) |
| 1-307; |
| 8.96(d,J=2.4Hz,1H),8.61 (dd,J=4.8,1.2Hz,1H),8.17(s,1H),8.04(ddd,J=8.1,2.4,1.2Hz,1H), 7.45(dd,J=8.1,4.8Hz,1H), 3.55-3.90(m,2H),1.45-1.50(m,2H),1.20-1.30(m,2H),1.17(t,J=7. 2Hz,3H) |
| 1-308; |
| 8.92(d,J=2.4Hz,1H),8.62(dd,J=4.8,1.5Hz,1H),8.05-7.95(m,2H),7.45(dd,J=8.4,4.8Hz,1H), 5.35(brs,1H),3.80-3.75(m, 2H),3.26(s,3H),1.42(s,9H) |
| 1-309; |
| 8,98-8.97(m,1H),8.63-8.60(m,1H),8.15(s,1H),8.03-7.98(m,1H),7.46-7.41(m,1H),5.60-5.4 0(m,1H),4.70-4.50(m,1H), 4.15-3.98(m,1H),3.48(s,3H),2.86-2.49(m,2H),1.41(d,J=6.9Hz, 3H),1.38-1.24(m,3H) |
| 1-310; |
| 8,98(d,J=2.7Hz,1H),8.62(dd,J=4.8,1.5Hz,1H),8.18(s,1H),8.00(ddd,J=8.4,2.7,1.5Hz,1H), 7.43(dd,J=8.4,4.8Hz,1H), 5.52(d,J=10.5Hz,1H),4.57(d,J=10.5Hz,1H),4.12(q,J=7.2Hz,1H) ,3.49(s,3H),3.34-3.20(m,1H),3.19-3.07(m,1H), 1.67(d,J=7.2Hz,3H),1.38(t,J=7.5Hz,3H) |
| 1-313; |
| 8.94(d,J=2.4Hz,1H),8.64(dd,J=4.8,1.5Hz,1H),8.03(s,1H),8.02(ddd,J=8.4,2.4,1.5Hz,1H), 7.45(dd,J=8.4,4.8Hz,1H), 5.20-5.10(m,1H),3.77(d,J=4.8Hz,2H),3.43(s,3H),3.14(s,3H) |
| 1-314; |
| (the isomer contained in a ratio of 52)8.98(d,J=2.7Hz,1H),8.61(dd,J=4.8,1.2Hz,1H),8 .25(s,1H),8.05-7.95(m,1H), 7.50-7.35(m,1H),3.84(dd,J=11.3,3.3Hz,1H),3.32(s,3H),3.00-2.50(m,2H),2.15-1.85(m,2H),1.28(t,J=7.1Hz,3H), 1.10-0.95(m,3H) |
| (the isomer contained in a ratio of 48)8.97(d,J=2.7Hz,1H),8.60(dd,J=4.8,1.2Hz,1H),8 .30(s,1H),8.05-7.95(m,1H), 7.50-7.35(m,1H),4.00-3.95(m,1H),3.33(s,3H),3.00-2.50(m,2H ),2.15-1.85(m,2H),1.36(t,J=7.1Hz,3H),1.10-0.95(m, 3H) |
| 1-315; |
| 8.93(d,J=2.7Hz,1H),8.63(dd,J=4.8,1.2Hz,1H),8.04(s,1H),8.05-8.00(m,1H),7.46(dd,J=8.3 ,4.8Hz,1H),4.00-3.40(m, 3H),1.49(d,J=6.5Hz,3H),1.20-1.10(m,12H) |
| 1-318; |

(continued)

| No. Proton NMR chemical shift (in $CDCl_3$): σ(ppm) |
|---|
| 8.96(d,J=2.7Hz,1H),8.64(dd,J=4.8,1.2Hz,1H),8.17(s,1H),8.04(ddd,J=8.4,2.7,1.2Hz,1H), 7.47(dd,J=8.4,4.8Hz,1H), 4.60-4.43(m,1H),4.15-4.00(m,1H),3.70-3.55(m,1H),2.60(q,J=6. 9Hz,2H),1.63(d,J=6.9Hz,3H),1.29-1.23(m,3H), 1.11(t,J=6.9Hz,3H) |
| 1-319; |
| 8,93(d,J=2.7Hz,1H),8.60(dd,J=4.8,1.2Hz,1H),8.17(s,1H),8.02(ddd,J=8.4,2.7,1.2Hz,1H), 7.43(dd,J=8.4,4.8Hz,1H), 4.70-3.10(m,1H),2.71-2.59(m,1H),2.56-2.44(m,1H),1.57(d,J=6. 6Hz,3H),1.23(t,J=7.5Hz,3H) |
| 1-320; |
| 8.91(d,J=3.0Hz,1H),8.61(dd,J=4.8,1.5Hz,1H),8.46(d,J=6.0Hz,2H),8.00(ddd,J=8.3,3.0,1. 5Hz,1H),7.89(s,1H), 7.45(dd,J=8.3,4.8Hz,1H),7.20(d,J=6.0Hz,2H),3.80-3.50(m,2H),3.78( d,J=13.7Hz,1H),3.74(d,J=13.7Hz,1H),3.33(q, J=6.8Hz,1H),1.47(d,J=6.8Hz,3H),1.14(t,J= 7.1Hz,3H) |
| 1-324; |
| 8.92(d,J=2.4Hz,1H),8.63-8.55(m,1H),8.09-7.90(m,2H),7.42(dd,J=8.1,4.8Hz,1H),4.16(q,J =8.4Hz,2H),1.43(brs,9H) |
| 1-328; |
| 8.89(d,J=2.4Hz,1H),8.58(dd,J=4.8,1.SHz,1H),8.00(s,1H),8.00-7.90(m,1H),7.68(d,J=7.2 Hz,1H),7.56(t,J=7.2H,1H), 7.45-7.35(m,2H),3.43(s,3H) |
| 1-331; |
| 8.05-8.00(m,2H),7.91(s,1H),7.60-7.45(m,2H),7.30-7.20(m,3H)3.53(s,2H),3.26(s,3H) |
| 1-333; |
| 8.93(d,J=2.7Hz,1H),8.61(dd,J=4.8,1.2Hz,1H),8.01(ddd,J=8.4,2.7,1.2Hz,1H),7.97(s,1H), 7.44(dd,J=8.4,4.8Hz,1H), 6.64(d,J=3.6Hz,1H),6.30(d,J=3.6Hz,1H),3.40(s,3H) |
| 1-335; |
| 8.86(d,J=2.7Hz,1H),8.60(dd,J=4.8,1.2Hz,1H),7.96(ddd,J=8.4,2.7,1.2Hz,1H),7.86(s,1H), 7.43(dd,J=8.4,4.8Hz,1H), 3.79(s,2H),3.40(s,3H),2.57(s,3H),2.01(s,3H) |
| 1-336; |
| 8.85-8.80(m,1H),8.80-8.75(m,1H),8.58(dd,J=4.8,1.5Hz,1H),8.30(s,1H),8.05-7.95(m,1H), 7.95-7.85(m,1H),7.40(dd, J=8.1,4.8Hz,1H),3.43(s,3H),2.59(s,3H) |
| 1-337; |
| 8.88(d,J=2.7Hz,1H),8.63(d,J=2.7Hz,1H),8.49(d,J=2.7Hz,1H),8.30-8.25(m,1H),7.98(ddd, J=8.4,2.7,1.5Hz,1H), 7.78(s,1H),7.65-7.50(m,1H),7.45(dd,J=8.4,4.8Hz,1H),7.30-7.25(m, 1H),3.56(s,2H),3.25(s,3H) |
| 1-340; |
| 8.96(d,J=2.4Hz,1H),8.62(dd,J=4.8,1.2Hz,1H),8.10(s,1H),8.04(ddd,J=8.1,2.4,1.2Hz,1H), 7.45(dd,J=8.1,4.8Hz,1H), 3.85-7.70(m,2H),3.59(t,J=6.6Hz,2H),3.33(q,J=6.9Hz,1H),2.73( t,J=6.6Hz,2H),1.98(qui,J=6.6Hz,2H),1.47(d, J=6.9Hz,3H),1.17(t,J=7.2Hz,3H) |
| 1-341; |
| 8.94(d,J=2.4Hz,1H),8.63-8.56(m,1H),8.10(s,1H),8.03(ddd,J=8.1,2.4,1.2Hz,1H),7.48-7.4 1 (m,1H),5.08-4.52(m,1H), 3.99-3.35(m,1H),2.85-2.65(m,2H),2.52-2.39(m,1H),1.98(s,3H) ,1.14(d,J=6.6Hz,3H) |
| 1-342; |
| 8.95(d,J=2.4Hz,1H),8.63(dd,J=4.8,1.2Hz,1H),8.21(brs,1H),8.01(ddd,J=8.1,2.4,1.2Hz,1H ),7.45(dd,J=8.1,4.8Hz, 1H),5.08-4.76(m,1H),3.72-3.49(m,1H),3.41-3.20(m,1H),2.68-2.44 (m,2H),1.46(d,J=6.9Hz,3H),1.17(t,J=7.8Hz,3H) |
| 1-343; |
| 8.91 (d,J=2.4Hz,1H),8.56(dd,J=4.8,1.2Hz,1H),8.06-7.98(m,1H),7.91(brs,1H),7.41(dd,J=8 .1,4.8Hz,1H),3.42(d, J=7.2Hz,2H),1.44(brs,9H),1.08-0.94(m,1H),0.54-0.44(m,2H),0.22-0. 11 (m,2H) |
| 1-344; |
| 8.93(d,J=2.4Hz,1H),8.62(dd,J=4.8,1.5Hz,1H),8.03(ddd,J=8.4,2.4,1.5Hz,1H),7.98(s,1H), 7.45(dd,J=8.4,4.8Hz,1H), 7.24(d,J=4.2Hz,1H),7.02(d,J=4.2Hz,1H),3.39(s,3H),2.45(s,3H) |
| 1-346; |
| 9.25-9.15(m,1H),9.15-9.10(m,1H),8.85-8.75(m,1H),8.60(dd,J=4.8,1.5Hz,1H),7.95-7.85( m,1H),7.82(s,1H), 7.50-7.45(m,1H),7.41(dd,J=8.4,4.8Hz,1H),3.45(s,3H) |
| 1-348; |

(continued)

No.

Proton NMR chemical shift (in $CDCl_3$): σ(ppm)

8.83(d,J=2.7Hz,1H),8.60(dd,J=4.8,1.5Hz,1H),8.00-7.90(m,1H),7.76(s,1H),7.42(dd,J=8.4 ,4.8Hz,1H),3.40(s,3H), 2.33(s,3H),2.27(s,3H)

1-349;

8.89(d,J=2.7Hz,1H),8.64(dd,J=4.8,1.5Hz,1H),8.00(ddd,J=8.4,2.7,1.5Hz,1H),7.94(s, H), 7.46(dd,J=8.4,4.8Hz,1H), 3.90(q,J=7.2Hz,2H),2.90(s,3H),1.28(t,J=7.2Hz,3H)

1-350;

8.94(d,J=2.7Hz,1H),8.62(dd,J=4.8,1.5Hz,1H),8.03(ddd,J=8.4,2.7,1.5Hz,1H),7.93(s,1H), 7.46(dd,J=8.4,4.8Hz,1H), 3.75(s,3H),3.72(q,J=7.2Hz,2H),3.32(s,2H),1.18(t,J=7.2Hz,3H)

1-351;

8.95(d,J=2.7Hz,1H),8.61(dd,J=4.8,1.5Hz,1H),7.99(ddd,J=8.4,2.7,1.5Hz,1H),7.93(s,1H), 7.43(dd,J=8.4,4.8Hz,1H), 6.05(s,1H),4.55-4.45(m,2H),3.66(q,J=7.2Hz,2H),2.90-2.75(m,2 H),1.95-1.80(m,1H),1.09(t,J=7.2Hz,3H), 1.05-1.00(m,2H),0.70-0.65(m,2H)

1-352;

8,99(d,J=2.7Hz,1 H),8.61(dd,J=4.8,1.5Hz,1H),8.34(s,1H),8.03(ddd,J=8.4,2.7,1.5Hz,1H), 7.43(dd,J=8.4,4.8Hz,1H), 4.17-4.08(m,4H),3.85-3.60(m,2H),2.88(d,J=21.9Hz,2H),1.36-1. 23(m,6H),1.17(t,J=7.2Hz,3H)

1-353;

10.61(brs,1H),9.52(s,1H),8,99(d,J=2.7Hz,1H),8.58(dd,J=4.8,1.2Hz,1H),8.01(ddd,J=8.4, 2.7,1.2Hz,1H),7.42(dd, J=8.4,4.8Hz,1H),4.21(q,J=7.2Hz,1H),2.67-2.49(m,2H),1.64(d,J=7 .2Hz,3H),1.29(t,J=7.5Hz,3H)

1-354;

8.96(d,J=2.4Hz,1H),8.62(dd,J=4.8,1.2Hz,1H),8.09(s,1H),8.05(ddd,J=8.1,2.4,1.2Hz,1H), 7.46(dd,J=8.1,4.8Hz,1H), 4.56(q,J=6.9Hz,1H),4.10-3.95(m,1H),3.90-3.70(m,2H),3.65-3.5 0(m,1H),3.35-3.10(m,2H),1.53(d,J=6.9Hz,3H), 1.16(t,J=7.2Hz,3H)

1-355;

8.93(d,J=2.4Hz,1H),8.59(dd,J=4.8,1.2Hz,1H),8.03(s,1H),8.01(ddd,J=8.1,2.4,1.2Hz,1H), 7.43(dd,J=8.1,4.8Hz,1H), 3.81(q,J=7.2Hz,2H),3.02(s,2H),1.34(s,6H),1.23(t,J=7.2Hz,3H)

1-356;

(the isomer contained in a ratio of 5)8.93(d,J=2.7Hz,1H),8.52(dd,J=5.1,1.2Hz,1H),8. 51 (s,1H),8.00(ddd,J=8.4,2.7, 1.2Hz,1H),7.39(dd,J=8.4,5.1Hz,1H),6.92(brs,1H),4.64(q,J= 7.2Hz,1H),3.88(s,3H),2.65-2.44(m,2H),1.45(d,J=7.2Hz, 3H),1.26(t,J=7.5Hz,3H)

(the isomer contained in a ratio of 2)8.92(d,J=2.7Hz,1H),8.58(dd,J=4.5,1.2Hz,1H),8. 08(s,1H),8.00(ddd,J=8.4,2.7, 1.2Hz,1H),7.57(dd,J=8.4,4.5Hz,1H),6.38(brs,1H),3.43(q,J= 7.2Hz,1H),3.87(s,3H),2.65-2.44(m,2H),1.47(d,J=7.2Hz, 3H),1.24(t,J=7.5Hz,3H)

1-359;

8.96(d,J=2.7Hz,1H),8.62(dd,J=4.8,1.5Hz,1H),8.05(ddd,J=8.3,2.7,1.5Hz,1H),8.02(s,1H) ,7.45(dd,J=8.3,4.8Hz,1H), 3.76(q,J=7.2Hz,2H),2.89(s,1H),1.20(t,J=7.2Hz,3H)

1-360;

8.96(d,J=2.7Hz,1H),8.56(dd,J=4.8,1.5Hz,1H),8.03(ddd,J=8.4,2.7,1.5Hz,1H),8.01(s,1H), 7.42(dd,J=8.4,4.8Hz,1H), 4.00-3.70(m,1H),3.70-3.45(m,1H),3.40-3.25(m,1H),2.58(q,J=7. 2Hz,2H),2.28(s,3H),1.44(d,J=6.9Hz,3H),1.16(t, J=7.8Hz,3H),1.15(t,J=7.2Hz,3H)

1-361;

8.83(d,J=2.4Hz,1H),8.59(dd,J=4.8,1.5Hz,1H),7.93(ddd,J=8.4,2.4,1.5Hz,1H),7.89(s,1H), 7.41(dd,J=8.4,4.8Hz,1H), 3.93(q,J=7.2Hz,2H),1.30(t,J=7.2Hz,3H)

1-362;

8.93(d,J=2.7Hz,1H),8.63(dd,J=4.8,1.5Hz,1H),8.05(ddd,J=8.4,2.7,1.5Hz,1H),7.98(s,1H), 7.48(d,J=3.9Hz,1H), 7.46(dd,J=8.4,4.8Hz,1H),7.40(d,J=3.9Hz,1H),3.87(q,J=7.2Hz,2H),2. 49(s,3H),1.28(t,J=7.2Hz,3H)

1-363;

8.96(d,J=2.7Hz,1H),8.61(dd,J=4.8,1.2Hz,1H),7.99(ddd,J=8.4,2.7,1.2Hz,1H),7.94(s,1H), 7.44(dd,J=8.4,4.8Hz,1H), 6.24(s,1H),4.35-4.30(m,2H),3.64(q,J=7.2Hz,2H),2.85-2.75(m,2 H),2.36(s,3H),1.07(t,J=7.2Hz,3H)

1-367;

(continued)

| No. Proton NMR chemical shift (in $CDCl_3$): σ(ppm) |
|---|
| (the isomer contained in a ratio of 65)8.98-8.92(m,1H),8.62(dd,J=4.8,1.2Hz,1H),8.08-8.01(m,1H),7.97(s,1H),7.45(dd, J=8.1,4.8Hz,1H),3.78(s,3H),3.70(q,J=7.2Hz,2H),3.35(s, 2H),3.28(s,2H),1.98(s,3H),1.15(t,J=7.2Hz,3H) |
| (the isomer contained in a ratio of 35)8.98-8.92(m,1H),8.62(dd,J=4.8,1.2Hz,1H),8.08-8.01(m,1H),7.98(s,1H),7.45(dd, J=8.1,4.8Hz,1H),3.78(s,3H),3.70(q,J=7.2Hz,2H),3.47(s, 2H),3.24(s,2H),1.96(s,3H),1.15(t,J=7.2Hz,3H) |
| 1-370; |
| 9.05-8.90(m,1H),8.58(dd,J=4.8,1.SHz,1H),8.14(s,1H),8.10-7.95(m,1H),7.41(dd,J=8.1,4. 8Hz,1H),4.20-4.00(m,2H), 3.50-3.30(m,1H),3.10-3.00(m,1H),2.5-2.35(m,1H),2.25(s,3H), 1.59(d,J=7.2Hz,3H),1.38(t,J=7.2Hz,3H),1.19(t, J=7.2Hz,3H) |
| 1-371; |
| 8.94(d,J=2.4Hz,1H),8.62(dd,J=4.8,1.2Hz,1H),8.09-8.00(m,2H),7.45(dd,J=8.1,4.8Hz,1H), 3.93-3.20(m,2H),2.86(dd, J=12.6,8.4Hz,1H),2.78-2.64(m,1H),2.47(dd,J=12.6,5.4Hz,1H), 2.01(s,3H),1.16(d,J=6.6Hz,3H),1.04-0.90(m,1H), 0.54-0.43(m,2H),0.24-0.15(m,2H) |
| 1-373; |
| (the isomer contained in a ratio of 4)9.00-8.90(m,1H),8.65-8.60(m,1H),8.05-8.00(m,1 H),7.94(s,1H),7.50-7.40(m,1H), 7.24(d,J=4.2Hz,1H),7.00(d,J=4.2Hz,1H),3.88(s,3H),3.87 (q,J=7.2Hz,2H),2.12(s,3H),1.24(t,J=7.2Hz,3H) |
| (the isomer contained in a ratio of 3)9.00-8.90(m,1H),8.65-8.60(m,1H),8.05-8.00(m,1 H),7.98(s,1H),7.50-7.40(m,1H), 7.38(d,J=4.2Hz,1H),7.19(d,J=4.2Hz,1H),3.87(q,J=7.2Hz, 2H),3.80(s,3H),2.24(s,3H),1.26(t,J=7.2Hz,3H) |
| 1-376; |
| 8.95(d,J=2.7Hz,1H), 8.63(dd,J=4.8,1.2Hz,1H),8.04(ddd, J=8.3,2.7,1.2Hz,1H),8.03(s,1H),7.46(dd,J=8.3,4.8H z,1H),7.37(brs,1H),3.90(s, 2H),3.88(s,2H),3.74(q,J=7.1Hz,2H),3.44(s,3H),1.19(t,J=7.1Hz ,3H) |
| 1-377; |
| 8.84(d,J=2.7Hz,1H),8.75-8.65(m,1H),8.60(dd,J=4.8,2.1Hz,1H),8.00-7.95(m,1H),7.92(dd, J=8.4,2.1 Hz,1H),7.86(s, 1H),7.75-7.60(m,1H),7.43(dd,J=8.4,4.8Hz,1H),3.90(q,J=7.2Hz,2 H),1.29(t,J=7.2Hz,3H) |
| 1-378; |
| 8.85(d,J=2.7Hz,1H),8.59(dd,J=4.8,1.5Hz,1H),7.94(s,1H),7.93(ddd,J=8.4,2.7,1.5Hz,1H), 7.86(dd,J=4.8,1.2Hz,1H), 7.75(dd,J=4.2,1.2Hz,1H),7.41(dd,J=8.4,4.8Hz,1H),7.16(dd,J=4 .8,4.2Hz,1H),3.90(q,J=7.2Hz,2H),1.26(t,J=7.2Hz, 3H) |
| 1-380; |
| 8.95(d,J=2.1Hz,1H),8.64(dd,J=4.5,1.5Hz,1H),8.04(ddd,J=8.4,2.1,1.5Hz,1H),7.94(s,1H), 7.47(dd,J=8.4,4.5Hz,1H), 4.00-3.75(m,4H),3.75-3.65(m,2H),3.05-2.95(m,1H),2.30-2.15( m,1H),2.00-1.90(m,1H),1.16(t,J=7.2Hz,3H) |
| 1-383; |
| 8,95(d,J=2.7Hz,1H),8.62(dd,J=4.8,1.5Hz,1H),8.10(brs,1H),8.04(ddd,J=8.4,2.7,1.5Hz,1H ),7.45(dd,J=8.4,4.8Hz, 1H),5.52-5.41(m,1H),4.90-4.40(m,1H),3.75-3.61(m,2H),3.42-3.30 (m,1H),2.66-2.52(m,2H),1.49(d,J=6.9Hz,3H), 1.25(t,J=6.9Hz,3H),1.16(t,J=7.5Hz,3H) |
| 1-386; |
| 8,94(d,J=2.7Hz,1H),8.62(dd,J=4.8,1.5Hz,1H),8.14(brs,1H),8.03(ddd,J=8.1,2.7,1.5Hz,1H ),7.45(dd,J=8.1,4.8Hz, 1H),4.16-4.09(m,1H),3.65-3.50(m,3H),3.40-3.30(m,1H),3.35(s,3H ),2.65-2.51(m,2H),1.47(d,J=6.6Hz,3H),1.17(t, J=7.5Hz,3H) |
| 1-387; |
| 8.97(d,J=2.4Hz,1H),8.63(dd,J=4.8,1.5Hz,1H),8.21(s,1H),8.00(ddd,J=8.1,2.4,1.5Hz,1H), 7.45(dd,J=8.1,4.8Hz,1H), 4.20-4.13(m,1H),4.07(q,J=7.2Hz,1H),3.65-4.56(m,3H),3.40-3.2 8(m,1H),3.34(s,3H),3.18-3.08(m,1H),1.65(d, J=7.2Hz,3H),1.38(t,J=7.5Hz,3H) |
| 1-388; |
| 8.96(d,J=2.4Hz,1H),8.85(dd,J=4.8,1.2Hz,1H),7.96(ddd,J=8.1,2.4,1.2Hz,1H),7.70(s,1H), 7.44(dd,J=8.1,4.8Hz,1H), 7.30-7.05(m,5H),3.90-3.65(m,3H),3.65-3.40(m,1H),3.30(q,J=6. 9Hz,1H),1.48(d,J=6.9Hz,3H),1.14(t,J=7.2Hz,3H) |
| 1-390; |
| 8.96(d,J=2.4Hz,1H),8.62(dd,J=4.8,1.2Hz,1H),8.06(ddd,J=8.1,2.4,1.2Hz,1H),7.99(s,1H), 7.47(dd,J=8.1,4.8Hz,1H), 3.85-3.60(m,2H),3.40-3.20(m,1H),2.80-2.60(m,1H),2.60-2.45( m,1H),2.40-2.15(m,2H),2.10-2.00(m,1H), 2.00-1.70(m,2H),1.60-1.25(m,3H),1.16(t,J=7.2 Hz,3H) |

(continued)

| No. Proton NMR chemical shift (in $CDCl_3$): σ(ppm) |
|---|
| 1-395; 8.89(d,J=2.7Hz,1H),8.50-8.40(m,1H),7.91(ddd,J=8.4,2.7,1.2Hz,1H),7.81(brs,1H),7.34(d d,J=8.4,4.8Hz,1H),4.02(s,3H),3.59(q,J=7.2Hz,2H),1.47(s,9H),1.15(t,J=7.2Hz,3H) |
| 1-397; 8.66(d,J=2.4Hz,1H),8.56(dd,J=4.8,1.2Hz,1H),8.26(d,J=2.1Hz,1H),7.88(s,1H),7.85(ddd,J =8.1,2.4,1.2Hz,1H),7.55(dd,J=5.4,2.1Hz,1H),7.37(dd,J=8.1,4.8Hz,1H),7.18(d,J=5.4Hz,1 H),4.91(q,J=6.9Hz,1H),3.90-3.60(m,2H),1.56(d,J=6.9Hz,3H),1.17(t,J=7.2Hz,3H) |
| 1-399; (the isomer contained in a ratio of 9)8,92(d,J=2.7Hz,1H),8.63(dd,J=5.1,1.5Hz,1H),8. 03(ddd,J=8.1,2.7,1.5Hz,1H),7.99(s,1H),7.46(dd,J=8.1,5.1Hz,1H),4.95-4.20(m,2H),3.81( s,3H),2.88(dd,J=12.3,8.7Hz,1H),2.80-2.75(m,1H),2.48(dd,J=12.3,5.1Hz,1H),2.03-2.00( m,3H),1.92(s,3H),1.19(d,J=6.6Hz,3H) (the isomer contained in a ratio of 1)8,92(d,J=2.7Hz,1H),8.63(dd,J=5.1,1.5Hz,1H),8. 03(ddd,J=8.1,2.7,1.5Hz,1H),7.99(s,1H),7.46(dd,J=8.1,5.1Hz,1H),4.95-4.20(m,2H),3.72( s,3H),2.88(dd,J=12.3,8.7Hz,1H),2.80-2.75(m,1H),2.48(dd,J=12.3,5.1Hz,1H),2.03-2.00( m,3H),1.89(s,3H),1.18(d,J=6.6Hz,3H) |
| 1-400; 8.92(d,J=2.4Hz,1H),8.63-8.57(m,1H),8.02(ddd,J=8.1,2.4,1.2Hz,1H),7.96(brs,1H),7.44(d d,J=8.1,4.8Hz,1H),3.40-3.00(m,2H),2.84(dd,J=12.3,9.0Hz,1H),2.82-2.60(m,1H)2.44(dd, J=12.3,4.2Hz,1H),1.99(s,3H),1.13(d,J=6.6Hz,3H),0.07(s,9H) |
| 1-402; 8.97(d,J=2.7Hz,1H),8.63(dd,J=4.8,1.5Hz,1H),7.96(s,1H),8.07(ddd,J=8.4,2.7,1.5Hz,1H), 7.47(dd,J=8.4,4.8Hz,1H),4.15-4.00(m,2H),3.75-3.60(m,2H),2.65-2.50(m,2H),2.45-2.30( m,1H),1.80-1.50(m,4H),1.44(s,9H),1.15(t,J=7.2Hz,3H) |
| 1-403; 9.10-9.00(m,1H),8.62(dd,J=4.8,1.5Hz,1H),8.20-8.05(m,2H),7.45-7.40(m,1H),4.25-4.05( m,1H),4.05-3.85(m,1H),2.85-2.60(m,2H),2.40-2.30(m,1H),1.85-1.75(m,2H),1.70-1.50(m, 2H),1.36(s,9H),1.35-1.20(m,2H),1.13(t,J=7.2Hz,3H) |
| 1-404; 8.90-8.80(m,1H),8.60(dd,J=4.8,1.5Hz,1H),7.95-7.85(m,1H),7.84(s,1H),7.41(dd,J=8.4,4. 8Hz,1H),4.61(s,2H),3.86(q,J=7.2Hz,2H),3.45(s,3H),1.27(t,J=7.2Hz,3H) |
| 1-405; 8.85(d,J=2.4Hz,1H),8.57(dd,J=4.8,1.5Hz,1H),7.95-7.90(m,1H),7.86(s,1H),7.38(dd,J=8.4 ,4.8Hz,1H),5.82(brs,2H),4.04(s,3H),3.10(q,J=7.2Hz,2H),1.25(t,J=7.2Hz,3H) |
| 1-406; 8.90(d,J=2.4Hz,1H),8.58(dd,J=4.8,1.5Hz,1H),8.05-7.95(m,1H),7.75(s,1H),7.42(dd,J=8.1 ,4.8Hz,1H),7.04(dd,J=4.2,1.2Hz,1H),6.13(dd,J=3.9,1.2Hz,1H),5.88(dd,J=4.2,3.9Hz,1H), 3.88(s,3H),3.83(q,J=7.2Hz,2H),1.26(t,J=7.2Hz,3H) |
| 1-407; 8.96(d,J=2.7Hz,1H),8.64(dd,J=4.8,1.2Hz,1H),8.05(ddd,J=8.3,2.7,1.2Hz,1H),8.03(s,1H), 7.47(dd,J=8.3,4.8Hz,1H),3.73(q,J=7.1Hz,2H),3.63(d,J=4.5Hz,2H),2.77(s,6H),1.18(t,J=7. 1Hz,3H) |
| 1-409; 8.95(d,J=3.0Hz,1H),8.66(dd,J=4.8,1.2Hz,1H),8.06(ddd,J=8.3,3.0,1.2Hz,1H),8.01(s,1H), 7.48(dd,J=8.3,4.8Hz,1H),7.40-7.35(m,1H),3.92(d,J=3.9Hz,2H),3.76(q,J=7.1Hz,2H),1.21 (t,J=7.1Hz,3H) |
| 1-411; 8.95(d,J=2.7Hz,1H),8.62(dd,J=4.8,1.2Hz,1H),8.10-8.00(m,1H),7.98(s,1H),7.46(dd,J=8.3 ,4.8Hz,1H),3.71(q,J=7.1Hz,2H),2.93(s,2H),2.23(s,6H),1.16(t,J=7.1Hz,3H) |
| 1-417; 8.95(d,J=2.7Hz,1H),8.62(dd,J=4.8,1.2Hz,1H),8.05-7.90(m,1H),8.03(s,1H),7.46(dd,J=8.3 ,4.8Hz,1H),3.84(s,2H),3.76(q,J=7.1Hz,2H),2.92(s,3H),1.43(s,9H),1.16(t,J=7.1Hz,3H) |
| 1-418; 8.95(d,J=2.4Hz,1H),8.62(dd,J=4.8,1.5Hz,1H),8.04(ddd,J=8.4,2.4,1.5Hz,1H),7.93(s,1H), 7.46(dd,J=8.4,4.8Hz,1H),3.71(q,J=7.2Hz,2H),2.75-2.55(m,2H),2.38(t,J=7.5Hz,2H),1.17( t,J=7.2Hz,3H) |

(continued)

| No. Proton NMR chemical shift (in $CDCl_3$): σ(ppm) |
|---|
| 1-420; 8.94(d,J=2.7Hz,1H),8.62(dd,J=4.8,1.5Hz,1H),8.03(ddd,J=8.4,2.7,1.5Hz,1H),7.94(s,1H), 7.45(dd,J=8.4,4.8Hz,1H), 5.25-5.15(m,1H),3.70(q,J=7.2Hz,2H),3.37(q,J=6.0Hz,2H),2.33 (t,J=6.0Hz,2H),1.55(s,9H),1.16(t,J=7.2Hz,3H) |
| 1-421; 8.97(d,J=2.7Hz,1H),8.70-8.60(m,2H),8.55-8.50(m,1H),8.10-8.05(m,1H),7.99(s,1H),7.73( d,J=15.3Hz,1H), 7.70-7.65(m,1H),7.46(dd,J=8.4,4.8Hz,1H),7.30-7.20(m,1H),6.49(d,J=15 .3Hz,1H),3.83(q,J=7.2Hz,2H),1.24(t, J=7.2Hz,3H) |
| 1-422; 8.98(d,J=2.7Hz,1H),8.65-8.60(m,1H),8.10-8.05(m,1H),7.97(s,1H),7.50(d,J=15.3Hz,1H), 7.46(dd,J=8.4,4.8Hz,1H), 7.40-7.30(m,1H),6.60-6.55(m,1H),6.45-6.40(m,1H),6.30(d,J=1 5.3Hz,1H),3.81(q,J=7.2Hz,2H),1.22(t,J=7.2Hz,3H) |
| 1-423; 8.97(d,J=2.7Hz,1H),8.62(dd,J=4.8,1.5Hz,1H),8.06(ddd,J=8.4,2.7,1.5Hz,1H),7.97(s,1H), 7.85(d,J=15.3Hz,1H), 7.45(dd,J=8.4,4.8Hz,1H),7.30-7.25(m,1H),7.20-7.15(m,1H),6.99(d d,J=4.8,3.6Hz,1H),6.21(d,J=15.3Hz,1H),3.82(q, J=7.2Hz,2H),1.22(t,J=7.2Hz,3H) |
| 1-424; 8.94(d,J=2.7Hz,1 H),8.62(dd,J=4.8,1.5Hz,1H),8.06(s,1H),8.01(ddd,J=8.4,2.7,1.5Hz,1H), 7.46(dd,J=8.4,4.8Hz,1H), 4.13(q,J=7.2Hz,2H),3.77(q,J=7.2Hz,2H),1.20(t,J=7.2Hz,3H),1. 13(t,J=7.2Hz,3H) |
| 1-426; 8.97(d,J=2.7Hz,1H),8.61(dd,J=4.8,1.2Hz,1H),8.06(ddd,J=8.4,2.7,1.2Hz,1H),7.97(s,1H), 7.47(dd,J=8.4,4.8Hz,1H), 4.10-3.40(m,2H),1.91(s,3H),1.59(s,6H),1.15(t,J=7.2Hz,3H) |
| 1-427; 8.95(d,J=2.4Hz,1H),8.63(dd,J=4.5,1.5Hz,1H),8.04(ddd,J=8.4,2.4,1.5Hz,1H),8.00(s,1H), 7.46(dd,J=8.4,4.5Hz,1H), 4.15-3.35(m,2H),1.24(t,J=7.2Hz,3H) |
| 1-429; 8.97(d,J=2.4Hz,1H),8.64(dd,J=4.8,1.5,1H),8.15-8.05(m,1H),7.93(brs,1H),7.46(dd,J=8.4, 4.8Hz,1H),3.60(q, J=7.2Hz,2H),1.41(s,9H),1.17(t,J=7.2Hz,3H) |
| 1-430; 8,98(d,J=2.7Hz,1H),8.63(dd,J=4.8,1.5Hz,1H),8.28(brs,1H),8.05(ddd,J=8.1,2.7,1.5Hz,1H ),7.46(dd,J=8.1,4.8Hz, 1H),5.70-5.20(m,1H),4.30-3.80(m,1H),3.40-3.29(m,1H),2.67-2.50 (m,2H),2.24(s,3H),1.48(d,J=6.6Hz,3H),1.17(t, J=7.5Hz,3H) |
| 1-431; 8.97(d,J=2.4Hz,1H),8.61(dd,J=4.8,1.2Hz,1H),8.10-8.05(m,1H),8.05(s,1H),7.44(dd,J=8.1 ,4.8Hz,1H),7.00(s,1H), 6.96(s,1H),4.17(q,J=6.9Hz,1H),3.95-3.75(m,1H),3.69(s,3H),3.50-3.30(m,1H),1.49(d,J=6.9Hz,3H),1.05(t,J=7.2Hz, 3H) |
| 1-432; 8.65(d,J=2.4Hz,1H),8.55(dd,J=4.8,1.2Hz,1H),8.26(dd,J=3.9,1.2Hz,1H),7.90(s,1H),7.83( ddd,J=8.1,2.4,1.2Hz,1H), 7.60-7.7.55(m,1H),7.40-7.30(m,1H),7.10-7.05(m,1H),6.80-6.70 (m,1H),4.82(q,J=6.9Hz,1H),3.95-3.75(m,1H), 3.70-3.50(m,1H),1.55(d,J=6.9Hz,3H),1.15(t ,J=7.2Hz,3H) |
| 1-433; 8.86(d,J=2.4Hz,1H),8.80-8.65(m,1H),8.56(dd,J=4.8,1.2Hz,1H),8.11(dd,J=9.0,2.7Hz,1H), 7.94(ddd,J=8.1,2.4,1.2Hz, 1H),7.93(s,1H),7.41(dd,J=8.1,4.8Hz,1H),7.22(d,J=9.0Hz,1H), 4.93(q,J=6.9Hz,1H),3.90-3.55(m,2H),1.58(d, J=6.9Hz,3H),1.17(t,J=7.2Hz,3H) |
| 1-434; 8.95(d,J=2.4Hz,1H),8.62(dd,J=4.8,1.2Hz,1H),8.07(s,1H),8.03(ddd,J=8.1,2.4,1.2Hz,1H), 7.45(dd,J=8.1,4.8Hz,1H), 4.49(t,J=5.4Hz,1H),3.71(q,J=7.2Hz,2H),3.34(s,6H),3.21(s,2H), 2.83(d,J=5.4Hz,2H),1.17(t,J=7.2Hz,3H) |
| 1-435; 8.96(brs,1H),8.43(dd,J=4.8,1.5Hz,1H),8.24(brs,1H),8.00-7.90(m,1H),7.31(dd,J=8.4,4.8H z,1H),6.38(brs,1H), 2.64(q,J=7.5Hz,2H),1.50(s,9H),1.32(t,J=7.5Hz,3H) |
| 1-439; |

(continued)

| No. Proton NMR chemical shift (in $CDCl_3$): σ(ppm) |
|---|
| 8.94(d,J=2.7Hz,1H),8.65-8.60(m,1H),8.05-8.00(m,1H),7.95(s,1H),7.45(dd,J=8.4,4.8Hz,1 H),3.86(s,2H),3.70(q, J=7.5Hz,2H),1.17(t,J=7.5Hz,3H),1.09(s,9H) |
| 1-440; |
| 8.96(d,J=2.4Hz,1H),8.65-8.60(m,1H),8.10-8.05(m,1H),7.94(s,1H),7.47(dd,J=8.4,4.8Hz,1 H),4.00-3.85(m,2H), 3.75-3.60(m,2H),3.30-3.20(m,2H),2.55-2.45(m,1H),2.00-1.85(m,2H) ,1.60-1.50(m,2H),1.15(t,J=7.2Hz,3H) |
| 1-443; |
| 8.96(d,J=2.7Hz,1H),8.61(dd,J=4.8,1.2Hz,1H),8.10-8.05(m,1H),7.99(s,1H),7.45(d,J=8.1, 4.8Hz,1H),3.80-3.60(m, 2H),1.45-1.35(m,1H),1.25-1.20(m,1H),1.20-1.10(m,1H),1.15(t,J= 7.2Hz,3H),1.01(d,J=6.0Hz,3H),0.60-0.50(m,1H) |
| 1-444; |
| 8.97(d,J=2.4Hz,1H),8.65-8.60(m,1H),8.10-8.05(m,1H),7.95(s,1H),7.46(dd,J=8.4,4.8Hz,1 H),3.69(q,J=7.2Hz,2H), 2.08(s,2H),1.16(t,J=7.2Hz,3H),1.01(s,9H) |
| 1-445; |
| 8.95(d,J=2.4Hz,1H),8.60(dd,J=4.8,1.5Hz,1H),8.07(ddd,J=8.4,2.4,1.5Hz,1H),7.92(s,1H), 7.47(dd,J=8.4,4.8Hz,1H), 5.15-5.10(m,2H),3.75(q,J=7.2Hz,2H),1.89(s,3H),1.19(t,J=7.2H z,3H) |
| 1-452; |
| 8.95(d,J=2.4Hz,1H),8.62(dd,J=4.8,1.2Hz,1H),8.05(ddd,J=8.1,2.4,1.2Hz,1H),7.99(s,1H), 7.46(dd,J=8.1,4.8Hz,1H), 4.58(t,J=7.2Hz,1H),3.73(q,J=7.2Hz,2H),3.10-2.75(m,4H),2.56( d,J=7.2Hz,2H),2.20-2.00(m,1H),1.90-1.70(m,1H), 1.17(t,J=7.2Hz,3H) |
| 1-453; |
| 8.97(d,J=2.4Hz,1H),8.59(dd,J=4.8,1.5Hz,1H),8.10(s,1H),8.05(ddd,J=8.1,2.4,1.5Hz,1H), 7.44(dd,J=8.1,4.8Hz,1H), 4.51(d,J=11.4Hz,1H),4.44(d,J=11.4Hz,1H),3.60-3.40(m,1H),3. 43(s,3H),3.26(s,3H),2.70-2.50(m,2H),1.44(d, J=6.9Hz,3H),1.17(t,J=7.2Hz,3H) |
| 1-456; |
| 8,95(d,J=2.4Hz,1H),8.62(dd,J=4.8,1.2Hz,1H),8.06(ddd,J=8.1,2.4,1.2Hz,1H),7.92(s,1H), 7.46(dd,J=8.1,4.8Hz,1H), 3.70(q,J=7.2Hz,2H),2.13(t,J=7.2Hz,2H),1.65-1.50(m,4H),1.30-1.20(m,9H),1.15(t,J=7.2Hz,2H),0.88-0.82(m,3H) |
| 1-457; |
| 8,95(d,J=2.7Hz,1H),8.61(dd,J=4.8,1.5Hz,1H),8.05(ddd,J=8.4,2.7,1.5Hz,1H),7.91(s,1H), 7.45(dd,J=8.4,4.8Hz,1H), 3.70(q,J=7.2Hz,2H),2.13(t,J=7.2Hz,2H),1.67-1.50(m,4H),1.27-1.13(m,17H),0.89-0.84(m,3H) |
| 1-458; |
| 8.93(d,J=2.7Hz,1H),8.60(dd,J=4.8,1.2Hz,1H),8.04(ddd,J=8.4,2.7,1.2Hz,1H),7.92(s,1H), 7.44(dd,J=8.4,4.8Hz,1H), 3.62(q,J=7.2Hz,2H),1.14(t,J=7.2Hz,3H),1.13(s,3H),1.10-1.05( m,2H),0.50-0.45(m,2H) |
| 1-459; |
| 8.94(d,J=2.7Hz,1H),8.65-8.60(m,1H),8.10-8.00(m,1H),7.86(s,1H),7.45(dd,J=8.4,4.8Hz,1 H),3.68(q,J=7.2Hz,2H), 3.15-3.00(m,1H),2.40-2.20(m,2H),1.95-1.75(m,4H),1.14(t,J=7.2 Hz,3H) |
| 1-461; |
| 8.94(d,J=2.4Hz,1H),8.61(dd,J=4.8,1.2Hz,1H),8.03(ddd,J=8.4,2.4,1.2Hz,1H),7.91(s,1H), 7.44(dd,J=8.4,4.8Hz,1H), 3.71(q,J=7.2Hz,2H),2.07(d,J=6.9Hz,2H),1.16(t,J=7.2Hz,3H),1. 10-1.00(m,1H),0.55-0.45(m,2H),0.10-0.05(m,2H) |
| 1-462; |
| 8.95(d,J=2.7Hz,1H),8.64(dd,J=4.5,1.5Hz,1H),8.05(ddd,J=8.4,2.7,1.5Hz,1H),7.98(s,1H), 7.47(dd,J=8.4,4.5Hz,1H), 5.82(s,1H),4.40(dd,J=9.0,3.3Hz,1H),3.73(q,J=7.2Hz,2H),3.04( dd,J=17.1,3.3Hz,1H),2.75(dd,J=17.1,9.0Hz,1H), 1.18(t,J=7.2Hz,3H) |
| 1-463; |
| 8.95(d,J=2.4Hz,1H),8.64(dd,J=4.8,1.5Hz,1H),8.04(ddd,J=8.4,2.4,1.5Hz,1H),8.00(s,1H), 7.47(dd,J=8.4,4.8Hz,1H), 5.96(s,1H),4.75-4.70(m,1H),3.80-3.60(m,2H),2.86(dd,J=17.1,4 .2Hz,1H),2.85(dd,J=17.1,3.3Hz,1H),1.18(t,J=7.2Hz, 3H) |
| 1-465; |
| (the isomer contained in a ratio of 2)8.95(d,J=2.4Hz,1H),8.62(dd,J=4.8,1.2Hz,1H),8. 63(s,1H),8.03(ddd,J=8.1,2.4, 1.2Hz,1H),7.46(dd,J=8.1,4.8Hz,1H),7.27(t,J=6.3Hz,1H),3.7 7(s,3H),3.71(q,J=7.2Hz,2H),3.30(d,J=6.3H,2H),3.15(s, 2H),1.17(t,J=7.2Hz,3H) |

(continued)

| No. |
|---|
| Proton NMR chemical shift (in $CDCl_3$): σ(ppm) |
| (the isomer contained in a ratio of 1)8.95(d,J=2.4Hz,1H),8.62(dd,J=4.8,1.2Hz,1H),8. 63(s,1H),8.03(ddd,J=8.1,2.4, 1.2Hz,1H),7.46(dd,J=8.1,4.8Hz,1H),6.74(t,J=6.3Hz,1H),3.8 2(s,3H),3.71(q,J=7.2Hz,2H),3.44(d,J=6.3H,2H),3.17(s, 2H),1.17(t,J=7.2Hz,3H) |
| 1-467; |
| 8.99(d,J=2.7Hz,1 H),8.51 (dd,J=4.8,1.5Hz,1H),8.35(s,1H),8.01(ddd,J=8.1,2.7,1.5Hz,1H), 7.37(dd,J=8.1,4.8Hz,1H), 6.54(brs,1H),2.50(s,3H),1.54(s,9H) |
| 1-469; |
| 8.70(d,J=2.4Hz,1H),8.55(dd,J=4.8,1.5Hz,1H),8.00-7.90(m,1H),7.85-7.80(m,1H),7.82(s,1 H),7.50-7.30(m,2H), 7.20-7.15(m,2H),3.45(s,3H) |
| 1-472; |
| 8.65-8.75(m,1H),8.55(d,J=3.6Hz,1H),7.80-8.00(m,2H),7.30-7.45(m,2H),7.10-7.25(m,2H) ,7.00-7.15(m,1H),3.90(m, 2H),2.48(s,3H),1.20-1.40(m,3H) |
| 1-473; |
| 8.77(d,J=2.4Hz,1H),8.56(dd,J=4.8,1.5Hz,1H),7.85-7.95(m,1H),7.66(brs,1H),7.38(dd,J=8 .1,4.8Hz,1H), 7.25-7.35(m,1H),7.10-7.20(m,3H),3.86(q,J=7.2Hz,2H),2.41(s,3H),1.26(t,J= 7.2Hz,3H) |
| 1-474; |
| 8.75-8.85(m,1H),8.50-8.60(m,1H),7.85-7.95(m,1H),7.70(s,1H),7.39(dd,J=8.7,4.8Hz,1H), 7.33(d,J=8.7Hz,2H), 7.08(d,J=8.7Hz,2H),3.85(q,J=7.2Hz,2H),2.43(s,3H),1.25(t,J=7.2Hz, 3H) |
| 1-475; |
| 8.78(d,J=3.0Hz,1H),8.57(dd,J=4.8,1.5Hz,1H),7.88(d,J=8.1Hz,1H),7.72(s,1H),7.66(s,1H) ,7.55-7.65(m,2H), 7.30-7.45(m,2H),3.88(q,J=7.2Hz,2H),1.27(t,J=7.2Hz,3H) |
| 1-476; |
| 8.82(d,J=2.4Hz,1H),8.58(dd,J=4.8,1.5Hz,1H),7.94(ddd,J=8.4,2.4,1.5Hz,1H),7.75(s,1H), 7.40(dd,J=8.4,4.8Hz,1H), 7.19(s,1H),7.16(d,J=8.1Hz,1H),6.93(d,J=8.1Hz,1H),3.85(q,J=7 .2Hz,2H),1.26(t,J=7.2Hz,3H) |
| 1-477; |
| (the isomer contained in a ratio of 4)8.98-8.93(m,2H),8.63-8.60(m,1H),8.10-8.00(m,2 H),7.50-7.40(m,1H),3.69(s,3H), 2.95-2.80(m,2H),2.60-2.45(m,1H),2.05-2.03(m,3H),1.28-1.20(m,3H) |
| (the isomer contained in a ratio of 1)8.98-8.93(m,2H),8.63-8.60(m,1H),8.10-8.00(m,2 H),7.50-7.40(m,1H),3.68(s,3H), 2.95-2.80(m,2H),2.60-2.45(m,1H),2.05-2.03(m,3H),1.28-1.20(m,3H) |
| 1-481; |
| (the isomer contained in a ratio of 11)8,95(d,J=2.4Hz,1H),8.56(dd,J=4.8,1.5Hz,1H),8 .05-8.01(m,2H),7.70(s,1H), 7.42(dd,J=8.4,4.8Hz,1H),6.41(brs,1H),3.86(s,3H),3.49(q,J=7. 2Hz,1H),2.63-2.56(m,2H),1.43(d,J=7.2Hz,3H),1.22(t, J=7.5Hz,3H) |
| (the isomer contained in a ratio of 9)8,97(d,J=2.7Hz,1H),8.50(dd,J=4.8,1.5Hz,1H),8. 42(s,1H),8.05-8.01(m,1H), 7.67(s,1H),7.39(dd,J=9.0,4.8Hz,1H),6.76(brs,1H),4.67(q,J=7. 2Hz,1H),3.86(s,3H),2.54-2.45(m,2H),1.47(d,J=7.2Hz, 3H),1.26(t,J=7.2Hz,3H) |
| 1-484; |
| 8.96(d,J=2.7Hz,1H),8.56(dd,J=4.8,1.8Hz,1H),8.10-8.00(m,1H),7.90(brs,1H),7.41(dd,J=8 .7,4.8Hz,1H), 4.30-3.90(m,1H),3.40-3.10(m,1H),2.90-2.30(m,5H),2.00(s,3H),1.40-1.20(m ,3H),1.20-1.00(m,6H) |
| 1-486; |
| (the isomer contained in a ratio of 7)8.92(d,J=2.7Hz,1H),8.49(dd,J=4.8,1.5Hz,1H),8. 02-7.98(m,1H),7.55(s,1H), 7.41(s,1H),7.38(dd,J=8.1,4.8Hz,1H),3.86(s,3H),3.71(q,J=7.2 Hz,1H),3.24(s,3H),2.62-2.52(m,2H),1.52(d,J=7.2Hz, 3H),1.21(t,J=7.5Hz,3H) |
| (the isomer contained in a ratio of 1)8.95-8.94(m,1H),8.54-8.52(m,1H),8.02-7.98(m,1 H),7.97(s,1H),7.41(s,1H), 7.38(dd,J=8.1,4.8Hz,1H),3.82(s,3H),3.71(q,J=7.2Hz,1H),3.17( s,3H),2.62-2.52(m,2H),1.52(d,J=7.2Hz,3H),1.21(t, J=7.5Hz,3H) |
| 1-487; |
| 8.97(d,J=2.4Hz,1H),8.66(dd,J=4.8,1.SHz,1H),7.91(ddd,J=8.4,2.7,1.2Hz,1H),7.97(s,1H), 7.47(dd,J=8.4,4.8Hz,1H), 3.64(q,J=7.2Hz,2H),3.28(s,3H),1.51(s,9H),1.20(t,J=7.2Hz,3H) |
| 1-488; |

(continued)

| No. Proton NMR chemical shift (in $CDCl_3$): σ(ppm) |
|---|
| 9.20-9.05(m,1H),8.70-8.35(m,2H),8.20-8.10(m,1H),7.50-7.35(m,1H),4.25-4.10(m,1H),3. 60-3.40(m,1H), 3.40-3.30(m,1H),3.30-3.15(m,1H),3.05-2.85(m,1H),2.80-2.75(m,3H),1.30 -1.15(m,6H) |
| 1-489; |
| 9.05-8.95(m,1H),8.60-8.50(m,1H),8.15-8.05(m,1H),8.05-8.00(m,1H),7.45-7.35(m,1H),4. 15-3.80(m,1H), 3.55-3.30(m,1H),3.20-2.65(m,2H),2.60-2.55(m,3H),2.55-2.50(m,1H),2.35 -2.30(m,3H),1.25-1.15(m,3H), 1.15-1.10(m,3H) |
| 1-490; |
| 9.01 (d,J=2.1Hz,1H),8.56(dd,J=4.8,1.2Hz,1H),8.09(s,1H),8.05-7.95(m,1H),7.41(dd,J=8.4 ,4.8Hz,1H),4.30-3.95(m, 1H),3.84(dd,J=13.5,10.8Hz,1H),3.45-3.20(m,2H),2.94(s,3H),2.8 7(dd,J=13.5,3.0Hz,1H),2.32(s,3H),1.16(t,J=6.9Hz, 3H),1.15(d,J=6.9Hz,3H) |
| 1-491; |
| 9.10-9.00(m,1H),8.65-8.60(m,1H),8.25-8.10(m,1H),8.10-8.00(m,1H),7.50-7.40(m,1H),4. 20-4.00(m,2H),3.45-3.30(m,2H),3.30-3.25(m,3H),3.25-3.15(m,1H),1.30-1.20(m,3H),1.20 -1.15(m,3H) |
| 1-495; |
| 8.93(d,J=2.1Hz,1H),8.60(dd,J=4.5,1.5Hz,1H),8.13(s,1H),8.02(ddd,J=8.1,2.1,1.5Hz,1H), 7.43(dd,J=8.1,4.8Hz,1H), 4.30-3.40(m,1H),2.96-2.78(m,1H),2.63(dd,J=12.6,6.0Hz,1H),2. 14(s,3H),1.32(d,J=6.9Hz,3H) |
| 1-496; |
| 8.99-8.91(m,2H),8.65-8.60(m,1H),8.10-8.03(m,2H),7.50-7.43(m,1H),3.69-3.68(m,3H),2. 85-2.55(m,4H), 2.15-2.04(m,3H) |
| 1-497; |
| 8.92(d,J=2.7Hz,1H),8.55(dd,J=4.8,1.2Hz,1H),8.8.00(ddd,J=8.4,2.7,1.2Hz,1H),7.90(s,1H ),7.41(dd,J=8.4,4.8Hz, 1H),3.56(q,J=7.2Hz,2H),3.19(s,3H),2,17(s,3H),1.26(t,J=7.2Hz,3H ) |
| 1-498; |
| 8.92(d,J=2.7Hz,1H),8.60(dd,J=4.8,1.8Hz,1H),8.02(ddd,J=8.4,2.7,1.5Hz,1H),7.92(s,1H), 7.55(s,1H),7.44(dd,J=8.4, 4.8Hz,1H),3.85(q,J=7.2Hz,2H),2.49(s,3H),1.27(t,J=7.2Hz,3H) |
| 1-500; |
| 8.92(d,J=2.7Hz,1H),8.58(dd,J=4.5,1.2Hz,1H),8.01(ddd,J=8.7,2.7,1.2Hz,1H),7.85(s,1H), 7.43(dd,J=8.7,4.5Hz,1H), 3.62(q,J=7.2Hz,2H),2.84(s,3H),2.00-2.15(m,1H),1.19(t,J=7.2H z,3H),0.65-0.75(m,2H),0.55-0.65(m,2H) |
| 1-502; |
| 9.15-8.85(m,1H),8.75-8.55(m,1H),8.30-8.00(m,2H),7.55-7.40(m,1H),3.77(q,J=7.2Hz,2H) ,1.52(s,9H),1.26(t, J=7.2Hz,3H) |
| 1-503; |
| 8.97(d,J=2.7Hz,1H),8.57(dd,J=4.8,1.2Hz,1H),8.02(ddd,J=8.7,2.7,1.2Hz,1H),7.87(brs,1H ),7.41(dd,J=8.7,4.8Hz, 1H),3.22(s,3H),1.46(brs,9H) |
| 1-504; |
| 8.91(d,J=2.4Hz,1H),8.59(dd,J=4.8,1.5Hz,1H),7.96(ddd,J=8.4,2.4,1.5Hz,1H),7.76(s,1H), 7.43(dd,J=8.4,4.8Hz,1H), 3.99(q,J=9.3Hz,2H),3.57(q,J=7.2Hz,2H),2.72(s,3H),1.20(t,J=7. 2Hz,3H) |
| 1-505; |
| 8.96(d,J=2.7Hz,1H),8.62(dd,J=4.8,1.2Hz,1H),8.05(ddd,J=8.3,2.7,1.2Hz,1H),8.01(s,1H), 7.47(dd,J=8.3,4.8Hz,1H), 3.48(d,J=6.8Hz,2H),2.80(t,J=7.4Hz,2H),2.47(t,J=7.4Hz,2H),2.0 7(s,3H),1.05-0.90(m,1H),0.55-0.40(m,2H), 0.25-0.10(m,2H) |
| 1-506; |
| (the isomer contained in a ratio of 10)9.04(d,J=2.7Hz,1H),8.59(dd,J=4.8,1.2Hz,1H),8 .31(s,1H),8.10-8.00(m,1H), 7.50-7.30(m,1H),4.10-3.05(m,5H),2.61(s,3H),1.23(d,J=6.8Hz ,3H),1.05-0.95(m,1H),0.55-0.45(m,2H),0.25-0.15(m, 2H) (the isomer contained in a ratio of 4)9.00(d,J=2.7Hz,1H),8.55(dd,J=4.8,1.2Hz,1H),8. 25(s,1H),8.10-8.00(m,1H), 7.50-7.30(m,1H),4.10-3.05(m,5H),2.61(s,3H),1.23(d,J=6.8Hz, 3H),1.05-0.95(m,1H),0.55-0.45(m,2H),0.25-0.15(m, 2H) |
| 1-507; |

(continued)

| No. Proton NMR chemical shift (in $CDCl_3$): σ(ppm) |
|---|
| (the isomer contained in a ratio of 10)8.98(d,J=2.7Hz,1H),8.62(dd,J=4.8,1.2Hz,1H),8 .06(ddd,J=8.3,2.7,1.2Hz,1H), 8.13(s,1H),7.46(dd,J=8.3,4.8Hz,1H),3.60-3.40(m,2H),3.20-2.75(m,2H),2.70-2.50(m,5H),1.05-0.80(m,1H), 0.55-0.40(m,2H),0.25-0.10(m,2H) |
| (the isomer contained in a ratio of 3)8.91(d,J=2.7Hz,1H),8.55(dd,J=4.8,1.2Hz,1H),7. 99(ddd,J=8.3,2.7,1.2Hz,1H), 8.13(s,1H),7.40(dd,J=8.3,4.8Hz,1H),3.60-3.40(m,2H),3.20-2.75(m,2H),2.70-2.50(m,5H),1.05-0.80(m,1H), 0.55-0.40(m,2H),0.25-0.10(m,2H) |
| 1-508; |
| (the isomer contained in a ratio of 10)8.98(d,J=2.7Hz,1H),8.63(dd,J=4.8,1.2Hz,1H),8 .25-8.00(m,2H),7.44(dd,J=8.3, 4.8Hz,1H),3.93(q.J=6.8Hz,1H),3.90-3.30(m,2H),2.90-2.70 (m,2H),1.50-1.20(m,6H),1.10-0.95(m,1H),0.55-0.45(m, 2H),0.25-0.15(m,2H) |
| (the isomer contained in a ratio of 1)8.98(d,J=2.7Hz,1H),8.63(dd,J=4.8,1.2Hz,1H),8. 25-8.00(m,2H),7.44(dd,J=8.3, 4.8Hz,1H),3.93(q.J=6.8Hz,1H),3.90-3.30(m,2H),2.65-2.50 (m,2H),1.50-1.20(m,6H),1.10-0.95(m,1H),0.65-0.55(m, 2H),0.30-0.25(m,2H) |
| 1-509; |
| 9.02(d,J=2.7Hz,1H),8.62(dd,J=4.8,1.2Hz,1H),8.23(s,1H),8.05-8.00(m,1H),7.43(dd,J=8.3 ,4.8Hz,1H),4.10-3.70(m, 2H),3.40-3.10(m,2H),3.05-2.85(m,1H),2.95(s,3H),1.21(d,J=6.8 Hz,3H),1.05-0.95(m,1H),0.55-0.45(m,2H), 0.25-0.15(m,2H) |
| 1-510; |
| 9.00-8.95(m,1H),8.70-8.60(m,1H),8.20-7.90(m,1H),8.10(s,1H),7.48(dd,J=8.3,4.8Hz,1H), 3.60-3.30(m,4H),2.96(s, 3H),2.80-2.70(m,2H),1.05-0.90(m,1H),0.65-0.40(m,2H),0.30-0.1 0(m,2H) |
| 1-514; |
| 8.95(d,J=2.7Hz,1H),8.60-8.55(m,1H),8.10-7.95(m,2H),7.44(dd,J=8.7,4.8Hz,1H),3.90-3.7 0(m,1H),3.70-7.50(m,1H), 2.86(dd,J=12.6,9.0Hz,1H),2.70-2.60(m,1H),2.55-2.35(m,3H),1 .20-1.10(m,9H) |
| 1-515; |
| (the isomer contained in a ratio of 2)9.05-9.00(m,1H),8.65-8.55(m,1H),8.30(s,1H),8. 05-7.95(m,1H),7.45-7.35(m,1H), 3.40-3.25(m,1H),3.30(s,3H),3.20-3.05(m,1H),2.80-2.50( m,3H),1.34(t,J=7.5Hz,3H),1.20(d,J=7.2Hz,3H) |
| (the isomer contained in a ratio of 1)9.00-8.95(m,1H),8.65-8.55(m,1H),8.20(s,1H),8. 05-7.95(m,1H),7.45-7.35(m,1H), 3.40-3.25(m,1H),3.25(s,3H),3.20-3.05(m,1H),2.80-2.50( m,3H),1.32(t,J=7.5Hz,3H),1.25(d,J=7.2Hz,3H) |
| 1-516; |
| (the isomer contained in a ratio of 2)9.03(d,J=2.4Hz,1H),8.65-8.55(m,1H),8.25(s,1H) ,8.05-7.95(m,1H),7.45-7.35(m, 1H),4.20-4.05(m,1H),3.50-3.30(m,1H),3.30-3.00(m,2H),2. 80-2.50(m,3H),1.34(t,J=7.5Hz,3H),1.30-1.10(m,6H) |
| (the isomer contained in a ratio of 1)8.98(d,J=2.4Hz,1H),8.65-8.55(m,1H),8.17(s,1H) ,8.05-7.95(m,1H),7.45-7.35(m, 1H),4.05-3.95(m,1H),3.50-3.30(m,1H),3.30-3.00(m,2H),2. 80-2.50(m,3H),1.31(t,J=7.5Hz,3H),1.30-1.10(m,6H) |
| 1-517; |
| 9.00(d,J=2.4Hz,1H),8.60(dd,J=4.8,1.8Hz,1H),8.24(s,1H),8.05-7.95(m,1H),7.42(dd,J=7.8 ,4.8Hz,1H),3.76(dd, J=13.5,11.1Hz,1H),3.45-3.30(m,1H),3.27(s,3H),3.10-2.95(m,2H),2.7 9(dd,J=13.5,2.4Hz,1H),1.41(t,J=7.5Hz,3H), 1.20(d,J=6.9Hz,3H) |
| 1-518; |
| 9.02(d,J=2.4Hz,1H),8.61 (dd,J=4.8,1.5Hz,1H),8.22(s,1H),8.05-7.95(m,1H),7.42(dd,J=8.7 ,4.8Hz,1H),4.15-4.00(m, 1H),3.74(dd,J=13.5,11.1Hz,1H),3.40-3.20(m,2H),3.05-2.95(m,2 H),2.77(dd,J=13.5,2.4Hz,1H),1.41(t,J=7.2Hz,3H), 1.20(d,J=6.9Hz,3H),1.16(t,J=7.2Hz,3H ) |
| 1-520; |
| 8.94(d,J=2.7Hz,1H),8.61(dd,J=4.8,1.2Hz,1H),8.04(ddd,J=8.1,2.7,1.2Hz,1H),7.94(s,1H), 7.45(dd,J=8.1,4.8Hz,1H), 3.70(q,J=7.2Hz,2H),2.48(t,J=6.9Hz,2H),2.26(t,J=7.2Hz,2H),2.0 4(s,3H),1.91(tt,J=7.2,6.9Hz,2H),1.14(t,J=7.2Hz,3H) |
| 1-523; |
| 9.03(d,J=2.4Hz,1H),8.70-8.65(m,1H),8.25-8.05(m,2H),7.52(dd,J=8.4,4.8Hz,1H),4.35-4.0 5(m,1H),3.35-3.15(m,1H), 2.90-2.55(m,2H),2.55-2.40(m,1H),2.03(s,3H),1.32(d,J=6.9Hz, 3H),1.18(t,J=7.2Hz,3H) |
| 1-524; |
| 9.05-8.95(m,1H),8.75-8.60(m,1H),8.25-8.00(m,2H),7.60-7.40(m,1H),4.50-4.10(m,1H),3. 40-3.20(m,3H), 3.30-2.40(m,4H),2.20-2.00(m,3H),1.40-1.30(m,3H),1.25-1.10(m,3H) |

(continued)

| No. Proton NMR chemical shift (in $CDCl_3$): σ(ppm) |
|---|
| 1-525; 9.02(d,J=2.4Hz,1H),8.75-8.65(m,1H),8.14(s,1H),8.10-8.05(m,1H),7.60-7.45(m,1H),4.05-3.65(m,1H),2.95-2.85(m, 1H),2.80-2.65(m,1H),2.60-2.55(m,1H),2.55-2.45(m,1H),2.13(s, 3H),1.22(d,J=6.9Hz,3H),1.30-1.15(m,3H) |
| 1-526; 8.94(d,J=2.7Hz,1H),8.50(dd,J=4.8,1.5Hz,1H),7.98(ddd,J=8.4,2.7,1.5Hz,1H),7.88(s,1H), 7.41 (dd,J=8.4,4.8Hz,1H), 4.04(s,3H),3.80-3.55(m,2H),2.95-2.70(m,2H),2.50-2.40(m,1H), 2.00(s,3H),1.14(t,J=7.2Hz,3H),1.13(d,J=6.9Hz,3H) |
| 1-527; 8.96(d,J=2.7Hz,1H),8.61(dd,J=4.8,1.2Hz,1H),8.04(ddd,J=8.1,2.7,1.2Hz,1H),7.98(s,1H), 7.45(dd,J=8.1,4.8Hz,1H), 3.77-3.62(m,2H),2.85-2.65(m,2H),2.55(s,3H),2.35(t,J=6.9Hz,2 H),2.16-2.02(m,2H),1.15(t,J=7.2Hz,3H) |
| 1-528; 8.95(d,J=2.7Hz,1H),8.61 (dd,J=4.8,1.2Hz,1H),8.02(ddd,J=8.1,2.7,1.2Hz,1H),7.97(s,1H), 7.44(dd,J=8.1,4.8Hz,1H), 3.70(q,J=7.2Hz,2H),3.10(t,J=7.2Hz,2H),2.90(s,3H),2.38(t,J=6. 9Hz,2H),2.15(tt,J=7.2,6.9Hz,2H),1.15(t,J=7.2Hz,3H) |
| 1-530; 8.91(d,J=2.7Hz,1H),8.60(dd,J=4.8,1.5Hz,1H),7.96(ddd,J=8.4,2.7,1.5Hz,1H),7.78(s,1H), 7.43(dd,J=8.4,4.8Hz,1H), 3.94(q,J=9.3Hz,2H),3.57(q,J=7.2Hz,2H),3.08(q,J=7.2Hz2H),1. 20(t,J=7.2Hz,3H),0.97(t,J=7.2Hz,3H) |
| 1-531; 8.86(d,J=2.4Hz,1H),8.62(dd,J=4.8,1.2Hz,1H),7.93(ddd,J=8.1,2.4,1.2Hz,1H),7.60-7.30( m,4H),6.96(d,J=8.7Hz,1H), 6.93(d,J=8.7Hz,1H),3.90-3.70(m,1H),3.70-3.50(m,1H),3.62(q ,J=6.9Hz,1H),1.41 (d,J=6.9Hz,3H),1.15(t,J=7.2Hz, 3H) |
| 1-532; 8.79(d,J=2.4Hz,1H),8.61(dd,J=4.8,1.2Hz,1H),7.92(ddd,J=8.1,2.4,1.2Hz,1H),7.49(s,1H), 7.43(dd,J=8.1,4.8Hz,1H), 7.25-6.85(m,4H),3.90-3.70(m,1H),3.72(q,J=6.9Hz,1H),3.70-3.5 5(m,1H),1.49(d,J=6.9Hz,3H),1.15(t,J=7.2Hz,3H) |
| 1-533; 8.85(d,J=2.7Hz,1H),8.62(dd,J=4.8,1.2Hz,1H),7.98(ddd,J=8.1,2.7,1.2Hz,1H),7.67(s,1H), 7.50-7.40(m,2H), 7.30-7.20(m,1H),7.06(ddd,J=7.5,7.5,1.2Hz,1H),6.96(ddd,J=8.7,7.5,1.2 Hz,1H),3.90-3.70(m,1H),3.73(q,J=6.9Hz, 1H),3.65-3.45(m,1H),1.45(d,J=6.9Hz,3H),1.13( t,J=7.2Hz,3H) |
| 1-534; 8.73(d,J=2.7Hz,1H),8.61(dd,J=4.8,1.2Hz,1H),7.91(ddd,J=8.1,2.7,1.2Hz,1H),7.44(ddd,J= 8.1,2.4,1.2Hz,1H), 7.30-7.20(m,2H),7.06(s,1H),7.04(d,J=7.5Hz,2H),3.90-3.70(m,1H),3.6 5(q,J=6.9Hz,1H),3.65-3.45(m,1H),2.30(s,3H), 1.46(d,J=6.9Hz,3H),1.14(t,J=7.2Hz,3H) |
| 1-535; 8.81(d,J=2.1Hz,1H),8.61(dd,J=4.8,1.2Hz,1H),7.60-7.70(m,1H),7.7-7.60(m,1H),7.50-7.30 (m,5H),3.90-3.55(m,3H), 1.51(d,J=7.2Hz,3H),1.17(t,J=7.2Hz,3H) |
| 1-536; 8.91(d,J=2.7Hz,1H),8.61(dd,J=4.8,1.5Hz,1H),7.96(ddd,J=8.4,2.7,1.5Hz,1H),7.79(s,1H), 7.43(dd,J=8.4,4.8Hz,1H), 4.22(q,J=9.3Hz,2H),4.16(s,2H),3.59(q,J=6.9Hz,2H),2.07(s,3H) ,1.21(t,J=6.9Hz,3H) |
| 1-539; (the isomer contained in a ratio of 78)8.95(d,J=2.4Hz,1H),8.61(dd,J=4.8,1.2Hz,1H),8 .04(ddd,J=8.1,2.4,1.2Hz,1H), 7.94(s,1H),7.45(dd,J=8.1,4.8Hz,1H),6.90(dd,J=15.0,7.5Hz, 1H),5.86(d,J=15.0Hz,1H),3.76(q,J=7.2Hz,2H),3.16(d, J=7.5Hz,2H),1.96(s,3H),1.28-1.11( m,3H)<br>(the isomer contained in a ratio of 22)8.95(d,J=2.4Hz,1H),8.61(dd,J=4.8,1.2Hz,1H),8 .05-7.99(m,1H),7.91(s,1H), 7.45(dd,J=8.1,4.8Hz,1H),5.86(d,J=15.0Hz,1H),5.50-5.38(m,1 H),3.69(q,J=7.5Hz,2H),3.00(d,J=7.2Hz,2H),1.96(s, 3H),1.28-1.11(m,3H) |
| 1-541; 9.50-8.50(m,1H),8.50-7.50(m,3H),7.00-6.90(m,1H),4.69(q,J=9.3Hz,2H),3.92(q,J=6.9Hz, 2H),2.87(s,3H),1.29(t, J=6.9Hz,3H) |
| 1-542; 8.98(d,J=2.4Hz,1H),8.66(d,J=4.5Hz,1H),8.20(s,1H),8.04(ddd,J=8.4,2.4,0.9Hz,1H),7.48( dd,J=8.4,4.5Hz,1H), 4.61(brs,2H),2.80(t,J=7.2Hz,2H),2.53(t,J=7.2Hz,2H),2.08(s,3H) |
| 1-543; |

(continued)

| No. | Proton NMR chemical shift (in $CDCl_3$): σ(ppm) |
|---|---|
| | 9.00(d,J=2.4Hz,1H),8.66(d,J=4.8Hz,1H),8.26(s,1H),8.04(m,1H),7.47(dd,J=8.4,4.8Hz,1H ),4.60(brs,2H), 3.20-3.11(m,1H),2.95-2.85(m,1H),2.79-2.74(m,2H),2.60(s,3H) |
| 1-544; | 9.00(m,1H),8.67(m,1H),8.27(s,1H),8.37(ddd,J=8.1,2.4,0.9Hz,1H),7.48(dd,J=8.1,4.8Hz,1 H),4.59(brs,2H),3.44(t, J=6.9Hz,2H),2.98(s,3H),2.80(t,J=6.9Hz,2H) |
| 1-545; | 8.95(d,J=2.7Hz,1H),8.63(dd,J=4.8,1.5Hz,1H),8.05(ddd,J=8.1,2.7,1.5Hz,1H),8.01(s,1H), 7.46(dd,J=8.1,4.8Hz,1H), 3.26(s,3H),2.87(dd,J=12.6,9.0Hz,1H),2.85-2.65(m,1H),2.48(dd ,J=12.6,5.1Hz,1H),2.03(s,3H),1.18(d,J=6.6Hz,3H) |
| 1-546; | 8.90(d,J=2.4Hz,1H),8.60(dd,J=4.5,1.5Hz,1H),8.02(ddd,J=8.1,2.4,1.5Hz,1H),7.99(s,1H), 7.43(dd,J=8.1,4.5Hz,1H), 3.70(s,3H),3.54(s,2H),3.35(s,3H),1.92(s,3H) |
| 1-568; | (the isomer contained in a ratio of 3)9.00-8.90(m,1H),8.65-8.55(m,1H),8.40(s,1H),8. 15-7.95(m,1H),7.50-7.40(m,1H), 5.55-5.40(m,1H),5.20-5.00(m,1H),3.70-3.45(m,2H),3.35 -3.15(m,1H),2.53(s,3H),1.27(d,J=7.2Hz,3H) (the isomer contained in a ratio of 2) 9.00-8.90(m,1H),8.65-8.55(m,1H),8.35(s,1H),8.15-7.95(m,1H),7.50-7.40(m,1H),5.55-5.4 0(m,1H),5.20-5.00(m,1H), 3.70-3.45(m,2H),3.35-3.15(m,1H),2.68(s,3H),1.29(d,J=7.2Hz, 3H) |
| 1-569; | 9.03(d,J=2.7Hz,1H),8.70-8.60(m,1H),8.40(s,1H),8.15-7.95(m,1H),7.45(dd,J=4.8,8.1Hz,1 H),5.10-4.90(m,1H), 4.35-4.10(m,2H),3.85-3.70(m,1H),3.40-3.30(m,1H),2.97(s,3H),1.25( d,J=7.2Hz,3H) |
| 1-573; | 8.90(d,J=2.7Hz,1H),8.59(dd,J=4.8,1.2Hz,1H),7.94(ddd,J=8.3,2.7,1.2Hz,1H),7.77(s,1H), 7.43(dd,J=8.3,4.8Hz,1H), 4.35(s,2H),4.03(q,J=9.2Hz,2H),3.61(q,J=7.1Hz,2H),3.20(s,3H) ,1.20(t,J=7.1Hz,3H) |
| 1-581; | (the isomer contained in a ratio of 7)9.04(d,J=3.0Hz,1H),8.60(dd,J=4.8,1.5Hz,1H),8. 25(s,1H),8.10-7.95(m,1H), 7.50-7.35(m,1H),3.35-3.10(m,2H),3.29(s,3H),2.75-2.55(m,1H ),2.60(s,3H),1.23(d,J=6.6Hz,3H) (the isomer contained in a ratio of 5) 8.99(d,J=2.4Hz,1H),8.61(dd,J=4.8,1.5Hz,1H),8.18(s,1H),8.10-7.95(m,1H),7.50-7.35(m,1 H),3.35-3.10(m,2H), 3.26(s,3H),2.75-2.55(m,1H),2.60(s,3H),1.27(d,J=6.6Hz,3H) |
| 1-582; | 9.01 (d,J=2.7Hz,1H),8.62(dd,J=4.8,1.5Hz,1H),8.20(s,1H),8.01(ddd,J=8.4,2.7,1.5Hz,1H), 7.43(dd,J=8,4.8Hz,1H), 3.82(dd,J=14.1,11.4Hz,1H),3.40-3.30(m,1H),3.28(s,3H),2.95(s,3 H),2.89(dd,J=14.1,2.7Hz,1H),1.22(d,J=6.9Hz,3H) |
| 1-583; | 8.97(d,J=2.7Hz,1H),8.58(dd,J=4.8,1.2Hz,1H),8.20(s,1H),8.00(ddd,J=8.4,2.7,1.2Hz,1H), 7.41 (dd,J=8.4,4.8Hz,1H), 4.06(d,J=12.6Hz,1H),3.95(d,J=12.6Hz,1H),3.76(s,3H),3.34(s,3 H),2.60(s,3H) |
| 1-584; | 8.96(d,J=2.7Hz,1H),8.59(dd,J=4.8,1.5Hz,1H),8.07(s,1H),7.98(ddd,J=8.4,2.7,1.5Hz,1H), 7.42(dd,J=8.4,4.8Hz,1H), 4.47(s,2H),3.80(s,3H),3.37(s,3H),2.88(s,3H) |
| 1-588; | 8.93(d,J=2.4Hz,1H),8.63(dd,J=5.1,1.2Hz,1H),8.06(ddd,J=8.4,2.4,1.2Hz,1H),7.98(s,1H), 7.47(dd,J=8.4,5.1Hz,1H), 4.72(d,J=10.2Hz,1H),4.05-3.45(m,2H).3.35-2.50(m,SH),1.23(d, J=6.9Hz,3H),1.18(t,J=7.2Hz,3H) |
| 1-590; | 8.90-8.80(m,1H),8.52(s,1H),8.50-8.40(m,1H),8.00-7.90(m,1H),7.85-7.75(m,3H),7.70-7.6 5(m,1H),7.30-7.25(m,1H), 7.15-7.10(m,1H),3.83(q,J=7.2Hz,2H),3.54(s,3H),1.19(t,J=7.2H z,3H) |
| 1-596; | 9.02(d,J=2.7Hz,1H),8.65-8.55(m,1H),8.19(s,1H),8.05-7.95(m,1H),7.42(dd,J=4.8,8.1Hz,1 H),4.15-3.95(m,1H), 3.60-3.30(m,2H),3.25-3.10(m,1H),2.85(s,6H),2.75-2.60(m,1H),1.21( d,J=7.2Hz,3H),1.17(t,J=7.2Hz,3H) |
| 1-598; | 8.84(d,J=2.7Hz,1H),8.56(dd,J=4.8,1.2Hz,1H),8.02(s,1H),7.96(ddd,J=8.4,2.7,1.2Hz,1H), 7.45-7.41(m,1H),5.50(m, 1H),4.14(d.J=6.0Hz,2H),3.68(q,J=7.2Hz,2H),1.16(t,J=7.2Hz,3H ) |

(continued)

| No. Proton NMR chemical shift (in $CDCl_3$): σ(ppm) |
|---|
| 1-599; 8.95(d,J=2.1Hz,1H),8.61(dd,J=4.8,1.5Hz,1H),8.42(ddd,J=4.8,1.5,0.9Hz,1H),8.07-8.02( m,2H),7.64(ddd,J=7.8,7.8, 2.1Hz,1H),7.45(ddd,J=8.1,4.8,0.9Hz,1H),7.32-7.28(m,1H),7.1 8-7.10(m,1H),5.67(m,1H),4.52(d,J=5.7Hz,2H), 3.72(q,J=7.2Hz,2H),1.17(t,J=7.2Hz,3H) |
| 1-601; 8.74(d,J=2.7Hz,1H),8.57(d,J=4.8Hz,1H),7.95-7.85(m,1H),7.50-7.20(m,7H),5.65(brs,1H), 4.16(brs,1H),2.92(dd, J=12.6,9.0Hz,1H),2.80-2.70(m,1H),2.51(dd,J=12.6,5.1Hz,1H),2.00 (s,3H),1.21(d,J=6.3Hz,3H) |
| 1-602; 8.74(d,J=2.4Hz,1H),8.58(dd,J=4.5,1.2Hz,1H),7.91(ddd,J=8.4,2.4,1.2Hz,1H),7.45-7.20( m,7H),4.90(brs,2H),2.85(t, J=7.2Hz,2H),2.51(t,J=7.2Hz,2H),2.08(s,3H) |
| 1-603; 8,85(d,J=2.4Hz,1H),8.59(dd,J=4.8,1.5Hz,1H),8.31(s,1H),7.99(ddd,J=8.1,2.4,1.5Hz,1H), 7.43(dd,J=8.1,4.8Hz,1H), 5.91(s,1H),5.45-5.05(m,1H),4.60-4.25(m,1H),3.96(s,6H),3.48( q,J=6.9Hz,1H),2.68-2.50(m,2H),1.51(d,J=6.9Hz, 3H),1.14(t,J=7.5Hz,3H) |
| 1-604; 8.76(d,J=2.7Hz,1H),8.56(dd,J=4.8,1.2Hz,1H),7.89(ddd,J=8.4,2.7,1.2Hz,1H),7.59(s,1H), 7.39(dd,J=8.4,4.8Hz,1H), 7.36-7.19(m,5H),5.80-5.20(m,1H),4.40-3.90(m,1H),3.46-3.27( m,1H),2.71-2.50(m,2H),1.51(d,J=6.9Hz,3H),1.17(t, J=7.5Hz,3H) |
| 2-003; 8.94(d,J=2.7Hz,1H),8.57(dd,J=4.8,1.2Hz,1H),8.15(s,1H),7.99(ddd,J=8.1,2.7,1.2Hz,1H), 7.42(dd,J=8.1,4.8Hz,1H), 6.42(dd,J=16.5,9.9Hz,1H),6.27(d,J=16.5Hz,1H),6.09(d,J=9.9H z,1H),3.28(s,3H) |
| 3-006; (the isomer contained, in a ratio of 2)8.89(d,J=2.1Hz,1H),8.54(dd,J=4.8,1.2Hz,1H),8. 01-7.99(m,1H),7.59(s,1H), 7.40(dd,J=8.4,4.8Hz,1H),4.12(q,J=7.2Hz,1H),2.71-2.53(m,2H ),2.42(s,3H),1.51(d,J=7.2Hz,3H),1.19(t,J=7.2Hz,3H) (the isomer contained in a ratio of 1)8.92-8.89(m,1H),8.54(dd,J=4.8,1.2Hz,1H),8.01-7.99(m,1H),7.93(s,1H),7.40(dd, J=8.4,4.8Hz,1H),3.89(q,J=6.9Hz,1H),2.71-2.53(m,2H),2. 65(s,3H),1.69(d,J=6.9Hz,3H),1.27(t,J=7.2Hz,3H) |
| 3-009; (the isomer contained in a ratio of 2)8,95(d,J=2.7Hz,1H),8.55-8.53(m,1H),8.05-8.0 1(m,1H),7.69(s,1H),7.50(s,1H), 7.43-7.39(m,1H),4.27(q,J=7.2Hz,1H),2.54(q,J=7.2Hz,2H ),2.38(s,3H),1.52(d,J=7.2Hz,3H),1.19(t,J=7.2Hz,3H) (the isomer contained in a ratio of 1)8,97(d,J=3.0Hz,1H),8.55-8.53(m,1H),8.05-8.0 1(m,1H),8.00(s,1H),7.84(s,1H), 7.43-7.39(m,1H),3.89(q,J=7.2Hz,1H),2.64(q,J=7.2Hz,2H ),2.63(s,3H),1.66(d,J=7.2Hz,3H),1.27(t,J=7.2Hz,3H) |
| 3-010; (the isomer contained in a ratio of 4)8.98-8.70(m,2H),8.60-8.50(m,1H),8.10-7.95(m,1 H),7.91(s,1H),7.50-7.35(m,1H), 4.09(s,3H),3.40-3.25(m,1H),3.00-2.50(m,2H),2.17(s,3H), 1.32(d,J=7.2Hz,3H) (the isomer contained in a ratio of 1)8.98-8.70(m,2H),8.60-8.50(m,1H),8.10-7.95(m,1 H),7.91(s,1H),7.50-7.35(m,1H), 3.79(s,3H),3.00-2.50(m,3H),2.13-2.00(m,3H),1.37-1.23( m,3H) |
| 3-012; (the isomer contained in a ratio of 77)8.97(s,1H),8.92(d,J=2.7Hz,1H),8.56(dd,J=4.8, 1.2Hz,1H),8.01(ddd,J=8.1,2.7, 1.2Hz,1H),7.90(s,1H),7.41(dd,J=8.1,4.8Hz,1H),4.15(s,3H ),4.15-4.02(m,1H),2.79-2.57(m,2H),1.57(d,J=7.2Hz,3H), 1.31-1.22(m,3H) (the isomer contained in a ratio of 23)8.92(d,J=2.7Hz,1H),8.62(s,1H),8.56(dd,J=4.8, 1.2Hz,1H),8.01(ddd,J=8.1,2.7, 1.2Hz,1H),7.90(s,1H),7.41(dd,J=8.1,4.8Hz,1H),4.15-4.02( m,1H),3.78(s,3H),2.79-2.57(m,2H),1.50(d,J=7.5Hz,3H), 1.31-1.22(m,3H) |
| 5-003; 8.80(d,J=2.4Hz,1H),8.67(dd,J=4.8,1.2Hz,11H),7.87(ddd,J=8.1,2.4,1.2Hz,H),7.61(brs,1H ),7.48(dd,J=8.1,4.8Hz, 1H),4.09-3.79(m,1H),3.57-3.27(m,1H),2.93-2.79(m,1H),2.77-2.57 (m,1H),2.39(dd,J=12.9,5.1Hz,1H),2.33(brs,3H), 1.99(s,3H),1.14(t,J=7.2Hz,3H),1.10(d,J= 6.9Hz,3H) |
| 5-004; 8.78(d,J=2.4Hz,1H),8.66(dd,J=4.8,1.2Hz,1H),7.90-7.82(m,1H),7.76-7.52(m,1H),7.47(dd, |

(continued)

| No. Proton NMR chemical shift (in $CDCl_3$): σ(ppm) |
|---|
| J=8.1,4.8Hz,1H),4.14-3.81(m,1H),3.55-3.21(m,2H),2.61-2.49(m,2H),2.31(brs,3H),1.43(d ,J=6.9Hz,3H),1.15(t, J=7.2Hz,3H),1.14(t,J=7.2Hz,3H) |
| 5-005; |
| 8.91(d,J=2.4Hz,1H),8.67(dd,J=4.8,1.5Hz,1H),7.95(ddd,J=8.4,2.4,1.5Hz,1H),7.68(s,1H), 7.46(dd,J=8.4,4.8Hz,1H), 3.61(q,J=7.2Hz,2H),1.45(s,9H),1.18(t,J=7.2Hz,3H) |
| 5-007; |
| 8.99(d,J=2.4Hz,1H),8.70(dd,J=4.8,1.5Hz,1H),8.04(ddd,J=8.1,2.4,1.5Hz,1H),7.48(dd,J=8 .1,4.8Hz,1H), 4.30-4.15(m,1H),3.85-3.70(m,1H),3.40-3.30(m,1H),2.70-2.50(m,4H),1.52(d ,6.9Hz,3H),1.07(t,J=7.5Hz,3H), 1.25-1.15(m,6H) |
| 6-001; |
| 9.21(s,1H),9.10(s,2H),8.13(s,1H),3.90-3.70(m,1H),3.70-3.50(s,1H),3.25(q,J=6.9Hz,1H), 2.70-2.50(m,2H),1.47(d, J=6.9Hz,3H),1.17(t,J=7.5Hz,3H),1.16(t,J=7.5Hz,3H) |
| 6-002; |
| 8.72(d,J=1.8Hz,1H),8.45-8.40(m,1H),8.08(s,1H),7.90-7.85(m,1H),3.90-3.70(m,1H),3.70-3.50(m,1H),3.29(q, J=6.9Hz,1H),2.65-2.50(m,2H),2.45(s,3H),1.47(d,J=6.9Hz,3H),1.16(t, J=7.2Hz,3H),1.15(t,J=7.5Hz,3H) |
| 6-003; |
| 8.49(d,J=1.8Hz,1H),8.31(d,J=2.4Hz,1H),8.09(s,1H),7.60(dd,J=2.4,1.8Hz,1H),3.86(s,3H) ,3.85-3.70(m,1H), 3.70-3.55(m,1H),3.29(q,J=6.9Hz,1H),2.65-2.50(m,2H),1.47(d,J=6.9Hz ,3H),1.17(t,J=7.5Hz,3H),1.16(t,J=7.2Hz,3H) |
| 6-010; |
| 8.35-8.25(m,1H),8.10-8.00(m,1H),8.00-7.90(m,1H),7.80-7.70(m,1H),7.40-7.35(m,1H),3. 91(brs,2H),3.28(s,3H), 1.75-1.60(m,1H),1.15-1.00(m,2H),0.75-0.65(m,2H) |
| 7-002; |
| 8.79(d,J=2.7Hz,1H),8.04(s,1H),7.90(dd,J=8.4,2.7Hz,1H),7.28(d,J=8.4Hz,1H),3.85-3.70( m,1H),3.70-3.55(m,1H), 3.30(q,J=6.9Hz,1H),2.62(s,3H),2.60-2.50(m,2H),1.46(d,J=6.9Hz ,3H),1.16(t,J=7.2Hz,3H),1.15(t,J=7.5Hz,3H) |
| 7-003; |
| 8.57(s,1H),8.55(d,J=4.8Hz,1H),7.78(s,1H),7.29(d,J=4.8Hz,1H),3.90-3.75(m,1H),3.70-3. 50(m,1H),3.31(q,J=6.9Hz, 1H),2.70-2.50(m,2H),2.37(s,3H),1.48(d,J=6.9Hz,3H),1.17(t,J= 7.5Hz,3H),1.16(t,J=7.2Hz,3H) |
| 7-004; |
| 8.61 (dd,J=4.8,1.BHz,1H),7.78(s,1H),7.70(dd,J=8.4,1.BHz,1H),7.30(dd,J=8.4,4.8Hz,1H), 3.95-3.70(m,1H), 3.7-3.50(m,1H),3.31(q,J=6.9Hz,1H),2.58(q,J=7.5Hz,2H),2.57(s,3H),1.4 8(d,J=6.9Hz,3H),1.18(t,J=7.5Hz,3H),1.16(t, J=7.2Hz,3H) |

[Table 12]

| No. Proton NMR chemical shift (in DMSO-d6):σ(ppm) |
|---|
| 1-004; |
| 9.02(d,J=2.7Hz,1H),8.80(s,1H),8.46(d,J=4.8Hz,1H),8.43(s,1H),8.19-8.13(m,1H),7.76(s, 1H),7.50(dd,J=4.8,8.7Hz, 1H),6.95(t,J=6.6Hz,1H),3.97-3.83(m,2H) |
| 1-009; |
| 10.62(s,1H),9.06(d,J=2.4Hz,1H),8.70(s,1H),8.47(dd,J=1.2,4.8Hz,1H),8.20(ddd,J=1.2,2. 4,8.1Hz,1H),7.91(s,1H), 7.82(d,J=8.4Hz,2H),7.77(d,J=8.4Hz,2H),7.62(d,J=16.0Hz,1H),7. 50(dd,J=4.8,8.1Hz,1H),6.88(d,J=16.0Hz,1H) |

The proton NMR chemical shifts in Tables 11 and 12 were measured by using $Me_4$ Si (tetramethylsilane) as the standard compound at 300 MHz.

Compounds of the present invention having a substituent having an asymmetric carbon were resolved to optically active substances by high performance liquid chromatography using an optically active column by the method described in detail below. However, in the present invention, there is no restriction to the method.

As a high performance liquid chromatograph, 10AVP system, manufactured by Shimadzu Corporation, was used.

The optical resolution was carried out under the following conditions.

Flow rate: 6.0ml/min Oven temp.: 30°C

Mobile phase:n-hexane:ethanol = 7:3 (volume ratio)

Column: CHIRALPAK AD-H (inner diameter 20 mm, length 250 mm, particle diameter 5 μm), manufactured by Dicel Corporation

Wavelength:254 nm

The method will be described in reference to Compound No. 1-048 of the present invention. Compound No. 1-048 (400 mg) was dissolved in 4 ml of a solvent mixture of n-hexane : ethanol = 1:1 (volume ratio). The resulting solution was subjected to optical resolution by high performance liquid chromatography under the conditions described above in forty 0.1-ml aliquots, and the fractions corresponding to peaks with retention times of 18.4 min and 21.1 min were collected. The solvent was evaporated from each fractions under reduced pressure to obtain 132.5 mg of white crystals (peak area percentage 99%, $[\alpha]_D{}^{23.9}$-10.00° ($CHCl_3$, c=0.510)} from the fraction having a retention time of 18.4 min and 128.2 mg of white crystals {peak area percentage 92%, $[\alpha]_D{}^{24.1}$+8.95° ($CHCl_3$, c=0.503)} from the fraction having a retention time of 21.1 min.

[TEST EXAMPLES]

Now, usefulness of the compounds of the present invention as pesticides will be described in detail by referring to the following Test Examples, but the present invention is by no means restricted thereto.

TEST EXAMPLE 1: Insecticidal Test on Nilaparvata lugens

10% emulsifiable concentrates (or 10%wettable powders) of compounds of the present invention were diluted with water containing a spreader to obtain 500 ppm solutions. Rice sheaths were soaked in the solutions for about 10 seconds, dried in air and put in test tubes. In each tube, five 2nd-instar nymphs of Nilaparvata lugens were released, and the tubes were capped with sponge and placed in an incubator at 25°C. 6 Days after, dead insects were counted, and the mortality (%) (the number of dead insects ÷ the number of released insects × 100) was calculated. The test was carried out in duplicate.

Among the compounds tested, the following compounds showed a mortality of at least 90%.

Compounds Nos.1-002, 1-007, 1-008, 1-011, 1-012, 1-013, 1-014, 1-018, 1-019, 1-020, 1-021, 1-022, 1-023, 1-026, 1-028, 1-031 to 1-054, 1-056 to 1-070, 1-073 to 1-080, 1-082 to 1-089, 1-093 to 1-107, 1-109 to 1-113, 1-115 to 1-124, 1-126, 1-130, 1-131, 1-134, 1-135, 1-140 to 1-144, 1-146 to 1-155, 1-157, 1-158, 1-162 to 1-173, 1-177, 1-178, 1-179, 1-184, 1-188, 1-189, 1-190, 1-193 to 1-199, 1-201 to 1-210, 1-212, 1-213, 1-216, 1-220, 1-221, 1-222, 1-223, 1-225 to 1-229, 1-231 to 1-244, 1-247, 1-248, 1-253, 1-254, 1-255, 1-258 to 1-271, 1-273, 1-275 to 1-278, 1-280 to 1-299, 1-301 to 1-307, 1-309, 1-310, 1-321 to 1-325, 1-327, 1-334, 1-335, 1-340 to 1-350, 1-352, 1-353, 1-355, 1-356, 1-358, 1-360, 1-361, 1-362, 1-364 to 1-367, 1-369 to 1-372, 1-375, 1-378 to 1-415, 1-417, 1-418, 1-421 to 1-430, 1-432, 1-434, 1-435, 1-437, 1-439, 1-441 to 1-444, 1-448, 1-450 to 1-453, 1-454 to 1-461, 1-463, 1-465, 1-467 to 1-476, 1-478 to 1-481, 1-485, 1-486, 1-488 to 1-491, 1-493 to 1-496, 1-498 to 1-518, 1-520 to 1-523, 1-525 to 1-527, 1-528, 1-529, 1-531 to 1-536, 1-540, 1-546 to 1-548, 1-551 to 1-555, 1-557, 1-559, 1-560, 1-562 to 1-567, 1-569 to 1-577, 1-579 to 1-588, 1-591 to 1-593, 1-594, 1-595, 1-596, 1-598, 1-599, 1-602, 1-604, 1-606, 1-607, 1-608, 2-002, 3-003, 3-004, 3-005, 3-006, 3-007, 3-008, 3-009, 3-010, 3-011, 3-012, 3-013, 4-001, 4-002, 5-005, 6-001, 6-002, 6-004, 6-005, 6-006, 6-008, 6-009, 6-010, 7-002, 7-003, 7-004, 1-031(S)-(+), 1-039(S)-(-), 1-048(R)-(+), 1-048(S)-(-), 1-050(S)-(+), 1-054(S)-(+), 1-118(+), 1-118(-), B-006, B-008, B-009, B-012, B-014 to B-016, B-018, B-020, B-021, C-001, C-002 and F-001 of the present invention.

TEST EXAMPLE 2: Insecticidal Test on Bemisia argentifolii

In styrol cups having an inner diameter of 7 cm, wet filter paper was laid, and kidney bean leaves cut to 3 cm were laid on the paper. 10% emulsifiable concentrates (or 10%wettable powders) of compounds of the present invention were diluted with water containing a spreader to obtain 500 ppm solutions. 2.5 ml of the solutions were sprayed from a rotary spray tower into the styrol cups (2.5 mg/$cm^2$). The leaves were dried in air, and adults of Bemisia argentifolii were released in the cups. The cups were closed and placed in an incubator at 25°C. 5 Days after, dead insects were counted, and the mortality was calculated by using the same equation as in Test Example 1. The test was carried out in duplicate.

Among the compounds tested, the following compounds showed a mortality of at least 90%.

Compounds Nos. 1-011, 1-012, 1-013, 1-018 to 1-023, 1-028, 1-031 to 1-034, 1-037, 1-038 to 1-040, 1-041, 1-042, 1-044, 1-045, 1-046, 1-048 to 1-054, 1-056, 1-059, 1-060, 1-061, 1-063 to 1-070, 1-073, 1-074, 1-076 to 1-090, 1-094 to 1-098, 1-100 to 1-107, 1-109 to 1-113, 1-115 to 1-124, 1-126, 1-127, 1-130, 1-134, 1-138 to 1-144, 1-146, 1-147, 1-149, 1-151, 1-152, 1-153, 1-154, 1-155, 1-157, 1-162, 1-163, 1-165, 1-167, 1-172, 1-173, 1-177, 1-178, 1-179, 1-183, 1-184, 1-188, 1-189, 1-190, 1-193, 1-194, 1-198, 1-201 to 1-206, 1-208, 1-209, 1-210, 1-212, 1-213, 1-220, 1-221, 1-222,

1-223, 1-225 to 1-237, 1-239 to 1-244, 1-248, 1-253, 1-254, 1-255, 1-259 to 1-267, 1-270, 1-271, 1-276, 1-284, 1-288 to 1-291, 1-294 to 1-296, 1-299, 1-303, 1-304, 1-305, 1-308 to 1-311, 1-313 to 1-319, 1-321 to 1-325, 1-327, 1-334, 1-335, 1-356, 1-360, 1-367, 1-369, 1-370, 1-383, 1-384, 1-389, 1-394, 1-396, 1-397, 1-407, 1-408, 1-409, 1-412, 1-414, 1-421, 1-426, 1-428, 1-432, 1-435, 1-438, 1-439, 1-441, 1-443, 1-444, 1-448, 1-458 to 1-461, 1-469, 1-475, 1-478, 1-479, 1-488, 1-490, 1-491, 1-493 to 1-496, 1-499, 1-501, 1-503 to 1-507, 1-509, 1-510, 1-513 to 1-520, 1-522, 1-523, 1-527, 1-528, 1-530, 1-535, 1-536, 1-546, 1-548, 1-551, 1-562 to 1-567, 1-569 to 1-573, 1-575, 1-576, 1-579, 1-581 to 1-584, 1-586 to 1-588, 1-602, 1-606, 3-001, 3-002, 3-004, 3-005, 3-010, 3-012, 3-013, 4-001, 5-004, 6-001, 6-004, 6-008, 6-009, 1-031(S)-(+), 1-039(S)-(-), 1-048(R)-(+), 1-048(S)-(-), 1-050(S)-(+), 1-054(S)-(+), 1-118(+) and 1-118(-) of the present invention.

TEST EXAMPLE 3: Insecticidal Test on Myzus persicae

Wet absorbent cotton was laid on glass dishes having an inner diameter of 3 cm, and covered with leaves of Canarium album cut into circles of the same diameter, and 4 apterous adults of Myzus persicae were released. After a day, 10% emulsifiable concentrates (or 10%wettable powders) of compounds of the present invention were diluted with water containing a spreader to obtain 500 ppm solutions, and the solution were sprayed from a rotary spray tower (2.5 $mg/cm^2$). The dishes were covered with lids and placed in an incubator at 25°C. 6 Days after, dead insects were counted, and the mortality was calculated by using the same equation as in Test Example 1. The test was carried out in duplicate.
Among the compounds tested, the following compounds showed a mortality of at least 90%.
Compounds Nos. 1-001, 1-002, 1-003, 1-011, 1-012, 1-013, 1-014, 1-018, 1-019, 1-020, 1-021, 1-022, 1-023, 1-026, 1-027, 1-028, 1-031 to 1-033, 1-035 to 1-068, 1-073 to 1-089, 1-092 to 1-107, 1-109 to 1-129, 1-132, 1-133, 1-134, 1-136, 1-137, 1-139 to 1-144, 1-146 to 1-155, 1-157, 1-158, 1-162 to 1-166, 1-168 to 1-174, 1-177, 1-178, 1-179, 1-183, 1-184, 1-186, 1-188 to 1-196, 1-198 to 1-210, 1-212 to 1-219, 1-220, 1-221, 1-222, 1-223, 1-225 to 1-244, 1-245, 1-247, 1-248, 1-252 to 1-255, 1-259 to 1-325, 1-327 to 1-349, 1-351 to 1-356, 1-360 to 1-365, 1-367 to 1-394, 1-396, 1-397, 1-399 to 1-411, 1-413, 1-414, 1-415, 1-417 to 1-421, 1-423, 1-425, 1-426, 1-427, 1-428, 1-430 to 1-437, 1-439 to 1-444, 1-446 to 1-448, 1-450 to 1-461, 1-463, 1-465, 1-467 to 1-470, 1-473, 1-475, 1-476, 1-477, 1-478 to 1-486, 1-488 to 1-491, 1-493 to 1-501, 1-503 to 1-523, 1-525 to 1-528, 1-529, 1-530 to 1-536, 1-540, 1-546 to 1-548, 1-551 to 1-554, 1-559, 1-560, 1-562 to 1-566, 1-568 to 1-576, 1-579 to 1-589, 1-591 to 1-593, 1-595, 1-596, 1-597, 1-598 to 1-607, 2-002, 3-001 to 3-004, 3-005, 3-006, 3-007, 3-008, 3-009, 3-010, 3-011, 3-012, 3-013, 4-001, 4-002, 5-004, 5-005, 5-007, 6-001, 6-002, 6-004, 6-005, 6-008, 6-009, 7-001 to 7-004, 1-031(S)-(+), 1-039(S)-(-), 1-048(R)-(+), 1-048(S)-(-), 1-050(S)-(+), 1-054(S)-(+), 1-118(+), 1-118(-), A-003, A-004, A-009, B-001 to B-004, B-006, B-008, B-009, B-014 to B-016, B-018, B-020, C-001, C-002, G-002, H-001, I-001 and J-001 of the present invention.

TEST EXAMPLE 4: Insecticidal Test on Aphis gossypii

Wet absorbent cotton was laid on glass dishes having an inner diameter of 3 cm, and covered with leaves of cucumber cut into circles of the same diameter, and 4 apterous adults of Aphis gossypii were released. After a day, 10% emulsifiable concentrates (or 10%wettable powders) of compounds of the present invention were diluted with water containing a spreader to obtain 500 ppm solutions, and the solution were sprayed from a rotary spray tower (2.5 $mg/cm^2$). The dishes were covered with lids and placed in an incubator at 25°C. 6 Days after, dead insects were counted, and the mortality was calculated by using the same equation as in Test Example 1. The test was carried out in duplicate.
Among the compounds tested, the following compounds shoed a mortality of at least 90%.
Compounds Nos. 1-001, 1-002, 1-011, 1-019, 1-020, 1-023, 1-026, 1-028, 1-031, 1-032, 1-033, 1-035 to 1-054, 1-056, 1-057, 1-058, 1-063 to 1-068, 1-073 to 1-080, 1-082 to 1-089, 1-092 to 1-107, 1-109 to 1-118, 1-120 to 1-124, 1-126, 1-134, 1-137, 1-139 to 1-144, 1-146, 1-147, 1-148, 1-149, 1-151, 1-153, 1-154, 1-155, 1-157, 1-158, , 1-162, 1-163, 1-165, 1-172, 1-173, 1-175 to 1-178, 1-182, 1-184, 1-186 to 1-194, 1-196, 1-198 to 1-210, 1-212 to 1-223, 1-225 to 1-229, 1-231 to 1-236, 1-238, 1-239, 1-240, 1-242, 1-243, 1-244, 1-246 to 1-249, 1-252, 1-254, 1-255, 1-259 to 1-271, 1-275 to 1-299, 1-301 to 1-323, 1-325, 1-326, 1-327, 1-330 to 1-335, 1-337 to 1-340, 1-343 to 1-356, 1-358, 1-360, 1-362, 1-364 to 1-367, 1-369 to 1-373, 1-375 to 1-387, 1-389, 1-390, 1-392 to 1-397, 1-399 to 1-410, 1-413 to 1-418, 1-420, 1-421, 1-425, 1-426, 1-427, 1-428, 1-429 to 1-435, 1-438 to 1-444, 1-448, 1-450, 1-451, 1-452, 1-454, 1-455, 1-456, 1-457, 1-459, 1-460, 1-461, 1-464, 1-465, 1-469, 1-470, 1-472 to 1-481, 1-484 to 1-486, 1-488 to 1-523, 1-527 to 1-538, 1-541 to 551, 1-554, 1-560, 1-562 to 1-567, 1-569, 1-570, 1-571, 1-573 to 1-577, 1-579 to 1-588, 1-591, 1-593, 1-595, 1-600 to 1-604, 3-001 to 3-013, 4-001, 4-002, 4-003, 5-001, 6-001, 6-002, 6-004, 6-008 to 6-010, 7-002, 7-003, 7-004, 1-031(S)-(+), 1-039(S)-(-), 1-048(R)-(+), 1-048(S)-(-), 1-050(S)-(+), 1-054(S)-(+), 1-118(+), 1-118(-), A-001, A-009, B-001 to B-003, B-006, B-008, B-009, B-014 to B-016 and B-019 to B-022 of the present invention.

TEST EXAMPLE 5: Soil Irrigation Test on Myzus persicae

10% emulsifiable concentrates of compounds of the present invention were diluted with tap water to obtain 100 ppm solutions. The soil around the bases of cabbage seedlings (at the 2.5 true leaf stage) planted in plastic cups was irrigated with 10 ml of the solutions, and the cups were placed in a greenhouse. One day after the irrigation, adults of Myzus persicae were released at a ratio of 20 insects per seedling, and the seedlings were incubated in the greenhouse. 6 Days after the release of the insects, living insects were counted, and the control value was calculated from the following equation.

$$\text{Control value (\%)} = \{1-(Cb\times Tai)/(Cai\times Tb)\}\times 100$$

Cb: the number of insects in a non-treated area before treatment
Cai: the final number of living insects in a non-treated area
Tb: the number of insects in a treated area before treatment
Tai: the final number of living insects in a treated area
Among the compounds tested, the following compounds showed a control value of at least 90%.
Compounds Nos. 1-011, 1-013, 1-019, 1-023, 1-031 to 1-033, 1-035, 1-036, 1-038, 1-039, 1-041 to 1-045, 1-048, 1-049, 1-050, 1-052, 1-053, 1-054, 1-057, 1-063, 1-073 to 1-076, 1-079, 1-080, 1-084, 1-086, 1-095, 1-096, 1-097, 1-099 to 1-104, 1-106, 1-107, 1-111 to 1-116, 1-118, 1-120, 1-122, 1-124, 1-134, 1-143, 1-146, 1-151, 1-154, 1-155, 1-156, 1-157, 1-158, 1-160, 1-162, 1-163, 1-177, 1-178, 1-179, 1-190, 1-193, 1-194, 1-196, 1-198, 1-201 to 1-210, 1-212, 1-225, 1-226, 1-229 to 1-237, 1-240, 1-242, 1-243, 1-244, 1-246, 1-247, 1-248, 1-249, 1-253, 1-255, 1-259, 1-261 to 1-267, 1-269, 1-270, 1-271, 1-275, 1-276, 1-281, 1-284, 1-285, 1-314, 1-316, 1-318, 1-319, 1-322, 1-323, 1-353, 1-360, 1-369, 1-370, 1-371, 1-383, 1-384, 1-389, 1-390, 1-478, 1-479, 1-484, 1-488 to 1-490, 1-493 to 1-496, 1-498 to 1-501, 1-504 to 1-508, 1-511, 1-513 to 1-518, 1-521, 1-537, 1-538, 1-542, 1-545, 1-547, 1-550, 1-551, 3-004, 3-010, 3-012, 6-001, 6-008, 6-009, 1-031(S)-(+), 1-039(S)-(-), 1-048(R)-(+), 1-048(S)-(-), 1-054(S)-(+), 1-118(+) and 1-118(-) of the present invention.

TEST EXAMPLE 6: Test on the effect of seed treatment on Aphis glycines

2.4 mg of compounds of the present invention were diluted with 97.6 μl of acetone. Four soybean seeds were put in each 50 ml plastic tube, and the solutions of compounds of the present invention were poured onto the seeds and stirred until the acetone evaporated completely so that the seeds were evenly coated with the compounds. The treated seeds were sown in pots, 4 seeds per pot, and placed in a greenhouse. After the primary leaf folded out, two adults of Aphis glycines were released per seedling. 7 Days after the release of the insects, living insects were counted, and the control value was calculated from the following equation.

$$\text{Control value (\%)}=\{1-(Cb\times Tai)/(Cai\times Tb)\}\times 100$$

wherein
Cb: the number of insects in a non-treated area before treatment
Cai: the final number of living insects in a non-treated area
Tb: the number of insects in a treated area before treatment
Tai: the final number of living insects in a treated area
Among the compounds tested, the following compounds showed a control value of at least 90%.
Compounds Nos. 1-031, 1-033, 1-035, 1-038 to 1-042, 1-048 to 1-054, 1-276, 1-048(R)-(+), 1-048(S)-(-), 1-118(+) and 1-118(-) of the present invention.

INDUSTRIAL APPLICABILITY

The novel pyrazole derivatives of the present invention are very useful compounds which are excellent in pesticidal activities, especially in insecticidal and miticidal activities, and have little harmful effect on non-target organisms such as mammals, fishes and beneficial insects.
The entire disclosures of Japanese Patent Application No. 2011-025875 filed on February 9, 2011, Japanese Patent Application No. 2011-106993 filed on May 12, 2011, Japanese Patent Application No. 2011-143871 filed on June 29, 2011, Japanese Patent Application No. 2011-176256 filed on August 11, 2011, Japanese Patent Application No. 2011-223837 filed on October 11, 2011, Japanese Patent Application No. 2011-234671 filed on October 26, 2011 and

Japanese Patent Application No. 2011-252596 filed on November 18, 2011 including specifications, claims and summaries are incorporated herein by reference in their entireties.

## Claims

1. A pyrazole derivative represented by the formula (1) or a salt thereof:

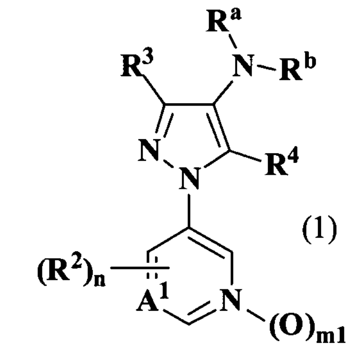

(1)

wherein $A^1$ is $-N(-O)_{m2}$ or $-CR^1$,

each of $R^1$ and $R^3$ is independently a hydrogen atom, a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkoxycarbonyl, $C_1$-$C_6$ haloalkoxycarbonyl, $C_1$-$C_6$ alkylaminosulfonyl, di($C_1$-$C_6$ alkyl)aminosulfonyl, $C_1$-$C_6$ alkylamino or di($C_1$-$C_6$ alkyl)amino,

$R^2$ is a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkoxycarbonyl, $C_1$-$C_6$ haloalkoxycarbonyl, $C_1$-$C_6$ alkylaminosulfonyl, di($C_1$-$C_6$ alkyl)aminosulfonyl, $C_1$-$C_6$ alkylamino or di($C_1$-$C_6$ alkyl)amino, provided that when n is an integer of at least 2,

each $R^2$ may be identical with or different from one another,

$R^4$ is a hydrogen atom, a halogen atom, cyano, nitro, -OH, -SH, $-NH_2$, $C_1$-$C_6$ alkyl, ($C_1$-$C_6$) alkyl optionally substituted with $R^{28a}$, $C_3$-$C_8$ cycloalkyl, ($C_3$-$C_8$) cycloalkyl optionally substituted with $R^{28a}$, $C_2$-$C_6$ alkenyl, ($C_2$-$C_6$) alkenyl optionally substituted with $R^{28a}$, $C_3$-$C_8$ cycloalkenyl, ($C_3$-$C_8$) cycloalkenyl optionally substituted with $R^{28a}$, $C_2$-$C_6$ alkynyl, ($C_2$-$C_6$) alkynyl optionally substituted with $R^{28a}$, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, $C_1$-$C_6$ haloalkylthio, $C_1$-$C_6$ haloalkylsulfinyl, $C_1$-$C_6$ haloalkylsulfonyl, $C_1$-$C_6$ alkylcarbonyl, $C_3$-$C_8$ cycloalkylcarbonyl, $C_1$-$C_6$ haloalkylcarbonyl, $C_3$-$C_8$ halocycloalkylcarbonyl, $C_1$-$C_6$ alkylamino or di($C_1$-$C_6$ alkyl)amino,

$R^a$ is a hydrogen atom, cyano, $C_1$-$C_{15}$ alkyl, ($C_1$-$C_{15}$) alkyl optionally substituted with $R^5$, $C_3$-$C_{15}$ cycloalkyl, ($C_3$-$C_{15}$) cycloalkyl optionally substituted with $R^5$, $C_2$-$C_{15}$ alkenyl, ($C_2$-$C_{15}$) alkenyl optionally substituted with $R^5$, $C_3$-$C_{12}$ cycloalkenyl, ($C_3$-$C_{15}$) cycloalkenyl optionally substituted with $R^5$, $C_2$-$C_{12}$ alkynyl, ($C_2$-$C_{15}$) alkynyl optionally substituted with $R^5$, $-OR^6$, $-S(O)_rR^6$, $-C(O)R^{7a}$, $-C(O)OR^{6a}$, $-NR^{8c}R^{8d}$, $-C(=NR^{8b})R^{7a}$, $-S(O)_rN(R^{8a})R^8$, phenyl, phenyl substituted with (Z)q, naphthyl, naphthyl substituted with (Z)q or D1-1 to D1-99,

$R^b$ is $-S(O)_rR^6$, $-C(O)R^7$, $-C(S)R^7$, $-C(O)OR^{6a}$, $-C(O)SR^{6a}$, $-C(S)OR^{6a}$, $-C(S)SR^{6a}$, $-C(O)N(R^{8a})R^8$, $-C(S)N(R^{8a})R^8$, $-C(O)N(R^{8b})N(R^{8a})R^8$, $-C(O)N(R^{88})OR^{6a}$, $-C(=NR^{8b})OR^{6a}$, $-C(=NR^{8b})SR^{68}$, $-C(=NR^{8b})N(R^{8a})R^8$, $-C(=NR^{8b})R^7$, D1-49, D1-51, D1-53, D1-59, D1-61 or D1-63 or $R^b$ may form $=C(R^{b2})R^{b3}$ together with $R^a$,

$R^{b2}$ is a hydrogen atom, $C_1$-$C_{15}$ alkyl or $-S(O)_rR^6$,

$R^{b3}$ is ($C_1$-$C_{15}$) alkyl optionally substituted with $R^{14}$, $-OR^6$, $-S(O)_rR^6$ or $-N(R^{8b})R^8$, or $R^{b3}$ may form, together with $R^{b2}$, a $C_4$-$C_6$ alkylene chain or a $C_4$-$C_6$ alkenylene chain to form a 5 to 7-membered ring together with the carbon atom attached to $R^{b3}$ and $R^{b2}$, wherein the alkylene chain or the alkenylene chain may contain from 1 to 3 oxygen

atoms, sulfur atoms or nitrogen atoms and may optionally be substituted with a halogen atom, a cyano group, a nitro group, $C_1$-$C_6$ alkyl, ($C_1$-$C_{12}$) alkyl optionally substituted with $R^{14}$, -S(O)$_r$$R^6$, -C(O)$R^7$, -C(S)$R^7$, -C(O)O$R^{6a}$, -C(O)S$R^{6a}$, -C(S)O$R^{6a}$, -C(S)S$R^{6a}$, -C(O)N($R^{8a}$)$R^8$, -C(S)N($R^{8a}$)$R^8$, -C(O)N($R^{8b}$)N($R^{8b}$)$R^8$, -C(O)N($R^{8a}$)O$R^6$, -C(=N$R^{8b}$)O$R^{6a}$, -C(=N$R^{8b}$)S$R^{6a}$, -C(=N$R^{8b}$)N($R^{8a}$)$R^8$, -C(=N$R^{8b}$)$R^7$, phenyl, phenyl substituted with (Z)q, an oxo group, a thioxo group, =N$R^{8b}$ or a $C_1$-$C_6$ alkylidene group, $R^5$ is a halogen atom, cyano, nitro, $C_3$-$C_{15}$ cycloalkyl, ($C_3$-$C_{15}$) cycloalkyl optionally substituted with $R^{14}$, -OH, -O$R^{11}$, -SH, -S(O)$^r$$R^{11}$, -C(O)$R^{12}$, -C(O)O$R^{11a}$, -C(O)S$R^{6a}$, -C(S)O$R^{6a}$, -C(S)S$R^{6a}$, -C(O)N($R^{138}$)$R^{13}$, -C(S)N($R^{13a}$)$R^{13}$, -C(=NOH)$R^{12}$, -C(=NO$R^{11}$)$R^{12}$, -N($R^{13a}$)$R^{13}$, -Si($R^{9a}$)($R^{9b}$)$R^9$, -P(O)(O$R^{10}$)$_2$, -P(S)(O$R^{10}$)$_2$, phenyl, phenyl substituted with $(Z^3)_q$, naphthyl, naphthyl substituted with (Z)q or D1-1 to D1-99, each of $R^6$ and $R^{6a}$ is independently $C_1$-$C_{15}$ alkyl, ($C_1$-$C_{15}$) alkyl optionally substituted with $R^{14}$, $C_3$-$C_{15}$ cycloalkyl, ($C_3$-$C_{15}$) cycloalkyl optionally substituted with $R^{14}$, $C_2$-$C_{15}$ alkenyl, ($C_2$-$C_{15}$) alkenyl optionally substituted with $R^{14}$, $C_3$-$C_{15}$ cycloalkenyl, ($C_3$-$C_{15}$) cycloalkenyl optionally substituted with $R^{14}$, $C_2$-$C_{15}$ alkynyl, ($C_2$-$C_{15}$) alkynyl optionally substituted with $R^{14}$, phenyl, phenyl substituted with (Z)q, naphthyl, naphthyl substituted with (Z)q or D1-1 to D1-99,

$R^7$ is a hydrogen atom, $C_1$-$C_{15}$ alkyl, ($C_1$-$C_{15}$) alkyl optionally substituted with $R^{14}$, $C_3$-$C_{15}$ cycloalkyl, ($C_3$-$C_{15}$) cycloalkyl optionally substituted with $R^{14}$, $C_2$-$C_{15}$ alkenyl, ($C_2$-$C_{15}$) alkenyl optionally substituted with $R^{14}$, $C_3$-$C_{15}$ cycloalkenyl, ($C_3$-$C_{15}$) cycloalkenyl optionally substituted with $R^{14}$, $C_2$-$C_{15}$ alkynyl, ($C_2$-$C_{15}$) alkynyl optionally substituted with $R^{14}$, -C(O)$R^{12}$, -C(O)O$R^{11}$, -C(O)N($R^{13a}$)$R^{13}$, -C(S)N($R^{13a}$)$R^{13}$, -C(=NOH)$R^{12}$, -C(=NO$R^{11}$)$R^{12}$, -C{=NN($R^{13a}$)$R^{13}$)$R^{12}$, phenyl, phenyl substituted with (Z)q, naphthyl, naphthyl substituted with (Z)q or D1-1 to D1-99, or $R^7$ may form, together with $R^a$, a $C_2$-$C_6$ alkylene chain or a $C_2$-$C_6$ alkenylene chain containing a double bond to form a 4 to 8-membered ring together with the carbon atom attached to $R^7$ and the nitrogen atom attached to $R^a$, wherein the alkylene chain or the alkenylene chain may contain one or two oxygen atoms, sulfur atoms or nitrogen atoms and may optionally be substituted with a halogen atom, cyano, nitro, $C_1$-$C_6$ alkyl, ($C_1$-$C_6$) alkyl optionally substituted with $R^{14}$, -OH, -O$R^{11}$, -SH, -S(O)$_r$$R^{11}$, an oxo group, a thioxo group, =N$R^{8b}$ or a $C_1$-$C_6$ alkylidene group,

$R^{7a}$ is a hydrogen atom or $R^{6a}$,

$R^8$ is a hydrogen atom, cyano, $R^{6a}$,-S(O)$_r$$R^{11}$, -C(O)$R^{12}$, -C(O)O$R^{11a}$, -C(O)N($R^{13a}$)$R^{13}$, -C(S)N($R^{13a}$)$R^{13}$, -C(=NOH)$R^{12}$, -C(=NO$R^{11}$)$R^{12}$, -S(O)$_2$N($R^{13a}$)$R^{13}$, phenyl, phenyl substituted with (Z)q, naphthyl, naphthyl substituted with (Z)q or D1-1 to D1-99, or $R^8$ may form, together with $R^{8a}$, a $C_2$-$C_7$ alkylene chain to form a 3 to 8-membered ring together with the nitrogen atom attached to $R^8$ and $R^{8a}$, wherein the alkylene chain may contain an oxygen atom, a sulfur atom or a nitrogen atom and may optionally be substituted with a halogen atom, cyano, $C_1$-$C_6$ alkyl, ($C_1$-$C_6$) alkyl optionally substituted with $R^{14}$, phenyl, phenyl substituted with (Z)q, an oxo group or a thioxo group, or $R^8$ may form =C($R^{8f}$)$R^{8e}$ together with $R^{8a}$,

$R^{8a}$ is a hydrogen atom, cyano, $C_1$-$C_{15}$ alkyl, ($C_1$-$C_{15}$) alkyl optionally substituted with $R^{14a}$, $C_3$-$C_{15}$ cycloalkyl, ($C_3$-$C_{15}$) cycloalkyl optionally substituted with $R^{14a}$, $C_2$-$C_{15}$ alkenyl, ($C_2$-$C_{15}$) alke or nyl optionally substituted with $R^{14a}$, $C_3$-$C_{15}$ cycloalkenyl, ($C_3$-$C_{15}$) cycloalkenyl optionally substituted with $R^{14a}$, $C_2$-$C_{15}$ alkynyl or ($C_2$-$C_{15}$) alkynyl optionally substituted with $R^{14a}$, or $R^{8a}$ may form, together with $R^a$, a $C_2$-$C_5$ alkylene chain to form a 5 to 8-membered ring together with the nitrogen atom attached to $R^{8a}$ and the nitrogen atom attached to $R^a$, wherein the alkylene chain may contain an oxygen atom, a sulfur atom or a nitrogen atom and may optionally be substituted with a halogen atom, cyano, nitro, $C_1$-$C_6$ alkyl, ($C_1$-$C_6$) alkyl optionally substituted with $R^{14}$, -OH, -O$R^{11}$, -SH, -S(O)$^r$$R^{11}$, an oxo group, a thioxo group or =N$R^{8b}$,

$R^{8b}$ is a hydrogen atom, cyano, nitro, $C_1$-$C_{15}$ alkyl, ($C_1$-$C_{15}$) alkyl optionally substituted with $R^{14}$, $C_3$-$C_{15}$ cycloalkyl, ($C_3$-$C_{15}$) cycloalkyl optionally substituted with $R^{14}$, $C_2$-$C_{15}$ alkenyl, ($C_2$-$C_{15}$) alkenyl optionally substituted with $R^{14}$, $C_3$-$C_{15}$ cycloalkenyl, ($C_3$-$C_{15}$) cycloalkenyl optionally substituted with $R^{14}$, $C_2$-$C_{15}$ alkynyl, ($C_2$-$C_{15}$) alkynyl optionally substituted with $R^{14}$, -O$R^{11}$, -S(O)$^r$$R^{11}$, -C(O)O$R^{11a}$, -C(O)$R^{12}$, -C(O)N($R^{13a}$)$R^{13}$, -C(S)N($R^{13a}$)$R^{13}$ or -S(O)$_2$N($R^{13a}$)$R^{13}$,

each of $R^{8c}$ and $R^{8d}$ is independently a hydrogen atom, $C_1$-$C_6$ alkyl, -S(O)$_r$$R^{11}$, -C(O)O$R^{11a}$, -C(O)$R^{12}$, -C(O)N($R^{13a}$)$R^{13}$ or -C(S)N($R^{13a}$)$R^{13}$,

$R^{8e}$ is a hydrogen atom, $C_1$-$C_{15}$ alkyl or -S(O)$_r$$R^{11}$,

$R^{8f}$ is $C_1$-$C_{15}$ alkyl, ($C_1$-$C_{15}$) alkyl optionally substituted with $R^{14}$, -O$R^{11}$, -S(O)$_r$$R^{11}$, -N($R^{13a}$)$R^{13}$, phenyl, phenyl substituted with (Z)q, naphthyl, naphthyl substituted with $(Z)_q$ or or D1-1 to D1-99, each of $R^9$, $R^{9a}$ and $R^{9b}$ is independently $C_1$-$C_6$ alkyl,

$R^{10}$ is a hydrogen atom or $C_1$-$C_6$ alkyl,

each of $R^{11}$ and $R^{11a}$ is independently $C_1$-$C_6$ alkyl, ($C_1$-$C_6$) alkyl optionally substituted with $R^{14a}$, $C_2$-$C_6$ alkenyl, ($C_2$-$C_6$) alkenyl optionally substituted with $R^{14a}$, $C_3$-$C_8$ cycloalkenyl, ($C_3$-$C_8$) cycloalkenyl optionally substituted with $R^{14a}$, $C_2$-$C_6$ alkynyl, ($C_2$-$C_6$) alkynyl optionally substituted with $R^{14a}$, $C_3$-$C_8$ cycloalkyl, ($C_3$-$C_8$) cycloalkyl optionally substituted with $R^{14a}$, -Si($R^{9a}$)($R^{9b}$)$R^9$, phenyl, phenyl substituted with (Z)q, naphthyl, naphthyl substituted with (Z)q or or D1-1 to D1-99,

$R^{12}$ is a hydrogen atom, $R^{11a}$, -C(O)$R^{16}$, -C(=NOH)$R^{16}$, -C(=NO$R^{15}$)$R^{16}$, phenyl, phenyl substituted with (Z)q,

naphthyl, naphthyl substituted with (Z)q or or D1-1 to D1-99, each of $R^{13}$ and $R^{13a}$ is independently a hydrogen atom, $R^{11a}$, $-S(O)_rR^{15}$, $-C(O)OR^{15a}$, $-C(O)R^{16}$, $-C(O)N(R^{17a})R^{17}$, $-C(S)N(R^{17a})R^{17}$, $-S(O)_2N(R^{17a})R^{17}$, phenyl, phenyl substituted with (Z)q, naphthyl, naphthyl substituted with (Z)q or or D1-1 to D1-99, or $R^{13}$ and $R^{13a}$ may form, together with each other, a $C_2$-$C_7$ alkylene chain to form a 3 to 8-membered ring together with the nitrogen atom attached to $R^{13}$ and $R^{13a}$, wherein the alkylene chain may contain an oxygen atom, a sulfur atom or a nitrogen atom and may optionally be substituted with a halogen atom, cyano, $C_1$-$C_6$ alkyl, ($C_1$-$C_6$) alkyl optionally substituted with $R^{14}$, phenyl, phenyl substituted with (Z)q, an oxo group or a thioxo group,

each of $R^{14}$ and $R^{14a}$ is independently a halogen atom, cyano, nitro, $C_3$-$C_8$ cycloalkyl, ($C_3$-$C_8$) cycloalkyl optionally substituted with $R^{19}$, -OH, $-OR^{15}$, -SH, $-S(O)_rR^{15}$, $-S(=NR^{17b})R^{15a}$, $-S(O)(=NR^{17b})R^{15a}$, -C(O)OH, $-C(O)OR^{15a}$, $-C(O)SR^{15a}$, $-C(S)OR^{15a}$, $-C(S)SR^{15a}$, $-C(O)R^{16}$, $-C(O)N(R^{17a})R^{17}$, $-C(S)N(R^{17a})R^{17}$, $-N(R^{17a})R^{17}$, $-C(=NOH)R^{16}$, $-C(=NOR^{15})R^{16}$, $-ON=C(R^{16a})R^{16}$, $-S(O)_2N(R^{17a})R^{17}$, $-Si(R^{9a})(R^{9b})R^9$, $-P(O)(OR^{10})_2$, $-P(S)(OR^{10})_2$, phenyl, phenyl substituted with (Z)q, naphthyl, naphthyl substituted with (Z)q or D1-1 to D1-99,

each of $R^{15}$ and $R^{15a}$ is independently cyano, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, $-S(O)_rR^{20}$, $-C(O)OR^{20a}$, $-C(O)R^{21}$, $-C(O)N(R^{22a})R^{22}$, $-C(S)N(R^{22a})R^{22}$, phenyl, phenyl substituted with $(Z)_{q2}$, naphthyl, naphthyl substituted with $(Z)_q$ or D1-1 to D1-99,

each of $R^{16}$ and $R^{16a}$ is independently a hydrogen atom, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, $-C(O)R^{21}$, phenyl, phenyl substituted with (Z)q, naphthyl, naphthyl substituted with (Z)q or D1-1 to D1-99,

each of $R^{17}$ and $R^{17a}$ is independently a hydrogen atom, cyano, $C_1$-$C_6$ alkyl, ($C_1$-$C_6$) alkyl optionally substituted with $R^{19}$, $C_2$-$C_6$ alkenyl, ($C_2$-$C_6$) alkenyl optionally substituted with $R^{19}$, $C_2$-$C_6$ alkynyl, ($C_2$-$C_6$) alkynyl optionally substituted with $R^{19}$, $C_3$-$C_8$ cycloalkyl, ($C_3$-$C_8$) cycloalkyl optionally substituted with $R^{19}$, $C_3$-$C_8$ cycloalkenyl, ($C_3$-$C_8$) cycloalkenyl optionally substituted with $R^{19}$, -CHO, $-S(O)^rR^{20}$, $-C(O)R^{21}$, $-C(O)OR^{20a}$, $-C(O)N(R^{22a})R^{22}$, $-C(S)N(R^{22a})R^{22}$ or $-S(O)_2N(R^{22a})R^{22}$, or $R^{17}$ and $R^{17a}$ may form, together with each other, a $C_2$-$C_7$ alkylene chain to form a 3 to 8-membered ring together with the nitrogen atom attached to $R^{17}$ and $R^{17a}$, wherein the alkylene chain may contain an oxygen atom, a sulfur atom or a nitrogen atom and may optionally be substituted with a halogen atom, cyano, $C_1$-$C_6$ alkyl, ($C_1$-$C_6$) alkyl optionally substituted with $R^{14}$, phenyl, phenyl substituted with (Z)q, an oxo group or a thioxo group,

$R^{17b}$ is a hydrogen atom, cyano, nitro, $-OR^{20}$, $-S(O)_rR^{20}$, $-C(O)OR^{20a}$, $-C(O)R^{21}$, $-C(O)N(R^{22a})R^{22}$, $-C(S)N(R^{22a})R^{22}$ or $-S(O)_2N(R^{22a})R^{22}$,

$R^{19}$ is a halogen atom, cyano, $C_3$-$C_8$ cycloalkyl, $-OR^{20}$, $-S(O)_rR^{20}$, $-C(O)OR^{20a}$, $-C(O)N(R^{22a})R^{22}$, $-C(S)N(R^{22a})R^{22}$, $-C(=NOR^{20})R^{21}$, $-Si(R^{9a})(R^{9b})R^9$, phenyl, phenyl substituted with (Z)q, naphthyl, naphthyl substituted with (Z)q or D1-1 to D1-99, each of $R^{20}$ and $R^{21}$ is independently a hydrogen atom, $C_1$-$C_6$ alkyl, ($C_1$-$C_6$) alkyl optionally substituted with $R^{32}$, $C_2$-$C_6$ alkenyl, ($C_2$-$C_6$) alkenyl optionally substituted with $R^{32}$, $C_2$-$C_6$ alkynyl, ($C_2$-$C_6$) alkynyl optionally substituted with $R^{32}$, $C_3$-$C_8$ cycloalkyl, ($C_3$-$C_8$) cycloalkyl optionally substituted with $R^{32}$, $C_3$-$C_8$ cycloalkenyl, ($C_3$-$C_8$) cycloalkenyl optionally substituted with $R^{32}$, phenyl, phenyl substituted with (Z)q, naphthyl, naphthyl substituted with (Z)q or D1-1 to D1-99,

each of $R^{20a}$, $R^{22}$ and $R^{22a}$ is independently a hydrogen atom, $C_1$-$C_6$ alkyl, ($C_1$-$C_6$) alkyl optionally substituted with $R^{32}$, $C_2$-$C_6$ alkenyl, ($C_2$-$C_6$) alkenyl optionally substituted with $R^{32}$, $C_2$-$C_6$ alkynyl, ($C_2$-$C_6$) alkynyl optionally substituted with $R^{32}$, $C_3$-$C_8$ cycloalkyl, ($C_3$-$C_8$) cycloalkyl optionally substituted with $R^{32}$, $C_3$-$C_8$ cycloalkenyl, ($C_3$-$C_8$) cycloalkenyl optionally substituted with $R^{32}$, phenyl, phenyl substituted with (Z)q, naphthyl, naphthyl substituted with (Z)q or D1-1 to D1-99,

D1-1 to D1-99 are rings represented by the following structures, respectively,

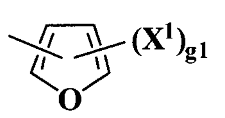
D1-1

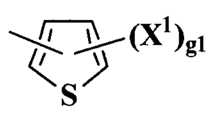
D1-2

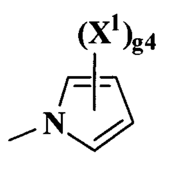
D1-3

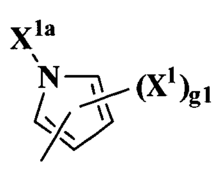
D1-4

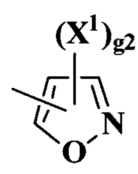
D1-5

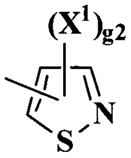

D1-6

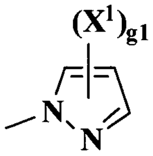

D1-7

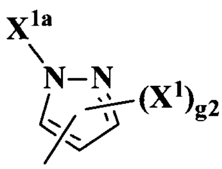

D1-8

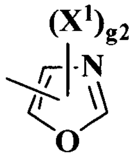

D1-9

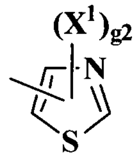

D1-10

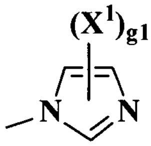

D1-11

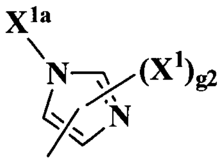

D1-12

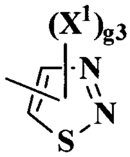

D1-13

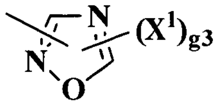

D1-14

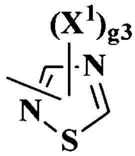

D1-15

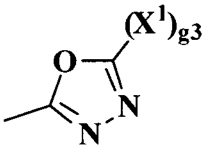

D1-16

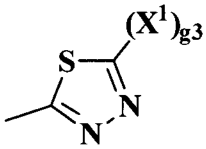

D1-17

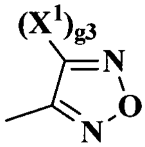

D1-18

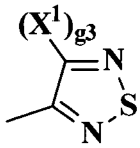

D1-19

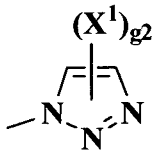

D1-20

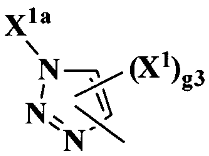

D1-21

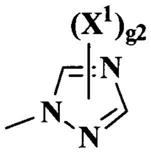

D1-22

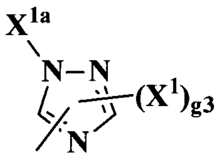

D1-23

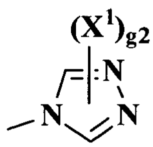

D1-24

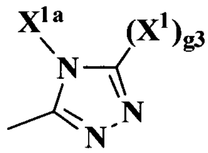

D1-25

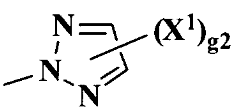

D1-26

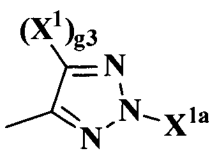

D1-27

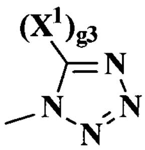

D1-28

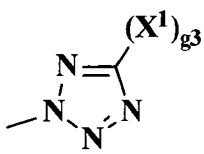

D1-29

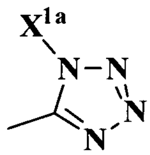

D1-30

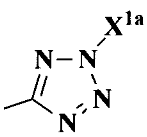

D1-31

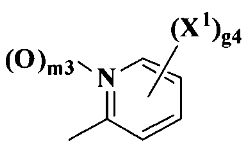

D1-32

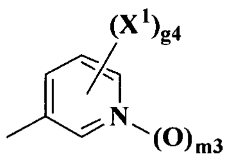

D1-33

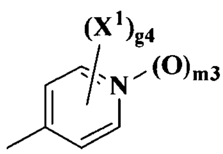

D1-34

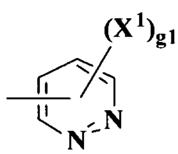

D1-35

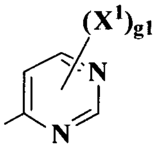
D1-36

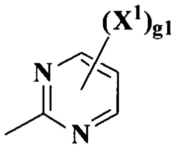
D1-37

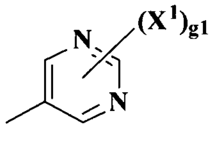
D1-38

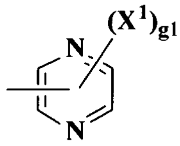
D1-39

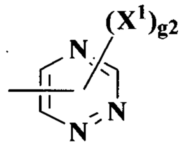
D1-40

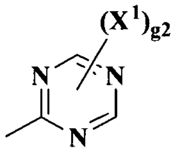
D1-41

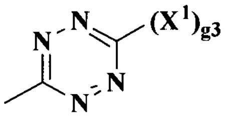
D1-42

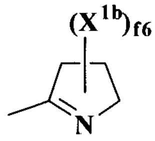
D1-43

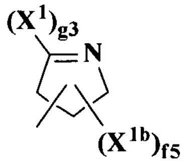
D1-44

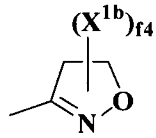
D1-45

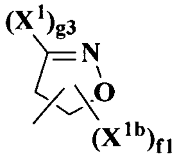
D1-46

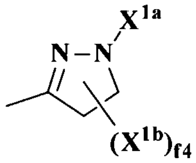
D1-47

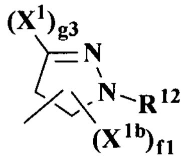
D1-48

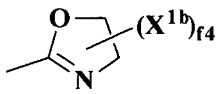
D1-49

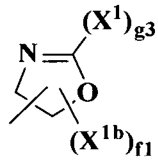
D1-50

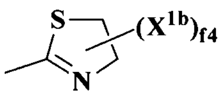
D1-51

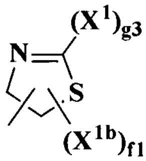
D1-52

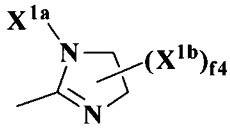
D1-53

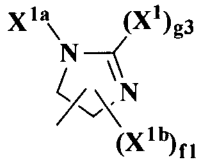
D1-54

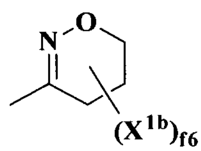
D1-55

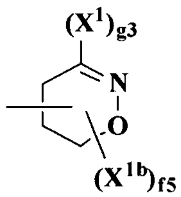
D1-56

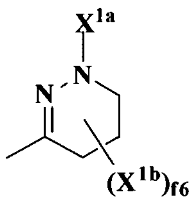
D1-57

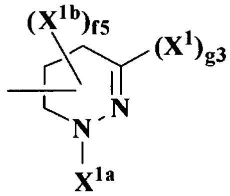
D1-58

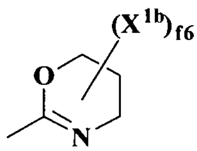
D1-59

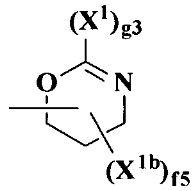
D1-60

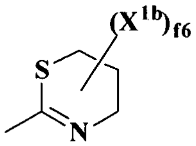
D1-61

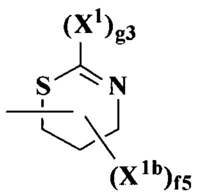
D1-62

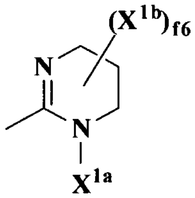
D1-63

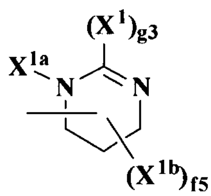
D1-64

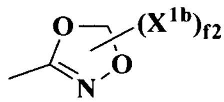
D1-65

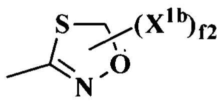

D1-66

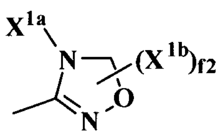

D1-67

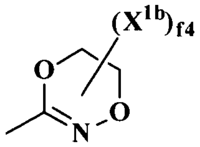

D1-68

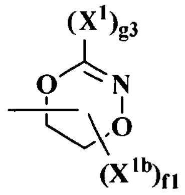

D1-69

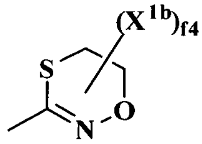

D1-70

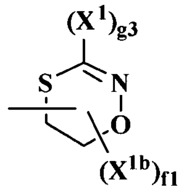

D1-71

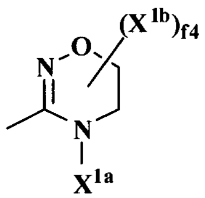

D1-72

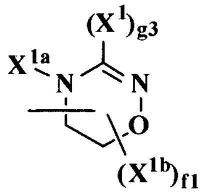

D1-73

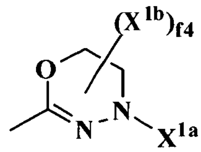

D1-74

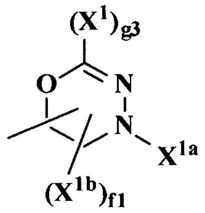

D1-75

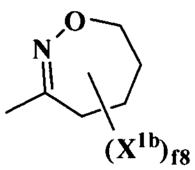

D1-76

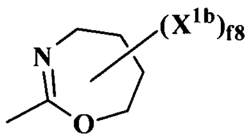

D1-77

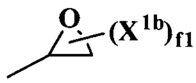

D1-78

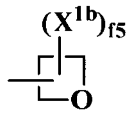

D1-79

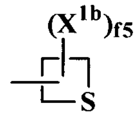

D1-80

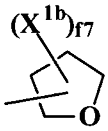

D1-81

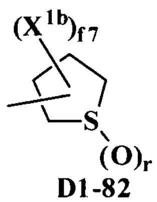

D1-82

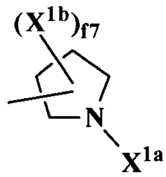

D1-83

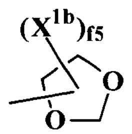

D1-84

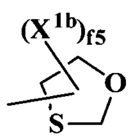

D1-85

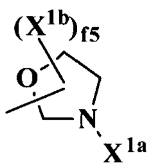

D1-86

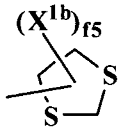

D1-87

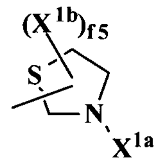

D1-88

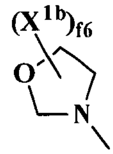

D1-89

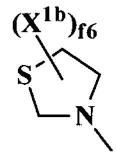

D1-90

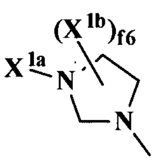

D1-91

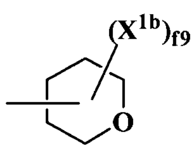

D1-92

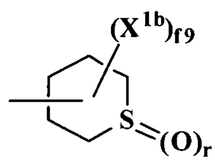

D1-93

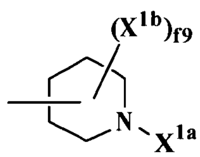

D1-94

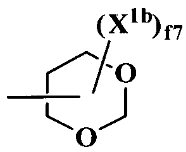

D1-95

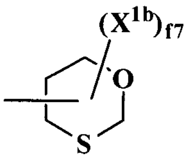

D1-96

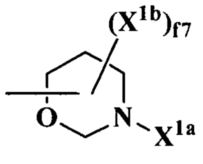

D1-97

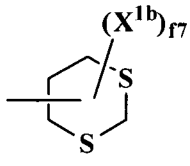

D1-98

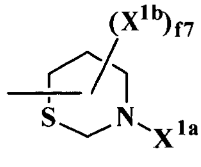

D1-99

$X^1$ is a halogen atom, cyano, nitro, -OH, -SH or $R^{24}$, provided that when g1, g2 or g4 is an integer of at least 2, each $X^1$ may be identical with or different from one another, and when there are two neighboring $X^1$'s, the two neighboring $X^1$'s may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon atoms attached to the $X^1$'s, a 5-membered ring or 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$X^{1a}$ is a hydrogen atom, cyano, -OH or $R^{24}$,

$X^{1b}$ is a halogen atom, cyano, nitro, -OH, -SH or $R^{24}$, provided that when f1, f2, f4, f5, f6, f7, f8 or f9 is an integer of at least 2, each $X^{1b}$ may be identical with or different from one another, and when there are two $X^{1b}$'s on the same carbon, the two $X^{1b}$'s may form oxo, thioxo, imino, $C_1$-$C_6$ alkylimino, $C_1$-$C_6$ alkoxyimino or $C_1$-$C_6$ alkylidene together with each other,

Z is a halogen atom, cyano, nitro, -OH, -SH or $R^{24}$, provided that q is an integer of at least 2, each Z may be identical with or different from one another, and when there are two neighboring Z's, the two neighboring Z's may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon atoms attached to the Z's, a 5-membered ring or a 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$Z^3$ is a halogen atom, nitro, -OH, -SH or $R^{24}$, provided that q is an integer of at least 2, each $Z^3$ may be identical with or different from one another, and when there are two neighboring $Z^3$'s, the neighboring two Z's may form $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-CH_2CH_2CH_2S-$ or $-OCH_2CH_2S-$ to form, together with the carbon atoms attached to the $Z^3$'s, a 5-membered ring or a 6-membered ring which may have one or more hydrogen atoms on the ring-constituting carbon atoms optionally replaced by one or more halogen atoms, one or more $C_1$-$C_6$ alkyl groups, one or more $C_1$-$C_6$ haloalkyl groups, one or more $C_1$-$C_6$ alkoxy groups, one or more $C_1$-$C_6$ alkylthio groups, one or more $C_1$-$C_6$ alkylsulfinyl groups or one or more $C_1$-$C_6$ alkylsulfonyl groups,

$R^{24}$ is a $C_1$-$C_6$ alkyl, ($C_1$-$C_6$) alkyl optionally substituted with $R^{28}$, $C_2$-$C_6$ alkenyl, ($C_2$-$C_6$) alkenyl optionally substituted with $R^{28}$, $C_3$-$C_8$ cycloalkenyl, ($C_3$-$C_8$) cycloalkenyl optionally substituted with $R^{28}$, $C_2$-$C_6$ alkynyl, ($C_2$-$C_6$) alkynyl optionally substituted with $R^{28}$, $C_3$-$C_8$ cycloalkyl, ($C_3$-$C_8$) cycloalkyl optionally substituted with $R^{28}$, $-OR^{25}$, $-S(O)_rR^{25}$, -C(O)OH, $-C(O)OR^{25a}$, $-C(O)R^{26}$, $-C(O)N(R^{27a})R^{27}$, $-C(S)N(R^{27a})R^{27}$, $-N(R^{27a})R^{27}$, $-C(=NOR^{25})R^{26}$ or $-S(O)_2N(R^{27a})R^{27}$,

each of $R^{25}$, $R^{25a}$ and $R^{26}$ is independently $C_1$-$C_6$ alkyl or ($C_1$-$C_6$) alkyl optionally substituted with $R^{32a}$,

each of $R^{27}$ and $R^{27a}$ is independently a hydrogen atom, $C_1$-$C_6$ alkyl, $-S(O)_rR^{34}$ or $-C(O)R^{34}$,

each of $R^{28}$ and $R^{28a}$ is independently a halogen atom, -OH, $-OR^{29}$, -SH or $-S(O)_rR^{29}$, each of $R^{29}$ and $R^{33}$ is independently $C_1$-$C_6$ alkyl,

each of $R^{32}$ and $R^{32a}$ is independently a halogen atom, $-OR^{33}$ or $-S(O)_rR^{33}$ $R^{34}$ is $C_1$-$C_6$ alkyl, $C_3$-$C_8$ cycloalkyl or $C_1$-$C_6$ haloalkyl,

each of n, g1 and f1 is independently an integer of from 0 to 3,

each of g2 and f2 is independently an integer of from 0 to 2,

g3 is an integer of from 0 to 1,

each of g4 and f4 is independently an integer of from 0 to 4,

f5 is an integer of from 0 to 5,

f6 is an integer of from 0 to 6,

f7 is an integer of from 0 to 7,

f8 is an integer of from 0 to 8,

f9 is an integer of from 0 to 9,

q is an integer of from 1 to 5,
q2 is an integer of from 0, 1 or 3 to 5,
each of m1, m2 and m3 is independently an integer of from 0 or 1, and
r is an integer of from 0 to 2.

**2.** The pyrazole derivative or a salt thereof according to claim 1, wherein $R^b$ is $-C(O)R^7$, $-C(S)R^7$, $-C(O)OR^{6a}$, $-C(O)SR^{6a}$, $-C(S)OR^{6a}$, $-C(S)SR^{6a}$, $-C(O)N(R^{8a})R^8$, $-C(S)N(R^{8a})R^8$, $-C(O)N(R^{8b})N(R^{8a})R^8$, $-C(O)N(R^{8a})OR^{6a}$, $-C(=NR^{8b})OR^{6a}$, $-C(=NR^{8b})SR^{6a}$, $-C(=NR^{8b})N(R^{8a})R^8$, $-C(=NR^{8b})R^7$, D1-49, D1-51, D1-53, D1-59, D1-61 or D1-63, or $R^b$ may form $=C(R^{b2})R^{b3}$ together with $R^a$.

**3.** The pyrazole derivative or a salt thereof according to claim 2, wherein $A^1$ is $-CR^1$, each of $R^1$ and $R^3$ is independently a hydrogen atom, a halogen atom, cyano, $-NH_2$, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfinyl, $C_1$-$C_6$ alkylsulfonyl or $C_1$-$C_6$ alkoxycarbonyl,
$R^2$ is a halogen atom, $C_1$-$C_6$ alkyl or $C_1$-$C_6$ alkoxy, and
$R^4$ is a hydrogen atom, a halogen atom, cyano, $-NH_2$, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfinyl or $C_1$-$C_6$ alkylsulfonyl.

**4.** The pyrazole derivative or a salt thereof according to claim 2, wherein $A^1$ is $-N(-O)_{m2}$,
$R^2$ is a halogen atom, $C_1$-$C_6$ alkyl or $C_1$-$C_6$ alkoxy, and
each of $R^3$ and $R^4$ is independently a hydrogen atom, a halogen atom, $C_1$-$C_6$ alkyl or $C_1$-$C_6$ alkoxy.

**5.** The pyrazole derivative or a salt thereof according to claim 3, wherein $R^1$ is a hydrogen atom, a halogen atom, cyano, $-NH_2$, $C_1$-$C_6$ alkyl, $C_1$-$C_6$ haloalkyl or $C_1$-$C_6$ alkoxy,
$R^2$ is $C_1$-$C_6$ alkyl,
$R^3$ is a hydrogen atom, a halogen atom, cyano, $C_1$-$C_6$ alkyl, ($C_1$-$C_6$) alkyl optionally substituted with $R^{28a}$, $C_1$-$C_6$ alkoxy, $C_1$-$C_6$ alkylthio, $C_1$-$C_6$ alkylsulfonyl or $C_1$-$C_6$ alkoxycarbonyl,
$R^4$ is a hydrogen atom, a halogen atom, $C_1$-$C_6$ alkyl or $C_1$-$C_6$ alkylthio,
$R^a$ is a hydrogen atom, $C_1$-$C_6$ alkyl, ($C_1$-$C_6$) alkyl optionally substituted with $R^5$, $C_2$-$C_6$ alkenyl, $C_2$-$C_6$ alkynyl, $-S(O)_rR^6$, $-C(O)R^{7a}$, $-C(O)OR^{6a}$, $-NR^{8c}R^{8d}$ or $-C(=NR^{8b})R^{7a}$,
$R^b$ is $-C(O)R^7$, $-C(S)R^7$, $-C(O)OR^{6a}$, $-C(O)SR^{6a}$, $-C(O)N(R^{8a})R^8$, $-C(S)N(R^{8a})R^8$, $-C(O)N(R^{8b})N(R^{8a})R^8$, $-C(=NR^{8b})SR^{6a}$, $-C(=NR^{8b})N(R^{8a})R^8$, $-C(=NR^{8b})R^7$ or D1-51, or $R^b$ may form $=C(R^{b2})R^{b3}$ together with $R^a$,
$R^{b2}$ is a hydrogen atom, $C_1$-$C_6$ alkyl or $-S(O)_rR^6$,
$R^{b3}$ is ($C_1$-$C_6$) alkyl optionally substituted with $R^{14}$, $-OR^6$ or $-N(R^{8b})R^8$, or $R^{b3}$ may form, together with $R^{b2}$, a $C_4$ alkylene chain to form a 5-membered ring together with the carbon atom attached to $R^{b3}$ and $R^{b2}$, wherein the alkylene chain may contain a sulfur atom and a nitrogen atom and may optionally be substituted with $-C(O)R^7$,
$R^5$ is a halogen atom, cyano, $C_3$-$C_8$ cycloalkyl, $-OR^{11}$, $-S(O)_rR^{11}$, $-C(O)OR^{11a}$, $-C(=NOR^{11})R^{12}$, $-Si(R^{9a})(R^{9b})R^9$, phenyl or D1-37,
$R^6$ is $C_1$-$C_6$ alkyl or ($C_1$-$C_6$) alkyl optionally substituted with $R^{14}$,
$R^{6a}$ is $C_1$-$C_6$ alkyl or phenyl substituted with (Z)q,
$R^7$ is $C_1$-$C_{15}$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$) alkenyl optionally substituted with $R^{14}$, $C_3$-$C_8$ cycloalkenyl, $C_2$-$C_6$ alkynyl, ($C_2$-$C_6$) alkynyl optionally substituted with $R^{14}$, $-C(O)OR^{11a}$, $-C(O)R^{12}$, $-C(O)N(R^{13a})R^{13}$, $-C(=NOR^{11})R^{12}$, phenyl substituted with (Z)q, D1-1, D1-2, D1-4, D1-5, D1-6, D1-8, D1-9, D1-10, D1-12, D1-13, D1-19, D1-32, D1-33, D1-35, D1-38, D1-45, D1-81, D1-82, D1-87, D1-88, D1-92 or D1-94, or $R^7$ may form, together with $R^a$, a $C_2$-$C_4$ alkylene chain or a $C_2$-$C_4$ alkenylene chain containing a double bond to form a 4 to 6-membered ring together with the carbon atom attached to $R^7$ and the nitrogen atom attached to $R^a$, wherein the alkylene chain may contain one or two oxygen atoms or nitrogen atoms and may optionally be substituted with a halogen atom, $C_1$-$C_6$ alkyl, ($C_1$-$C_6$) alkyl optionally substituted with $R^{14}$, $-OR^{11}$, $-S(O)_rR^{11}$, an oxo group or a methylidene group,
$R^{78}$ is a hydrogen atom or $C_1$-$C_6$ alkyl,
$R^8$ is a hydrogen atom, $C_1$-$C_6$ alkyl, ($C_1$-$C_6$) alkyl optionally substituted with $R^{14}$, $C_3$-$C_8$ cycloalkyl, $C_2$-$C_6$ alkenyl, $C_2$-$C_6$ alkynyl, $-C(O)R^{12}$, $-C(O)OR^{11a}$, $-C(=NOR^{11})R^{12}$, $-S(O)_2R^{11a}$, phenyl, phenyl substituted with (Z)q, D1-32, D1-33, D1-34 or D1-80,
$R^{8a}$ is a hydrogen atom, $C_1$-$C_6$ alkyl or ($C_1$-$C_6$) alkyl optionally substituted with $R^{14a}$, or $R^{8a}$ may form, together with $R^a$, a $C_2$-$C_3$ alkylene chain to form a 5 to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group, or $R^8$ and $R^{8a}$ may form, together with each other, a $C_5$ alkylene chain to form a 6-membered ring together with the nitrogen atom attached to $R^8$ and $R^{8a}$, wherein the alkylene chain may optionally be substituted with $C_1$-$C_6$ alkyl, or $R^8$ may form $=C(R^{8f})R^{8e}$ together with

$R^{8a}$,
$R^{8b}$ is a hydrogen atom, cyano, $C_1$-$C_6$ alkyl or -$OR^{11}$,
$R^{8c}$ is a hydrogen atom, $C_1$-$C_6$ alkyl or -C(O)$OR^{11a}$,
$R^{8d}$ is a hydrogen atom, -C(O)$OR^{11a}$ or -C(O)$R^{12}$,
$R^{8e}$ is a hydrogen atom,
$R^{8f}$ is -N($R^{13a}$)$R^{13}$,
each of $R^9$, $R^{9a}$, $R^{9b}$ and $R^{10}$ is independently $C_1$-$C_6$ alkyl,
$R^{11}$ is $C_1$-$C_6$ alkyl, ($C_1$-$C_6$) alkyl optionally substituted with $R^{14a}$ or $C_1$-$C_6$ alkenyl,
$R^{11a}$ is $C_1$-$C_6$ alkyl, ($C_1$-$C_6$) alkyl optionally substituted with $R^{14a}$ or phenyl,
$R^{12}$ is a hydrogen atom, $C_1$-$C_6$ alkyl, ($C_1$-$C_6$) alkyl optionally substituted with $R^{14a}$, $C_3$-$C_8$ cycloalkyl, -C(=$NOR^{15}$)$R^{16}$ or D1-2,
$R^{13}$ is $C_1$-$C_6$ alkyl or phenyl,
$R^{13a}$ is a hydrogen atom or $C_1$-$C_6$ alkyl,
$R^{14}$ is a halogen atom, cyano, $C_3$-$C_8$ cycloalkyl, -OH, -$OR^{15}$, -SH, -$S(O)_rR^{15}$, -S(=$NR^{17b}$)$R^{15a}$, -S(O)(=$NR^{17b}$)$R^{15a}$, -C(O)OH, -C(O)$OR^{15a}$, -C(O)N($R^{17a}$)$R^{17}$, -C(=$NOR^{15}$)$R^{16}$, -N($R^{17a}$)$R^{17}$, -ON=C($R^{16a}$)$R^{16}$, -$S(O)_2N(R^{17a})R^{17}$, -Si($R^{9a}$)($R^{9b}$)$R^9$, -P(O)$(OR^{10})_2$, phenyl, phenyl substituted with (Z)q, D1-1, D1-2, D1-5, D1-7, D1-8, D1-28, D1-32, D1-33, D1-34, D1-84, D1-85, D1-87, D1-93 or D1-98,
$R^{14a}$ is a halogen atom, -$OR^{15}$, -$S(O)_rR^{15}$, -Si($R^{9a}$)($R^{9b}$)$R^9$ or phenyl,
$R^{15}$ is cyano, $C_1$-$C_6$ alkyl, $C_2$-$C_6$ alkenyl, $C_2$-$C_6$ alkynyl, ($C_1$-$C_6$) alkyl optionally substituted with $R^{19}$, -C(O)$R^{21}$, phenyl substituted with $(Z)_{q2}$, D1-12, D1-32, D1-37 or D1-51,
$R^{15a}$ is $C_1$-$C_6$ alkyl,
$R^{16}$ is $C_1$-$C_6$ alkyl or ($C_1$-$C_6$) alkyl optionally substituted with $R^{19}$,
$R^{16a}$ is a hydrogen atom,
$R^{17}$ is a hydrogen atom, $C_1$-$C_6$ alkyl, ($C_1$-$C_6$) alkyl optionally substituted with $R^{19}$, -$S(O)_rR^{20}$, -C(O)$OR^{20a}$, -C(O)$R^{21}$, -C(O)N($R^{22a}$)$R^{22}$, -C(S)N($R^{22a}$)$R^{22}$, -C(=$NOR^{20}$)$R^{21}$, -$S(O)_2N(R^{22a})R^{22}$ or phenyl,
$R^{17a}$ is a hydrogen atom or $C_1$-$C_6$ alkyl, or $R^{17a}$ may form =C($R^{17c}$)$R^{17d}$ together with $R^{17}$, $R^{17b}$ is cyano,
$R^{17c}$ is -N($R^{22a}$)$R^{22}$,
$R^{17d}$ is a hydrogen atom,
$R^{19}$ is a halogen atom, cyano, $C_3$-$C_8$ cycloalkyl, -$OR^{20}$, -$S(O)_rR^{20}$, -C(O)$OR^{20a}$, -C(O)N($R^{22a}$)$R^{22}$, -C(=$NOR^{20}$)$R^{21}$, -Si($R^{9a}$)($R^{9b}$)$R^9$, phenyl or D1-34,
$R^{20}$ is $C_1$-$C_6$ alkyl or ($C_1$-$C_6$) alkyl optionally substituted with $R^{32}$,
$R^{20a}$ is $C_1$-$C_6$ alkyl,
$R^{21}$ is a hydrogen atom, $C_1$-$C_6$ alkyl or ($C_1$-$C_6$) alkyl optionally substituted with $R^{32}$,
$R^{22}$ is $C_1$-$C_6$ alkyl,
$R^{22a}$ is a hydrogen atom or $C_1$-$C_6$ alkyl,
$X^1$ is a halogen atom, cyano, nitro, $C_1$-$C_6$ alkyl, ($C_1$-$C_6$) alkyl optionally substituted with $R^{28}$, $C_3$-$C_8$ cycloalkyl, -$OR^{25}$, -$S(O)_rR^{25}$, -C(O)$R^{26}$, -C(=$NOR^{25}$)$R^{26}$ or -$S(O)_2N(R^{27a})R^{27}$, provided that when g2 is an integer of 2, each $X^1$ may be identical with or different from each other,
$X^{18}$ is $C_1$-$C_6$ alkyl, ($C_1$-$C_6$) alkyl optionally substituted with $R^{28}$, -C(O)$OR^{25a}$ or -C(O)$R^{26}$, $X^{1b}$ is ($C_1$-$C_6$) alkyl optionally substituted with $R^{28}$, provided that when f5 is an integer of 3, each $X^{1b}$ may be identical with or different from one another, and when there are two $X^{1b}$'s on the same carbon, the two $X^{1b}$'s may form oxo together with each other,
Z is a halogen atom, nitro, $C_1$-$C_6$ alkyl, ($C_1$-$C_6$) alkyl optionally substituted with $R^{28}$, -$S(O)_rR^{25}$, -C(O)OH or -C(O)N($R^{27a}$)$R^{27}$, provided that when q is an integer of at least 2, and there are two neighboring Z's , the two neighboring Z's may form -$OCH_2O$- to form, together with the carbon atoms attached to the Z's, a 5-membered ring which may have one or more hydrogen atoms on the ring-constituting carbon atoms optionally replaced by one or more halogen atoms,
each of $R^{25}$ and $R^{26}$ is independently $C_1$-$C_6$ alkyl or ($C_1$-$C_6$) alkyl optionally substituted with $R^{32a}$,
each of $R^{25a}$, $R^{29}$, $R^{33}$ and $R^{34}$ is independently $C_1$-$C_6$ alkyl,
$R^{27}$ is a hydrogen atom or -$S(O)_rR^{34}$,
$R^{27a}$ is a hydrogen atom,
each of $R^{28}$ and $R^{28a}$ is independently a halogen atom, -OH, -$OR^{29}$ or -$S(O)_rR^{29}$,
$R^{32}$ is a halogen atom, -$OR^{33}$ or -$S(O)_rR^{33}$,
$R^{32a}$ is a halogen atom or -$S(O)_rR^{33}$,
n is an integer of from 0 to 1,
each of g1 and g2 is independently an integer of from 0 to 2,
each of g3, f7, f9 and m3 is independently an integer of 0,
g4 is an integer of from 0 to 1,
f5 is an integer of from 0 to 3,

q is an integer of from 1 to 2,
q2 is an integer of 1, and
r is an integer of from 0 to 2.

**6.** The pyrazole derivative or a salt thereof according to claim 1, wherein $R^1$, $R^2$ and $R^4$ are hydrogen atoms,
$R^a$ is a hydrogen atom or $C_1$-$C_6$ alkyl,
$R^b$ is $-S(O)_rR^6$,
$R^6$ is $C_1$-$C_6$ alkyl, $C_2$-$C_6$ alkenyl or ($C_1$-$C_6$) alkyl optionally substituted with $R^{14}$,
$R^{14}$ is $-S(O)_rR^{15}$,
$R^{15}$ is $C_1$-$C_6$ alkyl, and
r is an integer of from 0 to 2.

**7.** The pyrazole derivative or a salt thereof according to claim 5, wherein $R^7$ is $C_1$-$C_{15}$ alkyl, ($C_1$-$C_6$) alkyl optionally substituted with $R^{14}$, $C_3$-$C_8$ cycloalkyl, ($C_3$-$C_8$) cycloalkyl optionally substituted with $R^{14}$, $C_2$-$C_6$ alkenyl, ($C_2$-$C_6$) alkenyl optionally substituted with $R^{14}$, $C_3$-$C_8$ cycloalkenyl, $C_2$-$C_6$ alkynyl, ($C_2$-$C_6$) alkynyl optionally substituted with $R^{14}$, $-C(O)OR^{11a}$, $-C(O)R^{12}$, $-C(O)N(R^{13a})R^{13}$, $-C(=NOR^{11})R^{12}$, phenyl substituted with (Z)q, D1-1, D1-2, D1-4, D1-5, D1-6, D1-8, D1-9, D1-10, D1-12, D1-13, D1-19, D1-32, D1-33, D1-35, D1-38, D1-45, D1-81, D1-82, D1-87, D1-88, D1-92 or D1-94,
$R^{8a}$ is a hydrogen atom, $C_1$-$C_6$ alkyl or ($C_1$-$C_6$) alkyl optionally substituted with $R^{14a}$, or
$R^8$ may form, together with $R^{8a}$, a $C_5$ alkylene chain to form a 6-membered ring together with the nitrogen atom attached to $R^8$ and $R^{8a}$, wherein the alkylene chain may optionally be substituted with $C_1$-$C_6$ alkyl, or $R^8$ and $R^{8a}$ may form $=C(R^{8f})R^{8e}$ together with each other.

**8.** The pyrazole derivative or a salt thereof according to claim 5, wherein $R^7$ may form, together with $R^a$, a $C_2$-$C_4$ alkylene chain or a $C_2$-$C_4$ alkenylene chain containing a double bond to form a 4 to 6-membered ring together with the carbon atom attached to $R^7$ and the nitrogen atom attached to $R^a$, wherein the alkylene chain may contain one or two oxygen atoms or nitrogen atoms and may optionally be substituted with a halogen atom, $C_1$-$C_6$ alkyl, ($C_1$-$C_6$) alkyl optionally substituted with $R^{14}$, $-OR^{11}$, $-S(O)_rR^{11}$, an oxo group or a methylidene group,
$R^8$ is a hydrogen atom, $C_1$-$C_6$ alkyl, ($C_1$-$C_6$) alkyl optionally substituted with $R^{14}$, $C_3$-$C_8$ cycloalkyl, $C_2$-$C_6$ alkenyl, $C_2$-$C_6$ alkynyl, $-C(O)R^{12}$, $-C(O)OR^{11a}$, $-C(=NOR^{11})R^{12}$, $-S(O)_2R^{11a}$, phenyl, phenyl substituted with (Z)q, D1-32, D1-33, D1-34 or D1-80,
$R^{8a}$ may form, together with $R^a$, a $C_2$-$C_3$ alkylene chain to form a 5 to 6-membered ring together with the nitrogen atom attached to $R^{8a}$ and the nitrogen atom attached to $R^a$, wherein the alkylene chain may contain an oxygen atom and may optionally be substituted with ($C_1$-$C_6$) alkyl optionally substituted with $R^{14}$ or an oxo group,
$R^{14}$ is a halogen atom or $-S(O)_rR^{15}$, and
$R^{15}$ is $C_1$-$C_6$ alkyl.

**9.** The pyrazole derivative or a salt thereof according to claim 4, wherein $R^4$ is a hydrogen atom,
$R^3$ is a halogen atom,
$R^a$ is $C_1$-$C_6$ alkyl,
$R^b$ is $-C(O)R^7$,
$R^7$ is ($C_1$-$C_6$) alkyl optionally substituted with $R^{14}$,
$R^{14}$ is $-S(O)_rR^{15}$,
$R^{15}$ is $C_1$-$C_6$ alkyl, and
m2, n and r are integers of 0.

**10.** A pesticide containing one or more pyrazole derivatives or salts thereof selected from the pyrazole derivatives and salts thereof as defined in claims 1 to 9, as active ingredient(s).

**11.** An agrochemical containing one or more pyrazole derivatives or salts thereof selected from the pyrazole derivatives and salts thereof as defined in claims 1 to 9, as active ingredient(s).

**12.** A parasticide for an internal or external parasite in or on a mammal or bird, which contains one or more pyrazole derivatives or salts thereof selected from the pyrazole derivatives and salts thereof as defined in claims 1 to 9, as active ingredient(s).

**13.** An insecticide or miticide containing one or more pyrazole derivatives or salts thereof selected from the pyrazole derivatives and salts thereof as defined in claims 1 to 9, as active ingredient(s).

**14.** A seed treatment agent containing one or more pyrazole derivatives or salts thereof selected from the pyrazole derivatives and salts thereof as defined in claims 1 to 9, as active ingredient(s).

**15.** The seed treatment agent according to claim 14, which is used to treat seeds by dipping.

**16.** A soil treatment agent containing one or more pyrazole derivatives or salts thereof selected from the pyrazole derivatives and salts thereof as defined in claims 1 to 9, as active ingredient(s).

**17.** The soil treatment agent according to claim 16, which is used to treat soil by irrigation.

## INTERNATIONAL SEARCH REPORT

International application No.
PCT/JP2012/052999

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D401/04, A01N43/56, A01N43/78, A01N43/80, A01N43/832, A01N47/18, A01N47/36, A01N55/00, A01P7/04, A61K31/4439, A61K31/5355, A61P33/14, C07D401/14, C07D413/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2011/128304 A2 (Bayer CropScience AG.), 20 October 2011 (20.10.2011), claims; page 45 (Family: none) | 1-17 |
| A | JP 63-174905 A (Tokuyama Soda Co., Ltd.), 19 July 1988 (19.07.1988), compound no.123 (Family: none) | 1-3,5,7,8,11 |
| A | JP 62-153273 A (Tokuyama Soda Co., Ltd.), 08 July 1987 (08.07.1987), compound no.61 (Family: none) | 1-3,5,7,8, 10,11,16,17 |

☒ Further documents are listed in the continuation of Box C.
☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 April, 2012 (17.04.12) | 01 May, 2012 (01.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/052999

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2004-231528 A (Sankyo Agro Co., Ltd.),<br>19 August 2004 (19.08.2004),<br>claims; page 11, tables<br>(Family: none) | 1-4,10-17<br>5-9 |
| X<br>A | JP 2007-529497 A (Pfizer Inc.),<br>25 October 2007 (25.10.2007),<br>claims; examples<br>& WO 2005/090313 A1 & AU 2005223483 A<br>& CA 2560510 A & EP 1735284 A1<br>& US 2008/0261940 A1 | 1,6,10-17<br>2-5,7-9 |
| X<br>A | JP 62-292760 A (Sand AG.),<br>19 December 1987 (19.12.1987),<br>claims; examples<br>& EP 248765 A2 & DK 8702900 A<br>& AU 8773824 A & BR 8702876 A<br>& CN 87104091 A & HU 45696 A<br>& ZA 8704059 A | 1-4,10-17<br>5-9 |
| A | JP 5-86054 A (Rhone-Poulenc Agrochimie),<br>06 April 1993 (06.04.1993),<br>claims<br>& EP 500209 A1 & NO 9200097 A<br>& CA 2059088 A & AU 9210251 A<br>& BR 9200219 A & IL 100678 A<br>& FI 9200221 A & HU 62571 A<br>& CN 1063283 A & US 5306694 A | 1-17 |
| A | JP 8-193067 A (Nippon Soda Co., Ltd.),<br>30 July 1996 (30.07.1996),<br>claims<br>(Family: none) | 1-17 |
| A | JP 2001-526669 A (Bayer AG.),<br>18 December 2001 (18.12.2001),<br>claims<br>& DE 19721031 A & CA 2290379 A<br>& WO 98/52938 A1 & AU 9880155 A<br>& EP 983261 A1 & BR 9815524 A | 1-17 |
| A | JP 2009-542686 A (Aventis Agriculture),<br>03 December 2009 (03.12.2009),<br>claims<br>& WO 2008/005489 A2 & AU 2007269584 A<br>& CA 2656617 A & US 2008/0031902 A1<br>& EP 2035390 A2 & ZA 2008010673 A<br>& KR 2009029284 A & CN 101511795 A<br>& US 2010/0249194 A1 | 1-17 |

Form PCT/ISA/210 (continuation of second sheet) (July 2009)

INTERNATIONAL SEARCH REPORT

International application No.
PCT/JP2012/052999

Continuation of A. CLASSIFICATION OF SUBJECT MATTER
(International Patent Classification (IPC))

*C07D401/04*(2006.01)i, *A01N43/56*(2006.01)i, *A01N43/78*(2006.01)i, *A01N43/80*(2006.01)i, *A01N43/832*(2006.01)i, *A01N47/18*(2006.01)i, *A01N47/36*(2006.01)i, *A01N55/00*(2006.01)i, *A01P7/04*(2006.01)i, *A61K31/4439*(2006.01)i, *A61K31/5355*(2006.01)i, *A61P33/14*(2006.01)i, *C07D401/14*(2006.01)i, *C07D413/14*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011128304 A
- WO 2009076454 A
- WO 2008044767 A
- US 2004214838 A
- JP 2003313103 A
- WO 99010350 A
- WO 97034893 A
- JP 63174905 A
- JP 62153273 A
- WO 2011048082 A
- US 2005146823 A
- JP 2011025875 A
- JP 2011106993 A
- JP 2011143871 A
- JP 2011176256 A
- JP 2011223837 A
- JP 2011234671 A
- JP 2011252596 A

### Non-patent literature cited in the description

- Farmaco. 1970, vol. 25, 592
- *Tetrahetron: Asymmetry,* 2003, vol. 14, 2587
- *Synlett,* 2004, vol. 4, 703
- *Journal of Organic Chemistry,* 2004, vol. 69, 5578
- *Journal of Heterocyclic Chemistry,* 1987, vol. 24, 1669
- *Journal of Heterocyclic Chemistry,* 1987, vol. 24, 693
- *Journal of Medicinal Chemistry,* 2002, vol. 45, 5397
- The Pesticide Manual. 2009